(19) Europäisches Patentamt
European Patent Office
Office européen des brevets

(11) EP 2 792 360 A1

(12) EUROPEAN PATENT APPLICATION

(43) Date of publication:
22.10.2014 Bulletin 2014/43

(51) Int Cl.:
*A61K 31/55* (2006.01) *C07D 487/04* (2006.01)

(21) Application number: 14165031.7

(22) Date of filing: 16.04.2014

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME

(30) Priority: 18.04.2013 EP 13164373
25.04.2013 EP 13165452
25.04.2013 EP 13165455
25.04.2013 EP 13165458
16.05.2013 EP 13168149
16.05.2013 EP 13168148
23.05.2013 EP 13169045

(71) Applicant: IP Gesellschaft für Management mbH
50931 Köln (DE)

(72) Inventor: Trinius, Frank
50931 Köln (DE)

(54) **(1aR,12bS)-8-cyclohexyl-11-fluoro-N-((1-methylcyclopropyl)sulfonyl)-1a-((3-methyl-3,8-diazabicyclo[3.2.1]oct-8-yl)carbonyl)-1,1a,2,2b-tetrahydrocyclopropa[d]indolo[2,1-a][2]benzazepine-5-carboxamide for use in treating HCV**

(57) The invention relates to an administration unit comprising a compound of formula

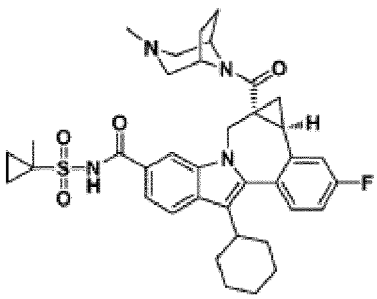

and/or pharmaceutically acceptable salts thereof, and to a packaging comprising the administration unit according to the invention.

EP 2 792 360 A1

## Description

CROSSREFERENCES

The present application claims priorities of European patent application no. EP13164373.6, filed on 18 April 2013; European patent application no. EP13165452.7, filed on 25 April 2013; European patent application no. EP13165455.0, filed on 25 April 2013; European patent application no. EP13165458.4, filed on 25 April 2013; European patent application no. EP13168149.6, filed on 16 May 2013; European patent application no. EP13168148.8, filed on 16 May 2013; and European patent application no. EP13169045.5, filed on 23 May 2013.

BACKGROUND ART

Many pharmaceuticals are marketed without any packaging. In various countries, small drug shops are a common source of medicine where stocking of prescription-only medicines and unpackaged tablets is the norm (Goodman C et al., Health Policy Plan. 2007 Nov;22(6):393-403). Furthermore, self-medication is common practice in various countries. For example, the most frequently proposed drugs to treat male urethritis are: ampicillin 250-mg capsules (44%); oxytetracyline 250-mg capsules (24%); and cotrimoxazole 450-mg tablets (12%), and these drugs are frequently sold unpackaged (Sow PS et al., Int J Infect Dis. 2002 Jun;6(2):108-12).

In Germany and other European countries, numerous pharmaceuticals are marketed in packaging wherein every single administration unit is individually packaged in a single packaging. Examples include but are not limited to Frubiase® Calcium (Boehringer Ingelheim) marketed as a liquid administration unit that is individually packaged in a glass ampoule; Orthomol Immun® Direktgranulat (Orthomol) marketed as administration unit in granulate form that is individually packaged in a bag; Orthomol Vital m® (Orthomol) marketed as a liquid administration unit that is individually packaged in a bottle having a lid containing an individually packaged tablet; Vitasprint® (Pfizer) and Celestamine® (MSD Sharp & Dohme) each marketed as a liquid administration unit that is individually packaged in a bottle; Alka-Seltzer® (Bayer) marketed as effervescent tablet that is individually packaged in a bag; Aspirin® Complex (Bayer), Aspirin® Effect (Bayer), Helmex® (Infectopharm), Monuril® Granulat (Pierre Fabre Pharma) each marketed as administration unit in granulate form that individually packaged in a bag; ellaOne® (HRA Pharma), Levonelle® (Bayer Schering Pharma), Pidana® (HRA Pharma), Fungata® (Pfizer), and Canesten® Gyn once (Bayer) each marketed as a single tablet that is individually packaged in a blister. Furthermore, numerous single-to-use syringes are marketed individually packaged.

It is an object of the invention to provide administration units containing drugs that have advantages compared to conventional administration units and conventional drugs, respectively. It is also an object of the invention to provide packaging containing administration units containing drugs that have advantages compared to conventional packaging. These objects have been achieved by the present invention.

SUMMARY OF THE INVENTION

The present invention relates to different drugs (active pharmaceutical ingredients) and certain forms of said drugs, such as stereoisomers, salts and/or polymorphs thereof. The present invention covers various aspects that are numbered (i), (ii), (iii), (iv), (v), (vi), and (vii). Aspects (i), (ii), (iii), (iv), (v), (vi), and (vii) are separate from one another.

The present invention relates to

an administration unit comprising (i) {drug1a}, {drug1b}, {drug1c}, or {drug1d}; (ii) {drug2a}; (iii) {drug3a}; (iv) {drug4a}, {drug4b}, {drug4c}, {drug4d}, {drug4e}, {drug4f}, or {drug4g}; (v) {drug5a} or {drug5b}; (vi) {drug6a}; or (vii) {drug7a};

a packaging comprising one or more of such administration units;

a method for manufacturing such an administration unit;

a method for manufacturing such a packaging; and

particles of (i) {drug1a}, {drug1b}, {drug1c}, or {drug1d}; (ii) {drug2a}; (iii) {drug3a}; (iv) {drug4a}, {drug4b}, {drug4c}, {drug4d}, {drug4e}, {drug4f}, or {drug4g}; (v) {drug5a} or {drug5b}; (vi) {drug6a}; or (vii) {drug7a}.

Certain embodiments of the invention are also disclosed in WO/2013/055584, WO/2013/059265, WO/2013/057722, WO/2013/057124, WO/2013/071272, WO/2013/068909, and WO/2013/074432, respectively. Any embodiment disclosed in any of these references is incorporated herein by reference as a preferred embodiment of the invention.

GENERAL DEFINITIONS

To avoid unnecessary repetitions, the description refers to the following abbreviations the meaning of which is defined hereinafter:

Aspect (i) of the present invention relates to 8-chloro-N-[(2-chloro-5-methoxyphenyl)sulfonyl]-6-(trifluoromethyl)-imidazo[1,2-alpha]pyridine-2-carboxamide

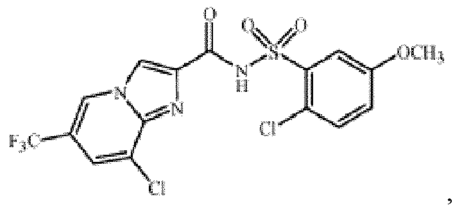

,

abbreviated as '{drug1}'. In particular, the present invention relates to polymorph A of 8-chloro-N-[(2-chloro-5-methoxyphenyl)sulfonyl]-6-(trifluoromethyl)-imidazo[1,2-alpha]pyridine-2-carboxamide, abbreviated as '{drug1a}', polymorph B of 8-chloro-N-[(2-chloro-5-methoxyphenyl)sulfonyl]-6-(trifluoromethyl)-imidazo[1,2-alpha]pyridine-2-carboxamide, abbreviated as '{drug1b}', polymorph C of 8-chloro-N-[(2-chloro-5-methoxyphenyl)sulfonyl]-6-(trifluoromethyl)-imidazo[1,2-alpha]pyridine-2-carboxamide, abbreviated as '{drug1c}', polymorph D of 8-chloro-N-[(2-chloro-5-methoxyphenyl)sulfonyl]-6-(trifluoromethyl)-imidazo[1,2-alpha]pyridine-2-carboxamide, abbreviated as '{drug1d}'; and polymorph TS of 8-chloro-N-[(2-chloro-5-methoxyphenyl)sulfonyl]-6-(trifluoromethyl)-imidazo[1,2-alpha]pyridine-2-carboxamide, abbreviated as '{drug1e}'. {drug1a}, {drug1b}, {drug1c}, {drug1d} and {drug1e} are a specific forms falling within the definition of {drug1}. Aspect (i) of the invention is separate from aspects (ii), (iii), (iv), (v), (vi), and (vii) of the invention. Thus, all embodiments of {drug1a} and {drug1b}, respectively, are only related to {drug1}, but neither to {drug2}, nor to {drug3}, nor to {drug4}, nor to {drug5}, nor to {drug6}, nor to {drug7}.

Aspect (ii) of the present invention relates to } (1aR,12bS)-8-cyclohexyl-11-fluoro-N-((1-methylcyclopropyl)sulfonyl)-1a-((3-methyl-3,8-diazabicyclo[3.2.1]oct-8-yl)carbonyl)-1,1a,2,12b-tetrahydrocyclopropa[d]indolo[2,1-alpha][2]benzazepine-5-carboxamide

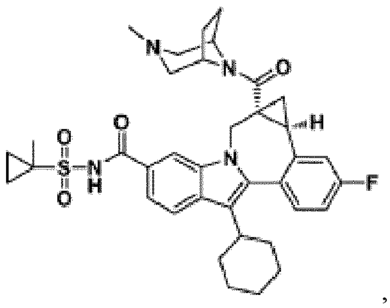

,

abbreviated as '{drug2}'. In particular, the present invention relates to a solid form of (1aR,12bS)-8-cyclohexyl-11-fluoro-N-((1-methylcyclopropyl)sulfonyl)-1a-((3-methyl-3,8-diazabicyclo[3.2.1]oct-8-yl)carbonyl)-1,1a,2, 12b-tetrahydrocyclopropa-[d]indolo[2,1-alpha][2]benzazepine-5-carboxamide and/or pharmaceutically acceptable salts thereof, abbreviated as '{drug2a}'. {drug2a} is a specific form falling within the definition of {drug2}. Aspect (ii) of the invention is separate from aspects (i), (iii), (iv), (v), (vi), and (vii) of the invention. Thus, all embodiments of {drug2a} are only related to {drug2}, but neither to {drug1}, nor to {drug3}, nor to {drug4}, nor to {drug5}, nor to {drug6}, nor to {drug7}.

Aspect (iii) of the present invention relates to 4-[2-(5-amino-1H-pyrazol-4-yl)-4-chlorophenoxy]-5-chloro-2-fluoro-N-(1,3-thiazol-4-yl)benzenesulfonamide, abbreviated as '{drug3}'. In particular, the present invention relates to tosylate salt of 4-[2-(5-amino-1H-pyrazol-4-yl)-4-chlorophenoxy]-5-chloro-2-fluoro-N-(1,3-thiazol-4-yl)benzenesulfonamide, abbreviated as '{drug3a}'. {drug3a} is a specific form falling within the definition of {drug3}. Aspect (iii) of the invention is separate from aspects (i), (ii), (iv), (v), (vi), and (vii) of the invention. Thus, all embodiments of {drug3a} are only related to {drug3}, but neither to {drug1}, nor to {drug2}, nor to {drug4}, nor to {drug5}, nor to {drug6}, nor to {drug7}.

Aspect (iv) of the present invention relates to {drug4} (1,1-dioxo-1lambda6-thiomorpholin-4-yl)-{6-[3-(4-fluoro-phenyl)-5-methyl-isoxazol-4-ylmethoxy]-pyridin-3-yl]-methanone (also referred to as 1,1-dioxo-4-thiomorpholinyl)-[6-[[3-(4-fluorophenyl)-5-methyl-4-isoxazolyl]methoxy]-3-pyridinyl]-methanone):

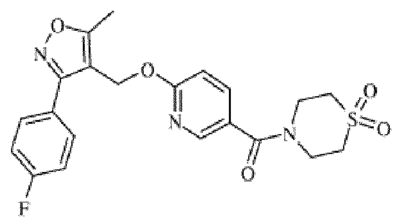

In particular, the present invention relates to form A of (1,1-dioxo-1lambda6-thiomorpholin-4-yl)-{6-[3-(4-fluoro-phenyl)-5-methyl-isoxazol-4-ylmethoxy]-pyridin-3-yl]-methanone, abbreviated as '{drug4a}'; form B of (1,1-dioxo-1lambda6-thiomorpholin-4-yl)-{6-[3-(4-fluoro-phenyl)-5-methyl-isoxazol-4-ylmethoxy]-pyridin-3-yl]-methanone, abbreviated as '{drug4b}'; form C of (1,1-dioxo-1lambda6-thiomorpholin-4-yl)-{6-[3-(4-fluoro-phenyl)-5-methyl-isoxazol-4-ylmethoxy]-pyridin-3-yl]-methanone, abbreviated as '{drug4c}'; form D of (1,1-dioxo-1lambda6-thiomorpholin-4-yl)-{6-[3-(4-fluoro-phenyl)-5-methyl-isoxazol-4-ylmethoxy]-pyridin-3-yl]-methanone, abbreviated as '{drug4d}'; form E of (1,1-dioxo-1lambda6-thiomorpholin-4-yl)-{6-[3-(4-fluoro-phenyl)-5-methyl-isoxazol-4-ylmethoxy]-pyridin-3-yl]-methanone, abbreviated as '{drug4e}'; amorphous form of (1,1-dioxo-1lambda6-thiomorpholin-4-yl)-{6-[3-(4-fluoro-phenyl)-5-methyl-isoxazol-4-ylmethoxy]-pyridin-3-yl]-methanone, abbreviated as '{drug4f}'; and gamma-CD inclusion complex ({drug4g}) of (1,1-dioxo-1lambda6-thiomorpholin-4-yl)-{6-[3-(4-fluoro-phenyl)-5-methyl-isoxazol-4-ylmethoxy]-pyridin-3-yl]-methanone, abbreviated as '{drug4g}'. {drug4a}, {drug4b}, {drug4c}, {drug4d}, {drug4e}, {drug4f}, and {drug4g}, respectively, are specific forms falling within the definition of {drug4}. Aspect (iv) of the invention is separate from aspects (i), (ii), (iii), (v), (vi), and (vii) of the invention. Thus, all embodiments of {drug4a} are only related to {drug4}, but neither to {drug 1}, nor to {drug2}, nor to {drug3}, nor to {drug5}, nor to {drug6}, nor to {drug7}.

Aspect (v) of the present invention relates to compound of formula (I)

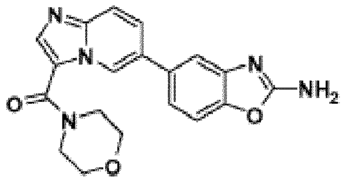

(I),

abbreviated as '{drug5}'. In particular, the present invention relates to crystalline form of HCl salt of compound of formula (I), abbreviated as '{drug5a}', and crystalline form of HBr salt of compound of formula (I), abbreviated as '{drug5b}'. {drug5a} and {drug5b} are specific forms falling within the definition of {drug5}. Aspect (v) of the invention is separate from aspects (i), (ii), (iii), (iv), (vi), and (vii) of the invention. Thus, all embodiments of {drug5a} are only related to {drug5}, but neither to {drug1}, nor to {drug2}, nor to {drug3}, nor to {drug4}, nor to {drug6}, nor to {drug7}.

Aspect (vi) of the present invention relates to N-methyl-2-[3-((E)-2-pyridin-2-yl-vinyl)-1H-indazol-6-ylsulfanyl]-benzamide

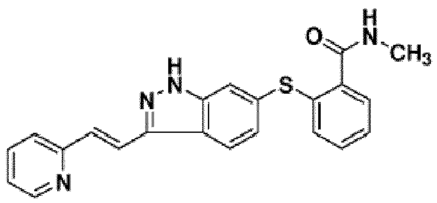

for the treatment of chronic myolegenous leukemia, abbreviated as '{drug6}'. In particular, the present invention relates to solid form of free molecule or pharmaceutical acceptable salts of N-methyl-2-[3-((E)-2-pyridin-2-yl-vinyl)-1H-indazol-6-ylsulfanyl]-benzamide for the treatment of chronic myolegenous leukemia, abbreviated as '{drug6a}'. {drug6a} is a specific form falling within the definition of {drug6}. Aspect (vi) of the invention is separate from aspects (i), (ii), (iii), (iv), (v), and (vii) of the invention. Thus, all embodiments of {drug6a} are only related to {drug6}, but neither to {drug1}, nor to {drug2}, nor to {drug3}, nor to {drug4}, nor to {drug5}, nor to {drug7}.

Aspect (vii) of the present invention relates to 4-Methyl-3-[[4-(3-pyridinyl)-2-pyrimidinyl]amino]-N-[5-(4-methyl-1H-imidazol-1-yl)-3-(trifluoromethyl)phenyl] benzamide or pharmaceutical acceptable salt thereof

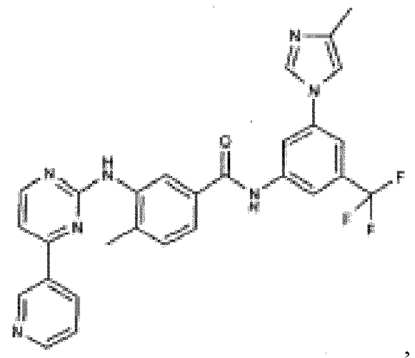

,

abbreviated as '{drug7}'. In particular, the present invention relates to solid dosage form comprising: (a) a core comprising 4-Methyl-3-[[4-(3-pyridinyl)-2-pyrimidinyl]amino]-N-[5-(4-methyl-1H-imidazol-1-yl)-3-(trifluoromethyl)phenyl] benzamide or pharmaceutical acceptable salt thereof and excipients; and (b) at least one polymer, said polymer coating said core, wherein disintegration of said solid dosage form is delayed by 4-15 minutes, which is useful for treating chronic myeloid leukemia and gastrointestinal stromal tumors, abbreviated as '{drug7a}'. {drug7a} is a specific form falling within the definition of {drug7}. Aspect (vii) of the invention is separate from aspects (i), (ii), (iii), (iv), (v), and (vi) of the invention. Thus, all embodiments of {drug7a} are only related to {drug7}, but neither to {drug1} nor to {drug2}, nor to {drug3}, nor to {drug4}, nor to {drug5}, nor to {drug6}.

Summarizing therefore the following abbreviations have the following meanings:

Aspect (i):

{drug1} = 8-chloro-N-[(2-chloro-5-methoxyphenyl)sulfonyl]-6-(trifluoromethyl)-imidazo[1,2-alpha]pyridine-2-carboxamide

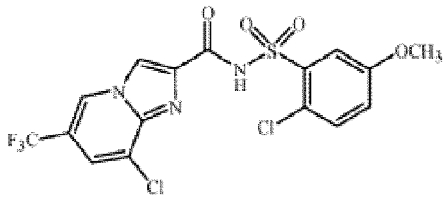

;

{drug1a} = polymorph A of 8-chloro-N-[(2-chloro-5-methoxyphenyl)sulfonyl]-6-(trifluoromethyl)-imidazo[1,2-alpha]-pyridine-2-carboxamide;
{drug1b} = polymorph B of 8-chloro-N-[(2-chloro-5-methoxyphenyl)sulfonyl]-6-(trifluoromethyl)-imidazo[1,2-alpha]-pyridine-2-carboxamide;
{drug1c} = polymorph C of 8-chloro-N-[(2-chloro-5-methoxyphenyl)sulfonyl]-6-(trifluoromethyl)-imidazo[1,2-alpha]-pyridine-2-carboxamide;
{drug1d} = polymorph D of 8-chloro-N-[(2-chloro-5-methoxyphenyl)sulfonyl]-6-(trifluoromethyl)-imidazo[1,2-alpha]-pyridine-2-carboxamide; and
{drug1e} = polymorph TS of 8-chloro-N-[(2-chloro-5-methoxyphenyl)sulfonyl]-6-(trifluoromethyl)-imidazo[1,2-alpha]-pyridine-2-carboxamide.

Aspect (ii):

{drug2} = (1aR,12bS)-8-cyclohexyl-11-fluoro-N-((1-methylcyclopropyl)sulfonyl)-1a-((3-methyl-3,8-diazabicyclo[3.2.1]-oct-8-yl)carbonyl)-1,1a,2,12b-tetrahydrocyclopropa[d]indolo[2,1-alpha][2]benzazepine-5-carboxamide

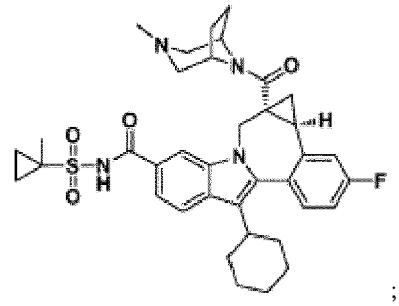

;

{drug2a} = solid form of (1aR,12bS)-8-cyclohexyl-11-fluoro-N-((1-methylcyclopropyl)sulfonyl)-1a-((3-methyl-3,8-diazabicyclo[3.2.1]oct-8-yl)carbonyl)-1,1a,2,12b-tetrahydrocyclopropa[d]indolo[2,1-alpha][2]benzazepine-5-carboxamide and/or pharmaceutically acceptable salts thereof.

Aspect (iii):

{drug3} = 4-[2-(5-amino-1H-pyrazol-4-yl)-4-chlorophenoxy]-5-chloro-2-fluoro-N-(1,3-thiazol-4-yl)benzenesulfonamide;

{drug3a} = tosylate salt of 4-[2-(5-amino-1H-pyrazol-4-yl)-4-chlorophenoxy]-5-chloro-2-fluoro-N-(1,3-thiazol-4-yl)benzenesulfonamid.

Aspect (iv):

{drug4} = (1,1-dioxo-1lambda6-thiomorpholin-4-yl)-{6-[3-(4-fluoro-phenyl)-5-methyl-isoxazol-4-ylmethoxy]-pyridin-3-yl]-methanone

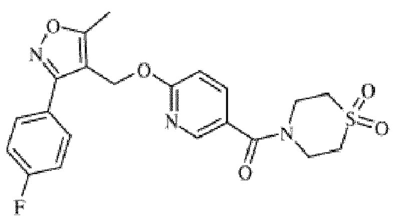

;

{drug4a} = form A of (1,1-dioxo-llambda6-thiomorpholin-4-yl)-{6-[3-(4-fluoro-phenyl)-5-methyl-isoxazol-4-ylmethoxy]-pyridin-3-yl]-methanone;

{drug4b} = form B of (1,1-dioxo-1lambda6-thiomorpholin-4-yl)-{6-[3-(4-fluoro-phenyl)-5-methyl-isoxazol-4-ylmethoxy]-pyridin-3-yl]-methanone;

{drug4c} = form C of (1,1-dioxo-1lambda6-thiomorpholin-4-yl)-{6-[3-(4-fluoro-phenyl)-5-methyl-isoxazol-4-ylmethoxy]-pyridin-3-yl]-methanone;

{drug4d} = form D of (1,1-dioxo-1lambda6-thiomorpholin-4-yl)-{6-[3-(4-fluoro-phenyl)-5-methyl-isoxazol-4-ylmethoxy]-pyridin-3-yl]-methanone;

{drug4e} = form E of (1,1-dioxo-1lambda6-thiomorpholin-4-yl)-{6-[3-(4-fluoro-phenyl)-5-methyl-isoxazol-4-ylmethoxy]-pyridin-3-yl]-methanone;

{drug4f} = amorphous form of (1,1-dioxo-1lambda6-thiomorpholin-4-yl)-{6-[3-(4-fluoro-phenyl)-5-methyl-isoxazol-4-ylmethoxy]-pyridin-3-yl]-methanone; and

{drug4g} = gamma-CD inclusion complex of (1,1-dioxo-1lambda6-thiomorpholin-4-yl)-{6-[3-(4-fluoro-phenyl)-5-methyl-isoxazol-4-ylmethoxy]-pyridin-3-yl]-methanone.

Aspect (v):

{drug5} = compound of formula (I)

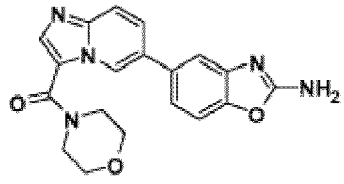

(I);

{drug5a} = crystalline form of HCl salt of compound of formula (I); and
{drug5b} = crystalline form of HBr salt of compound of formula (I).

Aspect (vi):

{drug6} = N-methyl-2-[3-((E)-2-pyridin-2-yl-vinyl)-1H-indazol-6-ylsulfanyl]-benzamide

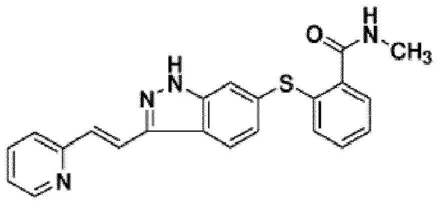

for the treatment of chronic myolegenous leukemia;
{drug6a} = solid form of free molecule or pharmaceutical acceptable salts of N-methyl-2-[3-((E)-2-pyridin-2-yl-vinyl)-1H-indazol-6-ylsulfanyl]-benzamide for the treatment of chronic myolegenous leukemia.

Aspect (vii):

{drug7} = 4-Methyl-3-[[4-(3-pyridinyl)-2-pyrimidinyl]amino]-N-[5-(4-methyl-1H-imidazol-1-yl)-3-(trifluoromethyl)-phenyl] benzamide or pharmaceutical acceptable salt thereof

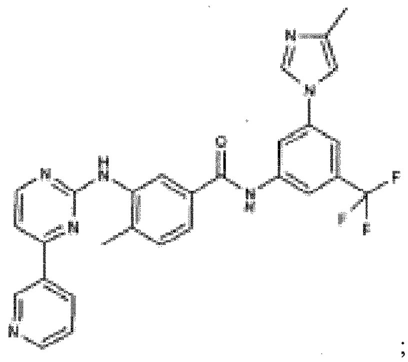

;

{drug7a} = solid dosage form comprising: (a) a core comprising 4-Methyl-3-[[4-(3-pyridinyl)-2-pyrimidinyl]amino]-N-[5-(4-methyl-1H-imidazol-1-yl)-3-(trifluoromethyl)phenyl]benzamide or pharmaceutical acceptable salt thereof and excipients; and (b) at least one polymer, said polymer coating said core, wherein disintegration of said solid dosage form is delayed by 4-15 minutes, which is useful for treating chronic myeloid leukemia and gastrointestinal stromal tumors.

SPECIFIC INORMATION CONCERNING ASPECT (I) OF THE INVENTION

Aspect (i) of the invention relates to a solid form of 8-chloro-N-[(2-chloro-5-methoxyphenyl)sulfonyl]-6-(trifluoromethyl)-imidazo[1,2-alpha]pyridine-2-carboxamide

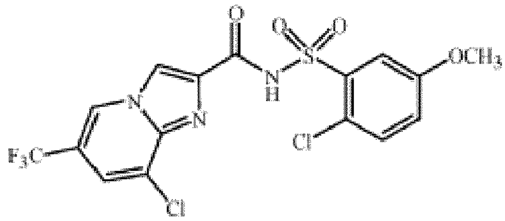

,

preferably to polymorphs A {drug1a}, B {drug1b}, C {drug1c}, D {drug1d}, and TS {drug1e} of 8-chloro-N-[(2-chloro-5-methoxyphenyl)sulfonyl]-6-(trifluoromethyl)-imidazo[1,2-alpha]pyridine-2-carboxamide, which are useful for treating as nematicide, insecticide and/or fungicide, preferred as nematicide to control (including prevention, reduction or elimination) parasitic nematodes. Polymorphs A, B, C and D of 8-chloro-N-[(2-chloro-5-methoxyphenyl)sulfonyl]-6-(trifluoromethyl)-imidazo[1,2-alpha]pyridine-2-carboxamide are described in WO/2013/055584, which is incorporated by reference. As used herein, polymorph A of 8-chloro-N-[(2-chloro-5-methoxyphenyl)sulfonyl]-6-(trifluoromethyl)-imidazo[1,2-alpha]pyridine-2-carboxamide is a crystalline form of 8-chloro-N-[(2-chloro-5-methoxyphenyl)sulfonyl]-6-(trifluoromethyl)-imidazo[1,2-alpha]pyridine-2-carboxamide characterized by a room-temperature powder Cu(Kalpha) X-ray diffraction pattern having at least the 20 reflection positions

| 2θ | 2θ |
|---|---|
| 30.367 | 25.973 |
| 29.131 | 25.604 |
| 27.995 | 24.285 |
| 37.611 | 23.582 |
| 26.49 | 19.789 |

Polymorphs A, B, C and D of 8-chloro-N-[(2-chloro-5-methoxyphenyl)sulfonyl]-6-(trifluoromethyl)-imidazo[1,2-alpha]pyridine-2-carboxamide and other embodiments of this invention is / are described, further characterised and can be obtained as described in WO/2013/055584.

This aspect of the invention relates to solid forms of 8-chloro-N-[(2-chloro-5-methoxyphenyl)sulfonyl]-6-(trifluoromethyl)-imidazo[1,2-a]pyridine-2-carboxamide, their preparation, compositions, and methods of use as nematocides. WO 2010/129500 discloses the nematocidal sulfonamide 8-chloro-N-[(2-chloro-5-methoxyphenyl)sulfonyl]-6-(trifluoromethyl)-imidazo[1,2-a]-pyridine-2-carboxamide and methods for its preparation, as well as the utility of this compound as a nematocide. New solid forms of this compound, their compositions and methods of their preparation and use have now been discovered. This aspect of the invention relates to solid forms of 8-chloro-N-[(2-chloro-5-methoxyphenyl)sulfonyl]-6-(trifluoromethyl)-imidazo[1,2-a]pyridine-2-carboxamide (also referred to as "Compound 1"). More particularly, this invention is directed to a polymorph of Compound 1 ({drug1}) designated Form A ({drug1a}) characterized by a powder X-ray diffraction pattern having at least the 20 reflection positions 30.367, 29.131, 27.995, 27.611, 26.49, 25.973, 25.604, 24.285, 23.582 and 19.789 degrees. This invention also relates to methods for the direct preparation of various solid forms of Compound 1 ({drug1}) (i.e. not starting with other solid forms of Compound 1). More particularly, this invention is directed to a method for preparing a desired polymorph of Compound 1 ({drug1}) comprising: forming a reaction mixture by contacting 2-chloro-5-methoxybenzene sulfonamide and 8-chloro-6-trifluoromethyl-imidazo[1,2-a]pyridine-2-carbonyl chloride in the presence of a first solvent to form a solid form of Compound 1 ({drug1}) and then mixing the solid form of Compound 1 ({drug1}) with a second solvent to convert the solid form to the polymorph Form A ({drug1a}). This invention also relates to methods for the conversion of one solid form of Compound 1 ({drug1}) into another. More particularly, this invention is directed to a method for preparing a polymorph of Compound 1 ({drug1}) designated Form A ({drug1a}), the method comprising: forming a slurry with a solvent of one or more solid forms of Compound 1 ({drug1}) selected from the group of forms B, C, D, solvates, amorphous forms and mixtures thereof with Form A ({drug1a}) and maintaining the slurry while the solid forms of Compound 1 ({drug1}) convert to polymorph Form A ({drug1a}). This invention also relates to compounds used in the method for preparation of Compound 1 ({drug1}) (i.e. 2-chloro-5-methoxybenzene sulfonamide and 8-chloro-6-trifluoromethyl-imidazo[1,2-a]pyridine-2-carbonyl chloride). This invention also relates to a nematocidal composition comprising (a) polymorph Form A ({drug1a}) of Compound 1 ({drug1}) and (b) at least one additional component selected from the group consisting of surfactants, solid diluents and liquid carriers. This invention also relates to a nematocidal composition comprising (a) polymorph Form A ({drug1a}) of Compound 1 ({drug1}); and (b) at least one other nematocide, insecticide and/or fungicide. This invention further relates

to a method protecting a plant from nematodes comprising applying to the plant, or portion, or seed thereof, or to the growing medium of the plant, a nematocidally effective amount of Compound 1 ({drug1}) comprising the polymorph Form A ({drug1a}).

Figure 1 of WO/2013/055584 shows Cu(Kalpha)-powder X-ray diffraction patterns of polymorph Forms A, B, C, D and TS of Compound 1 ({drug1}) showing absolute X-ray intensity in counts graphed against 2θ reflection positions in degrees.

The word "nematocide" is sometimes given the alternative spelling "nematicide" in the art. A nematocide is a compound used to control (including prevention, reduction or elimination) parasitic nematodes. As used to in the present disclosure and claims, the term "nematode" refers to a living organism of the Phylum Nematoda. As generally defined, a "parasite" lives or grows inside or feeds on another living organism (such as a plant) described as the "host". As referred to in the present disclosure and claims a "parasitic nematode" is particularly a nematode that injures or damages tissue or causes other forms of disease in plants. An "infestation" refers to the presence of nematodes in numbers that pose a risk to plants. The presence can be in the environment, e.g., on an agricultural crop or other type of plant. As referred to in the present disclosure and claims, the terms "parasiticidal" and "parasiticidally" refers to observable effects on a parasitic nematode to provide protection of a plant from the nematode. Parasiticidal effects typically relate to diminishing the occurrence or activity of the target parasitic nematode. Such effects on the nematode include necrosis, death, retarded growth, diminished mobility or lessened ability to remain on or in the host plant, reduced feeding and inhibition of reproduction. These effects on parasitic nematodes provide control (including prevention, reduction or elimination) of parasitic infestation of the plant. Therefore "control" of a parasitic nematode means achieving a parasiticidal effect on the nematode. The expressions "parasiticidally effective amount" and "biologically effective amount" in the context of applying a chemical compound to control a parasitic nematode refer an amount of the compound that is sufficient to control the parasitic nematode. The term "agronomic" refers to the production of field crops such as for food and fiber and includes the growth of soybeans and other legumes, cereal (e.g., wheat, oats, barley, rye, rice, maize/corn), leafy vegetables (e.g., lettuce, cabbage, and other cole crops), fruiting vegetables (e.g., tomatoes, pepper, eggplant, crucifers and cucurbits), potatoes, sweet potatoes, grapes, cotton, tree fruits (e.g., pome, stone and citrus), small fruit (berries, cherries) and other specialty crops (e.g., canola, sunflower, olives). The term "nonagronomic" refers to other than field crops, such as horticultural crops (e.g., greenhouse, nursery or ornamental plants not grown in a field), turf (e.g., sod farm, pasture, golf course, lawn, sports field, etc.), agro-forestry and vegetation management. As referred to in the present disclosure and claims, "plant" includes members of Kingdom Plantae, particularly seed plants (Spermatopsida), at all life stages, including young plants (e.g., germinating seeds developing into seedlings) and mature, reproductive stages (e.g., plants producing flowers and seeds). Portions of plants include geotropic members typically growing beneath the surface of the growing medium such as roots, tubers, bulbs and corms, and also members growing above the growing medium, such as foliage (including stems and leaves), flowers, fruits and seeds. Growing mediums include soil, liquid nutrent mediums, gel nutrent mediums or soil mixes with peat, bark, saw dust, sand, pumice, perlite, vermiculite and other similar products. As referred to herein, the term "seedling", used either alone or in a combination of words means a young plant developing from the embryo of a seed. The term "water-miscible" in the context of "water-miscible solvent" means a liquid solvent (including mixtures of solvent compounds) that is completely soluble in water (and water soluble in the solvent) in all proportions at the temperature of the (e.g., reaction) medium comprising the water-miscible solvent. Methanol, ethanol, acetone and acetonitrile are examples of water-miscible solvents. Conversely, the term "water-immiscible" in the context of a substance that is a "water-immiscible organic compound", "water-immiscible liquid component" or "water-immiscible liquid carrier" denotes that the substance is not soluble in water (and water soluble in the substance) in all proportions at relevant temperatures (for formulated compositions around room temperature). Typically water-immiscible substances used as liquid carriers or other liquid components in formulated compositions have little water solubility and water has little solubility in the water-immiscible substances. Often water-immiscible substances used in formulation are soluble in water in an extent of less than about 1%, or less than about 0.1%, or even less than about 0.01% by weight at about 20 °C. The expression "continuous liquid phase" in the context of liquid formulated compositions refers to the liquid phase formed by the liquid carrier. The continuous liquid phase provides the bulk liquid medium in which other formulating components are dissolved, dispersed (as solid particulates) or emulsified (as liquid droplets). When the liquid carrier is aqueous (water optionally containing dissolved water-soluble compounds), a liquid emulsified in the aqueous liquid carrier is formed by a water-immiscible liquid component. The term "room temperature" as used in this disclosure refers to a temperature between about 18 °C and about 26 °C. The term "polymorph" refers to a particular crystal form (i.e. structure of crystal lattice) of a chemical compound that can exist in more than one crystal form in the solid state.

Preferred Embodiments of the present invention include:

Embodiment 1. The polymorph of 8-chloro-N-[(2-chloro-5-methoxyphenyl)sulfonyl]-6-(trifluoromethyl)-imidazo[1,2-a]pyridine-2-carboxamide (Compound 1 ({drug1}) designated Form A ({drug1a}) in the Summary of the Invention and characterized by a room-temperature powder (CuKalpha) X-ray diffraction pattern having at least the reflection positions

| 2θ | 2θ |
|---|---|
| 30.367 | 25.973 |
| 29.131 | 25.604 |
| 27.995 | 24.285 |
| 27.611 | 23.582 |
| 26.49 | 19.789 |

Embodiment 2. The polymorph of 8-chloro-N-[(2-chloro-5-methoxyphenyl)sulfonyl]-6-(trifluoromethyl)-imidazo[1,2-a]pyridine-2-carboxamide (Compound 1 ({drug1})) designated Form B ({drug1b}) in the Summary of the Invention and characterized by a -100 °C simulated (CuKalpha) X-ray diffraction pattern having at least the 20 reflection positions

| 2θ | 2θ |
|---|---|
| 28.242 | 20.999 |
| 25.978 | 18.981 |
| 25.06 | 18.12 |
| 24.583 | 17.219 |
| 23.082 | 7.998 |

Embodiment 3. The polymorph of 8-chloro-N-[(2-chloro-5-methoxyphenyl)sulfonyl]-6-(trifluoromethyl)-imidazo[1,2-a]pyridine-2-carboxamide (Compound 1 ({drug1})) designated Form D ({drug4d}) ({drug1d}) in the Summary of the Invention and characterized by a -100 °C simulated (CuKalpha) X-ray diffraction pattern having at least the 20 reflection positions

| 2θ | 2θ |
|---|---|
| 27.323 | 18.398 |
| 25.581 | 17.821 |
| 23.958 | 14.558 |
| 22.459 | 12.182 |
| 20.68 | 5.943 |

Embodiment 4. The polymorph of 8-chloro-N-[(2-chloro-5-methoxyphenyl)sulfonyl]-6-(trifluoromethyl)-imidazo[1,2-a]pyridine-2-carboxamide (Compound 1 ({drug1})) designated Form TS ({drug1e}) in the Summary of the Invention and characterized by a room-temperature powder (CuKalpha) X-ray diffraction pattern having at least the 20 reflection positions

| 2θ | 2θ |
|---|---|
| 28.913 | 22.429 |
| 26.942 | 20.325 |
| 25.672 | 19.053 |
| 24.451 | 18.603 |
| 23.316 | 12.871 |

Embodiment 5. The method described in the Summary of the Invention for preparing the polymorph Form A ({drug1a}) of Embodiment 1 comprising forming a slurry with a solvent of one or more solid forms of Compound 1 ({drug1}) selected from the group of forms B ({drug1b}), C ({drug1c}), D ({drug1d}), solvates, amorphous forms and mixtures thereof with Form A ({drug1a}) and maintaining the slurry while the solid forms of Compound 1 ({drug1}) convert to polymorph Form

A ({drug1a}).

Embodiment 6. The method of Embodiment 5 wherein the solid form of Compound 1 ({drug1}) comprises polymorph Form B ({drug1b}).

Embodiment 7. The method of Embodiment 5 wherein the solid form of Compound 1 ({drug1}) comprises polymorph Form C ({drug1c}).

Embodiment 8. The method of Embodiment 5 wherein the solid form of Compound 1 ({drug1}) comprises polymorph Form D ({drug1d}).

Embodiment 9. The method of Embodiment 5 wherein the solid form of Compound 1 ({drug1}) comprises polymorph Form TS ({drug1e}).

Embodiment 10. The method of Embodiment 5 wherein the solid forms of Compound 1 ({drug1}) comprises a mixture of polymorphs Form A ({drug1a}) and Form B ({drug1b}).

Embodiment 11. The method of any one of Embodiments 5 through 10 wherein the slurry is heated to a temperature between 30 °C and the boiling point of the solvent and agitated.

Embodiment 11a. The method of any one of Embodiments 5 through 11 wherein the slurry is heated to a temperature between 55 °C and 100 °C and agitated.

Embodiment 11b. The method of any one of Embodiments 5 through 11a wherein the slurry is heated to a temperature between 65 °C and 95 °C and agitated.

Embodiment 12. The method of any one of Embodiments 5 through 10 wherein the slurry is agitated.

Embodiment 13. The method of any one of Embodiments 5 through 12 wherein the solvent comprises water, a C5-C8 alkane, a -C4 alkanol or a C3-C4 ketone.

Embodiment 14. The method of Embodiment 13 wherein the solvent comprises water, M-heptane, methanol or acetone.

Embodiment 15. The method of Embodiment 14 wherein the solvent comprises water, methanol or acetone.

Embodiment 16. The method of Embodiment 15 wherein the solvent comprises water or methanol.

Embodiment 17. The method of Embodiment 16 wherein the solvent comprises water.

Embodiment 18. The method described in the Summary of the Invention for preparing the polymorph Form A ({drug1a}) of Compound 1 ({drug1}) comprising, (A) contacting 8-chloro-6- trifluoromethyl-imidazo[1,2-a]pyridine-2-carbonyl chloride or a salt thereof and 2-chloro-5-methoxybenzene sulfonamide in the presence of a first solvent to form a reaction mixture containing an intermediate solid form of Compound 1 ({drug1}) (B) separating the intermediate solid form of Compound 1 ({drug1}) and (C) contacting the intermediate solid form of Compound 1 ({drug1}) with a second solvent optionally heated to a temperature between 30 °C and the boiling point of the second solvent to convert the intermediate solid form to the polymorph Form A ({drug1a}) of Compound 1 ({drug1}).

Embodiment 19. The method of Embodiment 18 wherein 8-chloro-6-trifluoromethyl- imidazo[1,2-a]pyridine-2-carbonyl chloride is prepared by contacting 8-chloro- 6-(trifluoromethyl)imidazo[1,2-a]pyridine-2-carboxylic acid with a chlorinating agent.

Embodiment 20. The method of Embodiment 19 wherein the chlorinating agent is thionyl chloride, oxalyl chloride or phosgene.

Embodiment 21. The method of Embodiment 20 wherein the chlorinating agent is thionyl chloride.

Embodiment 21a. The method of any one of Embodiments 19 through 21 wherein the molar ratio of the chlorinating agent to 8-chloro-6-trifluoromethyl-imidazo[1,2-a]pyridine-2-carboxylic acid is in the range of about 1.2: 1 to about 1.5: 1. Embodiment 22. The method of any one of Embodiments 19 through 21a wherein 8- chloro-6-trifluoromethyl-imidazo[1,2-a]pyridine-2-carbonyl chloride is prepared by chlorinating 8-chloro-6-trifluoromethyl-imidazo[1,2-a]pyridine-2- carboxylic acid in a chlorination solvent.

Embodiment 23. The method of Embodiment 22 wherein the chlorination solvent is toluene, xylenes, chlorobenzene, anisole, mesitylene or tetralin.

Embodiment 24. The method of Embodiment 23 wherein the chlorination solvent is toluene, xylenes or anisole. Embodiment 25. The method of Embodiment 24 wherein the chlorination solvent is toluene.

Embodiment 26. The method of any one of Embodiments 19 through 25 wherein 8- chloro-6-(trifluoromethyl)imidazo[1,2-a]pyridine-2-carboxylic acid is contacted with a chlorinating agent in the presence of N,N-dimethylformamide or N-formylpiperidine.

Embodiment 27. The method of Embodiment 26 wherein 8-chloro-6- (trifluoromethyl)imidazo[1,2-a]pyridine-2-carboxylic acid is contacted with a chlorinating agent in the presence of N-formylpiperidine.

Embodiment 27a. The method of Embodiment 26 wherein 8-chloro-6- (trifluoromethyl)imidazo[1,2-a]pyridine-2-carboxylic acid is contacted with a chlorinating agent in the presence of N,N-dimethylformamide.

Embodiment 28. The method of any one of Embodiments 19 through 27a wherein 8- chloro-6-(trifluoromethyl)imidazo[1,2-a]pyridine-2-carboxylic acid is contacted with a chlorinating agent in the temperature range of 0 to 85 °C.

Embodiment 29. The method of Embodiment 28 wherein 8-chloro-6- (trifluoromethyl)imidazo[1,2-a]pyridine-2-carboxylic acid is contacted with a thionyl chloride in the temperature range of 75 to 85 °C.

Embodiment 30. The method of any one of Embodiments 19 through 29 wherein excess chlorinating agent is removed

from the 8-chloro-6-trifluoromethyl-imidazo[1,2-a]pyridine-2-carbonyl chloride before it is contacted with 2-chloro-5-methoxybenzene sulfonamide.

Embodiment 31. The method of any one of Embodiments 18 through 30 wherein the 8- chloro-6-trifluoromethyl-imidazo[1,2-a]pyridine-2-carbonyl chloride in step (A) is in the form of an HCl salt.

Embodiment 32. The method of any one of Embodiments 22 through 31 wherein the 8- chloro-6-trifluoromethyl-imidazo[1,2-a]pyridine-2-carbonyl chloride in step (A) is in the form of a slurry in the chlorination solvent.

Embodiment 33. The method of any one of Embodiments 18 through 32 wherein the molar ratio of 8-chloro-6-trifluoromethyl-imidazo[1,2-a]pyridine-2-carboxylic acid and 2-chloro-5-methoxybenzene sulfonamide in step (A) is in the range of 1 : 1.1 to 1 : 1.

Embodiment 34. The method of any one of Embodiments 18 through 33 wherein in step (A) the 8-chloro-6-trifluoromethyl-imidazo[1,2-a]pyridine-2-carbonyl chloride and the 2-chloro-5-methoxybenzene sulfonamide are contacted in the presence of a base.

Embodiment 35. The method of Embodiment 34 wherein the base is a tertiary amine.

Embodiment 36. The method of Embodiment 35 wherein the base is tributylamine, triethylamine or diisopropylethylamine.

Embodiment 37. The method of Embodiment 36 wherein the base is tributylamine. Embodiment 38. The method of any one of Embodiments 34 through 37 wherein the molar ratio of base to 2-chloro-5-methoxybenzene sulfonamide in step (A) is in the range of 2.8: 1 to 3.5: 1.

Embodiment 39. The method of any one of Embodiments 22 through 38 wherein the first solvent comprises a mixture of the chlorination solvent with at least one solvent selected from ethyl acetate, tetrahydrofuran, dichloromethane and dichloroethane with the chlorination solvent.

Embodiment 40. The method of Embodiment 39 wherein the first solvent comprises a mixture of the chlorination solvent with ethyl acetate.

Embodiment 40a. The method of Embodiment 40 wherein the first solvent comprises a mixture of toluene with ethyl acetate. Embodiment 41. The method of any one of Embodiments 18 through 40a wherein in step (A) the 8-chloro-6-trifluoromethyl-imidazo[1,2-a]pyridine-2-carbonyl chloride and the 2-chloro-5-methoxybenzene sulfonamide are contacted in the temperature range of 0 to 25 °C.

Embodiment 42. The method of Embodiment 41 wherein in step (A) the 8-chloro-6- trifluoromethyl-imidazo[1,2-a]pyridine-2-carbonyl chloride and the 2-chloro-5- methoxybenzene sulfonamide are contacted in the temperature range of 15 to 25 °C. Embodiment 43. The method of any one of Embodiments 39 through 42 wherein when the reaction in step (A) is complete, at most 1 equivalent of aqueous acid for every equivalent of the base is added to neutralize the reaction mixture.

Embodiment 44. The method of Embodiment 43 wherein the aqueous acid is hydrochloric acid.

Embodiment 45. The method of Embodiments 43 or 44 wherein after addition of aqueous acid, the reaction mixture is heated in the range of 50 to 60 °C for in the range of one to two hours to form the intermediate solid form of Compound 1 ({drug1}). Embodiment 46. The method of any one of Embodiments 43 through 45 wherein after the reaction mixture is heated in the presence of aqueous acid, the reaction mixture is cooled to a temperature in the range of 5 to 15 °C.

Embodiment 47. The method of any one of Embodiments 18 through 46 wherein in step (B) the reaction mixture is filtered to separate the intermediate solid form of Compound 1 ({drug1}).

Embodiment 48. The method of Embodiment 47 wherein the intermediate solid form of Compound 1 ({drug1}) is a solvate.

Embodiment 48a. The method of Embodiment 48 wherein the intermediate solid form of Compound 1 ({drug1}) is a toluene solvate.

Embodiment 48b. The method of Embodiment 47 wherein the intermediate solid form of Compound 1 ({drug1}) is an unsolvated polymorph or mixture of polymorphs.

Embodiment 49. The method of any one of Embodiments 18 through 48b wherein the intermediate solid form of Compound 1 ({drug1}) separated in step (B) is contacted with a second solvent in step (C) to convert the intermediate solid form of Compound 1 ({drug1}) to polymorph Form A ({drug1a}).

Embodiment 50. The method of any one of Embodiments 18 through 49 wherein the temperature in step (C) is between 30 °C and the boiling point of the second solvent.

Embodiment 51. The method of Embodiment 50 wherein the temperature in step (C) is at least 30 °C.

Embodiment 51a. The method of Embodiment 50 wherein the temperature in step (C) is at least 55 °C.

Embodiment 52. The method of Embodiment 50 wherein the temperature in step (C) is at most the boiling point of the second solvent.

Embodiment 53. The method of any one of Embodiments 18 through 52 wherein the second solvent comprises water, methanol, acetone or w-heptane.

Embodiment 54. The method of Embodiment 53 wherein the second solvent comprises water or methanol.

Embodiment 55. The method of Embodiment 54 wherein the second solvent comprises water.

Embodiment 56. The method of any one of Embodiments 18 through 55 wherein the second solvent is water and the temperature of step (C) is in the range of 90 to 100 °C.

Embodiment 57. The method of any one of Embodiments 18 through 54 wherein the second solvent is methanol and the temperature of step (C) is in the range of 55 to 65 °C.

Embodiment 58. The method of any one of Embodiments 18 through 57 wherein when the conversion in step (C) is complete, the second solvent is cooled and polymorph Form A ({drug1a}) is separated from the second solvent by filtration. Embodiments of this invention, including Embodiments 1-58 above as well as any other embodiments described herein, can be combined in any manner.

Compound 1 ({drug1}) is 8-chloro-N-[(2-chloro-5-methoxyphenyl)sulfonyl]-6-(trifluoromethyl)-imidazo[1,2-a]pyridi-2-carboxamide and has the following molecular structure:

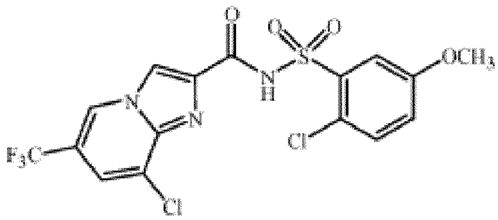

1

The solid state of Compound 1 ({drug1}) has been discovered to be preparable in more than one solid form. These solid forms include an amorphous solid form, in which there is no long-range order in the positions of molecules (e.g., foams and glasses). These solid forms also include crystalline forms, in which constituent molecules are arranged in an orderly repeating pattern extending in all three spatial dimensions. The term "polymorph" refers to a particular crystalline form of a chemical compound that can exist in more than one crystal structure (e.g. lattice type) in the solid state. The term "packing polymorphs" refers to particular crystalline forms of a compound having different crystal packing. Crystalline forms of Compound 1 ({drug1}) in this invention relate to embodiments which include a single polymorph (i.e. single crystalline form) and to embodiments which include a mixture of polymorphs (i.e. different crystalline forms). Polymorphs can differ in such chemical, physical and biological properties as crystal shape, density, hardness, color, chemical stability, melting point, hygroscopicity, suspensibility, solubility, dissolution rate and biological availability. One skilled in the art will appreciate that a polymorph of Compound 1 ({drug1}) can exhibit beneficial effects (e.g., suitability for preparation of useful formulations, stability, improved biological performance) relative to another polymorph or a mixture of polymorphs of Compound 1 ({drug1}). Differences with respect to chemical stability, filterability, solubility, hygroscopicity, melting point, solid density and flowability can have a significant effect on the development of production methods and formulations, and efficacy of nematode control. Preparation and isolation of particular polymorphs of Compound 1 ({drug1}) have now been achieved.

One crystalline polymorph form of Compound 1 ({drug1}) designated as polymorph Form TS ({drug1e}), is a 1 : 1 (molar ratio) toluene solvate. Polymorph Form TS ({drug1e}) can be characterized by X-ray powder diffraction, single crystal X-ray structure analysis and Differential Scanning Calorimetry. The powder X-ray diffraction pattern of polymorph Form TS ({drug1e}) of Compound 1 ({drug1}) is shown in Figure 1 of WO/2013/055584. The corresponding 20 values are tabulated in Table 8 of Characterization Example 5. Polymorph Form TS ({drug1e}) of Compound 1 ({drug1}) can be identified by a room-temperature powder Cu(Kalpha) X-ray diffraction pattern having at least the 20 reflection positions (in degrees)

| 2θ | 2θ |
|---|---|
| 28.913 | 22.429 |
| 26.942 | 20.325 |
| 25.672 | 19.053 |
| 24.451 | 18.603 |
| 23.316 | 12.871 |

Single crystal X-ray diffraction can also be used to characterize polymorph Form TS ({drug1e}). A description of single crystal X-ray diffraction of polymorph Form TS ({drug1e}) is provided in Characterization Example 10. Crystals of polymorph Form TS ({drug1e}) have a triclinic unit cell and typically exhibit a needle-like morphology. Polymorph Form TS ({drug1e}) of Compound 1 ({drug1}) can also be characterized by Differential Scanning Calorimetry. DSC indicates the melting point of polymorph Form TS ({drug1e}) is about 217 °C. The details of a DSC experiment are provided in

Characterization Example 11. Polymorph Form TS ({drug1e}) can be prepared directly during the preparation of Compound 1 ({drug1}) from its starting materials in the presence of toluene solvent as described in Preparation Example 1. Polymorph Form TS ({drug1e}) can also be prepared by slow evaporation of a saturated solution of Compound 1 ({drug1}) in toluene. Polymorph Form TS ({drug1e}) can be converted into other polymorph forms or mixtures of forms as described in Preparation Examples 2 through 4.

A second crystalline polymorph form of Compound 1 ({drug1}) is designated as polymorph Form A ({drug1a}). This solid form is unsolvated. Polymorph Form A ({drug1a}) can be characterized by X-ray powder diffraction, single crystal X-ray structure analysis and Differential Scanning Calorimetry (DSC). The powder X-ray diffraction pattern of polymorph Form A ({drug1a}) of Compound 1 ({drug1}) is shown in Figure 1 of WO/2013/055584. The corresponding 20 values are tabulated in Table 4 of Characterization Example 1. Polymorph Form A ({drug1a}) of Compound 1 ({drug1}) can be identified by a room-temperature powder (CuKalpha) X-ray diffraction pattern having at least the 20 reflection positions (in degrees)

| 2θ | 2θ |
|---|---|
| 30.367 | 25.973 |
| 29.131 | 25.604 |
| 17.995 | 24.285 |
| 27.611 | 23.582 |
| 26.49 | 19.789 |

Single crystal X-ray diffraction can also be used to characterize polymorph Form A ({drug1a}). A description of single crystal X-ray diffraction of polymorph Form A ({drug1a}) is provided in Characterization Example 6. Crystals of polymorph Form A ({drug1a}) have a triclinic unit cell and typically exhibit a irregular block morphology. Polymorph Form A ({drug1a}) of Compound 1 ({drug1}) can also be characterized by Differential Scanning Calorimetry. DSC indicates the melting point of polymorph Form A ({drug1a}) is about 219 °C. The details of a DSC experiment are provided in Characterization Example 1 1. Polymorph Form A ({drug1a}) is physically and chemically stable in its pure solid form (shown in Characterization Example 13). Pure Polymorph Form A ({drug1a}) can be prepared by desolvating the toluene solvate (Form TS ({drug1e})) via heating in a solvent like water or methanol as described in Preparation Examples 3 and 4. Polymorph Form A ({drug1a}) of Compound 1 ({drug1}) can also be prepared by heating a mixture of polymorph Forms A and B at or near the boiling point of a solvent and then cooling back to room temperature or lower as described in Preparation Example 5. Methanol, water, acetone or M-heptane are particularly useful solvents for this method.

Another crystalline polymorph form of Compound 1 ({drug1}) is designated as Polymorph Form B ({drug1b}). This solid form is unsolvated. Polymorph Form B ({drug1b}) can be characterized by X-ray powder diffraction, single crystal X-ray structure analysis and Differential Scanning Calorimetry. Single crystal X-ray diffraction can be used to characterize polymorph Form B ({drug1b}). A description of single crystal X-ray diffraction of polymorph Form B ({drug1b}) is provided in Characterization Example 7. Crystals of polymorph Form B ({drug1b}) have a triclinic unit cell and typically exhibit a prism morphology. A simulated powder pattern was calculated from the atomic coordinates and cell parameters determined from the single crystal structure for polymorph Form B ({drug1b}) of Compound 1 ({drug1}) and is shown in Figure 1 of WO/2013/055584. The corresponding 20 values of the powder X-ray diffraction pattern of polymorph Form B ({drug1b}) are tabulated in Table 5 of Characterization Example 2. Polymorph Form B ({drug1b}) of Compound 1 ({drug1}) can be identified by a -100 °C simulated powder (CuKalpha) X-ray diffraction pattern having at least the 20 reflection positions (in degrees)

| 2θ | 2θ |
|---|---|
| 28.242 | 20.999 |
| 25.978 | 18.981 |
| 25.06 | 18.12 |
| 34.583 | 77.219 |
| 23.082 | 7.998 |

Polymorph Form B ({drug1b}) of Compound 1 ({drug1}) can also be characterized by Differential Scanning Calorimetry. DSC indicates the melting point of polymorph Form B ({drug1b}) is about 218 °C. The details of a DSC

experiment are provided in Characterization Example 11. Polymorph Form B ({drug1b}) can be obtained as a mixture with polymorph Form A ({drug1a}) by desolvation of the toluene solvate (Form TS ({drug1e})) as described in Preparation Example 2. Polymorph Form B ({drug1b}) can be prepared by heating the mixture of polymorph Forms A and B in dichloromethane as described in Preparation Example 5. Polymorph Form B ({drug1b}) of Compound 1 ({drug1}) can also be prepared by thermal gradient sublimation at 160 °C.

Another crystalline polymorph form of Compound 1 ({drug1}) is designated as polymorph Form C ({drug1c}). This solid form is unsolvated. Polymorph Form C ({drug1c}) can be characterized by X-ray powder diffraction and single crystal X-ray structure analysis. Single crystal X-ray diffraction can be used to characterize polymorph Form C ({drug1c}). A description of single crystal X-ray diffraction of polymorph Form C ({drug1c}) at -100 °C is provided in Characterization Example 8 and at 23 °C in Characterization Example 14. Crystals of polymorph Form C ({drug1c}) have a triclinic unit cell and typically exhibit a triangular plate morphology. A simulated powder pattern was calculated from the atomic coordinates and cell parameters determined from the single crystal structure for polymorph Form C ({drug1c}) at -100 °C of Compound 1 ({drug1}) and is shown in Figure 1 of WO/2013/055584. The corresponding 2θ values of the -100 °C simulated powder (CuKalpha) X-ray diffraction pattern of polymorph Form C ({drug1c}) are tabulated in Table 6 of Characterization Example 3. The corresponding 2θ values of the room temperature simulated powder (CuKalpha) X-ray diffraction pattern of polymorph Form C ({drug1c}) are tabulated in Table 22 of Characterization Example 15. Polymorph Form C ({drug1c}) of Compound 1 ({drug1}) can be prepared by thermal gradient sublimation at 160 °C.

Another crystalline polymorph form of Compound 1 ({drug1}) is designated as polymorph Form D ({drug1d}). This solid form is unsolvated. Polymorph Form D ({drug1d}) can be characterized by X-ray powder diffraction, single crystal X-ray structure analysis and Differential Scanning Calorimetry. Single crystal X-ray diffraction can be used to characterize polymorph Form D ({drug1d}). A description of single crystal X-ray diffraction of polymorph Form D ({drug1d}) is provided in Characterization Example 9. Crystals of polymorph Form D ({drug1d}) have a triclinic unit cell and typically exhibit an irregular block morphology. A simulated powder pattern was calculated from the atomic coordinates and cell parameters determined from the single crystal structure for polymorph Form D ({drug1d}) of Compound 1 ({drug1}) and is shown in Figure 1 of WO/2013/055584. The corresponding 2θ values of the powder X-ray diffraction pattern of polymorph Form D ({drug1d}) are tabulated in Table 7 of Characterization Example 4. Polymorph Form D ({drug1d}) of Compound 1 ({drug1}) can be identified by a -100 °C simulated powder (CuKalpha) X-ray diffraction pattern having at least the 2θ reflection positions (in degrees)

| 2θ | 2θ |
|---|---|
| 27.323 | 18.398 |
| 25.581 | 17.821 |
| 23.958 | 14.558 |
| 22.459 | 12.182 |
| 20.68 | 5.943 |

Polymorph Form D ({drug1d}) of Compound 1 ({drug1}) can also be characterized by Differential Scanning Calorimetry. DSC indicates the melting point of polymorph Form D ({drug1d}) is about 218 °C. The details of a DSC experiment are provided in Characterization Example 11. Pure polymorph Form D ({drug1d}) can be prepared by heating the mixture of polymorph Forms A and B in acetonitrile or acetic acid as described in Preparation Examples 5 and 6.

Compound 1 ({drug1}) can also exist as an amorphous solid. The powder X-ray diffraction pattern (pXRD) for the amorphous form of Compound 1 ({drug1}) shows a broad reflection pattern across the two-theta angle lacking distinct reflection signals and thus is readily distinguished from the pXRD patterns of crystalline forms of Compound 1 ({drug1}). The amorphous solid form can be prepared by standard methods known in the art, such as evaporation to dryness of solutions containing Compound 1 ({drug1}) by quick cooling of melted Compound 1 ({drug1}) by spray drying a solution of Compound 1 ({drug1}) or by freeze-drying a frozen solution containing Compound 1 ({drug1}).

Compound 1 ({drug1}) can be prepared by a variety of methods. One method involves coupling the starting acid 8-chloro-6-(trifluoromethyl)imidazo[1,2-a]pyridine-2-carboxylic acid and 2-chloro-5-methoxybenzenesulfonamide with any number of amide bond forming coupling reagents. An especially useful method utilizes 1-(3-dimethyl-aminopropyl)-3-ethyl-carbodiimide hydrochloride and is described in Synthesis Example 1 in World Patent Publication WO 2010/129500. Another method utilizes the mixed anhydride of the starting carboxylic acid as a method of promoting easy amid bond formation with the sulfonamide. Some of the most useful reagents used to make the mixed anhydride of the starting carboxylic acid are ethyl chloroformate and isobutyl chloroformate. Another method to prepare Compound 1 ({drug1}) involves the formation of an ester of the starting acid and reacting it with the sodium salt of the sulfonamide. Useful esters of the starting acid are the methyl- or ethyl-ester. The sodium salt of the sulfonamide can be prepared by

reaction with sodium hydride. Compound 1 ({drug1}) can also be prepared from the acid chloride of the starting carboxylic acid and coupling with the sulfonamide as described in Preparation Example 1.

The preparation of polymorph Form A ({drug1a}) of Compound 1 ({drug1}) can be accomplished by a process wherein Compound 1 ({drug1}) is prepared from its starting materials (Preparation Example 1) to initially yield an intermediate solid form of Compound 1 ({drug1}). The intermediate solid form initially isolated can be a mixture of polymorph forms, a polymorph form other than Form A ({drug1a}) or a solvate of Compound 1 ({drug1}). The intermediate solid form of Compound 1 ({drug1}) can be converted into pure polymorph Form A ({drug1a}) by a variety of methods (Preparation Examples 2 through 5 and Characterization Example 19). An especially useful method to prepare the polymorph Form A ({drug1a}) of Compound 1 ({drug1}) is a process wherein the intermediate solid form of Compound 1 ({drug1}) is a toluene solvate (Polymorph Form TS ({drug1e})). Polymorph Form TS ({drug1e}) is prepared directly from precursor starting materials as shown in Scheme 1. The method involves treating a compound of Formula 2 (8-chloro-6-(trifluoromethyl)imidazo[1,2-a]pyridine-2-carboxylic acid) with a chlorinating agent in the presence of a chlorinating solvent (toluene) to make the acid chloride of Compound 3. The acid chloride Compound 3 is then treated with a compound of Formula 4 (2-chloro-5-methoxybenzenesulfonamide) in the presence of base to form a salt of Compound 1 ({drug1}). When the reaction is complete the mixture is treated with aqueous acid to neutralize any excess base and ensure formation of the neutral acyl sulfonamide product. The aqueous slurry is warmed and stirred to dissolve salts and encourage the product to crystallize out of solution. The product crystallizes as the toluene solvate of Compound 1 ({drug1}) (Form TS ({drug1e})) and is separated by solid-liquid separation (e.g. filtration) and either dried to form the pure solvate or processed further to form polymorph Form A ({drug1a}).

Scheme 1

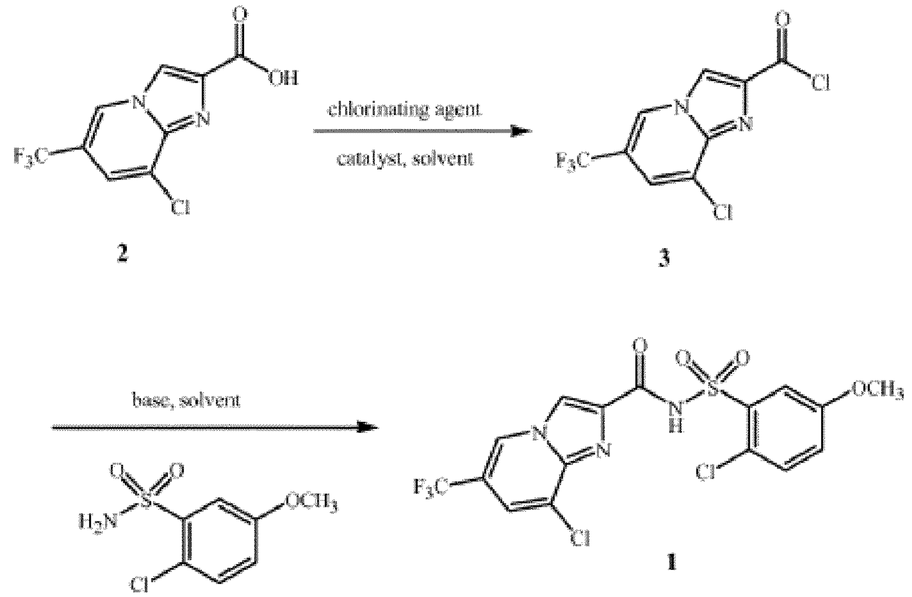


The reaction corresponding to the first part of Scheme 1 is typically run using 1 to 2 molar equivalents of the chlorinating agent relative to Compound 2. More typically the molar ratio of the chlorinating agent to the compound of Formula 2 is in the range of about 1.2: 1 to about 1.5: 1. A larger ratio of chlorinating agent to Compound 2 is needed if Compound 2 contains some residual water. Chlorinating agents that are useful for this transformation include thionyl chloride, oxalyl chloride or phosgene. Thionyl chloride is especially useful. The formation of the acid chloride is usually catalyzed by the addition of a formamide in the range of 1 to 10 weight percent relative to Compound 2. Useful catalysts for acid chloride formation include N,N-dimethylformamide and N-formylpiperidine. Solvents useful for the chlorination in Scheme 1 (chlorination solvent) are any solvents that are inert to the chlorination reagent. Solvents that are especially useful are toluene, xylenes, chlorobenzene, anisole, mesitylene and tetralin. Toluene is an especially useful solvent. The formation of the acid chloride (Compound 3) is usually done in a temperature range appropriate for the chlorination reagent usually in the range of 0 to 85 °C or near the boiling point of the chlorinating reagent. The lower temperatures are appropriate for oxalyl chloride or phosgene. A temperature in the range of 75 to 85 °C is useful for thionyl chloride. The progress of the reaction may be monitored by the formation of the methyl ester of Compound 2. An aliquot of the reaction mixture is treated with methanol and is analyzed by HPLC to determine the ratio of unreacted Compound 2 and the ester from reaction of Compound 3 with methanol. Reaction times are typically in the range of 2 to 3 hours. Finally, to separate the acid chloride from the chlorinating agent, the reaction mixture is heated to the boiling point of the reaction

mixture to remove excess chlorinating agent (thionyl chloride) and reduce the amount of solvent. The reaction mass is concentrated to about one-half volume and the resultant slurry (Compound 3 in chlorination solvent) is cooled to room temperature. When thionyl chloride is the chlorinating agent and toluene is the chlorination solvent then the resultant slurry is the hydrochloride salt of Compound 3 in toluene. The second part of Scheme 1 involves the reaction of the Compound of Formula 3 and the sulfonamide of Formula 4 to form the acyl sulfonamide Compound 1 ({drug1}). The molar ratio of reactants is usually in the range of 1 to 1.1 equivalents of Compound 4 to 1 equivalent of Compound 2 with a ratio of 1.05 equivalents of Compound 4 to 1 equivalent of Compound 2 being especially useful. The coupling reaction is run in the presence of a base to neutralize the equivalent of hydrogen chloride released. The quantity of base used is usually in the range of 2.5 to 4 equivalents relative to the sulfonamide, with a range of 2.8 to 3.5 being especially useful. The base is used to neutralize the equivalent of HCl from the acid chloride salt starting material (the nitrogen containing heterocycle in Compound 3 forms a hydrochloride salt in strong acidic conditions) and the equivalent of HCl generated in the reaction of the acid chloride and sulfonamide. The base also removes a proton from the acidic acylsulfonamide functional group in the product to form a salt of the product. A variety of tertiary amines can be used as bases for this coupling reaction. Examples are tributylamine, triethylamine, and diisopropylethylamine. Solvents useful for the second part of Scheme 1 are polar aprotic solvents that provide some solubility for the sulfonamide and Compound 1 ({drug1}). Solvents that are useful include ethyl acetate, tetrahydrofuran, dichloromethane and dichloroethane. Ethyl acetate is especially useful. The slurry of acid chloride from part A is usually diluted with ethyl acetate in a ratio of about 1 volume of toluene slurry to 1 to 2 volumes of ethyl acetate. The "first solvent" of the process to prepare polymorph Form A ({drug1a}) of Compound 1 ({drug1}) (step (A)) is a mixture of the chlorination solvent and the solvent added for solubility in the coupling reaction (e.g. ethyl acetate). The reaction mixture (Compound 3 in the solvent mixture) is cooled to a temperature in the range of 0 to 15°C and treated with the Compound 4. The tertiary amine base is then added dropwise and the reaction mixture allowed to warm to room temperature. The reaction is stirred for a time in the range of 2 to 18 hours. The reaction is again monitored by treating an aliquot of the reaction mixture with methanol and observing the relative ratios of methyl ester of Compound 2, Compound 4 and Compound 1 ({drug1}). Upon completion of the reaction, the reaction mixture is usually diluted with water to dissolve salts and reduce the solubility of the product, thus promoting the crystallization of product of high purity. Aqueous acid is then added to the reaction mixture to form a salt of any excess tertiary amine that was not already in the hydrochloride salt form. This acidification is necessary to release the product Compound 1 ({drug1}) in its neutral form from the tertiary amine salt that forms with the acidic acylsulfonamide functional group in the product. Typically at least about 1 molar equivalent of acid is added for every equivalent of tertiary amine base in excess of the number of equivalents of acid chloride used in the reaction. More than 1 equivalent of acid for every equivalent of tertiary amine base used in the reaction can be added to ensure an acidic environment, although to minimize cost and waste disposal, typically not more than about 0.5 equivalent of excess acid is added. Other water-soluble acids can be used in place of hydrochloric acid. An example of another suitable water-soluble acid is sulfuric acid. For multi protic acids the molar equivalents of acids may have to be adjusted according to the number of available protons. When the addition of the acid is complete, the reaction mixture is usually heated in the range of 50 to 60 °C and stirred in the range of 1 to 2 hours. This procedure promotes formation of larger size crystals to facilitate filtration. The reaction slurry is then cooled to a temperature in the range of 5 to 15 °C and filtered. The wet solid is washed several times with water, to remove traces of salts and excess acid. The wet solid is then also washed several times with toluene to displace any remaining water and ethyl acetate from the solid product. This crude wet solid is a 1 : 1 (molar ratio) solvate of Compound 1 ({drug1}) and toluene (polymorph Form TS ({drug1e})). The toluene solvate (Form TS ({drug1e})) of the product is formed from toluene solvent used in the first part of the process that was carried into the second part of the process to prepare Compound 1 ({drug1}). If the chlorination is performed with a solvent other than toluene the resultant intermediate solid form of Compound 1 ({drug1}) will not be isolated as a toluene solvate. The crude product Compound 1 ({drug1}) can be isolated as a solvate of any solvent that is part of the "first solvent" mixture used in the coupling process, if it forms a strong solvate. Alternatively, when the solvents used in the preparation of Compound 1 ({drug1}) do not have a tendency to form solvates (e.g. o-xylene) then the intermediate solid form of Compound 1 ({drug1}) product can be isolated as an unsolvated polymorph or mixture of polymorphs. Compound 1 ({drug1}) in the form of a solvate, unsolvated polymorph or mixture of polymorphs is initially "separated" from the reaction mixture by filtration to yield a wet solid or wet cake. The separated solid form of Compound 1 ({drug1}) can then be further "isolated" by drying or removing the last traces of solvent adhering to the external surface of the solid. The separated wet solid or isolated dry solid can then be further converted to other polymorph forms. The isolated solid can also be characterized by a variety of analytical methods.

The crude wet solid polymorph Form TS ({drug1e}) can be used as is for further conversion as described in Preparation Example 3. Polymorph Form TS ({drug1e}) can be desolvated and converted to polymorph Form A ({drug1a}) by forming a slurry in water and distilling at about 95-96 °C in an apparatus that allows for the removal of toluene into the distillate by azeotropic distillation, e.g. using a Dean-Stark trap. The mixture is heated for 3 to 5 hours and water collected in the Dean-Stark trap is returned to the reaction to maintain constant reaction volume while toluene is removed from the slurry. The reaction is cooled to ambient temperature, filtered and dried under vacuum (8-15 kPa absolute

pressure) at 55 °C for one hour. The resultant product is pure polymorph Form A ({drug1a}) as determined by pXRD. Variations of this procedure resulting in the same conversion of polymorph Form TS ({drug1e}) to Form A ({drug1a}) are described in Preparation Example 4. Both water and methanol and mixtures of water and methanol can act as the solvent for the desolvation procedure by distillation, e.g. with the Dean-Stark apparatus. The desolvation/polymorph conversion reaction can be accomplished at a temperature between about 30 °C and the boiling point of the solvent. The desolvation/polymorph conversion reaction is especially efficient at a temperature between about 55 °C and the boiling point of the solvent (the boiling point of the solvent varies depending on the solvent or solvent mixture used) as shown in Table 2 of Preparation Example 4. The consistent result is pure polymorph Form A ({drug1a}) indicating that it is the most stable polymorph form in the range of studied reaction conditions. The crude wet solid of polymorph Form TS ({drug1e}) can also be desolvated by drying in a vacuum oven at about 90 °C (8-15 kPa absolute pressure) for about 4 days to give a mixture of polymorph Forms A and B as described in Preparation Example 2. The mixture of polymorph Forms A and B that results from the desolvation of polymorph Form TS ({drug1e}) can then be further converted into other polymorph forms as described in Preparation Example 5. A sample of polymorph Forms A and B, originally derived from desolvation of Form TS ({drug1e}), is suspended in a solvent and heated and stirred for a time period and then cooled and isolated by filtering and drying in a vacuum oven. A variety of solvents can be used in this conversion procedure and the particular polymorph form that results depends on the solvent used. The results are summarized in Table 3 of Preparation Example 5. A variety of solvents give pure polymorph Form A ({drug1a}). Heating under agitation at 95-100 °C for 3 hours in water or n-heptane results in polymorph Form A ({drug1a}). Heating under agitation at 60 °C for 3 hours in methanol also results in polymorph Form A ({drug1a}). The starting polymorph mixture dissolved in some of the solvents upon warming and therefore those solvent's solutions were cooled to or below ambient temperature to encourage crystallization. The crystal form conversion in these solvents resulted in a variety of polymorph forms. Acetone (also water, methanol and n-heptane) resulted in polymorph Form A ({drug1a}), dichloromethane resulted in polymorph Form B ({drug1b}) and both acetonitrile and acetic acid resulted in polymorph Form D ({drug1d}). The relative stability of pure polymorphs and mixtures of polymorphs of Compound 1 ({drug1}) were studied in water heated to 95 °C or methanol heated to 55 °C in Characterization Example 12. In all cases the starting polymorph or mixtures of polymorphs converted to Form A ({drug1a}). These experiments indicate that Form A ({drug1a}) is the most thermodynamically stable polymorph form under the conditions studied. The data in Characterization Example 12 shows that polymorph Form B ({drug1b}) and polymorph Form D ({drug1d}) can act as intermediates to prepare polymorph Form A ({drug1a}). Polymorph Form TS ({drug1e}) is also demonstrated to be an intermediate to prepare polymorph Form A ({drug1a}) in Preparation Examples 3 and 4. Seed crystals were not used in the above described polymorph conversions, however, seed crystals can be used to promote conversion and/or increase the rate of conversion of one polymorph into another. The polymorph conversion reactions are often agitated by a variety of methods even if not explicitly stated. The form of agitation can be from shaking the reaction vessel or by stirring with a magnetic or mechanical stirrer. The polymorph conversion reactions can also be agitated by the boiling action of the solvent. Without further elaboration, it is believed that one skilled in the art using the preceding description can utilize the present invention. The following Examples are, therefore, to be construed as merely illustrative, and not limiting of the disclosure in any way whatsoever. Abbreviations used in the examples are as follows: rpm is revolutions per minute, pXRD is powder X-ray diffraction, wt% is percent by weight measured by HPLC (using a calibration standard), a% is percent by area measured by HPLC at a wavelength of 230 nm, DSC is differential scanning calorimetry, TGA is thermal gravimetric analysis and KFT is Karl-Fischer titration.

Analytical methods used in the preparation examples are described below or in the Characterization Examples: Powder X-Ray Diffraction (p-XRD): Powder X-ray diffraction was used to identify the crystalline phases of various samples of Compound 1 ({drug1}) Data were obtained with a Philips X'PERT automated powder diffractometer, Model 3040. The radiation produced by a copper anode X-ray source includes Cu-K(alphal), Cu-K(alpha2) and Cu-K(beta). The diffractometer was equipped with a nickel filter that removes the Cu-K(beta) radiation leaving Cu-K(alphal) and Cu-K(alpha2) in the raw data. The peaks originating from Cu-K(alpha2) are removed during the find peaks routine in the Jade Software (MDI/Jade software version 9.1) leaving the listed maxima from Cu-K(alphal). The wavelength for Cu-K(alphal) or (Cu-Kalpha) radiation listed in International Tables for X-ray Crystallography is 0.154056 nm. The listed 20 X-ray maxima are for Cu-K(alphal) radiation which is the strongest radiation produced by a copper anode X-ray source and is sometimes simply abbreviated as Cu-K(alpha) or Cu-Ka. Thermo-gravimetric Analysis (TGA): Thermo-gravimetric Analysis was performed on a Thermal Analysis Q5000 equipment to determine the relative weight loss of a sample as a function of temperature. Test samples (2-6 mg) were accurately weighed into sample pans (platinum, 100 μl). The samples were heated from starting temperature (25 °C) to final temperature (250 or 300 °C) at a heating rate of 10 °C/min under a nitrogen flow of 25 mL/min. The TGA scans were analyzed and plotted using Thermal Analysis Advantage thermal analysis software. High Performance Liquid Chromatography (HPLC) HPLC was used to determine the purity of Compound 1 ({drug1}) and intermediates. An Agilent 1 100/1200 series HPLC system with DAD/UV detector and reverse-phase column (Agilent Zorbax® SB C18 (4.6 x 150) mm, 3.5μm, Part No. 863953-902) was used. Flow rate was 1 mL/min, run time 25 min, injection volume 3.0 μl, and the column oven temperature was 40 °C. A mobile phase gradient according to Table 1 was used wherein mobile phase A was 0.075% by volume orthophosphoric acid and mobile Phase

B was acetonitrile (HPLC grade). Mobile phase A was prepared by thoroughly mixing 0.75 mL of orthophosphoric acid (AR grade) with 1000 mL of deionized water (Milli-Q grade) and filtering through a membrane filter (0.45 μm pore size). Standards were prepared by weighing 30.0 mg of the standard into a 100 mL standard volumetric flask, dissolving and diluting with the diluent. Samples were prepared by weighing 30.0 mg of the sample into a 100 mL standard volumetric flask, dissolving and diluting with the diluent. For analysis, the HPLC system and column were equilibrated with initial mobile phase. In sequence, a blank sample, a standard sample and the test sample were run. The retention time for Compound 1 ({drug1}) was about 14.8 min. Peaks appearing in the blank sample were not integrated, all other peaks were integrated and a% purity reported from the sample chromatogram. For wt% determination the concentration of test sample was calibrated against the standard sample.

Table 1 - Mobile Phase Gradient Table:

| Time (min) | Volume Fraction of Mobile Phase A (%) | Volume Fraction of Mobile Phase B (%) |
|---|---|---|
| 0 | 80 | 20 |
| 15 | 30 | 70 |
| 19 | 10 | 90 |
| 25 | 10 | 90 |

Proton-Nuclear Magnetic Resonance (1H-NMR): Proton-NMR analysis was performed on a Bruker Advance 300/400 instrument. The operational frequency was 400 MHz, spectral frequency range 0-16 ppm, delay time 2 seconds, pulse width of 12 μs, minimum number of scans was 8. Samples were prepared by weighing about 0.01 g of samples or reference standards, adding 0.6 mL of DMSO-d6 to dissolve the contents and transferring into NMR tubes. Deuterated DMSO (DMSO-d6) was from Cambridge Isotope Laboratory. Water Content: Water content analysis was performed by Karl-Fischer titration (KFT).

PREPARATION EXAMPLE 1: Synthesis of Toluene Solvate Form of Compound 1 ({drug1}) (Form TS ({drug1e})) Step A: Preparation of 8-chloro-6-(trifluoromethyl)imidazo[1,2-a]pyridine-2-carbonyl chloride: To a 3000 mL three-neck round bottom flask equipped with an overhead stirrer, thermo pocket, addition funnel and nitrogen tube was charged toluene (1000 mL), N-formyl piperidine (3.54 g, 0.031 mol) and thionyl chloride (67 g, 0.559 moles) at 23 °C under nitrogen atmosphere. The resultant reaction mass was heated to 82 °C and to this 8-chloro-6-(trifluoromethyl)imidazo[1,2-a]pyridine-2-carboxylic acid (100 g, 0.373 moles) (prepared as in WO 2010/129500) was charged lot wise (5 lots) over a period of 60 min. The walls of the reactor were rinsed with 500 mL toluene. After addition, the resultant reaction mass was stirred at 90 °C for 75 min and the progress of the reaction was monitored by HPLC. For this, 0.5 mL of the reaction mass was diluted with 3 mL of methanol and the formation of acid chloride was analyzed indirectly by detecting its corresponding methyl ester by HPLC). After 2 hours, HPLC analysis indicated about 0.35 a% of unreacted 8-chloro-6-(trifluoromethyl)imidazo[1,2-a]pyridine-2-carboxylic acid and about 99.0 a% of 8-chloro-6-(trifluoromethyl)imidazo[1,2-a]pyridine-2-carboxylic acid methyl ester. The resultant reaction mass was further heated to 140 °C (oil bath temperature) and distilled at about 109 °C (mass temperature) and 105-107 °C (vapor temperature) at atmospheric pressure over a period of 2.5 hours to remove toluene (about 600 mL) and excess thionyl chloride present in the reaction mass. After distillation, the reaction mass was gradually cooled to 30 °C over a period of 60 min. The concentration of 8-chloro-6-(trifluoromethyl)imidazo[1,2-a]pyridine-2-carboxylic acid was about 0.07 a% and the concentration of 8-chloro-6-(trifluoromethyl)imidazo[1,2-a]pyridine-2-carboxylic acid methyl ester about 99.2 a% as measured by HPLC at 230 nm. Step B: Preparation of 8-chloro-N-[(2-chloro-5-methoxyphenyl)sulfonyl]-6-(trifluoromethyl)-imidazo[1,2-a]pyridine-2-carboxamide (Compound 1 ({drug1})): The resultant acid chloride solution from Step A was cooled to 0 °C over a period of 30 min and to this, ethyl acetate (400 mL) was charged under a nitrogen atmosphere at 0 °C. The resultant reaction mass was stirred at 0 °C for 5 min and to this 2-chloro-5-methoxybenzenesulfonamide (90 g, 0.391 moles) (prepared as in WO 2010/129500) was charged. To the resultant reaction mass tributylamine (242 g, 1.305 moles) was added drop-wise over a period of 60 min using an addition funnel. A temperature increase of 8 °C was observed during the addition. After the addition, the resultant reaction mass was stirred at 10 °C for 30 min and the temperature was gradually raised to 25 °C. The progress of the reaction was monitored. For this, 0.5 mL the reaction mass was diluted with 3 mL of methanol and analyzed by HPLC analysis at 230 nm. After about 15 min at 25 °C, the concentration of 8-chloro-6-(trifluoromethyl)imidazo[1,2-a]pyridine-2-carboxylic acid methyl ester was about 4.30 a%, 8-chloro-6-(trifluoromethyl)imidazo[1,2-a]pyridine-2-carboxylic acid about 1.81 a%, unreacted 2-chloro-5-methoxybenzenesulfonamide was about 2.86 a% and Compound 1 ({drug1}) was about 86.5 a%. The resultant reaction mass was stirred overnight at 25 °C and the progress of the reaction was monitored by HPLC at 230 nm. After 15 hours at 25 °C, the concentration of 8-chloro-6-(trifluoromethyl)imidazo[1,2-a]pyridine-2-carboxylic acid methyl ester was about 0.84 a%, 8-chloro-6-(trifluoromethyl)imidazo[1,2-a]pyridine-2-carboxylic acid about 1.72 a%, unreacted 2-chloro-5-methoxybenzenesulfonamide

about 2.20 a% and Compound 1 ({drug1}) about 91.9 a%. The reaction mass was stirred at 25 °C and to this, water (360 mL) was charged at 25 °C over a period of 60 min. To the resultant reaction mixture, a solution of HCl (32 wt%, 191 g) in 200 mL water was added over a period of 45 min. During the HCl addition, the reaction mass became a clear solution initially and then gradually became a hazy liquid during the end of the addition. A temperature increase of 9 °C was observed during this addition. After the addition, the resultant reaction mass was heated to 55 °C, stirred for 60 min, gradually cooled to 5 °C, then stirred at 5 °C for 30 min and filtered. The wet cake was washed with water (3 times with 3100 mL) and vacuum-dried on a Buchner funnel. The vacuum-dried material was analyzed for the chloride content which indicated no significant amount of chloride salts present. The wet cake was washed with toluene (2 x 400 mL) and vacuum-dried on a Buchner funnel for about 12 hours. The crude product was obtained as 185 grams of an off-white solid. The toluene and ethyl acetate content in the product were 17.3 wt% and 0.855 wt%, respectively. The water content was 0.84 wt%. The HPLC purity of the crude product (wet sample) was 99.8 a% and 80.0 wt%. The yield based on HPLC wt% analysis was 85%. 1H-NMR was consistent with Compound 1 ({drug1}) [(DMSO-d6) δ 3.86 (s, 3H), 7.30 (d, 1H), 7.57 (dd, 1H), 7.64 (d, 1H), 7.96 (d, 1H), 8.84 (s, 1H), 9.34 (d, 1H)] containing toluene. The molar ratio of toluene and Compound 1 ({drug1}) was about 1.06 indicating a 1 : 1 toluene solvate. The pXRD diffraction pattern was consistent with the toluene solvate (Form TS ({drug1e})) of Compound 1 ({drug1}). The crude wet solid was used for form conversion studies.

PREPARATION EXAMPLE 2: Preparation of Mixed Forms A and B of Compound 1 ({drug1}): The toluene solvate of Compound 1 ({drug1}) was prepared as described in Preparation Example 1 and was desolvated by drying in a vacuum oven (8-15 kPa absolute pressure) at 90 °C for 4 days. The toluene content in the product was 0.11 wt% and the water content was 0.09 wt%. 1H-NMR was consistent with Compound 1 ({drug1}) [(DMSO-d6) δ 3.86 (s, 3H), 7.30 (d, 1H), 7.57 (dd, 1H), 7.64 (d, 1H), 7.96 (d, 1H), 8.84 (s, 1H), 9.34 (d, 1H)]. The purity by HPLC was 99.9 a% and 99.0 wt%. The DSC thermogram showed two endotherms with peak temperatures of 21 1.1 °C and 219.1 °C. The pXRD pattern confirms that the material was crystalline and corresponded to a mixture of crystals of Form A ({drug1a}) and Form B ({drug1b}).

PREPARATION EXAMPLE 3: Conversion of the Toluene-Solvate of Compound 1 ({drug1}) to Form A ({drug1a}) To a 500 mL three-neck round-bottom flask equipped with overhead stirrer, oil bath, a Dean-Stark apparatus and temperature probe was charged 25 g of Compound 1 ({drug1}) wet cake prepared according to Preparation Example 1 (toluene content = 17.3 wt%) and water (75 mL) at 25 °C. The resultant reaction mass was heated to 95 °C (reaction mass temperature) and maintained at 95-96 °C over a period of 5 hours while stirring at about 850 rpm. The water collected from the Dean-Stark apparatus was recycled to maintain about constant reaction volume while toluene was removed from the reaction mass. After about 3 hours no further distillation of toluene was observed. A slurry sample was taken from the reaction mass under agitation. The toluene and ethyl acetate content of the slurry was determined by GC analysis as 56 ppm and 17 ppm, respectively. About 10 mL of the sample was taken from the reaction mixture, cooled to 25 °C, filtered and vacuum-dried on a Buchner funnel for 15 min. The wet cake showed about 429 ppm of toluene and 36 ppm of ethyl acetate. The wet cake was dried in a vacuum oven at 55 °C (8-15 kPa absolute pressure) for about 1 hour and analyzed by DSC and pXRD. Both DSC and pXRD data was consistent with Form A ({drug1a}) of Compound 1 ({drug1}). Since the portion of the sample from the reaction mass indicated the conversion to Form A ({drug1a}), the entire reaction mass was filtered, dried in a vacuum oven (8-15 kPa absolute pressure) at 55 °C for 1 hour. The dried product was analyzed by pXRD and DSC. Both DSC and pXRD data was consistent with Form A ({drug1a}) of Compound 1 ({drug1}).

PREPARATION EXAMPLE 4: Additional Polymorph Conversion Studies of the Toluene-Solvate (Form TS ({drug1e})) of Compound 1 ({drug1}) Form-conversion experiments according to Preparative Example 3 were conducted with water, methanol and the mixture thereof as the suspension medium. The experimental conditions and apparatus used were as described in Preparative Example 3 unless otherwise noted. In each experiment 25 g of the wet cake of Compound 1 ({drug1}) prepared according to Preparation Example 1 (toluene content = 17.3 wt%) were used as starting material. The experimental conditions are summarized in Table 2. The conditions of Preparative Example 3 are included for reference. The suspensions were subjected to azeotropic distillation under reflux conditions to remove the toluene using the Dean-Stark apparatus. After 3 to 5 hours no more toluene was visibly removed and the resultant slurries were filtered, dried in a vacuum oven (8-15 kPa absolute pressure) at 55 °C for 1 hour and analyzed by DSC and pXRD. The DSC and pXRD data of all the examples listed in Table 2 were consistent with Form A ({drug1a}) of Compound 1 ({drug1}).

Table 2 - Experimental Conditions of Polymorph Conversion Studies and Resulting Form:

| Example | Amount of Compound **1** (g) | Starting Polymorph Form | Volume of Water (mL) | Volume of Methanol (mL) | Slurry Temperature (°C) | Polymorph Form Obtained |
|---|---|---|---|---|---|---|
| 3 | 25 | TS | 75 | - | 95-96 | A |

(continued)

| Example | Amount of Compound **1** (g) | Starting Polymorph Form | Volume of Water (mL) | Volume of Methanol (mL) | Slurry Temperature (°C) | Polymorph Form Obtained |
|---|---|---|---|---|---|---|
| 4a | 25 | TS | 125 | - | 95.96 | A |
| 4b | 25 | TS | 175 | - | 95-96 | A |
| 4c | 25 | TS | 125 | - | 95-96 | A |
| 4d | 25 | TS | 100 | 25 | 95-96 | A |
| 4c | 25 | TS | - | 100 | 63 | A |
| 4f | 25 | TS | 125 | - | 85-87 | A |
| 4g | 25 | TS | 125 | - | 85-87 | A |

PREPARATION EXAMPLE 5: Solvent Screening to Prepare Various Crystal Forms of Compound 1 ({drug1}) A set of solvents was evaluated for the preparation of various crystal forms including solvate forms of Compound 1 ({drug1}). The starting material of Compound 1 ({drug1}) was prepared according to Preparation Example 2. Aliquots of Compound 1 ({drug1}) thus prepared were either dissolved or slurried in the selection of solvents listed in Table 3 and treated according to the following descriptions. The resulting dry materials were analyzed by 1H-NMR, pXRD, DSC and TGA. The endothermic DSC events and resulting crystal forms are also reported in Table 3. In Example 5a, 1 g of Compound 1 ({drug1}) was dissolved in 6.5 mL of acetone at 56 °C. The solution was slowly cooled to about 5 °C over a period of 1 h. The resulting crystals were filtered, suction dried for 1 h and dried in a vacuum oven at 65 °C and 8 kPa absolute pressure for 12 h. Analysis bypXRD, DSC, TGA and 1H-NMR of the resulting material indicated Form A ({drug1a}). In Example 5b, 1 g of Compound 1 ({drug1}) was slurried in 10 mL of methanol, refluxed for 3 h, filtered, cooled to about 25 °C, suction dried for 1 h and dried in a vacuum oven at 70 °C and 8 kPa absolute pressure for 12 h. Analysis bypXRD, DSC, TGA and 1H-NMR of the resulting material indicated Form A ({drug1a}). In Example 5c, 1 g of Compound 1 ({drug1}) was slurried in 10 mL of deionized water, refluxed for 3 h, cooled to about 25 °C, filtered, suction dried for 1 h and dried in a vacuum oven at 70 °C and 8 kPa absolute pressure for 12 h. Analysis by pXRD, DSC, TGA and 1H-NMR of the resulting material indicated Form A ({drug1a}). In Example 5d, 1 g of Compound 1 ({drug1}) was slurried in 10 mL of n-heptane, refluxed for 3 h, cooled to about 25 °C, filtered, suction dried for 1 h and dried in a vacuum oven at 70 °C and 8 kPa absolute pressure for 12 h. Analysis bypXRD, DSC, TGA and 1H-NMR of the resulting material indicated Form A ({drug1a}). In Example 5e, 1 g of Compound 1 ({drug1}) was dissolved in 14 mL of ethyl acetate at 65 °C. The solution was cooled to 5 °C over a period of 1 h. The resulting crystals were filtered, suction dried for 1 h and dried in a vacuum oven at 65 °C and 8 kPa absolute pressure for 12 h. Analysis by pXRD, DSC, TGA and ^H-NMR of the resulting material indicated a solvate form containing ethyl acetate. In Example 5f, 1 g of Compound 1 ({drug1}) was refluxed in 10 mL of iso-propanol for 3 h, cooled to about 25 °C, filtered, suction dried for 1 h and dried in a vacuum oven at 65 °C and 8 kPa absolute pressure for 12 h. Analysis by pXRD, DSC, TGA and 1H-NMR of the resulting material indicated a solvate form containing z'so-propanol. In Example 5g, 1 g of Compound 1 ({drug1}) was refluxed in 10 mL of methyl tert-butyl ether for 3 h, cooled to about 25 °C, filtered, suction dried for 1 h and dried in a vacuum oven at 65 °C and 8 kPa absolute pressure for 12 h. Analysis by pXRD, DSC, TGA and 1H-NMR of the resulting material indicated a solvate form containing methyl tert-butyl ether. In Example 5h, 1 g of Compound 1 ({drug1}) was dissolved in 12 mL of acetonitrile at 65 °C. The solution was slowly cooled to 5 °C over a period of 4 h. The resulting crystals were filtered, suction dried for 1 h and dried in a vacuum oven at 65 °C and 8 kPa absolute pressure for 12 h. Analysis bypXRD, DSC, TGA and ^H-NMR of the resulting material indicated Form D ({drug1d}). In Example 5i, 1 g of Compound 1 ({drug1}) was dissolved in 12 mL of tetrahydrofuran at 65 °C. The solution was slowly cooled to 25 °C over a period of 4 h. The resulting crystals were filtered, suction dried for 1 h and dried in a vacuum oven at 65 °C and 8 kPa absolute pressure for 12 h. Analysis by pXRD, DSC, TGA and 1H-NMR of the resulting material indicated solvate form containing tetrahydrofuran. In Example 5j, 1 g of Compound 1 ({drug1}) was slurried in 12 mL of ethanol, refluxed for 3 h, cooled to about 25 °C, filtered, suction dried for 1 h and dried in a vacuum oven at 70 °C and 8 kPa absolute pressure for 12 h. Analysis by pXRD, DSC, TGA and 1H-NMR of the resulting material indicated solvate form containing ethanol. In Example 5k, 1 g of Compound 1 ({drug1}) was slurried in 10 mL of decalin, heated at 120 °C for 3 h, cooled to about 25 °C, filtered, suction dried for 1 h and dried in a vacuum oven at 90 °C and 8 kPa absolute pressure for 12 h. Analysis by pXRD, DSC, TGA and 1H-NMR of the resulting material indicated solvate form containing decalin. In Example 51, 1 g of Compound 1 ({drug1}) was dissolved in 12.5 mL of methyl z'sobutyl ketone at 65 °C. The solution was cooled to about 25 °C over a period of 3 h. The resulting crystals were filtered, suction dried for 1 h and dried in a vacuum oven at 90

°C and 8 kPa absolute pressure for 12 h. Analysis by pXRD, DSC, TGA and 1H-NMR of the resulting material indicated solvate form containing methyl z'so-butyl ketone. In Example 5m, 1 g of Compound 1 ({drug1}) was dissolved in 6 mL of mesitylene at 120 °C. The resulting solution was slowly cooled to about 25 °C over a period of 4 h. The resulting crystals were filtered, suction dried for 1 h and dried in a vacuum oven at 90 °C and 8 kPa absolute pressure for 12 h. Analysis by pXRD, DSC, TGA and 1H-NMR of the resulting material indicated a mixture of Forms A and B. In Example 5n, 1 g of Compound 1 ({drug1}) was dissolved in 17 mL of toluene at 90 °C. The resulting solution was slowly cooled to about 25 °C over a period of 4 h. The resulting crystals were filtered, suction dried for 1 h and dried in a vacuum oven at 90 °C and 8 kPa absolute pressure for 12 h. Analysis by pXRD, DSC, TGA and 1H-NMR of the resulting material indicated a solvate form containing toluene. The residual toluene was remaining in the product even after additional 12 hr drying under the above drying conditions. In Example 5o, 1 g of Compound 1 ({drug1}) was dissolved in 15 mL of dichloromethane at 25 °C. The resulting solution was slowly cooled to about 5 °C and maintained at 5 °C for 30 min. The resulting crystals were filtered, suction dried for 1 h and dried in a vacuum oven at 65 °C and 8 kPa absolute pressure for 12 h. Analysis by pXRD, DSC, TGA and 1H-NMR of the resulting material indicated Form B ({drug1b}). In Example 5p, 1 g of Compound 1 ({drug1}) was slurried in 10 mL of tetralin at 120 °C for 3 h, slowly cooled to about 25 °C, filtered, suction dried for 1 h and dried in a vacuum oven at 90 °C and 8 kPa absolute pressure for 12 h. Analysis by pXRD, DSC, TGA and 1H-NMR of the resulting material indicated a mixture of Forms A and B. In Example 5q, 1 g of Compound 1 ({drug1}) was dissolved in 9 mL of 1,4-dioxane at 65 °C. The resulting solution was slowly cooled to about 25 °C over 4 h and maintained at 25 °C for 12 h. The resulting crystals were filtered, suction dried for 1 h and dried in a vacuum oven at 70 °C and 8 kPa absolute pressure for 12 h. Analysis by pXRD, DSC, TGA and 1H-NMR of the resulting material indicated a solvate form containing 1,4-dioxane. In Example 5r, 1 g of Compound 1 ({drug1}) was dissolved in 7 mL of acetic acid at 80 °C. The resulting solution was slowly cooled to about 25 °C over 4 h and maintained at 25 °C for 12 h. The resulting crystals were filtered, suction dried for 1 h and dried in a vacuum oven at 70 °C and 8 kPa absolute pressure for 12 h. Analysis by pXRD, DSC, TGA and 1H-NMR of the resulting material indicated Form D ({drug1d}). In Example 5s, 1 g of Compound 1 ({drug1}) was dissolved in 7 mL of z'so-propyl acetate at 70 °C. The resulting solution was slowly cooled to about 25 °C over 4 h and maintained at 25 °C for 12 h. The resulting crystals were filtered, suction dried for 1 h and dried in a vacuum oven at 70 °C and 8 kPa absolute pressure for 12 h. Analysis by pXRD, DSC, TGA and 1H-NMR of the resulting material indicated a solvate form containing z'so-propyl acetate. In Example 5t, 1 g of Compound 1 ({drug1}) was slurried in 10 mL of o-xylene at 100 °C, cooled to about 25 °C, filtered, suction dried for 1 h and dried in a vacuum oven at 90 °C and 8 kPa absolute pressure for 12 h. Analysis by pXRD, DSC, TGA and 1H-NMR of the resulting material indicated a mixture of Forms A and B.

Table 3 - Crystal Form Conversion Studies Using Various Solvents:

| Ex. No. | Solvent | Polymorph Form Obtained | DSC Endotherm 1 (°C) | DSC Endotherm 2 (°C) |
|---|---|---|---|---|
| 5a | acetone | A | 210.9 | 218.9 |
| 5b | methanol | A | 209.9 | 218.7 |
| 5c | water | A | 212,1 | 218.7 |
| 5d | a-heptane | A | 212.8 | 219.1 |
| 5e | ethyl acetate | Solvate | 210.8 | 218.6 |
| 5f | *iso*-propanol | Solvate | 211.4 | 218.3 |
| 5g | methyl *tert*-butyl ether | Solvate | 210.3 | 218.4 |
| 5h | acetonitrile | D | 212.8 | 219.4 |
| 5i | Tetrahydrofuran | Solvate | 210.5 | 218.6 |
| 5j | ethanol | Solvate | 208.2 | 218.7 |
| 5k | decalin | Solvate | 211.1 | 218.3 |
| 5l | methyl *iso*-butyl ketone | Solvate | 211.6 | 218.9 |
| 5m | mesitylene | A + B | 211.8 | 218.4 |
| 5n | toluene | Solvate | 210.6 | 218.8 |
| 5o | dichloromethane | B | 210.5 | 218.5 |
| 5p | tetralin | A + B | 212.9 | 219.0 |
| 5q | 1,4-dioxane | Solvate | 210.8 | 218.9 |

(continued)

| Ex. No. | Solvent | Polymorph Form Obtained | DSC Endotherm 1 (°C) | DSC Endotherm 2 (°C) |
|---|---|---|---|---|
| 5r | acetic acid | D | 213.1 | 219.5 |
| 5s | *iso*-propyl acetate | Solvate | 211.6 | 218.9 |
| 5t | *o*-xylene | A + B | 212.0 | 218.6 |

PREPARATION EXAMPLE 6: Preparation of Polymorph Form D ({drug1d}) of Compound 1 ({drug1}): Polymorph Form D ({drug1d}) of Compound 1 ({drug1}) was prepared by heating Compound 1 ({drug1}) prepared according to Preparation Example 2 with acetonitrile at 65 °C for 5 minutes. The clear solution obtained was gradually cooled to 5 °C over 4 hours and maintained at that temperature for 12 hours without disturbance. The crystals formed were filtered and dried at 65 °C in a vacuum oven (8 kPa absolute pressure) for 12 hours. The isolated solid was found to have a unique pXRD diffraction pattern indicating a distinct crystal form (polymorph Form D ({drug1d})). Form D ({drug1d}) was also prepared according the above procedure using acetic acid as the solvent as evidenced by displaying the same pXRD pattern. Both the sample crystallized from acetonitrile and acetic acid were also analyzed by single crystal XRD as described in the Characterization Examples below.

PREPARATION EXAMPLE 7: Stability of a Mixture of Crystal Forms A and B in a Liquid Formulation: The mixture of polymorphs Form A ({drug1a}) and Form B ({drug1b}) of Compound 1 ({drug1}) was prepared as described in Preparation Example 2. The presence of both polymorph forms was confirmed by pXRD. A suspension concentrate Formulation X containing Compound 1 ({drug1}) of mixed polymorph Forms A and B was prepared. The composition of Formulation X is given in the table below. All ingredients were combined in the order of ingredients listed in the table to yield a total amount of 6.5 grams. The mixture of combined ingredients was milled with an attritor mill in a 30 ml size flask equipped with a variable-speed overhead impeller using 14.3 grams of 0.8 to 1.0 mm sized glass beads. The flask content was agitated at room temperature for 5 min at 4000 rpm followed by 13 min at 6000 rpm. The resulting formulation was evaluated under a light microscope (Leica, model DM LS) at 400 to 1000-fold magnification to evaluate the homogeneity, size and shape of the particles of Compound 1 ({drug1}) in the formulation. The particles were found to be of irregular shape and in the narrow range of about 3 to 10 μm. The sample was left standing for about 15 hours at room temperature and then reexamined under the microscope; it was found that larger cubical crystals in the size range between about 5 to 30 μm had formed. Also, clusters of dentritic crystals of a length between about 50 to 200 μm had formed. Such changes in crystal size and morphology constitutes an undesirable formulation instability which may result in undesired effects such as the active compound sedimenting out or the larger crystals not providing the full extend of bioefficacy owing to their reduced specific surface area. The formulation sample, after standing for a total of 18 hours at room temperature, was re-milled for 45 min at 6000 rpm using the same equipment and conditions as described above. The observation under the microscope showed that the particles of Compound 1 ({drug1}) were well dispersed in the size range of about 3 to 10 μm. The sample was split and stored for 14 days at room temperature and at 54 °C, respectively. The reexamination of the two stored samples under the microscope showed no signs of crystal growth or change in morphology for either storage temperature indicathg good particle size stability in the formulation. The concentration of Compound 1 ({drug1}) in the samples stored at room temperature and 54 °C were determined by HPLC as 49.7 wt% and 51.2 wt%, respectively, indicating good chemical stability in the formulation. To determine the crystal form of Compound 1 ({drug1}) in the formulation sample that had been re-milled after crystal growth, Compound 1 ({drug1}) was separated from the formulation as follows. An aliquot of the formulation (0.72 grams) was centrifuged in a 1.5 ml centrifuge tube for 6 cycles of 30 min each. After each centrifugation the supernatant was removed, replaced with deionized water and the tube content was thoroughly mixed. After the final centrifugation cycle the supernatant was discarded and the solids were dried at 40 °C for about 70 hours. Analysis by pXRD and DSC of the resulting material indicated pure polymorph Form A ({drug1a}).

Formulation Example X

| Ingredient | Concentration (wt%) |
|---|---|
| water | 40.15 |
| silicones | 0.3 |
| xanthan gum | 0.2 |
| attapulgite clay | 0.5 |
| biocide | 0.05 |
| propylene glycol | 1.5 |

(continued)

Formulation Example X

| Ingredient | Concentration (wt%) |
|---|---|
| glycerol | 3.0 |
| methylmethacrylate ethoxylated copolymer | 3.0 |
| ethylene oxide/propylene oxide block copolymer and ethoxylated alcohol | 2.0 |
| polymorph Forms A and B of Compound 1 | 49.3 |

PREPARATION EXAMPLE 8: Preparation and Isolation of 8-chloro-6-(trifluoromethyl)imidazo[1,2-a]pyridine-2-carbonyl chloride: To a 250 mL four neck round bottom flask, was charged toluene (50 mL), N-formyl piperidine (0.177 g, 1.6 mmol) and thionyl chloride (3.37 g, 27.8 mmol) at 23-25 °C under a nitrogen atmosphere. The resultant reaction mass was heated to 82 °C over a period of 20 min and to this 8-chloro-6-(trifluoromethyl)imidazo[1,2-a]pyridine-2-carboxylic acid (5.0 g, 18.6 mmol) was added portion wise over a period of 25 min. Additional toluene (25 mL) was also added. During the addition of the acid, the reaction mass changed from a slurry to a pale green solution liberating HCl gas. The resultant mass was heated to 90 °C and stirred for 90 min and the progress of the reaction was monitored by HPLC (0.5 mL of the reaction mass was diluted with 3 mL of methanol and analyzed for the formation of acid chloride as its corresponding methyl ester). After 90 min, HPLC analysis (230 nm) indicated the unreacted acid 0.32 A% and the methyl ester 99.24 A%. The resultant reaction mass was distilled at -109 °C (mass temperature) at atmospheric pressure over a period of 30 min to remove the toluene-thionyl chloride mixture (~50 mL). During the distillation the reaction mass turned dark brown. The reaction mass was gradually cooled to 30 °C over a period of 30 mins and a sample was analyzed by HPLC. The HPLC (at 230 nm) analysis indicated the unreacted acid - 0.33 % and the formation of methyl ester - 99.12 %. The title acid chloride was completely dried at 50 °C for 30 min under vacuum with a stream of nitrogen flow, to remove residual toluene and analyzed by HPLC and 1H NMR. The title acid chloride was isolated as a grey solid (6.5 g). HPLC purity (230 nm) of 95.60 % AP (as methyl ester). 1H-NMR (CDC13) $\delta$ 7.57 (s, 1H), 8.53 (s, 1H), 8.56 (s, 1H). 1H-NMR (DMSO-d6) $\delta$ 7.90 (s, 1H), 8.68 (s, 1H), 9.30 (s, 1H).

CHARACTERIZATION EXAMPLE 1: X-Ray Powder Diffraction for Compound 1 ({drug1}) Polymorph Form A ({drug1a}): Powder X-ray diffraction was used to identify the crystalline phases of various samples of Compound 1 ({drug1}). Data were obtained with a Philips X'PERT automated powder diffractometer, Model 3040. The diffractometer was equipped with automatic variable anti- scatter and divergence slits, X'Celerator RTMS detector, and Ni filter. The radiation was Cu-K(alpha) (45 kV, 40 mA). Data were collected at room temperature from 3 to 50 degrees 2-theta using a continuous scan with an equivalent step size of 0.02 degrees and a count time of 320 seconds per step in theta-theta geometry. Samples were ground with an agate mortar and pestle as needed and prepared on low background amorphous silica specimen holders as a thin layer of powdered material. MDI/Jade software version 9.1 is used with the International Committee for Diffraction Data database PDF4+ 2008 for phase identification. Cu-K(alphal) X-ray diffraction maxima for Form A ({drug1a}) of Compound 1 ({drug1}) were calculated using the MDI/Jade "Find Peaks" routine and are listed Table 4.

Table 4 - 2Θ X-ray Maxima (in degrees) for Polymorph Form A ({drug1a}) of Compound 1 ({drug1}):

| 2θ | 2θ | 2θ | 2θ | 2θ | 2θ | 2θ |
|---|---|---|---|---|---|---|
| 11.651 | 21.026 | 25.973 | 30.652 | 36.967 | 42.451 | 47.813 |
| 12.854 | 21.543 | 26.490 | 33.905 | 37.703 | 42.935 | 48.167 |
| 13.705 | 23.097 | 27.308 | 32.657 | 37.956 | 43.538 | 48.648 |
| 14.056 | 23.582 | 27.611 | 33.042 | 38.607 | 44.089 | 49.118 |
| 15.426 | 24.285 | 27.995 | 34.629 | 38.992 | 44.740 | 49.502 |
| 18.286 | 24.584 | 29.131 | 35.028 | 39.875 | 45.926 | |
| 18.836 | 24.954 | 29.764 | 35.614 | 40.443 | 46.644 | |
| 19.789 | 25.604 | 30.367 | 35.982 | 41.632 | 47.279 | |

CHARACTERIZATION EXAMPLE 2: Simulated X-Ray Powder Diffraction Pattern for Compound 1 ({drug1}) Polymorph Form B ({drug1b}): A simulated powder pattern was calculated from the atomic coordinates and cell parameters determined from the single crystal structure for polymorph Form B ({drug1b}) of Compound 1 ({drug1}). This is

based on data collected at -100 °C. The X-ray pattern was calculated using the Cambridge Mercury program with Cu wavelength (0.154056 nm), 3 to 50 degrees 2-theta and a step size of 0.02 degrees. Peak positions were selected from the calculated pattern using the MDI/Jade software version 9. Cu-K(alphal) X-ray diffraction maxima for Form B ({drug1b}) of Compound 1 ({drug1}) were calculated using the MDI/Jade "Find Peaks" routine and are listed Table 5.

Table 5 - 2Θ X-ray Maxima (in degrees) for Polymorph Form B ({drug1b}) of Compound 1 ({drug1}):

| 2θ | 2θ | 2θ | 2θ | 2θ | 2θ | 2θ |
|---|---|---|---|---|---|---|
| 7.998 | 15.259 | 20.999 | 27.283 | 32.382 | 37.442 | 43.139 |
| 8.362 | 15.778 | 21.880 | 27.581 | 32.758 | 37.903 | 43.478 |
| 9.460 | 16.038 | 22.718 | 29.242 | 32.961 | 38.340 | 44.259 |
| 10.417 | 16.341 | 23.082 | 28.642 | 33.342 | 38.537 | 45.199 |
| 10.938 | 16.603 | 23.341 | 29.139 | 33.943 | 39.340 | 45.438 |
| 11.997 | 17.219 | 23.979 | 29.657 | 34.400 | 39.742 | 46.102 |
| 12.339 | 18.120 | 24.583 | 30.177 | 34.683 | 39.942 | 46.399 |
| 12.738 | 18.683 | 24.822 | 30.520 | 35.161 | 40.241 | 47.100 |
| 13.083 | 18.981 | 25.060 | 30.921 | 35.358 | 41.001 | 48.120 |
| 14.020 | 19.502 | 25.978 | 31.479 | 36.040 | 42.559 | 49.097 |
| 14.443 | 20.320 | 26.519 | 31.958 | 36.463 | 42.782 | |

CHARACTERIZATION EXAMPLE 3: Simulated X-Ray Powder Diffraction Pattern for Compound 1 ({drug1}) Polymorph Form C ({drug1c}):

A simulated powder pattern was calculated from the atomic coordinates and cell parameters determined from the single crystal structure for polymorph Form C ({drug1c}) of Compound 1 ({drug1}). This is based on data collected at -100 °C. The X-ray pattern was calculated using the Cambridge Mercury program with Cu wavelength (0.154056 nm), 3 to 50 degrees 2-theta and a step size of 0.02 degrees. Peak positions were selected from the calculated pattern using the MDI/Jade software version 9. Cu-K(alphal) X-ray diffraction maxima for Form C ({drug1c}) of Compound 1 ({drug1}) were calculated using the MDI/Jade "Find Peaks" routine and are listed Table 6.

Table 6 - 2Θ X-ray Maxima (in degrees) for Polymorph Form C ({drug1c}) of Compound 1 ({drug1})

| 2θ | 2θ | 2θ | 2θ | 2θ | 2θ | 2θ |
|---|---|---|---|---|---|---|
| 6.181 | 15.442 | 20.760 | 25.837 | 31.279 | 36.920 | 42.080 |
| 7.222 | 15.777 | 21.161 | 26.300 | 31.878 | 37.480 | 42.662 |
| 7.603 | 16.423 | 21.585 | 26.557 | 32.499 | 37.719 | 43.141 |
| 8.363 | 16.859 | 22.120 | 27.160 | 33.061 | 38.239 | 44.44 |
| | | | | | | |
| 8.657 | 17.360 | 22.420 | 27.520 | 33.479 | 38.457 | 44.899 |
| 9.377 | 17.697 | 22.996 | 28.180 | 33.737 | 38.956 | 45.141 |
| 11.860 | 18.340 | 23.542 | 28.661 | 34.418 | 39.378 | 46.300 |
| 12.421 | 18.583 | 23.880 | 29.281 | 34.662 | 39.601 | 47.319 |
| 13.041 | 19.098 | 24.379 | 29.579 | 35.541 | 40.360 | 47.639 |
| 13.583 | 19.420 | 24.701 | 30.001 | 35.961 | 41.059 | 48.239 |
| 14.479 | 19.899 | 25.181 | 30.502 | 36.239 | 41.640 | 48.825 |
| 15.041 | 20.360 | 25.622 | 30.761 | 36.618 | 41.861 | |

CHARACTERIZATION EXAMPLE 4: Simulated X-Ray Powder Diffraction Pattern for Compound 1 ({drug1})

Polymorph Form D ({drug1d}): A simulated powder pattern was calculated from the atomic coordinates and cell parameters determined from the single crystal structure for polymorph Form D ({drug1d}) of Compound 1 ({drug1}). This is based on data collected at -100 °C. The X-ray pattern was calculated using the Cambridge Mercury program with Cu wavelength (0.154056 nm), 3 to 50 degrees 2-theta and a step size of 0.02 degrees. Peak positions were selected from the calculated pattern using the MDI/Jade software version 9. Cu-K(alphal) X-ray diffraction maxima for Form D ({drug1d}) of Compound 1 ({drug1}) were calculated using the MDI Jade "Find Peaks" routine and are listed Table 7.

Table 7 - 2Θ X-ray Maxima (in degrees) for Polymorph Form D ({drug1d}) of Compound 1 ({drug1}):

| 2θ | 2θ | 2θ | 2θ | 2θ | 2θ | 2θ |
|---|---|---|---|---|---|---|
| 5.981 | 16.160 | 24.099 | 28.717 | 32.343 | 37.858 | 46.103 |
| 10.342 | 17.821 | 24.679 | 28.921 | 32.658 | 39.200 | 46.420 |
| 11.641 | 18.001 | 25.121 | 29.162 | 33.060 | 39.521 | 47.980 |
| 12.263 | 18.478 | 25.279 | 29.516 | 33.442 | 40.160 | 48.797 |
| 12.520 | 19.320 | 25.682 | 29.801 | 34.420 | 40.461 | |
| 14.598 | 20.778 | 26.120 | 29.943 | 35.421 | 41.160 | |
| 14.840 | 21.281 | 26.922 | 30.143 | 36.683 | 41.556 | |
| 15.378 | 22.583 | 27.497 | 31.219 | 37.023 | 42.641 | |
| 15.620 | 23.320 | 28.460 | 31.600 | 37.383 | 43.620 | |

CHARACTERIZATION EXAMPLE 5: X-Ray Powder Diffraction Pattern for Compound 1 ({drug1}) Polymorph Form TS ({drug1e}): Powder X-ray diffraction was used to characterize the toluene solvate polymorph form (Polymorph Form TS ({drug1e})) of Compound 1 ({drug1}). Data were obtained with a Philips X'PERT automated powder diffractometer, Model 3040. The diffractometer was equipped with automatic variable anti-scatter and divergence slits, X'Celerator RTMS detector, and Ni filter. The radiation was Cu-K(alpha) (45 kV, 40 mA). Data were collected at room temperature from 3 to 50 degrees 2-theta using a continuous scan with an equivalent step size of 0.02 degrees and a count time of 320 seconds per step in theta-theta geometry. Samples were lightly ground with an agate mortar and pestle as needed and prepared on low background silicon specimen holders as a thin layer of powdered material. MDI/Jade software version 9.1 was used with the International Committee for Diffraction Data database PDF4+ 2008 for phase identification. Cu-K(alphal) X-ray diffraction maxima for Form TS ({drug1e}) of Compound 1 ({drug1}) were calculated using the MDI/Jade "Find Peaks" routine and are listed Table 8.

Table 8 - 2Θ X-ray Maxima (in degrees) for Polymorph Form TS ({drug1e}) of Compound 1 ({drug1}):

| 2θ | 2θ | 2θ | 2θ | 2θ | 2θ | 2θ |
|---|---|---|---|---|---|---|
| 6.889 | 14.508 | 18.603 | 24.451 | 32.222 | 36.906 | 42.015 |
| 8.608 | 14.908 | 19.053 | 25.672 | 32.671 | 37.452 | 43.869 |
| 9.997 | 15.728 | 20.325 | 26.942 | 33.561 | 38.323 | 45.173 |
| 11.433 | 16.481 | 21.643 | 27.945 | 33.994 | 39.057 | 46.092 |
| 12.871 | 16.998 | 22.429 | 28.913 | 34.528 | 40.711 | 47.514 |
| 13.606 | 17.433 | 23.316 | 30.951 | 36.114 | 41.548 | 48.148 |

CHARACTERIZATION EXAMPLE 6: Single Crystal X-Ray Diffraction for Polymorph Form A ({drug1a}) of Compound 1 ({drug1}) Suitable single crystals for polymorph Form A ({drug1a}) were grown from slow evaporation of methanol. A colorless irregular block with approximate dimensions of 0.10 x 0.10 x 0.04 mm was chosen for data collection and mounted on a polymer loop. Single crystal data was collected using a Bruker Platform goniometer with an Apex-II detector. The diffractometer was equipped with an incident beam monochromator using Mo-Kα radiation (λ = 0.71073 A) and a monocap collimator. The crystals were cooled in a -100 °C nitrogen flow during data collection. The data were indexed and integrated using the Apex-II suite of programs including Sainplus and SADABS. The triclinic cell parameters were determined to be: a = 8.483(3) A, b = 10.004(3) A, c = 11.638(4) A, alpha = 86.690(5) °, beta = 87.984(5) °, gamma = 65.1 14(4) °, volume = 894.4(5) A3. The space group was determined to be P-1. The molecular weight was 468.23

g/mol giving a calculated density of 1.739 g/cm3, and μ(Mo) = 0.54 mm"1 for Z = 2. Data reduction led to 3684 unique data from a two-theta range = 3.50 to 53.12°. Structure solution and refinements were performed using the Shelxtl program suite with refinement based on F2 with scattering factors from Int. Tab. Vol C Tables 4.2.6.8 and 6.1.1.4. The final refinement statistics include a data/parameter ratio = 13.90, goodness- of-fit on F2 = 1.02, R indices [I>4sigma(I)] R1 = 0.0506, wR2 = 0.0977, R indices(all data) R1 = 0.0951, wR2 = 0.1 141, max difference peak and hole = 0.310 and -0.379 e/A3. The atomic fractional coordinates( x 104) and equivalent isotropic displacement parameters are listed in Tables 9 and 10. U(eq) is defined as one third of the trace of the orthogonalized Uij tensor. The estimated standard deviations are shown in parentheses.

Table 9 - Atomic Coordinates (x 104) and Equivalent Isotropic Displacement Parameters (A2 103) for Compound 1 ({drug1}) Polymorph Form A ({drug1a}):

| Atom | x | v | z | U(eq) |
|---|---|---|---|---|
| Cl(1) | -561(1) | -1094(1) | 6924(1) | 43(1) |
| Cl(2) | 2856(2) | 1915(1) | 10437(1) | 62(1) |
| S(1) | 4552(1) | 2760(1) | 8088(1) | 32(1) |
| F(1) | -6506(2) | 2428(2) | 4590(2) | 44(1) |
| F(2) | -5576(3) | 3508(2) | 3277(2) | 49(1) |
| F(3) | -4749(3) | 1156(2) | 3306(2) | 54(1) |
| O(1) | 1474(3) | 4766(2) | 6746(2) | 37(1) |
| O(2) | 5691(3) | 1379(3) | 8579(2) | 49(1) |
| O(3) | 5180(3) | 3493(3) | 7231(2) | 45(1) |
| N(1) | 2988(3) | 2445(3) | 7517(2) | 30(1) |
| N(2) | 403(3) | 1635(3) | 6768(2) | 30(1) |
| N(4) | -1720(3) | 2916(3) | 5502(2) | 26(1) |
| C(1) | 1618(4) | 3510(4) | 6917(3) | 29(1) |
| C(2) | 373(4) | 2995(3) | 6476(3) | 27(1) |
| C(3) | -911(4) | 3799(4) | 5719(3) | 28(1) |
| C(5) | -891(4) | 1609(3) | 6177(3) | 27(1) |
| C(6) | -1524(4) | 513(3) | 6103(3) | 30(1) |
| C(7) | -2841(4) | 743(4) | 5388(3) | 32(1) |
| C(8) | -3613(4) | 2086(4) | 4711(3) | 29(1) |
| C(9) | -3054(4) | 3157(4) | 4776(3) | 30(1) |
| C(10) | -5083(4) | 2298(4) | 3966(3) | 36(1) |
| C(11) | 3454(4) | 4034(3) | 9144(3) | 26(1) |
| C(12) | 2725(4) | 3667(4) | 10134(3) | 36(1) |
| C(13) | 1858(5) | 4738(5) | 10897(3) | 51(1) |
| C(14) | 1684(5) | 6159(5) | 10692(4) | 56(1) |
| C(15) | 2388(4) | 6525(4) | 9708(4) | 44(1) |
| C(16) | 3282(4) | 5461(3) | 8930(3) | 33(1) |
| 0(4) | 2424(7) | 7917(6) | 9159(6) | 46(2) |
| | | | | |
| Atom | x | y | z | U(eq) |
| C(17) | 1161(9) | 9199(8) | 9661(7) | 50(2) |

(continued)

| Atom | x | y | z | U(eq) |
|---|---|---|---|---|
| O(4') | 2039(6) | 7914(5) | 9778(5) | 39(2) |
| C(17') | 2858(9) | 8429(8) | 8874(6) | 40(2) |

Table 10 - Hydrogen Coordinates (x 104) and Isotropic Displacement Parameters (A2 x 103) for Compound 1 ({drug1}) Polymorph Form A ({drug1a}):

| Atom | x | y | z | U(eq) |
|---|---|---|---|---|
| H(3A) | -1180 | 4749 | 5415 | 34 |
| H(7A) | -3248 | 18 | 5337 | 38 |
| H(9A) | -3563 | 4037 | 4338 | 36 |
| H(13A) | 1379 | 4498 | 11565 | 61 |
| H(14A) | 1090 | 6873 | 11219 | 67 |
| H(16A) | 3765 | 5705 | 8266 | 39 |
| H(1) | 3010(40) | 1620(40) | 7630(30) | 26(9) |
| H(17A) | 1226 | 10061 | 9297 | 75 |
| H(17B) | 1380 | 9161 | 10469 | 75 |
| H(17C) | 23 | 9242 | 9556 | 75 |
| H(17D) | 2567 | 9456 | 8956 | 61 |
| H(17E) | 2461 | 8300 | 8144 | 61 |
| H(17F) | 4095 | 7877 | 8916 | 61 |

CHARACTERIZATION EXAMPLE 7: Single Crystal X-Ray Diffraction for Polymorph Form B ({drug1b}) of Compound 1 ({drug1}): Suitable single crystals of polymorph Form B ({drug1b}) of Compound 1 ({drug1}) were grown from thermal gradient sublimation at 160 °C. A colorless prism with approximate dimensions of 0.40 x 0.26 x 0.13 mm was chosen for data collection and mounted on a polymer loop. Single crystal data was collected using a Bruker Platform goniometer with an Apex-II detector. The diffractometer is equipped with an incident beam monochromator using Mo-Ka radiation ($\lambda$ = 0.71073 A) and a monocap collimator. The crystals were cooled in a -100° C nitrogen flow during data collection. The data were indexed and integrated using the Apex-II suite of programs including Sainplus and SADABS. The triclinic cell parameters were determined to be: a = 1 1.6429(17) A, b = 12.0937(17) A, c = 14.859(2) A, alpha = 109.171(2) °, beta = 92.359(2) °, gamma = 106.342(2) °, volume = 1875.6(5) A3. The space group was determined to be P-1. The molecular weight was 468.23 g/mol giving a calculated density of 1.658 g/cm3, and $\mu$(Mo) = 0.52 mm" 1 for Z = 4. Data reduction led to 8320 unique data from a two-theta range = 2.94 to 54.50°. Structure solution and refinements were performed using the Shelxtl program suite with refinement based on F2 with scattering factors from Int. Tab. Vol C Tables 4.2.6.8 and 6.1.1.4. The final refinement statistics include a data/parameter ratio = 13.80, goodness-of-fit on F2 = 1.06, R indices [I>4sigma(I)] R1 = 0.0446, wR2 = 0.1012, R indices(all data) R1 = 0.0732, wR2 = 0.1 120, max difference peak and hole = 0.354 and-0.453 e/A3. The atomic fractional coordinates (x 104) and equivalent isotropic displacement parameters are listed in Tables 1 1 and 12. U(eq) is defined as one third of the trace of the orthogonalized Uij tensor. The estimated standard deviations are shownin parentheses.

Table 11 - Atomic Coordinates (x 104) and Equivalent Isotropic Displacement Parameters (A2 x 103) for Compound 1 ({drug1}) Polymorph Form B ({drug1b}):

| Atom | x | y | z | U(eq) |
|---|---|---|---|---|
| Cl(1) | 9215(1) | 2511(1) | 5201(1) | 40(1) |
| Cl(2) | 12637(1) | 398(1) | 6790(1) | 43(1) |
| Cl(21) | 9857(1) | 8175(1) | 2427(1) | 56(1) |

(continued)

| Atom | x | y | z | U(eq) |
|---|---|---|---|---|
| Cl(22) | 7769(1) | 1721(1) | 1632(1) | 46(1) |
| S(1) | 14843(1) | 2991(1) | 7570(1) | 27(1) |
| S(21) | 5885(1) | 3011(1) | 2823(1) | 29(1) |
| F(1) | 11222(2) | 5634(2) | 2620(1) | 51(1) |
| F(2) | 9386(2) | 5058(2) | 2883(1) | 47(1) |
| F(3) | 10074(2) | 3794(2) | 1859(1) | 50(1) |
| F(21) | 9708(2) | 8703(2) | -1033(2) | 50(1) |
| F(22) | 8228(2) | 9345(2) | -592(1) | 51(1) |
| F(23) | 7908(2) | 7651(2) | -1780(1) | 50(1) |
| O(1) | 15222(2) | 3594(2) | 5823(1) | 32(1) |
| O(2) | 15978(2) | 3936(2) | 7792(1) | 33(1) |
| O(3) | 14209(2) | 2833(2) | 8341(1) | 35(1) |
| O(4) | 17604(2) | 649(2) | 6058(2) | 40(1) |
| O(21) | 4965(2) | 3179(2) | 983(1) | 33(1) |
| O(22) | 4817(2) | 3289(2) | 3094(2) | 37(1) |
| O(23) | 6841(2) | 3215(2) | 3546(1) | 36(1) |
| O(24) | 2664(2) | -1058(2) | 1334(2) | 41(1) |
| N(1) | 13905(3) | 3245(2) | 6861(2) | 28(1) |
| N(2) | 12055(2) | 3283(2) | 5595(2) | 27(1) |
| N(4) | 12302(2) | 4104(2) | 4441(2) | 26(1) |
| N(21) | 6521(3) | 3877(2) | 2211(2) | 30(1) |
| N(22) | 7666(2) | 5770(2) | 1618(3) | 29(1) |
| N(24) | 7309(2) | 5998(2) | 203(2) | 26(1) |
| C(1) | 14219(3) | 3510(2) | 6049(2) | 25(1) |
| C(2) | 13250(3) | 3662(2) | 5486(2) | 25(1) |
| C(3) | 13421(3) | 4156(3) | 4779(2) | 27(1) |
| C(5) | 11482(3) | 3544(2) | 4942(2) | 26(1) |
| C(6) | 10240(3) | 3303(3) | 4650(2) | 29(1) |
| C(7) | 9881(3) | 3673(3) | 3946(2) | 32(1) |
| C(8) | 10766(3) | 4306(3) | 3503(2) | 30(1) |
| C(9) | 11950(3) | 4518(3) | 3741(2) | 29(1) |
| C(10) | 10365(3) | 4704(3) | 2724(2) | 35(1) |
| C(11) | 15046(3) | 1589(2) | 6871(2) | 26(1) |
| C(12) | 14097(3) | 475(3) | 6557(2) | 32(1) |
| C(13) | 14335(3) | -598(3) | 6047(2) | 34(1) |
| C(14) | 15493(3) | -580(3) | 5873(2) | 34(1) |
| C(15) | 16435(3) | 522(3) | 6182(2) | 30(1) |
| C(16) | 16193(3) | 1609(3) | 6679(2) | 29(1) |

(continued)

| Atom | x | y | z | U(eq) |
|---|---|---|---|---|
| C(17) | 17922(4) | -453(3) | 5647(3) | 46(1) |
| C(21) | 5955(3) | 3886(2) | 1383(2) | 28(1) |
| C(22) | 6678(3) | 4840(2) | 1042(2) | 26(1) |
| C(23) | 6447(3) | 4944(3) | 175(2) | 28(1) |
| C(25) | 8026(3) | 6471(3) | 1101(2) | 28(1) |
| C(26) | 8967(3) | 7601(3) | 1327(2) | 34(1) |
| C(27) | 9146(3) | 8181(3) | 676(2) | 36(1) |
| C(28) | 8374(3) | 7646(3) | -225(2) | 31(1) |
| C(29) | 7485(3) | 6573(3) | -461(2) | 28(1) |
| C(30) | 8560(3) | 8321(3) | -914(2) | 36(1) |
| C(31) | 5448(3) | 1474(2) | 2009(2) | 26(1) |
| C(32) | 6258(3) | 918(3) | 1535(2) | 30(1) |
| C(33) | 5848(3) | -318(3) | 975(2) | 36(1) |
| C(34) | 4655(3) | -1007(3) | 883(2) | 34(1) |
| C(35) | 3848(3) | -459(3) | 1359(2) | 30(1) |
| C(36) | 4245(3) | 794(3) | 1904(2) | 29(1) |
| C(37) | 2241(4) | -2352(3) | 810(3) | 52(1) |

Table 12 - Hydrogen Coordinates (x 104) and Isotropic Displacement Parameters (A2 x 103) for Compound 1 ({drug1}) Polymorph Form B ({drug1b}):

| Atom | x | y | z | U(eq) |
|---|---|---|---|---|
| H(1) | 13230(30) | 3160(20) | 6978(18) | 10(7) |
| H(3) | 14080(30) | 4460(30) | 4550(20) | 24(8) |
| H(7) | 9040(30) | 3560(30) | 3780(20) | 44(10) |
| H(9) | 12600(20) | 4960(20) | 3477(18) | 16(7) |
| H(13) | 13680(30) | -1300(30) | 5870(20) | 28(8) |
| H(14) | 15620(30) | -1310(30) | 5560(20) | 50(10) |
| H(16) | 16810(30) | 2340(30) | 6860(20) | 29(8) |
| H(17) | 18850(40) | -150(30) | 5690(20) | 47(10) |
| H(17A) | 17470(30) | -980(30) | 5000(30) | 47(10) |
| H(17B) | 17690(30) | -990(30) | 6000(30) | 49(10) |
| H(21) | 7250(30) | 4290(30) | 2360(30) | 50(11) |
| H(23) | 5860(30) | 4480(20) | -310(20) | 21(7) |
| H(27) | 9760(30) | 8870(30) | 810(20) | 45(10) |
| H(29) | 6950(30) | 6140(30) | -1030(20) | 36(9) |
| H(33) | 6400(30) | -650(30) | 670(20) | 43(10) |
| H(34) | 4340(30) | -1920(30) | 500(20) | 46(9) |
| H(36) | 3700(30) | 1180(30) | 2210(20) | 32(8) |

(continued)

| Atom | x | y | z | U(eq) |
|---|---|---|---|---|
| H(37) | 1360(40) | -2660(30) | 890(30) | 60(12) |
| H(37A) | 2670(30) | -2750(30) | 1070(30) | 50(11) |
| H(37B) | 2260(40) | -2520(40) | 100(30) | 75(13) |

CHARACTERIZATION EXAMPLE 8: Single Crystal X-Ray Diffraction for Polymorph Form C ({drug1c}) of Compound 1 ({drug1}) Suitable single crystals of polymorph Form C ({drug1c}) of Compound 1 ({drug1}) were grown from thermal gradient sublimation at 160 °C. A colorless triangular plate with approximate dimensions of 0.13 x 0.13 x 0.06 mm was chosen for data collection and mounted on a polymer loop. Single crystal data was collected using a Bruker Platform goniometer with an Apex-II detector. The diffractometer was equipped with an incident beam monochromator using Mo-K$\alpha$ radiation ($\lambda$ = 0.71073A) and a monocap collimator. The crystals were cooled in a -100 °C nitrogen flow during data collection. The data were indexed and integrated using the Apex-II suite of programs including Sainplus and SADABS. The triclinic cell parameters were determined to be: a = 11.816(4) A, b = 15.036(5) A, c = 21.625(8) A, alpha = 92.255(6)°, beta = 92.597(5) °, gamma = 107.947(5) °, volume = 3646(2) A3. The space group was determined to be P-1. The molecular weight was 468.23 g/mol giving a calculated density of 1.706 g/cm3, and $\mu$(Mo) = 0.53 mm" 1 for Z = 8. Data reduction led to 1 1680 unique data from a two-theta range = 3.62 to 48.48°. Structure solution and refinements were performed using the Shelxtl program suite with refinement based on F2 with scattering factors from Int. Tab. Vol C Tables 4.2.6.8 and 6.1.1.4. The final refinement statistics include a data/parameter ratio = 11.13, goodness-of-fit on F2 = 0.97, R indices [I>4sigma(I)] RI = 0.0595, wR2 = 0.1201, R indices(all data) RI = 0.1454, wR2 = 0.1546, max difference peak and hole = 0.890 and -0.357 e/A3. The atomic fractional coordinates(x 104) and equivalent isotropic displacement parameters are listed in Tables 13 and 14. U(eq) is defined as one third of the trace of the orthogonalized Uij tensor. The estimated standard deviations are shown in parentheses.

Table 13 - Atomic Coordinates (x 104) and Equivalent Isotropic Displacement Parameters (A2 103) for Compound 1 ({drug1}) Polymorph Form C ({drug1c}):

| Atom | x | y | z | U(eq) |
|---|---|---|---|---|
| Cl(1) | 6400(1) | 6726(1) | 286(1) | 44(1) |
| Cl(2) | 8884(2) | 9826(1) | 2927(1) | 60(1) |
| Cl(21) | 4766(2) | 4474(1) | 1777(1) | 64(1) |
| Cl(22) | 3672(1) | 5663(1) | -310(1) | 47(1) |
| Cl(41) | -1571(2) | 8384(1) | 4287(1) | 51(1) |
| Cl(42) | -2104(1) | 5101(1) | 2310(1) | 50(1) |
| Cl(61) | -2362(1) | 7296(1) | 2514(1) | 44(1) |
| Cl(62) | 1367(1) | 9154(1) | 5072(1) | 44(1) |
| S(1) | 6067(1) | 9674(1) | 2720(1) | 36(1) |
| S(21) | 2573(2) | 7356(1) | 33(1) | 40(1) |
| S(41) | 750(1) | 5488(1) | 2654(1) | 35(1) |
| S(61) | 2152(1) | 7311(1) | 4686(1) | 36(1) |
| F(1) | 6635(4) | 9361(3) | -1935(2) | 67(1) |
| F(2) | 7359(4) | 8227(4) | -1897(2) | 94(2) |
| F(3) | 5493(4) | 7950(3) | -2006(2) | 76(1) |
| F(21) | 5541(3) | 6625(3) | 4390(2) | 62(1) |
| F(22) | 4429(4) | 5213(3) | 4307(2) | 70(1) |
| F(23) | 6251(3) | 5541(3) | 4114(2) | 62(1) |
| F(41) | -2215(4) | 7759(3) | 6688(2) | 74(1) |

(continued)

| Atom | x | y | z | U(eq) |
|---|---|---|---|---|
| F(42) | -3901(4) | 6885(3) | 6346(2) | 69(1) |
| F(43) | -2665(3) | 6280(3) | 6762(2) | 59(1) |
| F(61) | -721(3) | 8196(3) | 192(2) | 66(1) |
| F(62) | 749(3) | 9422(3) | 416(2) | 56(1) |
| F(63) | -1022(3) | 9418(3) | 586(2) | 64(1) |
| O(1) | 6083(4) | 10736(3) | 1590(2) | 42(1) |
| O(2) | 6210(3) | 8915(3) | 3055(2) | 38(1) |
| O(3) | 4969(4) | 9858(3) | 2708(2) | 45(1) |
| O(4) | 7499(5) | 13124(3) | 3513(2) | 67(2) |
| O(21) | 3416(4) | 8404(3) | 1265(2) | 42(1) |
| O(22) | 1805(4) | 7893(3) | 142(2) | 48(1) |
| O(23) | 3604(4) | 7755(3) | -300(2) | 50(1) |
| O(24) | -1363(4) | 5143(3) | -928(2) | 44(1) |
| O(41) | 56(3) | 4553(3) | 3832(2) | 35(1) |
| O(42) | 780(4) | 6183(3) | 2224(2) | 40(1) |
| O(43) | 1826(3) | 5342(3) | 2871(2) | 41(1) |
| O(44) | 186(4) | 2227(3) | 1824(2) | 57(1) |
| O(61) | 3329(4) | 8208(3) | 3584(2) | 36(1) |
| O(62) | 2817(4) | 6711(3) | 4507(2) | 42(1) |
| O(63) | 1163(4) | 6960(3) | 5056(2) | 43(1) |
| O(64) | 6113(3) | 8915(3) | 5767(2) | 39(1) |
| N(1) | 6349(4) | 9441(3) | 2002(2) | 34(1) |
| N(2) | 6290(4) | 8632(3) | 838(2) | 30(1) |
| N(4) | 6322(4) | 9259(3) | -80(2) | 32(1) |
| N(21) | 2965(5) | 7016(4) | 698(2) | 44(1) |
| N(22) | 4007(4) | 6278(3) | 1604(2) | 32(1) |
| N(24) | 4376(4) | 6743(3) | 2608(2) | 31(1) |
| N(41) | 77(4) | 5774(3) | 3242(2) | 31(1) |
| N(42) | -936(4) | 6537(3) | 4124(2) | 30(1) |
| N(44) | -1560(4) | 6033(3) | 5037(2) | 30(1) |
| N(61) | 1602(4) | 7655(3) | 4062(2) | 31(1) |
| N(62) | 310(4) | 7796(3) | 2988(2) | 31(1) |
| N(64) | 1005(4) | 8472(3) | 2118(2) | 31(1) |
| C(1) | 6214(5) | 9972(4) | 1521(3) | 32(2) |
| C(2) | 6259(5) | 9528(4) | 907(3) | 32(2) |
| C(3) | 6282(5) | 9940(4) | 356(3) | 32(2) |
| C(5) | 6318(5) | 8484(4) | 238(3) | 27(1) |
| C(6) | 6379(5) | 7676(4) | -107(3) | 33(2) |

(continued)

| Atom | x | y | z | U(eq) |
|---|---|---|---|---|
| C(7) | 6438(5) | 7708(5) | -735(3) | 39(2) |
| C(8) | 6424(5) | 8527(4) | -1034(3) | 33(2) |
| C(9) | 6356(5) | 9295(5) | -712(3) | 37(2) |
| C(10) | 6495(6) | 8534(6) | -1718(3) | 49(2) |
| C(11) | 7193(6) | 10723(4) | 2970(3) | 37(2) |
| C(12) | 8367(6) | 10756(5) | 3083(3) | 43(2) |
| C(13) | 9183(7) | 11568(5) | 3341(3) | 57(2) |
| C(14) | 8865(7) | 12324(6) | 3481(3) | 57(2) |
| C(15) | 7713(81) | 12321(4) | 3363(3) | 50(2) |
| C(16) | 6822(6) | 11500(5) | 3092(3) | 43(2) |
| C(17) | 6329(6) | 13094(5) | 3426(3) | 53(2) |
| C(21) | 3414(5) | 7600(5) | 1228(3) | 36(2) |
| C(22) | 3792(5) | 7111(4) | 1715(3) | 29(1) |
| C(23) | 4033(5) | 7411(4) | 2331(3) | 32(2) |
| C(25) | 4359(5) | 6064(4) | 2150(3) | 31(2) |
| C(26) | 4720(5) | 5309(4) | 2339(3) | 37(2) |
| C(27) | 5029(5) | 5238(4) | 2929(3) | 41(2) |
| C(28) | 5002(5) | 5950(4) | 3385(3) | 36(2) |
| C(29) | 4684(5) | 6676(4) | 3223(3) | 33(2) |
| C(30) | 5306(6) | 5831(5) | 4046(3) | 47(2) |
| C(31) | 1724(5) | 6269(4) | -327(2) | 31(2) |
| C(32) | 2190(5) | 5557(4) | -485(3) | 32(2) |
| C(33) | 1468(5) | 4728(4) | -763(3) | 35(2) |
| C(34) | 287(6) | 4623(5) | -906(3) | 39(2) |
| C(35) | -183(5) | 5331(4) | -768(3) | 32(2) |
| C(36) | 520(5) | 6151(4) | -474(3) | 35(2) |
| C(37) | -1866(5) | 5879(5) | -836(3) | 46(2) |
| C(41) | -203(5) | 5260(5) | 3757(3) | 30(1) |
| C(42) | -807(5) | 5681(4) | 4205(3) | 28(1) |
| C(43) | -1190(5) | 5346(4) | 4760(3) | 29(1) |
| C(45) | -1403(5) | 6733(4) | 4634(3) | 28(1) |
| C(46) | -1717(5) | 7546(4) | 4813(3) | 34(2) |
| C(47) | -2170(5) | 7599(4) | 5372(3) | 35(2) |
| C(48) | -2279(5) | 6860(4) | 5776(3) | 33(2) |
| C(49) | -1989(5) | 6085(5) | 5617(3) | 37(2) |
| C(50) | -2770(6) | 6933(5) | 6392(3) | 46(2) |
| C(51) | -230(5) | 4393(4) | 2346(2) | 30(2) |
| C(52) | -1440(5) | 4251(4) | 2185(3) | 34(2) |

(continued)

| Atom | x | y | z | U(eq) |
|---|---|---|---|---|
| C(53) | -2098(6) | 3397(5) | 1914(3) | 41(2) |
| C(54) | -1611(6) | 2699(5) | 1789(3) | 45(2) |
| C(55) | -419(6) | 2850(4) | 1936(3) | 40(2) |
| C(56) | 264(5) | 3695(4) | 2224(3) | 35(2) |
| C(57) | -450(7) | 1370(5) | 1493(4) | 73(2) |
| C(61) | 2255(6) | 8011(4) | 3571(3) | 28(1) |
| C(62) | 1538(5) | 8145(4) | 3029(3) | 31(2) |
| C(63) | 1978(5) | 8563(4) | 2509(3) | 31(2) |
| C(65) | 10(5) | 8010(4) | 2430(3) | 25(1) |
| C(66) | -1125(5) | 7837(4) | 2120(3) | 28(1) |
| C(67) | -1205(5) | 8116(4) | 1546(3) | 32(2) |
| C(68) | -167(5) | 8584(4) | 1243(3) | 36(2) |
| C(69) | 919(5) | 8758(5) | 1529(3) | 37(2) |
| C(70) | -276(6) | 8904(5) | 615(3) | 45(2) |
| C(71) | 3137(5) | 8336(4) | 5069(2) | 28(1) |
| C(72) | 2789(5) | 9102(4) | 5244(3) | 30(2) |
| C(73) | 3582(5) | 9836(4) | 5586(2) | 31(2) |
| C(74) | 4710(5) | 9801(4) | 5765(2) | 32(2) |
| C(75) | 5043(5) | 9037(4) | 5603(3) | 29(1) |
| C(76) | 4259(5) | 8300(4) | 5236(2) | 29(1) |
| C(77) | 6895(5) | 9597(4) | 6204(3) | 42(2) |

Table 14 - Hydrogen Coordinates (x 104) and Isotropic Displacement Parameters (A2 x 103) for Compound 1 ({drug1}) Polymorph Form C ({drug1c}):

| Atom | x | y | z | U(eq) |
|---|---|---|---|---|
| H(1A) | 6596 | 8954 | 1925 | 41 |
| H(21A) | 2885 | 6417 | 723 | 53 |
| H(41A) | -119 | 6292 | 3227 | 38 |
| H(61A) | 840 | 7608 | 4042 | 37 |
| H(3A) | 6273 | 10558 | 288 | 38 |
| H(7A) | 6489 | 7179 | -972 | 47 |
| H(9A) | 6332 | 9839 | -915 | 44 |
| H(13A) | 9988 | 11587 | 3421 | 69 |
| H(14A) | 9441 | 12871 | 3665 | 68 |
| H(16A) | 6023 | 11489 | 3001 | 52 |
| H(17A) | 6235 | 13679 | 3596 | 80 |
| H(17B) | 5822 | 12566 | 3637 | 80 |
| H(17C) | 6096 | 13018 | 2981 | 80 |

(continued)

| Atom | x | y | z | U(eq) |
|---|---|---|---|---|
| H(23A) | 3970 | 7974 | 2520 | 39 |
| H(27A) | 5268 | 4718 | 3049 | 49 |
| H(29A) | 4668 | 7148 | 3524 | 40 |
| H(33A) | 1782 | 4231 | -856 | 42 |
| H(34A) | -212 | 4052 | -1102 | 47 |
| H(36A) | 192 | 6637 | -370 | 42 |
| H(37A) | -2714 | 5656 | -970 | 69 |
| H(37B) | -1455 | 6407 | -1078 | 69 |
| H(37C) | -1777 | 6081 | -395 | 69 |
| H(43A) | -1198 | 4764 | 4919 | 35 |
| H(47A) | -2412 | 8124 | 5492 | 42 |
| H(49A) | -2076 | 5595 | 5893 | 44 |
| H(53A) | -2921 | 3286 | 1809 | 49 |
| H(54A) | -2092 | 2115 | 1602 | 54 |
| H(56A) | 1081 | 3793 | 2337 | 42 |
| H(57A) | 87 | 996 | 1427 | 110 |
| H(57B) | -764 | 1502 | 1091 | 110 |
| H(57C) | -1112 | 1021 | 1732 | 110 |
| H(63A) | 2791 | 8857 | 2433 | 37 |
| H(67A) | -1967 | 7999 | 1338 | 39 |
| H(69A) | 1615 | 9074 | 1327 | 45 |
| H(73A) | 3359 | 10372 | 5700 | 37 |
| H(74A) | 5253 | 10312 | 6002 | 38 |
| H(76A) | 4497 | 7778 | 5103 | 35 |
| H(77A) | 7548 | 9370 | 6350 | 64 |
| H(77B) | 6449 | 9698 | 6557 | 64 |
| H(77C) | 7222 | 10188 | 6004 | 64 |

CHARACTERIZATION EXAMPLE 9: Single Crystal X-Ray Diffraction for Polymorph Form D ({drug1d}) of Compound 1 ({drug1}): Suitable single crystals of polymorph Form D ({drug1d}) of Compound 1 ({drug1}) were grown by slow evaporation of a saturated solution of Compound 1 ({drug1}) in acetonitrile. A colorless irregular block with approximate dimensions of 0.50 x 0.50 x 0.33 mm was chosen for data collection and mounted on a polymer loop. Single crystal data was collected using a Bruker Platform goniometer with an Apex-II detector. The diffractometer was equipped with an incident beam monochromator using Mo-K$\alpha$ radiation ($\lambda$ = 0.71073 A) and a monocap collimator. The crystals were cooled in a-100° C nitrogen flow during data collection. The data were indexed and integrated using the Apex-II suite of programs including Sainplus and SADABS. The triclinic cell parameters were determined to be: a = 7.223(3) A, b = 8.676(4) A, c = 14.905(6) A, alpha = 92.207(6) °, beta = 97.182(7)°, gamma = 99.385(6) °, volume = 912.6(7) A3. The space group was determined to be P-1. The molecular weight was 468.23 g/mol giving a calculated density of 1.704 g/cm3, and $\mu$(Mo) = 0.53 mm"1 for Z = 2. Data reduction led to 4449 unique data from a two-theta range = 4.76 to 56.88°. Structure solution and refinements were performed using the Shelxtl program suite with refinement based on F2 with scattering factors from Int. Tab. Vol C Tables 4.2.6.8 and 6.1.1.4. The final refinement statistics include a data/parameter ratio = 16.66, goodness-of-fit on F2 = 1.00, R indices [I>4sigma(I)] R1 = 0.0466, wR2 = 0.1221, R indices(all data) R1

= 0.0718, wR2 = 0.1362, max difference peak and hole = 0.379 and -0.394 e/A3. The atomic fractional coordinates(x 104) and equivalent isotropic displacement parameters are listed in Tables 15 and 16. U(eq) is defined as one third of the trace of the orthogonalized Uij tensor. The estimated standard deviations are shown in parentheses.

Table 15 - Atomic Coordinates (x 104) and Equivalent Isotropic Displacement Parameters (A2 x 103) for Compound 1 ({drug1}) Polymorph Form D ({drug1d}):

| Atom | x | y | z | U(eq) |
|---|---|---|---|---|
| 0(4) | 1339(3) | -2648(2) | 3615(1) | 49(1) |
| S(1) | 4949(1) | 2693(1) | 3312(1) | 36(1) |
| Cl(1) | 12928(1) | 5241(1) | 1308(1) | 43(1) |
| F(1) | 13968(2) | 1644(2) | -1576(1) | 48(1) |
| O(1) | 4162(2) | 1171(2) | 1398(1) | 41(1) |
| N(1) | 6173(3) | 2856(3) | 2440(2) | 36(1) |
| C(1) | 5682(3) | 2018(3) | 1619(2) | 32(1) |
| Cl(2) | 8842(1) | 1369(1) | 4055(1) | 48(1) |
| F(2) | 12443(2) | 3251(2) | -2282(1) | 51(1) |
| O(2) | 6042(3) | 3790(2) | 3997(1) | 46(1) |
| C(2) | 7200(3) | 2233(3) | 1034(2) | 32(1) |
| N(2) | 8877(3) | 3242(2) | 1299(1) | 32(1) |
| F(3) | 11181(2) | 816(2) | -2290(1) | 52(1) |
| O(3) | 3039(3) | 2824(2) | 2997(1) | 44(1) |
| C(3) | 7183(4) | 1454(3) | 216(2) | 39(1) |
| N(4) | 8915(3) | 1993(2) | -47(1) | 33(1) |
| | | | | |
| C(5) | 9893(3) | 3085(3) | 634(2) | 31(1) |
| C(6) | 1726(3) | 3857(3) | 493(2) | 32(1) |
| C(7) | 12457(3) | 3499(3) | -271(2) | 34(1) |
| C(8) | 11386(3) | 2355(3) | -936(2) | 33(1) |
| C(9) | 9639(4) | 1613(3) | -825(2) | 37(1) |
| C(10) | 12227(4) | 2016(3) | -1778(2) | 39(1) |
| C(11) | 4973(3) | 739(3) | 3610(2) | 32(1) |
| C(12) | 6625(3) | 176(3) | 3922(2) | 35(1) |
| C(13) | 6522(4) | -1388(3) | 4108(2) | 39(1) |
| C(14) | 4776(4) | -2387(3) | 4004(2) | 40(1) |
| C(15) | 3129(4) | -1807(3) | 3719(2) | 36(1) |
| C(16) | 3234(3) | -244(3) | 3513(2) | 34(1) |
| C(17) | 1087(5) | -4247(3) | 3840(2) | 52(1) |

Table 16 - Hydrogen Coordinates (x 104) and Isotropic Displacement Parameters (A2 x 103) for Compound 1 ({drug1}) Polymorph Form D ({dru1d}):

| Atom | x | y | z | U(eq) |
|---|---|---|---|---|
| H(1) | 7050(40) | 3210(30) | 2544(18) | 24(8) |
| H(3A) | 6187 | 704 | -101 | 47 |
| H(7A) | 13680 | 4010 | -364 | 41 |
| H(9A) | 8933 | 854 | -1269 | 44 |
| H(13A) | 7646 | -1781 | -4308 | 47 |
| H(14A) | 4714 | -3459 | 4128 | 48 |
| H(16A) | 2113 | 148 | 3306 | 41 |
| H(17A) | -266 | -4648 | 3827 | 79 |
| H(17B) | 1746 | -4324 | 4449 | 79 |
| H(17C) | 1608 | -4866 | 3401 | 79 |

CHARACTERIZATION EXAMPLE 10: Single Crystal X-Ray Diffraction for Polymorph Form TS ({drug1e}) of Compound 1 ({drug1}): Suitable single crystals for the toluene solvate of Compound 1 ({drug1}) (designated polymorph Form TS ({drug1e})) were grown by slow evaporation of a saturated solution of Compound 1 ({drug1}) in toluene. A colorless needle with approximate dimensions of 0.48 x 0.13 x 0.04 mm was chosen for data collection and mounted on a polymer loop. Single crystal data were collected using a Bruker Platform goniometer with an Apex-II detector. The diffractometer is equipped with an incident beam monochromator using Mo-Kα radiation (λ = 0.71073 A) and a monocap collimator. The crystals were cooled in a -100 °C nitrogen flow during data collection. The data were indexed and integrated using the Apex-II suite of programs including Sainplus and SADABS. The triclinic cell parameters were determined to be: a = 12.547(6) A, b = 15.165(7) A, c = 15.311(7) A, alpha = 100.594(9) °, beta = 109.609(8) °, gamma = 1 10.924(8) °, volume = 2405.8(19) A3. The space group was determined to be P-1. The molecular weight was 560.36 g/mol giving a calculated density of 1.547 g/cm3, and μ(Mo) = 0.42 mm"1 for Z = 4. Data reduction led to 10653 unique data from a two-theta range = 3.48 to 54.44°. Structure solution and refinements were performed using the Shelxtl program suite with refinement based on F2 with scattering factors from Int. Tab. Vol C Tables 4.2.6.8 and 6.1.1.4. The final refinement statistics include a data/parameter ratio = 16.31, goodness-of-fit on F2 = 1.02, R indices [I>4sigma(I)] R1 = 0.0727, wR2 = 0.1676, R indices(all data) R1 = 0.1546, wR2 = 0.2053, max difference peak and hole = 0.641 and-0.637 e/A3. The atomic fractional coordinates(x 104) and equivalent isotropic displacement parameters are listed in Tables 17 and 18. U(eq) is defined as one third of the trace of the orthogonalized Uij tensor. The estimated standard deviations are shown in parentheses.

Table 17 - Atomic Coordinates (x 104) and Equivalent Isotropic Displacement Parameters (A2 103) for Compound 1 ({drug1}) Polymorph Form TS ({drug1e}):

| Atom | x | y | z | U(eq) |
|---|---|---|---|---|
| Cl(1) | 4975(1) | 1411(1) | 2566(1) | 53(1) |
| Cl(2) | 114(1) | 2917(1) | 505(1) | 58(1) |
| Cl(21) | 1524(1) | 1282(1) | -13(1) | 50(1) |
| Cl(22) | 7874(1) | 3395(1) | 3083(1) | 58(1) |
| S(1) | 2877(1) | 4894(1) | 1388(1) | 36(1) |
| S(21) | 7216(1) | 5258(1) | 3748(1) | 34(1) |
| F(1) | 5308(3) | 2050(2) | 6851(2) | 60(1) |
| F(2) | 4357(3) | 588(2) | 5748(2) | 63(1) |
| F(3) | 6348(3) | 1455(3) | 6287(3) | 76(1) |
| F(21) | 845(3) | 1366(3) | -3764(2) | 65(1) |
| F(22) | 1629(3) | 350(2) | -3557(2) | 66(1) |

(continued)

| Atom | x | y | z | U(eq) |
|---|---|---|---|---|
| F(23) | 2696(3) | 1749(2) | -3651(2) | 62(1) |
| O(1) | 3274(3) | 5092(2) | 3429(2) | 40(1) |
| O(2) | 2613(3) | 4373(3) | 407(2) | 47(1) |
| O(3) | 3920(3) | 5885(2) | 1903(3) | 43(1) |
| O(4) | 816(3) | 7018(2) | 2121(3) | 48(1) |
| O(21) | 7020(3) | 5485(2) | 1840(2) | 39(1) |
| O(22) | 6914(3) | 4706(2) | 4361(2) | 44(1) |
| O(23) | 7210(3) | 6215(2) | 3903(2) | 44(1) |
| O(24) | 11876(3) | 7562(3) | 4794(3) | 55(1) |
| N(1) | 3126(3) | 4153(3) | 2015(3) | 32(1) |
| N(2) | 4142(3) | 3090(3) | 3025(3) | 29(1) |
| N(4) | 4399(3) | 3041(3) | 4535(3) | 29(1) |
| N(21) | 6163(3) | 4503(3) | 2618(3) | 31(1) |
| N(22) | 4119(3) | 3178(3) | 791(3) | 30(1) |
| N(24) | 4031(3) | 3083(3) | -711(3) | 29(1) |
| C(1) | 3403(4) | 4405(3) | 3013(3) | 32(1) |
| C(2) | 3831(4) | 3765(3) | 3480(3) | 26(1) |
| C(3) | 3987(4) | 3756(3) | 4406(3) | 32(1) |
| C(5) | 4478(4) | 2654(3) | 3673(3) | 31(1) |
| C(6) | 4878(4) | 1896(3) | 3625(4) | 35(1) |
| C(7) | 5145(4) | 1551(3) | 4389(4) | 37(1) |
| C(8) | 5029(4) | 1963(3) | 5241(4) | 36(1) |
| C(9) | 4669(4) | 2709(3) | 5319(3) | 33(1) |
| C(10) | 5267(5) | 1535(4) | 6039(4) | 44(1) |
| C(11) | 1509(4) | 4968(3) | 1392(3) | 33(1) |
| C(12) | 332(4) | 4134(3) | 990(4) | 38(1) |
| C(13) | -702(4) | 4273(4) | 971(4) | 43(1) |
| C(14) | -582(4) | 5230(4) | 1334(4) | 42(1) |
| C(15) | 579(4) | 6052(4) | 1731(4) | 36(1) |
| C(16) | 1633(4) | 5922(3) | 1773(4) | 36(1) |
| C(17) | -250(5) | 7204(4) | 2029(4) | 51(1) |
| C(21) | 6202(4) | 4726(3) | 1797(3) | 29(1) |
| C(22) | 5169(4) | 3956(3) | 854(3) | 29(1) |
| C(23) | 5127(4) | 3920(3) | -56(3) | 31(1) |
| C(25) | 3447(4) | 2664(3) | -157(3) | 28(1) |
| C(26) | 2271(4) | 1776(3) | -689(3) | 33(1) |
| C(27) | 1791(4) | 1348(3) | -1674(3) | 34(1) |
| C(28) | 2456(4) | 1803(3) | -2195(3) | 31(1) |

(continued)

| Atom | x | y | z | U(eq) |
|---|---|---|---|---|
| C(29) | 3547(4) | 2656(3) | -1715(3) | 32(1) |
| C(30) | 1912(5) | 1324(4) | -3276(4) | 42(1) |
| C(31) | 8710(4) | 5430(3) | 3815(3) | 31(1) |
| C(32) | 8999(4) | 4644(4) | 3571(4) | 39(1) |
| C(33) | 10224(5) | 4854(4) | 3700(4) | 45(1) |
| C(34) | 11158(5) | 5834(4) | 4098(4) | 45(1) |
| C(34) | 10883(4) | 6621(4) | 4372(4) | 42(1) |
| C(36) | 9649(4) | 6417(4) | 4213(3) | 35(1) |
| C(37) | 11653(5) | 8372(4) | 5147(6) | 77(2) |
| C(40) | 582(7) | 2435(6) | 3159(6) | 104(3) |
| C(41) | 1006(5) | 1600(5) | 3079(5) | 72(2) |
| C(42) | 1132(6) | 1203(5) | 2253(5) | 66(2) |
| C(43) | 1515(6) | 476(6) | 2168(6) | 76(2) |
| C(44) | 1832(6) | 105(5) | 2992(8) | 104(3) |
| C(45) | 1677(6) | 548(6) | 3814(6) | 78(2) |
| C(46) | 1282(6) | 1266(6) | 3819(5) | 80(2) |
| C(50) | 6001(8) | 1857(6) | -648(9) | 144(5) |
| C(51) | 4910(12) | 1078(9) | -849(11) | 159(5) |
| C(52) | 4059(10) | 307(7) | -1675(6) | 98(3) |
| C(53) | 2955(10) | -523(8) | -1811(8) | 124(3) |
| C(54) | 2697(11) | -556(9) | -1003(8) | 125(4) |
| C(55) | 3450(17) | 147(14) | -140(10) | 181(7) |
| C(56) | 4560(12) | 994(9) | -24(8) | 116(4) |

Table 18 - Hydrogen Coordinates (x 104) and Isotropic Displacement Parameters (A2 x 103) for Compound 1 ({drug1}) Polymorph Form TS ({drug1e}):

| Atom | x | y | z | U(eq) |
|---|---|---|---|---|
| H(1A) | 3082 | 3582 | 1708 | 39 |
| H(21A) | 5550 | 3933 | 2537 | 37 |
| H(3A) | 3841 | 4158 | 4862 | 39 |
| H(7A) | 5409 | 1037 | 4353 | 45 |
| H(9A) | 4608 | 2992 | 5897 | 40 |
| H(13A) | -1508 | 3706 | 706 | 52 |
| H(14A) | -1306 | 5314 | 1308 | 50 |
| H(16A) | 2444 | 6489 | 2065 | 44 |
| H(17A) | 37 | 7930 | 2289 | 76 |
| H(17B) | -658 | 6875 | 2403 | 76 |
| H(17C) | -858 | 6935 | 1331 | 76 |

(continued)

| Atom | x | y | z | U(eq) |
|---|---|---|---|---|
| H(23A) | 5731 | 4379 | -201 | 37 |
| H(27A) | 1015 | 747 | -2022 | 41 |
| H(29A) | 3977 | 2960 | -2066 | 38 |
| H(33A) | 10419 | 4320 | 3513 | 54 |
| H(34A) | 11995 | 5973 | 4185 | 54 |
| H(36A) | 9448 | 6954 | 4376 | 42 |
| H(37A) | 12456 | 8992 | 5482 | 115 |
| H(37B) | 11291 | 8243 | 5613 | 115 |
| H(37C) | 11059 | 8445 | 4591 | 115 |
| H(40A) | 39 | 2329 | 3502 | 156 |
| H(40B) | 106 | 2417 | 2495 | 156 |
| H(40C) | 1329 | 3091 | 3529 | 156 |
| H(42A) | 942 | 1450 | 1723 | 79 |
| H(43A) | 1577 | 210 | 1585 | 92 |
| H(44A) | 2119 | -395 | 2977 | 125 |
| H(45A) | 1856 | 335 | 4370 | 94 |
| H(46A) | 1197 | 1545 | 4386 | 96 |
| H(50A) | 5833 | 2242 | -1081 | 215 |
| H(50B) | 6582 | 1608 | -760 | 215 |
| H(50C) | 6388 | 2292 | 43 | 215 |
| H(52A) | 4208 | 312 | -2243 | 117 |
| H(53A) | 2410 | -1040 | -2438 | 149 |
| H(54A) | 1958 | -1102 | -1079 | 150 |
| H(55A) | 3271 | 104 | 410 | 217 |
| H(56A) | 5082 | 1514 | 603 | 140 |

CHARACTERIZATION EXAMPLE 11: Differential Scanning Calorimetry Experiments: The DSC curve for pure polymorph Form A ({drug1a}) of Compound 1 ({drug1}) was observed to exhibit a sharp endotherm with an onset temperature at 212 °C (signal maximum at 212.6 °C) immediately followed or overlapped by an exotherm with a signal maximum at 213 °C. These endothermic-exothermic events were followed by a main melting endotherm at an onset temperature of 218 °C (signal maximum at 219 °C, end point 225 °C, heat of transition 63 J/g). The DSC curve for polymorph Form B ({drug1b}) of Compound 1 ({drug1}) was observed to exhibit a minor endotherm with an onset temperature of 205 °C (signal maximum at 208 °C, heat of transition 4 J/g) and a sharp major endotherm with an onset temperature at 217.9 °C (signal maximum at 218 °C, heat of transition 56 J/g). The DSC curve for polymorph Form D ({drug1d}) of Compound 1 ({drug1}) was observed to exhibit a minor endotherm at an onset temperature of 21 1 °C (maximum at 212 °C, heat of transition 10 J/g) and a sharp major endotherm at an onset temperature of 218 °C (maximum at 219 °C, heat of transition 62 J/g). The DSC curve for polymorph Form TS ({drug1e}) of Compound 1 ({drug1}) (toluene solvate) was observed to exhibit four endotherms. Endotherm 1 was a broad endotherm with an onset temperature of 1 18 °C (signal maximum at 137 °C, heat of transition 74 J/g). Endotherm 2 had an onset temperature at 200 °C (signal maximum at 202 °C, heat of transition 6 J/g). Endotherm 3 had an onset temperature at 207 °C (signal maximum at 208 °C, heat of transition 3 J/g). Endotherm 4 had an onset temperature at 216 °C (signal maximum at 217 °C, heat of transition 42 J/g).

The DSC curve of mixtures of polymorph Forms A and B of Compound 1 ({drug1}) prepared from polymorph

Form TS ({drug1e}) according to Preparation Example 2 were observed to exhibit a minor endotherm with an onset temperature at 208 °C (signal maximum at 211 °C, heat of transition 4.6 J/g) and a sharp major endotherm with an onset temperature at 218 °C (signal maximum at 219 °C, heat of transition 58 J/g).

CHARACTERIZATION EXAMPLE 12 - Relative Stability Experiments: The relative stability of various crystal forms of Compound 1 ({drug1}) were subjected to non-competitive and competitive interconversion experiments. For the non-competitive experiments, only a single starting crystal form was used to study the potential conversion to another more stabb form. For the competitive experiments, two or more crystal forms were mixed together and studied for the potential conversion to a more stable form. The experimental conditions are described below and summarized in Table 19. In Example 12a, Form A ({drug1a}) of Compound 1 ({drug1}) (0.4 g) prepared according to Preparation Example 5c was refluxed in deionized water (4 mL) at about 95 °C for 3 hours. The slurry was cooled to 25-30 °C, filtered, suction dried for 1 hour and dried in a vacuum oven at 70 °C and 8 kPa absolute pressure for 12 hours. Analysis by pXRD, DSC, TGA and 1H-NMR of the resulting material indicated that the crystal form remained unchanged, i.e. Form A ({drug1a}). In Example 12b, Form B ({drug1b}) of Compound 1 ({drug1}) (0.4 g) prepared according to Preparation Example 5f was refluxed in deionized water (4 mL) at about 95 °C for 3 hours. The slurry was cooled to 25-30 °C, filtered, suction dried for 1 hour and dried in a vacuum oven at 70 °C and 8 kPa absolute pressure for 12 hours. Analysis by pXRD, DSC, TGA and 1 H-NMR of the resulting material indicated Form A ({drug1a}). In Example 12c, Form D ({drug1d}) of Compound 1 ({drug1}) (0.4 g) prepared according to Preparation Example 5g was refluxed in deionized water (4 mL) at about 95 °C for 3 hours. The slurry was cooled to 25-30 °C, filtered, suction dried for 1 hour and dried in a vacuum oven at 70 °C and 8 kPa absolute pressure for 12 hours. Analysis by pXRD, DSC, TGA and 1H-NMR of the resulting material indicated Form A ({drug1a}). In Example 12d, Form TS ({drug1e}) of Compound 1 ({drug1}) (1 g) prepared according to Preparation Example 1 was refluxed in deionized water (10 mL) at about 95 °C for 3 hours. The slurry was cooled to 25-30 °C, filtered, suction dried for 1 hour and dried in a vacuum oven at 65 °C and 8 kPa absolute pressure for 12 hours. Analysis by pXRD, DSC, TGA and 1H-NMR of the resulting material indicated Form A ({drug1a}). In Example 12e, Form A ({drug1a}) (0.6 g) and Form B ({drug1b}) (0.6 g) of Compound 1 ({drug1}) prepared according to Preparation Examples 5c and 5f, respectively, were blended as solids and refluxed in deionized water (12 mL) at about 95 °C for 3 hours. The slurry was cooled to 25-30 °C, filtered, suction dried for 1 hour and dried in a vacuum oven at 65 °C and 8 kPa absolute pressure for 12 hours. Analysis by pXRD, DSC, TGA and 1H-NMR of the resulting material indicated Form A ({drug1a}). In Example 12f, Form B ({drug1b}) (0.6 g) and Form D ({drug1d}) (0.6 g) of Compound 1 ({drug1}) prepared according to Preparation Examples 5f and 5g, respectively, were blended as solids and refluxed in deionized water (12 mL) at about 95 °C for 3 hours. The slurry was cooled to 25-30 °C, filtered, suction dried for 1 hour and dried in a vacuum oven at 65 °C and 8 kPa absolute pressure for 12 hours. Analysis by pXRD, DSC and 1H-NMR of the resulting material indicated Form A ({drug1a}). In Example 12g, Form A ({drug1a}) (0.6 g) and Form D ({drug1d}) (0.6 g) of Compound 1 ({drug1}) prepared according to Preparation Examples 5c and 5g, respectively, were blended as solids and refluxed in deionized water (12 mL) at about 95 °C for 3 hours. The slurry was cooled to 25-30 °C, filtered, suction dried for 1 hour and dried in a vacuum oven at 65 °C and 8 kPa absolute pressure for 12 hours. Analysis by pXRD, DSC and 1H-NMR of the resulting material indicated Form A ({drug1a}). In Example 12h, Form A ({drug1a}) (0.25 g), Form B ({drug1b}) (0.25 g), Form D ({drug1d}) (0.25 g) and Form TS ({drug1e}) (0.25 g) of Compound 1 ({drug1}) prepared according to Preparation Examples 5c, 5f, 5g, and 1, respectively, were blended as solids and refluxed in deionized water (10 mL) at about 95 °C for 3 hours. The slurry was cooled to 25-30 °C, filtered, suction dried for 1 hour and dried in a vacuum oven at 65 °C and 8 kPa absolute pressure for 12 hours. Analysis by pXRD, DSC and 1H-NMR of the resulting material indicated Form A ({drug1a}). In Example 12i, Form A ({drug1a}) (0.25 g), Form B ({drug1b}) (0.25 g), Form D ({drug1d}) (0.25 g) and mixed Forms A and B (0.25 g) of Compound 1 ({drug1}) prepared according to Preparation Examples 5c, 5f, 5g and 2, respectively, were blended as solids and refluxed in deionized water (10 mL) at about 95 °C for 3 hours. The slurry was cooled to 25-30 °C, filtered, suction dried for 1 hour and dried in a vacuum oven at 65 °C and 8 kPa absolute pressure for 12 hours. Analysis by pXRD, DSC and 1H-NMR of the resulting material indicated Form A ({drug1a}). In Example 12j, Form A ({drug1a}) (0.25 g), Form B ({drug1b}) (0.25 g), Form D ({drug1d}) (0.25 g) and mixed Forms A and B (0.25 g) of Compound 1 ({drug1}) prepared according to Preparation Examples 5c, 5f, 5g and 2, respectively, were blended as solids and heated in methanol (10 mL) at about 55 °C for 3 hours. The slurry was cooled to 25-30 °C, filtered, suction dried for 1 hour and dried in a vacuum oven at 55 °C and 1.3 kPa absolute pressure for 12 hours. Analysis by pXRD, DSC and 1H-NMR of the resulting material indicated Form A ({drug1a}). In Example 12k, Form A ({drug1a}) (0.9 g), Form B ({drug1b}) (0.9 g), Form D ({drug1d}) (0.9 g) of Compound 1 ({drug1}) prepared according to Preparation Examples 5c, 5f, and 5g, respectively, were blended as solids and heated in deionized water (27 mL) at about 55 °C for 168 hours. The slurry was cooled to 25-30 °C, filtered, suction dried for 1 hour and dried in a vacuum oven at 65 °C and 8 kPa absolute pressure for 12 hours. Analysis by pXRD, DSC and 1H-NMR of the resulting material indicated Form A ({drug1a}). In Example 121, the mixed Forms A and B (2.0 g) of Compound 1 ({drug1}) prepared according to Preparation Example 2 was added to a 100 mL three-neck round-bottom flask equipped with magnetic stirrer and temperature probe. Deionized water (40 mL) was added and the resulting slurry was stirred at 25 °C for about 168 hours. The slurry filtered, suction dried for 1 hour and dried in a vacuum oven at 65 °C and 8 kPa absolute pressure for 12 hours. Analysis by

pXRD, DSC and 1H-NMR of the resulting material indicated Form A ({drug1a}).

Table 19 -Relative Stability Experiments for Various Crystal Forms of Compound 1 ({drug1})

| Example | Starting Crystal Form | Solvent | Temperature (°C); time (h) | Obtained Crystal Form |
|---|---|---|---|---|
| 12a | A | water | 95; 3 | A |
| 12b | B | water | 95; 3 | A |
| 12c | D | water | 95; 3 | A |
| 12d | TS | water | 95; 3 | A |
| 12e | A, B | water | 95; 3 | A |
| 12f | B, D | water | 95; 3 | A |
| 12g | A, D | water | 95; 3 | A |
| 12h | A, B, D, TS | water | 95; 3 | A |
| 12i | A, B, D, A+B | water | 95; 3 | A |
| 12j | A, B, D, A+B | methanol | 55; 3 | A |
| 12k | A, B, D | water | 55; 168 | A |
| 121 | A+B | water | 25; 168 | A |

CHARACTERIZATION EXAMPLE 13 - Stability Experiment for Polymorph Form A ({drug1a}) of Compound 1 ({drug1}): The physical stability of Form A ({drug1a}) of Compound 1 ({drug1}) was determined as follows. Compound 1 ({drug1}) prepared according to Preparation Example 3 was analyzed by pXRD, DSC, HPLC and 1H-NMR and found to be of pure crystal Form A ({drug1a}) of 99.9 % purity (by HPLC peak area at 230 nm detection wavelength). An aliquot of the sample (3.0 g) was placed in a primary polyethylene bag, the primary bag was flushed with nitrogen gas and sealed. The primary polyethylene bag was then placed in a secondary polyethylene bag which was again flushed with nitrogen gas and a silica gel sachet was placed between the inner and the outer bag. The double bagged material was then placed in a triple laminated aluminum pouch and placed in a stability chamber at 40 °C for 30 days. Analysis by HPLC and 1H-NMR of the resulting material indicated pure Form A ({drug1a}) of Compound 1 ({drug1}) of 99.9 % purity (by HPLC peak area at 230 nm). Analysis by pXRD and DSC indicated pure polymorphForm A ({drug1a}). The results confirm both chemical stability of Compound 1 ({drug1}) as well as the stability of polymorph Form A ({drug1a}) under the conditions studied. CHARACTERIZATION EXAMPLE 14 - Single Crystal X-Ray Diffraction for Polymorph Form C ({drug1c}) of Compound 1 ({drug1}): Suitable single crystals for polymorph Form C ({drug1c}) of Compound 1 ({drug1}) were grown from thermal gradient sublimation at 250 °C. A colorless irregular plate with approximate dimensions -0.320 x 0.230 x 0.060mm was chosen for data collection and mounted on a polymer loop. Single crystal data were collected using a Bruker Platform goniometer with an Apex-II detector. The diffractometer is equipped with an incident beam monochromator using MoKa radiation ($\lambda$ = 0.71073A) and a monocap collimator. The crystals were run at room temperature (23 °C). The data were indexed and integrated using the Apex-II suite of programs including Sainplus and SADABS. The triclinic cell parameters were determined to be: a = 14.835(7) A, b = 15.216(8) A, c = 18.790(10) A, alpha = 90.306(7) °, beta = 93.619(7) °, gamma = 1 13.045(7) °, volume = 3893(3) A3. The space group was determined to be P-1. The molecular weight was 468.23 giving a calculated density of 1.598g/cm3, and $\mu$(Mo) = 0.50mm"1 for Z = 8. Data reduction led to 12368 unique data from a two-theta range = 2.18 to 48.66°. Structure solution and refinements were performed using the Shelxtl program suite with refinement based on F2 with scattering factors from Int. Tab. Vol C Tables 4.2.6.8 and 6.1.1.4. The final refinement statistics include a data/parameter ratio = 11.78, goodness-of-fit on F2 = 1.29, R indices [I>4sigma(I)] R1 = 0.1124, wR2 = 0.2544, R indices(all data) R1 = 0.2440, wR2 = 0.2969, max difference peak and hole = 0.656 and-0.435 e/A3. The asymmetric unit contains four molecules. The form undergoes a crystallographic phase change when the crystals were cooled. The same crystallite was cooled to -100 °C and the resulting unit cell parameters were triclinic, P-1, a = 11.816(4) A, b = 15.036(5) A, c = 21.625(8) A, alpha = 92.255(6) °, beta = 92.597(5) °, gamma = 107.947(5) °, Vol = 3646(2) A3, Z = 8. The atomic fractional coordinates(x 104) and equivalent isotropic displacement parameters are listed and U(eq) is defined as one third cf the trace of the orthogonalized Uij tensor. The estimated standard deviations are shown in parentheses.

Table 20 - Atomic Coordinates (x 104) and Equivalent Isotropic Displacement Parameters (A2 x 103) for Compound 1 ({drug1}) Polymorph Form C ({drug1c}) at Room Temperature:

| Atom | x | y | z | U(eq) |
|---|---|---|---|---|
| Cl(1) | 4670(3) | 13564(3) | 3673(3) | 108(1) |
| S(1) | 1417(2) | 89900(2) | 3990(2) | 65(1) |
| F(1) | 8439(7) | 14244(9) | 4765(8) | 181(6) |
| O(1) | 3384(5) | 8914(6) | 4286(4) | 63(2) |
| N(1) | 2497(6) | 9779(6) | 3957(5) | 63(3) |
| C(1) | 3379(8) | 9633(10) | 4162(6) | 59(3) |
| Cl(2) | 1838(3) | 9382(3) | 2330(2) | 107(1) |
| P(2) | 8467(7) | 14127(8) | 3653(8) | 171(5) |
| O(2) | 1334(6) | 8480(6) | 4678(5) | 79(3) |
| N(2) | 4144(6) | 11407(7) | 3965(5) | 53(2) |
| C(2) | 4247(7) | 10565(8) | 4112(5) | 50(3) |
| F(3) | 8678(5) | 13101(7) | 4328(6) | 141(4) |
| O(3) | 740(5) | 9310(5) | 3776(5) | 81(3) |
| C(3) | 5184(8) | 10643(8) | 4262(6) | 56(3) |
| O(4) | 690(7) | 5473(7) | 3345(6) | 100(3) |
| N(4) | 5739(6) | 11615(7) | 4166(5) | 55(2) |
| C(5) | 5081(8) | 12039(8) | 4010(6) | 52(3) |
| C(6) | 5483(9) | 13038(9) | 3902(7) | 68(3) |
| C(7) | 6491(9) | 13545(9) | 3980(6) | 68(3) |
| C(8) | 7099(8) | 13062(10) | 4151(7) | 66(3) |
| C(9) | 6737(8) | 12148(9) | 4241(6) | 66(3) |
| C(10) | 8165(11) | 13633(14) | 4262(13) | 116(6) |
| C(11) | 1374(8) | 8024(9) | 3354(7) | 60(3) |
| C(12) | 1529(8) | 8254(9) | 2653(8) | 71(4) |
| C(13) | 1416(10) | 7550(13) | 2146(8) | 87(4) |
| C(14) | 1127(9) | 6643(13) | 2348(9) | 94(5) |
| C(15) | 987(8) | 6381(10) | 3064(8) | 71(4) |
| C(16) | 1098(7) | 7116(9) | 3557(7) | 65(4) |
| C(17) | 429(12) | 4715(11) | 2852(10) | 142(7) |
| Cl(21) | -386(3) | 768(3) | 557(2) | 113(1) |
| S(21) | 3458(3) | 4973(3) | 1524(2) | 84(1) |
| F(21) | -3470(7) | 1165(10) | -889(6) | 194(6) |
| O(21) | 1767(6) | 5507(7) | 1015(5) | 91(3) |
| N(21) | 2265(7) | 4326(8) | 1364(6) | 84(3) |
| C(21) | 1586(9) | 4665(12) | 1078(7) | 69(4) |
| Cl(22) | 2787(3) | 4447(3) | 3157(2) | 114(1) |
| F(22) | -3951(8) | 871(11) | 145(8) | 209(7) |

(continued)

| Atom | x | y | z | U(eq) |
|---|---|---|---|---|
| O(22) | 3845(6) | 4303(6) | 1803(5) | 98(3) |
| N(22) | 514(8) | 2977(8) | 837(5) | 68(3) |
| C(22) | 634(9) | 3935(9) | 860(6) | 63(3) |
| F(23) | -3741(8) | 2247(9) | -295(7) | 177(5) |
| O(23) | 3798(7) | 5471(6) | 903(5) | 109(4) |
| C(23) | -188(11) | 4043(10) | 647(7) | 73(4) |
| O(24) | 4400(8) | 8393(8) | 2211(6) | 110(3) |
| N(24) | -892(8) | 3151(8) | 464(5) | 72(3) |
| C(25) | -401(9) | 2512(10) | 583(6) | 65(3) |
| C(26) | -967(11) | 1527(10) | 415(7) | 73(4) |
| C(27) | -1900(11) | 1273(11) | 160(7) | 91(5) |
| C(28) | -2371(11) | 1913(12) | 29(7) | 79(4) |
| C(29) | -1858(10) | 2823(12) | 186(7) | 76(4) |
| C(30) | -3393(14) | 1514(19) | -229(11) | 134(8) |
| C(31) | 3518(9) | 5823(10) | 2194(8) | 74(4) |
| C(32) | 3231(9) | 5579(9) | 2877(8) | 75(4) |
| C(33) | 3283(9) | 6314(12) | 3353(8) | 89(5) |
| C(34) | 3658(9) | 7281(12) | 3122(9) | 85(5) |
| C(35) | 3979(10) | 7468(12) | 2464(10) | 86(4) |
| C(36) | 3868(9) | 6762(11) | 1969(8) | 84(4) |
| C(37) | 4462(11) | 9140(11) | 2628(9) | 117(6) |
| Cl(41) | 12222(2) | 12142(3) | 2485(2) | 92(1) |
| S(41) | 13696(2) | 11329(3) | 5916(2) | 72(1) |
| F(41) | 8722(7) | 12391(8) | 2197(6) | 141(4) |
| O(41) | 11559(6) | 10827(6) | 5869(4) | 69(2) |
| N(41) | 12934(6) | 11401(6) | 5260(4) | 61(3) |
| C(41) | 11946(8) | 11132(8) | 5315(7) | 54(3) |
| Cl(42) | 14434(3) | 13607(3) | 5610(2) | 105(1) |
| F(42) | 8192(7) | 10930(8) | 2016(6) | 160(4) |
| 0(42) | 13290(6) | 10410(6) | 6221(5) | 83(3) |
| N(42) | 11841(6) | 11547(6) | 4061(5) | 56(3) |
| C(42) | 11402(8) | 11256(7) | 4692(6) | 44(3) |
| F(43) | 7846(7) | 11502(9) | 2938(5) | 149(4) |
| O(43) | 14622(6) | 11639(7) | 5610(4) | 94(3) |
| C(43) | 10451(8) | 11113(7) | 4625(6) | 55(3) |
| O(44) | 13303(7) | 12294(8) | 8454(5) | 92(3) |
| N(44) | 10263(6) | 11316(6) | 3941(5) | 58(3) |
| C(45) | 11128(8) | 11594(8) | 3586(7) | 53(3) |

(continued)

| Atom | x | y | z | U(eq) |
|---|---|---|---|---|
| C(46) | 11154(8) | 11825(8) | 2881(7) | 57(3) |
| C(47) | 10330(10) | 11825(9) | 2555(7) | 84(4) |
| C(48) | 9429(9) | 11571(9) | 2898(7) | 70(4) |
| C(49) | 9411(8) | 11318(8) | 3592(7) | 64(3) |
| C(50) | 8555(12) | 11585(14) | 2526(10) | 99(5) |
| C(51) | 13735(7) | 12186(9) | 6559(7) | 56(3) |
| C(52) | 14059(8) | 13152(10) | 6413(7) | 70(4) |
| C(53) | 14149(9) | 13814(9) | 6983(8) | 84(4) |
| C(54) | 13868(9) | 13451(11) | 7640(8) | 86(5) |
| C(55) | 13543(9) | 12513(12) | 7787(8) | 77(4) |
| C(56) | 13458(8) | 11865(9) | 7232(7) | 69(4) |
| C(57) | 12973(14) | 11357(13) | 8624(9) | 135(7) |
| Cl(61) | 2116(3) | 798(3) | 973(2) | 107(1) |
| S(61) | 1366(3) | 4063(3) | -1109(2) | 73(1) |
| F(61) | 5652(9) | 1888(11) | 2485(6) | 182(6) |
| O(61) | 3563(6) | 4694(6) | -937(5) | 75(3) |
| N(61) | 2059(7) | 3768(7) | -523(5) | 64(3) |
| C(61) | 3074(10) | 4077(10) | -558(7) | 68(4) |
| Cl(62) | 619(3) | 1748(3) | -1210(2) | 102(1) |
| F(62) | 6661(11) | 2798(12) | 1845(12) | 288(11) |
| O(62) | 1807(7) | 5075(6) | -1209(4) | 85(3) |
| N(62) | 2901(7) | 2719(8) | 234(5) | 62(3) |
| C(62) | 3502(10) | 3547(8) | -65(6) | 55(3) |
| F(63) | 6029(13) | 1417(15) | 1633(7) | 249(10) |
| O(63) | 414(6) | 3643(7) | -838(5) | 98(3) |
| C(63) | 4436(9) | 3767(9) | 128(7) | 59(3) |
| O(64) | 1969(7) | 4070(6) | -3699(5) | 90(3) |
| N(64) | 4456(7) | 3084(8) | 586(5) | 66(3) |
| C(65) | 3485(11) | 2471(10) | 625(7) | 69(4) |
| C(66) | 3312(10) | 1619(9) | 1020(7) | 74(4) |
| C(67) | 4065(12) | 1504(11) | 141((7) | 83(4) |
| C(68) | 5007(12) | 2198(13) | 1416(8) | 88(4) |
| C(69) | 5229(10) | 2981(11) | 995(8) | 81(4) |
| C(70) | 5785(14) | 2080(20) | 1862(13) | 127(7) |
| C(71) | 1361(8) | 3412(10) | -1888(7) | 62(3) |
| C(72) | 1026(8) | 2440(9) | -1937(7) | 66(3) |
| C(73) | 977(9) | 1965(10) | -3579(8) | 77(4) |
| C(74) | 1292(8) | 2507(10) | -3180(8) | 69(4) |

(continued)

| Atom | x | y | z | U(eq) |
|---|---|---|---|---|
| C(75) | 1620(9) | 3487(10) | -3138(7) | 67(4) |
| C(76) | 1667(8) | 3952(9) | -2495(8) | 69(4) |
| C(77) | 1778(12) | 3604(11) | -4407(7) | 116(6) |

Table 21 - Hydrogen Coordinates (x 104) and Isotropic Displacement Parameters (A2 x 103) for Compound 1 ({drug1}) Polymorph Form C ({drug1c}) at Room Temperature:

| Atom | x | y | z | U(eq) |
|---|---|---|---|---|
| H(1A) | 2536 | 10329 | 3817 | 75 |
| H(3A) | 5404 | 10169 | 4395 | 67 |
| H(7A) | 6759 | 14202 | 3919 | 82 |
| H(9A) | 7160 | 11845 | 4358 | 79 |
| H(13A) | 1538 | 7703 | 1673 | 105 |
| H(14A) | 1014 | 6166 | 2001 | 113 |
| H(16A) | 980 | 6973 | 4031 | 78 |
| H(17A) | 193 | 4123 | 3096 | 213 |
| H(17B) | 991 | 4765 | 2603 | 213 |
| H(17C) | -79 | 4732 | 2517 | 213 |
| H(21A) | 2053 | 3732 | 1467 | 101 |
| H(23A) | -272 | 4617 | 627 | 88 |
| H(27A) | -2268 | 627 | 61 | 110 |
| H(29A) | -2151 | 3258 | 109 | 91 |
| H(33A) | 3077 | 6176 | 3812 | 107 |
| H(34A) | 3678 | 7775 | 3425 | 102 |
| H(36A) | 4020 | 6904 | 1500 | 101 |
| H(37A) | 4909 | 9722 | 2440 | 175 |
| H(37B) | 4697 | 9070 | 3103 | 175 |
| H(37C) | 3825 | 9162 | 2638 | 175 |
| H(41A) | 13172 | 11620 | 4862 | 73 |
| H(43A) | 10006 | 10913 | 4977 | 66 |
| H(47A) | 10339 | 11997 | 2080 | 100 |
| H(49A) | 8838 | 11150 | 3829 | 76 |
| H(53A) | 14389 | 14468 | 6912 | 101 |
| H(54A) | 13903 | 13879 | 8007 | 103 |
| H(56A) | 13213 | 11214 | 7315 | 83 |
| H(57A) | 12855 | 11299 | 9122 | 203 |
| H(57B) | 13458 | 11109 | 8527 | 203 |
| H(57C) | 12373 | 11002 | 8344 | 203 |
| H(61A) | 1786 | 3408 | -181 | 77 |

(continued)

| Atom | x | y | z | U(eq) |
|---|---|---|---|---|
| H(63A) | 4972 | 4282 | -18 | 70 |
| H(67A) | 3943 | 961 | 1682 | 100 |
| H(69A) | 5871 | 3427 | 982 | 98 |
| H(73A) | 741 | 1302 | -2608 | 93 |
| H(74A) | 1280 | 2202 | -3613 | 83 |
| H(76A) | 1899 | 4615 | -2466 | 83 |
| H(77A) | 1957 | 4081 | -4763 | 173 |
| H(77B) | 1093 | 3203 | -4482 | 173 |
| H(77C) | 2159 | 3223 | -4439 | 173 |

CHARACTERIZATION EXAMPLE 15

X-Ray Powder Diffraction Pattern for Compound 1 ({drug1}) Polymorph Form C ({drug1c}) Powder X-ray diffraction was used to characterize polymorph Form C ({drug1c}) of Compound 1 ({drug1}). Data were obtained with a Philips X'PERT automated powder diffractometer, Model 3040. The diffractometer was equipped with automatic variable anti-scatter and divergence slits, X'Celerator RTMS detector, and Ni filter. The radiation was Cu-K(alpha) (45 kV, 40 mA). Data were collected at room temperature from 3 to 50 degrees 2-theta using a continuous scan with an equivalent step size of 0.02 degrees and a count time of 320 seconds per step in theta-theta geometry. Samples were lightly ground with an agate mortar and pestle as needed and prepared on low background silicon specimen holders as a thin layer of powdered material. MDI/Jade software version 9.1 was used with the International Committee for Diffraction Data database PDF4+ 2008 for phase identification. Cu-K(alphal) X-ray diffraction maxima for Form C ({drug1c}) of Compound 1 ({drug1}) were calculated using the MDI/Jade "Find Peaks" routine and are listed Table 22.

Table 22 - 2Θ X-ray Maxima (in degrees) for Polymorph Form C ({drug1c}) of Compound 1 ({drug1}):

| 2θ | 2θ | 2θ | 2θ | 2θ | 2θ | 2θ |
|---|---|---|---|---|---|---|
| 7.691 | 17.198 | 20.909 | 25.371 | 30.149 | 36.6 | 42.498 |
| 7.991 | 18.035 | 21.797 | 25.674 | 30.634 | 37.389 | 45.142 |
| 11.133 | 18.636 | 22.214 | 25.956 | 31.272 | 38.054 | 45.99 |
| 12.587 | 18.939 | 23.299 | 26.409 | 31.619 | 38.442 | 46.229 |
| 13.305 | 19.389 | 23.547 | 27.395 | 32.056 | 38.651 | 48.188 |
| 13.757 | 19.889 | 24.103 | 28.498 | 32.898 | 40.661 | 49.561 |
| 15.463 | 20.312 | 24.269 | 28.728 | 33.594 | 40.86 | |
| 16.683 | 20.476 | 24.438 | 29.808 | 33.813 | 41.721 | |

Formulation/Utility: A solid form of Compound 1 ({drug1}) will generally be used as a parasitic nematode control active ingredient in a composition, i.e. formulation, with at least one additional component selected from the group consisting of surfactants, solid diluents and liquid carriers (i.e. liquid fluids that carry the active and possibly other ingredients; also called liquid diluents). The formulation or composition ingredients are selected to be consistent with the physical properties of the active ingredient, mode of application and environmental factors such as soil type, moisture and temperature. Useful formulations of nematocidal active ingredients generally include both liquid and solid compositions. Liquid compositions include solutions (e.g., emulsifiable concentrates), emulsions (including micro-emulsions), dispersions and suspensions, and combinations of these forms (e.g., suspo-emulsions). The term "suspension" particularly refers to a dispersion of particulates that has been stabilized by addition of a chemical additive to minimize or stop sedimentation of the active ingredient. In a dispersion or suspension of particulates (e.g., aqueous suspension concentrate and oil dispersion formulations), a liquid carrier forms a continuous liquid phase in which the particulates (e.g., of a solid form of Compound 1 ({drug1})) are dispersed or suspended. In a composition that combines a suspension or

dispersion of particulates with an emulsion containing a second (immiscible) liquid (e.g., a suspo-emulsion formulation), a liquid carrier forms a continuous liquid phase in which not only the particulates are suspended but also droplets (i.e. non-continuous liquid phase) of the second liquid are emulsified. Dispersions and suspensions may be aqueous (i.e. containing mainly water as the liquid carrier) or non-aqueous (i.e., comprising water-immiscible organic compounds, commonly referred to as "oil", as the liquid carrier) according to the nature of the liquid carrier forming the continuous liquid phase. The general types of aqueous liquid compositions include soluble concentrates, suspension concentrates, capsule suspensions, concentrated emulsions, micro-emulsions and suspo-emulsions. Thus in suspo-emulsions the liquid carrier forming the continuous liquid phase is aqueous (i.e. contains water as its main constituent) and a water-immiscible liquid component is emulsified in the aqueous liquid carrier. The general types of non-aqueous liquid compositions include emulsifiable concentrates, micro-emulsifiable concentrates, dispersible concentrates and oil dispersions. Suspension concentrates contain particulates dispersed in a continuous liquid phase and exists as particulate dispersions on addition to water. Suspo-emulsions and oil dispersions form both particulate dispersions and emulsions that coexist on addition to water, where one or more of these phases may contain active ingredient. (In the present compositions, the particulate dispersions comprise a solid form of Compound 1 ({drug1}).). The general types of solid compositions include dusts, powders, granules, pellets, prills, pastilles, tablets, filled films (including seed coatings) and the like, which can be water-dispersible ("wettable") or water-soluble. Films and coatings formed from film-forming liquids are particularly useful for seed treatment, in addition to having applications in both liquid and solid formulation types in general. Active ingredients can be encapsulated (including micro-encapsulated) and further formed into a liquid suspension or dispersion or into a solid formulation, to protect the active ingredient or control or delay release of the active ingredient on application to the target. Alternatively, the entire formulation, including the active ingredient, can be encapsulated (or "overcoated"). Encapsulation can also control or delay release of the active ingredient. High-strength compositions can be prepared and used as intermediates for subsequent use in preparing lower strength liquid and solid formulations. Sprayable formulations are typically extended in a suitable medium before spraying. Such liquid and solid formulations are formulated to be readily diluted in the spray medium, usually water. Spray volumes can range from about one to several thousand liters per hectare, but more typically are in the range from about ten to several hundred liters per hectare. Sprayable formulations can be tank mixed with water or another suitable medium for foliar treatment by aerial or ground application, or for application to the growing medium of the plant. Liquid and dry formulations can be metered directly into drip irrigation systems or metered into the furrow during planting. Liquid and solid formulations can be applied onto seeds of crops and other desirable vegetation as seed treatments before planting to protect developing roots and other subterranean plant parts and/or foliage through systemic uptake. Although the solid forms of Compound 1 ({drug1}) according to the present invention can be used to prepare liquid solutions, emulsifiable concentrates and emulsions by combining with a solvent dissolving the solid forms, the solid forms can only retain their identity in formulated compositions containing Compound 1 ({drug1}) as a solid (e.g., particles). The nematocidal compositions of the present invention wherein the composition comprises at least one solid form of Compound 1 ({drug1}) thus include liquid compositions containing Compound 1 ({drug1}) as a solid (e.g., dispersions, suspensions, suspo-emulsions) and solid compositions of Compound 1 ({drug1}). Even though all polymorph forms and the amorphous solid form of Compound 1 ({drug1}) can be used to prepare nematocidal compositions of the present invention, polymorph Form A ({drug1a}) is particularly useful for forming nematocidal compositions, especially liquid compositions, having excellent physical as well as chemical stability. Although all polymorph forms and the amorphous solid form of Compound 1 ({drug1}) are relatively stable (metastable) when isolated and maintained near room temperature, they are nevertheless thermodynamically unstable relative to polymorph Form A ({drug1a}). Therefore, they are inherently susceptible to conversion to polymorph Form A ({drug1a}). Contact with moisture, subjection to higher temperatures or long time periods may promote conversion to a more stable crystal form. Contact with solvents generally also promotes conversion of crystal forms. Therefore liquid compositions comprising other polymorph forms, mixtures of polymorph forms or the amorphous solid form of Compound 1 ({drug1}) are particularly vulnerable to spontaneous recrystallization to polymorph Form A ({drug1a}) (see Preparation Example 7). Because of minimal nucleation and slow growth, the polymorph Form A ({drug1a}) crystals formed will be relatively few and large. This can result in both decreased biological efficacy and increased settling of the active ingredient, because high biological activity and suspensibility depend upon small particle size of solid active ingredient dispersed in liquid compositions. Using polymorph Form A ({drug1a}) to prepare nematocidal compositions removes the risk of later recrystallization in the compositions. Also, a formulation containing a less stable crystal form than Form A ({drug1a}) may change its biological activity over the course of its shelf life as the ratio of crystal forms change. This is generally highly undesired as required use rates (amount of active ingredient per hectare) would change unpredictably. Accordingly, of note is a nematocidal composition of the invention comprising polymorph Form A ({drug1a}) of Compound 1 ({drug1}). Both liquid and solid formulations comprising at least one solid form of Compound 1 ({drug1}) will typically contain effective amounts of active ingredient, solid diluent or liquid carrier, and surfactant within the following approximate ranges, which add up to 100 percent by weight. General ranges of amounts of active ingredient (i.e. a solid form ofCompound 1 ({drug1}) and optionally other active ingredients), diluent and surfactant components in the present composition comprising at least one solid form of Compound 1 ({drug1}) are as follows:

| Formulation Type | Composition in Weight Percent | | |
|---|---|---|---|
| | Active Ingredient | Diluent | Surfactant |
| Water-Dispersible Granules, Tablets and Powders | 0.001-90 | 0-99.999 | 0-25 |
| Oil Dispersions, Aqueous Suspensions | 1-60 | 40-99 | 0-50 |
| Dusts | 1-25 | 70-99 | 0-5 |
| Granules and Pellets | 0.001-95 | 5-99.999 | 0-20 |
| High Strength Compositions | 90-99 | 0-10 | 0-10 |

Solid diluents include, for example, clays such as bentonite, montmorillonite, attapulgite and kaolin, gypsum, cellulose, titanium dioxide, zinc oxide, starch, dextrin, sugars (e.g., lactose, sucrose), silica, talc, mica, diatomaceous earth, urea, calcium carbonate, sodium carbonate and bicarbonate, and sodium sulfate. Typical solid diluents are described in Watkins et al, Handbook of Insecticide Dust Diluents and Carriers, 2nd Ed., Dorland Books, Caldwell, New Jersey. Liquid diluents include, for example, water, N,N-dimethylalkanamides (e.g., N,N-dimethylformamide), limonene, dimethyl sulfoxide, N-alkylpyrrolidones (e.g., N-methylpyrrolidinone), ethylene glycol, triethylene glycol, propylene glycol, dipropylene glycol, polypropylene glycol, propylene carbonate, butylene carbonate, paraffins (e.g., white mineral oils, normal paraffins, isoparaffins), alkylbenzenes, alkylnaphthalenes, glycerine, glycerol triacetate, sorbitol, triacetin, aromatic hydrocarbons, dearomatized aliphatics, alkylbenzenes, alkylnaphthalenes, ketones such as cyclohexanone, 2-heptanone, isophorone and 4-hydroxy-4-methyl-2-pentanone, acetates such as isoamyl acetate, hexyl acetate, heptyl acetate, octyl acetate, nonyl acetate, tridecyl acetate and isobornyl acetate, other esters such as alkylated lactate esters, dibasic esters and γ-butyrolactone, and alcohols, which can be linear, branched, saturated or unsaturated, such as methanol, ethanol, wpropanol, isopropyl alcohol, M-butanol, isobutyl alcohol, w-hexanol, 2-ethylhexanol, w-octanol, decanol, isodecyl alcohol, isooctadecanol, cetyl alcohol, lauryl alcohol, tridecyl alcohol, oleyl alcohol, cyclohexanol, tetrahydrofurfuryl alcohol, diacetone alcohol and benzyl alcohol. Liquid diluents also include glycerol esters of saturated and unsaturated fatty acids (typically C^-C22), such as plant seed and fruit oils (e.g., oils of olive, castor, linseed, sesame, corn (maize), peanut, sunflower, grapeseed, safflower, cottonseed, soybean, rapeseed, coconut and palm kernel), animal-sourced fats (e.g., beef tallow, pork tallow, lard, cod liver oil, fish oil), and mixtures thereof. Liquid diluents also include alkylated fatty acids (e.g., methylated, ethylated, butylated) wherein the fatty acids may be obtained by hydrolysis of glycerol esters from plant and animal sources, and can be purified by distillation. Typical liquid diluents are described in Marsden, Solvents Guide, 2nd Ed., Interscience, New York, 1950. The solid and liquid compositions of the present invention often include one or more surfactants. When added to a liquid, surfactants (also known as "surface-active agents") generally modify, most often reduce, the surface tension of the liquid. Depending on the nature of the hydrophilic and lipophilic groups in a surfactant molecule, surfactants can be useful as wetting agents, dispersants, emulsifiers or defoaming agents. Surfactants can be classified as nonionic, anionic or cationic. Nonionic surfactants useful for the present compositions include, but are not limited to: alcohol alkoxylates such as alcohol alkoxylates based on natural and synthetic alcohols (which may be branched or linear) and prepared from the alcohols and ethylene oxide, propylene oxide, butylene oxide or mixtures thereof; amine ethoxylates, alkanolamides and ethoxylated alkanolamides; alkoxylated triglycerides such as ethoxylated soybean, castor and rapeseed oils; alkylphenol alkoxylates such as octylphenol ethoxylates, nonylphenol ethoxylates, dinonyl phenol ethoxylates and dodecyl phenol ethoxylates (prepared from the phenols and ethylene oxide, propylene oxide, butylene oxide or mixtures thereof); block polymers prepared from ethylene oxide or propylene oxide and reverse block polymers where the terminal blocks are prepared from propylene oxide; ethoxylated fatty acids; ethoxylated fatty esters and oils; ethoxylated methyl esters; ethoxylated tristyrylphenol (including those prepared from ethylene oxide, propylene oxide, butylene oxide or mixtures thereof); fatty acid esters, glycerol esters, lanolin-based derivatives, polyethoxylate esters such as polyethoxylated sorbitan fatty acid esters, polyethoxylated sorbitol fatty acid esters and polyethoxylated glycerol fatty acid esters; other sorbitan derivatives such as sorbitan esters; polymeric surfactants such as random copolymers, block copolymers, alkyd peg (polyethylene glycol) resins, graft or comb polymers and star polymers; polyethylene glycols (pegs); polyethylene glycol fatty acid esters; silicone-based surfactants; and sugar-derivatives such as sucrose esters, alkyl polyglycosides and alkyl polysaccharides. Useful anionic surfactants include, but are not limited to: alkylaryl sulfonic acids and their salts; carboxylated alcohol or alkylphenol ethoxylates; diphenyl sulfonate derivatives; lignin and lignin derivatives such as lignosulfonates; maleic or succinic acids or their anhydrides; olefin sulfonates; phosphate esters such as phosphate esters of alcohol alkoxylates, phosphate esters of alkylphenol alkoxylates and phosphate esters of styryl phenol ethoxylates; protein-based surfactants; sarcosine derivatives; styryl phenol ether sulfate; sulfates and sulfonates of oils and fatty acids; sulfates and sulfonates of ethoxylated alkylphenols; sulfates of alcohols; sulfates of ethoxylated alcohols; sulfonates of amines and amides such as N,N-alkyltaurates; sulfonates of benzene, cumene, toluene, xylene, and dodecyl and tridecylbenzenes; sulfonates of condensed naphthalenes; sulfonates of naphthalene and alkyl naphthalene; sulfonates of fractionated petroleum; sulfosuccinamates; and sulfosuccinates and their derivatives such as dialkyl sulfosuccinate salts. Useful cationic surfactants include, but are not limited to: amides and ethoxylated amides; amines such as N-alkyl propanediamines, tripropylenetriamines and dipropylenetetramines, and ethoxylated amines, ethoxylated diamines and propoxylated amines (prepared from the amines and ethylene oxide, propylene oxide, butylene oxide or mixtures thereof); amine salts such as amine acetates and diamine salts; quaternary ammonium salts such as simple quaternary salts, ethoxylated quaternary salts and diquaternary salts; and amine oxides such as alkyldimethylamine oxides and bis-(2-hydroxyethyl)-alkylamine oxides. Also useful for the present compositions are mixtures of nonionic and anionic surfactants or mixtures of nonionic and cationic surfactants. Nonionic, anionic and cationic surfactants and their recommended uses are disclosed in a variety of published references including McCutcheon 's Emulsifiers and Detergents, annual American and International Editions published by McCutcheon's Division, The Manufacturing Confectioner Publishing Co.; Sisely and Wood, Encyclopedia of Surface Active Agents, Chemical Publ. Co., Inc., New York, 1964; and A. S. Davidson and B. Milwidsky, Synthetic Detergents, Seventh Edition, John Wiley and Sons, New York, 1987. Compositions of this invention may also contain formulation auxiliaries and additives, known to those skilled in the art as formulation aids (some of which may be considered to also function as solid diluents, liquid diluents or surfactants). Such formulation auxiliaries and additives may control: pH (buffers), foaming during processing (antifoams such polyorganosiloxanes), sedimentation of active ingredients (suspending agents), viscosity (thixotropic or pseudoplastic thickeners), in-container microbial growth (antimicrobials), product freezing (antifreezes), color (dyes/pigment dispersions), wash-off (film formers or sticking agents), evaporation (evaporation retardants), and other formulation attributes. Film formers include, for example, polyvinyl acetates, polyvinyl acetate copolymers, polyvinylpyrrolidone-vinyl acetate copolymer, polyvinyl alcohols, polyvinyl alcohol copolymers and waxes. Examples of formulation auxiliaries and additives include those listed in McCutcheon 's Volume 2: Functional Materials, annual International and North American editions published by McCutcheon's Division, The Manufacturing Confectioner Publishing Co.; and PCT Publication WO 03/024222. The solid forms of Compound 1 ({drug1}) and any other active ingredients are typically incorporated into the present compositions by dissolving the active ingredient in a solvent or by grinding in a liquid or dry diluent. Solutions, including emulsifiable concentrates, can be prepared by simply mixing the ingredients. If the solvent of a liquid composition intended for use as an emulsifiable concentrate is water-immiscible, an emulsifier is typically added to emulsify the active-containing solvent upon dilution with water. Active ingredient slurries, with particle diameters of up to 2000 μm can be wet milled using media mills to obtain particles with average diameters below 3 μm. Aqueous slurries can be made into finished suspension concentrates (see, for example, U.S. 3,060,084) or further processed by spray-drying to form water-dispersible granules. Dry formulations usually require dry milling processes, which produce average particle diameters in the 2 to 10 μm range. Dusts and powders can be prepared by blending and grinding (such as with a hammer mill or fluid-energy mill). Granules and pellets can be prepared by spraying the active material upon preformed granular carriers or by agglomeration techniques. See Browning, "Agglomeration", Chemical Engineering, December 4, 1967, pages 147^18; Perry 's Chemical Engineer 's Handbook, 4th Ed., McGraw-Hill, New York, 1963, pages 8-57 and following, and WO 91/13546. Pellets can be prepared as described in U.S. 4, 172,714. Water-dispersible and water-soluble granules can be prepared as taught in U.S. 4, 144,050, U.S. 3,920,442 and DE 3,246,493. Tablets can be prepared as taught in U.S. 5, 180,587, U.S. 5,232,701 and U.S. 5,208,030. Films can be prepared as taught in GB 2,095,558 and U.S. 3,299,566. For further information regarding the art of formulation, see T. S. Woods, "The Formulator's Toolbox - Product Forms for Modern Agriculture" in Pesticide Chemistry and Bioscience, The Food-Environment Challenge, T. Brooks and T. R. Roberts, Eds., Proceedings of the 9th International Congress on Pesticide Chemistry, The Royal Society of Chemistry, Cambridge, 1999, pp. 120-133. See also U.S. 3,235,361, Col. 6, line 16 through Col. 7, line 19 and Examples 10-41; U.S. 3,309, 192, Col. 5, line 43 through Col. 7, line 62 and Examples 8, 12, 15, 39, 41, 52, 53, 58, 132, 138-140, 162-164, 166, 167 and 169-182; U.S. 2,891,855, Col. 3, line 66 through Col. 5, line 17 and Examples 1-4; Klingman, Weed Control as a Science, John Wiley and Sons, Inc., New York, 1961, pages 81-96; Hance et al, Weed Control Handbook, 8th Ed., Blackwell Scientific Publications, Oxford, 1989; and Developments in formulation technology, PJB Publications, Richmond, UK, 2000. The following formulation examples are presented to further illustrate but not limit the disclosure in any way whatsoever. All percentages are given by weight and all formulations are prepared using conventional techniques. Without further elaboration, it is believed that one skilled in the art using the preceding descriptions and references can utilize the present invention to its fullest extent.

Formulation Example A

| High Strength Concentrate | |
|---|---|
| polymorph Form A of Compound **1** | 98.5% |
| silica aerogel | 0.5% |
| synthetic amorphous fine silica | 1.0% |

(continued)

Formulation Example B

| Wettable Powder | |
|---|---|
| polymorph Forms A and B of Compound **1** | 65.0% |
| dodecylphenol polyethylene glycol ether | 2.0% |
| sodium ligninsulfonate | 4.0% |
| sodium silicoaluminate | 6.0% |
| montmorillonite (calcined) | 23.0% |

Formulation Example C

| Granule | |
|---|---|
| polymorph Form A of Compound 1 | 10.0% |
| attapulgite granules (low volatile matter, 0.71/0.30 mm; U.S.S. No. 25-50 sieves) | 90.0% |

Formulation Example D

| Extruded Pellet | |
|---|---|
| polymorph Form A of Compound **1** | 25.0% |
| anhydrous sodium sulfate | 10.0% |
| crude calcium ligninsulfonate | 5.0% |
| sodium alkylnaphthalenesulfonate | 1.0% |
| calcium/magnesium bentonite | 59.0% |

Formulation Example E

| Emulsifiable Concentrate | |
|---|---|
| polymorph Forms A and B of Compound **1** | 10.0% |
| polyoxyethylene sorbitol hexoleate | 20.0% |
| $C_6$-$C_{10}$ fatty acid methyl ester | 70.0% |

Formulation Example F

| Microemulsion | |
|---|---|
| polymorph Form A of Compound **1** | 5.0% |
| polyvinylpyrrolidone-vinyl acetate copolymer | 30.0% |
| alkylpolyglycoside | 30.0% |
| glyceryl monooleate | 15.0% |
| water | 20.0% |

Formulation Example G

| Seed Treatment | |
|---|---|
| polymorph Form A of Compound **1** | 20.00% |
| polyvinylpyrrolidone-vinyl acetate copolymer | 5.00% |
| montan acid wax | 5.00% |
| calcium ligninsulfonate | 1.00% |
| polyoxyethylene/polyoxypropylene block copolymers | 1.00% |
| stearyl alcohol (POE 20) | 2.00% |
| polyorganosilane | 0.20% |
| colorant red dye | 0.05% |
| water | 65.75% |

Formulation Example H

| Fertilizer Stick | |
|---|---|
| polymorph Form A of Compound **1** | 2.50% |
| pyrrolidone-styrene copolymer | 4.80% |
| tristyrylphenyl 16-ethoxylate | 2.30% |
| talc | 0.80% |

(continued)

Formulation Example H

| Fertilizer Stick | |
|---|---|
| corn starch | 5.00% |
| Nitrophoska® Permanent 15-9-15 slow-release fertilizer (BASF) | 36.00% |
| kaolin | 38.00% |
| water | 10.60% |

The solid forms of Compound 1 ({drug1}) and their compositions are thus useful agronomically for protecting field crops from parasitic nematodes, and also nonagronomically for protecting other horticultural crops and plants from phytophagous parasitic nematodes. This utility includes protecting crops and other plants (i.e. both agronomic and nonagronomic) that contain genetic material introduced by genetic engineering (i.e. transgenic) or modified by mutagenesis to provide advantageous traits. Examples of such traits include tolerance to herbicides, resistance to phytophagous pests (e.g., insects, mites, aphids, spiders, nematodes, snails, plant-pathogenic fungi, bacteria and viruses), improved plant growth, increased tolerance of adverse growing conditions such as high or low temperatures, low or high soil moisture, and high salinity, increased flowering or fruiting, greater harvest yields, more rapid maturation, higher quality and/or nutritional value of the harvested product, or improved storage or process properties of the harvested products. Transgenic plants can be modified to express multiple traits. Examples of plants containing traits provided by genetic engineering or mutagenesis include varieties of corn, cotton, soybean and potato expressing an insecticidal Bacillus thuringiensis toxin such as YIELD GARD®, KNOCKOUT®, STARLINK®, BOLLGARD®, NuCOTN® and NEWLEAF®, and herbicide-tolerant varieties of corn, cotton, soybean and rapeseed such as ROUNDUP READY®, LIBERTY LINK®, IMI, STS and CLEARFIELD, as well as crops expressing N-acetyltransferase (GAT) to provide resistance to glyphosate herbicide, or crops containing the HRA gene providing resistance to herbicides inhibiting acetolactate synthase (ALS). The solid forms of Compound 1 ({drug1}) and their compositions may interact synergistically with traits introduced by genetic engineering or modified by mutagenesis, thus enhancing phenotypic expression or effectiveness of the traits or increasing the parasitic nematode control effectiveness of the present compounds and compositions. In particular, the solid forms of Compound 1 ({drug1}) and their compositions may interact synergistically with the phenotypic expression of proteins or other natural products toxic to parasitic nematodes to provide greater-than-additive control of these pests. Compositions of this invention can also optionally comprise plant nutrients, e.g., a fertilizer composition comprising at least one plant nutrient selected from nitrogen, phosphorus, potassiun, sulfur, calcium, magnesium, iron, copper, boron, manganese, zinc, and molybdenum. Of note are compositions comprising at least one fertilizer composition comprising at least one plant nutrient selected from nitrogen, phosphorus, potassium, sulfur, calcium and magnesium. Compositions of the present invention which further comprise at least one plant nutrient can be in the form of liquids or solids. Of note are solid formulations in the form of granules, small sticks or tablets. Solid formulations comprising a fertilizer composition can be prepared by mixing the compound or composition of the present invention with the fertilizer composition together with formulating ingredients and then preparing the formulation by methods such as granulation or extrusion. Alternatively solid formulations can be prepared by spraying a solution or suspension of a compound or composition of the present invention in a volatile solvent onto a previous prepared fertilizer composition in the form of dimensionally stable mixtures, e.g., granules, small sticks or tablets, and then evaporating the solvent. Solid forms of Compound 1 ({drug1}) can exhibit activity against a wide spectrum of parasitic nematodes that live or grow inside or feed on plants (e.g., foliage, fruit, stems, roots or seeds) or animals and humans (e.g., vascular or digestive systems or other tissues) and therefore damage growing and stored agronomic crops, forestry, greenhouse crops, ornamentals and nursery crops, or afflict animal and human health. Crops of particular interest are fruiting vegetables such as solanaceous and cucurbit crops, plantation crops such as banana and coffee, root crops such as potatoes, onion and carrots, and field crops such as tobacco, peanut, cotton, sugarcane and soybean. Solid forms of Compound 1 ({drug1}) can have activity on members of both classes Adenophorea and Secementea of the Phylum Nematoda, including economically important members of the orders Enoplida, Dorylaimida, Rhabditida, Strongylida, Ascarida, Oxyurida, Spirurida, Tylenchida and Aphelenchida, such as but not limited to economically important agricultural pests such as root-knot nematodes of the genus Meloidogyne, cyst nematodes of the genera Heterodera and Globodera, lesion nematodes of the genus Pratylenchus, reniform nematodes of the genus Rotylenchulus, burrowing nematodes of the genus Radopholus, sting nematodes of the genus Belonolaimus, spiral nematodes of the genera Helicotylenchus and Scutellonema, citrus nematodes of the genus Tylenchulus, stubby root nematodes of the genera Trichodorus and Paratrichodorus, dagger nematodes of the genus Xiphinema, stunt nematodes of the genus Tylenchorhynchus, needle nematodes of the genera Longidorus and Paralongidorus, lance nematodes of the genus Hoplolaimus, ring nematodes of the family Criconematidae, stem nematodes of the genera Ditylenchus and Anguina, and foliar/stem nematodes of the genera Aphelenchoides and Rhadinaphelenchus; and animal and human health parasites (i.e. economically important roundworms such as Strongylus vulgaris in horses, Toxocara canis in dogs, Haemonchus contortus in sheep, Dirofllaria immitis in dogs, etc.). Of note is use of solid forms of Compound 1 ({drug1}) for controlling southern root-knot nematode (Meloidogyne incognita). Those skilled in the art will appreciate that solid forms of Compound 1 ({drug1}) are not equally effective against all growth stages of all nematodes. Solid forms of Compound 1 ({drug1}) can also have activity on members of the Phylum Platyhelminthes, classes Cestoda (Tapeworms) and Trematoda (Flukes), including parasites (i.e. economically important flukes and tapeworms) afflicting animal and human health (e.g., Anoplocephala perfoliata in horses, Fasciola hepatica in ruminants, etc.). Solid forms of Compound 1 ({drug1}) can also be mixed with one or more other biologically active compounds or agents including insecticides, fungicides, nematocides, bactericides, acaricides, herbicides, herbicide safeners, growth regulators such as insect molting inhibitors and rooting stimulants, chemosterilants, semiochemicals, repellents, attractants, pheromones, feeding stimulants, other biologically active compounds or entomopathogenic bacteria, virus or fungi to form a multi-component pesticide giving an even broader spectrum of agronomic and nonagronomic utility. Thus the present invention also pertains to a composition comprising a solid form of Compound 1 ({drug1}) and an effective amount of at least one additional biologically active compound or agent and can further comprise at least one of surfactants, solid diluents or liquid diluents. For mixtures of the present invention, the other biologically active compounds or agents can be formulated together with the solid forms of Compound 1 ({drug1}), to form a premix, or the other biologically active compounds or agents can be formulated separately from the solid forms of Compound 1 ({drug1}) and the two formulations combined together before application (e.g., in a spray tank) or, alternatively, applied in succession. Examples of such biologically active compounds or agents with which solid forms of Compound 1 ({drug1}) can be formulated are insecticides such as abamectin, acephate, acequinocyl, acetamiprid, acrinathrin, amidoflumet, amitraz, avermectin, azadirachtin, azinphos-methyl, bifenthrin, bifenazate, bistrifluron, borate, buprofezin, cadusafos, carbaryl, carbofuran, cartap, carzol, chlorantraniliprole, chlorfenapyr, chlorfluazuron, chlorpyrifos, chlorpyrifos-methyl, chromafenozide, clofentezin, clothianidin, cyantraniliprole, cyflumetofen, cyfluthrin, beta-cyfluthrin, cyhalothrin, gamma-cyhalothrin, lambda-cyhalothrin, cypermethrin, alpha-cypermethrin, zeta-cypermethrin, cyromazine, deltamethrin, diafenthiuron, diazinon, dieldrin, diflubenzuron, dimefluthrin, dimehypo, dimethoate, dinotefuran, diofenolan, emamectin, endosulfan, esfenvalerate, ethiprole, etofenprox, etoxazole, fenbutatin oxide, fenothiocarb, fenoxycarb, fenpropathrin, fenvalerate, fipronil, flonicamid, flubendiamide, flucythrinate, flufenerim, flufenoxuron, fluvalinate, tau-fluvalinate, fonophos, formetanate, fosthiazate, halofenozide, hexaflumuron, hexythiazox, hydramethylnon, imidacloprid, indoxacarb, insecticidal soaps, isofenphos, lufenuron, malathion, metaflumizone, metaldehyde, methamidophos, methidathion, methiodicarb, methomyl, methoprene, methoxychlor, metofluthrin, monocrotophos, methoxyfenozide, nitenpyram, nithiazine, novaluron, noviflumuron, oxamyl, parathion, parathion-methyl, permethrin, phorate, phosalone, phosmet, phosphamidon, pirimicarb, profenofos, profluthrin, propargite, protrifenbute, pymetrozine, pyrafluprole, pyrethrin, pyridaben, pyridalyl, pyrifluquinazon, pyriprole, pyriproxyfen, rotenone, ryanodine, spinetoram, spinosad, spirodiclofen, spiromesifen, spirotetramat, sulprofos, tebufenozide, tebufenpyrad, teflubenzuron, tefluthrin, terbufos, tetrachlorvinphos, tetramethrin, thiacloprid, thiamethoxam, thiodicarb, thiosultap-sodium, tolfenpyrad, tralomethrin, triazamate, trichlorfon, triflumuron, Bacillus thuringiensis delta-endotoxins, entomopathogenic bacteria, entomopathogenic viruses and entomopathogenic fungi. Of note are insecticides such as abamectin, acetamiprid, acrinathrin, amitraz, avermectin, azadirachtin, bifenthrin, buprofezin, cadusafos, carbaryl, cartap, chlorantraniliprole, chlorfenapyr, chlorpyrifos, clothianidin, cyantraniliprole, cyfluthrin, beta-cyfluthrin, cyhalothrin, gamma-cyhalothrin, lambda-cyhalothrin, cypermethrin, alpha-cypermethrin, zeta-cypermethrin, cyromazine, deltamethrin, dieldrin, dinotefuran, diofenolan, emamectin, endosulfan, es fenvalerate, ethiprole, etofenprox, etoxazole, fenothiocarb, fenoxycarb, fenvalerate, fipronil, flonicamid, flubendiamide, flufenoxuron, fluvalinate, formetanate, fosthiazate, hexaflumuron, hydramethylnon, imidacloprid, indoxacarb, lufenuron, metaflumizone, methiodicarb, methomyl, methoprene, methoxyfenozide, nitenpyram, nithiazine, novaluron, oxamyl, pymetrozine, pyrethrin, pyridaben, pyridalyl, pyriproxyfen, ryanodine, spinetoram, spinosad, spirodiclofen, spiromesifen, spirotetramat, tebufenozide, tetramethrin, thiacloprid, thiamethoxam, thiodicarb, thiosultap-sodium, tralomethrin, triazamate, triflumuron, Bacillus thuringiensis delta-endotoxins, all strains of Bacillus thuringiensis and all strains of Nucleo polyhydrosis viruses. One embodiment of biological agents for mixing with solid forms of Compound 1 ({drug1}) include entomopathogenic bacteria such as Bacillus thuringiensis, and the encapsulated delta-endotoxins of Bacillus thuringiensis such as MVP® and MVPII® bioinsecticides prepared by the CellCap® process (CellCap®, MVP® and MVPII® are trademarks of Mycogen Corporation, Indianapolis, Indiana, USA); entomopathogenic fungi such as green muscardine fungus; and entomopathogenic (both naturally occurring and genetically modified) viruses including baculovirus, nucleopolyhedro virus (NPV) such as Helicoverpazea nucleopolyhedrovirus (HzNPV), Anagrapha falcifera nucleopolyhedrovirus (AfNPV); and granulosis virus (GV) such as Cydia pomonella granulosis virus (CpGV). Of particular note is such a combination where the other invertebrate pest control active ingredient belongs to a different chemical class or has a different site of action than solid forms of Compound 1 ({drug1}). In certain instances, a combination with at least one other invertebrate pest control active ingredient having a similar spectrum of control but a different site of action will be particularly advantageous for resistance management. Thus, a composition of the present invention can further comprise at least one additional invertebrate pest control active ingredient having a similar spectrum of control but belonging to a different chemical class or having a different site of action. These additional biologically

active compounds or agents include, but are not limited to, sodium channel modulators such as bifenthrin, cypermethrin, cyhalothrin, lambda-cyhalothrin, cyfluthrin, beta-cyfluthrin, deltamethrin, dimefluthrin, esfenvalerate, fenvalerate, indoxacarb, metofluthrin, profluthrin, pyrethrin and tralomethrin; cholinesterase inhibitors such as chlorpyrifos, methomyl, oxamyl, thiodicarb and triazamate; neonicotinoids such as acetamiprid, clothianidin, dinotefuran, imidacloprid, nitenpyram, nithiazine, thiacloprid and thiamethoxam; insecticidal macrocyclic lactones such as spinetoram, spinosad, abamectin, avermectin and emamectin; GABA (γ-aminobutyric acid)-gated chloride channel antagonists such as avermectin or blockers such as ethiprole and fipronil; chitin synthesis inhibitors such as buprofezin, cyromazine, flufenoxuron, hexaflumuron, lufenuron, novaluron, noviflumuron and triflumuron; juvenile hormone mimics such as diofenolan, fenoxycarb, methoprene and pyriproxyfen; octopamine receptor ligands such as amitraz; molting inhibitors and ecdysone agonists such as azadirachtin, methoxyfenozide and tebufenozide; ryanodine receptor ligands such as ryanodine, anthranilic diamides such as chlorantraniliprole, cyantraniliprole and flubendiamide; nereistoxin analogs such as cartap; mitochondrial electron transport inhibitors such as chlorfenapyr, hydramethylnon and pyridaben; lipid biosynthesis inhibitors such as spirodiclofen and spiromesifen; cyclodiene insecticides such as dieldrin or endosulfan; pyrethroids; carbamates; insecticidal ureas; and biological agents including nucleopolyhedro viruses (NPV), members of Bacillus thuringiensis, encapsulated delta-endotoxins of Bacillus thuringiensis, and other naturally occurring or genetically modified insecticidal viruses. Further examples of biologically active compounds or agents with which solid forms of Compound 1 ({drug1}) can be formulated are: fungicides such as acibenzolar, aldimorph, amisulbrom, azaconazole, azoxystrobin, benalaxyl, benomyl, benthiavalicarb, benthiavalicarb-isopropyl, binomial, biphenyl, bitertanol, blasticidin-S, Bordeaux mixture (Tribasic copper sulfate), boscalid/nicobifen, bromuconazole, bupirimate, buthiobate, carboxin, carpropamid, captafol, captan, carbendazim, chloroneb, chlorothalonil, chlozolinate, clotrimazole, copper oxychloride, copper salts such as copper sulfate and copper hydroxide, cyazofamid, cyflunamid, cymoxanil, cyproconazole, cyprodinil, dichlofluanid, diclocymet, diclomezine, dicloran, diethofencarb, difenoconazole, dimethomorph, dimoxystrobin, diniconazole, diniconazole-M, dinocap, discostrobin, dithianon, dodemorph, dodine, econazole, etaconazole, edifenphos, epoxiconazole, ethaboxam, ethirimol, ethridiazole, famoxadone, fenamidone, fenarimol, fenbuconazole, fencaramid, fenfuram, fenhexamide, fenoxanil, fenpiclonil, fenpropidin, fenpropimorph, fentin acetate, fentin hydroxide, ferbam, ferfurazoate, ferimzone, fiuazinam, fludioxonil, flumetover, fluopicolide, fluoxastrobin, fluquinconazole, fluquinconazole, flusilazole, flusulfamide, flutolanil, fiutriafol, folpet, fosetyl-aluminum, fuberidazole, furalaxyl, furametapyr, hexaconazole, hymexazole, guazatine, imazalil, imibenconazole, iminoctadine, iodicarb, ipconazole, iprobenfos, iprodione, iprovalicarb, isoconazole, isoprothiolane, kasugamycin, kresoxim-methyl, mancozeb, mandipropamid, maneb, mapanipyrin, mefenoxam, mepronil, metalaxyl, metconazole, methasulfocarb, metiram, metominostrobin/fenominostrobin, mepanipyrim, metrafenone, miconazole, myclobutanil, neo-asozin (ferric methanearsonate), nuarimol, octhilinone, ofurace, orysastrobin, oxadixyl, oxolinic acid, oxpoconazole, oxycarboxin, paclobutrazol, penconazole, pencycuron, penthiopyrad, perfurazoate, phosphonic acid, phthalide, picobenzamid, picoxystrobin, polyoxin, probenazole, prochloraz, procymidone, propamocarb, propamocarb-hydrochloride, propiconazole, propineb, proquinazid, prothioconazole, pyraclostrobin, pryazophos, pyrifenox, pyrimethanil, pyrifenox, pyrolnitrine, pyroquilon, quinconazole, quinoxyfen, quintozene, silthiofam, simeconazole, spiroxamine, streptomycin, sulfur, tebuconazole, techrazene, tecloftalam, tecnazene, tetraconazole, thiabendazole, thifluzamide, thiophanate, thiophanate-methyl, thiram, tiadinil, tolclofos-methyl, tolyfluanid, triadimefon, triadimenol, triarimol, triazoxide, tridemorph, trimoprhamide tricyclazole, trifloxystrobin, triforine, triticonazole, uniconazole, validamycin, vinclozolin, zineb, ziram, and zoxamide; nematocides such as aldicarb, imicyafos, oxamyl and fenamiphos; bactericides such as streptomycin; acaricides such as amitraz, chinomethionat, chlorobenzilate, cyhexatin, dicofol, dienochlor, etoxazole, fenazaquin, fenbutatin oxide, fenpropathrin, fenpyroximate, hexythiazox, propargite, pyridaben and tebufenpyrad. In certain instances, combinations of solid forms of Compound 1 ({drug1}) with other biologically active (particularly invertebrate pest control) compounds or agents (i.e. active ingredients) can result in a greater-than-additive (i.e. synergistic) effect. Reducing the quantity of active ingredients released in the environment while ensuring effective pest control is always desirable. When synergism with invertebrate pest control active ingredients occurs at application rates giving agronomically satisfactory levels of invertebrate pest control, such combinations can be advantageous for reducing crop production cost and decreasing environmental load. Solid forms of Compound 1 ({drug1}) and compositions thereof can be applied to plants genetically transformed to express proteins toxic to invertebrate pests (such as Bacillus thuringiensis delta-endotoxins). Such an application may provide a broader spectrum of plant protection and be advantageous for resistance management. The effect of the exogenously applied compounds of this invention may be synergistic with the expressed toxin proteins. General references for these agricultural protectants (i.e. insecticides, fungicides, nematocides, acaricides, herbicides and biological agents) include The Pesticide Manual, 13th Edition, C. D. S. Tomlin, Ed., British Crop Protection Council, Farnham, Surrey, U.K., 2003 and The BioPesticide Manual, 2nd Edition, L. G. Copping, Ed., British Crop Protection Council, Farnham, Surrey, U.K., 2001. For embodiments where one or more of these various mixing partners are used, the weight ratio of these various mixing partners (in total) to a solid form of Compound 1 ({drug1}) is typically between about 1 :3000 and about 3000: 1. Of note are weight ratios between about 1 :300 and about 300: 1 (for example ratios between about 1 :30 and about 30: 1). One skilled in the art can easily determine through simple experimentation the biologically effective amounts of active ingredients necessary for the

desired spectrum of biological activity. It will be evident that including these additional components can expand the spectrum of parasitic nematodes controlled beyond the spectrum controlled by a solid form of Compound 1 ({drug1}) alone. Table A lists specific combinations of a solid form of Compound 1 ({drug1}) with other invertebrate pest control agents illustrative of the mixtures, compositions and methods of the present invention and includes additional embodiments of weight ratio ranges for application rates. The first column of Table A lists the specific invertebrate control agents (e.g., "Abamectin" in the first line). The second column of Table A lists the mode of action (if known) or chemical class of the invertebrate pest control agents. The third column of Table A lists embodiment(s) of ranges of weight ratios for rates at which the invertebrate pest control agent can be applied relative to a solid form of Compound 1 ({drug1}) (e.g., "50: 1 to 1 :50" of abamectin relative to a solid form of Compound 1 ({drug1}) by weight). Thus, for example, the first line of Table A specifically discloses the combination of a solid form of Compound 1 ({drug1}) with abamectin can be applied in a weight ratio between 50: 1 to 1 :50. The remaining lines of Table A are to be construed similarly.

Table A

| Invertebrate Pest Control Agent | Mode of Action or Chemical Class | Typical Weight Ratio |
|---|---|---|
| Abamectin | macrocyclic lactones | 50:1 to 1:50 |
| Acetamiprid | neonicotinoids | 150:1 to 1:200 |
| Amitraz | octopamine receptors ligands | 200:1 to 1:100 |
| | | |
| Invertebrate Pest Control Agent | Mode of Action or Chemical Class | Typical Weight Ratio |
| Avermectin | macrocyclic lactones | 50:1 to 1:50 |
| Azadirachtin | ecdysone agonists | 100:1 to 1:120 |
| Beta-cyfluthrin | sodium channel modulators | 150:1 to 1:200 |
| Bifenthrin | sodium channel modulators | 100.1 to 1:10 |
| Buprofezin | chitin synthesis inhibitors | 500:1 to 1:50 |
| Cartap | nereistoxin analogs | 100: 1 to 1:200 |
| Chlorantraniliprole | ryanodine receptor ligands | 100:1 to 1:120 |
| Chlorfenapyr | mitochondrial electron transport inhibitors | 300:1 to 1:200 |
| Chlorpyrifos | cholinesterase inhibitors | 500:1 to 1:200 |
| Clothianidin | neonicotinoids | 100:1 to 1:400 |
| Cyantraniliprole | ryanodine receptor ligands | 100:1 to 1:120 |
| Cyfluthrin | sodium channel modulators | 150:1 to 1:200 |
| Cyhalothrin | sodium channel modulators | 150:1 to 1:200 |
| Cypermethrin | sodium channel modulators | 150:1 to 1:200 |
| Cyromazine | chitin synthesis inhibitors | 400:1 to 1:50 |
| Deltamethrin | sodium channel modulators | 50:1 to 1:400 |
| Dieldrin | cyclodiene insecticides | 200:1 to 1:100 |
| Dinotefuran | neonicotinoids | 150:1 to 1:200 |
| Diofenolan | molting inhibitor | 150:1 to 1:200 |
| Emamectin | macrocyclic lactones | 50:1 to 1:10 |
| Endosulfan | cyclodiene insecticides | 200:1 to 1:100 |
| Esfenvalerate | sodium channel modulators | 100:1 to 1:400 |
| Ethiprole | GABA-regulated chloride channel blockers | 200:1 to 1:100 |
| Fenothiocarb | | 150:1 to 1:200 |
| Fenoxycarb | juvenile hormone mimics | 500:1 to 1:100 |

(continued)

| Invertebrate Pest Control Agent | Mode of Action or Chemical Class | Typical Weight Ratio |
|---|---|---|
| Fenvalerate | sodium channel modulators | 150:1 to 1:200 |
| Fipronil | GABA-regulated chloride channel blockers | 150:1 to 1:100 |
| Flonicamid | | 200:1 to 1:100 |
| Flubendiamide | ryanodine receptor ligands | 100:1 to 1:120 |
| Flufenoxuron | chitin synthesis inhibitors | 200:1 to 1:100 |
| Hexaflumuron | chitin synthesis inhibitors | 300:1 to 1:50 |
| Hydramethylnon | mitochondrial electron transport inhibitors | 150:1 to 1:250 |
| Imidacloprid | neonicotinoids | 1000:1 to 1:1000 |
| Indoxacarb | sodium channel modulators | 200:1 to 1:50 |
| Lambda-cyhalothrin | sodium channel modulators | 50:1 to 1:250 |
| Lufenuron | chitin synthesis inhibitors | 500:1 to 1:250 |
| Metaflumizone | | 200:1 to 1:200 |
| Methomyl | cholinesterase inhibitors | 500:1 to 1:100 |
| Methoprene | juvenile hormone mimics | 500:1 to 1:100 |
| Methoxyfenozide | ecdysone agonists | 50:1 to 1:50 |
| Nitenpyram | neonicotinoids | 150:1 to 1:200 |
| Nithiazine | neonicotinoids | 150:1 to 1:200 |
| Novaluron | chitin synthesis inhibitors | 500:1 to 1:150 |
| Oxamyl | cholinesterase inhibitors | 200:1 to 1:200 |
| Pymetrozine | | 200:1 to 1:100 |
| Pyrethrin | Sodium channel modulators | 100:1 to 1:10 |
| Pyridaben | mitochondrial electron transport inhibitors | 200:1 to 1:100 |
| Pyridalyl | | 200:1 to 1:100 |
| Pyriproxyfen | juvenile hormone mimics | 500:1 to 1:100 |
| | | |
| Ryanodine | ryanodine receptors ligands | 100:1 to 1:120 |
| Spinetoram | macrocyclic lactones | 150:1 to 1:100 |
| Spinosad | macrocyclic lactones | 500:1 to 1:10 |
| Spirodiclofen | lipid biosynthesis inhibitors | 200:1 to 1:200 |
| Spiromesifen | lipid biosynthesis inhibitors | 200:1 to 1:200 |
| Tebufenozide | ecdysone agonists | 500:1 to 1:250 |
| Thiacloprid | neonicotinoids | 100:1 to 1:200 |
| Thiamethoxam | neonicotinoids | 1250:1 to 1:1000 |
| Thiodicarb | cholinesterase inhibitors | 500:1 to 1:400 |
| Thiosultap-sodium | | 150:1 to 1:100 |
| Tralomethrin | sodium channel modulators | 150:1 to 1:200 |
| Triazamate | cholinesterase inhibitors | 250:1 to 1:100 |
| Triflumuron | chitin synthesis inhibitors | 200:1 to 1:100 |

(continued)

| Invertebrate Pest Control Agent | Mode of Action or Chemical Class | Typical Weight Ratio |
|---|---|---|
| *Bacillus thuringiensis* | biological agents | 50:1 to 1:10 |
| | | |
| *Bacillus thuringiensis* delta-endotoxin | biological agents | 50:1 to 1:10 |
| NPV (e.g., Gemstar) | Biological agents | 50:1 to 1:10 |

Of note is the composition of the present invention wherein the at least one additional biologically active compound or agent is selected from the invertebrate pest control agents listed in Table A above. The weight ratios of a solid form of Compound 1 ({drug1}) to the additional invertebrate pest control agent typically are between 1000:1 and 1:1000, with one embodiment being between 500:1 and 1:500, another embodiment being between 250:1 and 1:200 and another embodiment being between 100:1 and 1:50. Listed below in Table B are embodiments of specific compositions comprising a solid form of Compound 1 ({drug1}) (polymorph Form A ({drug1a})) and an additional invertebrate pest control agent.

Table B

| Mixture No. | Cmpd. **1** Form | and | Invertebrate Pest Control Agent | Typical Mixture Ratios (by weight) | | | | | | | | |
|---|---|---|---|---|---|---|---|---|---|---|---|---|
| B-1 | A | and | Abamectin | 100:1 | 10:1 | 5:1 | 2:1 | 1:1 | 1:2 | 1:5 | 1:10 | 1:100 |
| B-2 | A | and | Acetamiprid | 100:1 | 10:1 | 5:1 | 2:1 | 1:1 | 1:2 | 1:5 | 1:10 | 1:100 |
| B-3 | A | and | Amitraz | 100:1 | 10:1 | 5:1 | 2:1 | 1:1 | 1:2 | 1:5 | 1:10 | 1:100 |
| B-4 | A | and | Avermectin | 100:1 | 10:1 | 5:1 | 2:1 | 1:1 | 1:2 | 1:5 | 1:10 | 1:100 |
| B-5 | A | and | Azadirachtin | 100:1 | 10:1 | 5:1 | 2:1 | 1:1 | 1:2 | 1:5 | 1:10 | 1:100 |
| B-5a | A | and | Bensultap | 100:1 | 10:1 | 5:1 | 2:1 | 1:1 | 1:2 | 1:5 | 1:10 | 1:100 |
| B-6 | A | and | Beta-cyfluthrin | 100:1 | 10:1 | 5:1 | 2:1 | 1:1 | 1:2 | 1:5 | 1:10 | 1:100 |
| B-7 | A | and | Bifenthrin | 100:1 | 10:1 | 5:1 | 2:1 | 1:1 | 1:2 | 1:5 | 1:10 | 1:100 |
| B-8 | A | and | Buprofezin | 100:1 | 10:1 | 5:1 | 2:1 | 1:1 | 1:2 | 1:5 | 1:10 | 1:100 |
| B-9 | A | and | Cartap | 100:1 | 10:1 | 5:1 | 2:1 | 1:1 | 1:2 | 1:5 | 1:10 | 1:100 |
| B-10 | A | and | Chlorantraniliprole | 100:1 | 10:1 | 5:1 | 2:1 | 1:1 | 1:2 | 1:5 | 1:10 | 1:100 |
| B-11 | A | and | Chlorfenapyr | 100:1 | 10:1 | 5:1 | 2:1 | 1:1 | 1:2 | 1:5 | 1:10 | 1:100 |
| B-12 | A | and | Chlorpyrifos | 100:1 | 10:1 | 5:1 | 2:1 | 1:1 | 1:2 | 1:5 | 1:10 | 1:100 |
| B-13 | A | and | Clothianidin | 100:1 | 10:1 | 5:1 | 2:1 | 1:1 | 1:2 | 1:5 | 1:10 | 1:100 |
| B-14 | A | and | Cyantraniliprole | 100:1 | 10:1 | 5:1 | 2:1 | 1:1 | 1:2 | 1:5 | 1:10 | 1:100 |
| B-15 | A | and | Cyfluthrin | 100:1 | 10:1 | 5:1 | 2:1 | 1:1 | 1:2 | 1:5 | 1:10 | 1:100 |
| B-16 | A | and | Cyhalothrin | 100:1 | 10:1 | 5:1 | 2:1 | 1:1 | 1:2 | 1:5 | 1:10 | 1:100 |
| B-17 | A | and | Cypermethrin | 100:1 | 10:1 | 5:1 | 2:1 | 1:1 | 1:2 | 1:5 | 1:10 | 1:100 |
| B-18 | A | and | Cyromazine | 100:1 | 10:1 | 5:1 | 2:1 | 1:1 | 1:2 | 1:5 | 1:10 | 1:100 |
| B-19 | A | and | Deltamethrin | 100:1 | 10:1 | 5:1 | 2:1 | 1:1 | 1:2 | 1:5 | 1:10 | 1:100 |
| B-20 | A | and | Dieldrin | 100:1 | 10:1 | 5:1 | 2:1 | 1:1 | 1:2 | 1:5 | 1:10 | 1:100 |
| B-21 | A | and | Dinotefuran | 100:1 | 10:1 | 5:1 | 2:1 | 1:1 | 1:2 | 1:5 | 1:10 | 1:100 |
| B-22 | A | and | Diofenolan | 100:1 | 10:1 | 5:1 | 2:1 | 1:1 | 1:2 | 1:5 | 1:10 | 1:100 |
| B-23 | A | and | Emamectin | 100:1 | 10:1 | 5:1 | 2:1 | 1:1 | 1:2 | 1:5 | 1:10 | 1:100 |

(continued)

| Mixture No. | Cmpd. **1** Form | and | Invertebrate Pest Control Agent | Typical Mixture Ratios (by weight) | | | | | | | | |
|---|---|---|---|---|---|---|---|---|---|---|---|---|
| B-24 | A | and | Endosulfan | 100:1 | 10:1 | 5:1 | 2:1 | 1:1 | 1:2 | 1:5 | 1:10 | 1:100 |
| B-25 | A | and | Esfenvalerate | 100:1 | 10:1 | 5:1 | 2:1 | 1:1 | 1:2 | 1:5 | 1:10 | 1:100 |
| B-26 | A | and | Ethiprole | 100:1 | 10:1 | 5:1 | 2:1 | 1:1 | 1:2 | 1:5 | 1:10 | 1:100 |
| B-27 | A | and | Fenothiocarb | 100:1 | 10:1 | 5:1 | 2:1 | 1:1 | 1:2 | 1:5 | 1:10 | 1:100 |
| | | | | | | | | | | | | |
| B-28 | A | and | Fenoxycarb | 100:1 | 10:1 | 5:1 | 2:1 | 1:1 | 1:2 | 1:5 | 1:10 | 1:100 |
| B-29 | A | and | Fenvalerate | 100:1 | 10:1 | 5:1 | 2:1 | 1:1 | 1:2 | 1:5 | 1:10 | 1:100 |
| B-30 | A | and | Fipronil | 100:1 | 10:1 | 5:1 | 2:1 | 1:1 | 1:2 | 1:5 | 1:10 | 1:100 |
| B-31 | A | and | Flonicamid | 100:1 | 10:1 | 5:1 | 2:1 | 1:1 | 1:2 | 1:5 | 1:10 | 1:100 |
| B-32 | A | and | Flubendiamide | 100:1 | 10:1 | 5:1 | 2:1 | 1:1 | 1:2 | 1:5 | 1:10 | 1:100 |
| B-33 | A | and | Flufenoxuron | 100:1 | 10:1 | 5:1 | 2:1 | 1:1 | 1:2 | 1:5 | 1:10 | 1:100 |
| B-34 | A | and | Hexaflumuron | 100:1 | 10:1 | 5:1 | 2:1 | 1:1 | 1:2 | 1:5 | 1:10 | 1:100 |
| B-35 | A | and | Hydramethylnon | 100:1 | 10:1 | 5:1 | 2:1 | 1:1 | 1:2 | 1:5 | 1:10 | 1:100 |
| B-36 | A | and | Imidacloprid | 100:1 | 10:1 | 5:1 | 2:1 | 1:1 | 1:2 | 1:5 | 1:10 | 1:100 |
| B-37 | A | and | Indoxacarb | 100:1 | 10:1 | 5:1 | 2:1 | 1:1 | 1:2 | 1:5 | 1:10 | 1:100 |
| B-38 | A | and | Lambda-cyhalothrin | 100:1 | 10:1 | 5:1 | 2:1 | 1:1 | 1:2 | 1:5 | 1:10 | 1:100 |
| B-39 | A | and | Lufenuron | 100:1 | 10:1 | 5:1 | 2:1 | 1:1 | 1:2 | 1:5 | 1:10 | 1:100 |
| B-40 | A | and | Metaflumizone | 100:1 | 10:1 | 5:1 | 2:1 | 1:1 | 1:2 | 1:5 | 1:10 | 1:100 |
| B-41 | A | and | Methomyl | 100:1 | 10:1 | 5:1 | 2:1 | 1:1 | 1:2 | 1:5 | 1:10 | 1:100 |
| B-42 | A | and | Methoprene | 100:1 | 10:1 | 5:1 | 2:1 | 1:1 | 1:2 | 1:5 | 1:10 | 1:100 |
| B-43 | A | and | Methoxyfenozide | 100:1 | 10:1 | 5:1 | 2:1 | 1:1 | 1:2 | 1:5 | 1:10 | 1:100 |
| B-44 | A | and | Nitenpyram | 100:1 | 10:1 | 5:1 | 2:1 | 1:1 | 1:2 | 1:5 | 1:10 | 1:100 |
| B-45 | A | and | Nithiazine | 100:1 | 10:1 | 5:1 | 2:1 | 1:1 | 1:2 | 1:5 | 1:10 | 1:100 |
| B-46 | A | and | Novaluron | 100:1 | 10:1 | 5:1 | 2:1 | 1:1 | 1:2 | 1:5 | 1:10 | 1:100 |
| B-47 | A | and | Oxamyl | 100:1 | 10:1 | 5:1 | 2:1 | 1:1 | 1:2 | 1:5 | 1:10 | 1:100 |

(continued)

| Mixture No. | Cmpd. **1** Form | and | Invertebrate Pest Control Agent | Typical Mixture Ratios (by weight) | | | | | | | | |
|---|---|---|---|---|---|---|---|---|---|---|---|---|
| B-48 | A | and | Phosmet | 100:1 | 10:1 | 5:1 | 2:1 | 1:1 | 1:2 | 1:5 | 1:10 | 1:100 |
| B-49 | A | and | Pymetrozine | 100:1 | 10:1 | 5:1 | 2:1 | 1:1 | 1:2 | 1:5 | 1:10 | 1:100 |
| B-50 | A | and | Pyrethrin | 100:1 | 10:1 | 5:1 | 2:1 | 1:1 | 1:2 | 1:5 | 1:10 | 1:100 |
| B-51 | A | and | Pyridaben | 100:1 | 10:1 | 5:1 | 2:1 | 1:1 | 1:2 | 1:5 | 1:10 | 1:100 |
| B-52 | A | and | Pyridalyl | 100:1 | 10:1 | 5:1 | 2:1 | 1:1 | 1:2 | 1:5 | 1:10 | 1:100 |
| B-53 | A | and | Pyriproxyfen | 100:1 | 10:1 | 5:1 | 2:1 | 1:1 | 1:2 | 1:5 | 1:10 | 1:100 |
| B-54 | A | and | Ryanodine | 100:1 | 10:1 | 5:1 | 2:1 | 1:1 | 1:2 | 1:5 | 1:10 | 1:100 |
| B-55 | A | and | Spinetoram | 100:1 | 10:1 | 5:1 | 2:1 | 1:1 | 1:2 | 1:5 | 1:10 | 1:100 |
| B-56 | A | and | Spinosad | 100:1 | 10:1 | 5:1 | 2:1 | 1:1 | 1:2 | 1:5 | 1:10 | 1:100 |
| B-57 | A | and | Spirodiclofen | 100:1 | 10:1 | 5:1 | 2:1 | 1:1 | 1:2 | 1:5 | 1:10 | 1:100 |
| B-58 | A | and | Spiromesifen | 100:1 | 10:1 | 5:1 | 2:1 | 1:1 | 1:2 | 1:5 | 1:10 | 1:100 |
| | | | | | | | | | | | | |
| B-59 | A | and | Spirotetramat | 100:1 | 10:1 | 5:1 | 2:1 | 1:1 | 1:2 | 1:5 | 1:10 | 1:100 |
| B-59a | A | and | Sulfoxaflor | 100:1 | 10:1 | 5:1 | 2:1 | 1:1 | 1:2 | 1:5 | 1:10 | 1:100 |
| B-60 | A | and | Tebufenozide | 100:1 | 10:1 | 5:1 | 2:1 | 1:1 | 1:2 | 1:5 | 1:10 | 1:100 |
| | | | | | | | | | | | | |
| B-60a | A | and | Tefluthrin | 100:1 | 10:1 | 5:1 | 2:1 | 1:1 | 1:2 | 1:5 | 1:10 | 1:100 |
| B-61 | A | and | Thiacloprid | 100:1 | 10:1 | 5:1 | 2:1 | 1:1 | 1:2 | 1:5 | 1:10 | 1:100 |
| B-62 | A | and | Thiamethoxam | 100:1 | 10:1 | 5:1 | 2:1 | 1:1 | 1:2 | 1:5 | 1:10 | 1:100 |
| B-63 | A | and | Thiodicarb | 100:1 | 10:1 | 5:1 | 2:1 | 1:1 | 1:2 | 1:5 | 1:10 | 1:100 |
| B-64 | A | and | Thiosultap-sodium | 100:1 | 10:1 | 5:1 | 2:1 | 1:1 | 1:2 | 1:5 | 1:10 | 1:100 |
| B-65 | A | and | Tolfenpyrad | 100:1 | 10:1 | 5:1 | 2:1 | 1:1 | 1:2 | 1:5 | 1:10 | 1:100 |
| B-66 | A | and | Tralomethrin | 100:1 | 10:1 | 5:1 | 2:1 | 1:1 | 1:2 | 1:5 | 1:10 | 1:100 |
| B-67 | A | and | Triazamate | 100:1 | 10:1 | 5:1 | 2:1 | 1:1 | 1:2 | 1:5 | 1:10 | 1:100 |
| B-68 | A | and | Triflumuron | 100:1 | 10:1 | 5:1 | 2:1 | 1:1 | 1:2 | 1:5 | 1:10 | 1:100 |

(continued)

| Mixture No. | Cmpd. **1** Form | and | Invertebrate Pest Control Agent | Typical Mixture Ratios (by weight) | | | | | | | | |
|---|---|---|---|---|---|---|---|---|---|---|---|---|
| B-69 | A | and | *Bacillus thuringiensis* | 100:1 | 10:1 | 5:1 | 2:1 | 1:1 | 1:2 | 1:5 | 1:10 | 1:100 |
| B-70 | A | and | *Bacillus thuringiensis* delta-endotoxin | 100:1 | 10:1 | 5:1 | 2:1 | 1:1 | 1:2 | 1:5 | 1:10 | 1:100 |
| B-71 | A | and | NPV (e.g., Gemstar) | 100:1 | 10:1 | 5:1 | 2:1 | 1:1 | 1:2 | 1:5 | 1:10 | 1:100 |

Listed below in Table C are embodiments of specific compositions comprising a solid form of Compound 1 ({drug1}) (polymorph Form A ({drug1a})) and an additional fungicide.

Table C

| Mixture No. | Cmpd. **1** Form | and | Fungicide | Typical Mixture Ratios (by weight) | | | | | | | | |
|---|---|---|---|---|---|---|---|---|---|---|---|---|
| C-1 | A | and | Probenazole | 100:1 | 10:1 | 5:1 | 2:1 | 1:1 | 1:2 | 1:5 | 1:10 | 1:100 |
| C-2 | A | and | Tiadinil | 100:1 | 10:1 | 5:1 | 2:1 | 1:1 | 1:2 | 1:5 | 1:10 | 1:100 |
| C-3 | A | and | Isotianil | 100:1 | 10:1 | 5:1 | 2:1 | 1:1 | 1:2 | 1:5 | 1:10 | 1:100 |
| C-4 | A | and | Pyroquilon | 100:1 | 10:1 | 5:1 | 2:1 | 1:1 | 1:2 | 1:5 | 1:10 | 1:100 |
| C-5 | A | and | Metominostrobin | 100:1 | 10:1 | 5:1 | 2:1 | 1:1 | 1:2 | 1:5 | 1:10 | 1:100 |
| C-6 | A | and | Flutolanil | 100:1 | 10:1 | 5:1 | 2:1 | 1:1 | 1:2 | 1:5 | 1:10 | 1:100 |
| C-7 | A | and | Validamycin | 100:1 | 10:1 | 5:1 | 2:1 | 1:1 | 1:2 | 1:5 | 1:10 | 1:100 |
| C-8 | A | and | Furametpyr | 100:1 | 10:1 | 5:1 | 2:1 | 1:1 | 1:2 | 1:5 | 1:10 | 1:100 |
| C-9 | A | and | Pencycuron | 100:1 | 10:1 | 5:1 | 2:1 | 1:1 | 1:2 | 1:5 | 1:10 | 1:100 |
| C-10 | A | and | Simeconazole | 100:1 | 10:1 | 5:1 | 2:1 | 1:1 | 1:2 | 1:5 | 1:10 | 1:100 |
| C-11 | A | and | Orysastrobin | 100:1 | 10:1 | 5:1 | 2:1 | 1:1 | 1:2 | 1:5 | 1:10 | 1:100 |
| C-12 | A | and | Trifloxystrobin | 100:1 | 10:1 | 5:1 | 2:1 | 1:1 | 1:2 | 1:5 | 1:10 | 1:100 |
| C-13 | A | and | Isoprothiolane | 100:1 | 10:1 | 5:1 | 2:1 | 1:1 | 1:2 | 1:5 | 1:10 | 1:100 |
| C-14 | A | and | Azoxystrobin | 100:1 | 10:1 | 5:1 | 2:1 | 1:1 | 1:2 | 1:5 | 1:10 | 1:100 |
| | | | | | | | | | | | | |
| C-15 | A | and | Tricyclazole | 100:1 | 10:1 | | 2:1 | 1:1 | 1:2 | 1:5 | 1:10 | 1:100 |
| C-16 | A | and | Hexaconazole | 100:1 | 10:1 | 5:1 | 2:1 | 1:1 | 1:2 | 1:5 | 1:10 | 1:100 |
| C-17 | A | and | Difenoconazole | 100:1 | 10:1 | 5:1 | 2:1 | 1:1 | 1:2 | 1:5 | 1:10 | 1:100 |
| C-18 | A | and | Cyproconazole | 100:1 | 10:1 | 5:1 | 2:1 | 1:1 | 1:2 | 1:5 | 1:10 | 1:100 |
| C-19 | A | and | Propiconazole | 100:1 | 10:1 | 5:1 | 2:1 | 1:1 | 1:2 | 1:5 | 1:10 | 1:100 |
| C-20 | A | and | Fenoxanil | 100:1 | 10:1 | 5:1 | 2:1 | 1:1 | 1:2 | 1:5 | 1:10 | 1:100 |
| C-21 | A | and | Ferimzone | 100:1 | 10:1 | 5:1 | 2:1 | 1:1 | 1:2 | 1:5 | 1:10 | 1:100 |
| C-22 | A | and | Fthalide | 100:1 | 10:1 | 5:1 | 2:1 | 1:1 | 1:2 | 1:5 | 1:10 | 1:100 |
| C-23 | A | and | Kasugamycin | 100:1 | 10:1 | 5:1 | 2:1 | 1:1 | 1:2 | 1:5 | 1:10 | 1:100 |
| C-24 | A | and | Picoxystrobin | 100:1 | 10:1 | 5:1 | 2:1 | 1:1 | 1:2 | 1:5 | 1:10 | 1:100 |
| C-25 | A | and | Penthiopyrad | 100:1 | 10:1 | 5:1 | 2:1 | 1:1 | 1:2 | 1:5 | 1:10 | 1:100 |
| C-26 | A | and | Famoxadone | 100:1 | 10:1 | 5:1 | 2:1 | 1:1 | 1:2 | 1:5 | 1:10 | 1:100 |
| C-27 | A | and | Cymoxanil | 100:1 | 10:1 | 5:1 | 2:1 | 1:1 | 1:2 | 1:5 | 1:10 | 1:100 |
| C-28 | A | and | Proquinazid | 100:1 | 10:1 | 5:1 | 2:1 | 1:1 | 1:2 | 1:5 | 1:10 | 1:100 |
| C-29 | A | and | Flusilazole | 100:1 | 10:1 | 5:1 | 2:1 | 1:1 | 1:2 | 1:5 | 1:10 | 1:100 |
| C-30 | A | and | Mancoeb | 100:1 | 10:1 | 5:1 | 2:1 | 1:1 | 1:2 | 1:5 | 1:10 | 1:100 |
| | | | | | | | | | | | | |
| C-31 | A | and | Copper hydroxide | 100:1 | 10:1 | 5:1 | 2:1 | 1:1 | 1:2 | 1:5 | 1:10 | 1:100 |
| C-32 | A | and | Fluopyram | 100:1 | 10:1 | 5:1 | 2:1 | 1:1 | 1:2 | 1:5 | 1:10 | 1:100 |
| C-33 | A | and | (a) | 100:1 | 10:1 | 5:1 | 2:1 | 1:1 | 1:2 | 1:5 | 1:10 | 1:100 |

(a) 1-[4-[4-[5-(2,6-difluorophenyl)-4,5-dihydro-3-isoxazolyl]-2-thiazolyl]-1-piperidinyl]-2-[5-methyl-3- (trifluoromethyl)- 1 - pyrazol-1-yljethanone: Parasitic nematodes are controlled in agronomic and nonagronomic applications by applying a solid form of Compound 1 ({drug1}), typically in the form of a composition, in a biologically effective amount, to the environment of the pests, including the agronomic and/or nonagronomic locus of infestation, to the area to be protected, or directly on the pests to be controlled. Thus the present invention comprises a method for controlling a parasitic nematode in agronomic and/or nonagronomic applications, comprising contacting the parasitic nematode or its environment with a biologically effective amount of a solid form of Compound 1 ({drug1}) or with a composition comprising at least one such compound or a composition comprising at least one such compound and at least one additional biologically active compound or agent. Examples of suitable compositions comprising a solid form of Compound land at least one additional biologically active compound or agent include granular compositions wherein the additional active compound is present on the same granule as the compound of the invention or on granules separate from those of the compound of the invention. To achieve contact with a solid form of Compound 1 ({drug1}) or composition of the invention to protect a field crop from parasitic nematodes, the solid form of Compound 1 ({drug1}) or composition is typically applied to the seed of the crop before planting, to the foliage (e.g., leaves, stems, flowers, fruits) of crop plants, or to the soil or other growth medium before or after the crop is planted. One embodiment of a method of contact is by spraying. Alternatively, a granular composition comprising a compound of the invention can be applied to the plant foliage or the soil. Solid forms of Compound 1 ({drug1}) can also be effectively delivered through plant uptake by contacting the plant with a composition comprising a compound of this invention applied as a soil drench of a liquid formulation, a granular formulation to the soil, a nursery box treatment or a dip of transplants. Of note is a composition of the present invention in the form of a soil drench liquid formulation. Also of note is a method for controlling a parasitic nematode comprising contacting the parasitic nematode or its environment with a biologically effective amount of a solid form of Compound 1 ({drug1}) or with a composition comprising a biologically effective amount of a solid form of Compound 1 ({drug1}). Of further note is this method wherein the environment is soil and the composition is applied to the soil as a soil drench formulation. Of further note is that solid forms of Compound 1 ({drug1}) are also effective by localized application to the locus of infestation. Other methods of contact include application of a solid form of Compound 1 ({drug1}) or a composition of the invention by direct and residual sprays, aerial sprays, gels, seed coatings, microencapsulations, systemic uptake, baits, ear tags, boluses, foggers, fumigants, aerosols, dusts and many others. One embodiment of a method of contact involves a dimensionally stable fertilizer granule, stick or tablet comprising a solid form of Compound 1 ({drug1}) or composition of the invention. The solid forms of Compound 1 ({drug1}) can also be impregnated into materials for fabricating invertebrate control devices (e.g., insect netting). Solid forms of Compound 1 ({drug1}) are also useful in seed treatments for protecting seeds from parasitic nematodes. In the context of the present disclosure and claims, treating a seed means contacting the seed with a biologically effective amount of a solid form of Compound 1 ({drug1}) which is typically formulated as a composition of the invention. This seed treatment protects the seed from invertebrate soil pests and generally can also protect roots and other plant parts in contact with the soil of the seedling developing from the germinating seed. The seed treatment may also provide protection of foliage by translocation of Compound 1 ({drug1}) or a second active ingredient within the developing plant. Seed treatments can be applied to all types of seeds, including those from which plants genetically transformed to express specialized traits will germinate. Representative examples of genetically transformed plants include those expressing proteins toxic to parasitic nematodes, such as Bacillus thuringiensis toxin or those expressing herbicide resistance such as glyphosate acetyltransferase, which provides resistance to glyphosate. Seed treatments with solid forms of Compound 1 ({drug1}) can also increase vigor of plants growing from the seed. One method of seed treatment is by spraying or dusting the seed with a solid form of Compound 1 ({drug1}) (i.e. as a formulated composition) before sowing the seeds. Compositions formulated for seed treatment generally comprise a film former or adhesive agent. Therefore typically a seed coating composition of the present invention comprises a biologically effective amount of a solid form of Compound 1 ({drug1}) and a film former or adhesive agent. Seed can be coated by spraying a flowable suspension concentrate directly into a tumbling bed of seeds and then drying the seeds. Alternatively, other formulation types such as wetted powders, solutions, suspoemulsions, emulsifiable concentrates and emulsions in water can be sprayed on the seed. This process is particularly useful for applying film coatings on seeds. Various coating machines and processes are available to one skilled in the art. Suitable processes include those listed in P. Kosters et al, Seed Treatment: Progress and Prospects, 1994 BCPC Mongraph No. 57, and references listed therein. Solid forms of Compound 1 ({drug1}) and their compositions, both alone and in combination with other insecticides, nematicides, and fungicides, are particularly useful in seed treatment for crops including, but not limited to, maize or corn, soybeans, cotton, cereal (e.g., wheat, oats, barley, rye and rice), potatoes, vegetables and oilseed rape. Other insecticides or nematicides with which solid forms of Compound 1 ({drug1}) can be formulated to provide mixtures useful in seed treatment include but are not limited to abamectin, acetamiprid, acrinathrin, amitraz, avermectin, azadirachtin, bensultap, bifenthrin, buprofezin, cadusafos, carbaryl, carbofuran, cartap, chlorantraniliprole, chlorfenapyr, chlorpyrifos, clothianidin, cyantraniliprole, cyfluthrin, beta-cyfluthrin, cyhalothrin, gamma-cyhalothrin, lambda-cyhalothrin, cypermethrin, alpha-cypermethrin, zeta-cypermethrin, cyromazine, deltamethrin, dieldrin, dinotefuran, diofenolan, emamectin, endosulfan, esfenvalerate, ethiprole, etofenprox, etoxazole, fenothiocarb,

fenoxycarb, fenvalerate, fipronil, flonicamid, flubendiamide, flufenoxuron, fluvalinate, formetanate, fosthiazate, hexaflumuron, hydramethylnon, imidacloprid, indoxacarb, lufenuron, metaflumizone, methiocarb, methomyl, methoprene, methoxyfenozide, nitenpyram, nithiazine, novaluron, oxamyl, pymetrozine, pyrethrin, pyridaben, pyridalyl, pyriproxyfen, ryanodine, spinetoram, spinosad, spirodiclofen, spiromesifen, spirotetramat, sulfoxaflor, tebufenozide, tetramethrin, thiacloprid, thiamethoxam, thiodicarb, thiosultap-sodium, tralomethrin, triazamate, triflumuron, Bacillus thuringiensis delta-endotoxins, all strains of Bacillus thuringiensis and all strains ofNucleo polyhydrosis viruses. Fungicides with which solid forms of Compound 1 ({drug1}) can be formulated to provide mixtures useful in seed treatment include but are not limited to amisulbrom, azoxystrobin, boscalid, carbendazim, carboxin, cymoxanil, cyproconazole, difenoconazole, dimethomorph, fluazinam, fludioxonil, fluquinconazole, fluopicolide, fluoxastrobin, flutriafol, fluxapyroxad, ipconazole, iprodione, metalaxyl, mefenoxam, metconazole, myclobutanil, paclobutrazole, penflufen, picoxystrobin, prothioconazole, pyraclostrobin, sedaxane, silthiofam, tebuconazole, thiabendazole, thiophanate-methyl, thiram, trifloxystrobin and triticonazole. Compositions comprising solid forms of Compound 1 ({drug1}) useful for seed treatment can further comprise bacteria and fungi that have the ability to provide protection from the harmful effects of plant pathogenic fungi or bacteria and/or soil born animals such as nematodes. Bacteria exhibiting nematicidal properties may include but are not limited to Bacillus firmus, Bacillus cereus, Bacillius subtiliis and Pasteuria penetrans. A suitable Bacillus firmus strain is strain CNCM 1-1582 (GB-126) which is commercially available as BioNem™. A suitable Bacillus cereus strain is strain NCMM 1-1592. Both Bacillus strains are disclosed in US 6,406,690. Other suitable bacteria exhibiting nematicidal activity are B. amyloliquefaciens IN937a and B. subtilis strain GB03. Bacteria exhibiting fungicidal properties may include but are not limited to B. pumilus strain GB34. Fungal species exhibiting nematicidal properties may include but are not limited to Myrothecium verrucaria, Paecilomyces lilacinus and Purpureocillium lilacinum. Seed treatments can also include one or more nematicidal agents of natural origin such as the elicitor protein called harpin which is isolated from certain bacterial plant pathogens such as Erwinia amylovora. An example is the Harpin-N-Tek seed treatment technology available as N-Hibit™ Gold CST. Seed treatments can also include one or more species of legume-root nodulating bacteria such as the microsymbiotic nitrogen-fixing bacteria Bradyrhizobium japonicum. These inocculants can optionally include one or more lipo-chitooligosaccharides (LCOs), which are nodulation (Nod) factors produced by rhizobia bacteria during the initiation of nodule formation on the roots of legumes. For example, the Optimize® brand seed treatment technology incorporates LCO Promoter Technology™ in combination with an inocculant. Seed treatments can also include one or more isoflavones which can increase the level of root colonization by mycorrhizal fungi. Mycorrhizal fungi improve plant growth by enhancing the root uptake of nutrients such as water, sulfates, nitrates, phosphates and metals. Examples of isoflavones include, but are not limited to, genistein, biochanin A, formononetin, daidzein, glycitein, hesperetin, naringenin and pratensein. Formononetin is available as an active ingredient in mycorrhizal inocculant products such as PHC Colonize® AG. Seed treatments can also include one or more plant activators that induce systemic acquired resistance in plants following contact by a pathogen. An example of a plant activator which induces such protective mechanisms is acibenzolar-S-methyl. The treated seed typically comprises a solid form of Compound 1 ({drug1}) in an amount from about 0.1 g to 1 kg per 100 kg of seed (i.e. from about 0.0001 to 1% by weight of the seed before treatment). A flowable suspension formulated for seed treatment typically comprises from about 0.5 to about 70% of the active ingredient, from about 0.5 to about 30% of a film-forming adhesive, from about 0.5 to about 20% of a dispersing agent, from 0 to about 5% of a thickener, from 0 to about 5% of a pigment and/or dye, from 0 to about 2% of an antifoaming agent, from 0 to about 1% of a preservative, and from 0 to about 75% of a volatile liquid diluent. The solid forms of Compound 1 ({drug1}) are also suitable for treatment of plant propagation material other than seed, such as fruit, tubers or plant seedlings. The propagation material can be treated with the compounds before planting, or the compounds can be applied to the planting site when the propagation material is being planted.

For agronomic applications, the rate of application required for effective control (i.e. "biologically effective amount") will depend on such factors as the species of nematode to be controlled, the nematode's life cycle, life stage, its size, location, time of year, host crop or animal, feeding behavior, mating behavior, ambient moisture, temperature, and the like. Under normal circumstances, application rates of about 0.01 to 2 kg of active ingredients per hectare are sufficient to control nematodes in agronomic ecosystems, but as little as 0.0001 kg/hectare may be sufficient or as much as 8 kg/hectare may be required. For nonagronomic applications, effective use rates will range from about 1.0 to 50 mg/square meter but as little as 0.1 mg/square meter may be sufficient or as much as 150 mg/square meter may be required. One skilled in the art can easily determine the biologically effective amount necessary for the desired level of parasitic nematode control.

The invention relates to an administration unit comprising polymorph A of 8-chloro-N-[(2-chloro-5-methoxyphenyl)sulfonyl]-6-(trifluoromethyl)-imidazo[1,2-alpha]pyridine-2-carboxamide. The administration unit according to the invention is useful for treating various diseases and disorders which include but are not limited to as nematicide, insecticide and/or fungicide, preferred as nematicide to control (including prevention, reduction or elimination) parasitic nematodes.

SPECIFIC INORMATION CONCERNING ASPECT (II) OF THE INVENTION

Aspect (ii) of the invention concerns forms of {drug2}, especially (1aR,12bS)-8-cyclohexyl-11-fluoro-N-((1-methylcyclopropyl)sulfonyl)-1a-((3-methyl-3,8-diazabicyclo[3.2.1]oct-8-yl)carbonyl)-1,1a,2,12b-tetrahydrocyclopropa-[d]indolo[2,1-alpha][2]benzazepine-5-carboxamide (Compound 2, formula II, {drug2}). The invention relates to a form of compound of formula II ({drug2}) and/or pharmaceutically acceptable salts thereof, namely to solid form of compound of formula II ({drug2}) and/or pharmaceutically acceptable salts thereof, which is useful for treating those infected with HCV and/or for treatment of hepatitis C. Compound of formula II and/or pharmaceutically acceptable salts thereof and / or solid form of compound of formula II ({drug2}) and/or pharmaceutically acceptable salts thereof is described in WO/2013/059265, which is incorporated by reference. Compound of formula II and/or pharmaceutically acceptable salts thereof is named (1aR,12bS)-8-cyclohexyl-11-fluoro-N-((1-methylcyclopropyl)sulfonyl)-1a-((3-methyl-3,8-diazabicyclo[3.2.1]oct-8-yl)carbonyl)-1,1a,2,12b-tetrahydrocyclopropa[d]indolo[2,1-alpha][2]benzazepine-5-carboxamide (Compound 2, formula II, {drug2}) and is represented by:

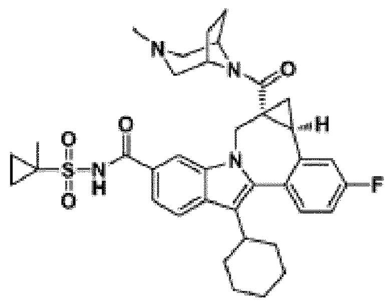

The invention relates to an administration unit comprising compound of formula II ({drug2}) and/or pharmaceutically acceptable salts thereof and / or solid form of compound of formula II ({drug2}) and/or pharmaceutically acceptable salts thereof ({drug2a}). The administration unit according to the invention is useful for treating various diseases and disorders which include but are not limited to those infected with HCV and/or for treatment of hepatitis C. Preferred embodiments of the administration unit are (as described in WO/2013/059265 and cited here):

The disclosure relates to (1aR,12bS)-8-cyclohexyl-11-fluoro-N-((1-methylcyclopropyl)sulfonyl)-1a-((3-methyl-3,8-diaza-bicyclo[3.2.1]oct-8-yl)carbonyl)-1,1a,2,12b-tetrahydrocyclopropa[d]indolo[2,1-a][2]benzazepine-5-carboxamide (Compound 2, formula II, {drug2}), including pharmaceutically acceptable salts, as well as compositions and methods of using the compound. The compound has activity against hepatitis C virus (HCV) and may be useful in treating those infected with HCV. Hepatitis C virus (HCV) is a major human pathogen, infecting an estimated 170 million persons worldwide. Hepatitis C virus (HCV) is the most common bloodbome infection in the USA and worldwide and is the leading cause of liver transplantation (Eric Chak et.al. Liver International 2011, 1090-1 101) A substantial fraction of these HCV infected individuals develop serious progressive liver disease, including cirrhosis and hepatocellular carcinoma (Lauer, G. M.; Walker, B. D. N. Engl. J. Med. 2001, 345, 41-52). HCV is a positive-stranded RNA virus. Considerable heterogeneity is found within the nucleotide and encoded amino acid sequence throughout the HCV genome. At least six major genotypes have been characterized, and more than 50 subtypes have been described adding to the need for new therapies. The genome consists of approximately 9500 nucleotides and has a single open reading frame (ORF) encoding a single large polyprotein of about 3000 amino acids. In infected cells, this polyprotein is cleaved at multiple sites by cellular and viral proteases to produce the structural and non-structural (NS) proteins. The NS proteins (NS3, NS4A, NS4B, NS5A, and NS5B) are required for viral RNA replication. NS3 is a serine protease that mediates cleavage of the polyprotein. The NS4A protein is a cofactor for the NS3 protease. The complex formation of the NS3 protein with NS4A seems necessary to the processing events, enhancing the proteolytic efficiency at all of the sites. The NS3 protein also exhibits nucleoside triphosphatase and RNA helicase activities. NS5B (also referred to as HCV polymerase) is a RNA-dependent RNA polymerase that is involved in the replication of HCV. The NS5B RNA-dependent RNA polymerase (RdRp) is essential to the replication cycle of HCV (Tomei L, Altamura S, Paonessa G, et al. Antivir Chem Chemother 2005, 16, 225-245.). The HCV NS5B protein is described in "Structural Analysis of the Hepatitis C Virus RNA Polymerase in Complex with Ribonucleotides (Bressanelli; S. et al, Journal of Virology 2002, 3482-3492; and Defrancesco and Rice, Clinics in Liver Disease 2003, 7, 21 1-242. The NS5B crystal structure reveals a typical right-handed polymerase containing thumb, palm and finger domains surrounding the active site. (Lesburg C.A., Cable, M.B., Ferrari, et al. Nat Struc Biol 1999, 6, 937-943.) NS5B is the catalytic enzyme responsible for RNA replication and participates in higher order complexes at intracellular lipid membranes in association with various viral proteins and nucleic acids as well as host proteins. (El Hage

N and Luo G. J Gen Virol 2003, 84, 2761-2769. Gao L, Aizaki H, He JW, et al. J Virol 2004,75, 3480-3488) Examples of NS5B protein-protein interactions include binding to the NS3 helicase domain, facilitating RNA unwinding, and binding to the NS5A protein, a regulator of viral replication. (Jennings, et al. Biochemistry 2008,^7, 1126-1135. McCormick CJ, Brown D, Griffin S, et al. J Gen Virol 2006, 87(Pt 1), 93-102.) HCV NS5B polymerase inhibitors can be divided into two classes based on their mode of inhibition: nucleoside (NUC) inhibitors compete with natural substrates and non-nucleoside inhibitors (NNI) are non-competitive allosteric inhibitors. Both NUC inhibitors and NNI have clinical proof of principal of antiviral activity via inhibition of the NS5B target (Gelman, MA. and JS. Glenn (201 1) Mixing the right hepatitis C inhibitor cocktail. Trends in Molecular Medicine 17: 1, 34-46; Soriano V., E. Vispo, E. Poveda, P. Labarga, L. Martin- Carbonero, JV. Femandez-Montero and P. Barreiro (201 1) Directly acting antivirals against hepatitis C virus. J Antimicrob Chemother 66, 1673-1686). I prevent conformational transitions of the polymerase that are required for the initiation of RNA synthesis (Ma FL, V. Leveque, A. De Witte, W. Li, T. Hendricks, SM. Clausen, N. Cammack, K. Klumpp. (2005) Inhibition of native hepatitis C virus replicase by nucleotide and non-nucleoside inhibitors. Virology 332, 8 -15). Co-crystals of NNI show that they bind to one of at least three distinct sites on the polymerase, consistent with the diverse patterns of resistance observed for these inhibitors in vitro and in vivo (Beaulieu, P. (2009) Recent advances in the development of NS5B polymerase inhibitors for the treatment of hepatitis C virus infection. Expert Opinion on Therapeutic Patents. 19, 145-64). These studies substantiate observations in the HCV RNA replicon system in which inhibition of NS5B blocks viral replication. (Tomei L, Altamura S, Paonessa G, et al. Antivir Chem Chemother 2005, 16, 225-245.). Previously, the most effective HCV therapy employed a combination of alpha-interferon and ribavirin, leading to sustained efficacy in only 40% of genotype 1 patients (Poynard, T. et al. Lancet 1998, 352, 1426-1432). Clinical results demonstrate that pegylated alpha-interferon is superior to unmodified alpha-interferon as monotherapy (Zeuzem, S. et al. N. Engl. J. Med. 2000, 343, 1666-1672). However, even with experimental therapeutic regimens involving combinations of pegylated alpha-interferon and ribavirin, a substantial fraction of patients did not have a sustained reduction in viral load. In 2011, improved therapies for genotype 1 patients that include an HCV NS3 protease inhibitor, a small molecule direct acting antiviral (DAA), plus interferon and ribavirin were approved by FDA. Two protease inhibitors (INCrVEK™ (Telaprevir) and VICTRELIS™ (Boceprevir) were approved; thus a choice of drug exists for combination therapy with interferon and ribavirin. (Ghany, MG., DR. Nelson, DB. Strader, DL. Thomas, and LB. Seeff. (2011) An Update on Treatment of Genotype 1 Chronic Hepatitis C Virus Infection: 201 1 Practice Guideline by the American Association for the Study of Liver Diseases. Hepatology, 54(4): 1433-1444). Currently, significant research efforts are focused on further improvement of cure rates, by improving tolerability, addressing the needs of patients whose virus or genetic markers make their disease less responsive to interferon based therapy, and shortening duration of therapy. Interferons with fewer side effects and interferon free regimens of small molecule DAA combinations are being tested. Because of the rapid replication rate and development of resistance by HCV, it is believed that treatment regimens will necessarily be combinations of agents.

Hepatitis C infected patients typically have a long (>10 years) asymptomatic phase of disease that occurs before substantial hepatic injury and symptoms are manifested. For this reasons, HCV infected individuals initially maintain a high quality of life or may not even know they are infected. Since all currently approved treatments include interferon and ribavirin which are associated with serious side effects, and since the recently approved protease inhibitors are associated with additional side effects (rash and anemia), many HCV infected patients choose to delay therapy until more acceptable regimens, expected within this decade, are approved. In the future, agents that have actual or perceived serious liabilities, such as risk for causing cardiovascular events or severe hepatotoxicity, will not be widely utilized for therapy. Thus, current research is focused on the development of safe and effective inhibitor combinations that can deliver a cure for HCV infection in the absence of interferon. Considerable efforts aimed at identifying direct acting antiviral agents which inhibit Hepatitis C virus replication have been disclosed in the art. (Gelman, MA. and JS. Glenn (2011) Mixing the right hepatitis C inhibitor cocktail. Trends in Molecular Medicine 17: 1, 34-46; Soriano V., E. Vispo, E. Poveda, P. Labarga, L. Martin-Carbonero, JV. Fernandez-Montero and P. Barreiro (2011) Directly acting antivirals against hepatitis C virus. J Antimicrob Chemother 66, 1673-1686. The general methodology used by pharmaceutical companies to identify compounds that have the potential to be used in the treatment of HCV in human patients is similar to the methodology applied to other drug discovery targets. Initial assessment of potency vs. the therapeutic target (in this case the NS5B enzyme targeted for inhibition of hepatitis C) is done with enzyme and cell based assays. Compounds with acceptable potency are profiled in additional in vitro assays to assess their suitability for achieving good pharmacokinetic (PK) profiles in animal models (rodent or higher species). Examples are (i) in vitro assays to assess metabolic stability in the presence of microsomal membranes prepared from liver cells of human and other species, and (ii) permeability assay systems such as Caco-2 or PAMPA to assess the potential for absorption. In vitro assays such as general cytotoxicity and cytochrome P450 enzyme inhibition (indicates the potential for drug-drug interactions) are also used to assess potential safety liabilities. One serious liability which all drug discovery programs have developed in vitro strategies to avoid is the prolongation of myocardial repolarization and lengthening the QT interval on the electrocardiogram, as these properties have been associated with an increased risk for the development of life-threatening ventricular arrhythmias and death. Compounds with this liability would obviously not be useful for the treatment of Hepatitis C. In almost every case, drugs that increase the QT interval also block a specific potassium channel [human Ether-a-go-go Related Gene (hERG)] in in vitro assays. The prolongation of repolarization is of particular importance because it has been associated with an increased risk for the subsequent development of malignant ventricular arrhythmias and death. In the presence of prolonged myocardial repolarization, some individuals may develop a distinct form of ventricular tachycardia known as torsades de pointes. The development of such drug related new or worsened ventricular arrhythmias is termed proarrhythmia. Routine in vitro assays include hERG potassium ion channel assays (in silico, high-throughput flux and patch-clamp electrophysiology) and a Purkinje fiber action potential assay. The hERG screens will identify compounds that likely will affect the cardiac rapidly activating delayed rectifier potassium current (IKr). Most drugs that prolong cardiac repolarization do so by blocking this current. For compounds in clinical development where therapeutic exposures are known, experts have estimated that a margin of 30-fold or greater between hERG IC50 and the therapeutic Cmax of compound not bound to protein could be sufficient for safety from hERG-mediated arrhythmias associated with QTc prolongation; although Redfem et al. suggest that increasing the margin even further would be prudent. (Redfern, W. S.; Carlsson, L.; Davis, A. S.; Lynch, W. G.; MacKenzie, I.; Palethorpe, S.; Siegl, P. K. S.; Strang, I.; Sullivan, A. T.; Wallis, R.; Camm, A. J.; Hammond, T. G. Relationships between preclinical cardiac electrophysiology, clinical QT interval prolongation and torsade de pointes for a broad range of drugs: evidence for a provisional safety margin in drug development. Cardiovascular Research (2003), 58(1), 32-45. De Bruin, M. L.; Pettersson, M.; Meyboom, R. H. B.; Hoes, A. W.; Leufkens, H. G. M. Anti-HERG activity and the risk of drug-induced arrhythmias and sudden death. European Heart Journal (2005), 26(6), 590-597). For the assessment of risk in preclinical programs, some guidelines have been developed by ICH (International Conference On Harmonisation Of Technical Requirements For Registration Of Pharmaceuticals For Human Use), and these are also provided on the FDA web site for guidance to industry. An excerpt of a pertinent section (2.2) on profiling preclinical compounds follows: 2.2. ICH S7B strategy: The chemical class of drug candidates determines the preclinical safety strategy. The golden standard for the in vitro IKr assay is the test for HERG interaction by means of patch-clamp studies. The in vivo telemetry assay allows the study of QT interval with integrated risk assessment. For in vitro cardiac action potential duration (APD) studies multicellular preparation from animal heart is needed to study potential adverse effects of drug candidates on the whole concert of cardiac voltage-gated ion channels. For in vitro assessment the gold standard (patch clamp assay) is also used to meet FDA regulatory recommendations (hERG assay); however, this assay is low through-put and in silico and high-throughput flux (flipr) assays are used for initial screening of greater numbers of compounds. Flux results (flipr) are validated with the patch clamp assay. Because in vivo assessments such as telemetry in animals are so labor intensive and costly, they are employed exclusively for compounds of interest with respect to the entire compound profile. These are compounds for which in vitro assays suggest there is a high likelihood they could continue to advance if profiled further. Alternatively, compounds used to provide a benchmark to validate in vitro assays may be assessed in vivo. This holds true for advanced in vitro APD studies as well. Purkinje fiber testing is also low-throughput and complements the hERG assay by assessing all of the major ionic currents which contribute to the cardiac action potential. Signals for effects on other cardiac ionic currents can be detected in this action potential assay and followed-up in patch-clamp studies on candidate cardiac ion channels (e.g. Na, Ca or other K channels such as Iks). A number of compounds which are inhibitors of HCV NS5B are in clinical development or have advanced to clinical studies and been discontinued for various reasons. More specific to this application, HCV NS5B inhibitors which bind to a site referred to in the art as Site 1 have been disclosed in U.S. Patents 7,399,758, 7,485,633 and published U.S. patent application 2009130057. The novel compound of the present invention which falls within the definition of Formula I in US application publication 2009130057 is not disclosed or described in that application. Surprisingly, it has been discovered that (2R)-2-[[(4-chlorophenyl)sulfonyl][[2-fluoro-4-(1,2,4-oxadiazol-3-yl)phenyl]methyl]amino]-5,5,5-trifluoropentanamide possesses unique attributes which make it useful for the treatment of hepatitis C.

The present invention relates to (1aR,12bS)-8-cyclohexyl-11-fluoro-N-((1-methylcyclopropyl)sulfonyl)-1a-((3-methyl-3,8-diazabicyclo[3.2.1]oct-8-yl)carbonyl)-1,1a,2,12b-tetrahydrocyclopropa[d]indolo[2,1-a][2]benzazepine-5-carboxamide having the Formula II, its pharmaceutical formulations, and its use in treating hepatitis C.

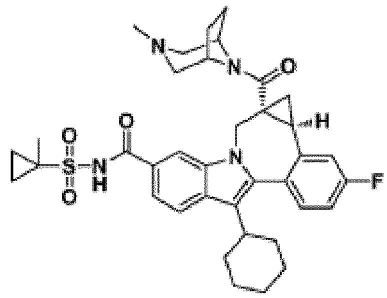

(II)

One aspect of the invention is the compound (1aR,12bS)-8-cyclohexyl-11-fluoro-N-((1-methylcyclopropyl)sulfonyl)-la-((3-methyl-3,8-diazabicyclo[3.2.1]oct-8-yl)carbonyl)-1,1a,2,12b-tetrahydrocyclopropa[d]indolo[2,1-a][2]benzazepine-5-carboxamide, or a pharmaceutically acceptable salt thereof.

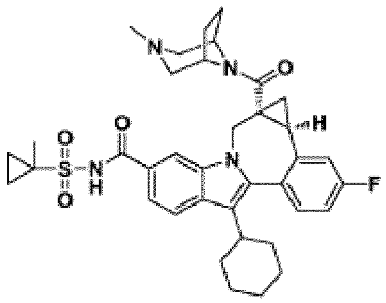

Another aspect of the invention is a pharmaceutical composition comprising a therapeutically effective amount of (1aR,12bS)-8-cyclohexyl-11-fluoro-N-((1-methylcyclopropyl)sulfonyl)-1a-((3-methyl-3,8-diazabicyclo[3.2.1]oct-8-yl)carbonyl)-1,1a,2,12b-tetrahydrocyclopropa[d]indolo[2,1-a][2]benzazepine-5-carboxamide, or a pharmaceutically acceptable salt thereof, and a pharmaceutically acceptable adjuvant, carrier or diluent. Another aspect of the invention is a composition comprising a therapeutically effective amount of (1aR,12bS)-8-cyclohexyl-11-fluoro-N-((1-methylcyclopropyl)sulfonyl)-1a-((3-methyl-3,8-diazabicyclo[3.2.1]oct-8-yl)carbonyl)-1,1a,2,12b-tetrahydrocyclopropa[d]indolo[2,1-a][2]benzazepine-5-carboxamide and a compound having anti-HCV activity. Another aspect of the invention is a composition comprising a therapeutically effective amount of (1aR,12bS)-8-cyclohexyl-11-fluoro-N-((1-methylcyclopropyl)sulfonyl)-1a-((3-methyl-3,8-diazabicyclo[3.2.1]oct-8-yl)carbonyl)-1,1a,2,12b-tetrahydrocyclopropa[d]indolo[2,1-a][2]benzazepine-5-carboxamide where the compound having anti-HCV activity is an interferon. Another aspect of the invention is where the interferon is selected from interferon alpha 2B, pegylated interferon alpha, consensus interferon, interferon alpha 2A, and lymphoblastoid interferon tau. Another aspect of the invention is a composition comprising a therapeutically effective amount of (1aR,12bS)-8-cyclohexyl-11-fluoro-N-((1-methylcyclopropyl)sulfonyl)-1a-((3-methyl-3,8-diazabicyclo[3.2.1]oct-8-yl)carbonyl)-1,1a,2,12b-tetrahydrocyclopropa[d]indolo[2,1-a][2]benzazepine-5-carboxamide where the compound having anti-HCV activity is a cyclosporin. Another aspect of the invention is where the cyclosporin is cyclosporin A. Another aspect of the invention is a composition comprising a therapeutically effective amount of (1aR,12bS)-8-cyclohexyl-11-fluoro-N-((1-methylcyclopropyl)sulfonyl)-1a-((3-methyl-3,8-diazabicyclo[3.2.1]oct-8-yl)carbonyl)-1,1a,2,12b-tetrahydrocyclopropa-[d]indolo[2,1-a][2]benzazepine-5-carboxamide where the compound having anti-HCV activity is selected from the group consisting of interleukin 2, interleukin 6, interleukin 12, a compound that enhances the development of a type 1 helper T cell response, interfering RNA, anti-sense RNA, Imiqimod, ribavirin, an inosine 5'-monophospate dehydrogenase inhibitor, amantadine, and rimantadine. Another aspect of the invention is a composition comprising a therapeutically effective amount of (1aR,12bS)-8-cyclohexyl-11-fluoro-N-((1-methylcyclopropyl)sulfonyl)-1a-((3-methyl-3,8-diazabicyclo[3.2.1]oct-8-yl)carbonyl)-1,1a,2,12b-tetrahydrocyclopropa[d]indolo[2,1-a][2]benzazepine-5-carboxamide where the compound having anti-HCV activity is effective to inhibit the function of a target selected from HCV metalloprotease, HCV serine protease, HCV polymerase, HCV helicase, HCV NS4B protein, HCV entry, HCV assembly, HCV egress, HCV NS5A protein, IMPDH, and a nucleoside analog for the treatment of an HCV infection. Another aspect of the invention is a composition comprising a therapeutically effective amount of (1aR,12bS)-8-cyclohexyl-11-fluoro-N-((1-methylcyclopropyl)sulfonyl)-1a-((3-methyl-3,8-diazabicyclo[3.2.1]oct-8-yl)carbonyl)-1,1a,2,12b-tetrahydrocyclopropa[d]indolo[2,1-a][2]benzazepine-5-carboxamide, or a pharmaceutically acceptable salt thereof, a pharmaceutically acceptable carrier, an interferon and ribavirin. Another aspect of the invention

is a method of treating an HCV infection in a patient comprising administering to the patient a therapeutically effective amount of (1aR,12bS)-8-cyclohexyl-11-fluoro-N-((1-methylcyclopropyl)sulfonyl)-1a-((3-methyl-3,8-diazabicyclo[3.2.1]oct-8-yl)carbonyl)-1,1a,2,12b-tetrahydrocyclopropa[d]indolo[2,1-a][2]benzazepine-5-carboxamide, or a pharmaceutically acceptable salt thereof. In another embodiment the compound is effective to inhibit the function of the HCV replicon. In another embodiment the compound is effective to inhibit the function of the HCV NS5B protein. Another aspect of the invention is a method of treating an HCV infection in a patient comprising administering to the patient a therapeutically effective amount of a compound, or (1aR,12bS)-8-cyclohexyl-11-fluoro-N-((1-methylcyclopropyl)sulfonyl)-1a-((3-methyl-3,8-diazabicyclo[3.2.1]oct-8-yl)carbonyl)-1,1a,2,12b-tetrahydrocyclopropa[d]indolo[2,1-a][2]benzazepine-5-carboxamide, in conjunction with (prior to, after, or concurrently) another compound having anti-HCV activity. Another aspect of the invention is the method where the other compound having anti-HCV activity is an interferon. Another aspect of the invention is the method where the interferon is selected from interferon alpha 2B, pegylated interferon alpha, consensus interferon, interferon alpha 2A, and lymphoblastoid interferon tau. Another aspect of the invention is the method where the other compound having anti-HCV activity is a cyclosporin. Another aspect of the invention is the method where the cyclosporin is cyclosporin A. Another aspect of the invention is the method where the other compound having anti-HCV activity is selected from interleukin 2, interleukin 6, interleukin 12, a compound that enhances the development of a type 1 helper T cell response, interfering RNA, anti-sense RNA, Imiqimod, ribavirin, an inosine 5'-monophospate dehydrogenase inhibitor, amantadine, and rimantadine. Another aspect of the invention is the method where the other compound having anti-HCV activity is effective to inhibit the function of a target selected from the group consisting of HCV metalloprotease, HCV serine protease, HCV polymerase, HCV helicase, HCV NS4B protein, HCV entry, HCV assembly, HCV egress, HCV NS5A protein, IMPDH, and a nucleoside analog for the treatment of an HCV infection. Another aspect of the invention is the method where the other compound having anti-HCV activity is effective to inhibit the function of target in the HCV life cycle other than the HCV NS5B protein. The invention includes all pharmaceutically acceptable salt forms of the compound. Pharmaceutically acceptable salts are those in which the counter ions do not contribute significantly to the physiological activity or toxicity of the compounds and, as such, function as pharmacological equivalents. These salts can be made according to common organic techniques employing commercially available reagents. Some anionic salt forms include acetate, acistrate, besylate, bromide, camsylate, chloride, citrate, fumarate, glucouronate, hydrobromide, hydrochloride, hydroiodide, iodide, lactate, maleate, mesylate, nitrate, pamoate, phosphate, succinate, sulfate, tartrate, tosylate, and xinofoate. Some cationic salt forms include ammonium, aluminum, benzathine, bismuth, calcium, choline, diethylamine, diethanolamine, lithium, magnesium, meglumine, 4-phenylcyclohexylamine, piperazine, potassium, sodium, tromethamine, and zinc. As the compound of the present invention possesses asymmetric carbon atoms, the present invention includes stereoisomeric forms of the compound of Formula II. The use of a single designation such as (R) or (S) is intended to include mostly one stereoisomer. Mixtures of isomers can be separated into individual isomers according to known methods, e.g. fractional crystallization, adsorption chromatography or other suitable separation processes. Resulting racemates can be separated into antipodes in the usual manner after introduction of suitable salt-forming groupings, e.g. by forming a mixture of diastereosiomeric salts with optically active salt-forming agents, separating the mixture into diastereomeric salts and converting the separated salts into the free compounds. The enantiomeric forms may also be separated by fractionation through chiral high pressure liquid chromatography columns. The invention is intended to include all isotopes of atoms occurring in the present compounds. Isotopes include those atoms having the same atomic number but different mass numbers. By way of general example and without limitation, isotopes of hydrogen include deuterium and tritium. Isotopes of carbon include 13C and 14C. Isotopically-labeled compounds of the invention can generally be prepared by conventional techniques known to those skilled in the art or by processes analogous to those described herein, using an appropriate isotopically-labeled reagent in place of the non-labeled reagent otherwise employed. Such compounds may have a variety of potential uses, for example as standards and reagents in determining biological activity. In the case of stable isotopes, such compounds may have the potential to favorably modify biological, pharmacological, or pharmacokinetic properties. "Therapeutically effective" means the amount of agent required to provide a meaningful patient benefit as understood by practitioners in the field of hepatitis and HCV infection. "Patient" means a person infected with the HCV virus and suitable for therapy as understood by practitioners in the field of hepatitis and HCV infection. "Treatment," "therapy," "regimen," "HCV infection," and related terms are used as understood by practitioners in the field of hepatitis and HCV infection. The above therapeutic agents, when employed in combination with the compound of the present invention, may be used, for example, in those amounts indicated in the Physician's Desk Reference (PDR), where applicable or as otherwise determined by one of ordinary skill in the art. However, it will be understood that the amount of the compound actually administered will be determined by a physician, in the light of the relevant circumstances including the condition to be treated, the choice of compound to be administered, the chosen route of administration, the age, weight, and response of the individual patient, and the severity of the patient's symptoms. For therapeutic use, the pharmacologically active compound of Formula I will normally be administered as a pharmaceutical composition comprising as the (or an) essential active ingredient at least one such compound in association with a solid or liquid pharmaceutically acceptable carrier and, optionally, with pharmaceutically acceptable adjuvants and excipients employing standard and conventional

techniques. The pharmaceutical compositions include suitable dosage forms for oral, parenteral (including subcutaneous, intramuscular, intradermal and intravenous), transdermal, sublingual, bronchial or nasal administration. Thus, if a solid carrier is used, the preparation may be tableted, placed in a hard gelatin capsule in powder or pellet form, or in the form of a troche or lozenge. The solid carrier may contain conventional excipients such as binding agents, fillers, tableting lubricants, disintegrants, wetting agents and the like. The tablet may, if desired, be film coated by conventional techniques. Oral preparations include push-fit capsules made of gelatin, as well as soft, scaled capsules made of gelatin and a coating, such as glycerol or sorbitol. Push- fit capsules can contain active ingredients mixed with a filler or binders, such as lactose or starches, lubricants, such as talc or magnesium stearate, and, optionally, stabilizers. In soft capsules, the active compounds may be dissolved or suspended in suitable liquids, such as fatty oils, liquid, or liquid polyethylene glycol with or without stabilizers. If a liquid carrier is employed, the preparation may be in the form of a syrup, emulsion, soft gelatin capsule, sterile vehicle for injection, an aqueous or nonaqueous liquid suspension, or may be a dry product for reconstitution with water or other suitable vehicle before use. Liquid preparations may contain conventional additives such as suspending agents, emulsifying agents, wetting agents, non-aqueous vehicle (including edible oils), preservatives, as well as flavoring and/or coloring agents. For parenteral administration, a vehicle normally will comprise sterile water, at least in large part, although saline solutions, glucose solutions and like may be utilized. Injectable suspensions also may be used, in which case conventional suspending agents may be employed. Conventional preservatives, buffering agents and the like also may be added to the parenteral dosage forms. For topical or nasal administration, penetrants or permeation agents thatare appropriate to the particular barrier to be permeated are usedin the formulation. Such penetrants are known in the art. The pharmaceutical compositions are prepared by conventional techniques appropriate to the desired preparation containing appropriate amounts of the active ingredient, that is, the compound of Formula I according to the invention. See, for example, Remington 's Pharmaceutical Sciences, Mack Publishing Company, Easton, PA, 17th edition, 1985. The invention also encompasses methods where the compound is given in combination therapy. That is, the compound can be used in conjunction with, but separately from, other agents useful in treating hepatitis and HCV infection. In these combination methods, the compound will generally be given in a daily dose of 1-100 mg/kg body weight in conjunction with other agents. The other agents generally will be given in the amounts used therapeutically. The specific dosing regime, however, will be determined by a physician using sound medical judgement.

Some examples of compounds suitable for compositions and methods are listed in Table 1.

Table 1:

| *Brand Name* | *Physiological Class* | *Type of Inhibitor or Target* | *Source Company* |
|---|---|---|---|
| NIM811 | | Cyclophilin Inhibitor | Novartis |
| Zadaxin | | Immuno-modulator | Sciclone |
| Suvus | | Methylene blue | Bioenvision |
| Actilon (CPG10101) | | TLR9 agonist | Coley |
| Batabulin (T67) | Anticancer | β-tubulin inhibitor | Tularik Inc., South San Francisco, CA |
| ISIS 14803 | Antiviral | antisense | ISIS Pharmaceuticals Inc, Carlsbad, CA/Elan Phamaceuticals Inc., New York, NY |
| | | | |
| Summetrel | Antiviral | antiviral | Endo Pharmaceuticals Inc., Chadds Ford, PA |
| GS-9132 (ACH-806) | Antiviral | HCV Inhibitor | Achillion / Gilead |
| Pyrazolopyrimidine compounds and salts From WO-2005047288 26 May 2005 | Antiviral | HCV Inhibitors | Arrow Therapeutics Ltd. |
| Levovirin | Antiviral | IMPDH inhibitor | Ribapharm Inc., Costa Mesa, CA |
| Merimepodib (VX-497) | Antiviral | IMPDH inhibitor | Vertex Pharmaceuticals Inc., Cambridge, MA |

(continued)

| *Brand Name* | *Physiological Class* | *Type of Inhibitor or Target* | *Source Company* |
|---|---|---|---|
| XTL-6865 (XTL-002) | Antiviral | monoclonal antibody | XTL Biopharmaceutica is Ltd., Rehovot, Isreal |
| Telaprevir (VX-950, LY-570310) | Antiviral | NS3 serine protease inhibitor | Vertex Pharmaceuticals Inc., Cambridge MA/ Eli Lilly and Co. Inc., Indianapolis, IN |
| HCV-796 | Antiviral | NS5B Replicase inhibitor | Wyeth / Viropharma |
| NM-283 | Antiviral | NS5B Replicase Inhibitor | Idenix / Novartis |
| | | | |
| GL-59728 | Antiviral | NS5B Replicase Inhibitor | Gene Labs / Novartis |
| GL-60667 | Antiviral | NS5B Replicase Inhibitor | Gene Labs / Novartis |
| 2'C MeA | Antiviral | NS5B Replicase Inhibitor | Gilead |
| PSI 6130 | Antiviral | NS5B Replicase Inhibitor | Roche |
| R1626 | Antiviral | NS5B Replicase Inhibitor | Roche |
| 2'C Methyl adenosine | Antiviral | NS5B Replicase Inhibitor | Merck |
| JTK-003 | Antiviral | RdRp inhibitor | Japan Tobacco Inc., Tokyo, Japan |
| | | | |
| Levovirin | Antiviral | ribavirin | ICN Pharmaceuticals, Costa Mesa, CA |
| Ribavirin | Antiviral | ribavirin | Schering-Plough Corporation, Kenilworth, NJ |
| Viramidine | Antiviral | Ribavirin Prodrug | Ribapharm Inc., Costa Mesa, CA |
| Heptazyme | Antiviral | ribozyme | Ribozyme Pharmaceuticals Inc., Boulder, CO |
| BILN-2061 | Antiviral | serine protease inhibitor | Boehringer Ingelheim Pharma KG, Ingelheim, Germany |
| SCH 503034 | Antiviral | serine protease inhibitor | Schering Plough |
| Zadazim | Immune modulator | Immune modulator | SciClone Pharmaceuticals Inc., San Mateo, CA |
| Ceplene | Immunomodulator | immune modulator | Maxim Pharmaceuticals Inc., San Diego, CA |
| CellCept | Imnunosuppressant | HCV IgG immuno-suppressant | F. Hoffmann-La Roche LTD, Basel, Switzerland |

(continued)

| *Brand Name* | *Physiological Class* | *Type of Inhibitor or Target* | *Source Company* |
|---|---|---|---|
| Civacir | Immunosuppressant | HCV IgG immuno-suppressant | Nabi Biopharmaceutica is Inc., Boca Raton, FL |
| | | | |
| Albuferon - α | Interferon | albumin IFN-α2b | Human Genome Sciences Inc., Rockville, MD |
| Infergen A | Interferon | IFN alfacon-1 | InterMune Pharmaceuticals Inc., Brisbane, CA |
| Omega IFN | Interferon | IFN-ω | Intarcia Therapeutics |
| IFN-β and EMZ701 | Interferon | IFN-β and EMZ701 | Therapeutics Inc., Ontario, Canada |
| Rebif | Interferon | IFN-β1a | Serono, Geneva, Switzerland |
| Roferon A | Interferon | IFN-α2a | F. Hoffmann-La Roche LTD, Basel, Switzerland |
| | | | |
| Intron A | Interferon | TFN-α2b | Schering-Plough Corporation, Kenilworth, NJ |
| Intron A and Zadaxin | Interferon | IFN-α2b/α1-thymosin | RegeneRx Biopharma. Inc., Bethesda, MD/ SciClone Pharmaceuticals Inc, San Mateo, CA |
| Rebetron | Interferon | IFN-α2b/ribavirin | Schering-Plough Corporation, Kenilworth, NJ |
| Actimmune | Interferon | INF-γ | InterMune Inc., Brisbane, CA |
| Interferon-β | Interferon | Interferon-β-1a | Serono |
| Multiferon | Interferon | Long lasting IFN | Viragen/ Valentis |
| Wellferon | Interferon | Lympho-blastoid IFN-αn1 | GlaxoSmithKline plc, Uxbridge, UK |
| Omniferon | Interferon | natural IFN-α | Viragen Inc., Plantation, FL |
| Pegasys | Interferon | PEGylated IFN-α2a | F. Hoffmann-La Roche LTD, Basel, Switzerland |
| Pegasys and Ceplene | Interferon | PEGylated IFN-α2a/ immune modulator | Maxim Pharmaceuticals Inc., San Diego, CA |
| Pegasys and Ribavirin | Interferon | PEGylated IFN-α2a/ribavirin | F. Hoffmann-La Roche LTD, Basel, Switzerland |
| | | | |
| PEG-Intron | Interferon | PEGylated IFN-α2b | Schering-Plough Corporation, Kenilworth, NJ |
| PEG-Intron / Ribavirin | Interferon | PEGylaled IFN-α2b/ribavirin | Schering-Plough Corporation, Kenilworth, NJ |
| IP-501 | Liver protection | antifibrotic | Indevus Pharmaceuticals Inc., Lexington, MA |

(continued)

| *Brand Name* | *Physiological Class* | *Type of Inhibitor or Target* | *Source Company* |
|---|---|---|---|
| IDN-6556 | Liver protection | caspase inhibitor | Idun Pharmaceuticals Inc., San Diego, CA |
| | | | |
| ITMN-191 (R-7227) | Antiviral | serine protease inhibitor | Pharmaceuticals Inc., Brisbane, CA |
| GL-59728 | Antiviral | NS5B Replicase Inhibitor | Genelabs |
| ANA-971 | Antiviral | TLR-7 agonist | Anadys |
| Boceprevir | Antiviral | serine protease inhibitor | Schering Plough |
| TMS-435 | Antiviral | serine protease inhibitor | Tibotec BVBA, Mechelen, Belgium |
| BI-201335 | Antiviral | serine protease inhibitor | Boehringer Ingelheim Pharma KG, Ingelheim, Germany |
| MK-7009 | Antiviral | serine protease inhibitor | Merck |
| PF-00868554 | Antiviral | replicase inhibitor | Pfizer |
| ANA598 | Antiviral | Non-Nucleoside NS5B Polymerase Inhibitor | Anadys Pharmaceuticals, Inc., San Diego, CA, USA |
| IDX375 | Antiviral | Non-Nucleoside Replicase Inhibitor | Idenix Pharmaceuticals, Cambridge, MA, USA |
| BILB 1941 | Antiviral | NS5B Polymerase Inhibitor | Boehringer Ingelheim Canada Ltd R&D, Laval, QC, Canada |
| PSI-7851 | Antiviral | Nucleoside Polymerase | Inhibitor Pharmasset, Princeton, NJ, USA |
| | | | |
| PSI-7977 | Antiviral | Nucleotide NS5B Polymerase Inhibitor | Pharmasset, Princeton, NJ, USA |
| VCH-759 | Antiviral | NS5B Polymerase Inhibitor | ViroChem Pharma |
| VCH-916 | Antiviral | NS5B Polymerase Inhibitor | ViroChem Pharma |
| GS-9190 | Antiviral | NS5B Polymerase Inhibitor | Gilead |
| Peg-interferon lamda | Antiviral | Interferon | ZymoGenetics/Br istol-Myers Squibb |

Synthetic Methods: A route to prepare Compound 2 is shown in Scheme 1. This route utilizes Bayliss-Hillman and palladium cross coupling chemistry approaches. A Bayliss-Hillman reaction provided the unsaturated ester. DABCO or (4s)-quinuclidin-3-ol can be utilized in this initial step as the base. The hydroxy was acetylated to provide the acetate. Conjugate addition of the indole under basic conditions provided the precurser to cyclization, which is the substrate for a palladium cross coupling reaction which gave the cycloheptene. Use of dimethyl sulfoxonium ylide provided the racemic cyclopropane. This compound was resolved via SFC chiral chromatography. This separation was amenable to larger

scales such as >100g. The tert-butyl ester was treated with TFA in CH2Cl2 to give the acid, which was isolated as crude product after removal of CH2Cl2 and TFA. The crude acid was coupled with bis-HCl salt of (1R,5S)-3-methyl-3,8-diazabicyclo[3.2.1]octane in CH2Cl2 in the presence of HATU and Hunig's base to afford the amide after purification via column chromatography. Hydrolysis with LiOH in a mixture of MeOH and THF yielded penultimate compound after extractive work-up and trituration with CH2Cl2. The final coupling of the acid with 1-methylcyclopropane-1-sulfonamide facilitated by EDC and DMAP afforded Compound 2. The material obtained by column chromatography purification contained ~ 3% (¾(¾ residual solvent which was difficult to remove under high vacuum at 80 °C. CH2Cl2 was removed by repeatedly dissolving the API in MeOH and evaporating, and final high vacuum drying at 60 °C for 24 h. The intermediate 1-methylcyclopropane-1-sulfonamide was prepared based on previously reported method (Synlett 2006, 5, 725-278) (Scheme 2). N-(tert-butyl)-1-methylcyclopropane-1 -sulfonamide was synthesized using a one-pot procedure from N-(tert-butyl)-3-chloropropane-1 - sulfonamide through n-BuLi promoted intramolecular cyclization, lithiation and alkylation with methyl iodide. The removal of the Boc protecting group with TFA afforded 1-methylcyclopropane-1-sulfonamide.

Scheme 1:

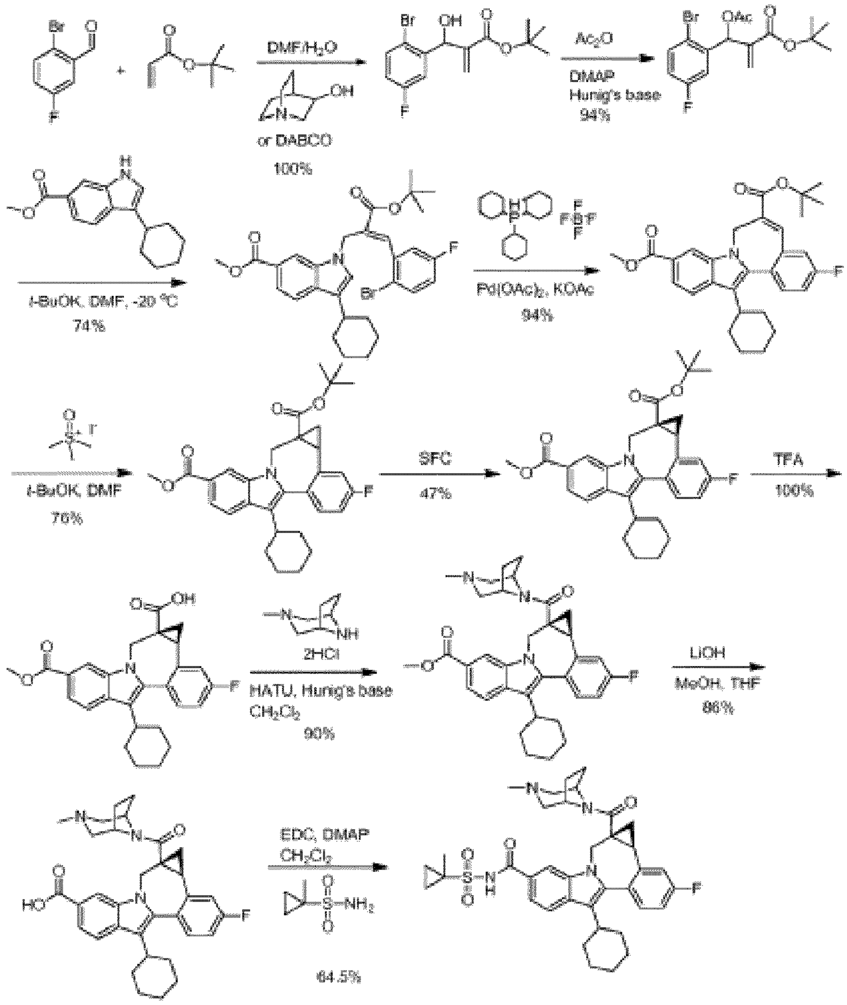


Scheme 2:

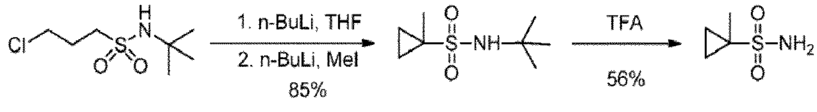

Preparation 1:

tert-butyl 2-((2-bromo-5-fluorophenyl)(hydroxy)methyl)acrylate

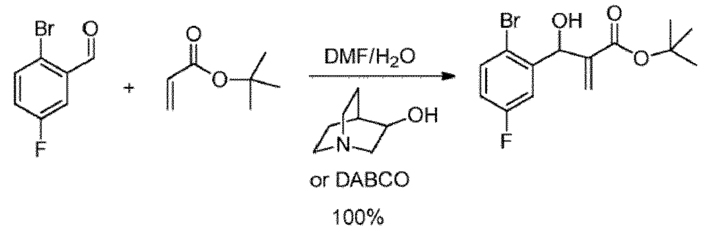


In a 1 L flask was added 2-bromo-5-fluorobenzaldehyde (10.0 g, 49.3 mmol), (4s)-quinuclidin-3-ol (6.26 g, 49.3 mmol) in DMF (28 mL) and water (12 mL). To this reaction mixture was added tert-butyl acrylate (14.3 mL, 99.0 mmol). The mixture was then stirred at rt for 19h. At this time LCMS indicated consumption of starting materials. The reaction mixture was diluted with ether (500 mL) and the layers were separated. The aqueous layer was extracted twice with ether (200 mL each). The combined organic phases were washed with 0.1N HCl, water and brine. It was then dried over MgS04, filtered and concentrated to yield 18.0 g (>100%) of the product as a yellow oil. 1H NMR (400 MHz, CDC13) $\delta$ 7.53 (dd, J=8.8, 5.3 Hz, 1H), 7.32 (dd, J=9.7, 3.1 Hz, 1H), 6.93 (ddd, J=8.7, 7.7, 3.1 Hz, 1H), 6.29 (s, 1H), 5.85 (s, 1H), 5.52 (t, J=1.1 Hz, 1H), 1.50 (s, 9H). LCMS: (RT = 2.06 min), Column: PHENOMENEX-LUNA 2.0 x 30mm (3$\mu$m), Mobile Phase: gradient 0-100% B, Solvent A (90% Water : 10% Methanol: 0.1% TFA), Solvent B (10% Water :90% Methanol: 0.1% TFA), Flow rate: 1.0 mL/min; (M+H+23) = 355.09. Alternate Preparation. This procedure uses DABCO as a more economical substitute for (4s)-quinuclidin-3-ol. A I L flask was charged with 2-bromo-5-fluorobenzaldehyde (13.4 g, 66.0 mmol), DABCO (14.81 g, 132 mmol), DMF (53.6 mL) and water (13.4 mL). To this reaction mixture was added tert-butyl acrylate (12.68g, 99.0 mmol). The mixture was then stirred at rt for 12h. At this time TLC indicated consumption of starting material. The reaction mixture was diluted with 25 mL of water and then extracted with two 100 mL portions of MTBE. The combined organic phases were washed with 0.1N HCl, water and brine. It was then dried over MgS04, filtered and concentrated to yield 25 g (>100%) of the product as a colorless oil which was used without further purification.

Preparation 2:

tert-butyl 2-(acetoxy(2-bromo-5-fluorophenyl)methyl)acrylate

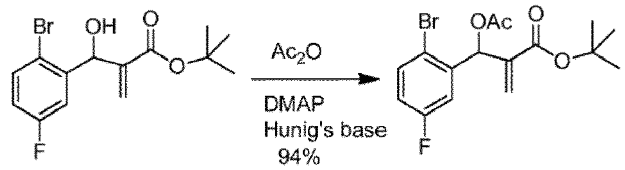


To a mixture of the hydroxy starting material (18 g, 54.4 mmol) in DCM (500 mL) was added acetic anhydride (6.15 mL, 65.2 mmol), Hunig's base (12.3 mL, 70.7 mmol) and DMAP (0.066 g, 0.54 mmol). This mixture was stirred at rt for 2h and then concentrated in vacuo. The residue was diluted with ether (500mL) and washed with 0. IN HCl, water and brine. The organic phase was then dried over MgS04, filtered and concentrated in vacuo to yield 19 g (94%) of the acetate as yellow oil. 1H NMR (400 MHz, CDC13) $\delta$ 7.57 (dd, J=8.8, 5.3 Hz, 1H), 7.09 (dd, J=9.3, 3.0 Hz, 1H), 6.96 (ddd, J=8.8, 7.8, 3.0 Hz, 1H), 6.92 (d, J=0.8 Hz, 1H), 6.45-6.41 (m, 1H), 5.54-5.49 (m, 1H), 2.18-2.15 (s, 3H), 1.47-1.43 (s, 9H). LCMS: (RT = 1.46 min), Column: PHENOMENEX-LUNA 2.0 x 30mm (3$\mu$m), Mobile Phase: gradient 60-100% B, Solvent A (90% Water : 10% Methanol: 0.1% TFA), Solvent B (10% Water :90% Methanol: 0.1% TFA), Flow rate: 1.0 mL/min; (M+H+23) = 397.08. Preparation 3:

(E)-methyl 1-(3-(2-bromo-5-fluorophenyl)-2-(tert-butoxycarbonyl)allyl)-3-cyclohexyl-1H-indole-6-carboxylate

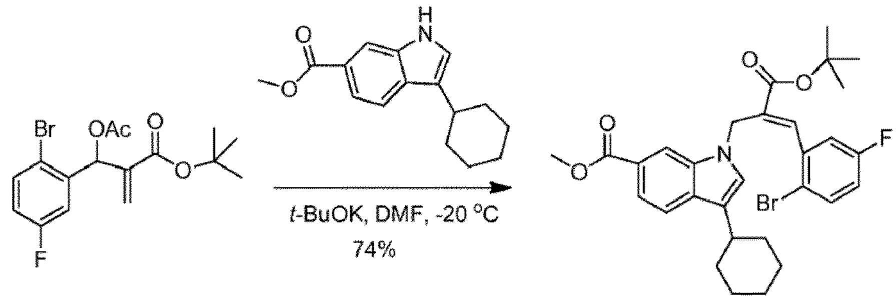


To a 500 mL flask was added the ester (4.27 g, 16.6 mmol) and DMF (100 mL). The mixture was stirred at rt until complete dissolution was achieved. Solid potassium tert-butoxide (16.6 mL, 16.6 mmol) was added over a period of 5 min. During this addition the colorless solution turned to a blue-yellow color. The reaction mixture was stirred at rt for 1h. It was then cooled to -47°C(acetonitrile/dry ice bath). A solution of tert-butyl 2-(acetoxy(2-bromo-5-fluorophenyl)methyl)acrylate (6.2 g, 16.61 mmol) in DMF (20mL) was added which caused the reaction to turn to a red color. The temperature was held at-40 to -45°C for 1h and then warmed to -20°C over a 2h period. The solution turned into a light tan color. The reaction mixture was carefully quenched with ice water maintaining the temperature below -15°C. The resulting mixture was diluted with EtOAc to provide an organic and aqueous phase. The organic layer was washed with water, dried over MgS04, filtered, and concentrated in vacuo to yield 8.0 g of the crude product as a light tan solid. It was then triturated with MeOH to isolate 7g (74%) of the product as a white solid. 1H NMR (400 MHz, CDC13) δ 7.78-7.83 (m, 2H), 7.75 (dd, J=1.25, 8.28 Hz, 1H), 7.58-7.64 (m, 2H), 6.89-7.00 (m, 3H), 5.02 (s, 2H), 3.94 (s, 3H), 2.73-2.85 (m, 1H), 2.04 (d, J=1 1.04 Hz, 2H), 1.75-1.90 (m, 3H), 1.38-1.50 (m, 5H), 1.37 (s, 9H). LCMS: (RT = 0.56 min), Column: PHENOMENEX-LU A 2.0 x 30mm (3μm), Mobile Phase: gradient 100% B, Solvent B (10% Water :90% Methanol: 0.1% TFA), Flow rate: 1.0 mL/min; (M+H) = 572.27.

Preparation 4:

10-methyl 6-(2-methyl-2-propanyl) 13-cyclohexyl-3-fluoro-7H-indolo[2,1-a][2]benzazepine-6,10-dicarboxylate

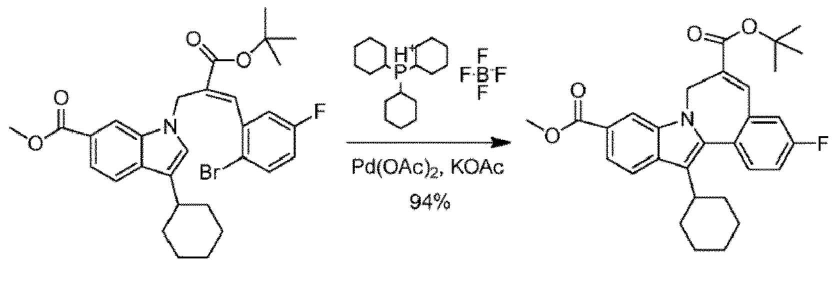


A mixture of starting material (13.0 g, 22.8 mmol), Pd(OAc)2 (0.256 g, 1.14 mmol), potassium acetate (7.83 g, 80.0 mmol), tricyclohexylphosphonium tetrafluoroborate (0.629 g, 1.71 mmol) in N,N-dimethylacetamide (50 mL) was placed under a N2 atmosphere (vacuum/N2 refill for 3 times). It was then heated at 122°C for 6h. The reaction mixture became dark yellow/brown and some black solid precipitate was present. It was cooled to rt and diluted with EtOAc (800 mL). The mixture was then washed with water (3x), dried over MgS04, filtered, and concentrated in vacuo to yield 12 g of a yellow solid. The solid was triturated with hexane to give 10.5 g (94%) of the product as a yellow solid. IH NMR (400 MHz, CDC13) δ 8.31 (d, J=1.0 Hz, IH), 7.90 (d, J=8.5 Hz, IH), 7.78 (dd, J=8.5, 1.5 Hz, IH), 7.74 (s, IH), 7.60 (dd, J=8.5, 5.8 Hz, IH), 7.24 (td, J=9.0, 2.8 Hz, 2H), 5.77-5.55 (m, IH), 4.15 (m, IH), 3.98 (s, 3H), 2.88-2.77 (m, IH), 2.19-1.33 (m, 10H), 1.66 (s, 9H). LCMS: (RT = 0.66 min), Column: PHENOMENEX-LUNA 2.0 x 30mm (3μm), Mobile Phase: gradient 100% B, Solvent B (10% Water :90% Methanol: 0.1% TFA), Flow rate: 1.0 mL/min; (M+H) = 490.35.

Preparation 5:

5-methyl 1a-(2-methyl-2-propanyl)(1a*R*,12b*S*)-8-cyclohexyl-11-fluoro-1,12b-dihydrocyclopropa[*d*]indolo[2,1-*a*][2]benzazepine-1a,5(2*H*)-dicarboxylate

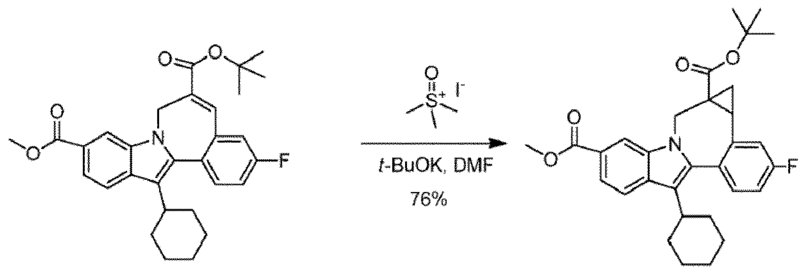


To a mixture trimethylsulfoxonium iodide (1.68 g, 7.64 mmol) in DMF (10 mL) was added potassium tert-butoxide (7.64 mL, 7.64 mmol). The mixture was stirred at rt for 1h. The starting material (3.4 g, 6.94 mmol) was added to the reaction mixture and stirring was continued for another 2h. A tan precipitate was formed. Water (20mL) was added to the reaction mixture and the resulting off-white solid was collected by filtration and washed with water. This crude product was dried in vacuo to obtain 2.9 g of an off-white solid which was triturated with hexane to give 2.7 g of the racemic diester the product as a white solid. 1H NMR (400 MHz, CDC13) (rotamers) δ 8.32 (d, J=1.0 Hz, 0.5H), 8.17 (s, 0.5H), 7.91-7.84 (m, 1H), 7.81-7.73 (m, 1H), 7.38-7.29 (m, 1.5H), 7.24 (dd, J=9.7, 2.6 Hz, 0.5H), 7.09 (tt, J=8.3, 2.5 Hz, 1H), 5.45 (d, J=15.6 Hz, 0.5H), 5.18 (d, J=15.3 Hz, 0.5H), 4.06 (d, J=15.1 Hz, 0.5H), 3.99 (s, 1.5H), 3.97 (s, 1.5H), 3.43 (d, J=15.1 Hz, 0.5H), 2.96-2.86 (m, 1H), 2.83-2.73 (m, 0.5H), 2.59 (dd, J=10.0, 6.8 Hz, 0.5H), 2.18-1.27 (m, 11H), 1.55(s, 4.5H), 1.31 (s, 4.5H), 1.16 (dd, J=6.0, 4.3 Hz, 0.5H), 0.37 (t, J=6.1 Hz, 0.5H). LCMS: (RT = 0.57 min), Column: PHENOMENEX-LUNA 2.0 x 30mm (3μm), Mobile Phase: gradient 100% B, Solvent B (10% Water :90% Methanol: 0.1% TFA), Flow rate: 1.0 mL/min; (M+H) = 504.39.

Preparation 6:

5-methyl 1a-(2-methyl-2-propanyl)(1a*R*,12b*S*)-8-cyclohexyl-11-fluoro-1,12b-dihydrocyclopropa[*d*]indolo[2,1-*a*][2]benzazepine-1a,5(2*H*)-dicarboxylate

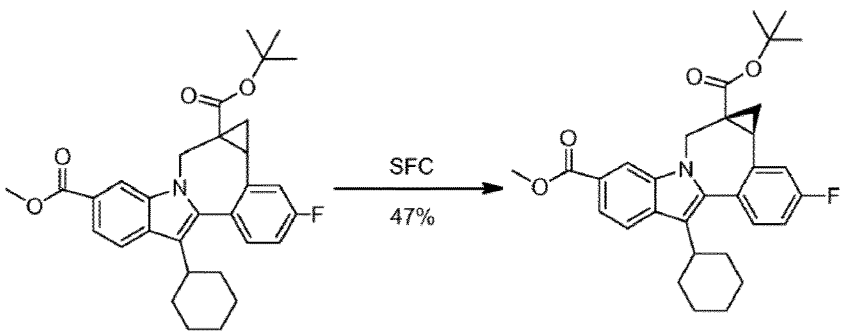


Racemic compound was chirally separated to yield 1.35 g as a white solid. The following conditions were used for the chiral separation and the faster eluting compound was the desired enantiomer. Column: ChiralPak AD-H, 30 x 250mm, 5mm; Mobile Phase: 20% MeOH / 80% C02; Pressure: 150 bar; Temperature: 35°C; Flow Rate: 70 mL/min; UV: 210 nm. 1H NMR (500 MHz, CDC13) (rotamers) δ 8.34 - 8.29 (m, 0.5H), 8.17 (s, 0.5H), 7.91-7.85 (m, 1H), 7.78 (dd, J=18.8, 1.4 Hz, 1H), 7.38-7.21 (m, 2H), 7.14-7.05 (m, 1H), 5.50-5.41 (m, 0.5H), 5.22-5.15 (m, 0.5H), 4.06 (d, J=15.3 Hz, 0.5H), 3.99 (s, 1.5H), 3.97 (s, 1.5H), 3.48-3.39 (m, 0.5H), 2.96-2.86 (m, 1H), 2.81-2.72 (m, 0.5H), 2.63-2.53 (m, 0.5H), 2.18-1.24 (m, 1 1H), 1.55 (s, 4.5H), 1.32 (s, 4.5H), 1.16 (dd, J=6.1, 4.3 Hz, 0.5H), 0.38 (t, J=6.1 Hz, 0.5H).

Preparation 7:

(1a*S*,12b*R*)-8-cyclohexyl-11-fluoro-5-(methoxycarbonyl)-1,12b-dihydrocyclopropa[*d*]indolo[2,1-*a*][2]benzazepine-1a(2*H*)-carboxylic acid

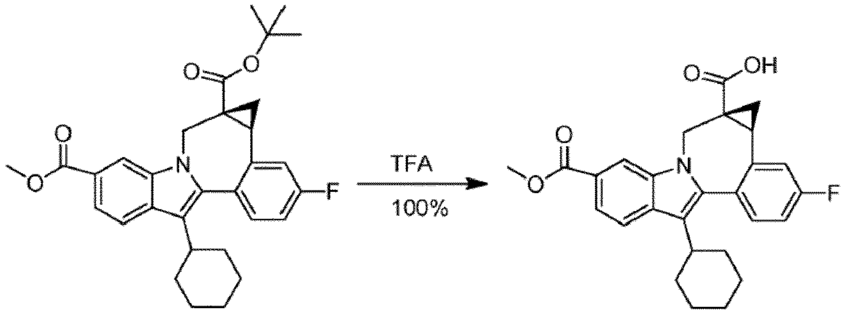


A mixture of starting material (700 mg, 1.39 mmol) and TFA (5 mL, 64.9 mmol) was stirred at rt for 3h. LCMS indicated complete consumption of the diester. The reaction mixture was then concentrated in vacuo to yield 630 mg(100%) of the product as an off-white solid. 1H NMR (400 MHz, CDC13)(rotamers) δ 8.41 (d, J=1.0 Hz, 0.6H), 8.16 (s, 0.4H), 7.91 (d, J=8.8 Hz, 0.4H), 7.85 (d, J=8.5 Hz, 0.6H), 7.75 (dd, J=8.4, 1.4 Hz, 0.4H), 7.67 (dd, J=8.4, 1.4 Hz, 0.6H), 7.49-7.34 (m, 2H), 7.25-7.13 (m, 1H), 5.50 (d, J=15.6 Hz, 0.6H), 5.28 (d, J=15.1 Hz, 0.4H), 4.05 (d, J=15.1 Hz, 0.4H), 3.96 (s, 1.2H), 3.95 (s, 1.8H), 3.52-3.45 (m, 0.6H), 3.00-2.74 (m, 2H), 2.23-1.23 (m, 11.6H), 0.24 (t, J=6.0 Hz, 0.4H); LCMS: (RT = 2.60 min), Column: PHENOMENEX-LU A 2.0 x 30mm (3μm), Mobile Phase: gradient 0-100% B, Solvent A (90% Water : 10% Methanol: 0.1% TFA), Solvent B (10% Water :90% Methanol: 0.1% TFA), Flow rate: 1.0 mL/min (M+H) = 448.29.

Preparation 8:

methyl (1a*R*,12b*S*)-8-cyclohexyl-11-fluoro-1a-((3-methyl-3,8-diazabicyclo[3.2.1]oct-8-yl)carbonyl)-1,1a,2,12b-tetrahydrocyclopropa[*d*]indolo[2,1-*a*][2]benzazepine-5-carboxylate

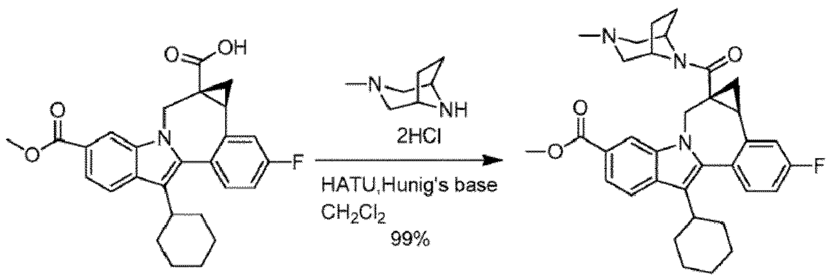


A mixture of starting acid (300 mg, 0.670 mmol), (1R,5S)-3-methyl-3,8-diazabicyclo[3.2.1]octane bishydrochloride (160 mg, 0.804 mmol), 2-(1H-benzo [d] [1,2,3]triazol- 1 -yl)- 1, 1,3,3 -tetramethylisouronium tetrafluoroborate (HATU)(258 mg, 0.804 mmol) and N-ethyl-N-isopropylpropan-2-amine(Hunig's base)(0.467 mL, 2.68 mmol) in DCM (4 mL) was stirred at rt for 2h. The reaction mixture was then concentrated in vacuo. The residue was diluted with EtOAc, washed with water(5X), dried(MgS04), filtered, and concentrated in vacuo to yield 370 mg (99%) of the product as a light yellow solid. 1H NMR (500 MHz, DMSO-d6) (rotamers) δ 8.17 (s, 0.35H), 8.03 (s, 0.65H), 7.93 (d, J=8.5 Hz, 0.35H), 7.89 (d, J=8.4 Hz, 0.65H), 7.69 (dd, J=8.5, 1.3 Hz, 0.35H), 7.62 (dd, J=8.5, 1.3 Hz, 0.65H), 7.51-7.25 (m, 3H), 5.12 (d, J=15.6 Hz, 0.65H), 4.94 (d, J=15.1 Hz, 0.35H), 4.32 (br s, 1H), 4.11 (d, J=15.3 Hz, 0.35H), 4.03-3.96 (m, 0.65H), 3.90 (s, 1H), 3.88 (s, 2H), 3.64 (br s, 0.35H), 3.61 (d, J=15.4 Hz, 0.65H), 2.94-2.84 (m, 1H), 2.79-1.05 (m, 24H). LCMS: (RT = 2.26 min), Column: PHENOMENEX-LUNA 2.0 x 30mm (3μm), Mobile Phase: gradient 0-100% B, Solvent A (90% Water : 10% Methanol: 0.1% TFA), Solvent B (10% Water :90% Methanol: 0.1% TFA), Flow rate: 1.0 mL/min; (M+H) = 556.50.

Preparation 9:

(1a*R*,12b*S*)-8-cyclohexyl-11-fluoro-1a-((3-methyl-3,8-diazabicyclo[3.2.1]oct-8-yl)carbonyl)-1,1a,2,12b-tetrahydrocyclopropa[*d*]indolo[2,1-*a*][2]benzazepine-5-carboxylic acid

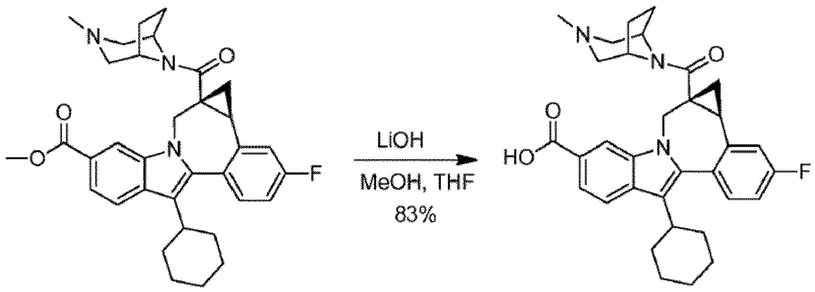


A mixture of the ester (400 mg, 0.720 mmol), IN NaOH (3.60 mL, 3.60 mmol) and MeOH (4 mL) was refluxed for 3h. It was then concentrated in vacuo. The residue was diluted with water and acidified with IN HCl until reaching pH=4. The white precipitate which formed was collected by filtration and washed with water. The white solid was dried to yield 360 mg(83%) of the product as an off-white solid. 1H NMR (500MHz, DMSO-d6)(rotamers) δ 8.16 (s, 0.35H), 8.02 (s, 0.65H), 7.90 (d, J=8.4 Hz, 0.35H), 7.86 (d, J=8.4 Hz, 0.65H), 7.69 (d, J=8.4 Hz, 0.35H), 7.63 (d, J=8.4 Hz, 0.65H), 7.51-7.38 (m, 2H), 7.34-7.24 (m, 1H), 5.15 (d, J=15.3 Hz, 0.65H), 4.99 (d, J=16.2 Hz, 0.35H), 4.56-4.40 (m, 1H), 4.18-4.06 (m,1H), 3.65-3.57 (m, 1H), 2.94-2.84 (m, 1H), 2.79-1.15(m, 24H). LCMS: (RT = 2.19 min), Column: PHENOMENEX-LUNA 2.0 x 30mm (3μm), Mobile Phase: gradient 0-100% B, Solvent A (90% Water : 10% Methanol: 0.1% TFA), Solvent B (10% Water :90% Methanol: 0.1% TFA), Flow rate: 1.0 mL/min; (M+H) = 542.52.

Preparation 10

(1a*R*,12b*S*)-8-cyclohexyl-11-fluoro-*N*-((1-methylcyclopropyl)sulfonyl)-1a-((3-methyl-3,8-diazabicyclo[3.2.1]oct-8-yl)carbonyl)-1,1a,2,12b-tetrahydrocyclopropa[d]indolo[2,1-*a*][2]benzazepine-5-carboxamide

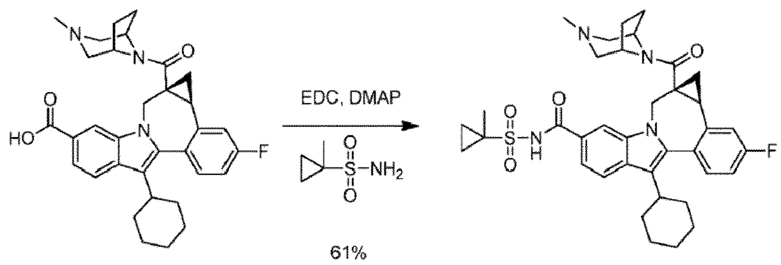


A mixture of the starting acid (40 mg, 0.074 mmol), 1-methylcyclopropane-1-sulfonamide (20.0 mg, 0.15 mmol), EDC (28 mg, 0.15 mmol), DMAP (27 mg, 0.22 mmol) in DCM (2 mL) was stirred at rt for 16h. It was then concentrated and purified by preparative HPLC (Column: Waters Sunfire C18 OBD 30 x 100mm, Gradient: 50-75% B, Solvent A: 90% Water : 10% Methanol: 0.1% TFA, Solvent B: 10% Water :90% Methanol: 0.1% TFA, Gradient Time = 18 min, Stop Time = 20 min, Flow Rate = 25 ml/min, UV detection, Wavelength: 220 nm) to give 31 mg (61%) of the product as a white solid. 1H NMR (500MHz, CDC13)(major rotamer) δ 8.01 (br s, 1H), 7.88 (d, J=7.9 Hz, 1H), 7.58 (br d, 1H), 7.36 (m, 1H) 7.29 (d, J=2.5 Hz 1H), 7.20 (dd, J=9.5, 2.5 Hz, 1H), 7.10-7.06 (m, 1H), 5.20 (d, J=15.1 Hz, 1H), 4.42 (br s, 1H), 4.15 (d, J=14.5 Hz, 1H), 3.58 (d, J=15.4 Hz, 1H), 3.39 (br s, 1H), 2.91 (t, J=12.0 Hz, 1H), 2.74 (m, 1H), 2.70 (m, 1H), 2.56 (br s, 1H), 2.22 (m, 1H), 1.99 (m, 2H), 1.98 (m, 2H), 1.82 (m, 2H), 1.55 (m, 2H), 1.50 (m, 5H), 1.25 (m 2H), 1.20 (t, J=6.1 Hz, 1H), 0.95 (m, 4H). LCMS: (RT = 2.16 min), Column: PHENOMENEX-LUNA 2.0 x 30mm (3μm), Mobile Phase: gradient 0-100% B, Solvent A (90% Water : 10% Methanol: 0.1% TFA), Solvent B (10% Water :90% Methanol: 0.1% TFA), Flow rate: 1.0 mL/min; (M+H) = 659.27.

Preparation 11:

5-methyl 1a-(2-methyl-2-propanyl)(1a*R*,12b*S*)-8-cyclohexyl-11-fluoro-1,12b-dihydrocyclopropa[*d*]indolo[2,1-a][2]benzazepine-1a,5(2*H*)-dicarboxylate

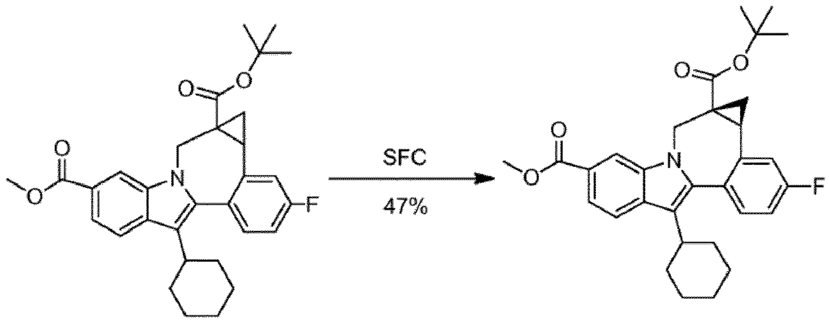


Conditions used for the SFC separation of enatiomers on preparative scale are described below. A single 580 g batch of racemic product was divided and separated in two runs as described below. 275.6 grams was separated using the following preparative SFC conditions. The purity of the target isomer fraction was determined by the analytical SFC conditions. The yield of the target isomer is approximately 120 grams. 211.8 grams was separated using the following preparative SFC conditions. The purity of the target isomer fraction was determined by the analytical SFC conditions. The yield of the target isomer is approximately 102 grams. Chiral purity of the target isomer from both runs was > 99.9%.

Preparation 12:

Preparation 12 (1a*S*,12b*R*)-8-cyclohexyl-11-fluoro-5-(methoxycarbonyl)-1,12b-dihydrocyclopropa[*d*]indolo[2,1-*a*][2]benzazepine-1a(2*H*)-carboxylic acid

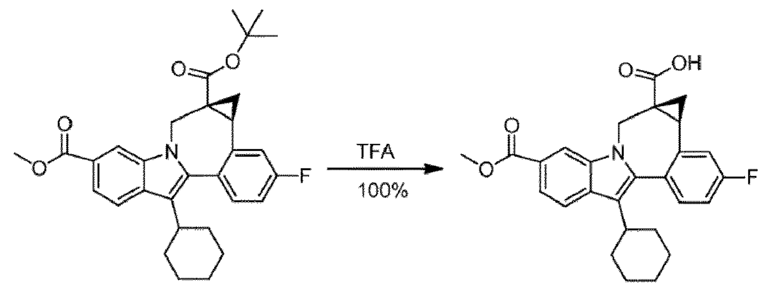


To a solution of starting material (87 g, 173 mmol) in CH2Cl2 (870 mL) cooled with an ice bath was added trifluoroacetic acid (394 g, 3455 mmol). The reaction mixture was allowed to warm to rt and stirred for 22 h. The solvent and TFA were removed and the residue was dissolved in CH2Cl2 (1000 mL). The solution was washed with water (4 x 500 mL), brine (500 mL), dried over MgS04, filtered and concentrated in vacuo and high vacuum to give the crude product (4b5"5aR)-12-cyclohexyl-3-fluoro-9-(methoxycarbonyl)-4b,5,5a,6-tetrahydrobenzo[3,4]cyclopropa-[5,6]azepino[1,2-a]indole-5a-carboxylic acid (79 g, 177 mmol, 102 % yield) as a yellow oil which was used for the next step without further purification. ¾ NMR (400 MHz,CDC13) δ 8.39 (s, 0.5 H), 8.17 (bs, 1.5 H), 7.86-7.90 (m, 1 H), 7.73-7.81 (m, 1 H), 7.23-7.38 (m, 2 H), 7.10-7.16 (m, 1H), 5.48 (d, J= 16.0 Hz, 0.5 H), 5.24 (d, J= 16.0 Hz, 0.5 H), 4.08-4.20 (m, 1 H), 4.0 (s, 3 H), 3.98 (d, J= 16.0 Hz, 0.5 H), 3.46 (d, J= 16.0 Hz, 0.5 H), 2.90-3.03 (m, 1 H), 2.75-2.80 (m, 1 H), 1.21-2.18 (m, 10 H), 0.89-0.93 (m, 0.5 H), 0.50-0.53 (m, 0.5 H).

Preparation 13:

methyl (1a*R*,12b*S*)-8-cyclohexyl-11-fluoro-1a-((3-methyl-3,8-diazabicyclo[3.2.1]oct-8-yl)carbonyl)-1,1a,2,12b-tetrahydrocyclopropa[*d*]indolo[2,1-*a*][2]benzazepine-5-carboxylate

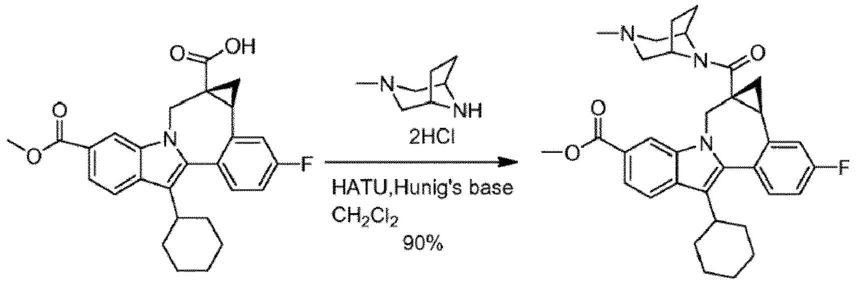


To a 2 flushed, three necked, 3L round bottom flask was added starting acid (79 g, 177 mmol), CH2C12 (1000 mL), bis-HCl salt of (1R,55)-3-methyl-3,8-diazabicyclo[3.2.1]octane (42.23 g, 212 mmol), 0-(7-azabenzotriazol- 1 -yl)-N,N,N',N'-tetramethyluronium hexafluorophosphate (81 g, 212 mmol) and NN-di-iso-propylethylamine (123 mL, 706 mmol) respectively. The reaction mixture was stirred at rt for 3 h. It was diluted with water (1000 mL). After stirring for 10 mins, the organic phase was separated and the aqueous layer was extracted with CH2Cl2 (500 mL). The combined organic extract were washed with water (2 x 1000 mL), brine (1000 mL), dried over MgS04, filtered and concentrated in vacuo to ~ 200 mL. The solution was then subjected to a flash silica gel column (packed with hexane) using 3% TEA/30 to 70% EtOAc in hexane to give the product (88 g, 158 mmol, 90 % yield) as a yellow foam. ¾ NMR (500 MHz,CDC13) δ 8.12 (bs, 0.5 H), 8.08 (bs, 0.5 H), 7.87-7.92 (m, 1 H), 7.76-7.83 (m, 1 H), 7.28-7.36 (m, 2 H), 7.08-7.15 (m, 1 H), 5.21 (d, J= 15.0 Hz, 0.5 H), 4.24 (d, J= 15.0 Hz, 0.5 H), 4.12-4.17 (m, 1.5 H), 3.93-3.98 (m, 3.5 H), 3.61 (d, J = 15.0 Hz, 0.5 H), 3.38 (bs, 0.5 H), 2.94 (t, J= 15.0 Hz, 1 H), 2.72-2.81 (m, 2 H), 1.06-2.52 (m, 22 H).

Preparation 14:

Preparation 14 (1a*R*,12b*S*)-8-cyclohexyl-11-fluoro-1a-((3-methyl-3,8-diazabicyclo[3.2.1]oct-8-yl)carbonyl)-1,1a,2,12b-tetrahydrocyclopropa[*d*]indolo[2,1-*a*][2]benzazepine-5-carboxylic acid

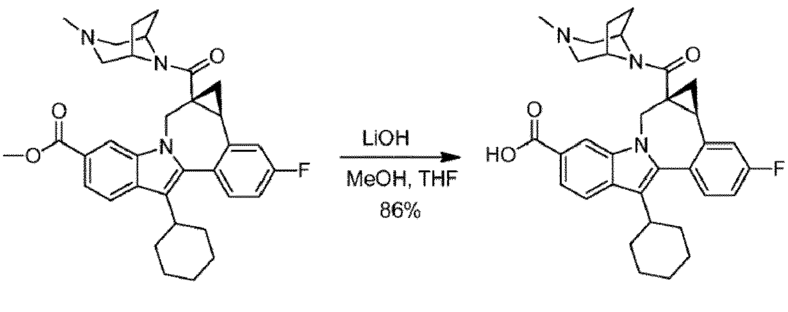


To a solution of starting ester (50 g, 90 mmol) in THF (400 mL) and MeOH (100 mL) was added a solution of lithium hydroxide (10.77 g, 450 mmol) in water (100 mL). The mixture was heated to 50 °C for 16 h. HPLC indicated the completion of the reaction. After cooling, the organic solvents were removed and the residual yellow slurry was extracted with EtOAc (2 x 400 mL). The combined organic extracts were washed with brine (500 mL), dried over Na2S04, filtered and concentrated in vacuo to give a crude solid which was triturated with CH2C12 (80 mL, rt to -20 °C) to afford the product (41.76 g, 77 mmol, 86 % yield) as a white solid. 99.8% HPLC area purity. ¾ NMR (300 MHz, DMSO-d6) δ 8.00 (bs, 1 H), 7.79 (t, J= 9.0 Hz, 1 H), 7.58-7.66 (m, 1 H), 7.26-7.32 (m, 2 H), 7.07-7.17 (m, 1 H), 4.79-5.07 (m, 1 H), 4.04-4.26 (m, 2 H), 3.88 (bs, 1 H), 3.57 (d, J= 15 Hz, 1 H), 3.21 (s, 3 H), 1.03 -2.89 (m, 21 H).

Preparation 15:

Preparation 15 (1a*R*,12b*S*)-8-cyclohexyl-11-fluoro-*N*-((1-methylcyclopropyl)sulfonyl)-1a-((3-methyl-3,8-diazabicyclo[3.2.1]oct-8-yl)carbonyl)-1,1a,2,12b-tetrahydrocyclopropa[*d*]indolo[2,1-*a*][2]benzazepine-5-carboxamide

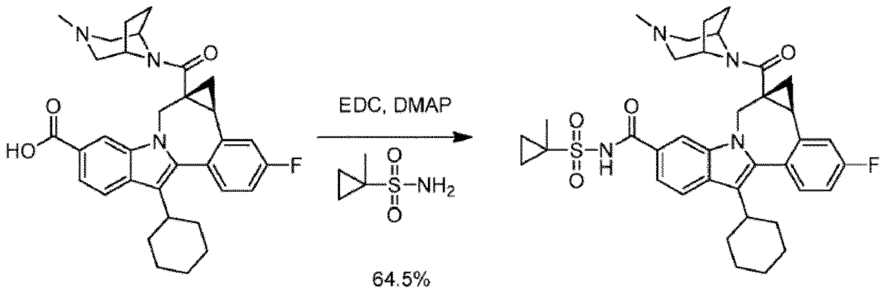


A mixture of starting material (27.3 g, 50.4 mmol), 1-methylcyclopropane- 1 -sulfonamide (20.44 g, 151 mmol), 4-dimethylaminopyridine (24.63 g, 202 mmol) and EDC (29.0 g, 151 mmol) in CH2Cl2 (250 mL) was stirred at rt for 72 h. The mixture was acidified with 1 N HCl (~ 200 mL) to PH = 4. After stirring for 30 mins, the organic phase was separated and the aqueous layer was extracted with CH2Cl2 (200 mL). The combined organic extracts were washed with water (3 X 250 mL, small amount of NaCl was added to help separation), brine (250 mL), dried over MgS04, filtered and concentrated in vacuo to give a crude mixture which was dissolved in CH2Cl2 (100 mL). The solution was subjected to asilica gel column using 3% MeOH in CH2CH2 as the eluent to yield the product as a white solid (21.5 g, 32.5 mmol, 64.5 % yield). 99.8% HPLC area purity. The above solid contained ~ 3% CH2Cl2. It was dissolved in MeOH (50 mL) and the solvent was evaporated in vacuo. The dried material was redissolved in MeOH (50 mL) and the solvent was removed in vacuo to give a residue which was dried in a high vacuo oven at 60 °C for 24 h to yield the product (21g) as an off white solid. H NMR (500 MHz,CDC13) δ 8.01 (bs, 1 H), 7.88 (d, J= 7.9 Hz, 1 H), 7.58 (bs, 1 H), 7.36 (m, 1 H), 7.29 (d, J= 2.5 Hz, 1 H), 7.20 (dd, J= 9.5 and 2.5 Hz, 1 H), 7.06-7.10 (m, 1 H), 5.20 (d, J= 15.1 Hz, 1 H), 4.42 (bs, 1 H), 4.15 (d, J= 14.5 Hz, 1 H), 3.58 (d, J= 15.4 Hz, 1 H), 3.39 (bs, 1 H), 2.91 (t, J= 12.0 Hz, 1 H), 2.74 (m, 1 H), 2.70 (m, 1 H), 2.56 (bs, 1 H), 2.22 (m, 1 H), 1.99 (m, 2 H), 1.98 (m, 2 H), 1.82 (m, 2 H), 1.55 (m, 2 H), 1.50 (m, 5 H), 1.25 (m, 2 H), 1.20 (t, J= 6.1 Hz, 1 H), 0.95 (m, 4 H).

Preparation 16

*N*-(tert-butyl)-1-methylcyclopropane-1-sulfonamide

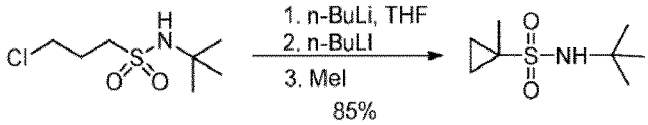


To a four necked, 5 L round bottom flask was added N-(tert-butyl)-3-chloropropane-1-sulfonamide (Previously dried by azeotroping with 3 x 1OOmL toluene) (100 g, 468 mmol) and THF (1500 niL). This was cooled to an internal temperature of -69 °C then butyl lithium (2.5M in hexanes, 412 mL, 1.02 mol) was added dropwise over a period of 55 min while keeping the internal temperature below -65 °C. The ice bath was removed and the reaction mixture was warmed to rt over 1.5 h and then cooled back down to an internal temperature of -69 °C. Butyl lithium (196 mL, 515 mmol) was added to the reaction mixture over a period of 25 min while keeping the internal temperature below -65 °C. The reaction mixture was then warmed to rt over the course of 1.5h. The reaction mixture was recooled to an internal temperature of -69 °C and iodo methane (58.5 mL, 936 mmol) was added dropwise over a period of 40 min while keeping the internal temperature below-65 °C. The reaction mixture was then warmed to an internal temperature of -50 °C over the course of 4h. The cold bath was removed and a solution of saturated NH4C1 (1000 mL) was added. The quenched reaction mixture was transferred to a separatory funnel along with ethyl acetate (100 mL) and water (500 mL). The layers were separated and the aqueous layer was extracted with ethyl acetate. (3 x 75 mL). The combined organic layers were

washed with brine (700 mL), dried with MgS04, and concentrated in vacuo to an off white solid which was dried under high vac for 30 min to yield *N*-(tert-butyl)-1-methylcyclopropane-1 -sulfonamide as a white solid (88.5 g, 99% yield). ¾ NMR (500 MHz, CDC13) δ 4.07 (bs, 1 H), 1.51 (s, 3 H), 1.38-1.41 (m, 2 H), 1.36 (s, 9 H), 0.77-0.80 (m, 2 H).

Preparation 17:

1-methylcyclopropane-1-sulfonamide

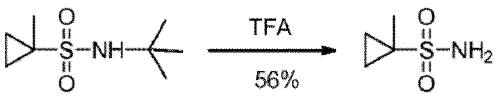


To a one necked, 1 L round bottom flask was added N-(tert-butyl)-1-methylcyclopropane-1 -sulfonamide (78.6 g, 411 mmol) and TFA (340 mL). The brown solution was stirred overnight at rt. The reaction mixture was concentrated (at 50 °C) to a brown oil. A stream of 2 was blown into the oil for about 35 minutes at the end of which a brown semi-solid formed. The semi-solid was suspended in ethyl acetate (80 mL) and hexane (231 mL). The suspension was stirred at rt for 10 minutes then vacuum filtered. The filter cake was rinsed with hexane then dried overnight to give a crude tan solid (51.6g). The solid was recrystallized from a mixture of ethyl acetate (190 mL) and hexane (270 mL), (heat to reflux, cooled to rt and vacuum filtered at rt) to give an off white solid (37.6 g, 68% yield). The off white solid (37.6 g) was dissolved in hot ethyl acetate (257 mL), diluted with hexane (177 mL) then refluxed for 5 minutes. The hot solution was cooled to rt, the resulting white suspension was vacuum filtered, and the filter cake was dried to a white solid (56.6 g, 416 mmol, 56% yield). 1H NMR (500 MHz, DMSO-d6) δ 6.72 (bs, 2 H), 1.44 (s, 3 H), 1.13 (dd, J= 6.1 and 4.0 Hz, 2 H), 0.74 (dd, J= 6.1 and 4.0 Hz, 2 H). 13C NMR (125 MHz, DMSO-d6) 5 36.4, 17.6, 12.1.

Compound 2 was evaluated for antiviral activity as determined by the following assays:

Biological Methods: HCVNS5B RdRp cloning, expression, and purification. The cDNA encoding the NS5B protein of HCV, genotype lb, was cloned into the pET21a expression vector. The protein was expressed with an 18 amino acid C-terminal truncation to enhance the solubility. The E. coli competent cell line BL21(DE3) was used for expression of the protein. Cultures were grown at 37 °C for ~ 4 hours until the cultures reached an optical density of 2.0 at 600 nm. The cultures were cooled to 20 °C and induced with 1 mM IPTG. Fresh ampicillin was added to a final concentration of 50 μg/mL and the cells were grown overnight at 20 °C. Cell pellets (3L) were lysed for purification to yield 15-24 mgs of purified NS5B. The lysis buffer consisted of 20 mM Tris-HCl, pH 7.4, 500 mM NaCl, 0.5% triton X-100, 1 mM DTT, ImM EDTA, 20% glycerol, 0.5 mg/mL lysozyme, 10 mM MgCi2, 15 ug/mL deoxyribonuclease I, and Complete TM protease inhibitor tablets (Roche). After addition of the lysis buffer, frozen cell pellets were resuspended using a tissue homogenizer. To reduce the viscosity of the sample, aliquots of the lysate were sonicated on ice using a microtip attached to a Branson sonicator. The sonicated lysate was centrifuged at 100,000 x g for 30 minutes at 4 °C and filtered through a 0.2 μm filter unit (Coming). The protein was purified using two sequential chromatography steps: Heparin sepharose CL-6B and polyU sepharose 4B. The chromatography buffers were identical to the lysis buffer but contained no lysozyme, deoxyribonuclease I, MgC12 or protease inhibitor and the NaCl concentration of the buffer was adjusted according to the requirements for charging the protein onto the column. Each column was eluted with a NaCl gradient which varied in length from 5-50 column volumes depending on the column type. After the final chromatography step, the resulting purity of the enzyme is >90% based on SDS-PAGE analysis. The enzyme was aliquoted and stored at -80 °C. HCVNS5B RdRp enzyme assay. An on-bead solid phase homogeneous assay was used in a 384-well format to assess NS5B inhibitors (WangY-K, Rigat K, Roberts S, and Gao M (2006) Anal Biochem, 359: 106-1 11). The biotinylated oligo dT12 primer was captured on streptavidin-coupled imaging beads (GE, RPNQ0261) by mixing primer and beads in IX buffer and incubating at room temperature for three hours. Unbound primer was removed after centrifugation. The primer-bound beads were resuspended in 3X reaction mix (20 mM Hepes buffer, pH 7.5, dT primer coupled beads, poly A template, 3H-UTP, and RNAse inhibitor (Promega 2515)). Compounds were serially diluted 1 :3 in DMSO and aliquoted into assay plates. Equal volumes (10 μl) of water, 3X reaction mix, and enzyme in 3X assay buffer (60 mM Hepes buffer, pH 7.5, 7.5 mM MgCl2, 7.5 mM KCl, 3 mM DTT, 0.03 mg/mL BSA, 6 % glycerol) were added to the diluted compound on the assay plate. Final concentration of components in 384-well assay: 0.36 nM template, 15 nM primer, 0.29 μM 3H-UTP (0.3 μθï), 1.6 ν/μl RNAse inhibitor, 7 nM NS5B enzyme, 0.01 mg/mL BSA, 1 mM DTT, and 0.33 μg/μL beads, 20 mM Hepes buffer, pH 7.5, 2.5 mM MgCl2, 2.5 mM KCl, and 0.1 % DMSO. Reactions were allowed to proceed for 4 hours at 30° C and terminated by the addition of 50 mM EDTA (10 μl). After incubating for at least 15 minutes, plates

were read on an Amersham LEADseeker multimodality imaging system. IC50 values for compounds were determined using ten different [I]. IC50 values were calculated from the inhibition using the four-parameter logistic formula y = A+((B-A)/(1+((C/x)AD))), where A and B denote minimal and maximal % inhibition, respectively, C is the IC50, D is hill slope and x represents compound concentration. Cell lines. The cell lines used to evaluate compounds consist of a human hepatocyte derived cell line (Huh-7) that constitutively expresses a genotype lb HCV replicon containing a Renilla luciferase reporter gene. These cells were maintained in Dulbecco's modified Eagle medium (DMEM) containing 10% FBS, 100 U/mL penicillin/streptomycin and 1.0 mg/mL G418. HCV replicon luciferase assay. To evaluate compound efficacy, titrated compounds were transferred to sterile 384-well tissue culture treated plates, and the plates were seeded with HCV replicon cells (50 at a density of 2.4 x 103 cells/well) in DMEM containing 4 % FBS (final DMSO concentration at 0.5 %). After 3 days incubation at 37°C, cells were analyzed for Renilla Luciferase activity using the EnduRen substrate (Promega cat #E6485) according to the manufacturer's directions. Briefly, the EnduRen substrate was diluted in DMEM and then added to the plates to a final concentration of 7.5 μM. The plates were incubated for at least 1 h at 37°C then read on a Viewlux Imager (PerkinElmer) using a luminescence program. The 50% effective concentration (EC50) was calculated using using the four-parameter logistic formula noted above. To assess cytotoxicity of compounds, Cell Titer-Blue (Promega) was added to the EnduRen-containing plates and incubated for at least 4 hrs at 37°C. The fluorescence signal from each well was read using a Viewlux Imager. All CC50 values were calculated using the four-parameter logistic formula. Enzyme and replicon data for compound 2 ({drug2}) is reported in Table 2.

Table 2.

| Structure | $EC_{50}$, (μM) | $IC_{50}$ (μM) |
|---|---|---|
| 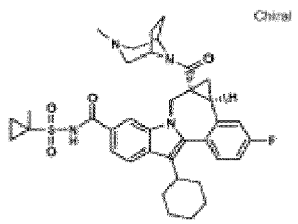 | 0.007 | 0.005 |

Compound 2 was evaluated for ion channel activity as determined by the following assays.

The ion channel responsible for the rapid component of the cardiac delayed rectifier potassium current (IKr) is encoded by the potassium channel gene, hERG (human ether-a-go-go-related gene). Mutations in hERG cause the chromosome 7-linked form of congenital long QT syndrome (LQT2). Prolongation of the QT interval and increased action potential duration (APD) can lead to the potentially fatal ventricular arrhythmia, torsade de pointes. Method for assessing hERG potassium channel with Flux. The hERG Flux assay is a relatively high throughput functional assay for predicting hERG inhibition which is FLIPR-based, and it has been validated against known IKr inhibitors and compared to patch-clamp, binding and in silico modeling data. The hERG Flux assay was validated with clinical drugs that inhibit the hERG potassium channel and also prolong the cardiac QT interval. For 85% of compounds tested to date, the hERG Flux IC50 data is right-shifted with respect to patch clamp IC50 determinations by a median- fold difference of about seven, and the remaining 15% show greater than 10-fold shift. Accordingly, the hERG Flux data should be interpreted as follows: an IC50 of < 5 μM is considered potent with a high probability of having a sub-micromolar IC50 in the hERG patch-clamp assay; 5 - 80 μM should be considered moderately potent, and > 80 μM should be considered weak. Cell Preparation. HEK293 cells stably-expressing hERG channels were grown in Dulbecco's Modified Eagle's Media supplemented with 10% Sigma fetal bovine serum, non-essential amino acids, 2mM L-glutamine and 500 μg/mL G418, at 37°C in a 5% C02 incubator. Cell are plated into 384-well Corning poly-D-lysine coated black/clear plates at a density of 2 x 104 cells per well (20 μί) in 10% serum media, and incubated for 15-24 hours at 37°C in a 5% CO2 incubator until a confluent monolayer of cells is obtained. Loading ofBTC Dye. A 2 mM stock of BTC-AM dye (Molecular Probes, Eugene, OR) is prepared in 100% DMSO and then added 1 : 1 to 10% (w/v) pluronic acid in DMSO on the day of assay. The dye is then diluted in hERG external EP buffer. This BTC dye mixture (30 μί) is added to the cells and produces a final loading concentration of 2.5 μM. Cells are incubated at 21°C for 45 minutes. The hERG external EP buffer contains 140 mM NaCl, 4.0 mM KCl, 1.8 mM CaCl2, 1.0 mM MgCl2, 10 mM HEPES, pH 7.3 and 10 mM glucose; this is the same buffer used for patch-clamp experiments (all buffer components obtained from Sigma Chemical). Sample Preparation. Test samples are diluted to 10 mM DMSO and serially-diluted at a 1 :2 ratio in DMSO in a 384-well plate. 2.5 μί serially-diluted test sample was transferred to 75 μί of hERG external electrophysiology (EP) buffer. After dye loading and buffer exchange, 10 μί of aqueous-diluted compounds are added to the cells of the two replicate plates. Compounds are pre-incubated with the cells for 30-45 minutes before the assay is read on the FLIPR. Sample preparation and the assay dilutions yield a ten-point final concentration range from 80 μM to 0.156 nM. FLIPR Assay. Cells loaded with dye are read on the FLIPR384 (Molecular Devices, Sunnyvale, CA), which excites the dye using the 488 nm line of an argon laser. Emission was filtered using a 540 ± 30 nm bandpass filter. hERG channels are stimulated to open by the addition of 10 μί/well EP buffer containing 33 mM K2SO4 and 0.66 mM Tl2SO4 (Sigma/Aldrich). For each plate, data are collected every second for a period of 10 seconds, at which time the thallium-containing stimulus buffer is added. Data collection proceeds every second for 50 seconds, and then continues every three seconds for an additional 2 minutes. The addition of stimulus buffer produces a final volume of 50 μί at assay read, to give a final DMSO content of 0.65%. Data analysis. The statistical robustness of the hERG Flux assay is determined from blank and totals wells. The totals wells (columns 21 and 22) define maximal hERG activation for each compound test plate (no test compound present), and the blanks wells (columns 23 and 24), which contain a saturating concentration of the hERG channel inhibitors (dofetilide or E-4031), define 100% hERG channel inhibition. The raw fluorescence units data files generated on the FLIPR plate reader are automatically exported and processed by in-house data analysis tools. The reduced percent inhibition data for each concentration of test compound were fit using MathIQ fitting engine (ID Business Solutions Limited, Surrey, UK). Data were analyzed by fitting maximum amplitudes of change in fluorescence, for thallium flux for a given condition of test compound. Potencies (IC50 values) of compounds were calculated from the average of the two 10-point concentration response curves.

Table 3 : FLIPR Data

| Structure | $IC_{50}$ (μM) |
|---|---|
| 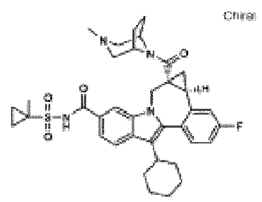 | 23.4 |

Method for assessing hERG potassium channels with patch clamp. Cell line. Human embryonic kidney (HEK293) cells stably transfected with human ether-a-go-go related gene (hERG) cDNA were used in the hERG assay. The biophysical and pharmacological properties of recombinant hERG channels expressed in HEK293 cells and of native IKr channels in human cardiac cells are nearly identical. Several known hERG blockers, including dofetilide, terfenadine, cisapride and E-4031 inhibit recombinant hERG currents in the hERG stable cell line and IKr currents in isolated cardiac myocytes with identical potency. Patch clamp. Membrane current recordings were made with a Multiclamp 700 series integrating patch-clamp amplifier (Axon Instruments, Foster City, California) using the whole-cell variant of the patch-clamp technique. Cells expressing hERG potassium channels were placed in a plexiglass bath chamber, mounted on the stage of an inverted microscope, and perfused continuously with bath solution. The hERG bath solution, which replaced the cell culture media during experiments, contained (in mM): 140 NaCl, 4 KCl, 1.8 CaC12, 1 MgCl2, 10 glucose, 10 HEPES (pH 7.4, NaOH). Borosilicate glass pipettes had tip resistances of 2 to 4 MΩ when filled with an internal solution containing (in mM): 130 KCl, 1 MgCl2, 1 CaC12, 5 ATP-K2, 10 EGTA, 10 HEPES (pH 7.2, KOH). Initially, a current- voltage relationship was generated in the control bath solution using the following voltage protocol. hERG currents were elicited by 2 second step depolarizations applied from a holding potential of -80 mV to test potentials ranging from -70 mV to +60 mV. The voltage steps were applied in 20 second intervals. Tail currents were elicited upon repolarization to -65 mV for 3 seconds. While still perfusing with control bath solution, the voltage protocol was switched to one where repetitive test pulses (0.05 Hz) were applied from a holding potential of -80 mV to +20 mV for 2 seconds. Tail currents were elicited following the test pulses by stepping the voltage to -65 mV for 3 seconds. After recording the steady-state current for 2 to 5 minutes in the absence of test article (control), the bath solution was switched to one containing the lowest concentration of test article to be used. The peak tail current was monitored until a new steady-state in the presence of test article was achieved. This was followed by the application of the next higher concentration of test article to be tested, and was repeated until all concentrations of test article had been evaluated. Effects of test article on hERG channel were calculated by measuring inhibition of peak tail currents. Percent inhibition of tail currents was plotted as a function of test article concentration to quantify hERG channel inhibition. Test article effects were calculated using tail currents because there are no endogenous tail currents in plasmid-transfected control HEK293 cells. Membrane currents were sampled at rates at least 2 times the low pass filter rate. The flow rate was kept constant throughout the experiments. All currents were recorded at room temperature ~ 25°C.

Table 4: Patch Clamp Data.

| Structure | Conc. (μM) | % Inhibition (Mean ± SE) | Replicates |
|---|---|---|---|
| 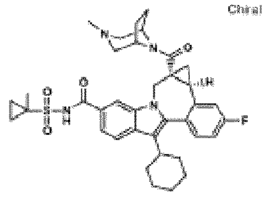 Chiral | 10 | 30.5 ± 1.9 | 3 |
| 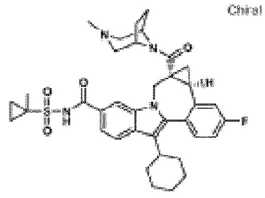 Chiral | 30 | 43.4 ± 2.0 | 3 |

SPECIFIC INORMATION CONCERNING ASPECT (III) OF THE INVENTION

Aspect (iii) of the invention concerns forms of {drug3}, especially a solid form of 4-[2-(5-amino-1H-pyrazol-4-yl)-4-chlorophenoxy]-5-chloro-2-fluoro-N-(1,3-thiazol-4-yl)benzenesulfonamide ({drug3}), namely to tosylate salt of 4-[2-(5-amino-1 H-pyrazol-4-yl)-4-chlorophenoxy]-5-chloro-2-fluoro-N-(1,3-thiazol-4-yl)benzenesulfonamide ({drug3a}), which is useful for treating diseases associated with high blood uric acid levels such as hyperuricemia, including renal disorders associated with hyperuricemia (e.g. urinary calculi); and gout, including gouty tophus and gouty arthritis. The tosylate salt of 4-[2-(5-amino-1 H-pyrazol-4-yl)-4-chlorophenoxy]-5-chloro-2-fluoro-N-(1,3-thiazol-4-yl)benzenesulfonamide is described in WO/2013/057722, which is incorporated by reference. As used herein, tosylate salt of 4-[2-(5-amino-1 H-pyrazol-4-yl)-4-chlorophenoxy]-5-chloro-2-fluoro-N-(1,3-thiazol-4-yl)benzenesulfonamide is a crystalline form of 4-[2-(5-amino-1H-pyrazol-4-yl)-4-chlorophenoxy]-5-chloro-2-fluoro-N-(1,3-thiazol-4-yl)benzenesulfonamide characterized in WO/2013/ 057722 and tosylate salt of 4-[2-(5-amino-1 H-pyrazol-4-yl)-4-chlorophenoxy]-5-chloro-2-fluoro-N-(1,3-thiazol-4-yl)benzenesulfonamide can be obtained as described in WO/2013/057722: New salt and medical use: The Invention relates to a new medical use for 4-[2-(5-amino-1 H-pyrazol-4-yl)-4-chlorophenoxy]-5-chloro-2-fluoro-N-(1,3-thiazol-4-yl)benzenesulfonamide, to an improved pharmaceutically acceptable salt of 4-[2-(5-amino-1 H-pyrazol-4-yl)-4-chlorophenoxy]-5-chloro-2-fluoro-N-(1,3-thiazol-4-yl)benzenesulfonamide and to compositions thereof. The compound 4-[2-(5-amino-1 H-pyrazol-4-yl)-4-chlorophenoxy]-5-chloro-2-fluoro-N-(1,3-thiazol-4-yl)benzenesulfonamide is a voltage-gated sodium channel (Nav) inhibitor, more specifically a Nav1.7 inhibitor, and is disclosed as Example 788 in international patent application publication number WO2010/079443, the entire contents of which are incorporated herein by reference. As a Nav1.7 inhibitor the compound is potentially useful in the treatment of a wide range of disorders, particularly pain, including: acute pain; chronic pain; neuropathic pain; inflammatory pain; visceral pain; nociceptive pain including post-surgical pain; and mixed pain types involving the viscera, gastrointestinal tract, cranial structures, musculoskeletal system, spine, urogenital system, cardiovascular system and CNS, including cancer pain, back and orofacial pain. Uric acid is the final product of purine metabolism in humans. In humans, unlike many other animals, uric acid is not further broken down, but is predominantly (70%) excreted into the urine with the remaining 30% excreted in faeces. Hyperuricemia is defined as an excessive production or decreased excretion of uric acid and can occur as an overproduction or under excretion of serum uric acid (sUA), or a combination of the both. Renal under excretion of uric acid is the primary cause of hyperuricemia in about 90% of cases, while overproduction is the cause in less than 10%. Increased sUA concentration above than 6.8mg/dl_results in crystallisation of uric acid in the form of salts, such as monosodium urate, and to precipitation of these crystals in joints, on tendons and in the surrounding tissues. These crystals (known as tophi) trigger a local immune-mediated inflammatory reaction, leading to gout. The risk of gout increases with increased sUA levels. Renal under excretion of uric acid is the primary cause of hyperuricemia in about 90% of cases, while overproduction is the cause in less than 10%. The risk of gout increases with increased uric acid levels. Gout is a painful condition that can present in a number of ways, although the most usual is a recurrent attack of acute inflammatory arthritis (a red, tender, hot, swollen joint) often occurring in big toes, heels, knees, wrists and fingers. Gout is treated by agents to both decrease the cause and effects of uric acid crystal inflammation and pain. The pain associated with gout is commonly treated with pain and anti-inflammatory drugs such as nonsteroidal anti-inflammatory drugs (NSAIDs), colchicine and steroids. Agents that decrease sUA levels may be used to treat the cause of gout. These include agents that: inhibit the enzymes that result in uric acid production, such as xanthine oxidase inhibitors (e.g. allopurinol, febuxostat or tisopurine), or purine nucleoside phosphorylase (PNP) inhibitors (e.g. ulodesine); metabolise uric acid, such as urate

oxidases - also known as uricases (e.g. pegloticase); or increase the excretion of uric acid in the urine (uricosurics), Uricosurics include agents that inhibit the transporters responsible for renal reabsorption of uric acid back into the blood, such as benziodarone, isobromindione, probenecid and sulphinpyrazone, and URAT-1 inhibitors (e.g. benzbromarone). URAT-1 is also known as solute carrier family 22 (organic anion/cation transporter), member 12, and is encoded by the gene SLC22A12. Human genetic analysis has demonstrated that polymorphisms in the SLC22A12 gene are directly associated with changes in serum uric acid. Inhibitors of uric acid transport, such as URAT-1, are therefore effective in the treatment of gout. There is a continuing need to provide new treatments for gout that are more effective and/or are better tolerated. Surprisingly, it has now been found that 4-[2-(5-amino-1 H-pyrazol-4-yl)-4-chlorophenoxy]-5-chloro-2-fluoro-N-(1,3-thiazol-4-yl)benzenesulfonamide reduces blood uric acid levels. As shown herein, 4-[2-(5-amino-1 H-pyrazol-4-yl)-4-chlorophenoxy]-5-chloro-2-fluoro-N-(1,3-thiazol-4-yl)benzenesulfonamide is an inhibitor of URAT-1. This uric acid lowering effect is discussed in more detail hereinafter with reference to the data in Tables 5 to 9 and Figures 7 and 8 of WO/2013/057722. These data were obtained using oral dispersions prepared from 4-[2-(5-amino-1 H-pyrazol-4-yl)-4-chlorophenoxy]-5-chloro-2-fluoro-N-(1,3-thiazol-4-yl)benzenesulfonamide and the tosylate salt thereof. 4-[2-(5-Amino-1 H-pyrazol-4-yl)-4-chlorophenoxy]-5-chloro-2-fluoro-N-(1,3-thiazol-4-yl)benzenesulfonamide is therefore useful in the treatment of diseases associated with high blood uric acid levels such as hyperuricemia, including renal disorders associated with hyperuricemia (e.g. urinary calculi); and gout, including gouty tophus and gouty arthritis. It also follows that 4-[2-(5-amino-1 H-pyrazol-4-yl)-4-chlorophenoxy]-5-chloro-2-fluoro-N-(1,3-thiazol-4-yl)benzenesulfonamide is useful in the treatment of a disease where a URAT-1 inhibitor is indicated. In a first aspect the invention provides 4-[2-(5-amino-1 H-pyrazol-4-yl)-4-chlorophenoxy]- 5- chloro-2-fluoro-N-(1,3-thiazol-4-yl)benzenesulfonamide or a pharmaceutically acceptable salt thereof for the treatment of a disease associated with elevated blood uric acid levels. In one embodiment the disease associated with elevated blood uric acid levels is hyperuricemia. In another embodiment the disease associated with elevated blood uric acid levels is gout. In another aspect the invention provides 4-[2-(5-amino-1 H-pyrazol-4-yl)-4-chlorophenoxy]-5-chloro-2-fluoro-N-(1,3-thiazol-4-yl)benzenesulfonamide or a pharmaceutically acceptable salt thereof for the treatment of a disease where a URAT-1 inhibitor is indicated. In another aspect the invention provides the use of 4-[2-(5-amino-1 H-pyrazol-4-yl)-4-chlorophenoxy]-5-chloro-2-fluoro-N-(1,3-thiazol-4-yl)benzenesulfonamide or a pharmaceutically acceptable salt thereof in the manufacture of a medicament for the treatment of a disease associated with elevated blood uric acid levels. In another aspect the invention provides the use of 4-[2-(5-amino-1 H-pyrazol-4-yl)-4-chlorophenoxy]-5-chloro-2-fluoro-N-(1,3-thiazol-4-yl)benzenesulfonamide or a pharmaceutically acceptable salt thereof in the manufacture of a medicament for the treatment of a disease where a URAT-1 inhibitor is indicated. In another aspect the invention provides a method of treating a disease associated with elevated blood uric acid levels which comprises administering an effective amount of 4-[2-(5-amino-1 H-pyrazol-4-yl)-4-chlorophenoxy]-5-chloro-2-fluoro-N-(1,3-thiazol-4-yl)benzenesulfonamide or a pharmaceutically acceptable salt thereof. In another aspect the invention provides a method of treating a disease where a URAT-1 inhibitor is indicated which comprises administering an effective amount of 4-[2-(5-amino-1 H-pyrazol-4-yl)-4-chlorophenoxy]-5-chloro-2-fluoro-N-(1,3-thiazol-4-yl)benzenesulfonamide or a pharmaceutically acceptable salt thereof. Surprisingly, the tosylate salt of 4-[2-(5-amino-1 H-pyrazol-4-yl)-4-chlorophenoxy]-5-chloro-2-fluoro-N-(1,3-thiazol-4-yl)benzenesulfonamide has been found to have a number of unexpected properties making it especially suitable for the preparation of pharmaceutically acceptable formulations. The tosylate salt shows improved chemical stability over the free base, in particular with regard to formulation and storage. It can also be made in crystalline form, affording better solid form stability than the free base. Surprisingly the tosylate salt showed greater stability with regard to disassociation than other salts, and also demonstrated good aqueous solubility. Accordingly, in another aspect the invention provides the tosylate salt of 4-[2-(5-amino-1 H-pyrazol-4-yl)-4-chlorophenoxy]-5-chloro-2-fluoro-N-(1,3-thiazol-4-yl)benzenesulfonamide.

In one embodiment the tosylate salt of 4-[2-(5-amino-1 H-pyrazol-4-yl)-4-chlorophenoxy]-5-chloro-2-fluoro-N-(1,3-thiazol-4-yl)benzenesulfonamide is a crystalline solid. In a further embodiment the crystalline solid is characterized by a powder X-ray diffraction (PXRD) pattern which shows three, four, five or six of the characteristic two-theta (20) peaks selected from the set of peaks defined by tables 4 and 4a that follow, by using CuKalphal X-ray radiation (wavelength = 1.5406 A).

In a further embodiment, the crystalline solid is characterized by a PXRD pattern which shows any three, four, five or six of the characteristic 20 peaks selected from the group consisting of: 9.0, 9.3, 10.0, 10.7, 1 1.6, 12.5, 12.9, 13.2, 13.8, 14.4, 16.0, 16.6, 17.5, 17.8, 18.1, 21.4 and 23.4° (+/- 0.2° 20), more preferably from the group consisting of 9.0, 9.3, 10.0, 10.7, 1 1.6, 12.9, 13.2, 16.0, 16.6, 17.5, 17.8, 18.1, 21.4 and 23.4° (+/-0.2° 20), most preferably from the group consisting of 1 1.6, 12.9, 16.0, 17.5, 17.8, 18.1 ° (+/- 0.2° 20) by using CuKalphal X-ray radiation (wavelength = 1.5406A). In a further embodiment the crystalline solid is characterized by a powder X-ray diffraction (PXRD) pattern which shows main two-theta (20) peaks at 9.0, 10.7, 16.0, 21.4 and 23.4° (+/- 0.1 ° 2Θ) by using CuKalphal X-ray radiation (wavelength = 1.540562 A).

4-[2-(5-Amino-1 H-pyrazol-4-yl)-4-chlorophenoxy]-5-chloro-2-fluoro-N-(1,3-thiazol-4-yl)benzenesulfonamide or a pharmaceutically acceptable salt thereof, such as the tosylate salt, will generally be administered as a formulation in association with one or more pharmaceutically acceptable excipients. The term 'excipient' is used herein to describe

any ingredient other than the aforementioned benzenesulfonamide. The choice of excipient will to a large extent depend on factors such as the particular mode of administration, the effect of the excipient on solubility and stability, and the nature of the dosage form. In another aspect the invention provides a pharmaceutical composition comprising the tosylate salt of 4-[2-(5-amino-1 H-pyrazol-4-yl)-4-chlorophenoxy]-5-chloro-2-fluoro-N-(1,3-thiazol-4-yl)benzenesulfonamide together with one or more pharmaceutically acceptable excipients. Pharmaceutical compositions suitable for the delivery of 4-[2-(5-Amino-1 H-pyrazol-4-yl)-4-chlorophenoxy]-5-chloro-2-fluoro-N-(1,3-thiazol-4-yl)benzenesulfonamide or a pharmaceutically acceptable salt thereof, such as the tosylate salt, and methods for their preparation will be readily apparent to those skilled in the art. Such compositions and methods may be found, for example, in "Remington's Pharmaceutical Sciences", 19th Edition (Mack Publishing Company, 1995). Suitable modes of administration include oral, parenteral, topical, inhaled/intranasal, rectal/intravaginal, and ocular/aural administration. Formulations suitable for the aforementioned modes of administration may be formulated to be immediate and/or modified release. Modified release formulations include delayed-, sustained-, pulsed-, controlled-, targeted and programmed release. 4-[2-(5-Amino-1 H-pyrazol-4-yl)-4-chlorophenoxy]-5-chloro-2-fluoro-N-(1,3-thiazol-4-yl)benzenesulfonamide or a pharmaceutically acceptable salt thereof, such as the tosylate salt, may be administered orally. Oral administration may involve swallowing, so that the drug enters the gastrointestinal tract, or buccal or sublingual administration may be employed by which the drug enters the blood stream directly from the mouth. Formulations suitable for oral administration include solid formulations such as tablets, capsules containing particulates, liquids, or powders, lozenges (including liquid-filled), chews, multi- and nano-particulates, gels, solid solution, liposome, films, ovules, sprays, liquid formulations and buccal/mucoadhesive patches. Liquid formulations include suspensions, solutions, syrups and elixirs. Such formulations may be employed as fillers in soft or hard capsules and typically comprise a carrier, for example, water, ethanol, polyethylene glycol, propylene glycol, methylcellulose, or a suitable oil, and one or more emulsifying agents and/or suspending agents. Liquid formulations may also be prepared by the reconstitution of a solid, for example, from a sachet. 4-[2-(5-Amino-1 H-pyrazol-4-yl)-4-chlorophenoxy]-5-chloro-2-fluoro-N-(1,3-thiazol-4-yl)benzenesulfonamide or a pharmaceutically acceptable salt thereof, such as the tosylate salt, may also be used in fast-dissolving, fast-disintegrating dosage forms such as those described in Expert Opinion in Therapeutic Patents, V_(6), 981 - 986, by Liang and Chen (2001). For tablet dosage forms, depending on dose, 4-[2-(5-amino-1 H-pyrazol-4-yl)-4-chlorophenoxy]-5-chloro-2-fluoro-N-(1,3-thiazol-4-yl)benzenesulfonamide or a pharmaceutically acceptable salt thereof, such as the tosylate salt, the drug may make up from 1 weight % to 80 weight % of the dosage form, more typically from 5 weight % to 60 weight % of the dosage form. In addition to the drug, tablets generally contain a disintegrant. Examples of disintegrants include sodium starch glycolate, sodium carboxymethyl cellulose, calcium carboxymethyl cellulose, croscarmellose sodium, crospovidone, polyvinylpyrrolidone, methyl cellulose, microcrystalline cellulose, lower alkylsubstituted hydroxypropyl cellulose, starch, pregelatinised starch and sodium alginate. Generally, the disintegrant will comprise from 1 weight % to 25 weight %, preferably from 5 weight % to 20 weight % of the dosage form. Binders are generally used to impart cohesive qualities to a tablet formulation. Suitable binders include microcrystalline cellulose, gelatin, sugars, polyethylene glycol, natural and synthetic gums, polyvinylpyrrolidone, pregelatinised starch, hydroxypropyl cellulose and hydroxypropyl methylcellulose. Tablets may also contain diluents, such as lactose (monohydrate, spray-dried monohydrate, anhydrous and the like), mannitol, xylitol, dextrose, sucrose, sorbitol, microcrystalline cellulose, starch and dibasic calcium phosphate dihydrate. Tablets may also optionally comprise surface active agents, such as sodium lauryl sulfate and polysorbate 80, and glidants such as silicon dioxide and talc. When present, surface active agents may comprise from 0.2 weight % to 5 weight % of the tablet, and glidants may comprise from 0.2 weight % to 1 weight % of the tablet.

Tablets also generally contain lubricants such as magnesium stearate, calcium stearate, zinc stearate, sodium stearyl fumarate, and mixtures of magnesium stearate with sodium lauryl sulphate. Lubricants generally comprise from 0.25 weight % to 10 weight %, preferably from 0.5 weight % to 3 weight % of the tablet. Other possible ingredients include anti-oxidants, colourants, flavouring agents, preservatives and taste-masking agents. Exemplary tablets contain up to about 80% drug, from about 10 weight % to about 90 weight % binder, from about 0 weight % to about 85 weight % diluent, from about 2 weight % to about 10 weight % disintegrant, and from about 0.25 weight % to about 10 weight % lubricant. Tablet blends may be compressed directly or by roller to form tablets. Tablet blends or portions of blends may alternatively be wet-, dry-, or melt-granulated, melt congealed, or extruded before tabletting. The final formulation may comprise one or more layers and may be coated or uncoated; it may even be encapsulated. The formulation of tablets is discussed in "Pharmaceutical Dosage Forms: Tablets", Vol. 1, by H. Lieberman and L. Lachman (Marcel Dekker, New York, 1980). Suitable modified release formulations for the purposes of the invention are described in US Patent No. 6, 106,864. Details of other suitable release technologies such as high energy dispersions and osmotic and coated particles are to be found in "Pharmaceutical Technology On-line", 25(2), 1 -14, by Verma et al (2001). The use of chewing gum to achieve controlled release is described in WO 00/35298. 4-[2-(5-Amino-1 H-pyrazol-4-yl)-4-chlorophenoxy]-5-chloro-2-fluoro-N-(1,3-thiazol-4-yl)benzenesulfonamide or a pharmaceutically acceptable salt thereof, such as the tosylate salt, may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include

needle (including microneedle) injectors, needle-free injectors and infusion techniques. Parenteral formulations are typically aqueous solutions which may contain excipients such as salts, carbohydrates and buffering agents (preferably to a pH of from 3 to 9), but, for some applications, they may be more suitably formulated as a sterile nonaqueous solution or as a dried form to be used in conjunction with a suitable vehicle such as sterile, pyrogen-free water. The preparation of parenteral formulations under sterile conditions, for example, by lyophilisation, may readily be accomplished using standard pharmaceutical techniques well known to those skilled in the art. The solubility of 4-[2-(5-amino-1 H-pyrazol-4-yl)-4-chlorophenoxy]-5-chloro-2-fluoro-N-(1,3-thiazol-4-yl)benzenesulfonamide or a pharmaceutically acceptable salt thereof, such as the tosylate salt, used in the preparation of parenteral solutions may be increased by the use of appropriate formulation techniques, such as the incorporation of solubility-enhancing agents. Formulations for parenteral administration may be formulated to be immediate and/or modified release. Modified release formulations include delayed-, sustained-, pulsed-, controlled-, targeted and programmed release.

4-[2-(5-Amino-1 H-pyrazol-4-yl)-4-chlorophenoxy]-5-chloro-2-fluoro-N-(1,3-thiazol-4-yl)benzenesulfonamide or a pharmaceutically acceptable salt thereof, such as the tosylate salt, may also be administered topically to the skin or mucosa, that is, dermally or transdermally. Typical formulations for this purpose include gels, hydrogels, lotions, solutions, creams, ointments, dusting powders, dressings, foams, films, skin patches, wafers, implants, sponges, fibres, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated - see, for example, J Pharm Sci, 88 (10), 955-958, by Finnin and Morgan (October 1999). Other means of topical administration include delivery by electroporation, iontophoresis, phonophoresis, sonophoresis and microneedle or needle-free (e.g. Powderject™, Bioject™, etc.) injection. 4-[2-(5-Amino-1 H-pyrazol-4-yl)-4-chlorophenoxy]-5-chloro-2-fluoro-N-(1,3-thiazol-4-yl)benzenesulfonamide or a pharmaceutically acceptable salt thereof, such as the tosylate salt, can also be administered intranasally or by inhalation, typically in the form of a dry powder (either alone, as a mixture, for example, in a dry blend with lactose, or as a mixed component particle, for example, mixed with phospholipids, such as phosphatidylcholine) from a dry powder inhaler or as an aerosol spray from a pressurised container, pump, spray, atomiser (preferably an atomiser using electrohydrodynamics to produce a fine mist), or nebuliser, with or without the use of a suitable propellant, such as 1, 1, 1,2-tetrafluoroethane or 1,1,1,2,3,3,3-heptafluoropropane. For intranasal use, the powder may comprise a bioadhesive agent, for example, chitosan or cyclodextrin. The pressurised container, pump, spray, atomizer, or nebuliser contains a solution or suspension of the compound(s) of the invention comprising, for example, ethanol, aqueous ethanol, or a suitable alternative agent for dispersing, solubilising, or extending release of the active, a propellant(s) as solvent and an optional surfactant, such as sorbitan trioleate, oleic acid, or an oligolactic acid. Prior to use in a dry powder or suspension formulation, the drug product is micronised to a size suitable for delivery by inhalation (typically less than 5 microns). This may be achieved by any appropriate comminuting method, such as spiral jet milling, fluid bed jet milling, supercritical fluid processing to form nanoparticles, high pressure homogenisation, or spray drying. Capsules (made, for example, from gelatin or hydroxypropylmethylcellulose), blisters and cartridges for use in an inhaler or insufflator may be formulated to contain a powder mix of the drug product, a suitable powder base such as lactose or starch and a performance modifier such as 1-leucine, mannitol, or magnesium stearate. The lactose may be anhydrous or in the form of the monohydrate, preferably the latter. Other suitable excipients include dextran, glucose, maltose, sorbitol, xylitol, fructose, sucrose and trehalose. Suitable flavours, such as menthol and levomenthol, or sweeteners, such as saccharin or saccharin sodium, may be added to those formulations of the invention intended for inhaled/intranasal administration. In the case of dry powder inhalers and aerosols, the dosage unit is determined by means of a valve which delivers a metered amount. Units in accordance with the invention are typically arranged to administer a metered dose or "puff" containing from ^g to 100mg of the compound of list (I). The overall daily dose will typically be in the range ^g to 200mg which may be administered in a single dose or, more usually, as divided doses throughout the day. 4-[2-(5-Amino-1 H-pyrazol-4-yl)-4-chlorophenoxy]-5-chloro-2-fluoro-N-(1,3-thiazol-4-yl)benzenesulfonamide or a pharmaceutically acceptable salt thereof, such as the tosylate salt, may also be administered directly to the eye or ear, typically in the form of drops of a micronised suspension or solution in isotonic, pH-adjusted, sterile saline. Other formulations suitable for ocular and aural administration include ointments, biodegradable (e.g. absorbable gel sponges, collagen) and non-biodegradable (e.g. silicone) implants, wafers, lenses and particulate or vesicular systems, such as niosomes or liposomes. A polymer such as crossed-linked polyacrylic acid, polyvinylalcohol, hyaluronic acid, a cellulosic polymer, for example, hydroxypropylmethylcellulose, hydroxyethylcellulose, or methyl cellulose, or a heteropolysaccharide polymer, for example, gelan gum, may be incorporated together with a preservative, such as benzalkonium chloride. Such formulations may also be delivered by iontophoresis. 4-[2-(5-Amino-1 H-pyrazol-4-yl)-4-chlorophenoxy]-5-chloro-2-fluoro-N-(1,3-thiazol-4-yl)benzenesulfonamide or a pharmaceutically acceptable salt thereof, such as the tosylate salt, may be combined with soluble macromolecular entities, such as cyclodextrin and suitable derivatives thereof or polyethylene glycol-containing polymers, in order to improve their solubility, dissolution rate, taste-masking, bioavailability and/or stability for use in any of the aforementioned modes of administration. Drug-cyclodextrin complexes, for example, are found to be generally useful for most dosage forms and administration routes. Both inclusion and non-inclusion complexes may be used. As an alternative to direct complexation with the drug, the cyclodextrin may be used

as an auxiliary additive, i.e. as a carrier, diluent, or solubiliser. Most commonly used for these purposes are alpha-, beta- and gamma-cyclodextrins, examples of which may be found in I nternational Patent Applications Nos. WO 91/1 1 172, WO 94/02518 and WO 98/55148. For administration to human patients, the total daily dose of 4-[2-(5-amino-1 H-pyrazol-4-yl)-4-chlorophenoxy]-5-chloro-2-fluoro-N-(1,3-thiazol-4-yl)benzenesulfonamide or a pharmaceutically acceptable salt thereof, such as the tosylate salt, is typically in the range 1 mg to 10g, such as 10mg to 1 g, for example 25mg to 500mg depending, of course, on the mode of administration and efficacy. The total daily dose may be administered in single or divided doses and may, at the physician's discretion, fall outside of the typical range given herein, depending on the age, weight and response of the particular patient. 4-[2-(5-amino-1 H-pyrazol-4-yl)-4-chlorophenoxy]-5-chloro-2-fluoro-N-(1,3-thiazol-4-yl)benzenesulfonamide or a pharmaceutically acceptable salt thereof, such as the tosylate salt, may be usefully combined with another pharmacologically active compound, or with two or more other pharmacologically active compounds, for the treatment of gout. Such combinations offer the possibility of significant advantages, including patient compliance, ease of dosing and synergistic activity. In the combinations that follow the compound of the invention may be administered simultaneously, sequentially or separately in combination with the other therapeutic agent or agents. 4-[2-(5-Amino-1 H-pyrazol-4-yl)-4-chlorophenoxy]-5-chloro-2-fluoro-N-(1,3-thiazol-4-yl)benzenesulfonamide, or a pharmaceutically acceptable salt thereof, such as the tosylate salt, may be administered in combination with one or more agents selected from:

- an anti-inflammatory drug such as an NSAID (e.g. celecoxib), colchicine or a steroid;
- a xanthine oxidase inhibitor (e.g. allopurinol, febuxostat or tisopurine) or a purine nucleoside phosphorylase (PNP) inhibitor (e.g. ulodesine);
- a uricase (e.g. pegloticase or rasburicase); or a
- a uricosuric, such as an agent that inhibits the transporters responsible for renal reabsorption of uric acid back into the blood, such as benziodarone, isobromindione, probenecid and sulphinpyrazone, or a URAT-1 inhibitor (e.g. benzbromarone).

It is to be appreciated that all references herein to treatment include curative, palliative and prophylactic treatment. 4-[2-(5-Amino-1 H-pyrazol-4-yl)-4-chlorophenoxy]-5-chloro-2-fluoro-N-(1,3-thiazol-4-yl)benzenesulfonamide may be prepared by any method known in the art for the preparation of compounds of analogous structure and, in particular, by the specific methods described in WO2010/079443, such as that set out in Example 788. The invention is illustrated by the following non-limiting examples.

Example 1 - Preparation of 4-r2-(5-amino-1 H-pyrazol-4-yl)-4-chlorophenoxyl-5-chloro-2-fluoro-N-(1,3-thiazol-4-yl)benzenesulfonamide tosylate: To 4-[2-(5-amino-1 H-pyrazol-4-yl)-4-chlorophenoxy]-5-chloro-2-fluoro-N-(1,3-thiazol-4-yl)benzenesulfonamide (example 788 WO2010/079443, 36.75 g, 73.45 mmol) in ethyl acetate (20 mL/g, 735 mL) was added methanol (3 mL/g, 1 10.25 mL) and the mixture heated to 50°C. A solution of p-toluenesulfonic acid monohydrate (13.27 g, 69.77 mmol) in methanol (2 mL/g, 73.50 mL) was added to the reaction mixture over 6 minutes via dropping funnel followed by addition of further methanol (1 mL/g, 36.75 mL). The reaction mixture was cooled to room temperature, filtered under vacuum and the solid washed with ethyl acetate:methanol (9: 1, 2 x 37 mL). The solid was dried in vacuo at 50°C overnight to provide the title compound as a free flowing off-white solid (43.99 g, 65.41 mmol, 89%). Details of the spectroscopic analysis of the tosylate salt of 4-[2-(5-amino-1 H-pyrazol-4-yl)-4-chlorophenoxy]-5-chloro-2-fluoro-N-(1,3-thiazol-4-yl)benzenesulfonamide follow: Infrared (I R) Spectroscopy: The infrared absorption spectrum was recorded using single reflection Attenuated Total Reflectance (ATR). The spectrum was acquired at 4 cm-1 resolution using a ThermoNicolet Avatar 360 FT IR spectrometer and a Smart Golden Gate™ accessory. This approach required no sample preparation. The spectrum is shown in Figure 1 of WO/2013/057722. Mass Spectrometry (MS): The full scan mass spectra are presented in Figure 2 of WO/2013/057722and Figure 3 of WO/2013/057722and were obtained by electrospray positive (ES+) and negative (ES-) ionization respectively. Data were recorded using a Bruker MaXis Quadrupole Time of Flight mass spectrometer fitted with an electrospray source. Internal calibration was performed using a sodium formate solution, which gave an observed maximum mass deviation of 0.2mDa (ES+) and 0.3mDa (ES-) over the mass range m/z 113 to m/z 997. The accurate mass measurement, theoretical monoisotopic mass and molecular formula of the observed adduct and fragment ions for the ES+ and ES- data are shown in Tables 1 and 2 respectively. Corresponding mass spectra are show in Figures 2 and 3 of WO/2013/057722 respectively.

Table 1. ES+ Accurate Mass Data

| Mass Measurement (m/z) | Ion Assignment | Formula of Ion | Theoretical Monoisotopic Mass (m/z) | Mass Deviation (mDa) |
|---|---|---|---|---|
| 499.9820 | [M+H]+ | $C_{18}H_{13}Cl_2FN_5O_3S_2$+ | 499.9815 | 0.5 |

(continued)

| Mass Measurement (m/z) | Ion Assignment | Formula of Ion | Theoretical Monoisotopic Mass (m/z) | Mass Deviation (mDa) |
|---|---|---|---|---|
| 521.9634 | [M+Na]+ | $C_{18}H_{12}Cl_2FN_5O_3S_2Na+$ | 521.9635 | 0.1 |

Table 2. ES- Accurate Mass Data

| Mass Measurement (m/z) | Ion Assignment | Formula of Ion | Theoretical Monoisotopic Mass (m/z) | Mass Deviation (mDa) |
|---|---|---|---|---|
| 171.0123 | [M-H]- for para toluenesulfonic acid | $C_7H_7O_3S-$ | 171.0121 | 0.2 |
| 365.0138 | [2M+Na-2H]-for paratoluenesulfonic acid | $C_{14}H_{14}O_6S_2Na-$ | 365.0135 | 0.3 |
| 477.9609 | [M-H-HF]- | $C_{18}H_{10}Cl_2N_5O_3S_2-$ | 477.9608 | 0.1 |
| 497.9673 | [M-H]- | $C_{18}H_{11}Cl_2FN_5O_3S_2-$ | 497.9670 | 0.3 |
| 519.9491 | [M+Na-2H]- | $C_{18}H_{10}Cl_2FN_5O_3S_2Na-$ | 519.9489 | 0.2 |
| 669.9865 | [M+ paratoluenesulfonic acid H]- | $C_{25}H_{19}Cl_2FN_5O_6S_3-$ | 669.9864 | 0.1 |
| 691.9684 | [M+ paratoluenesulfonic acid+Na-2H]- | $C_{25}H_{18}Cl_2FN_5O_6S_3Na-$ | 691.9683 | 0.1 |

Nuclear Magnetic Resonance (NMR) Spectroscopy: The proton (1H) NMR spectrum was acquired in solution in DMSO d6. Data were obtained at 30°C on a Bruker AVANCE I II 600 MHz NMR spectrometer equipped with a triple resonance cryogenic probe tuned for protons at 599.77 MHz. The spectrum was referenced to DMSO d5 (2.50 ppm). The 1H NMR spectrum, shown in Figure 4 of WO/2013/057722 and labeled with reference to the structure below, reveals the presence of 12 aromatic and three aliphatic (one CH3 group) protons. 1H chemical shift assignments are summarized in Table 3.

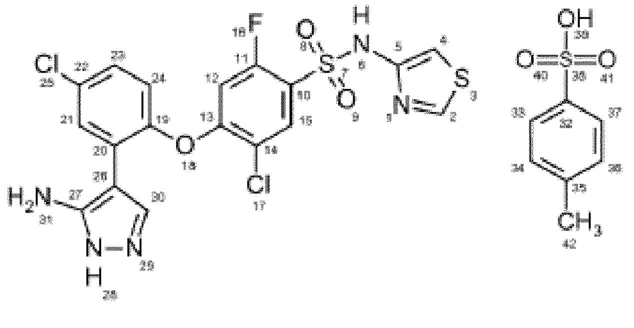

Table 3. 1 H Assignments in DMSO d6

| Atom Number | $^1$H Chemical Shift (ppm) | $^1$H Multiplicity | No. of $^1$H | J (Hz) |
|---|---|---|---|---|
| 42 | 2.29 | singlet | 3 | - |
| 12 | 6.95 | doublet | 1 | 10.8 |
| 4 | 7.10 | doublet | 1 | 2.2 |
| 34, 36 | 7.11 | doublet | 2 | 7.9 |
| 24 | 7.22 | doublet | 1 | 8.7 |

(continued)

| Atom Number | $^1$H Chemical Shift (ppm) | $^1$H Multiplicity | No. of $^1$H | J (Hz) |
|---|---|---|---|---|
| 37, 33, 23 | 7.43 - 7.50 | multiplet | 3 | - |
| | | | | |
| 21 | 7.65 | doublet | 1 | 2.6 |
| 15 | 7.91 | doublet | 1 | 7.1 |
| 30 | 7.94 | singlet | 1 | - |
| 2 | 8.91 | doublet | 1 | 2.2 |

Ultraviolet/Visible (UV/Vis) Spectrophotometry: The UV/Visible spectrum was acquired using a Hitachi U-3000 spectrophotometer in methanol at a concentration of 1.09 mg/100 ml. and is shown in Figure 5 of WO/2013/057722. Two Amax are observed at 281 and at 240 nm. Characterisation of the 4-r2-(5-amino-1 H-pyrazol-4-yl)-4-chlorophenoxyl-5-chloro-2-fluoro-N-(1,3-thiazol-4-yl)benzenesulfonamide tosylate by PXRD: The powder X-ray diffraction pattern of 4-[2-(5-amino-1 H-pyrazol-4-yl)-4-chlorophenoxy]-5-chloro-2-fluoro-N-(1,3-thiazol-4-yl)benzenesulfonamide tosylate was determined using a Bruker-AXS Ltd. D4 ENDEAVOR powder X-ray diffractometer fitted with an automatic sample changer, a theta-theta goniometer geometry, automatic beam divergence slit and a PSD Vantec-1 detector. The sample was prepared for analysis by mounting onto a low background silicon wafer specimen mount with a 0.5mm cavity. The specimen was rotated whilst being irradiated with copper KCH X-rays (wavelength = 1.5406 Angstroms) with the X-ray tube operated at 35kV/40mA. The analysis was in continuous mode set for data acquisition at 0.2 second count per 0.018° step size over a two theta range of 2° to 55° at room temperature. Peak search was carried out using the threshold and width parameters set to 1 and 0.3, respectively, within the Eva software released by Bruker-AXS. The instrument calibration was verified using a corundum reference standard (NIST: SRM 1976 XRD flat plate intensity standard). Due to differences in instruments, samples, and sample preparation, the peak values are reported herein with an estimate of variability for the peak values. This is common practice in the solid-state chemical arts because of the variation inherent in peak values. A typical variability of the 20 x-axis value for powder x-ray diffraction is on the order of plus or minus 0.2° 20. Variability in peak intensity is a result of how individual crystals are oriented in the sample container with respect to the external X-ray source (known as "preferred orientation"). This orientation effect does not provide structural information about the crystal. Additionally, one skilled in the art would also recognize that the intensities of characteristic peaks described above would change when the crystalline material of the present invention is mixed or diluted with additional components, such as pharmaceutical excipients. For this reason, the person skilled in the art would appreciate that the PXRD method described above may have to be optimized slightly to enable detection of the characteristic peaks within a mixture of components. This optimization may include the use of a more intense X-ray source (wavelength = 1.5406 Angstroms), a slightly different step size, or a slightly different step time. The skilled person will also appreciate that measurements using a different wavelength will result in different shifts according to the Bragg equation - $\eta\lambda$ = 2d sinO. Such further PXRD patterns generated by use of alternative wavelengths are considered to be alternative representations of the PXRD patterns of the crystalline materials of the present invention and as such are within the scope of the present invention. The PXRD pattern is shown in Figure 6 of WO/2013/057722. The main 20 peak positions and relative intensities are listed in Tables 4 and 4a.

Table 4: Characteristic diffraction peaks of 4-[2-(5-amino-1 H-pyrazol-4-yl)-4-chlorophenoxy]-5-chloro-2-fluoro-N-(1,3-thiazol-4-yl)benzenesulfonamide tosylate ($\pm$ 0.2° 2$\Theta$) with relative intensity cut-off$\geq$ 10%

| Angle (°2$\theta$) | Intensity % | | Angle (°2$\theta$) | Intensity % |
|---|---|---|---|---|
| 9.0 | 10.8 | | 29.5 | 18.9 |
| 10.7 | 16.6 | | 29.7 | 32.1 |
| 12.5 | 17.6 | | 30.0 | 12.5 |
| 12.9 | 18.1 | | 30.6 | 20.9 |
| 16.0 | 68.0 | | 31.4 | 13.5 |
| 16.6 | 16.7 | | 32.9 | 10.8 |
| | | | | |

(continued)

| Angle (°2θ) | Intensity % | | Angle (°2θ) | Intensity % |
|---|---|---|---|---|
| 17.5 | 27.0 | | 33.5 | 10.8 |
| 17.8 | 85.4 | | 33.7 | 15.8 |
| 18.1 | 16.3 | | 34.1 | 18.9 |
| 20.2 | 31.8 | | 34.4 | 12.1 |
| 20.8 | 69.1 | | 35.0 | 13.9 |
| 21.1 | 69.1 | | 35.3 | 16.6 |
| 21.4 | 100.0 | | 35.6 | 16 |
| 21.7 | 66.7 | | 36.6 | 11.5 |
| 22.9 | 15.8 | | 37.7 | 10.3 |
| 23.4 | 89.3 | | 38.0 | 12.7 |
| 24.0 | 50.1 | | 38.3 | 12.7 |
| | | | | |
| 24.5 | 17.7 | | 39.7 | 10.8 |
| 24.8 | 24.0 | | 41.0 | 10.3 |
| 25.3 | 15.4 | | 41.5 | 10.3 |
| 25.7 | 12.1 | | 41.8 | 11.1 |
| 26.0 | 10.4 | | 42.4 | 11.3 |
| 26.6 | 42.0 | | 43.6 | 11.1 |
| 27.4 | 29.6 | | 44.0 | 11.2 |
| 28.5 | 16.5 | | 45.1 | 10.9 |
| 29.2 | 14.4 | | 47.1 | 11.2 |

Table 4a: Characteristic diffraction peaks of 4-[2-(5-amino-1 H-pyrazol-4-yl)-4-chlorophenoxy]-5-chloro-2-fluoro-N-(1,3-thiazol-4-yl)benzenesulfonamide tosylate (± 0.2° 2θ) with less than 10 % relative intensity.

| Angle (°2θ) | Intensity % |
|---|---|
| 9.3 | 3.6 |
| 10.0 | 5.7 |
| 11.6 | 6.9 |
| 13.2 | 7.2 |
| 13.8 | 3.8 |
| 14.4 | 4.1 |

Example 2 - Preparation of 4-f2-(5-amino-1 H-pyrazol-4-yl)-4-chlorophenoxyl-5-chloro-2-fluoro-N-(1,3-thiazol-4-yl)benzenesulfonamide spray dried dispersion (SDD): Tetrahydrofuran (unstabilized, 14.5 kg) and water (0.76 kg) were added to a stainless-steel tank equipped with a top mount mixer. 4-[2-(5-Amino-1 H-pyrazol-4-yl)-4-chlorophenoxy]-5-chloro-2-fluoro-N-(1,3-thiazol-4-yl)benzenesulfonamide (742.4 g) was then added to the solution and mixed for at least 1 hour until all solids had fully dissolved. Hydroxypropyl methylcellulose acetate succinate (medium grade granular, 1338.4 g) was added to the solution and mixed until fully dissolved. The solution was then spray dried under nitrogen gas using the conditions tabulated below.

| Process | Process Parameters | Target | Target Range |
|---|---|---|---|
| (A) Preheating | Nitrogen drying-gas flow | 1850 g/min | 1550 to 2150 g/min |
| | Dryer inlet temperature ($T_{in}$) | 110°C | 100°C to 120°C |
| (B) Warm-Up/Shutdown | Nitrogen drying-gas flow | 1850 g/min | 1550 to 2150 g/min |
| | $T_{in}$ | 140°C | 100°C to 120°C |
| | Dryer outlet temperature ($T_{out}$) | 52°C | 47°C to 57°C |
| | Solvent atomization pressure | 600 psi | 450 to 750 psi |
| | Solvent feed rate | 140 g/min | 115 to 165 g/min |
| (C) Feed-Solution Processing | Nitrogen drying-gas flow | 1850 g/min | 1550 to 2150 g/min |
| | $T_{in}$ | 110°C | 100°C to 120°C |
| | $T_{out}$ | 45°C | 40°C to 50°C |
| | Solution atomization pressure | 500 psi | 400 to 600 psi |
| | Solution feed rate | 155 g/min | 130 to 180 g/min |

NB: 95:5 (w/w%) of tetrahydrofuran (unstabilized): Water was used for system start up/shutdown. The resulting SDD of 4-[2-(5-amino-1 H-pyrazol-4-yl)-4-chlorophenoxy]-5-chloro-2-fluoro-N-(1,3-thiazol-4-yl)benzenesulfonamide was then tray dried in a convection tray dryer at 40°C/50% relative humidity (RH) for a minimum of 6 hours, followed by a ramp up to 40°C/75%RH for an additional minimum of 25 hours. The SDD was stored at 2 to 8°C until required.

Example 3 - Preparation of dispersions for oral administration: (a) Using the tosylate salt of 4-r2-(5-Amino-1 H-pyrazol-4-yl)-4-chlorophenoxyl-5- chloro-2-fluoro-N-(1,3-thiazol-4-yl)benzenesulfonamide: Methylcellulose vehicle (0.5% w/v) was prepared as follows. Water for irrigation (600 ml.) was heated in a beaker to between 80°C and 90°C. Methylcellulose (5 g) powder was added, with stirring, until the powder had fully dispersed. The dispersion was then transferred into an ice bath and cooled rapidly whilst adding chilled water for irrigation (400 ml.) to give a clear solution. The dispersion for oral administration was prepared by weighing the required quantity of 4-[2-(5-amino-1 H-pyrazol-4-yl)-4-chlorophenoxy]-5-chloro-2-fluoro-N- (1,3-thiazol-4-yl)benzenesulfonamide tosylate (from 10mg to 2400mg) into an appropriately sized glass amber dosing bottle and adding a volume of vehicle (0.5% (w/v) methylcellulose). The volume of vehicle added was dependant on dose: 15 ml. for doses of drug in the range 10 mg to < 30 mg; and 50 ml. for doses of drug in the range 30 mg to 2400 mg, so that the drug concentration was in the range 0.6 to 50 mg/mL. The dispersion was stored at 2 to 8°C and administered within 72 hours directly from the dosing containers. After administration the glass dosing bottle was rinsed with two approximate equal aliquots of drinking water such that the total volume administered, including dosing volume, was 240 ml_. (b) Using the SDD of 4-[2-(5-Amino-1 H-pyrazol-4-yl)-4-chlorophenoxyl-5-chloro-2- fluoro-N-(1,3-thiazol-4-yl)benzenesulfonamide: Methylcellulose vehicle 0.5% (w/v) was prepared using the procedure set out in Example 3(a) above. The dispersion for oral administration was prepared by weighing the required quantity of 4-[2-(5-amino-1 H-pyrazol-4-yl)-4-chlorophenoxy]-5-chloro-2-fluoro-N- (1,3-thiazol-4-yl)benzenesulfonamide SDD into an appropriately sized glass amber dosing bottle and adding a volume of vehicle (0.5% (w/v) Methylcellulose). The volume of vehicle added was dependent on dose: 20 ml. to 100 ml. over the dose range 10 mg to 2400 mg, so that the drug concentration was in the range 0.6 to 50 mg/mL. The dispersion was stored at 2 to 8°C and administered within 72 hours directly from the dosing containers. After administration the glass dosing bottle was rinsed with two approximate equal aliquots of drinking water such that the total volume administered, including dosing volume, was 240 ml_. Biological activity: The ability of 4-[2-(5-amino-1 H-pyrazol-4-yl)-4-chlorophenoxy]-5-chloro-2-fluoro-N-(1,3-thiazol-4-yl)benzenesulfonamide and pharmaceutically acceptable salts thereof, such as the tosylate salt, to lower blood uric acid levels was demonstrated in the following experiments. Uric acid measurements were performed using a commercial colorimetric assay kit (Beckman Coulter).

Example 4 - Single dose study: a double-blind, randomized, placebo-controlled, crossover study in six cohorts of healthy subjects: Single doses of 4-[2-(5-amino-1 H-pyrazol-4-yl)-4-chlorophenoxy]-5-chloro-2-fluoro-N-(1,3-thiazol-4-yl)benzenesulfonamide SDD ('SDD') dispersion for oral administration, ranging from 10 mg to 2400 mg, were investigated. In addition, single doses of 4-[2-(5-amino-1 H-pyrazol-4-yl)-4-chlorophenoxy]-5-chloro-2-fluoro-N-(1,3-thiazol-4-yl)benzenesulfonamide tosylate (TS') dispersion for oral administration, ranging from 200 mg to 1000 mg, were also investigated. The following doses were investigated and all doses were given in the fasted state with the exception of 200 mg and 1000 mg SDD dispersions, which were given in both the fasted state and after a high-fat meal ('fed').

Cohort 1 : 10 mg SDD, 100 mg SDD, 300 mg SDD, 200 mg TS, placebo
Cohort 2: 30 mg SDD, 300 mg SDD, 200 mg SDD (fed), placebo
Cohort 3: 100 mg SDD, 200 mg SDD, 300 mg SDD, placebo
Cohort 4: 450 mg SDD, 600 mg SDD, 800 mg SDD, 1000 mg SDD, placebo
Cohort 5: 600 mg TS, 1000 mg TS, 1000 mg SDD (fed)
Cohort 6: 1250 mg SDD, 1600 mg SDD, 2000 mg SDD, 2400 mg SSD, placebo

A total of 61 subjects (all male) participated and all received at least one dose of SDD or TS dispersion for oral administration.

Mean data of uric acid levels per dose group are given as follows:

- SDD fasted: Table 5
- SDD fed and fasted Table 6
- TS Table 7

Table 5

| Dose (mg) | Time (hours) | Mean uric acid (mg/dl) |
|---|---|---|
| 0 | 0 | 5.54 |
| 0 | 48 | 5.68 |
| 10 | 0 | 5.59 |
| 10 | 48 | 5.04 |
| 30 | 0 | 5.64 |
| 30 | 48 | 5.85 |
| 100 | 0 | 5.70 |
| 100 | 48 | 5.46 |
| 200 | 0 | 5.62 |
| 200 | 48 | 5.10 |
| 300 | 0 | 5.63 |
| | | |
| 300 | 48 | 4.77 |
| 450 | 0 | 5.78 |
| 450 | 48 | 4.47 |
| 600 | 0 | 6.00 |
| 600 | 48 | 4.55 |
| 800 | 0 | 5.88 |
| 800 | 48 | 4.15 |
| 1000 | 0 | 5.87 |
| 1000 | 48 | 3.73 |
| 1250 | 0 | 5.81 |
| 1250 | 48 | 3.65 |
| 1600 | 0 | 5.52 |
| 1600 | 48 | 3.64 |
| 2000 | 0 | 5.40 |
| 2000 | 48 | 3.55 |

Table 6

| Dose (mg) | Time (hours) | Mean uric acid (mg/dL) |
|---|---|---|
| 0 | 0 | 5.59 |
| 0 | 48 | 5.71 |
| 10 | 0 | 5.59 |
| 10 | 48 | 5.04 |
| 30 | 0 | 5.64 |
| 30 | 48 | 5.85 |
| 100 | 0 | 5.70 |
| 100 | 48 | 5.46 |
| 200 | 0 | 5.62 |
| 200 | 48 | 5.10 |
| 200 Fed | 0 | 5.92 |
| 200 Fed | 48 | 4.92 |
| 300 | 0 | 5.63 |
| 300 | 48 | 4.77 |
| 450 | 0 | 5.78 |
| 450 | 48 | 4.47 |
| 600 | 0 | 6.00 |
| 600 | 48 | 4.55 |
| 800 | 0 | 5.88 |
| 800 | 48 | 4.15 |
| 1000 | 0 | 5.87 |
| 1000 | 48 | 3.73 |
| 1000 Fed | 0 | 6.41 |
| 1000 Fed | 48 | 4.13 |
| 1250 | 0 | 5.81 |
| 1250 | 48 | 3.65 |
| 1600 | 0 | 5.52 |
| 1600 | 48 | 3.64 |
| 2000 | 0 | 5.48 |
| 2000 | 48 | 3.82 |
| 2400 | 0 | 5.68 |
| 2400 | 48 | 3.60 |

Table 7

| Dose (mg) | Time (hours) | Mean uric acid (mg/dL) |
|---|---|---|
| 200 | 0 | 5.92 |
| 200 | 48 | 5.43 |

(continued)

| Dose (mg) | Time (hours) | Mean uric acid (mg/dL) |
|---|---|---|
| 600 | 0 | 6.21 |
| 600 | 48 | 5.04 |
| 1000 | 0 | 6.40 |
| 1000 | 48 | 4.86 |

A dose related decrease in uric acid in blood was noted at 48 hours postdose. Although values generally remained within the normal range (3.5 to 7.2 mg/dL) at doses of 10 to 1000 mg, at doses of 1250 to 2400 mg the uric acid level reductions were more marked, with at least half of the subjects having values below the lower limit of normal (LLN) at 48 hours postdose. All predose and follow-up values were above the LLN. All of the subjects on placebo (n=42) had uric acid values within the normal range, except for one subject who was just below the LLN at 48 h postdose.

Example 5 - Multiple dose study: a double-blind, randomized, placebo-controlled, study in healthy subjects: Multiple oral doses of 100 mg twice daily (BI D), 300 mg BI D, and 600 mg BI D of 4-[2-(5-amino-1 H-pyrazol-4-yl)-4-chlorophenoxy]-5-chloro-2-fluoro-N-(1,3-thiazol-4-yl)benzenesulfonamide SDD dispersion for oral administration, and placebo, were investigated for 14 days. Subjects were fasted overnight prior to the morning doses and for at least 2 hours prior to the evening doses. Food was withheld for at least 2 hours postdose. A total of 30 subjects (all male) were enrolled in the study and 27 subjects completed the study (three subjects were withdrawn due to adverse events during treatment with 600 mg BI D 4-[2-(5-amino-1 H-pyrazol-4-yl)-4-chlorophenoxy]-5-chloro-2-fluoro-N-(1,3-thiazol-4-yl)benzenesulfonamide).

Mean data of uric acid levels per dose group are given in Table 8 and Figure 7 of WO/2013/057722.

Table 8

| Dose (mg) | Time (hours) | Mean uric acid (mg/dL) |
|---|---|---|
| 0 | 0 | 5.47 |
| 0 | 4 | 5.40 |
| 0 | 7 | 4.08 |
| 0 | 10 | 5.40 |
| 0 | 14 | 5.23 |
| 0 | 16 | 5.12 |
| 0 | 26 | 5.33 |
| 100 | 0 | 4.85 |
| 100 | 4 | 3.91 |
| 100 | 7 | 3.38 |
| 100 | 10 | 4.13 |
| 100 | 14 | 4.04 |
| 100 | 16 | 5.05 |
| | | |
| 100 | 26 | 5.30 |
| 300 | 0 | 5.88 |
| 300 | 4 | 3.44 |
| 300 | 7 | 3.50 |
| 300 | 10 | 3.73 |
| 300 | 14 | 3.64 |
| 300 | 16 | 4.66 |

(continued)

| Dose (mg) | Time (hours) | Mean uric acid (mg/dL) |
|---|---|---|
| 300 | 26 | 6.73 |
| 600 | 0 | 5.56 |
| 600 | 4 | 2.31 |
| 600 | 7 | 2.45 |
| 600 | 10 | 2.74 |
| 600 | 14 | 2.56 |
| 600 | 16 | 4.34 |
| 600 | 26 | 6.43 |

A dose related decrease in uric acid in blood was apparent by Day 4 (first postdose assessment) with the lowest mean values occurring on Days 4 or 7. Five of 8 subjects had uric acid values below the LLN on Day 7 at 100 mg BID (actual range for subjects with low uric acid was 2.6 to 3.4 mg/dL; one subject was below the limit at baseline; actual value 2.7 mg/dL) and at 300 mg (actual range for subjects with low uric acid was 2.2 to 3.4 mg/dL). All subjects had uric acid values below the LLN on Days 4 and 7 of 600 mg BID dosing (actual range was <1.5 to 3.0 mg/dL). Values generally increased on Days 10 and 14 despite continued dosing, although all except 1 subject had returned to the normal range by Day 16 (2 days post-last dose). This remaining subject had the lowest uric value at baseline (4.7 mg/dL) with subsequent values of 2.2 mg/dL (Day 4), <1.5 mg/dL (Day 7), 1.6 mg/dL (Day 10), 3.1 mg/dL (Day 14) and 4.9 mg/dL at follow-up. All subjects receiving placebo (n=6) had uric acid concentrations within the normal range at all timepoints.

Example 6 - Multiple dose study: a double-blind, randomized, placebo-controlled, study in healthy subjects and elderly subjects: Multiple oral doses of 4-[2-(5-amino-1 H-pyrazol-4-yl)-4-chlorophenoxy]-5-chloro-2-fluoro-N-(1,3-thiazol-4-yl)benzenesulfonamide TS dispersion for oral administration, and placebo, were investigated for 14 days, as follows:

- healthy subjects, 300 mg twice daily (BID,)
- healthy subjects, 450 mg BI D, and
- elderly subjects, 300 mg BID.

Subjects were fasted overnight prior to the morning doses and for at least 2 hours prior to the evening doses. Food was withheld for at least 2 hours postdose. A total of 49 subjects were enrolled in the study of which 39 received 4-[2-(5-amino-1 H-pyrazol-4-yl)-4-chlorophenoxy]-5-chloro-2-fluoro-N-(1,3-thiazol-4-yl)benzenesulfonamide TS and 10 received placebo. Mean blood uric acid level and urine excretion data, per dose group, are given in Table 9. Figure 8 of WO/2013/057722 shows percent uric acid excreted in urine.

Table 9

| Dose (mg) | Time (days) | Mean uric acid (mg/dL) | Fraction excreted of uric acid (%) |
|---|---|---|---|
| 0 | 0 | 5.37 | NA |
| 0 | 3 | 5.33 | NA |
| 0 | 6 | 5.38 | NA |
| 0 | 8 | 5.61 | NA |
| 0 | 10 | 5.47 | NA |
| 0 | 14 | 5.39 | NA |
| 300 | 0 | 5.56 | NA |
| 300 | 1 | NA | 8.44 |
| 300 | 3 | 3.50 | NA |
| 300 | 6 | 3.23 | 17.41 |
| | | | |

(continued)

| Dose (mg) | Time (days) | Mean uric acid (mg/dL) | Fraction excreted of uric acid (%) |
|---|---|---|---|
| 300 | 8 | 3.54 | NA |
| 300 | 10 | 3.79 | 14.99 |
| 300 | 14 | 3.78 | 6.43 |
| 450 | 0 | 5.89 | NA |
| 450 | 1 | NA | 8.61 |
| 450 | 3 | 3.05 | NA |
| 450 | 6 | 3.00 | 19.81 |
| 450 | 8 | 3.21 | NA |
| 450 | 10 | 3.45 | 17.09 |
| 450 | 14 | 3.56 | 7.69 |
| 300 elderly | 0 | 5.43 | NA |
| 300 elderly | 1 | NA | 6.17 |
| 300 elderly | 3 | 3.41 | NA |
| 300 elderly | 6 | 2.99 | 13.79 |
| 300 elderly | 8 | 3.57 | NA |
| 300 elderly | 10 | 3.37 | 13.02 |
| 300 elderly | 14 | 3.59 | 7.66 |

Concentrations of uric acid in blood decreased following dosing with 4-[2-(5-amino-1 H-pyrazol-4-yl)-4-chlorophenoxy]-5-chloro-2-fluoro-N-(1,3-thiazol-4-yl)benzenesulfonamide. All subjects had blood uric acid values above the LLN on Day 1. Subjects who received placebo had blood uric acid concentrations above the LLN throughout the study. The median blood uric acid concentrations fell below LLN on Day 3 for subjects who received 450 mg BID 4, and the median was below the LLN for all cohorts (300 mg and 450 mg BID) on Day 6. Blood uric acid concentrations returned to above the LLN for all subjects within 2 days of cessation of 4-[2-(5-amino-1 H-pyrazol-4-yl)-4-chlorophenoxy]-5-chloro-2-fluoro-N-(1,3-thiazol-4-yl)benzenesulfonamide dosing (i.e. by Day 16). Uric acid was also measured in urine collected over 24 hours prior to dosing on Day 1, and then on Days 6, 14 and 16. The percent excreted fraction of uric acid in urine was calculated and analyzed with a linear mixed effects model. A summary of these data is presented in Figure 8 of WO/2013/057722. The data suggest that the excreted fraction of uric acid in urine increases during dosing with 4-[2-(5-amino-1H-pyrazol-4-yl)-4-chlorophenoxy]-5-chloro-2-fluoro-N-(1,3-thiazol-4-yl)benzenesulfonamide and returns to baseline by Day 16.

Example 7 - URAT-1 Inhibitor activity: The potency of 4-[2-(5-amino-1H-pyrazol-4-yl)-4-chlorophenoxy]-5-chloro-2-fluoro-N-(1,3-thiazol-4-yl)benzenesulfonamide as an inhibitor of the URAT-1 tranporter was deermined as follows. HEK293 cells were grown in medium consisting of Dulbecco's modified Eagle medium (DMEM) with L-GlutaMax (4.5 g of glucose per litre), supplemented with heat-inactivated foetal calf serum (10 % v/v), 100 U/mL penicillin and 100 μg/mL streptomycin. The HEK cells were routinely cultured in 75 cm2 tissue culture flasks in a humidified incubator at approximately 37°C in approximately 95% air/5% C02. Near confluent HEK cell cultures were harvested by trypsinisation, resuspended in culture medium and the process was repeated once or twice weekly to provide sufficient cells for use.

For uptake experiments, HEK293 cells were seeded onto poly-D-Lysine-coated 24-well plates at a density of 4 x 105 cells per well. The cells were cultured for 1 day at approximately 37°C in a humidified incubator containing approximately 5% C02 in air. Thereafter, cells were transfected with either pcDNA3.1 /hygro/URAT1 (HEK-U RAT1 cells) or pcDNA3.1/hygro (HEK-control cells) using Lipofectamine 2000 reagent. After approximately 24 hours at approximately 37°C in a humidified incubator containing approximately 5% C02 in air, cells were used for experiments. One day after transfection, culture medium was removed from the wells and the cells were pre-incubated with 0.2 ml. of chlorine-free incubation medium (125 mM Na-gluconate, 4.8 mM K-gluconate, 1.3 mM Ca-gluconate, 1.2 mM KH2P04, 1.2 mM MgS04, 5.6 mM D-glucose, 25 mM HEPES, pH 7.4), in the absence and presence of 4-[2-(5-amino-1H-pyrazol-4-yl)-4-chlorophenoxy]-5-chloro-2-fluoro-N-(1,3-thiazol-4-yl)benzenesulfonamide (0 - 30 μM), for 15 minutes at approximately 37°C. Thereafter, the incubation medium was removed and 0.2 ml. of chlorine-free incubation medium containing

[14C]-uric acid (20 μM) was added, in triplicate, in the absence and presence of 4-[2-(5-amino-1H-pyrazol-4-yl)-4-chlorophenoxy]-5-chloro-2-fluoro-N-(1,3-thiazol-4-yl)benzenesulfonamide (0 - 30 μM). Cells were incubated for 2 minutes at approximately 37°C. At the end of the incubation, the medium was aspirated and the monolayers rapidly rinsed twice with 1 ml. of ice-cold incubation medium. Subsequently, the cells were solubilised in 0.5 ml. of 0.5 N NaOH, and aliquots of cell lysate samples from each well were collected in scintillation vials. The concentrations of [14C]-uric acid were determined by liquid scintillation counting (LSC). The inhibition of [14C]-uric acid transport was also determined in the presence of the known inhibitor benzbromarone (30 μM). Final organic solvent were less than 1 % (v/v). The protein content of solubilised HEK cells was determined by the Bradford method using Bio-Rad Bradford Reagent with bovine serum albumin (BSA) as the protein standard (concentration range 0-1 mg/mL). The BSA solution or the solubilised cells were mixed with diluted dye reagent concentrate (Bio-Rad). The absorbance was measured at 595 nm after incubation at room temperature for 10 minutes. The amount of radioactivity present in cell lysate samples was determined by liquid scintillation counting (LSC). Liquid scintillant (Hionic Fluor™) was added to all samples and radioactivity was determined by LSC on a Tri-Carb 3100TR liquid scintillation counter using QuantaSmart™ software in which all counts were converted to DPM using tSIE/AEC (transformed Spectral Index of external standards coupled to Automatic Efficiency Correction). Calibration procedures for the instruments are established at the testing facilities. All samples were counted for at least 2 minutes. Background values were measured with each sample sequence using liquid scintillant in the absence of sample. The accumulation (pmol/mg protein) of [14C]-Uric acid into HEK cells was calculated and the IC50 values, defined as the concentration of inhibitor required for 50% inhibition of transport, was calculated using GraphPad Prism version 4.00 using the Hill equation.

Data: The inhibition of uric acid uptake by 4-[2-(5-amino-1H-pyrazol-4-yl)-4-chlorophenoxy]-5-chloro-2-fluoro-N-(1,3-thiazol-4-yl)benzenesulfonamide measured by the methodology described above, normalised to the standard compound benzbromarone, is 3.54uM. The invention relates to an administration unit comprising tosylate salt of 4-[2-(5-amino-1H-pyrazol-4-yl)-4-chlorophenoxy]-5-chloro-2-fluoro-N-(1,3-thiazol-4-yl)benzenesulfonamide. The administration unit according to the invention is useful for treating various diseases and disorders which include but are not limited to diseases associated with high blood uric acid levels such as hyperuricemia, including renal disorders associated with hyperuricemia (e.g. urinary calculi); and gout, including gouty tophus and gouty arthritis.

SPECIFIC INORMATION CONCERNING ASPECT (IV) OF THE INVENTION

Aspect (iv) of the invention concerns forms of {drug4}, especially form X of compound of formula (IV), which is named (1,1-dioxo-1lambda6-thiomorpholin-4-yl)-{6-[3-(4-fluoro-phenyl)-5-methyl-isoxazol-4-ylmethoxy]-pyridin-3-yl]-methanone and which is useful for treating treatment or prevention of acute and/or chronic neurological disorders, cognitive disorders, Alzheimer's disease, memory deficits, schizophrenia, positive, negative and/or cognitive symptoms associated with schizophrenia, bipolar disorders, autism, etc. Form X of compound of formula (IV) is described in WO/2013/057124, which is incorporated by reference. As used herein, form X of compound of formula (IV) is a crystalline form of compound of formula (IV) selected from list of crystalline forms described in WO/2013/057124, prefereably form A ({drug4a}), form B ({drug4b}), form C ({drug4c}), form D ({drug4d}), form E ({drug4e}), amorphous form ({drug4f}) and / or inclusion complex ({drug4g}). Compound of formula (IV) is described as (1,1-dioxo-1lambda6-thiomorpholin-4-yl)-{6-[3-(4-fluoro-phenyl)-5-methyl-isoxazol-4-ylmethoxy]-pyridin-3-yl]-methanone as depicted in formula (IV):

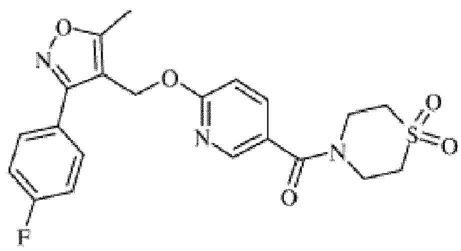

as well optionally as their solvates, inclusion complexes with other suitable compounds, solvates of their inclusion complexes with other suitable compounds. Form X of compound of formula (IV) can be characterized as described in WO/2013/057124 and also produced as described in WO/2013/057124: The instant invention relates to novel solid forms of compounds of formula (IV)

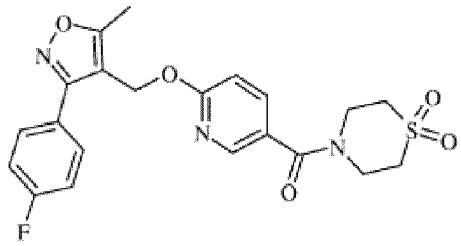

as well as their solvates, inclusion complexes with other suitable compounds, solvates of their inclusion complexes with other suitable compounds, processes for their manufacture, 5 pharmaceutical compositions containing these solid forms, and their use as medicaments.

Polymorphism is the ability of a compound to crystallize as more than one distinct crystal species. Different polymorphic forms (or polymorphs) have different arrangements or conformations of the molecules in the crystal lattice. If a solid does not possess a distinguishable 0 crystal lattice and the molecular arrangement of molecules is disordered, it is considered amorphous. The amorphous state is structurally similar to the liquid state [W. McCrone, Phys. Chem. Org. Solid State (1965) 2:725767]. Polymorphic forms of a drug substance can have different chemical, physical and physicotechnical properties. Differences can result from e.g. packing of molecules in the crystal 5 structure (density, refractive index, conductivity, hygroscopicity), thermodynamic properties (melting point, heat capacity, vapor pressure, solubility), kinetic properties (dissolution rate, stability), surface properties (surface free energy, interfacial tension, shape, morphology), and mechanical properties (compactibility, tensile strength). These properties can have a direct effect on the ability to process and manufacture the active pharmaceutical ingredient (API) and the 0 drug product. Polymorphism further has pharmacological implications due to altered solid state properties and suitability for a particular formulation. Thus, polymorphism of an API can affect the quality, safety, efficacy and developability of a drug product and is therefore of fundamental importance [D. Giron et ah, J. Therm. Anal. Cal. (2004) 77:709]. In addition to polymorphic modifications, an API can be crystallized in different salt forms with an appropriate counterion. Similar to polymorphism, salt forms are varying from each other in the degree of solubility and many other physical and chemical factors, as denoted above. As compared to the free acid or free base of the API, an appropriate salt form might provide improved aqueous solubility, dissolution rate, hygroscopicity, chemical stability, melting point, or mechanical properties. Solvates, also known as pseudopolymorphs, are crystal forms having either stoichiometric or nonstoichiometric amounts of a solvent incorporated in the crystal lattice. If the incorporated solvent is water, the solvate is commonly known as a hydrate. Salts and inclusion complexes both are multicomponent systems. Salts are formed by ionic bonding interactions with complete proton transfer between acid and base whereas in inclusion complexes the molecules are neutral in the crystalline state and are connected mainly through hydrogen bonds or Van der Waals interactions [S.L. Morissette et al., Adv. Drug Del. Rev. (2004) 56:275-300]. Cyclodextrins are comprised of six, seven, or eight glucose units, respectively, and have hydrophilic cavity exteriors and hydrophobic cavity interiors [V.J. Stella et ah, Adv. Drug Del. Rev. (2007) 59:677-694]. These properties are responsible for their aqueous solubility and ability to incorporate hydrophobic molecular moieties within their cavities. Cyclodextrins can be employed as inclusion complex formers for inclusion complexes with APIs, in which the API is trapped by a cavity of cyclodextrin molecules. It is reported in the literature that the crystal structures of cyclodextrin inclusion complexes are typically dominated by the spatial arrangement of the host molecules. Thereby the cyclodextrin may form a defined packing arrangement similar to a crystalline state, whereas the API does not occupy well defined lattice positions [T. Uyar et al., Cryst. Growth Des. (2006) 6:1113-1119, T. Toropainen et al., Pharm. Res. (2007) 24:1058-1066]. Among the commercially available cyclodextrins, gamma-cyclodextrin (gamma-CD) is reported to be stable and has been found safe for oral administration [I.C. Munro et al., Regulatory Toxicology and Pharmacology (2004) 39:S3-S13]. However, gamma-cyclodextrins are not used in marketed drug preparations up to now. A monograph has only recently (12/2008) been included in the European pharmacopoeia. The formation of inclusion complexes with cyclodextrins is not predictable and needs comprehensive experimental investigation. In those cases where inclusion complexes with gamma-cyclodextrin are formed, most active pharmaceutical ingredients form a 2: 1 complex (ratio between inclusion complex former and API). The formation of cyclodextrin inclusion complexes and their guest to host stoichiometries are highly dependent on the molecular structures and the geometrical sizes of the guest molecules [T. Uyar et ah, Cryst. Growth Des. (2006) 6:1113-1119].

The compound of formula (IV), its manufacture, its pharmacological activity as inverse agonists of the GABA A a5 receptor, and its use for the treatment, prevention and/or delay of progression of various central nervous system (CNS) conditions have been described in WO 2009/071476. Based on its physicochemical properties, the compound of formula (IV), as described in WO 2009/071476, is a BCS 2 compound, exhibiting low aqueous solubility and high permeability, according to the biopharmaceutical classification system [G.L. Amidon, H. Lennernas, V.P. Shah, J.R. Crison, Pharm. Res. (1995) 12:413-420]. Hence the limited oral bioavailability is a major issue for oral formulation development. If anhydrous solid forms of the compound of formula (IV), as described in WO 2009/071476, are selected

for clinical development, a physical instability in terms of hydrate formation during pharmaceutical processing and/or storage of the drug product is possible. Anhydrous solid form A of the compound of formula (IV) as described in WO 2009/071476 and herein, has further been found to be only metastable and hence may convert into different solid forms. Hence there is a need to find new solid forms which feature enhanced physicochemical properties and improved bioavailability. Further, the discovery of new solid forms of an API (polymorphs, solvates, salts, inclusion complexes) enlarges the repertoire of materials that a formulation scientist has available with which to design a pharmaceutical dosage form of a drug with a targeted release profile or other desired characteristics. Therefore, there is a need to find more solid forms of the compound of formula (IV). It has now been surprisingly found, that under certain conditions new solid forms, particularly crystalline or amorphous forms, most particularly crystalline forms, of the compound of formula (IV) may be obtained, which are described hereinafter, which have advantageous utilities and properties. They exhibit substantially different and superior physical and physicochemical properties which may be beneficial in various aspects relevant in API and drug product development, e.g. for dissolution of API, stability and shelf live of API and drug product, and/or facilitated routes of manufacturing or purification. The instant invention provides novel solid forms of the compound of formula (IV) with improved solubility, dissolution rate, oral bioavailability as well as increased stability of the API. In addition, the instant invention provides novel inclusion complexes of compounds of formula (IV) with cyclodextrins. Such inclusion complexes further feature improved dissolution rate and bioavailability. The new solid forms as described herein are distinguishable by X-ray powder diffraction, crystal structure analysis, vibrational spectroscopy, magnetic resonance and mass spectroscopy, calorimetry, thermogravimmetry, dynamic vapour sorption as well as by microscopy. Unless otherwise defined, all technical and scientific terms used herein have the same meaning as commonly understood by one of ordinary skill in the art to which this invention belongs. Although methods and materials similar or equivalent to those described herein can be used in the practice or testing of the invention, suitable methods and materials are described below. All publications, patent applications, patents, and other references mentioned herein are incorporated by reference in their entirety. The nomenclature used in this Application is based on IUPAC systematic nomenclature, unless indicated otherwise. Any open valency appearing on a carbon, oxygen, sulfur or nitrogen atom in the structures herein indicates the presence of a hydrogen, unless indicated otherwise. The term "optional" or "optionally" denotes that a subsequently described event or circumstance may but need not occur, and that the description includes instances where the event or circumstance occurs and instances in which it does not. The term "substituent" denotes an atom or a group of atoms replacing a hydrogen atom on the parent molecule. The term "substituted" denotes that a specified group bears one or more substituents. Where any group may carry multiple substituents and a variety of possible substituents is provided, the substituents are independently selected and need not to be the same. The term "unsubstituted" means that the specified group bears no substituents. The term "optionally substituted" means that the specified group is unsubstituted or substituted by one or more substituents, independently chosen from the group of possible substituents. When indicating the number of substituents, the term "one or more" means from one substituent to the highest possible number of substitution, i.e. replacement of one hydrogen up to replacement of all hydrogens by substituents. The term "halogen" denotes fluoro, chloro, bromo, or iodo. Particular halogen is fluoro. The term "alkyl" denotes a monovalent linear or branched saturated hydrocarbon group of 1 to 12 carbon atoms. In particular embodiments, alkyl has 1 to 7 carbon atoms, and in more particular embodiments 1 to 4 carbon atoms. Examples of alkyl include methyl, ethyl, propyl, isopropyl, n-butyl, iso-butyl, sec-butyl, or tert-butyl. Particular alkyl is methyl. The term "alkoxy" denotes a group of the formula -O-R', wherein R' is an alkyl group. Examples of alkoxy moieties include methoxy, ethoxy, isopropoxy, and tert-butoxy. The term "haloalkyl" denotes an alkyl group wherein at least one of the hydrogen atoms of the alkyl group has been replaced by same or different halogen atoms, particularly fluoro atoms. Examples of haloalkyl include monofluoro-, difluoro- or trifluoro-methyl, -ethyl or -propyl, for example 3,3,3-trifluoropropyl, 2-fluoroethyl, 2,2,2-trifluoroethyl, fluoromethyl, or trifluoromethyl. The term "perhaloalkyl" denotes an alkyl group where all hydrogen atoms of the alkyl group have been replaced by the same or different halogen atoms. The term "hydroxyalkyl" denotes an alkyl group wherein at least one of the hydrogen atoms of the alkyl group has been replaced by a hydroxy group. Examples of hydroxyalky include hydroxymethyl, 2-hydroxyethyl, 2-hydroxypropyl, 3-hydroxypropyl, 1- (hydroxymethyl)-2-methylpropyl, 2-hydroxybutyl, 3 -hydroxybutyl, 4-hydroxybutyl, 2,3 -dihydroxypropyl, 2-hydroxy-1-hydroxymethylethyl, 2,3-dihydroxybutyl, 3,4-dihydroxybutyl or 2- (hydroxymethyl)-3 - hydroxypropyl. The term "heterocycloalkyl" denotes a monovalent saturated or partly unsaturated mono-or bicyclic ring system of 3 to 9 ring atoms, comprising 1, 2, or 3 ring heteroatoms selected from N, O and S, the remaining ring atoms being carbon. In particular embodiments, heterocycloalkyl is a monovalent saturated monocyclic ring system of 4 to 7 ring atoms, comprising 1, 2, or 3 ring heteroatoms selected from N, O and S, the remaining ring atoms being carbon. Examples for monocyclic saturated heterocycloalkyl are aziridinyl, oxiranyl, azetidinyl, oxetanyl, pyrrolidinyl, tetrahydrofuranyl, tetrahydro-thienyl, pyrazolidinyl, imidazolidinyl, oxazolidinyl, isoxazolidinyl, thiazolidinyl, piperidinyl, tetrahydropyranyl, tetrahydrothiopyranyl, piperazinyl, morpholinyl, thiomorpholinyl, 1,1-dioxo-thiomorpholin-4-yl, azepanyl, diazepanyl, homopiperazinyl, or oxazepanyl. Examples for bicyclic saturated heterocycloalkyl are 8-aza-bicyclo[3.2.1]octyl, quinuclidinyl, 8-oxa-3-aza-bicyclo[3.2.1]octyl, 9-aza-bicyclo[3.3.1]nonyl, 3-oxa-9-aza-bicyclo[3.3. 1]nonyl, or 3-thia-9-aza-bicyclo[3.3. 1]nonyl. Examples for partly unsaturated heterocycloalkyl are dihydrofuryl, imidazolinyl, dihydro-oxazolyl, tetrahydro-pyridinyl, or dihydropyranyl. Particular heterocycloalkyl is (1,1-dioxo-1 6-thiomorpholin-4-yl). The term "aromatic" denotes the conventional idea of aromaticity as defined in the literature, in particular in IUPAC - Compendium of Chemical Terminology, 2nd, A. D. McNaught & A. Wilkinson (Eds). Blackwell Scientific Publications, Oxford (1997).

The term "aryl" denotes a monovalent aromatic carbocyclic mono- or bicyclic ring system comprising 6 to 10 carbon ring atoms. Examples of aryl moieties include phenyl and naphthyl. Particular aryl is phenyl. The term "heteroaryl" denotes a monovalent aromatic heterocyclic mono- or bicyclic ring system of 5 to 12 ring atoms, comprising 1, 2, 3 or 4 heteroatoms selected from N, O and S, the remaining ring atoms being carbon. Examples of heteroaryl moieties include pyrrolyl, furanyl, thienyl, imidazolyl, oxazolyl, thiazolyl, triazolyl, oxadiazolyl, thiadiazolyl, tetrazolyl, pyridinyl, pyrazinyl, pyrazolyl, pyridazinyl, pyrimidinyl, triazinyl, azepinyl, diazepinyl, isoxazolyl, benzofuranyl, isothiazolyl, benzothienyl, indolyl, isoindolyl, isobenzofuranyl, benzimidazolyl, benzoxazolyl, benzoisoxazolyl, benzothiazolyl, benzoisothiazolyl, benzooxadiazolyl, benzothiadiazolyl, benzotriazolyl, purinyl, quinolinyl, isoquinolinyl, quinazolinyl, or quinoxalinyl.

The term "active pharmaceutical ingredient" (or "API") denotes the compound in a pharmaceutical composition that has a particular biological activity. The term "pharmaceutically acceptable" denotes an attribute of a material which is useful in preparing a pharmaceutical composition that is generally safe, non-toxic, and neither biologically nor otherwise undesirable and is acceptable for veterinary as well as human pharmaceutical use. The terms "pharmaceutically acceptable excipient" and "therapeutically inert excipient" can be used interchangeably and denote any pharmaceutically acceptable ingredient in a pharmaceutical composition having no therapeutic activity and being non-toxic to the subject administered, such as disintegrators, binders, fillers, solvents, buffers, tonicity agents, stabilizers, antioxidants, surfactants, carriers, diluents or lubricants used in formulating pharmaceutical products. The term "pharmaceutical composition" denotes a mixture or solution comprising a therapeutically effective amount of an active pharmaceutical ingredient together with pharmaceutically acceptable excipients to be administered to a mammal, e.g., a human in need thereof. The term "solid form" or "form" is a general term to denote a crystal form and/or amorphous form of a solid material. The terms "crystal form" and "crystalline form" can be used interchangeably to denote polymorphs and pseudo-polymorphs of a crystalline solid. The terms "polymorph" and "modification" can be used synonymously to denote one particular crystal structure in which a compound can crystallize. Different polymorphs have different arrangements or conformations of the molecules in the crystal lattice but all share the same elemental composition. The term "polymorphism" denotes the ability of a compound to form more than one polymorph. The term "enantiotropy" denotes the relationship between two or more polymorphs of the same substance in which the rank order of thermodynamic stabilities of the polymorphs changes reversibly at a defined temperature. The term "monotropy" denotes the relationship between two or more crystal forms of the same substance in which the rank order of thermodynamic stabilities of the polymorphs is retained at all temperatures below the melting point. A "metastable" form is a crystal form which does not have the highest rank order of thermodynamic stability. The terms "solvate" and "pseudo-polymorph" can be used synonymously to denote a crystal having either stoichiometric or nonstoichiometric amounts of a solvent incorporated in the crystal lattice. If the incorporated solvent is water, the solvate formed is a "hydrate". When the incorporated solvent is alcohol, the solvate formed is an "alcoholate". The term "salt" denotes a material which is composed of two components, an acid and a base with a clearly defined stoichiometric ratio of the two salt formers. Salt crystals are formed by ionic bonding interactions with complete transfer of hydrogen ions between acid and base. The term "crystal shape" denotes the basic body element(s) (polyhedron(s)) of which a single crystal is built up. The crystal shape is described by the Miller indices of the lattice planes of the polyhedron(s). The term "crystal habit" denotes the crystal morphology and hence the physical appearance of a solid form. Variations of crystal habit are caused by different growth rates of lattice planes. The term "equivalent spherical diameter" (or ESD) of a non-spherical object, e.g. an irregularly-shaped particle, is the diameter of a sphere of equivalent volume. The terms "d50 value" and "mass-median diameter" (or MMD) can be used interchangeably and denote the average particle size by mass, i.e. the average equivalent diameter of a particle, which is defined as the diameter where 50% (w) of the particles of the ensemble have a larger equivalent spherical diameter, and the other 50% (w) have a smaller equivalent spherical diameter. The term "amorphous form" denotes a solid material which does not possess a distinguishable crystal lattice and the molecular arrangement of molecules lacks a long-range order. In particular, amorphous denotes a material that does not show a sharp Bragg diffraction peak. Bragg' s law describes the diffraction of crystalline material with the equation "2d · sin(theta) = n · lambda", wherein "d" denotes perpendicular distance (in Angstroms) between pairs of adjacent planes in a crystal ("d-spacing"), "theta" denotes the Bragg angle, "lambda" denotes the wavelength and "n" is an integer. When Bragg' s law is fulfilled, the reflected beams are in phase and interfere constructively so that Bragg diffraction peaks are observed in the X-ray diffraction pattern. At angles of incidence other than the Bragg angle, reflected beams are out of phase and destructive interference or cancellation occurs. Amorphous material does not satisfy Bragg' s law and no sharp Bragg diffraction peaks are observed in the X-ray diffraction pattern. The XRPD pattern of an amorphous material is further characterized by one or more amorphous halos. The term "inclusion complex" denotes a stoichiometric multicomponent complex. In contrast to salts, no or only partial proton transfer is expected in inclusion complexes. An inclusion complex can be an amorphous form or a crystalline form. Particularly, an inclusion complex is a crystalline form. Inclusion complex formers are solid at room temperature. Particular inclusion complex former is cyclodextrin, most particularly gamma-cyclodextrin (gamma-CD). Particularly the

inclusion complex former is in crystalline state in the inclusion complex. Particularly, an inclusion complex is a stoichiometric 1: 1 or a 2: 1 inclusion complex (ratio between inclusion complex former and API). Most particularly, an inclusion complex is a stoichiometric 1 : 1 inclusion complex (ratio between inclusion complex former and API). Inclusion complexes can form solvates, hydrates and can exist as different polymorphic forms. The term "Form A" as used herein denotes the crystalline anhydrous polymorphic form A of (1,1-dioxo-1lambda6-thiomorpholin-4-yl)-{6-[3-(4-fluoro-phenyl)-5-methyl-isoxazol-4-ylmethoxy]-pyridin-3-yl]-methanone. The term "Form B" as used herein denotes the crystalline polymorphic form B of (1,1-dioxo-1lambda6-thiomorpholin-4-yl)-{6-[3-(4-fluoro-phenyl)-5-methyl-isoxazol-4-ylmethoxy]-pyridin-3-yl]-methanone monohydrate. The term "Form C" as used herein denotes the crystalline anhydrous polymorphic form C of (1,1-dioxo-1lambda6-thiomorpholin-4-yl)-{6-[3-(4-fluoro-phenyl)-5-methyl-isoxazol-4-ylmethoxy]-pyridin-3-yl]-methanone. The term "Form D" as used herein denotes the crystalline polymorphic form D of (1,1-dioxo-1lambda6-thiomorpholin-4-yl)-{6-[3-(4-fluoro-phenyl)-5-methyl-isoxazol-4-ylmethoxy]-pyridin-3-yl]-methanone trifluoroethanol mono-solvate. The term "Form E" as used herein denotes the anhydrous crystalline polymorphic form E of (1,1-dioxo-1lambda6-thiomorpholin-4-yl)-{6-[3-(4-fluoro-phenyl)-5-methyl-isoxazol-4-ylmethoxy]-pyridin-3-yl]-methanone. The term "Amorphous Form" as used herein denotes the amorphous form of (1,1-dioxo-1lambda6-thiomorpholin-4-yl)-{6-[3-(4-fluoro-phenyl)-5-methyl-isoxazol-4-ylmethoxy]-pyridin-3-yl]-methanone. The term "gamma-CD inclusion complex" as used herein denotes the crystalline 1: 1 inclusion complex of (1,1-dioxo-1lambda6-thiomorpholin-4-yl)-{6-[3-(4-fluoro-phenyl)-5-methyl-isoxazol-4-ylmethoxy]-pyridin-3-yl]-methanone with γ-Cyclodextrin.

The term"XRPD" denotes the analytical method of X-Ray Powder Diffraction. XRPD patterns were recorded at ambient conditions in transmission geometry with a STOE STADI P diffractometer (Cu K alpha radiation source, primary monochromator, position sensitive detector, angular range 3° to 42° 2Theta, approximately 60 minutes total measurement time). The repeatability of the angular values is in the range of 2Theta ±0.2°. The term "approximately" given in combination with an angular value denotes the repeatability which is in the range of 2Theta ±0.2°. The samples were prepared and analyzed without further processing (e.g. grinding or sieving) of the substance. The relative XRPD peak intensity is dependent upon many factors such as structure factor, temperature factor, crystallinity, polarization factor, multiplicity, and Lorentz factor. Relative intensities may vary considerably from one measurement to another due to preferred orientation effects. Humidity Controlled XRPD analyses were performed in reflection geometry with a Siemens D5000 Diffractometer (Cu radiation source, Ni K beta filter, Scintillation detector, angular range 3° to 42° 2Theta, approximately 180 minutes total measurement time per humidity level). The diffractometer is equipped with an MRI (Materials Research Instruments) humidity chamber. The humidity within the chamber is adjusted with an ANSYCO humidity controller (SYCOS H-HOT). For single crystal structure analysis a single crystal sample was mounted in a nylon loop on a goniometer and measured at ambient conditions. Alternatively, the crystal was cooled in a nitrogen stream during measurement. Data were collected on a GEMINI R Ultra diffractometer from Oxford Diffraction. Cu-radiation of 1.54 A wavelength was used for data collection. Data was processed with the Oxford Diffraction CRYSALIS software. The crystal structure was solved and refined with standard crystallographic software. In this case the program ShelXTL from Bruker AXS (Karlsruhe) was used. The abbreviation "FWHM" denotes the full width at half maximum, which is a width of a peak (e.g. appearing in a spectrum, particularly in an XRPD pattern) at its half height. The term "sharp Bragg diffraction peak" in connection with X-ray diffraction patterns denotes a peak which is observed if Bragg' s law of diffraction is fulfilled. Generally, the FWHM of a sharp Bragg diffraction peak is less than 0.5° 2-theta. The term "amorphous halo" in connection with X-ray diffraction patterns denotes an approximately bell- shaped diffraction maximum in the X-ray powder diffraction pattern of an amorphous material. The FWHM of an amorphous halo is on principle larger than the FWHM of the peak of crystalline material. The terms "FTIR" and "IR" denote the analytical method of infrared spectroscopy. The IR-spectra of the samples are recorded as film of a Nujol suspension consisting of approx. 5 mg of sample and approx. 5 mg of Nujol (mineral oil) between two sodium chloride plates (cross section 13 mm) in transmittance with a FTIR- spectrometer. The spectra were recorded in spectral range between 4000 cm"1 and 600 cm"1, resolution 2 cm"1, and 300 coadded scans on a Magna 860 (thermo/Nicolet) equipped with a DTGS detector. The term "Raman" denotes the analytical method of Raman spectroscopy. For recording Raman spectra, the samples were spread on a glass slide. Raman spectra were recorded in the range of 150-3800 cm"1 with an ARAMIS (HoribaJobinYvon) Raman microscope equipped with a Peltier cooled CCD detector, at excitation of 633 nm, a 1200 1/mm grating, a x50 objective and with 3 exposures of 3s, or 7s for weak Raman scatterers. The term "DSC" denotes the analytical method of Differential Scanning Calorimetry. DSC thermograms were recorded using a Mettler-Toledo™ differential scanning calorimeter DSC820, DSC821 or DSC1 with a FRS05 sensor. System suitability tests were performed with Indium as reference substance and calibrations were carried out using Indium, Benzoic acid, Biphenyl and Zinc as reference substances. For the measurements, approximately 2 - 6 mg of sample were placed in aluminum pans, accurately weighed and hermetically closed with perforation lids. Prior to measurement, the lids were automatically pierced resulting in approx. 1.5 mm pin holes. The samples were then heated under a flow of nitrogen of about 100 mL/min using heating rates of usually 10 K/min. For the measurements of amorphous forms, approximately 2 - 6 mg of sample were placed in aluminum pans, accurately weighed and hermetically closed. The samples were then heated under a flow of nitrogen of about 100 mL/min using heating rates of 10 K/min. The term "onset" denotes the intersection point of the baseline before transition and

the interflection tangent. The term "glass transition temp" (Tg) denotes the temperature above which a glassy amorphous solid becomes rubbery. The term "TGA" denotes the analytical method of Thermo Gravimetric Analysis. TGA analysis was performed on a Mettler-Toledo™ thermogravimetric analyzer (TGA850 or TGA851). System suitability tests were performed with Hydranal as reference substance and calibrations using Aluminum and Indium as reference substances.

For the thermogravimetric analyses, approx. 5 - 10 mg of sample were placed in aluminum pans, accurately weighed and hermetically closed with perforation lids. Prior to measurement, the lids were automatically pierced resulting in approx. 1.5 mm pin holes. The samples were then heated under a flow of nitrogen of about 50 mL/min using a heating rate of 5 K/min. The term "micronization" denotes a process whereby the particle size of a solid material is diminished to a d50 value of less than IOμm by the aid of a suitable method, such as milling, bashing or grinding. The term "polishing filtration" denotes a filtration process wherein a solution is filtrated using a 0.2 μm filter, particularly a Pall N66 Posidyne® 0.2 μm filter cartridge, to remove fine particles. The term "distillative solvent exchange" denotes a thermal distillation under reduced or normal pressure wherein one liquid (solvent or antisolvent) is replaced by another liquid (solvent or antisolvent), usually under constant reactor liquid level. The term "solvent" denotes any kind of liquid in which the product is at least partially soluble (solubility of product > lg/1). The term "antisolvent" denotes any kind of liquid in which the product is insoluble or at maximum sparingly soluble (solubility of product < O.Olmol/1). The term "anti-solvent crystallization" denotes a process wherein supers aturation and as a result thereof crystallisation is achieved by addition of an antisolvent to the product solution. The term "ambient conditions" denotes conditions as experienced in a standard laboratory, e.g. atmospheric pressure, air, ambient temperature between 18 °C and 28 °C, humidity between 30 %rH and 80 %rH. The term "hygroscopicity" describes the ability of a solid material to adsorb moisture. The hygroscopicity of a given API is characterized [European Pharmacopoeia - 6th Edition (2008), Chapter 5.11) by the increase in mass when the relative humidity is raised from 0 %rH to 90 %rH: o non-hygroscopic: weight increase Am < 0.2%; o slightly hygroscopic: weight increase 0.2% < Am < 2.0%; o hygroscopic: weight increase 2.0% < Am < 15.0%; o very hygroscopic: weight increase Am > 15.0%; o deliquescent: sufficient water is adsorbed to form a liquid. The IUPAC lamda convention (W.H. Powell, Pure & Appl. Chem. (1984) 56(6): 769-778) provides a general method for indicating nonstandard valence states of heteroatoms in a molecule. The bonding number "n" of a heteroatom is the sum of the total number of valence bonds to adjacent atoms, if any, and the number of attached hydrogen atoms. The bonding number of a heteroatom is standard when it has the value given in the following table: n=4: C, Si, Ge, Sn, Pb; n=3 B, N, P, As, Sb, Bi, n=2: O, S, Se, Te, Po; n=l; F, CI, Br, I, At. A nonstandard bonding number of a (neutral) heteroatom is indicated by the symbol "λη", where "n" is the bonding number. If the locant, the number indicating the position within the molecule, for a heteroatom with a nonstandard bonding number is used, the λη symbol is cited immediately after this locant. The terms (1,1-dioxo-1 6-thiomorpholin-4-yl)-, (1,1-dioxo-1 6-thiomorpholin-4-yl)-, (1,1-dioxo-1 6-thiomorpholin-4-yl)-, and (1,1-dioxo-thiomorpholin-4-yl)- are used herein interchangeably to denote a thiomorpholinyl-radical wherein the sulfur ringatom is substituted with two oxo groups of the structure as follows:

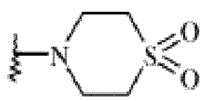

In detail, the present invention relates novel solid forms, particularly crystalline or amorphous forms, most particularly crystalline forms, of compounds of formula (IV)

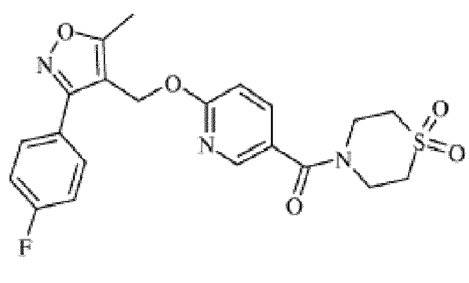

or a solvate thereof; or an inclusion complex thereof with one or more inclusion complex forming agents; or a solvate of an inclusion complex thereof with one or more inclusion complex forming agents.

(1,1-dioxo-1lambda6-thiomorpholin-4-yl)-{6-[3-(4-fluoro-phenyl)-5-methyl-isoxazol-4-ylmethoxy]-pyridin-3-yl]-methanone [CAS No. 1159600-41-5] refers to the compound of formula (IV) and vice versa. In a particular embodiment, the invention relates to solid forms of compounds of formula (IV) as described above characterized by an XRPD pattern comprising at least one XRPD peak in the range of angles of diffraction 2Theta of 10.3° to 13.3°. In a particular embodiment,

the invention relates to solid forms of a compound of formula (IV) as described above; or a solvate thereof; or an inclusion complex thereof with one or more inclusion complex forming agents; or a solvate of an inclusion complex thereof with one or more inclusion complex forming agents; characterized by an XRPD pattern comprising at least one XRPD peak in the range of angles of diffraction 2Theta of 10.3° to 13.3°. In a particular embodiment of the invention, the solid form of a compound of formula (IV) as described above is a crystalline form. In a particular embodiment of the invention, the solid form of a compound of formula (IV) as described above is present in the specified solid form in a purity of at least 90 (w/w), particularly at least 95 (w/w), most particularly at least 99 (w/w). (1,1-Dioxo-16-thiomorpholin-4-yl)-{6-[3-(4-fluoro-phenyl)-5-methyl-isoxazol-4-ylmethoxy]-pyridin-3-yl}-methanone in anhydrous polymorphic form A (Form A ({drug4a})) has been described in WO 2009/071476. Form A ({drug4a}) has been found to be a metastable polymorph with a melting temperature of approx. 145 °C (extrapol. peak DSC). Due to its metastable character Form A ({drug4a}) is not optimally suited for drug product development.

Form A ({drug4a}) is characterized by XRPD peaks at angles of diffraction 2Theta of 3.3°, 10.1°, 14.2°, 14.4°, 15.7°, 16.1°, 17.2°, 17.3°, 19.5°, 19.8°, 20.2°, 20.8°, 22.5°, 24.8°, 25.0°, 25.9°, 27.7°; particularly by XRPD peaks observed at an angle of diffraction 2Theta of 14.4°, 20.2°, 22.5°, 25.9°. Form A ({drug4a}) is characterized by the XRPD diffraction pattern of Figure 1 of WO/2013/057124. Form A ({drug4a}) is characterized by an XRPD diffraction pattern comprising XRPD peaks at peak positions as denoted in Table 2. Form A ({drug4a}) is characterized by the FTIR spectrum of Figure 8 of WO/2013/057124. Form A ({drug4a}) is characterized by the Raman spectrum of Figure 14 of WO/2013/057124. Form A ({drug4a}) is characterized by a melting point with onset temperature (DSC) in the range of about 141 °C to 145 °C. It has been found that (1,1-dioxo-1lambda6-thiomorpholin-4-yl)-{6-[3-(4-fluoro-phenyl)-5-methyl-isoxazol-4-ylmethoxy]-pyridin-3-yl]-methanone can be isolated, depending upon the method of preparation, in other different crystalline and amorphous modifications, which are distinguishable by their X-ray powder diffraction patterns, vibrational spectra and their melting behaviour and which exhibit surprising but relevant advantages beneficial for API and drug product development and administration as compared to previously described Form A ({drug4a}).

Besides the previously described Form A ({drug4a}) of (1,1-dioxo-1lambda6-thiomorpholin-4-yl)-{6-[3-(4-fluoro-phenyl)-5-methyl-isoxazol-4-ylmethoxy]-pyridin-3-yl]-methanone, two further polymorphic anhydrous forms (Form C ({drug4c}) and Form E ({drug4e})), one monohydrate form (Form B ({drug4b})), a trifluoroethanol form (Form D ({drug4d})), as well as an amorphous form were discovered and characterised.

Form B ({drug4b}) of (1,1-dioxo-1lambda6-thiomorpholin-4-yl)-{6-[3-(4-fluoro-phenyl)-5-methyl-isoxazol-4-ylmethoxy]-pyridin-3-yl]-methanone is a hygroscopic mono-hydrate that transforms into Form A ({drug4a}) upon heating to >100 °C. Stability of Form B ({drug4b}) is substantially increased as compared to Form A ({drug4a}) in the presence of humidity, e.g. at ambient conditions. Temperature Controlled XRPD analyses of Form B ({drug4b}) show a phase transition to Form A ({drug4a}) at elevated temperature. In the temperature range 105-135°C only Form A ({drug4a}) is present. In the temperature range of 65-95°C an intermediate state is observed that is characterized by significant changes in peak positions. One particular embodiment of the invention relates to crystalline (1,1-dioxo-1lambda6-thiomorpholin-4-yl)-{6-[3-(4-fluorophenyl)-5-methyl-isoxazol-4-ylmethoxy]-pyridin-3-yl]-methanone monohydrate in polymorphic form B (Form B ({drug4b})) as described herein. In a particular embodiment of the invention, Form B ({drug4b}) is characterized by an XRPD diffraction pattern comprising XRPD peaks at angles of diffraction 2Theta of approximately 13.3°, 20.6°, 22.5°. In a particular embodiment of the invention, Form B ({drug4b}) is characterized by an XRPD diffraction pattern comprising XRPD peaks at angles of diffraction 2Theta of approximately 10.9°, 13.0°, 13.3°, 14.1°, 14.8°, 16.5°, 17.0°, 18.9°, 20.6°, 21.0°, 22.5°, 23.4°, 24.8°, 26.9°. In a particular embodiment of the invention, Form B ({drug4b}) is characterized by the XRPD diffraction pattern of Figure 2 of WO/2013/057124. In a particular embodiment of the invention, Form B ({drug4b}) is characterized by an XRPD diffraction pattern comprising XRPD peaks at peak positions as denoted in Table 3. In a particular embodiment of the invention, Form B ({drug4b}) is characterized by the FTIR spectrum of Figure 9 of WO/2013/057124. In a particular embodiment of the invention, Form B ({drug4b}) is characterized by the Raman spectrum of Figure 15 of WO/2013/057124. In a particular embodiment of the invention, Form B ({drug4b}) is characterized by a broad endothermic signal from 90 °C to 110 °C accompanied by weight loss (measured by TGA).

Form C ({drug4c}) of (1,1-dioxo-1lambda6-thiomorpholin-4-yl)-{6-[3-(4-fluoro-phenyl)-5-methyl-isoxazol-4-ylmethoxy]-pyridin-3-yl]-methanone has been found to be more stable than Form A ({drug4a}). In fact, Form C ({drug4c}) has been found to be the most stable polymorph overall. Form C ({drug4c}) is in addition less hygroscopic than Form A ({drug4a}) and has a melting temperature of approx. 151 °C (extrapol. peak DSC). The solubility in simulated gastric fluid (SGF) of Form C ({drug4c}) is considerably improved as compared to Form B ({drug4b}). In the presence of water, Form C ({drug4c}) transforms into Form B ({drug4b}) in suspended state, whereas storage at 100 rH at ambient temperature for a prolonged period of time, e.g. for 30 days does not induce this phase change. Temperature Controlled XRPD analyses of polymorphs Form A ({drug4a}) and Form C ({drug4c}) do not show solid form changes at elevated temperature. One particular embodiment of the invention relates to crystalline (1,1-dioxo-1lambda6-thiomorpholin-4-yl)-{6-[3-(4-fluorophenyl)-5-methyl-isoxazol-4-ylmethoxy]-pyridin-3-yl]-methanone in anhydrous polymorphic form C (Form C ({drug4c})) as described herein. In a particular embodiment of the invention, Form C ({drug4c}) is characterized by an XRPD diffraction pattern comprising XRPD peaks at angles of diffraction 2Theta of approximately 17.4°, 23.4°.

In a particular embodiment of the invention, Form C ({drug4c}) is characterized by an XRPD diffraction pattern comprising XRPD peaks at angles of diffraction 2Theta of approximately 11.7°, 17.4°, 23.4°.

In a particular embodiment of the invention, Form C ({drug4c}) is characterized by an XRPD diffraction pattern comprising XRPD peaks at angles of diffraction 2Theta of approximately 10.5°, 11.7°, 14.2°, 16.3°, 16.7°, 17.4°, 17.9°, 19.3°, 23.4°, 24.7°, 25.1°, 25.9°. In a particular embodiment of the invention, Form C ({drug4c}) is characterized by the XRPD diffraction pattern of Figure 3 of WO/2013/057124. In a particular embodiment of the invention, Form C ({drug4c}) is characterized by an XRPD diffraction pattern comprising XRPD peaks at peak positions as denoted in Table 4. In a particular embodiment of the invention, Form C ({drug4c}) is characterized by the FTIR spectrum of Figure 10 of WO/2013/057124. In a particular embodiment of the invention, Form C ({drug4c}) is characterized by the Raman spectrum of Figure 16 of WO/2013/057124. In a particular embodiment of the invention, Form C ({drug4c}) is characterized by a melting point with onset temperature (DSC) in the range of about 146 °C to 150 °C.

Form D ({drug4d}) of (1,1-dioxo-1lambda6-thiomorpholin-4-yl)-{6-[3-(4-fluoro-phenyl)-5-methyl-isoxazol-4-ylmethoxy]-pyridin-3-yl]-methanone is a trifluoroethanol mono-solvate that can be generated by crystallization from trifluoroethanol/methanol mixtures. Form D ({drug4d}) offers the benefit over Form A ({drug4a}), that it is readily obtainable in case trifhioroethanol is employed in the manufacturing process. One particular embodiment of the invention relates to crystalline(1,1-dioxo-1lambda6-thiomorpholin-4-yl)-{6-[3-(4-fluoro-phenyl)-5-methyl-isoxazol-4-ylmethoxy]-pyridin-3-yl]-methanone trifhioroethanol mono-solvate in polymorphic form D (Form D ({drug4d})) as described herein. Form D ({drug4d}) has a melting temperature of approx. 97.9°C (extrapol. peak DSC). In a particular embodiment of the invention, Form D ({drug4d}) is characterized by an XRPD diffraction pattern comprising XRPD peaks at angles of diffraction 2Theta of approximately 6.1°, 16.8°, 22.6°. In a particular embodiment of the invention, Form D ({drug4d}) is characterized by an XRPD diffraction pattern comprising XRPD peaks at angles of diffraction 2Theta of approximately 6.1°, 11.0°, 16.8°, 22.6°. In a particular embodiment of the invention, Form D ({drug4d}) is characterized by an XRPD diffraction pattern comprising XRPD peaks at angles of diffraction 2Theta of approximately 6.1°, 8.1°, 11.0°, 13.5°, 15.4°, 16.8°, 18.4°, 19.2°, 19.5°, 21.1°, 21.4°, 22.6°, 24.7°, 28.1°. In a particular embodiment of the invention, Form D ({drug4d}) is characterized by the XRPD diffraction pattern of Figure 4 of WO/2013/057124. In a particular embodiment of the invention, Form D ({drug4d}) is characterized by an XRPD diffraction pattern comprising XRPD peaks at peak positions as denoted in Table 5.

In a particular embodiment of the invention, Form D ({drug4d}) is characterized by the FTIR spectrum of Figure 11 of WO/2013/057124. In a particular embodiment of the invention, Form D ({drug4d}) is characterized by a melting point with onset temperature (DSC) in the range of about 96°C to 100 °C.

Form E ({drug4e}) of (1,1-dioxo-1lambda6-thiomorpholin-4-yl)-{6-[3-(4-fluoro-phenyl)-5-methyl-isoxazol-4-ylmethoxy]-pyridin-3-yl]-methanone is an anhydrate which exhibits only limited stability at ambient conditions. Form E ({drug4e}) is obtained by dehydration of Form B ({drug4b}) through storage at <5 rH. A rapid reconversion of Form E ({drug4e}) into Form B ({drug4b}) is observed upon exposure to >5 rH. Similarly, also upon drying monohydrate form B by means of Humidity Controlled XRPD analysis Form E ({drug4e}) is observed at 0 rH. One particular embodiment of the invention relates to (1,1-dioxo-1lambda6-thiomorpholin-4-yl)-{6-[3-(4-fluoro-phenyl)-5-methyl-isoxazol-4-ylmethoxy]-pyridin-3-yl]-methanone in anhydrous polymorphic form E (Form E ({drug4e})) as described herein.

In a particular embodiment of the invention, Form E ({drug4e}) is characterized by an XRPD diffraction pattern comprising XRPD peaks at angles of diffraction 2Theta of approximately 16.5°, 20.8°. In a particular embodiment of the invention, Form E ({drug4e}) is characterized by an XRPD diffraction pattern comprising XRPD peaks at angles of diffraction 2Theta of approximately 13.1°, 16.5°, 20.8°. In a particular embodiment of the invention, Form E ({drug4e}) is characterized by an XRPD diffraction pattern comprising XRPD peaks at angles of diffraction 2Theta of approximately 5.5°, 13.1°, 13.3°, 14.2°, 16.5°, 19.1°, 20.8°, 22.3°, 23.9°, 25.1°, 25.5°, 26.4°, 29.0°. In aparticular embodiment of the invention, Form E ({drug4e}) is characterized by an XRPD diffraction pattern comprising XRPD peaks at peak positions as denoted in Table 6. In a particular embodiment of the invention, Form E ({drug4e}) is characterized by the XRPD diffraction pattern of Figure 5 of WO/2013/057124. In a particular embodiment of the invention, Form E ({drug4e}) is characterized by the Raman spectrum of Figure 17 of WO/2013/057124.

The glass transition temperature of the amorphous Form of (1,1-dioxo-1lambda6-thiomorpholin-4-yl)-{6-[3-(4-fluoro-phenyl)-5-methyl-isoxazol-4-ylmethoxy]-pyridin-3-yl]-methanone is approx. 66 °C (midpoint of second heating). Amorphous material is slightly hygroscopic, but no phase transformation has been observed upon storage at 100 rH at ambient temperature. One particular embodiment of the invention relates to (1,1-dioxo-1lambda6-thiomorpholin-4-yl)-{6-[3-(4-fluorophenyl)-5-methyl-isoxazol-4-ylmethoxy]-pyridin-3-yl]-methanone (Amorphous Form ({drug4f})) as described herein. In a particular embodiment of the invention, Amorphous Form ({drug4f}) is characterized by at least one amorphous halo and a lack of a sharp Bragg diffraction peak in the XRPD diffraction pattern. In a particular embodiment of the invention, Amorphous Form ({drug4f}) is characterized by the XRPD diffraction pattern of Figure 6 of WO/2013/057124. In a particular embodiment of the invention, Amorphous Form ({drug4f}) is characterized by the FTIR spectrum of Figure 12 of WO/2013/057124. In a particular embodiment of the invention, Amorphous Form ({drug4f}) characterized by the Raman spectrum of Figure 18 of WO/2013/057124. In a particular embodiment of the invention,

Amorphous Form ({drug4f}) is characterized by a glass transition temperature Tg of 60°C to 70 °C, particularly 65°C to 67°C, most particularly 66°C.

In addition, a 1: 1 inclusion complex of (1,1-dioxo-1lambda6-thiomorpholin-4-yl)-{6-[3-(4-fluoro-phenyl)-5-methyl-isoxazol-4-ylmethoxy]-pyridin-3-yl]-methanone with γ-Cyclodextrin (gamma-CD inclusion complex ({drug4g})) with beneficial properties has been found in instant invention. The gamma-CD inclusion complex ({drug4g}) is highly crystalline (as confirmed by XRPD). The dried gamma-CD inclusion complex ({drug4g}) has been found to comprise a residual water content of approx. 7.3% (as confirmed by TGA). It was found, that dried gamma-CD inclusion complex ({drug4g}) and wet powder sample show different XRPD patterns. The crystal structure of the gamma-CD complex seems to be dependent on the water content of the sample. Water seems to stabilize the crystal structure of the described inclusion complex and a substantial loss of water could lead to changes of the crystal structure. gamma-CD inclusion complex ({drug4g}) comprising residual water has been found to have an improved solubility in water as compared to dried gamma-CD inclusion complexes [T. Toropainen et ah, Pharm. Res. (2007) 24:1058-1066]. The molar ratio between API and gamma-CD in the gamma-CD inclusion complex ({drug4g}) has been found to be 1: 1 (as confirmed by UPLC). A complex binding constant of 510.4 M"1 was calculated for the inclusion complex of compounds of formula (IV) and of gamma-CD, as described herein. This binding constant and in vitro dissolution profiles indicate an increased dissolution rate and thus enhanced bioavailability as compared to other solid forms (Figures 21 & 22 of WO/2013/057124). One particular embodiment of the invention relates to a 1: 1 inclusion complex of (1,1-dioxo-1lambda6-thiomorpholin-4-yl)-{6-[3-(4-fluoro-phenyl)-5-methyl-isoxazol-4-ylmethoxy]-pyridin-3-yl]-methanone with γ-Cyclodextrin (gamma-CD inclusion complex ({drug4g})) as described herein. One particular embodiment of the invention relates to a 1: 1 inclusion complex of (1,1-dioxo-1lambda6-thiomorpholin-4-yl)-{6-[3-(4-fluorophenyl)-5-methyl-isoxazol-4-ylmethoxy]-pyridin-3-yl]-methanone with γ-Cyclodextrin (gamma-CD inclusion complex ({drug4g})) as described herein, comprising a residual water content of 1% to 20%(w/w), particularly 5% to 15%(w/w), most particularly 8% to 12%(w/w). In a particular embodiment of the invention, Form E ({drug4e}) is characterized by an XRPD diffraction pattern comprising XRPD peaks at angles of diffraction 2Theta of approximately 7.4°, 14.9°, 16.7°, 21.8°. In a particular embodiment of the invention, Form E ({drug4e}) is characterized by an XRPD diffraction pattern comprising XRPD peaks at angles of diffraction 2Theta of approximately 7.4°, 12.1°, 14.9°, 16.7°, 21.8°. In a particular embodiment of the invention, Form E ({drug4e}) is characterized by an XRPD diffraction pattern comprising XRPD peaks at angles of diffraction 2Theta of approximately 3.8°, 5.2°, 7.4°, 9.2°, 10.6°, 11.5°, 11.8°, 12.1°, 14.2°, 14.9°, 15.8°, 16.7°, 19.2°, 20.3°, 21.2°, 21.8°, 22.5°, 23.7°, 26.8°. In a particular embodiment of the invention, gamma-CD inclusion complex ({drug4g}) is characterized by the XRPD diffraction pattern of Figure 7 of WO/2013/057124. In a particular embodiment of the invention, gamma-CD inclusion complex ({drug4g}) is characterized by an XRPD diffraction pattern comprising XRPD peaks at peak positions as denoted in Table 7. In a particular embodiment of the invention, gamma-CD inclusion complex ({drug4g}) is characterized by the FTIR spectrum of Figure 13 of WO/2013/057124. In a particular embodiment of the invention, gamma-CD inclusion complex ({drug4g}) is characterized by the Raman spectrum of Figure 19 of WO/2013/057124.

Table 1 lists the relevant crystal structure data of Form A ({drug4a}), Form B ({drug4b}), Form C ({drug4c}) and Form D ({drug4d}) of (1,1-dioxo-1 6-thiomorpholin-4-yl)-{6-[3-(4-fluoro-phenyl)-5-methyl-isoxazol-4-ylmethoxy]-pyridin-3-yl}-methanone. The crystal structures of Form A ({drug4a}), Form B ({drug4b}), Form C ({drug4c}) and Form D ({drug4d}) were refined. Form E ({drug4e}) crystallizes only at dry conditions and rehydrates at relative humidity >5, single crystalline samples are not available. The lattice constants, unit cell volume and calculated density are based on ambient temperature data. For this purpose the lattice constants obtained from single crystal structure analysis were refined with the experimental ambient conditions XRPD reference patterns using the software TOPAS 4.0, Bruker AXS.

Table 1: Single Crystal Structural Data of Forms A, B, C and D:

| Crystal form | Form A | Form B | Form C | Form D |
|---|---|---|---|---|
| Solid form description | anhydrate | monohydrate | anhydrate | trifluoroethanol monosolvate |
| Measuring Temperature | 295 K | 130 K | 100 K | 293 K |
| Crystal system | Monoclinic | Monoclinic | Triclinic | Monoclinic |
| Space group | $P2_{(1)}/c$ | $P2_{(1)}/n$ | P1 | $P2_{(1)}/c$ |
| Unit, cell dimensions | 26.1638 Å | 7.5969 Å | 7.653 Å | 14.6152 Å |
| b= | 6.3113 Å | 32.0909 Å | 7.86.7 Å | 16.6069 Å |
| c= | 12.4695 Å | 8.9480 Å | 17.394 Å | 10.6567 Å |
| α= | 90° | 90° | 81.078° | 90° |
| β= | 90.836° | 110.454° | 78.195° | 98.934° |

(continued)

| Crystal form | Form A | Form B | Form C | Form D |
|---|---|---|---|---|
| $\gamma$= | 90° | 90° | 8.7.98° | 90° |
| Cell volume | 2058.84 $Å^3$ | 2043.91 $Å^3$ | 1012.2 $Å^3$ | 2555.1 $Å^3$ |
| API molecules in unit cell | 4 | 4 | 2 | 4 |
| Calculated density | 1.437 $g/cm^3$ | 1.506 $g/cm^3$ | 1.462 $g/cm^3$ | 1.418 $g/cm^3$ |
| ambient temperature data | | | | |

Tables 2, 3 and 4: XRPD peak positions and relative intensities of major XRPD peaks of Forms A, B and C.

| Table 2 | |
|---|---|
| Form A | |
| 2Theta/° | rel. int./% * |
| 3.3 | 16.2 |
| 10.1 | 20.2 |
| 14.2 | 89.6 |
| 14.4 | 100 |
| 15.7 | 60.6 |
| 16.1 | 28.6 |
| 17.2 | 39.9 |
| 17.3 | 43.5 |
| 19.5 | 47.3 |
| 19.8 | 41.7 |
| 20.2 | 82.8 |
| 20.8 | 25.7 |
| 22.5 | 94.1 |
| 24.8 | 24 |
| 25.0 | 28.1 |
| 25.9 | 93.4 |
| 27.7 | 25.5 |

| Table 3 | |
|---|---|
| Forum B | |
| 2Theta/° | rel. int./% * |
| 10.9 | 28.8 |
| 13.0 | 28.7 |
| 13.3 | 70.4 |
| 14.1 | 27.3 |
| 14.8 | 49.9 |
| 16.5 | 45.7 |

(continued)

| Table 3 | |
|---|---|
| Forum B | |
| 2Theta/° | rel. int./% * |
| 17.0 | 29.9 |
| 18.9 | 49.6 |
| 20.6 | 98.6 |
| 21.0 | 52.8 |
| 22.5 | 100 |
| 23.4 | 43.1 |
| 24.8 | 32 |
| 26.9 | 33.1 |

| Table 4 | |
|---|---|
| Form C | |
| 2Theta/° | rel. int./% * |
| 5.2 | 8.2 |
| 10.5 | 14.4 |
| 11.7 | 15.5 |
| 14.2 | 8.1 |
| 16.3 | 26.2 |
| 16.7 | 32.8 |
| 17.4 | 34.9 |
| 17.9 | 8.9 |
| 19.3 | 25.6 |
| 23.4 | 100 |
| 24.7 | 15.3 |
| 25.1 | 14.7 |
| 25.9 | 31.3 |
| ** Relative intensities may vary considerably from one measurement to another.* | |

Tables 5, 6 and 7: XRPD peak positions and relative intensities of major XRPD peaks of Forms D, E and yCD inclusion complex:

| Table 5 | |
|---|---|
| Form D | |
| 2Theta/° | rel. int./% * |
| 6.1 | 18.1 |

(continued)

| Table 5 | |
|---|---|
| Form D | |
| 2Theta/° | rel. int./% * |
| 8.1 | 9.1 |
| 11.0 | 16.9 |
| 13.5 | 16.2 |
| 15.4 | 20.7 |
| 16.8 | 100 |
| 18.4 | 30.7 |
| 19.2 | 43.7 |
| 19.5 | 25.1 |
| 21.1 | 27.2 |
| 21.4 | 39.7 |
| 22.6 | 78.2 |
| 24.7 | 22.8 |
| 28.1 | 14.6 |

| Table 6 | |
|---|---|
| Form E | |
| 2Theta/° | rel. int./% * |
| 5.5 | 9.7 |
| 13.1 | 23.4 |
| 13.3 | 19.2 |
| 14.2 | 18.7 |
| 16.5 | 81 |
| 19.1 | 47.7 |
| 20.8 | 100 |
| 22.3 | 34.4 |
| 23.9 | 66.8 |
| 25.1 | 20.4 |
| 25.5 | 19.8 |
| 26.4 | 45.1 |
| 29.0 | 31 |

| Table 7 | |
|---|---|
| γ-CD inclusion complex | |
| 2Theta/° | rel. int./% |
| 3.8 | 14.8 |

(continued)

| Table 7 | |
|---|---|
| γ-CD inclusion complex | |
| 2Theta/° | rel. int./% |
| 5.2 | 11.6 |
| 7.4 | 100 |
| 9.2 | 12.1 |
| 10.6 | 13.8 |
| 11.5 | 32.9 |
| 11.8 | 21.3 |
| 12.1 | 38.6 |
| 14.2 | 49.7 |
| 14.9 | 61.1 |
| 15.8 | 47.1 |
| 16.7 | 60 |
| 19.2 | 27.2 |
| 20.3 | 26.9 |
| 21.2 | 28.6 |
| 21.8 | 62.3 |
| 22.5 | 32.9 |
| 23.7 | 31.3 |
| 26.8 | 20.7 |
| ** Relative intensities may vary considerably from one measurement to another.* | |

The invention further relates to a distillative solvent exchange process for the preparation of solid forms of compounds of formula (IV) as defined above comprising: a) dissolution of the educt solid form in a solvent; b) distillation of the solvent while keeping the reactor liquid level constant by replacing the distillate by an antisolvent; c) physical separation of the desired solid form from the suspension. In a particular embodiment, the desired solid form obtained by such distillative solvent exchange in step c) is crystalline (1,1-dioxo-1lambda6-thiomorpholin-4-yl)-{6-[3-(4-fluoro-phenyl)-5-methyl-isoxazol-4-ylmethoxy]-pyridin-3-yl]-methanone in anhydrous polymorphic form C (Form C ({drug4c})) as defined above. In a particular embodiment, the educt solid form in step a) is selected from Form A ({drug4a}) or Form B ({drug4b}), most particularly from Form B ({drug4b}). In a particular embodiment, the solvent employed in step a) is selected from THF, DMF or acetone or a mixture thereof, particularly selected from THF. In a particular embodiment, the antisolvent employed in step b) is selected from ethanol, iso-propanol, or n-heptane or a mixture thereof, particularly selected from ethanol. In a particular embodiment, step b) is performed at increased temperature, particularly at 50-80°C. In a particular embodiment, step b) is performed at reduced pressure, particularly at 100-300mbar. In a particular embodiment, step b) is optionally preceded or accompanied by seeding with the desired solid form as a powder or suspension, most particularly seeding with 1-10 (w/w) (in respect of final yield) of the desired solid form as a powder or suspension. In a particular embodiment, the physical separation in step c) is performed via filtration. The invention further relates to a high-shear process for the preparation of solid forms of compounds of formula (IV) as defined above comprising: d) injection of a solution of the educt solid form in a solvent into a high-shear mixer comprising an antisolvent; e) agitation of the rotor-stator system of the high-shear mixer; f) physical separation of the desired solid form from the suspension. In a particular embodiment, the desired solid form obtained by this high-shear process in step f) is crystalline (1,1-dioxo-1lambda6-thiomorpholin-4-yl)-{6-[3-(4-fluoro-phenyl)-5-methyl-isoxazol-4-ylmethoxy]-pyridin-3-yl]-methanone in anhydrous polymorphic form C (Form C ({drug4c})) as defined above. In a particular embodiment, the educt solid form in step d) is selected fromForm A ({drug4a}) or Form B ({drug4b}), particularly selected from Form B ({drug4b}). In a particular embodiment, the solution of educt solid form in step d) is injected at a constant flow rate of 1.6g/min. In a particular embodiment, the solvent employed in step d) is selected from THF, DMF or acetone or a mixture thereof, particularly selected from THF. In a particular embodiment, the antisolvent employed in step d) is selected from ethanol, iso-propanol, or n-heptane or a mixture thereof, particularly selected from n-heptane. In a particular embodiment, the antisolvent is circulated across the high-shear mixer in steps d) and e) at a constant velocity, particularly at a constant velocity of 20 1/h. In a particular embodiment, the antisolvent of step d) optionally comprises seeding particles of the desired solid form, particularly 1-10 (w/w) (in respect of final yield) of seeding particles of the desired solid form, most particularly 5-10 (w/w) (in respect of final yield) of seeding particles of the desired solid form. In a particular embodiment, the rotor-stator system in step e) is rotated at a rotation rate of 15000 RPM to 24000 RPM. In a particular embodiment, steps d) and e) are performed at decreased temperature, particularly at -20°C to 0°C, most particularly at -5°C. In a particular embodiment, the physical separation in step f) is performed via filtration. Another embodiment provides pharmaceutical compositions or medicaments comprising solid forms of compounds of formula (IV) as described herein and a pharmaceutically acceptable excipient, as well as methods of using the compounds of the invention to prepare such compositions and medicaments. Compositions are formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The solid forms of compounds of formula (IV) as described herein may be administered by any suitable means, including oral, topical (including buccal and sublingual), rectal, vaginal, transdermal, parenteral, subcutaneous, intraperitoneal, intrapulmonary, intradermal, intrathecal and epidural and intranasal, and, if desired for local treatment, intralesional administration. Parenteral infusions include intramuscular, intravenous, intraarterial, intraperitoneal, or subcutaneous administration. The solid forms of compounds of formula (IV) as described herein may be administered in any convenient administrative form, e.g., tablets, powders, capsules, solutions, dispersions, suspensions, syrups, sprays, suppositories, gels, emulsions, patches, etc. Such compositions may comprise components conventional in pharmaceutical preparations, e.g., diluents, carriers, pH modifiers, preservatives, solubilizers, stabilizers, wetting agents, emulsifiers, sweeteners, colorants, flavorants, salts for varying the osmotic pressure, buffers, masking agents, antioxidants, and further active agents. They can also comprise still other therapeutically valuable substances. A typical formulation is prepared by mixing a solid form of compounds of formula (IV) as described herein and a pharmaceutically acceptable excipient. Suitable excipients are well known to those skilled in the art and are described in detail in, e.g., Ansel H.C. et ah, Ansel's Pharmaceutical Dosage Forms and Drug Delivery Systems (2004) Lippincott, Williams & Wilkins, Philadelphia; Gennaro A.R. et ah, Remington: The Science and Practice of Pharmacy (2000) Lippincott, Williams & Wilkins, Philadelphia; and Rowe R.C, Handbook of Pharmaceutical Excipients (2005) Pharmaceutical Press, Chicago. The formulations may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents, flavoring agents, diluents and other known additives to provide an elegant presentation of the drug (i.e., a compound of the present invention or pharmaceutical composition thereof) or aid in the manufacturing of the pharmaceutical product (i.e., medicament). The dosage at which solid forms of compounds of formula (IV) as described herein can be administered can vary within wide limits and will, of course, be fitted to the individual requirements in each particular case. In general, in the case of oral administration a daily dosage of about 0.1 to 1000 mg per person of a solid form of compounds of formula (IV) as described herein should be appropriate, although the above upper limit can also be exceeded when necessary. A particular embodiment of the invention relates to a daily dosage of 0.1 to 1000 mg (p.o.), particularly of 10 to 500 mg (p.o.), most particularly of 75 to350 mg (p.o.). An example of a suitable oral dosage form is a tablet comprising about 100 mg to 500 mg of a solid form of compounds of formula (IV) as described herein compounded with about 90 to 30 mg anhydrous lactose, about 5 to 40 mg sodium croscarmellose, about 5 to 30 mg polyvinylpyrrolidone (PVP) K30, and about 1 to 10 mg magnesium stearate. The powdered ingredients are first mixed together and then mixed with a solution of the PVP. The resulting composition can be dried, granulated, mixed with the magnesium stearate and compressed to tablet form using conventional equipment. An example of an aerosol formulation can be prepared by dissolving a solid form of compounds of formula (IV) as described herein, for example 10 to 100 mg, in a suitable buffer solution, e.g. a phosphate buffer, adding a tonicifier, e.g. a salt such as sodium chloride, if desired. The solution may be filtered, e.g., using a 0.2 μm filter, to remove impurities and contaminants. The solid forms of compounds of formula (IV) as described herein, possess valuable pharmacological properties and have been found to be ligands for GABA A cc5 receptors. The solid forms of compounds of formula (IV) of the present invention can therefore be used, either alone or in combination with other drugs, for the treatment or prevention of diseases which are modulated by ligands for GABA A receptors containing the cc5 subunit. These diseases include, but are not limited to acute and/or chronic neurological disorders, cognitive disorders, Alzheimer's disease, memory deficits, schizophrenia, positive, negative and/or cognitive symptoms associated with schizophrenia, bipolar disorders, autism, Down syndrome, neurofibromatosis type I, sleep disorders, disorders of circadian rhythms, amyotrophic lateral sclerosis (ALS), dementia caused by AIDS, psychotic disorders, substance-induced psychotic disorder, anxiety disorders, generalized anxiety disorder, panic disorder, delusional disorder, obsessive/compulsive disorders, acute stress disorder, drug addictions, movement disorders, Parkinson's disease, restless leg syndrome, cognition deficiency disorders, multi-infarct dementia, mood disorders, depression, neuropsychiatric conditions, psychosis, attention-deficit/hyperactivity disorder, neuropathic pain, stroke, Multiple Sclerosis (MS), acute Meningitis, Fetal Alcohol Syndrome, attentional disorders, CNS conditions occurring after stroke, and need for cognition enhancement. The invention therefore also relates to pharmaceutical compositions comprising solid forms of compounds of formula (IV) as described herein and a pharmaceutically acceptable excipient. The invention likewise embraces solid forms of compounds of formula (IV) as described herein for use as therapeutically active substances. The invention likewise embraces solid forms of compounds of formula (IV) as described herein for use as therapeutically active substances for the treatment or prevention of diseases which are related to the GABA A a5 receptor. The invention likewise embraces solid forms of compounds of formula (IV) as described herein for use as therapeutically active substances for the treatment or prevention of acute and/or chronic neurological disorders, cognitive disorders, Alzheimer's disease, memory deficits, schizophrenia, positive, negative and/or cognitive symptoms associated with schizophrenia, bipolar disorders, autism, Down syndrome, neurofibromatosis type I, sleep disorders, disorders of circadian rhythms, amyotrophic lateral sclerosis (ALS), dementia caused by AIDS, psychotic disorders, substance-induced psychotic disorder, anxiety disorders, generalized anxiety disorder, panic disorder, delusional disorder, obsessive/compulsive disorders, acute stress disorder, drug addictions, movement disorders, Parkinson's disease, restless leg syndrome, cognition deficiency disorders, multi-infarct dementia, mood disorders, depression, neuropsychiatric conditions, psychosis, attention-deficit/hyperactivity disorder, neuropathic pain, stroke, Multiple Sclerosis (MS), acute Meningitis, Fetal Alcohol Syndrome, and attentional disorders, for stroke recovery therapy, or for use as cognitive enhancers. In another embodiment, the invention relates to a method for the treatment or prevention of diseases which are related to the GABA A a5 receptor, which method comprises administering solid forms of compounds of formula (IV) as described herein to a human being or animal.

In another embodiment, the invention relates to a method for the treatment or prevention of acute and/or chronic neurological disorders, cognitive disorders, Alzheimer's disease, memory deficits, schizophrenia, positive, negative and/or cognitive symptoms associated with schizophrenia, bipolar disorders, autism, Down syndrome, neurofibromatosis type I, sleep disorders, disorders of circadian rhythms, amyotrophic lateral sclerosis (ALS), dementia caused by AIDS, psychotic disorders, substance-induced psychotic disorder, anxiety disorders, generalized anxiety disorder, panic disorder, delusional disorder, obsessive/compulsive disorders, acute stress disorder, drug addictions, movement disorders, Parkinson's disease, restless leg syndrome, cognition deficiency disorders, multi-infarct dementia, mood disorders, depression, neuropsychiatric conditions, psychosis, attention-deficit/hyperactivity disorder, neuropathic pain, stroke, Multiple Sclerosis (MS), acute Meningitis, Fetal Alcohol Syndrome, and attentional disorders, for stroke recovery therapy, or for cognition enhancement, which method comprises administering solid forms of compounds of formula (IV), particularly compounds of formula (IV), as described herein to a human being or animal. The invention also embraces the use of solid forms of compounds of formula (IV) as described herein for the treatment or prevention of diseases which are related to the GABA A a5 receptor. The invention also embraces the use of solid forms of compounds of formula (IV) as described herein for the treatment or prevention of acute and/or chronic neurological disorders, cognitive disorders, Alzheimer's disease, memory deficits, schizophrenia, positive, negative and/or cognitive symptoms associated with schizophrenia, bipolar disorders, autism, Down syndrome, neurofibromatosis type I, sleep disorders, disorders of circadian rhythms, amyotrophic lateral sclerosis (ALS), dementia caused by AIDS, psychotic disorders, substance- induced psychotic disorder, anxiety disorders, generalized anxiety disorder, panic disorder, delusional disorder, obsessive/compulsive disorders, acute stress disorder, drug addictions, movement disorders, Parkinson's disease, restless leg syndrome, cognition deficiency disorders, multi-infarct dementia, mood disorders, depression, neuropsychiatric conditions, psychosis, attention-deficit/hyperactivity disorder, neuropathic pain, stroke, Multiple Sclerosis (MS), acute Meningitis, Fetal Alcohol Syndrome, and attentional disorders or for cognition enhancement. The invention also relates to the use of solid forms of compounds of formula (IV) as described herein for the preparation of medicaments for the treatment or prevention of diseases which are related to the GABA A a5 receptor, particularly for the treatment or prevention of acute and/or chronic neurological disorders, cognitive disorders, Alzheimer's disease, memory deficits, schizophrenia, positive, negative and/or cognitive symptoms associated with schizophrenia, bipolar disorders, autism, Down syndrome, neurofibromatosis type I, sleep disorders, disorders of circadian rhythms, amyotrophic lateral sclerosis (ALS), dementia caused by AIDS, psychotic disorders, substance-induced psychotic disorder, anxiety disorders, generalized anxiety disorder, panic disorder, delusional disorder, obsessive/compulsive disorders, acute stress disorder, drug addictions, movement disorders, Parkinson's disease, restless leg syndrome, cognition deficiency disorders, multi-infarct dementia, mood disorders, depression, neuropsychiatric conditions, psychosis, attention-deficit/hyperactivity disorder, neuropathic pain, stroke, Multiple Sclerosis (MS), acute Meningitis, Fetal Alcohol Syndrome, and attentional disorders, for stroke recovery therapy, or for the preparation of cognitive enhancers. Such medicaments comprise a compound as described above. More particularly, the present invention relates to the use of solid forms of compounds of formula (IV) as described

herein for the treatment, prevention and/or delay of progression of CNS conditions caused by neurodevelopmental defects which result in excessive GABAergic inhibition in the cortex and hippocampus, wherein the CNS condition is selected from cognitive deficits in Down Syndrome, in autism, in neurofibromatosis type I, or after stroke. The treatment or prevention of cognitive disorders, Alzheimer's disease, schizophrenia, positive, negative and/or cognitive symptoms associated with schizophrenia, Down syndrome, and neurofibromatosis type I, are particular embodiments of present invention. A particular embodiment of the invention embraces the treatment or prevention of Alzheimer's disease.

A particular embodiment of the invention embraces the treatment or prevention of Down syndrome. A particular embodiment of the invention embraces the treatment or prevention of neurofibromatosis type I. A particular embodiment of the invention embraces the recovery after stroke.

Description of the drawings of WO/2013/057124:Figure 1 of WO/2013/057124: XRPD pattern of Form A ({drug4a}). Figure 2 of WO/2013/057124: XRPD pattern of Form B ({drug4b}). Figure 3 of WO/2013/057124: XRPD pattern of Form C ({drug4c}). Figure 4 of WO/2013/057124: XRPD pattern of Form D ({drug4d}). Figure 5 of WO/2013/057124: XRPD pattern of Form E ({drug4e}), analyzed at 0%rH or after drying at 70°C. Figure 6 of WO/2013/057124: XRPD pattern of amorphous Form. Figure 7 of WO/2013/057124: XRPD pattern gamma-CD inclusion complex ({drug4g}). Figure 8 of WO/2013/057124: FT-IR spectrum of Form A ({drug4a}). Figure 9 of WO/2013/057124: FT-IR spectrum of Form B ({drug4b}). Figure 10 of WO/2013/057124: FT-IR spectrum of Form C ({drug4c}). Figure 11 of WO/2013/057124: FT-IR spectrum of Form D ({drug4d}). Figure 12 of WO/2013/057124: FT-IR spectrum of Amorphous Form ({drug4f}). Figure 13 of WO/2013/057124: FT-IR spectrum of gamma-CD inclusion complex ({drug4g}). Figure 14 of WO/2013/057124: Raman spectrum of Form A ({drug4a}). Figure 15 of WO/2013/057124: Raman spectrum of Form B ({drug4b}). Figure 16 of WO/2013/057124: Raman spectrum of Form C ({drug4c}). Figure 17 of WO/2013/057124: Raman spectrum of Form E ({drug4e}). Figure 18 of WO/2013/057124: Raman spectrum of Amorphous Form ({drug4f}). Figure 19 of WO/2013/057124: Raman spectrum of gamma-CD inclusion complex ({drug4g}). Figure 20 of WO/2013/057124: Phase solubility diagram of (1J-dioxo-1 6-thiomorpholin-4-yl)-{6-r3-(4-fluoro-phenyl)-5-methyl-isoxazol-4-ylmethoxyl-pyridin-3-yl|-methanone and gamma-CD. The API solubility is shown in [^g/mL]. The solid phase in equilibrium with the saturated solution was verified at selected points (arrows) by Raman and XRPD measurements to identify and confirm potential solid-state transformations, such as formation of polymorph B (monohydrate) from the initially used polymorph A, or conversion of the free API to the gamma-CD inclusion complex ({drug4g}). Figure 21 of WO/2013/057124: Mean in vitro dissolution profiles in SGF Mean in vitro dissolution profiles of micronized powders of Form A ({drug4a}) (o), Form B ({drug4b}) (T), Form C ({drug4c}) (■) and gamma-CD inclusion complex ({drug4g}) (Δ) in SGF at room temperature. Measurements were performed in triplicate (n=3).

Figure 22 of WO/2013/057124: Mean in vitro dissolution profiles in FeSSIF Mean in vitro dissolution profiles of micronized powders of Form A ({drug4a}) (o), Form B ({drug4b}) (T), Form C ({drug4c}) (■) and gamma-CD inclusion complex ({drug4g}) (Δ) in FeSSIF at room temperature. Measurements were performed in triplicate (n=3).

## Examples

The following examples 1 - 28 are provided for illustration of the invention. They should not be considered as limiting the scope of the invention, but merely as being representative thereof.

Example 1: Preparation of crystalline (1,1-dioxo-1lambda6-thiomorpholin-4-yl)-{6-[3-(4-fluoro-phenyl)-5-methyl-isoxazol-4-ylmethoxy]-pyridin-3-yl]-methanone in anhydrous polymorphic form A (Form A ({drug4a})): Form A ({drug4a}) can be prepared as described in WO 2009/071476. Step a: (E)- and/or (Z)-4-Fluoro-benzaldehyde oxime: To a suspension of 4-fluorobenzaldehyde (24.8 g, 200 mmol) (6.75 g, 54 mmol) and hydroxylamine hydrochloride (4.16 g, 60 mmol) in ethanol (4.3 mL) and water (13 mL) was added ice (25 g). Then a solution of sodium hydroxide (5.5 g, 138 mmol) in water (6.5 mL) was added dropwise within a 10 min period (temperature rises from -8 °C to + 7 °C) whereupon most of the solid dissolves. After 30 min stirring at room temperature a white solid precipitated and the resulting mixture was then diluted with water and acidified with HCl (4 N). The white precipitate was then filtered off, washed with water and dried under high vacuum to afford the title compound (23.3 g, 84%) which was obtained as a white solid. MS: m/e = 139.1 [M]+. Step b: (E)- and/or (Z)-N-Hvdroxy-4-fluoro-benzenecarboximidoyl chloride: To a solution of (E)- and/or (Z)-4-fluoro-benzaldehyde oxime (23.3 g, 167 mmol) (6.9 g, 50 mmol) in DMF (50 mL) was added N-chlorosuccinimide (6.6 g, 50 mmol) portionwise over 1 h, keeping the temperature below 35 °C. The reaction mixture was stirred at room temperature for 1 h. The mixture was then poured onto ice- water, and extracted with ethyl acetate. The combined organic layers were then washed with water and brine, dried over sodium sulfate and evaporated to afford the title compound (25.9 g, 89%) which was obtained as an off white solid. MS: m/e = 173.0 [M]+.

Step c: 3-(4-Fluoro-phenyl)-5-methyl-isoxazole-4-carboxylic acid ethyl ester: To a solution of (E)- and/or (Z)-N-hydroxy-4-fluoro-benzenecarboximidoyl chloride (15.4 g, 89 mmol) (11.1 g, 64 mmol) in diethylether (151 mL) was added ethyl 2-butynoate (7.2 g, 7.5 mL, 64 mmol) at 0 °C followed by the dropwise addition of triethylamine (7.8 g, 10.7 mL, 77 mmol) and the resulting mixture allowed to warm up to room temperature overnight. The mixture was then poured onto ice-water, and extracted with diethylether. The combined organic layers were then washed with water and brine,

dried over sodium sulfate and evaporated. Purification by chromatography (Si02, heptane:ethyl acetate = 100:0 to 1: 1) afforded the title compound (9.8 g, 44%) which was obtained as an off white solid. MS: m/e = 250.1 [M+H]+. Step d: r3-(4-Fluoro-phenyl)-5-methyl-isoxazol-4-yll-methanol: To a solution of 3-(4-fluoro-phenyl)-5-methyl-isoxazole-4-carboxylic acid ethyl ester (3.0 g, 12 mmol) (6.18 g, 25 mmol) in THF (320 mL) was added portionwise lithiumaluminiumhydride (528 mg, 14 mmol) at 0 °C and the reaction mixture was stirred at room temperature for 3 h. The mixture was then cooled to 0 °C and water (518 μl) added followed by sodium hydroxide (15% solution, 518 μl) and then again water (1.5 mL) and the mixture then stirred overnight at room temperature. The precipitate was then filtered off and washed with THF. The combined washings and filtrate were then evaporated. Purification by chromatography (Si02, heptane:ethyl acetate = 100:0 to 1: 1) afforded the title compound (1.8 g, 71%) which was obtained as a white solid. MS: m/e = 208.1 [M+H]+. Step e: 6- r3-(4-Fluoro-phenyl)-5-methyl-isoxazol-4-ylmethoxyl -nicotinic acid methyl ester: To a suspension of sodium hydride (55% dispersion in mineral oil, 852 mg, 20 mmol) in THF (27 mL) was added a solution of [3-(4-fluoro-phenyl)-5-methyl-isoxazol-4-yl]-methanol (103 mg, 0.55 mmol) (3.68 g, 18 mmol) in THF (54 mL) at 0 °C and the reaction mixture warmed to room temperature over 30 min. Then a solution of methyl 6-chloronicotinate (3.35 g, 20 mmol) in THF (1.5 mL) was added dropwise at 0 °C and the reaction mixture was stirred at room temperature overnight. The reaction mixture was then poured into aqueous sodium chloride (saturated) and the mixture was extracted with ethyl acetate. The combined organic layers were then washed with water and brine and then dried over sodium sulfate, filtered and evaporated. Purification by chromatography (Si02, heptane:ethyl acetate = 7:3) afforded the title compound (81 mg, 47%) which was obtained as a light yellow solid. MS: m/e = 343.3 [M+H]+. Step f: 6-r3-(4-Fluoro-phenyl)-5-methyl-isoxazol-4-ylmethoxyl-nicotinic acid: To a solution of 6-[3-(4-fluoro-phenyl)-5-methyl-isoxazol-4-ylmethoxy]-nicotinic acid methyl ester (1.4 g, 4.2 mmol) (538 mg, 1.1 mmol) in THF (5 mL) was added a solution of lithium hydroxide monohydrate (94 mg, 2.2 mmol) in water (5 mL) and methanol (1 mL) added and the resulting mixture stirred at room temperture overnight. The mixture was acidified to pH 4 with HCl (25%, 3 drops) and methanol (2 drops) added. A gum began to form and the mixture was cooled at 0 °C for 1.5 h and then the aqueous layer decanted off. Trituration with diethylether and hexane afforded the title compound (1.1 g, 78%) which was obtained as a white solid. MS: m/e = 327.3 [M-H]~. Step g: crystalline (U-dioxo-1 6-thiomorpholin-4-yl)-{6-r3-(4-fluoro-phenyl)-5-methyl-isoxazol-4-ylmethoxyl-pyridin-3-yl|-methanone in anhydrous polymorphic form A (Form A ({drug4a})) To a solution of 6-[3-(4-fluoro-phenyl)-5-methyl-isoxazol-4-ylmethoxy]-nicotinic acid (99 mg, 0.33 mmol (69 mg, 0.2 mmol)) in DMF (300 μί) were added 2-(1H-benzotriazole-1-yl)-1,1,3,3-tetramethyluronium tetrafluoroborate (71 mg, 0.22 mmol), N,N-diisopropyl ethyl amine (171 μl, 1.0 mmol) and thiomorpholine-S,S-dioxide (17.3 μl, 0.22 mmol). The resulting reaction mixture was stirred for 1 h at room temperature. Concentration and purification by chromatography (Si02, heptane:ethyl acetate = 100:0 to 1: 1) afforded the title compound (73 mg, 55%) as a white solid. MS: m/e = 446.1 [M+H]+.

Example 2: Preparation of Form A ({drug4a}): A solution of 0.1 g of (1,1-dioxo-1 6-thiomorpholin-4-yl)-{6-[3-(4-fluorophenyl)-5-methyl-isoxazol-4-ylmethoxy]-pyridin-3-yl}-methanone in 0.7 mL of 2-pentanol or THF was crash-cooled with liquid nitrogen, isolated by centrifugation at 25 °C and dried at 20 °C and reduced pressure at <5 mbar for 2 d.

Example 3: Preparation of Form A ({drug4a}): 152.4 mg of (1,1-dioxo-1lambda6-thiomorpholin-4-yl)-{6-[3-(4-fluorophenyl)-5-methyl-isoxazol-4-ylmethoxy]-pyridin-3-yl]-methanone were dissolved in 2.14 mL of 2-pentanol at 60 °C yielding a colorless solution. The solvent was evaporated slowly until dryness (perforated cover foil, 5 d at ambient conditions) to yield blade-like crystals.

Example 4: Preparation of Form A ({drug4a}): 700.0 g of 6-[3-(4-fluoro-phenyl)-5-methyl-isoxazol-4-ylmethoxy] -nicotinic acid (Ex. 1 step f), 10 L of THF and 469.0 g of 1,1-carbodiimidazol were stirred at ambient temperature for one hour. 407.0 g of thiomorpholine-S,S-dioxide, 12.0 g of 4-dimethylaminopyridine und 340 mL of triethylamine p. a. were added successively and refluxed under stirring over two nights. Additional 82.0 g of thiomorpholine-S,S-dioxide and 68.0 mL of triethylamine p. a. were added and further refluxed under stirring overnight (o.n.). The experiment was cooled down to approx. 30 °C. 10 L of desalinated water and 16 L of ethanol were added successively. The emerging solution was cooled down to 20 °C, seeded with 12 g of Form A ({drug4a}) and stirred at ambient temperature for 30 min. The suspension was reduced to 16 L at max. 35 °C. In order to replace THF, 20 L of ethanol were added. The suspension was stirred at ambient temperature o.n. and then filtrated. The filter cake was rinsed with 7.4 L of a 1: 1 desalinated water / ethanol mixture and dried at 50 °C o.n. yielding 820 g of Form A ({drug4a}) (86 %).

Example 5: Preparation of Form A ({drug4a}): 16.32 g of Form B ({drug4b}) were dissolved in 257 g THF at 50°C. To remove the water from the solution 172 g of THF were distilled off under reduced pressure at 80°C. Then this water free product solution was cooled to room temperature. Keeping the jacket temperature constant at -5°C, 238 g of heptane were circulated across a high- shear-mixer device with a velocity of 20 1/h by use of a peristaltic pump. After 5 Minutes the high-shear-mixer was started with a rotation rate of 15000 RPM to 24000 RPM and the product solution from above was pumped with a flow rate of 1.6g/min directly through the injector into the rotor-stator system. After addition was completed, the resulting crystals were filtered and dried at 40°C at 30 mbar for 15 h to yield Form A ({drug4a}).

Example 6: Preparation of Form A ({drug4a}): 100 g of Form B ({drug4b}) were dissolved in 1200 g THF at 50°C. About 50% of THF were distilled off at 70°C under reduced pressure (800 mbar) to yield a 20% (w/w) solution of Form B ({drug4b}) in THF. In a distillative solvent exchange, THF/water (of hydration) was exchanged against dry THF

at 800 mbar and at 70°C while keeping the solvent level constant until the water content was below 0.1% (w/w). 888g of heptane at 5°C as antisolvent were seeded with 1% (w/w) of Form A ({drug4a}). Subsequently the product solution was cooled to 50°C and was dosed during 30 minutes using a temperated hose underneath suface to the heptane present at 5 °C. The resulting crystals were filtered and dried at reduced pressure until constant weight to yield Form A ({drug4a}) (92%).

Example 7: Preparation of Form A ({drug4a}): 41g of Form B ({drug4b}) were dissolved in 170g THF at 50°C. 30 g of ethanol were added and the solution cooled to 30°C. In a distillative solvent exchange, the solvent (THF/ethanol) was exchanged to the anti-solvent ethanol at a temperature of 30°C and at reduced pressure (300 mbar) while the volume was kept constant by continuously replacing the distillate by a total of 340 g of ethanol. 20 minutes after start of the distillation, crystallization was initiated by seeding with 2% (w/w) of crysals of Form A ({drug4a}). Subsequently the pressure was reduced to 230 mbar. 50 minutes after start of the distillation, the pressure was reduced to 130 mbar. 67 minutes after start of the distillation, the solvent exchange was completed. The resulting suspension was stirred for 1.5 h at ambient temperature and subsequently filtered. The obtained crystals were dried in a vacuum dryer at 40°C overnight to yield 36.4 g of Form A ({drug4a}) (92.4%).

Example 8: Preparation of crystalline (1,1-dioxo-1lambda6-thiomorpholin-4-yl)-{6-[3-(4-fluoro-phenyl)-5-methyl-isoxazol-4-ylmethoxy]-pyridin-3-yl]-methanone monohydrate in polymorphic form B (Form B ({drug4b}})): Step a) (E)- and/or (Z)-4-fluoro-benzaldehvde oxime: To a suspension of 4-fluoro-benzaldehyde (30.4 g, 0.24 mol) in water (50 mL) was added at 0-5°C within 5 minutes a solution of hydroxylamine hydrochloride (17.7 g, 0.25 mol) in water (30 mL) and the resulting mixture stirred for 15 minutes at 0-5°C. The mixture was then treated at 15-25°C within 15 minutes with 32% NaOH (24.44 mL, 0.26 mol) and the resulting suspension was stirred for one additional hour and then extracted with ethyl acetate (3x100 mL). The combined organic layers were washed with water (2x100 mL) and subsequently concentrated to dryness to afford 31.9 g (95%) of the title oxime as a white solid. Step b) 3-(4-Fluoro-phenyl)-5-methyl-isoxazole-4-carboxylic acid ethyl ester:

To a suspension of 4-fluoro-benzaldehyde oxime (1.39 g, 10.0 mmol) in DMF (10 mL) was added portionwise within 5 minutes at 15 to 20°C N-chlorosuccinimide (1.36 g, 10.0 mmol) and the resulting mixture was stirred at room temperature for 90 minutes. The yellow solution (containing N-Hydroxy-4-fluoro-benzenecarboximidoyl chloride) was then treated within 2 minutes at room temperature with a solution of ethyl-3-(1-pyrrolidino)crotonate (1.89 g, 10.0 mmol) in 5 mL of DMF and the resulting solution was stirred at room temperature for 28 hours. The mixture was diluted with water (25 mL) and subsequently extracted with ethyl acetate (4x25 mL). The combined organic layers were washed with 1 M HCl (2x25 mL) and water (2x25 mL), dried over Na2S04 and subsequently concentrated to dryness (45°C/25 mbar) to afford 2.37 g (95%) of the title ester as a brownish solid with a purity of 100% (by GC) and 97% (by HPLC). Step c) 3-(4-Fluoro-phenyl)-5-methylisoxazole-4-carboxyric acid: A mixture of 179.5 g (0.72 mol) of 3-(4-Fluoro-phenyl)-5-methyl-isoxazole-4-carboxylic acid ethyl ester in 880 g of ethanol 95% was stirred at 20-30°C for 40 minutes and then treated with 78.5 g of solid sodium hydroxide. The resulting mixture was stirred for 5 h at 20-30°C. Ethanol was removed in vacuum at 45-50°C and the residue was subsequently treated with 500 g of water at 20-30°C to afford a clear solution. The solution was stirred for 40 minutes and filtered. To the filtrate was added 235 g of methyl tert-butyl ether and 600 g of water and the resulting mixture stirred for 20 min and then stood for 20 min. The layers were separated and the aqueous layer was acidified to pH <1 with hydrochloric acid. The crystals were filtered and washed with water to provide 147 g crude wet product. The crude wet product was suspended in 680 g of toluene and the mixture was heated at 75-85 °C for 7 h. The mixture was cooled to 20-30°C and stirred for 1 hour at this temperature. The crystals were filtered off and dried at 50-55°C in vacuum over night to afford 137 g (86 % yield) of the title acid as a white to slightly yellow solid with a purity of 99.9 % (HPLC). Step d) r3-(4-Fluorophenyl)-5-methyl-isoxazol-4-yll -methanol: A suspension of 448 g of tetrahydrofuran and 95 g (0.70 mol) of zinc chloride was stirred at 20-30°C for 1 h. 23.6 g (0.62 mol) of sodium borohydride were added in portions at 20-38 °C and the mixture subsequently stirred at 60-65°C for 3 h. A solution of 69 g (0.31 mol) of 3-(4-Fluoro-phenyl)-5-methyl-isoxazole-4-carboxylic acid in 220 g THF was added dropwise and the resulting mixture stirred at 60-65 °C for 16 h. The reaction was then quenched by the drop wise addition of mixture of 93 g of HCl in 202 g of water at 5-10°C. The mixture was stirred at this temperature for 2 h to dissolve the solids completely. The solvent was removed under reduced pressure with a jacket temperature of 35-40°C. To the residue were added 510 g of water. The resulting suspension was cooled to 20-30°C and the crystals were filtered off and washed with water. The crude wet product was stirred for 1 h in a mixture of 150 g of water, 31 g of HCl and 419 g of MTBE. The lower aqueous phase was removed and organic phase was dried with 25 kg of anhydrous sodium sulfate, stirred for 0.5 h and filtered under nitrogen. The filtrate was almost completely concentrated under reduced pressure at 40-45 °C. The residue was treated at 20-25 °C with 100 g of MTBE. The mixture was stirred at 55-60°C for 2 h, cooled to 0°C and subsequently stirred at this temperature for additional 2 h. The crystals were filtered off and dried at 45-50°C in vacuum over night to afford 42 g (66 % yield) of the title alcohol as an off-white solid with a purity of 99.9% (HPLC). Step e) 6-r3-(4-Fluoro-phenyl)-5-methyl-isoxazol-4-ylmethoxyl-nicotinonitrile: To a suspension of sodium hydride (60% in mineral oil, 7.9 g, 181 mmol, 1.5 eq.) in THF (65 mL) was added within 30 minutes at room temperature a solution of [3-(4-Fluorophenyl)-5-methyl-isoxazol-4-yl]- methanol (25.0 g, 121 mmol) and 6-chloronicotinonitrile (16.7 g, 121 mmol) in THF (120 mL)

and the resulting mixture was stirred for one hour. A solution of citric acid (18.5 g, 96.5 mmol) in water (185 mL) was added to the reaction mixture within 30 minutes. From the resulting THF/water mixture THF was distilled off under reduced pressure at a jacket temperature of 60°C and replaced by ethanol. In total 284 g of ethanol were added. The resulting suspension was stirred for one hour at room temperature. The crystals were filtered off, washed with a mixture of ethanol (60 mL) and water (60 mL) and subsequently dried at 50°C/<25 mbar to afford 36.5 g (91 corrected yield) of the title nitrile as an off-white solid with an assay of 93 %(w/w). Step f) 6- r3-(4-Fluoro-phenyl)-5-methyl-isoxazol-4-ylmethoxyl -nicotinic acid: 6-[3-(4-Fluorophenyl)-5-methyl-isoxazol-4-ylmethoxy]-nicotinonitrile (58.8 g, 190 mmol) was suspended in water (440 mL) and ethanol (600 mL) and treated with 32% sodium hydroxide solution (178 mL 1.92 mol). The mixture was heated to 50-55°C and subsequently stirred at this temperature for 15 hour. The slightly turbid mixture was polish filtered to remove the ether byproduct 6-[3-(4-Fluoro-phenyl)-5-methyl-isoxazol-4-ylmethoxymethyl-3-(4-fluoro-phenyl)-5-methyl-isoxazole. The first vessel and the transfer lines were rinsed with a mixture of water (50 mL) and ethanol (50 mL). The filtrate was treated at 20-25°C within one hour with 25% hydrochloric acid (approx. 280 mL) until the pH was <2.0. The resulting suspension was stirred for one hour at room temperature. The crystals were filtered off, washed with a mixture of ethanol (200 mL) and water (200 mL) and subsequently dried at 50°C/<25 mbar until constant weight to afford 52.0 g (83%) of the title acid as an off-white solid with a purity of 99.5 %. Step g) Purification of thiomorpholine-1,1 -dioxide HCl: A mixture of 60 g of thiomorpholine- 1,1 -dioxide HCl in 600 mL THF, 105 mL water and 30 mL DMF was heated to 63-66°C (slightly reflux) and the resulting clear to slightly turbid solution stirred at this temperature for 5 to 10 hours. The mixture was then treated at 63-66°C within 30 minutes with 300 mL of THF. The mixture was then cooled to 0-5°C within 3 hours and the resulting suspension stirred at this temperature for one additional hour. The crystals were filtered off, washed with THF (2x25 mL) and dried at 50°C and under reduced pressure (<20 mbar) to afford 56.6 g (94%) of thiomorpholine- 1,1 -dioxide HCl with a purity of 100 % (area) and a THF content of 0.14%. Step h) crystalline (1J-dioxo-1 6-thiomorpholin-4-yl)-{6-r3-(4-fluoro-phenyl)-5-methyl-isoxazol-4-ylmethoxyl-pyridin-3-yl|-methanone monohvdrate in polymorphic form B (Form B ({drug4b})): 6-[3-(4-Fluoro-phenyl)-5-methyl-isoxazol-4-ylmethoxy]-nicotinic acid (23.0 g, 70.1 mmol) and 1,1-carbonyldiimidazole (15.3 g, 94.6 mol, 1.35 eq.) were dissolved in THF (120 mL) and the resulting solution was stirred for one hour at room temperature. This solution was then added to a suspension of thiomorpholine- 1,1 -dioxide HCl (16.9 g, 98.5 mmol), DMAP (400 mg, 3.27 mmol) and triethylamine (9.78 g, 96.7 mmol) in THF (120 mL). The resulting mixture was heated to reflux temperature and subsequently stirred at this temperature for 50 hours. The mixture was cooled to room temperature and then treated within one hour with water (300 mL). From the resulting suspension THF was distilled off under reduced pressure and with a jacket temperature of 60°C and continuously replaced by ethanol (426 g) at constant volume. The suspension was cooled to room temperature and stirred for 2 hours. The crystals were filtered off, washed with a mixture of ethanol (100 mL) and water (100 mL) and subsequently dried at 55°C/<25 mbar until constant weight to afford 28.9 g (92%) of Form B ({drug4b}) as a colorless solid with purity of 99.7% (area) as measured by HPLC.

Example 9: Preparation of Form B ({drug4b}): Form A ({drug4a}) was aged for 8 days in an aqueous suspension. Isolation by filtration yielded crystalline blades which were rinsed with water and then dried at ambient conditions.

Example 10: Preparation of Form B ({drug4b}): 155.9 mg of Form A ({drug4a}) were dissolved in 2.2 mL of 15% water in acetone at 60 °C yielding a colorless solution. The solvent was evaporated slowly until dryness (perforated cover foil, 5 d at ambient conditions) to yield equant crystals.

Example 11: Preparation of Form B ({drug4b}): 509 mg of Form A ({drug4a}) were dissolved in 7.1 mL 15 %-vol. water/acetone at 60 °C yielding a colorless solution. Then the solvent was allowed to evaporate slowly over 8 days (perforated cover foil, ambient conditions). The residue was dried at 20 °C/<5 mbar o.n. (vacuum tray dryer), yielding 440 mg (86 %) of equant crystals.

Example 12: Preparation of Form B ({drug4b}): 10.0 g of Form C ({drug4c}) were dissolved in 50 mL of THF and 17 mL of DMF under stirring at ambient temperature. During a period of 30 minutes, the solution was gradually heated to 50-55°C and stirred at this temperature for 15 minutes. 75 mL of water were added dropwise during 2-3 hours under stirring at 50-55°C. The resulting suspension was stirred for additional 15 minutes at 50-55°C and afterwards gradually cooled to 15-20°C during 2-4 hours. The suspension was stirred for 5 hours at 15-20°C, filtered and washed with a small amount of water. The obtained crystals were dried for 12 hours at 40°C at reduced pressure (20mbar) yielding Form B ({drug4b}) (95%).

Example 13: Preparation of crystalline (1,1-dioxo-1lambda6-thiomorpholin-4-yl)-{6-[3-(4-fluoro-phenyl)-5-methyl-isoxazol-4-ylmethoxy]-pyridin-3-yl]-methanone in anhydrous polymorphic form C (Form C ({drug4c})): 4.5 kg of Form A ({drug4a}) were dissolved in 40 L of THF at ambient temperature. After polishing filtration the filter was rinsed by 5 L of THF. From the combined solutions solvent was distilled off at reduced pressure at a temperature below 70 °C while the volume was kept constant by continuously replacing the distillate by a total of 90 L of ethanol. The suspension was allowed to cool to ambient temperature over 12 hours. 25 L of ethanol were added, the suspension was heated to 78°C at ambient pressure, allowed to cool to ambient temperature over 12 hours and stirred for one additional hour. Crystallization at ambient pressure occurred at 78°Cto 70°C. 25 L of ethanol were distilled off at reduced pressure at 35-40°C and the suspension was allowed to cool to ambient temperature over 12 hours. The product was isolated by filtration

and rinsed by 20 L of ethanol. The crystals were dried in a vacuum tray dryer (50 °C/5 mbar for 3 d), yielding 4.1 kg (91%) colorless plate-like crystals. It was possible to reproduce the experiment on 10 g scale.

Example 14: Preparation of Form C ({drug4c}): 200 mg of Form A ({drug4a}) were stirred in 0.8 mL of ethyl acetate at ambient temperature for 14 days (suspension). After isolation of the solids by filtration and drying in a vacuum tray dryer (50 °C/<5 mbar for 12 h) Form C ({drug4c}) was obtained. Alternatively, ethanol or toluene can be used instead of ethyl acetate. Example 15: Preparation of Form C ({drug4c}): 41g of Form B ({drug4b}) were dissolved in 170g THF at 50°C. 30g of ethanol were added and the solution cooled to 30°C. In a distillative solvent exchange, the solvent (THF/ethanol) was exchanged to the anti-solvent ethanol at a temperature of 30°C and at reduced pressure (300 mbar) while the volume was kept constant by continuously replacing the distillate by a total of 340g of ethanol. 20 minutes after start of the distillation, the pressure was reduced to 230 mbar. 30 minutes after start of the distillation, the previously clear yellow solution became opaque. Two minutes later the opaque solution had turned into a thick suspension. 50 minutes after start of the distillation, the pressure was reduced to 130 mbar. 68 minutes after start of the distillation, the solvent exchange was completed. The resulting suspension was stirred for 2h at ambient temperature and subsequently filtered. The obtained crystals were dried in a vacuum dryer at 40°C over-night to yield 35.8 g of Form C ({drug4c}).

Example 16: Preparation of Form C ({drug4c}): IOg of Form B ({drug4b}) (22.4 mmol) were dissolved in 350 mL THF under stirring at ambient temperature, filtered and the filter rinsed with 40mL of THF. In a distillative solvent exchange, the solvent of the filtrate was exchanged to ethanol at a temperature of 60°C and at reduced pressure (100-300 mbar) while the volume was kept constant by continuously replacing the distillate by a total of 200 mL of ethanol. Crystallization was initiated after addition of the first 20ml of ethanol by seeding with crystals of Form C ({drug4c}). The resulting suspension was stirred for 1h at ambient temperature, subsequently filtered and rinsed with 50mL of ethanol. The obtained crystals were dried in a vacuum dryer at 50°C over-night to yield 8.8 g (88%) of Form C ({drug4c}).

Example 17: Preparation of Form C ({drug4c}): 82g (177 mmol) of Form B ({drug4b}) were dissolved in 340 g of THF at 50°C. 60 g of ethanol were added to prepare a 17% (w/w) solution of Form B ({drug4b}) in a THF/ethanol mixture of (85: 15 (w/w). The clear solution was allowed to cool to 35°C under stirring. A 10% (w/w) seeding suspension of 0.8 g of Form C ({drug4c}) suspended in 7.2g of a 50:50 (w/w) THF/ethanol mixture (10% (w/w) Form C ({drug4c}) in respect of final theoretical yield) was added and the reaction mixture was stirred for 30 min at ambient temperature. The pressure was decreased to 300 mbar while the temperature was increased to 50°C. In a distillative solvent exchange, the volume was kept constant by continuously replacing the distillate by a total 680 g of ethanol, which were added linearly (5.6 g/min) during a total time of 120 minutes. The reaction pressure is lowered, after 20 minutes of ethanol addition to 230 mbar, and after 50 minutes of total ethanol addition to 130 mbar. After 115 minutes of ethanol addition, the temperature was gradually lowered to 5°C during at a cooling speed of 1 °C/min (30 min cooling time). The suspension was stirred for 30 minutes at 5 °C, filtered and rinsed with 68 g of ethanol. The obtained crystals were dried at 40°C at 30 mbar for 16h to yield 98.5 % of Form C ({drug4c}). Alternatively, this preparation can be performed with acetone as solvent instead of THF. Alternatively, this preparation can be performed with isopropanol and/or n-heptane as anti-solvent instead of ethanol.

Example 18: Preparation of Form C ({drug4c}): 16.32 g of Form B ({drug4b}) were dissolved in 257 g THF at 50°C. To remove the water from the solution 172 g of THF were distilled off under reduced pressure at 80°C. Then this water free product solution was cooled to room temperature. To 238 g Heptane at a temperature of -5°C 1.6 g (10 %(w/w) in respect of final theoretical yield) of Form C ({drug4c}) were added under stirring as seeding material. Keeping the jacket temperature constant at -5°C, the resulting suspension was circulated across a high-shear-mixer device with a velocity of 20 1/h by use of a peristaltic pump. After 5 minutes the high- shear- mixer was started with a rotation rate of 15000 RPM to 24000 RPM and the product solution from above was pumped with a flow rate of 1.6g/min. directly through the injector into the rotor-stator system. After addition was completed, the resulting crystals were filtered and dried at 40°C at 30 mbar for 15 h to yield 91% of Form C ({drug4c}) with an average particle size d50 <10μm. When conducting Example 18 without seeds, Form A ({drug4a}) was obtained (see Example 5). Using 2%(w/w) Form C ({drug4c}) seeds, a mixture from Form A ({drug4a}) (dominant) and C was obtained. Employing 5%(w/w) Form C ({drug4c}) seeds, a mixture from Form C ({drug4c}) (dominant) and A was obtained.

Example 19: Preparation of Form C ({drug4c}): 14.12 g of Form B ({drug4b}) were dissolved in 240 g THF at 50°C. To remove the water from the solution 160g of THF were distilled off under reduced pressure at 80°C. The water free solution was cooled during 15 minutes to 25°C and 0.07 g Form C ({drug4c}) seeds (0.5%(w/w) in respect of final theoretical yield) were added. After 30 minutes of stirring the temperature was lowered over 135 minutes to 15°C and 9.0 g heptane were added in parallel. The resulting suspension was stirred for 30 minutes, then the temperature was raised over 15 minutes to 35 °C. After 30 minutes the temperature is cooled again over 165 minutes to 15°C and another 11 g of heptane were added in parallel. After 30 minutes of stirring the temperature was raised again to 35 °C and the suspension was stirred again for 30 minutes. Afterwards the temperature was lowered again to 15°C during 495 minutes and 33 g heptane were added in parallel. The resulting final suspension was stirred for additional 120 minutes, then filtered, dried at 40°C and 30 mbar for 16 hours to yield 94 % of Form C ({drug4c}) with an average particle size d50 >50μm.

Example 20: Preparation of crystalline (1,1-dioxo-1lambda6-thiomorpholin-4-yl)-{6-[3-(4-fluoro-phenyl)-5-methyl-isoxazol-4-ylmethoxy]-pyridin-3-yl]-methanone trifluoroethanol mono-solvate in polymorphic form D (Form D ({drug4d})): 40 mg of Form A ({drug4a}) was equilibrated in 400 μί 3: 1 trifluoroethanol/methanol (TFE/MeOH) mixture for 7 days at room temperature by head-over-head rotation with magnetic stir bars in 2 mL HPLC glass crimp vials. After equilibration the solid phase was separated from the liquid phase by centrifugation. The solvent was removed by a pipette and by strips of filter paper. The residual solids were dried at 40°C in a vacuum tray dryer to 10 h at 20 mbar.

Example 21: Preparation of Form D ({drug4d}): 2 g of Form A ({drug4a}) were dissolved in 20 mL of a 3: 1 trifluoroethanol/methanol mixture. Seed crystals of Form D ({drug4d}) were added and the mixture was stored closed at ambient temperature for 3 days. The residual column-shaped crystals were isolated by filtration (glass filter) and dried in a vacuum tray dryer (ambient temperature/20 mbar for 24 h).

Example 22: Preparation of crystalline (1,1-dioxo-1lambda6-thiomorpholin-4-yl)-{6-[3-(4-fluoro-phenyl)-5-methyl-isoxazol-4-ylmethoxy]-pyridin-3-yl]-methanone in anhydrous polymorphic form E (Form E ({drug4e})): 50 mg of Form B ({drug4b}) was subjected to dehydration/hydration cycles. At <5 rH reversible transformation into Form E ({drug4e}) was observed by means of Humidity Controlled XRPD.

Example 23: Preparation of Form E ({drug4e}): 50 mg of Form B ({drug4b}) was placed into a desiccator, where the sample was dried over
concentrated sulfuric acid for 36 h at ambient temperature.

Example 24: Preparation of amorphous (1,1-dioxo-1lambda6-thiomorpholin-4-yl)-{6-[3-(4-fluoro-phenyl)-5-methyl-isoxazol-4-ylmethoxy]-pyridin-3-yl]-methanone (Amorphous Form ({drug4f})): 0.554 g of Form C ({drug4c}) was dissolved in 4.0 mL of dichloromethane in a round bottom flask. The clear solution was rapidly concentrated using a rotary evaporator (40 °C outside temperature, vacuum stepwise reduced to 14 mbar). The residue was dried in a vacuum tray dryer (50 °C/<5 mbar for 2 days), yielding 0.498 g (90 %) of a colorless powder.

Example 25: Preparation of Amorphous Form ({drug4f}): 150 mg of Form A ({drug4a}) were molten at 160 °C in a glass vial using a heat gun and cooled to ambient temperature to yield amorphous material.

Example 26: Preparation of 1:1 inclusion complex of (1,1-dioxo-1lambda6-thiomorpholin-4-yl)-{6-[3-(4-fluoro-phenyl)-5-methyl-isoxazol-4-ylmethoxy]-pyridin-3-yl]-methanone with γ-Cyclodextrin (gamma-CD inclusion complex ({drug4g})): 300 mg of Form A ({drug4a}) was weighed into a 20 mL screw cap glass vial. 6 mL deionized water and gamma-CD at a molar ratio of 1:2 was added. The suspension was equilibrated at room temperature for 32 days by head-over-head rotation using a Heidolph Reax 2 mixer (VWR International AG, Dietikon, Switzerland). Solid liquid separation was performed with amicon Ultrafree-MC® centrifugal filter devices (0.45 μm Durapore PVDF membrane, Millipore, Bedford, MA) to yield crystals of the gamma-CD inclusion complex ({drug4g}).

Example 27: Phase Solubility Analysis: Phase solubility diagrams are used to characterize complex formation between two compounds and represent the solubility of the API as a function of the cyclodextrin concentration. The phase solubility diagram of (1,1-dioxo-1lambda6-thiomorpholin-4-yl)-{6-[3-(4-fluoro-phenyl)-5-methyl-isoxazol-4-ylmethoxy]-pyridin-3-yl]-methanone with gamma-cyclodextrin showed Bs-type behavior according to the classification of Higuchi [T. Higuchi et al., Adv. Anal. Chem. Instrum. (1965) 4:117-212] and Brewster [M. E. Brewster et al. Adv. Drug Delivery Rev. (2007) 59:645-666] (Figure 20 of WO/2013/057124). The API concentration first increased with increasing cyclodextrin concentration due to the complexation of the API with cyclodextrin molecules. After an initial increase in drug solubility, the maximum solubility of the complex was reached and the complex started to precipitate, indicating the formation of a less soluble inclusion complex (gamma-CD inclusion complex ({drug4g})). At the end of the plateau, the entire solid API has been consumed and further addition of API would result in depletion of API in the solution by complex formation and concomitant precipitation of the insoluble complex. 150 mM represents the solubility limit of gamma-cyclodextrin in aqueous solution. The binding constant (K) of the gamma-CD inclusion complex ({drug4g}) was calculated from the initial straight line portion of the phase solubility diagram by linear regression, according to the following equation [T. Higuchi et al., Adv. Anal. Chem. Instrum. (1965) 4:117-212]:

$$K = \frac{\text{slope}}{\text{intercept} \times (1 - \text{slope})} = \frac{0.00131}{0.00000257 \times (1 - 0.00131)} M^{-1} = 510.4 M^{-1} \qquad \text{(Eq. 1)}$$

The binding constant of the gamma-CD inclusion complex ({drug4g}) calculated according to equation (1) was 510.4 M-1. The binding constant (K) is a measure of the affinity of the API to enter the relatively apolar cavity of the CD. The desired situation is to have sufficient affinity, such as to enhance the concentration of total dissolved drug, but still allow for dissociation of the complex followed by absorption of the API. A binding constant of 510.4 M-1 in case of the gamma-CD inclusion complex ({drug4g}) is in a good range and suggests that an oral solid dosage form with an increased dissolution rate should be feasible.

Example 28: in vitro Dissolution Results: The in vitro dissolution studies performed in this work were conducted in a miniaturized system with 100 mL dissolution medium per experiment. In comparison to the 1000 mL vessels of the conventional USP apparatus the experimental set-up that was used here was scaled-down and simplified (magnetic

stirring instead of paddles, room temperature instead of 37 °C). The dissolution experiments were performed under non-sink conditions (drug concentration >10 of the solubility value). Simulated gastric fluid (SGF) was prepared with 2 g/L NaCl and 1 g/L Triton® X-100 in 0.1 N HCl. The resulting measured pH of SGF was 1.2. Simulated fed state intestinal fluid (FeSSIF) was prepared as previously reported in Galia E. et al. (Pharm. Res. (1996) 13:S-262) and contained 15 mM sodium taurocholate, 3.75 mM lecithin and had a pH 5.0. Oral absorption of a drug compound from a solid dosage form is dependent on dissolution rate and solubility. In the present work the in vitro dissolution of the gamma-CD inclusion complex ({drug4g}) was compared to micronized powders of polymorph Form A ({drug4a}), Form B ({drug4b}) and Form C ({drug4c}). Figure 21 of WO/2013/057124presents the dissolution profiles measured in simulated gastric fluid (SGF) and Figure 22 of WO/2013/057124shows the dissolution profiles in simulated fed state intestinal fluid (FeSSIF). In both dissolution media the gamma-CD inclusion complex ({drug4g}) behaves completely different compared to micronized powders of polymorph Form A ({drug4a}), Form B ({drug4b}) and Form C ({drug4c}). The gamma-CD inclusion complex ({drug4g}) achieved a much higher initial concentration in SGF and FeSSIF which rapidly dropped in the first 60 min to a level which was comparable to polymorph C values. In case of the micronized powders of polymorphs the saturation solubility of the specific polymorphs was relatively rapidly achieved (< 30 min) and the dissolved drug contents remained unchanged until the end of the experiment (180 min). Changes of pH values in the dissolution test samples taken at different time points were not observed. The ranking of the different solid forms with respect to dissolution speed and maximum drug concentration achieved was identical in both media. The differences in the dissolution profiles in SGF and FeSSIF can be explained by the different composition of the two media since the dissolution generally depends on a variety of factors such as pH, surfactant, buffer capacity, ionic strength, etc. The ability of the gamma-CD inclusion complex ({drug4g}) to form a supersaturated solution presents promising opportunities to increase the in vivo absorption and oral bioavailability compared to the crystalline pure phases of Form A ({drug4a}), Form B ({drug4b}) and Form C ({drug4c}). To maintain the supers aturation promoted by the gamma-CD inclusion complex ({drug4g}) the addition of specific precipitation inhibitors such as hydroxypropyl methylcellulose (HPMC), polyvinylpyrrolidone (PVP), etc. to the final dosage form can be beneficial. A prolongation of the supersaturated state can dramatically impact and improve in vivo absorption and bioavailability.

The invention relates to an administration unit comprising form X of compound of formula (IV). The administration unit according to the invention is useful for treating various diseases and disorders which include but are not limited to treatment or prevention of acute and/or chronic neurological disorders, cognitive disorders, Alzheimer's disease, memory deficits, schizophrenia, positive, negative and/or cognitive symptoms associated with schizophrenia, bipolar disorders, autism, etc.

SPECIFIC INORMATION CONCERNING ASPECT (V) OF THE INVENTION

Aspect (v) of the invention concerns forms of {drug5}, especially a solid form of compound of formula V, namely to crystalline form of HCl salt ({drug5a}) or HBr salt ({drug5b}) of compound of formula V

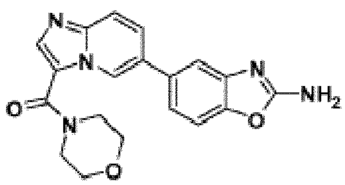

V,

which has the systematic name "(6-(2-aminobenzo[d]oxazol-5-yl)imidazo[1,2-a]pyridin-3-yl)(morpholino)methanone" and is useful for treating benign and malignant proliferative diseases, disorders such as allergic contact dermatitis, rheumatoid arthritis, osteoarthritis, inflammatory bowel diseases, chronic obstructive pulmonary disorder, psoriasis, multiple sclerosis, asthma. The crystalline form of HCl salt ({drug5a}) or HBr salt ({drug5b}) of compound of formula V ({drug5}) is described in WO/2013/071272, which is incorporated by reference. The compound of formula V ({drug5}) is represented by formula V:

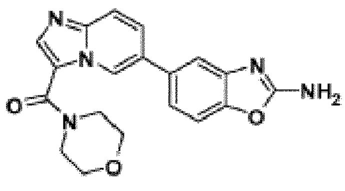

V.

Preferred crystalline form of HCl salt ({drug5a}) or HBr salt ({drug5b}) of compound of formula V ({drug5}) are described in WO/2013/071272: In one embodiment, the invention is directed to a method of making a pharmaceutically-acceptable polymorph of a compound of formula V, said method comprises (a) suspending or dissolving the compound of Formula V in an organic solvent system; (b) adding an acid to said suspension or solution to result in a mixture; (c) subjecting said mixture to at least one heating and cooling cycle; and (d) isolating said polymorph. In various embodiments, the organic solvent system includes methanol, ethanol, THF, nitromethane, acetonitrile, methylethyl ketone (MEK), ethyl acetate, 1,4-dioxane, dichloromethane, DMSO, methyl t-butyl ether (MTBE), isopropyl alcohol (IPA), acetone, N-methyl pyrrolidone (NMP), butyl acetate, or toluene. In one embodiment, the organic solvent system is an organic solvent which is nitromethane, THF, methylethyl ketone (MEK), or acetonitrile. In various embodiments, the acid may include naphthalene sulfonic acid, naphthalene disulfonic acid, L-aspartic acid, p-toluenesulfonic acid, ethane-1,2-disulfonic acid (EDSA), hydrochloric acid (HCl), hydrobromic acid (HBr), citric acid, 2-hydroxyethanesulfonic acid, fumaric acid, sulfuric acid, maleic acid, methanesulfonic acid (MSA), phosphoric acid, or oxalic acid. In one embodiment, the acid is hydrochloric acid (HCl) or hydrobromic acid (HBr). In various embodiments, the concentration of HCl may be from 0.1-5.0 M. In a further embodiment, the concentration of HCl is 1.0 M in THF. In various embodiments, the concentration of HBr may be from 0.1-5.0 M. In a further embodiment, the concentration of HBr is 1.0 M in THF. In various embodiments, the heating cycle may start between -5 °C and 30 °C. In various embodiments, the cooling cycle may start between 40 °C and 100 °C. In one embodiment, the heating and cooling cycle is carried out between 25 °C and 50 °C. In various embodiments, the heating or cooling cycle may be ramped at 0.1-10 "C-min"1. In one embodiment, the heating or cooling cycle is ramped at 1 "C-min"1. In various embodiments, the heating or cooling cycle may last from 1 hour to 10 weeks. The pharmaceutically-acceptable polymorph may be a crystalline salt. In various embodiments, it is a crystalline HCl salt ({drug5a}) or HBr salt ({drug5b}) of the compound of Formula V. In one embodiment, it is a crystalline HCl salt with X-ray powder diffraction peaks at 9.2(±0.4) degrees, 17.5(±0.4) degrees, 24.5(±0.4) degrees, 27.4((±0.4) degrees, and 28.2 (±0.4) degrees two theta. In another embodiment, is a crystalline HBr salt with X-ray powder diffraction peaks at 18.5(±0.4) degrees, 21.6(±0.4) degrees, 22.8(±0.4) degrees, and 26.1 (±0.4) degrees two theta. In one embodiment, the invention is directed to a method of making a pharmaceutically-acceptable polymorph of the compound of Formula V:

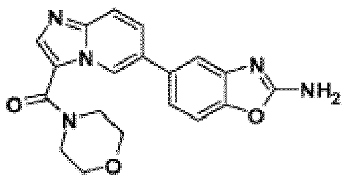

V.

said method comprises (a) dissolving the compound of Formula V in an aqueous acid to result in a solution; (b) mixing said solution with an organic solvent; and (d) isolating said salt. In various embodiments, the organic solvent may include methanol, ethanol, THF, nitromethane, acetonitrile, methylethyl ketone (MEK), ethyl acetate, 1,4-dioxane, dichloromethane, DMSO, methyl t-butyl ether (TBME), isopropyl alcohol (IP A), acetone, N-methyl pyrrolidone (NMP), butyl acetate, or toluene. In one embodiment, the organic solvent is THF. In various embodiments, the acid may include naphthalene sulfonic acid, naphthalene disulfonic acid, L-aspartic acid, p-toluenesulfonic acid, ethane- 1,2-disulfonic acid (EDSA), hydrochloric acid (HCl), hydrobromic acid (HBr), citric acid, 2-hydroxyethanesulfonic acid, fumaric acid, sulfuric acid, maleic acid, methanesulfonic acid (MSA), phosphoric acid, or oxalic acid. In one embodiment, the acid is hydrochloric acid (HCl) or hydrobromic acid (HBr).In various embodiments, the concentration of aqueous HCl may be from 1% to 37% by weight. In a further embodiment, the concentration of aqueous HCl is 36-37% by weight. In various embodiments, the concentration of aqueous HBr may be from 1%-48% by weight. In a further embodiment, the concentration of aqueous HBr is 47-48% by weight. In various embodiments, the pharmaceutically-acceptable salt may be a crystalline salt. In one embodiment, it is a crystalline HCl salt ({drug5a}) or HBr salt ({drug5b}) of the compound of Formula V. In one embodiment, it is a crystalline HCl salt with X-ray powder diffraction peaks at 8.0(±0.4) degrees, 16.0(±0.4) degrees,

19.0(±0.4) degrees, 26.5(±0.4) degrees, and 27.7(±0.4) degrees two theta. In another embodiment, is a crystalline HBr salt with X-ray powder diffraction peaks at 9.1 (±0.4) degrees, 17.3(±0.4) degrees, 21.1 (±0.4) degrees, and 27.2(±0.4) degrees two theta. In one embodiment, the invention is directed to a crystalline polymorph of the HCl salt of the compound of Formula V:

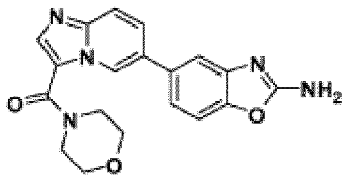

V.

In one embodiment, the crystalline HCl salt ({drug5a}) has X-ray powder diffraction peaks at 9.2(±0.4) degrees, 17.5(±0.4) degrees, 24.5(±0.4) degrees, 27.4(±0.4) degrees, and 28.2(±0.4) degrees two theta. In another embodiment, the crystalline HCl salt ({drug5a}) has X-ray powder diffraction peaks at 8.0(±0.4) degrees, 16.0(±0.4) degrees, 19.0 ((±0.4), 26.5 (±0.4), and 27.7(±0.4) degrees two theta.

In one embodiment, the invention is directed to a crystalline polymorph of the HBr salt ({drug5b}) of the compound of Formula V:

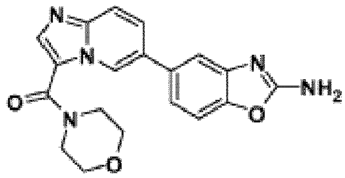

V.

In one embodiment, the crystalline HBr salt ({drug5b}) has X-ray powder diffraction peaks at 18.5(±0.4) degrees, 21.6(±0.4) degrees, 22.8(±0.4) degrees, and 26.1 (±0.4) degrees two theta. In another embodiment, the crystalline HBr salt ({drug5b}) has X-ray powder diffraction peaks at 9.1 (±0.4) degrees, 17.3(±0.4) degrees, 21.1 (±0.4) degrees, and 27.2(±0.4) degrees two theta.

In various embodiments, the invention is directed to a pharmaceutical composition comprising a therapeutically effective amount of an HCl salt ({drug5a}) of the compound of Formula V:

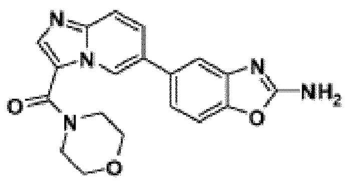

V.

and a pharmaceutically acceptable carrier. In one embodiment, the therapeutically effective HCl salt ({drug5a}) has X-ray powder diffraction peaks at 9.2(±0.4) degrees, 17.5(±0.4) degrees, 24.5(±0.4) degrees, 27.4(±0.4) degrees, and 28.2(±0.4) degrees two theta. In another embodiment, the therapeutically effective HCl salt has X-ray powder diffraction peaks at 8.0(±0.4) degrees, 16.0(±0.4) degrees, 19.0(±0.4) degrees, 26.5(±0.4) degrees, and 27.7(±0.4) degrees two theta.

Especially preferred are crystalline form of HCl salt ({drug5a}) or HBr salt ({drug5b}) of compound of formula V ({drug5}) as described in the claims of WO/2013/071272 and are defined as preferred embodiments hereinafter:

Embodiment 1. A method of making a pharmac e Compound of Formula V:

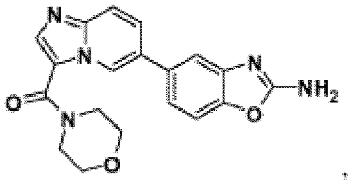

V,

said method comprising (a) suspending the compound of Formula V in an organic solvent; (b) adding an acid to said suspension to result in a mixture; (c) subjecting said mixture to at least one heating and cooling cycle; and (d) isolating said polymorph.

Embodiment 2. The method of Embodiment 1, wherein said organic solvent includes methanol, ethanol, THF, nitromethane, acetonitrile, methylethyl ketone (MEK), ethyl acetate, 1,4-dioxane, dichloromethane, DMSO, methyl t-butyl ether (MTBE), isopropyl alcohol (IPA), acetone, N-methyl pyrrolidone (NMP), butyl acetate, or toluene.

Embodiment 3. The method of Embodiment 1, wherein said acid includes naphthalene sulfonic acid, naphthalene disulfonic acid, L-aspartic acid, p-toluenesulfonic acid, ethane- 1,2-disulfonic acid (EDSA), hydrochloric acid (HCl), hydrobromic acid (HBr), citric acid, 2-hydroxyethanesulfonic acid, fumaric acid, sulfuric acid, maleic acid, methanesulfonic acid (MSA), phosphoric acid, or oxalic acid.

Embodiment 4. The method of Embodiment 1, wherein said heating cycle starts between -5 °C and 30 °C.

Embodiment 5. The method of Embodiment 1, wherein said cooling cycle starts between 40 °C -100 °C.

Embodiment 6. The method of Embodiment 1, wherein said at least one heating and cooling cycle is carried out between 25 °C and 50 °C.

Embodiment 7. The method of Embodiment 1, wherein said heating or cooling cycle is ramped at 0.1-10 °C-min-1, for example at 1 °C-min-1.

Embodiment 8. The method of Embodiment 1, wherein each said heating or cooling cycle lasts 1 hour to 10 weeks.

Embodiment 9. The method of Embodiment 1, wherein said organic solvent is nitromethane, THF, methylethyl ketone (MEK), or acetonitrile.

Embodiment 10. The method of Embodiment 9, wherein said acid is 0.1-5.0 M HCl in THF.

Embodiment 11. The method of Embodiment 10, wherein said acid is 1M HCl in THF.

Embodiment 12. The method of Embodiment 11, wherein said polymorph is a crystalline HCl salt.

Embodiment 13. The method of Embodiment 12, wherein said crystalline HCl salt has X-ray powder diffraction peaks at 9.2 (±0.4) degrees, 17.5 (±0.4) degrees, 24.5 (±0.4) degrees, 27.4 (±0.4) degrees, and 28.2 (±0.4) degrees two theta.

Embodiment 14. The method of Embodiment 9, wherein said acid is 0.1-5.0 M HBr in THF.

Embodiment 15. The method of Embodiment 14, wherein said acid is 1M HBr in THF.

Embodiment 16. The method of Embodiment 15, wherein said polymorph is a crystalline HBr salt.

Embodiment 17. The method of Embodiment 16, wherein said crystalline HBr salt has X-ray powder diffraction peaks at 18.5(±0.4) degrees, 21.6(±0.4) degrees, 22.8(±0.4) degrees, and 26.1 (±0.4) degrees two theta.

Embodiment 18. A method of making a pharmac the compound of Formula V:

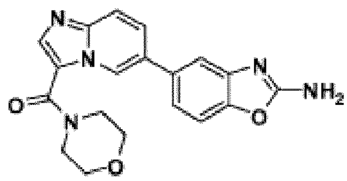

V

said method comprising (a) dissolving the compound of Formula V in an aqueous acid to result in a solution; (b) mixing said solution with an organic solvent; and (d) isolating said polymorph.

Embodiment 19. The method of Embodiment 18, wherein said aqueous acid is aqueous HCl.

Embodiment 20. The method of Embodiment 19, wherein the concentration of said aqueous HCl is 1% to 37% by weight.

Embodiment 21. The method of Embodiment 20, wherein the concentration of said aqueous HCl is 36-37% by weight.

Embodiment 22. The method of Embodiment 21, wherein said aqueous acid is aqueous HBr.

Embodiment 23. The method of Embodiment 22, wherein the concentration of said aqueous HBr is 1% to 48% by

weight.

Embodiment 24. The method of Embodiment 23, wherein the concentration of said aqueous HBr is 47-48% by weight.

Embodiment 25. The method of Embodiment 18, wherein said organic solvent includes methanol, ethanol, THF, nitromethane, acetonitrile, methylethyl ketone (MEK), ethyl acetate, 1,4-dioxane, dichloromethane, DMSO, methyl butyl ether (MTBE), isopropyl alcohol (IP A), acetone, n-methyl pyrrolidone (NMP), butyl acetate, or toluene.

Embodiment 26. The method of Embodiment 25, wherein said organic solvent is THF.

Embodiment 27. The method of Embodiment 26, wherein said aqueous acid is aqueous HCl with a concentration of 36-37% by weight.

Embodiment 28. The method of Embodiment 27, wherein said polymorph is a crystalline HCl salt.

Embodiment 29. The method of Embodiment 28, wherein said crystalline HCl salt has X-ray powder diffraction peaks at 8.0(±0.4) degrees, 16.0 (±0.4) degrees, 19.0 (±0.4) degrees, 26.5 (±0.4) degrees, and 27.7 (±0.4) degrees two theta.

Embodiment 30. The method of Embodiment 26, wherein said aqueous acid is aqueous HBr with a concentration of 47-48% by weight.

Embodiment 31. The method of Embodiment 30, wherein said polymorph is a crystalline HBr salt.

Embodiment 32. The method of Embodiment 31, wherein said crystalline HBr salt has X-ray powder diffraction peaks at 9.1 (±0.4) degrees, 17.3 (±0.4) degrees, 21.1 (±0.4) degrees, and 27.2 (±0.4) degrees two theta.

Embodiment 33. A crystalline polymorph of the HCl salt of the compound of Formula V:

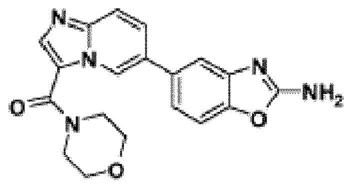

V.

Embodiment 34. The crystalline polymorph of Embodiment 33, wherein said crystalline polymorph has X-ray powder diffraction peaks at 9.2 (±0.4) degrees, 17.5 (±0.4) degrees, 24.5 (±0.4) degrees, 27.4 (±0.4) degrees, and 28.2 (±0.4) degrees two theta.

Embodiment 35. The crystalline polymorph of Embodiment 33, wherein said crystalline polymorph has X-ray powder diffraction peaks at 8.0 (±0.4) degrees, 16.0 (±0.4) degrees, 19.0 (±0.4) degrees, 26.5 (±0.4) degrees, and 27.7 (±0.4) degrees two theta.

Embodiment 36. A crystalline polymorph of the HBr salt of the compound of Formula V:

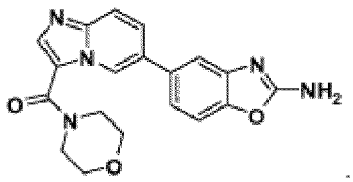

V.

Embodiment 37. The crystalline polymorph of Embodiment 36, wherein said crystalline polymorph has X-ray powder diffraction peaks at 18.5 (±0.4) degrees, 21.6 (±0.4) degrees, 22.8 (±0.4) degrees, and 26.1 (±0.4) degrees two theta.

Embodiment 38. The crystalline polymorph of Embodiment 36, wherein said crystalline polymorph has X-ray powder diffraction peaks at 9.1 (±0.4) degrees, 17.3 (±0.4) degrees, 21.1 (±0.4) degrees, and 27.2 (±0.4) degrees two theta.

Embodiment 39. A pharmaceutical composition comprising a therapeutically effective amount of the HCl salt of the compound of Formula V:

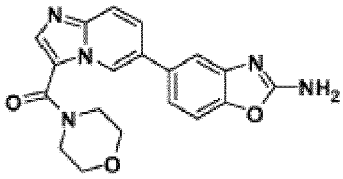

V

and a pharmaceutically-acceptable carrier.

Embodiment 40. The composition of Embodiment 39, wherein said HCl salt has X-ray powder diffraction peaks at 9.2 (±0.4) degrees, 17.5 (±0.4) degrees, 24.5 (±0.4) degrees, 27.4 (±0.4) degrees, and 28.2 (±0.4) degrees two theta.

Embodiment 41. The composition of Embodiment 39, wherein said HCl salt has X-ray powder diffraction peaks at 8.0 (±0.4) degrees, 16.0 (±0.4) degrees, 19.0 (±0.4) degrees, 26.5 (±0.4) degrees, and 27.7 (±0.4) degrees two theta.

The crystalline form of HCl salt ({drug5a}) or HBr salt ({drug5b}) of compound of formula V ({drug5}) can be obtained as decribed in WO/2013/071272: The chemical entities described herein can generally be synthesized by an appropriate combination of generally well known synthetic methods. Techniques useful in synthesizing these chemical entities are both readily apparent and accessible to those of skill in the relevant art, based on the instant disclosure. Many of the optionally substituted starting compounds and other reactants are commercially available, e.g., from Aldrich Chemical Company (Milwaukee, WI) or can be readily prepared by those skilled in the art using commonly employed synthetic methodology.

The polymorphs made according to the methods of the invention may be characterized by any methodology according to the art. For example, the polymorphs made according to the methods of the invention may be characterized by X-ray powder diffraction (XRPD), differential scanning calorimetry (DSC), thermogravimetric analysis (TGA), hot-stage microscopy, and spectroscopy (e.g., Raman, solid state nuclear magnetic resonance (ssNMR), and infrared (IR)). XRPD: Polymorphs according to the invention may be characterized by X-ray powder diffraction patterns (XRPD). The relative intensities of XRPD peaks can vary, depending upon the sample preparation technique, the sample mounting procedure and the particular instrument employed. Moreover, instrument variation and other factors can affect the 2-Θ values. Therefore, the XRPD peak assignments can vary by plus or minus about 0.1, 0.2, 0.3, or 0.4 degrees. For instance, in some embodiments the 2-Θ values of a Pattern of the invention vary by plus or minus about 0.4 degrees. In other embodiments, the 2-Θ values of a Pattern of the invention vary by plus or minus about 0.2 degrees. In still other embodiments, the 2-Θ values of a Pattern of the invention vary by plus or minus about 0.1 degrees. DSC: Polymorphs according to the invention can also be identified by its characteristic differential calorimeter scanning (DSC) trace such as shown in the Figures of WO/2013/071272. For DSC, it is known that the temperatures observed will depend upon the rate of temperature change as well as sample preparation technique and the particular instrument employed. Thus, the values reported herein relating to DSC thermograms can vary by plus or minus about 4, 6, 8 or 10°C. For instance, the values can vary by plus or minus about 6°C. TGA: The polymorphic forms of the invention may also give rise to thermal behavior different from that of the amorphous material or another polymorphic form. Thermal behavior may be measured in the laboratory by thermogravimetric analysis (TGA) which may be used to distinguish some polymorphic forms from others. In one aspect, the polymorph may be characterized by thermogravimetric analysis. GVS: Polymorphs according to the invention can also be identified by gravimetric vapor sorption (GVS), which measures rate and amount of solvent absorption by a sample. In one aspect, the polymorph may be characterized by gravimetric vapor sorption analysis. Polymorphs: The polymorph forms of the invention are useful in the production of medicinal preparations and can be obtained by means of a crystallization process to produce crystalline and semi-crystalline forms or a solidification process to obtain the amorphous form. In various embodiments, the crystallization is carried out by either generating the compound of Formula V in a reaction mixture and isolating the desired polymorph from the reaction mixture, or by dissolving raw compound in a solvent, optionally with heat, followed by crystallizing/solidifying the product by cooling (including active cooling) and/or by the addition of an antisolvent for a period of time. The crystallization or solidification may be followed by drying carried out under controlled conditions until the desired water content is reached in the end polymorphic form. In one aspect, the invention provides methods of making one or more polymorphs of the compound of the Formula V:

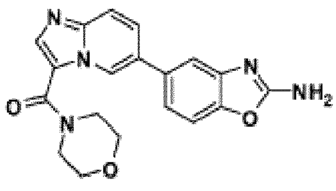

V,

or a pharmaceutically acceptable salt and/or solvate thereof. Polymorphs according to the methods of the invention can be selected from HCl Pattern 1, HCl Pattern 2, HBr Pattern 1, HBr Pattern 2, HBr Pattern 3, an amorphous form, and mixtures of more than one form. In addition, polymorphs made according to the invention may include solvates. In various embodiments, polymorphs of the invention are prepared as the free base, the mono-salt, or the bis-salt, such as the HCl salt or the bis-HCl salt of the compound of Formula V, or the HBr salt of the compound of Formula V. In various embodiments, the compound of Formula V is synthesized according to the following schemes.

Scheme 1

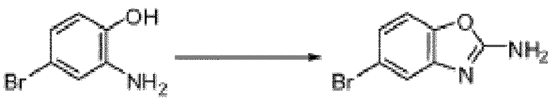

1 2

The conversion of compound 1 to compound 2 may be performed according to any method in the art. In one embodiment, compound 1 is treated with cyanogen bromide in methanol at a temperature above room temperature to yield compound 2. Scheme 2

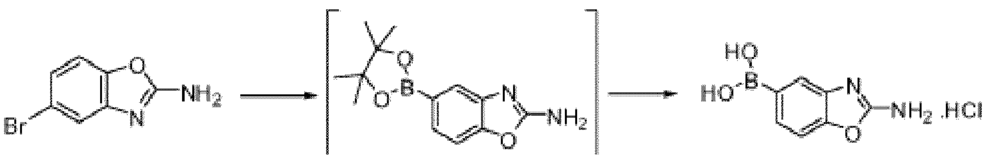

The conversion of compound 2 to compound 3 may be performed according to any method in the art. In various embodiments, compound 2 can be converted to compound 3a via a transition-metal catalyzed cross-coupling reaction with a diboron reagent. In one embodiment, compound 2 is treated with bis-(pinacolato)diboron, 1,1'-Bis[(Diphenylphosphino) ferrocene dichloropalladium (II) complexed with dichloromethane, and potassium acetate in 1,4-dioxane at an elevated temperature to give the boronic ester 3a. In one embodiment, compound 3a is further treated with acid, for example aqueous HCl, at elevated temperature to yield compound 3, the boronic acid derivative.

Scheme 3

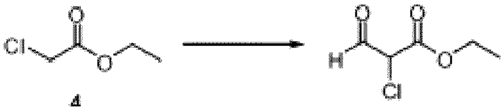

4

The conversion of compound 4 to compound 5 may be performed according to any method in the art. In one embodiment, the compound 4 is treated with ethyl formate and sodium ethoxide in methyl t-butyl ether to yield compound 5.

Scheme 4

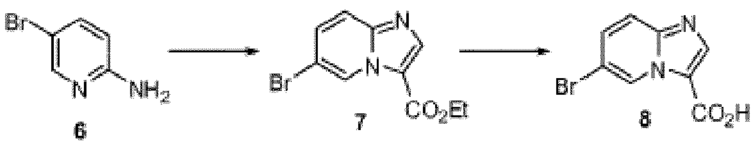

6 7 8

The conversion of compound 6 to compound 8 may be performed according to any method in the art. In one

embodiment, compound 5 and 6 are heated to reflux in methanol to yield compound 7. Further hydrolysis of compound 7 with aqueous NaOH provides compound 8 after acidic aqueous work-up.

Scheme 5

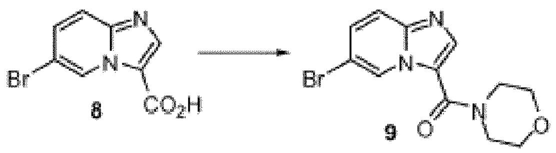

The conversion of compound 8 to compound 9 may be performed according to any method in the art. In one embodiment, compound 8 is first converted to its acid chloride upon treatment with thionyl chloride in 1,4-dioxane in the presence of a catalytic amount of DMF. The resulting acid chloride reacts with morpholine to yield compound 9.

Scheme 6

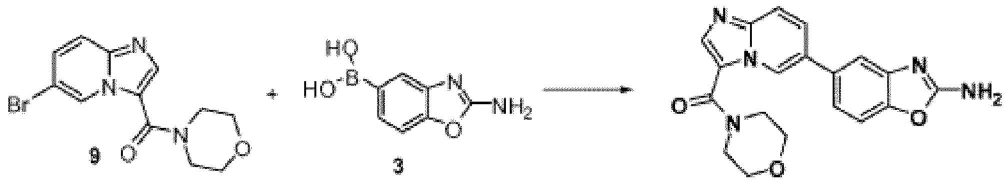

Formula V

The conversion of compound 8 to compound 9 may be performed according to any method in the art. In various embodiments, compound 9 can be coupled with compound 3 to yield the compound of Formula V via a cross-coupling reaction, such as a transition-metal catalyzed coupling reaction. In one embodiment, compounds 9 and 3 are heated in 1,4-dioxane/water in the presence of Pd(PPh3)4 and sodium carbonate to yield the compound of Formula V. Workup of the reaction product may include treatment of a solution of the compound of Formula V with activated charcoal to remove palladium. In one aspect, the invention is directed to methods of making polymorphs of the compound of the Formula V:

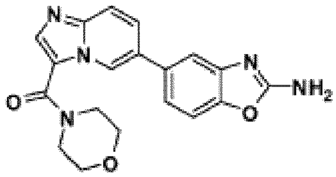

V

or a polymorph of a salt of a compound of Formula V, comprising suspending or dissolving the compound of Formula V in a solvent system, adding an acid to the suspension to result in a mixture, subjecting the mixture to a heating or cooling cycle, and isolating thepolymorph. In another aspect, a polymorph of the compound of Formula V, or a polymorph of a salt of a compound of Formula V, is prepared by suspending or dissolving a compound of Formula V in an aqueous acid, adding an organic solvent system, and isolating the polymorph. In various embodiments, the solvent system is an organic solvent system. For example, the solvent system includes methanol, ethanol, THF, nitromethane, acetonitrile, methylethyl ketone (MEK), ethyl acetate, 1,4-dioxane, dichloromethane, DMSO, methyl t-butyl ether (MTBE), isopropyl alcohol (IPA), acetone, N-methyl pyrrolidone (NMP), butyl acetate, or toluene. In various embodiments, the organic solvent system includes methanol, ethanol, THF, nitromethane, acetonitrile, methylethyl ketone (MEK), ethyl acetate, 1,4-dioxane, dichloromethane, DMSO, methyl t-butyl ether (MTBE), isopropyl alcohol (IPA), acetone, N-methyl pyrrolidone (NMP), butyl acetate, or toluene. In one embodiment, the organic solvent system is an organic solvent which is nitromethane, THF, methylethyl ketone (MEK), or acetonitrile. In various embodiments, the polymorph is obtained by suspending or dissolving a compound of Formula V in an aqueous solvent system comprising an acid. For example, the acid is naphthalene sulfonic acid, naphthalene disulfonic acid, L-aspartic acid, p-toluenesulfonic acid, ethane- 1,2-disulfonic acid (EDSA), hydrochloric acid (HCl), hydrobromic acid (HBr), citric acid, 2-hydroxyethanesulfonic acid, fumaric acid, sulfuric acid, maleic acid, methanesulfonic acid (MSA), phosphoric acid, or oxalic acid. For example, the compound of Formula V is suspended or dissolved in aqueous hydrochloric or hydrobromic acid, resulting in a slurry or solution.

Mixing or stirring may be performed. The concentration of aqueous hydrochloric acid may be, for example, between 1% and 37% by weight. In some embodiments, the concentration of hydrochloric acid is 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36% or 37% by weight. For example, 35-37% hydrochloric acid by weight is used. In other embodiments, aqueous hydrobromic acid is used and the concentration of aqueous hydrobromic acid is, for example, between 1% and 48% by weight. In some embodiments, the concentration of hydrobromic acid is 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, or 48% by weight. For example, 46-48% hydrobromic acid by weight is used. In some embodiments, a heating or cooling cycle is used to prepare a polymorph of the invention. For example, the heating cycle starts between -5°C and 30°C, between 0°C and 30°C, between 10°C and 30°C, or between 20°C and 30°C. In some embodiments, the heating cycle starts from room temperature (between about 20 and 25°C). The heating cycle is conducted up to a temperature of about 40, 45, 50, 55, 60, 65, 70, 75, 80, 85, 90, 95, 100°C or above. For example, the heating cycle is conducted up to a temperature between about 40°C and 100°C, 60°C and 100°C, 80°C and 100°C, or 90°C and 100°C. The heating may be conducted at any rate, for example between 0.1 and 10 °C per minute. In some embodiments, the heating is conducted or ramped at a rate of between 0.1 and 10 °C per minute, between 0.5 and 10 °C per minute, between 1 and 10 °C per minute, between 2 and 10 °C per minute, between 5 and 10 °C per minute, between 10 and 20 °C per minute or between 10 and 30 °C per minute. In some embodiments, a cooling cycle is used. For example, the cooling cycle starts between 15°C and 30°C, between 30°C and 60°C, between 40°C and 90°C, or between 40°C and 100°C. In some embodiments, the cooling cycle starts from room temperature (between about 20 and 25°C). In other embodiments, the cooling cycle starts from over 100°C. In other embodiments, the cooling cycle starts at a temperature between 70 and 100°C. The cooling cycle is conducted down to a temperature of about 90, 80, 70, 60, 50, 40, 30, 25, 20, 15, 10, 5, 0°C or below. For example, the cooling cycle is conducted down to a temperature between about 0°C and 40°C, 0°C and 30°C, 10°C and 30°C, or 15°C and 25°C. The cooling may be conducted at any rate, for example between 0.1 and 10 °C per minute. In some embodiments, the cooling is conducted or ramped at a rate of between 0.1 and 10 °C per minute, between 0.5 and 10 °C per minute, between 1 and 10 °C per minute, between 2 and 10 °C per minute, between 5 and 10 °C per minute, between 10 and 20 °C per minute or between 10 and 30 °C per minute. Each heating or cooling cycle may be performed as long as necessary to raise the temperature to the desired level. For example, a heating cycle may last between 1 minute and 5 minutes, between 5 minute and 10 minutes, between 10 minutes and 1 hour, or between 1 hour and 24 hours. In some embodiments, a cooling cycle is used which lasts between 1 hour and 10 weeks, or between 5 hours and 1 week, or between 24 hours and 72 hours. In one aspect, the invention is directed to methods of making polymorphs of the compound of the Formula V:

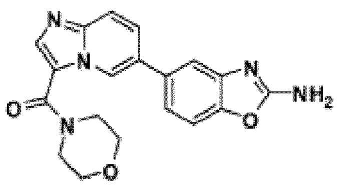

V,

or a polymorph of a salt of a compound of Formula V, either by isolation of the desired polymorph as the first solid form after synthesis of the compound of Formula V, or alternatively, by isolation of the desired polymorph as a transition from a prior solid form of the compound of Formula V. Transitions from one form to another are within the scope of the invention because they can be an alternative manufacturing method for obtaining the form desired for the production of the medicinal preparations. In various embodiments, the invention is directed to methods of making a polymorph of the compound of Formula V, wherein the method involves converting an isolated polymorph or mixture of polymorphs into a desired polymorph. In certain embodiments, the methods comprise exposing a composition comprising one or more polymorphs to conditions sufficient to convert at least about 50% of the total amount of original polymorph(s) into at least about 50% of the desired polymorph, and isolating the desired polymorph as needed.

HCl Pattern 2: In some embodiments, the polymorph according to the invention is HCl Pattern 2. An XRPD pattern of HCl Pattern 2 is shown in Figure 2 of WO/2013/071272. A TGA trace of HCl Pattern 2 is shown in Figure 4 of WO/2013/071272. A DSC trace of HCl Pattern 2 is shown in Figure 5 of WO/2013/071272. A GVS kinetic plot of HCl Pattern 2 is shown in Figure 6 of WO/2013/071272. A VT-XPvPD pattern of HCl Pattern 2 is depicted in Figure 12 of WO/2013/071272. A GVS isotherm plot of HCl Pattern 2 is shown in Figure 14 of WO/2013/071272.

In various embodiments, HCl Pattern 2 may be obtained in a mixture with non-HCl Pattern 2 polymorph forms. For example, in various embodiments, HCl Pattern 2 may be present as a composition further comprising one or more

non-HCl Pattern 2 polymorph forms. The amount of non-HCl Pattern 2 polymorph forms may vary. For example, in various embodiments, the weight ratio of polymorph HCl Pattern 2 to the total amount of one or more non-HCl Pattern 2polymorphs is greater than about 7: 1, greater than about 8: 1, greater than about 9: 1, greater than about 9.5: 1, or greater than about 99: 1. Similarly, when formulated in pharmaceutical compositions, various amounts of non-HCl Pattern 2polymorph forms may be present. In various embodiments the weight ratio of polymorph HCl Pattern 2 to the total amount of one or more non-HCl Pattern 2polymorphs in a pharmaceutical composition may be greater than about 7:1, greater than about 8:1, greater than about 9:1, greater than about 9.5: 1, or greater than about 99: 1. In various embodiments, HCl Pattern 2 may be produced by placing a compound of Formula V in a solvent or solvent system. For example, an organic solvent or solvent system is used. The compound of Formula V may form a suspension or slurry. Hydrochloric acid can be added to said suspension or slurry to result in a mixture. The mixture may be stirred, optionally with heating or cooling, until the desired amount of conversion to HCl Pattern 2 has occurred. In some embodiments, said mixture is subjected to at least one heating or cooling cycle without stirring. In various embodiments, the solvent system includes methanol, ethanol, THF, nitromethane, acetonitrile, methylethyl ketone (MEK), ethyl acetate, 1,4-dioxane, dichloromethane, DMSO, methyl t-butyl ether (MTBE), isopropyl alcohol (IP A), acetone, N-methyl pyrrolidone (NMP), butyl acetate, or toluene. In various embodiments, the organic solvent system includes methanol, ethanol, THF, nitromethane, acetonitrile, methylethyl ketone (MEK), ethyl acetate, 1,4-dioxane, dichloromethane, DMSO, methyl t-butyl ether (MTBE), isopropyl alcohol (IPA), acetone, N-methyl pyrrolidone (NMP), butyl acetate, or toluene. In one embodiment, the organic solvent system is an organic solvent which is nitromethane, THF, methylethyl ketone (MEK), or acetonitrile. In various embodiments, HCl Pattern 2 is obtained by recrystallization of a non-HCl Pattern 2 Form, including complete dissolution of the non-HCl Pattern 2 Form followed by filtration to remove any insoluble particles, and subsequent crystallization to yield HCl Pattern 2. In various embodiments, complete dissolution and filtration is not performed, in which case a slurry is formed which converts to HCl Pattern 2 without complete dissolution of one or more non-HCl Pattern 2 Forms. In various embodiments, HCl Pattern 2 is obtained by suspending or dissolving a compound of Formula V in an aqueous solvent system comprising hydrochloric acid. For example, the compound of Formula V is suspended or dissolved in aqueous hydrochloric acid, resulting in a slurry or solution. Mixing or stirring may be performed, and optional heating or cooling cycles may be performed. In one embodiment, no heating or cooling is performed. An organic solvent or solvent system may further be mixed with the slurry or solution. For example, HCl Pattern 2 is isolated following addition of an organic solvent system comprising methanol, ethanol, THF, nitromethane, acetonitrile, methylethyl ketone (MEK), ethyl acetate, 1,4-dioxane, dichloromethane, DMSO, methyl t-butyl ether (MTBE), isopropyl alcohol (IPA), acetone, N-methyl pyrrolidone (NMP), butyl acetate, or toluene. For example, the organic solvent system is an organic solvent which is nitromethane, THF, methylethyl ketone (MEK), or acetonitrile. The concentration of aqueous hydrochloric acid maybe, for example, between 1% and 37% by weight. In some embodiments, the concentration of hydrochloric acid is 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36% or 37% by weight. For example, 35-37% hydrochloric acid by weight is used. In various embodiments, the original solid form of the compound of Formula V contains greater than about 50% non-(HCl Pattern 2) polymorphs, and the desired polymorph is HCl Pattern 2. The conversion to HCl Pattern 2 may be performed for a period of time sufficient to convert at least about 50% of the total amount of non-HCl Pattern 2 polymorphs into HCl Pattern 2 of the compound of Formula V, with an optional isolation of HCl Pattern 2 from any non-HCl Pattern 2 polymorphs, as needed.

Salt Forms: In various embodiments, the compound of Formula V is a pharmaceutically acceptable salt. Pharmaceutically acceptable acid addition salts can be formed with inorganic acids and organic acids. Inorganic acids from which salts can be derived include, for example, hydrochloric acid, hydrobromic acid, sulfuric acid, nitric acid, phosphoric acid, and the like. Organic acids from which salts can be derived include, for example, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, benzene sulfonic acid, salicylic acid, 1,2-ethane disulfonic acid, and the like. Pharmaceutically acceptable base addition salts can be formed with inorganic and organic bases. Inorganic bases from which salts can be derived include, for example, sodium, potassium, lithium, ammonium, calcium, magnesium, iron, zinc, copper, manganese, aluminum, and the like. Organic bases from which salts can be derived include, for example, primary, secondary, and tertiary amines, substituted amines including naturally occurring substituted amines, cyclic amines, basic ion exchange resins, and the like, specifically such as isopropylamine, trimethylamine, diethylamine, triethylamine, tripropylamine, and ethanolamine. In some embodiments, the pharmaceutically acceptable base addition salt is chosen from ammonium, potassium, sodium, calcium, and magnesium salts. Bis salts (i.e. two counterions) and higher salts are encompassed within the meaning of pharmaceutically acceptable salts. In various embodiments, salts of Formula V may be formed with sulfuric acid, p-toluenesulfonic acid, D-glucaronic acid, ethane- 1,2-disulfonic acid (EDSA), 2-naphthalenesulfonic acid (NSA), hydrochloric acid (HCl) (mono and bis), hydrobromic acid (HBr), oxalic acid, naphthalene-1,5-disulfonic acid (NDSA), DL-mandelic acid, fumaric acid, sulfuric acid, maleic acid, methanesulfonic acid (MSA), benzenesulfonic acid (BSA), ethanesulfonic acid (ESA), L-malic acid, phosphoric acid, and aminoethanesulfonic acid (taurine). The invention provides compositions, including

pharmaceutical compositions, comprising one or more polymorphs of the present invention. The invention further provides methods for preparing compositions as described in the Examples, and particularly Examples 1-6. In various embodiments, the ratio of desired polymorph such as HCl Pattern 1, HCl Pattern 2, HBr Pattern 1, HBr Pattern 2, or HBr Pattern 3 to all other polymorphs maybe greater than about 5:1, 6: 1,7: 1,8: 1,9: 1, or more. The subject pharmaceutical compositions are typically formulated to provide a therapeutically effective amount of a polymorph of the present invention as the active ingredient, or a pharmaceutically acceptable salt, ester, prodrug, solvate, hydrate or derivative thereof. Where desired, the pharmaceutical compositions contain pharmaceutically acceptable salt and/or coordination complex thereof, and one or more pharmaceutically acceptable excipients, carriers, including inert solid diluents and fillers, diluents, including sterile aqueous solution and various organic solvents, permeation enhancers, solubilizers and adjuvants. The subject pharmaceutical compositions can be administered alone or in combination with one or more other agents, which are also typically administered in the form of pharmaceutical compositions. Where desired, the subject polymorphs and other agent(s) may be mixed into a preparation or both components may be formulated into separate preparations to use them in combination separately or at the same time. In some embodiments, the concentration of one or more of the polymorphs provided in the pharmaceutical compositions of the present invention is less than 100%, 90%, 80%, 70%, 60%, 50%, 40%, 30%, 20%, 19%, 18%, 17%, 16%, 15%,14%, 13%, 12%, 11%, 10%, 9%, 8%, 7%, 6%, 5%, 4%, 3%, 2%, 1%, 0.5%, 0.4%, 0.3%, 0.2%, 0.1%, 0.09%, 0.08%, 0.07%, 0.06%, 0.05%, 0.04%, 0.03%, 0.02%, 0.01%, 0.009%, 0.008%, 0.007%, 0.006%, 0.005%, 0.004%, 0.003%, 0.002%, 0.001%, 0.0009%, 0.0008%, 0.0007%, 0.0006%, 0.0005%, 0.0004%, 0.0003%, 0.0002%, or 0.0001% w/w, w/v or v/v. In some embodiments, the concentration of one or more of the polymorphs of the present invention is greater than 90%, 80%, 70%, 60%, 50%, 40%, 30%, 20%, 19.75%, 19.50%, 19.25% 19%, 18.75%, 18.50%, 18.25% 18%, 17.75%, 17.50%, 17.25% 17%, 16.75%, 16.50%, 16.25% 16%, 15.75%, 15.50%, 15.25% 15%, 14.75%, 14.50%, 14.25% 14%, 13.75%, 13.50%, 13.25% 13%, 12.75%, 12.50%, 12.25% 12%, 11.75%, 11.50%, 11.25% 11%, 10.75%, 10.50%, 10.25% 10%, 9.75%, 9.50%, 9.25% 9%, 8.75%, 8.50%, 8.25% 8%, 7.75%, 7.50%, 7.25% 7%, 6.75%, 6.50%, 6.25% 6%, 5.75%, 5.50%, 5.25% 5%, 4.75%, 4.50%, 4.25%, 4%, 3.75%, 3.50%, 3.25%, 3%, 2.75%, 2.50%, 2.25%, 2%, 1.75%, 1.50%, 125%, 1%, 0.5%, 0.4%, 0.3%, 0.2%, 0.1%, 0.09%, 0.08%, 0.07%, 0.06%, 0.05%, 0.04%, 0.03%, 0.02%, 0.01%, 0.009%, 0.008%, 0.007%, 0.006%, 0.005%, 0.004%, 0.003%, 0.002%, 0.001%, 0.0009%, 0.0008%, 0.0007%, 0.0006%, 0.0005%, 0.0004%, 0.0003%, 0.0002%, or 0.0001% w/w, w/v, or v/v. In some embodiments, the concentration of one or more of the polymorphs of the present invention is in the range from approximately 0.0001% to approximately 50%, approximately 0.001% to approximately 40 %, approximately 0.01% to approximately 30%, approximately 0.02% to approximately 29%, approximately 0.03% to approximately 28%, approximately 0.04% to approximately 27%, approximately 0.05% to approximately 26%, approximately 0.06% to approximately 25%, approximately 0.07% to approximately 24%, approximately 0.08% to approximately 23%, approximately 0.09% to approximately 22%, approximately 0.1% to approximately 21%, approximately 0.2% to approximately 20%, approximately 0.3% to approximately 19%, approximately 0.4% to approximately 18%, approximately 0.5% to approximately 17%, approximately 0.6% to approximately 16%, approximately 0.7% to approximately 15%, approximately 0.8% to approximately 14%, approximately 0.9% to approximately 12%, approximately 1% to approximately 10% w/w, w/v or v/v. v/v. In some embodiments, the concentration of one or more of the polymorphs of the present invention is in the range from approximately 0.001% to approximately 10%, approximately 0.01% to approximately 5%, approximately 0.02% to approximately 4.5%, approximately 0.03% to approximately 4%, approximately 0.04% to approximately 3.5%, approximately 0.05% to approximately 3%, approximately 0.06% to approximately 2.5%, approximately 0.07% to approximately 2%, approximately 0.08% to approximately 1.5%, approximately 0.09% to approximately 1%, approximately 0.1% to approximately 0.9% w/w, w/v or v/v. In some embodiments, the amount of one or more of the polymorphs of the present invention is equal to or less than 10 g, 9.5 g, 9.0 g, 8.5 g, 8.0 g, 7.5 g, 7.0 g, 6.5 g, 6.0 g, 5.5 g, 5.0 g, 4.5 g, 4.0 g, 3.5 g, 3.0 g, 2.5 g, 2.0 g, 1.5 g, 1.0 g, 0.95 g, 0.9 g, 0.85 g, 0.8 g, 0.75 g, 0.7 g, 0.65 g, 0.6 g, 0.55 g, 0.5 g, 0.45 g, 0.4 g, 0.35 g, 0.3 g, 0.25 g, 0.2 g, 0.15 g, 0.1 g, 0.09 g, 0.08 g, 0.07 g, 0.06 g, 0.05 g, 0.04 g, 0.03 g, 0.02 g, 0.01 g, 0.009 g, 0.008 g, 0.007 g, 0.006 g, 0.005 g, 0.004 g, 0.003 g, 0.002 g, 0.001 g, 0.0009 g, 0.0008 g, 0.0007 g, 0.0006 g, 0.0005 g, 0.0004 g, 0.0003 g, 0.0002 g, or 0.0001 g. In some embodiments, the amount of one or more of the polymorphs of the present invention is more than 0.0001 g, 0.0002 g, 0.0003 g, 0.0004 g, 0.0005 g, 0.0006 g, 0.0007 g, 0.0008 g, 0.0009 g, 0.001 g, 0.0015 g, 0.002 g, 0.0025 g, 0.003 g, 0.0035 g, 0.004 g, 0.0045 g, 0.005 g, 0.0055 g, 0.006 g, 0.0065 g, 0.007 g, 0.0075 g, 0.008 g, 0.0085 g, 0.009 g, 0.0095 g, 0.01 g, 0.015 g, 0.02 g, 0.025 g, 0.03 g, 0.035 g, 0.04 g, 0.045 g, 0.05 g, 0.055 g, 0.06 g, 0.065 g, 0.07 g, 0.075 g, 0.08 g, 0.085 g, 0.09 g, 0.095 g, 0.1 g, 0.15 g, 0.2 g, 0.25 g, 0.3 g, 0.35 g, 0.4 g, 0.45 g, 0.5 g, 0.55 g, 0.6 g, 0.65 g, 0.7 g, 0.75 g, 0.8 g, 0.85 g, 0.9 g, 0.95 g, 1 g, 1.5 g, 2 g, 2.5, 3 g, 3.5, 4 g, 4.5 g, 5 g, 5.5 g, 6 g, 6.5g, 7 g, 7.5g, 8 g, 8.5 g, 9 g, 9.5 g, or 10 g. In some embodiments, the amount of one or more of the polymorphs of the present invention is in the range of 0.0001-10 g, 0.0005-9 g, 0.001-8 g, 0.005-7 g, 0.01-6 g, 0.05-5 g, 0.1-4 g, 0.5-4 g, or 1-3 g. The polymorphs according to the invention are effective over a wide dosage range. For example, in the treatment of adult humans, dosages from 0.01 to 1000 mg, from 0.5 to 100 mg, from 1 to 50 mg per day, and from 5 to 40 mg per day are examples of dosages that may be used. An exemplary dosage is 10 to 30 mg per day or per week. The exact dosage will depend upon the route of administration, the form in which the polymorphs is administered, the subject to be treated, the body weight of the subject to be treated, and the preference and experience

of the attending physician. Described below are non-limiting exemplary pharmaceutical compositions and methods for preparing the same. Pharmaceutical compositions for oral administration: In some embodiments, the invention provides a pharmaceutical composition for oral administration containing a polymorph of the present invention, and a pharmaceutical excipient suitable for oral administration. In some embodiments, the invention provides a solid pharmaceutical composition for oral administration containing: (i) an effective amount of a compound of the present invention; optionally (ii) an effective amount of a second agent; and (iii) one or more pharmaceutical excipients suitable for oral administration. In some embodiments, the composition further contains: (iv) an effective amount of a third agent. In some embodiments, the pharmaceutical composition may be a liquid pharmaceutical composition suitable for oral consumption. Pharmaceutical compositions of the invention suitable for oral administration can be presented as discrete dosage forms, such as capsules, cachets, or tablets, or liquids or aerosol sprays each containing a predetermined amount of an active ingredient as a powder or in granules, a solution, or a suspension in an aqueous or non-aqueous liquid, an oil-in-water emulsion, or a water- in-oil liquid emulsion. Such dosage forms can be prepared by any of the methods of pharmacy, but all methods include the step of bringing the active ingredient into association with the carrier, which constitutes one or more necessary ingredients. In general, the compositions are prepared by uniformly and intimately admixing the active ingredient with liquid carriers or finely divided solid carriers or both, and then, if necessary, shaping the product into the desired presentation. For example, a tablet can be prepared by compression or molding, optionally with one or more accessory ingredients. Compressed tablets can be prepared by compressing in a suitable machine the active ingredient in a free-flowing form such as powder or granules, optionally mixed with an excipient such as, but not limited to, a binder, a lubricant, an inert diluent, and/or a surface active or dispersing agent. Molded tablets can be made by molding in a suitable machine a mixture of the powdered compound moistened with an inert liquid diluent. This invention further encompasses anhydrous pharmaceutical compositions and dosage forms comprising an active ingredient, since water can facilitate the degradation of some compounds. For example, water may be added (e.g., 5%) in the pharmaceutical arts as a means of simulating long-term storage in order to determine characteristics such as shelf-life or the stability of formulations over time. Anhydrous pharmaceutical compositions and dosage forms of the invention can be prepared using anhydrous or low moisture containing ingredients and low moisture or low humidity conditions. Pharmaceutical compositions and dosage forms of the invention which contain lactose can be made anhydrous if substantial contact with moisture and/or humidity during manufacturing, packaging, and/or storage is expected. An anhydrous pharmaceutical composition may be prepared and stored such that its anhydrous nature is maintained. Accordingly, anhydrous compositions may be packaged using materials known to prevent exposure to water such that they can be included in suitable formulary kits. Examples of suitable packaging include, but are not limited to, hermetically sealed foils, plastic or the like, unit dose containers, blister packs, and strip packs. An active ingredient can be combined in an intimate admixture with a pharmaceutical carrier according to conventional pharmaceutical compounding techniques. The carrier can take a wide variety of forms depending on the form of preparation desired for administration. In preparing the compositions for an oral dosage form, any of the usual pharmaceutical media can be employed as carriers, such as, for example, water, glycols, oils, alcohols, flavoring agents, preservatives, coloring agents, and the like in the case of oral liquid preparations (such as suspensions, solutions, and elixirs) or aerosols; or carriers such as starches, sugars, microcrystalline cellulose, diluents, granulating agents, lubricants, binders, and disintegrating agents can be used in the case of oral solid preparations, in some embodiments without employing the use of lactose. For example, suitable carriers include powders, capsules, and tablets, with the solid oral preparations. If desired, tablets can be coated by standard aqueous or nonaqueous techniques. Binders suitable for use in pharmaceutical compositions and dosage forms include, but are not limited to, corn starch, potato starch, or other starches, gelatin, natural and synthetic gums such as acacia, sodium alginate, alginic acid, other alginates, powdered tragacanth, guar gum, cellulose and its derivatives (e.g., ethyl cellulose, cellulose acetate, carboxymethyl cellulose calcium, sodium carboxymethyl cellulose), polyvinyl pyrrolidone, methyl cellulose, pre-gelatinized starch, hydroxypropyl methyl cellulose, microcrystalline cellulose, and mixtures thereof. Examples of suitable fillers for use in the pharmaceutical compositions and dosage forms disclosed herein include, but are not limited to, talc, calcium carbonate (e.g., granules or powder), microcrystalline cellulose, powdered cellulose, dextrates, kaolin, mannitol, silicic acid, sorbitol, starch, pre-gelatinized starch, and mixtures thereof.

The examples and preparations provided below further illustrate and exemplify the compositions of the present invention and methods of preparing such compositions. It is to be understood that the scope of the present invention is not limited in any way by the scope of the following examples and preparations. In the following examples molecules with a single chiral center, unless otherwise noted, exist as a racemic mixture. Those molecules with two or more chiral centers, unless otherwise noted, exist as a racemic mixture of diastereomers. Single enantiomers/diastereomers may be obtained by methods known to those skilled in the art.

Example 1:

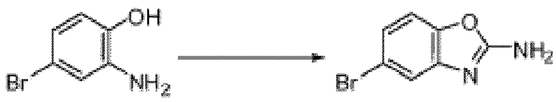

1 2

Preparation of compound 2: 1. 2-Amino-4-bromophenol (12574 g) and methanol (100.6 L, 79.6 kg) were charged to anappropriately sized reactor and stirredat room temperature (-18 to 20°C). 2. A solution of cyanogen bromide (8500 g) was prepared in methanol (25.1 L, 19.9 kg) in a separate 50 gallon reactor; this solution was charged to the starting material reaction mixture via an additional funnel (total reaction volume -150 L, 40 gal). 3. The reaction mixture was heated for -15 hours at 37 °C. 4. The reaction was monitored by HPLC until it was complete. 5. Upon completion, the reaction mixture was cooled to 18 - 20 °C. 6. 40% Sodium carbonate solution (12.41 Kg of Na2C03 in 30.2 Lwater) was added at ambienttemperature and the mixture was stirred for ~1 hour. 7. Solvent was distilled under vacuum at 30 - 40 °C to remove most of the methanol. 8. Water (50.2 L, 50.2 kg) was charged. 9. Ethyl acetate (125 L, 113kg) was addedand the resulting mixture was stirred for ~10 minutes. 10. The agitation was stopped and the layers were allowed to separate. 11. The lower aqueous phase was drained. 12. Water (50 L, 50kg) was added to the organic layer in the reactor and the resulting mixture was stirred for at least 20 minutes. 13. The agitation was stopped and the layers were allowed to separate. 14. The lower aqueous phase was drained and combined with the aqueous phase from step 11. 15. Brine (7.0 Kg of NaCl in25 kg of H20)was addedand the resulting mixture was stirred for ~10 minutes. 16. The agitation was stopped and the layers were allowed to separate. 17. The lower aqueous phase was drained and combined with the aqueous phases from step 14. 18. Magnesium sulfate (1.9 Kg) was added and the resulting mixture was stirred for at least 15 minutes. 19. The mixture was filtered into a cleaned reactor using lum in-line filter. 20. The reactor and filter lines were washed with ethyl acetate (12 L). 21. Distillation was carried out under vacuum at 30- 40 °C to minimum steerable volume (~2 volumes, ~25 L). 22. Heptane (63 L, 43 kg) was added. 23. Distillation was carried out under vacuum at 30- 40 °C to minimum stirrable volume (~2 volumes, ~25 L). 24. Steps 22 & 23were repeated one more time. 25. Heptane (32 L) was added and the resulting mixture was stirredforat least 3 hours at ambienttemperature (18 °C to 20 °C). 26. The reaction was monitored by HPLC. 27. A solidwas collected by vacuum filtration and washed with heptane (2 x 13 L, 8.9kg). 28. The solid was dried in a vacuum oven at ~47 °C to constant weight to give brown to light brown color solid (13510 g, 95% yield; HPLC purity 97.2%; 'HNMR (DMSO-d6, 300 MHz) δ 7.6 (s, 2H), 7.38-7.28 (m, 2H), 7.15-7.08 (1H)).

Example 2:

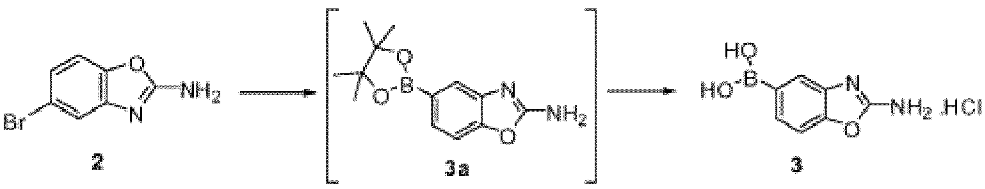

2 3a 3

Preparation of compound 4: 1. 5-Bromobenzo[b]oxazol-2-amine (Compound 2, 13400 g), bis-(pinacolato)diboron (19168 g), and 1,4-Dioxane (134 L) were added to an appropriately sized reactor and stirred at room temperature (-18 to 20 °C). 2. With stirring, the reaction mixture was sparged with nitrogen for -10 minutes at < 20 °C. 3. 1,1'-Bis[(Diphenylphosphino) ferrocene dichloropalladium (II) complexed with dichloromethane ((PdC^dppf), 2569 g)) and potassium acetate (KOAc, 18520 g) were added to the reactor. 4. With stirring, the sparging with nitrogen was continued for -10 minutes at < 20 °C. 5. The reaction mixture was heated to reflux (100 to 103 °C) under slight nitrogen blanket and stirred for 3 to 5 hours. 6. The reaction was monitored by HPLC. 7. Upon completion, the reaction mixture was cooled to 18-20 °C, filteredthrough a plug of silica gel (40.5 Kg; -30 wt%). 8. The product was further eluted with Ethyl acetate (37 mL / g) under slight vacuum. 9. The last eluting fraction of the sample was submittedfor TLC analysis. 10. The combined filtrates were concentrated under vacuum at 30-40 °C to a minimum stirrable volume (total -1.5 to 2 volumes). 11. 50% Aq. hydrochloric acid (1 : 1, Cone HCl: H20, 10 mL / g, 67 L of Cone. HClwith 67 L of Water) was charged to the thick slur in the reactor and the reaction mixture was heated to 80 to 84°C followed by stirring for 2- 4 hours at 80 to 84 °C. 12. The reaction was monitored by HPLC. 13. Upon completion, the reaction mixture was cooled to 18-20 °C. 14. A solid was collected via vacuum filtration and washed with 10% aqueous hydrochloric acid (1 :9, Cone HCl: H20) (13 L of Cone. HCl with 67 L of Water). 15. The light brown to brown solids (wet) was suspended in ethyl acetate (134 L) and stirredfor -30 minutes at 18-20 °C. 16. The solids was collected via vacuum filtration and washed

with ethyl acetate (67 L). 17. The solids was dried for -1 hour under nitrogen blanket and then dried in a vacuum oven at -50 °C to constant weight (-72 to 90 hours) with a slight nitrogen bleed to give compound 3 as a brown to light brown color solid (9479 g, 70% yield; HPLC purity 94.2%; 'HNMR (DMSO-d6, 300 MHz) δ 10.2-9.5 (1H), 7.85-7.71 (1H), 7.62-7.50 (1H)).

Example 3

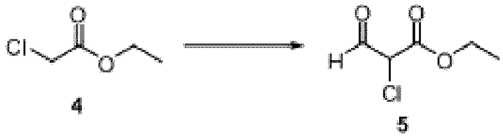

4

5

Preparation of compound 5: 1. Methyl t-butyl ether (MTBE, 102 L) was added to an appropriately sized reactor and stirred followed by the addition of sodium ethoxide (6230 g) at room temperature under nitrogen atmosphere. 2. The resulting suspension was stirred for 15 min at room temperature, then cooled to 5 to 0 °C. 3. A mixture of ethylformate (8015 g) and chloroethylacetate (10200 g) was added through the addition funnel over a 1 hour period at 0 °C to 5 °C. 4. The reaction mixture was stirred for 30 minutes at 0 to 5 °C. 5. The reaction mixture was allowed to warm to room temperature. 6. The reaction mixture was stirred for 6 to 18 hours at RT. 7. The reaction was monitored by GC. 8. The reaction mixture was cooled to 5-10 °C once the reaction was complete by GC analysis. 9. Water (51 L) was added over a minimum of 30 minutes at below 10 °C. 10. The reaction mixture was stirred for 30 minutes at 5-10 °C. 11. The reaction mixture was neutralized with cone. HCl (approximately 8 L) at below 10 °C until reaching pH 1-2. 12. The reaction mixture was warmed to room temperature (15°C to 25°C) and stirred for 30 minutes at RT. 13. The layers were separated and the top organic layer containing the product was collected. The lower aqueous layer was extracted with MTBE (51 L). 14. The two organic layers were combined and the lower aqueous layer was discarded. 15. The combined organic layer was washed with brine solution (51 L). 16. The MTBE was removed under vacuum with a jacket temp of 20-25 °C to minimum stirvolume. 17. The vacuum was discontinued and ethanol (51 L) was added to the reaction mixture.The remaining MTBE was distilled under atmospheric pressure until the internal temperature of the reaction mixture reached 70-78 °C. 18. This reaction mixture in ethanol was used for the next step with out further purification to give compound 5 as a pale yellow to brownish liquid (12530 g, assuming 100 % yield).

Example 4

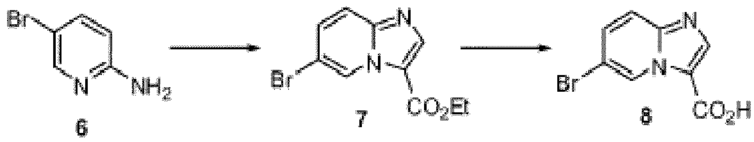

6

7

8

Preparation of compound 8: 1. An ethanol solution of compound 5 (12400 g in 51 L of ethanol) was added to an appropriately sized stainless steel reactor at room temperature under nitrogen atmosphere. 2. Compound 6 (9500 g) was added as a solid in one portion at room temperature. 3. The reaction mixture was heated to reflux (~78°C) and stirred for 1-2 days. 4. The reaction was monitored by HPLC. 5. Upon completion, the reaction mixture was allowed to cool to room temperature. 6. NaOH solution (9884 g solid pellets dissolved in 38 L of water) was added as a stream over a 30 min period at an internal temperature below 35 °C. 7. The reaction mixture was heated to reflux (~78°C) for 3 to 4 hours. 8. The reaction was monitored by HPLC. 9. Upon completion, the reaction mixture was cooled to an appropriate temperature to start solvent removal. 10. All ethanol (approximately 5 volumes of ethanol) was removed under vacuum at 40 to 45 °C. 11. The reaction mixture was cooled to room temperature. 12. Water (57 L; 6 vol) was added at room temperature. 13. The aqueous solution was washed with ethyl acetate (2 x 38 L) to remove all organic impurities. 14. The lower aqueous layer was cooled to 0-5 °C and acidified with cone. HCl (~15 L) until reaching pH 1-2. 15. The reaction mixture was stirred for 1 to 2 hours at 0 to 5 °C. 16. The mixturewas filtered and the cake was washed with water (2 x 38 L) and acetone (2 x 19L) followed by drying for 1-2 hours. 17. The solid collectedwas transferred back into an appropriately sized reactor. 18. Heptane (95 L; 10 vol) was addedto the reactor; the suspension was stirred for 4 to 5 hours at roomtemperature. 19. The solidwas collected by filtration and washed with heptane (2 x 19 L). 20. The

solid (15 kg) was suspended in methanol (75 L; 5 vol) at room temperature for 2 hours. 21. The suspension was filtered and the solid collected was washed with methanol (2x 5L). 22. The solid was dried under vacuum at 50°C to constant weight to give compound 8 as an off-white to white solid (10169 g, 83.3 % yield; HPLC purity 99.2%;1HNMR (DMSO-d6, 300 MHz) δ 9.4 (s, 1H), 8.3 (s, 1H), 7.85-7.67 (m, 2H)).

Example 5

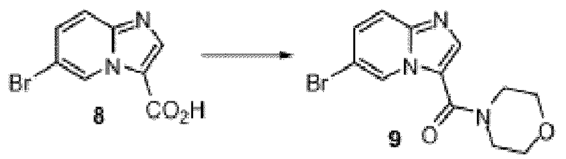

Synthesis of compound 9: 1. An appropriately sized round bottom flask (5 L) was equipped with a mechanical stirrer, thermocouple, addition funnel, nitrogen inlet and a cooling bath. 2. The flask was charged with dioxane (2.73 L) and compound 8 (273 g) at room temperature under a nitrogen atmosphere. 3. The resulting slurry was stirred for 15 minuntes at room temperature. 4. DMF (8.3 g) was added at room temperature. 5. The reaction mixture was cooled to 0°C to 5°C. 6. Thionyl Chloride (269 g) was slowly added via the addition funnel to the reaction mixture, maintaining an internal temperature between 0°C to 5°C. The addition funnel was rinsed with a minimum amount of 1,4-Dioxane (135 mL; 0.5 vol). 7. The reaction mixture was heated to reflux (98°C to 102°C) and stirred for 12 to 20 hours. 8. The reaction progress was monitored by HPLC. 9. Upon completion, the reaction mixture was cooled to 0°C to 10°C. 10. Morpholine (492 g) was added slowly via the addition funnel to the reaction mixture, maintainingan internal temperature between 0°C to 10°C under a nitrogen atmosphere. 11. The reaction mixture was warmed to room temperature and stirred at room temperature for 12 to 18 hours. 12. Progress of the reaction was monitored by HPLC. 13. Upon completion, the solvent was removed under vacuum at <50 °C to 3 volumes based on compound 8 to give a thick slurry. 14. The slurry was transferred into a round bottom flask and cooled to room temperature. 15. Water (5.46 L) was added at room temperature. 16. The resulting mixture was stirred for 3 hours at room temperature. 17. The solid was collected by filtration and washed with water (2 x 1.4 L) and heptane (2 x 1.4L). 18. The solid was dried under vacuum at 50°C to constant weight to givecompound 9 as a beige solid (296 g, 84 % yield; HPLC purity 99.5 %; 'HNMR (DMSO-d6, 300 MHz) δ 9.2 (s, 1H), 7.8 (s, 1H), 7.58-7.5 (d, 1H), 7.45-7.38 (d, 1H), 3.9-3.7 (m, 8H)).

Example 6

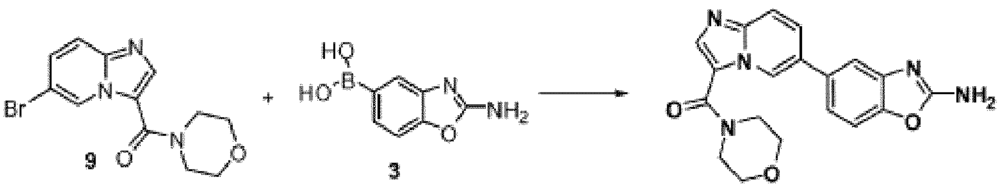

Formula V

Preparation of (6-(2-aminobenzo [d] oxazol-5 -yl)imidazo [1,2-a]pyridin-3 -yl)(morpholino)methanone (Formula V): 1. A 5 L three-neck round bottom flask was equipped with a mechanical stirrer, thermocouple probe, Nitrogen / vacuum inlet and reflux condenser and placed into a heating mantle. 2. 1,4-Dioxane (3.75 L) and water (1.25 L) were added at room temperature. 3. Compound 9 (250 g) and Compound 3 (210 g) were added to the flask at room temperature. 4. The reaction mixture was stirred for 10 min at room temperature. 5. Sodium carbonate (300 g) was added to the flask followed byPd(PPh3)4 (47 g) undemitrogen at room temperature. 6. With stirring, the reactor was sparged with nitrogen for -30 minutes at < 20°C. 7. The reaction mixture was deoxygenated by performing 5 to 6 vacuum / nitrogen cycles. 8. The reaction mixture was heated to reflux (88°C to 102°C) under slight nitrogen bubbling and stirredfor 5 to 8 hours. 9. The reaction was monitored by HPLC. 10. Upon completion, the reaction mixture was cooled to 75 - 80°C. 11. Water (3.75 L) and ethyl acetate (1.25 L) were added to the reaction mixture at 75 - 80°C. 12. The resulting mixture was cooled

to room temperature and stirred for 2 hours at room temperature. 13. The mixture was filtered and the solid collected was washed with water (2x 1.25 L), methanol (1.25 L) andethyl acetate (2 x 1.25 L). 14. The solidwas suspended in ethyl acetate (1.5 L) at room temperature for 1 hour. 15. The suspension was filtered and the solidcollected was washed with ethyl acetate (250 mL). 16. This process was repeated two more times. 17. The solidobtained was dried under vacuum to give a crude product (250 g, 85% yield; HPLC purity 98.1%; Pd level 1831 ppm). Further purification of the crude product: 18. The crude product (250g) was suspended in methanol (2.5 L) and HCl (158 mL) at room temperature. 19. The mixture was heated to 40 to 45°C to give a slightly cloudy solution. 20. Charcoal (250 g) was added to the reaction mixture at 40 to 45°C. 21. The resulting mixture was stirred for 30 minutes at 40 to 45 °C. 22. The hot solution was filtered through a poly pad with 2 inch celite bed and the cake was washed with methanol (3 x 500 mL). 23. The solution was recharged to the flask (Pd level: 330 ppm). 24. The solution was heated to 40 to 45°C. 25. Charcoal (50 g) and silica thiol (50 g) were added at 40 to 45°C. 26. The mixture was stirred for 30 minutes at 40 to 45°C. 27. The hot solution was filtered through a poly pad and the cake was washed with methanol (250 mL). 28. This process was repeated one more time. 29. The methanol was removed under vacuum at 30 - 35°C to ~2.5 L. 30. The solution was cooled to 10 to 15°C. 31. Concentrated aqueous ammonia solution (200 mL) was added until reaching pH 8-9. 32. The reaction mixture was cooled to 0 to 5°C and stirred for 1 to 2 hours at 0 to 5°C. 33. The mixture was filtered and the cake was washed with water (2 x 500 mL) and methanol (2 x 500mL). 34. The solid collected was transfered back into a round bottom flask and suspended in ethyl acetate (2.5 L) at roomtemperature for 2 to 3 hours. 35. The solid was collected by filtration and washed with ethyl acetate (750 mL). 36. The solid was dried under vacuum at ~50°C to constant weight to give thecompound of Formila I as an off-white solid (180 g, 61% yield; HPLC purity 98.5%; 'HNMR (DMSO-d6, 300 MHz) δ 9.1 (s, 1H), 8.1 (s, 1H), 7.8-7.65 (2H), 7.60-7.40 (m, 4H), 7.3-7.2 (1H), 3.8-3.6 (m, 8H); Pd level lppm).

Example 7: Instrument and Methodology Details: GVS: Sorption isotherms were obtained using a SMS DVS Intrinsic moisture sorption analyser, controlled by DVS Intrinsic Control software vl.0.0.30. The sample temperature was maintained at 25°C by the instrument controls. The humidity was controlled by mixing streams of dry and wet nitrogen, with a total flow rate of 200 mL.min-1 The relative humidity was measured by a calibrated Rotronic probe (dynamic range of 1.0 - 100%RH), located near the sample. The weight change (mass relaxation) of the sample as a function of %RH was constantly monitored by the microbalance (accuracy ±0.005 mg). Approximately 5 - 20 mg of sample was placed in a tared mesh stainless steel basket under ambient conditions. The sample was loaded and unloaded at 40%RH and 25°C (typical room conditions). A moisture sorption isotherm was performed as outlined below (2 scans giving 1 complete cycle). The standard isotherm was performed at 25°C at 10%RH intervals over a 0 90 %RH range. Data analysis was undertaken in Microsoft Excel using DVS Analysis Suite v6.0.0.7. Figure 14 of WO/2013/071272 shows a GVS isotherm plot of scaled-up HCl Pattern 2. XRPD: X-Ray Powder Diffraction patterns were collected either on a Bruker AXS C2 GADDS diffractometer or a Bruker AXS D8 diffractometer. On a Bruker AXS C2 GADDS diffractometer, the following parameters were used: Cu Ka radiation (40 kV, 40 mA), automated XYZ stage, laser videomicroscope for auto-sample positioning and a HiStar 2-dimensional area detector.X-ray optics consisted of a single Gobel multilayer mirror coupled with a pinhole collimator of 0.3 mm. A weekly performance check was carried out using a certified standard NIST 1976Corundum (flat plate). The beam divergence, i.e. the effective size of the X-ray beam on the sample, was approximately 4 mm. A Θ-Θ continuous scan mode was employed with a sample - detectordistance of 20 cm which gives an effective 2Θ range of 3.2° - 29.7°. In a typical experiment, the sampleis exposed to the X-ray beam for 120 seconds. The software used for data collectionwas GADDS for WNT 4.1.16 and the data were analyzed and presented using Diffrac PlusEVA vl 1.0.0.2 or vl3.0.0.2. Ambient conditions: Samples run under ambient conditions were prepared as flat plate specimens using powder as received without grinding. Approximately 1 - 2 mg of the sample was lightly pressed on aglass slide to obtain a flat surface. Non-ambient conditions: Samples run under non-ambient conditions were mounted on a silicon wafer with heat-conducting compound. The sample was then heated to the appropriate temperatureat 10.C.min_1and subsequently held isothermally for 1 minute before data collection was initiated. On a Bruker D8 diffractometer, the following parameters were used: Cu Ka radiation (40 kV, 40 mA), θ - 20 goniometer, and divergence of V4 and receiving slits, a Ge monochromator and a Lynxeye detector. The instrument is performance checked using a certified Corundum standard (NIST 1976). The software used for data collection was Diffrac Plus XRD Commander v2.5.0 and the data were analyzed and presented using Diffrac Plus EVA vl 1.0.0.2 or vl3.0.0.2. Samples were run under ambient conditions as flat plate specimens using powder as received. The sample was gently packed into a cavity cut into polished, zero-background (510) silicon wafer. The sample was rotated in its own plane during analysis. The details of the data collection are: · Angular range: 2 to 42°2Θ, · Step size: 0.05°2Θ, · Collection time: 0.5 s/step. XRPD Patterns for HCl Pattern 1, HCl Pattern 2, HBr Pattern 1, HBr Pattern 2, HBr Pattern 3 are shown in Figures 1, 2, 7, 10, and 13 of WO/2013/071272, respectively. Nuclear Magnetic Resonance (1H-NMR): NMR spectra were collected on a Bruker 400MHz instrument equipped with an auto-sampler, and controlled by a DRX400 console. Automated experiments were acquired using ICON-NMR v4.0.4 running with Topspin vl.3 using the standard Bruker loaded experiments. For non-routine spectroscopy, data were acquired through the use of Topspin alone. 'H-NMR spectra for HCl Pattern 1 and HCl Pattern 2 are shown in Figure 3 of WO/2013/071272. 1H-NMR spectra for HBr Pattern 1 and HBr Pattern 2 are shown in Figures 8 and 11 of WO/2013/071272, respectively. Differential Scanning Calorimetry (DSC): DSC data were collected

on a TA Instruments Q2000 equipped with a 50 position auto-sampler. The calibration for thermal capacity was carried out using sapphire and the calibration for energy and temperature was carried out using certified indium. Typically 0.5 - 3 mg of each sample, in a pin-holed aluminum pan, was heated at 10 °C -min~ 'from 25°C to 300°C. A purge of dry nitrogen at 50 mL-min_1was maintained over the sample. The instrument control software was Advantage for Q Series v2.8.0.392 and Thermal Advantage v4.8.3 and the data were analyzed using Universal Analysis v4.4A. DSC traces for HCl Pattern 2 and HBr Pattern 1 are shown in Figures 5 and 9 of WO/2013/071272, respectively. Thermo-Gravimetric Analysis (TGA): TGA data were collected on a TA Instruments Q500 TGA, equipped with a 16 position auto- sampler. The instrument was temperature calibrated using certified Alumel and Nickel. Typically 5 - 10 mg of each sample was loaded onto a pre-tared aluminum DSC pan and heated at 10 "C -min 1 from ambient temperature to 350°C. A nitrogen purge at 60 mL-min_1was maintained over the sample.

The instrument control software was Advantage for Q Series v2.8.0.392 and Thermal Advantage v4.8.3 and the data were analyzed using Universal Analysis v4.4A. Figure 4 of WO/2013/071272 shows a TGA trace for HCl Pattern 2. Ion Chromatography (IC): Data were collected on a Metrohm 861 Advanced Compact IC using IC Net software v2.3. Accurately weighed samples were prepared as stock solutions in an appropriate dissolving solution and diluted appropriately prior to testing. Quantification was achieved by comparison with standard solutions of known concentration of the ion being analyzed.

Table 1. IC Method Parameters for Anion Chromatography

| **Type of method** | **Anton exchange** |
|---|---|
| Column | Metrosep A Supp 5 - 250 (4.0 x 250 mm) |
| Column Temperature (°C) | Ambient |
| | |
| Injection (μL) | 20 |
| Detection | Conductivity detector |
| Flow Rate (mL/min) | 0.7 |
| Eluent | 3.2 mM sodium carbonate,<br>1.0 mM sodium hydrogen carbonate in 5% aqueous acetone. |

<u>Example 8:</u> Salt Selection: Preparation of the hydrochloride salt was performed in the 20 solvents used for the solubility screen in order to select the optimal solvents for the salt screen. Formula V free base (ca. 10 mg) was suspended in the selected solvents (50 volumes; 500 μl) with stirring at 40°C. HCl (1.1 equivalents; 29 of 1M solution in THF) was added, and the temperature was maintained for 30 minutes. The vials were subjected to heating/cooling cydes between room temperature and 50°C, four hours at each condition. After 1 day, an aliquot of each experiment was taken, isolated by vacuum filtration, dried by suction and analyzed by XRPD. The experiments were left cycling for further three days. An aliquot of each experiment was taken, isolated by vacuum filtration, dried by suction and analyzed by XRPD. Results are summarized in Table 2.

Table 2. Results of HCl salt screen from 20 different solvents

| **Entry** | **Solvent** | **Appearance on addition of acid** | **XRPD post 1 day maturation** | **XRPD post 4 days maturation** | **Other analysis** |
|---|---|---|---|---|---|
| -01 | Methanol | Suspension | New peaks + free base (loss of crystallinity) | Loss of crystallinity | Shifts suggest salt by $^1$H-NMR |
| -02 | Ethanol | Suspension | New peaks + free base (loss of crystallinity) | Amorphous | n/a |
| -03 | IPA | Suspension | New peaks + free base (loss of crystallinity) | Amorphous | n/a |
| -04 | Acetonitrile | Suspension | New peaks + free base | HCl Pattern 1 | Shifts suggest salt by $^1$H-NMR |
| -05 | Acetone | Suspension | New peaks + free base | Free base | n/a |

(continued)

| Entry | Solvent | Appearance on addition of acid | XRPD post 1 day maturation | XRPD post 4 days maturation | Other analysis |
|---|---|---|---|---|---|
| -06 | MEK | Suspension | New peaks + free base | HCl Pattern 1 | Shifts suggest salt by H-NMR |
| | | | | | |
| -07 | MIBK | Suspension | New peaks + free base | New peaks + free base | n/a |
| -08 | EtOAc | Suspension | New peaks + free base (loss of crystallinity) | New peaks + free base | n/a |
| -09 | IPAc | Suspension | New peaks + free base (loss of crystallinity) | New peaks + free base | n/a |
| -10 | DIPE | Suspension | New peaks + free base | New peaks + free base | n/a |
| | | | | | |
| -11 | TBME | Suspension | New peaks + free base | New peaks + free base | n/a |
| -12 | THF | Suspension | New peaks + free base (loss of crystallinity) | HCl Pattern 1 | Shifts suggest salt by $^{1}$H-NMR |
| -13 | 1-4-Dioxane | Suspension | New peaks free base (loss of crystallinity) | Amorphous | n/a |
| -14 | $MeNO_2$ | Suspension | HCl Pattern I | n/a | Shifts suggest salt by $^{1}$H-NMR (0.1 eqs residual solvent) |
| -15 | Toluene | Suspension | New peaks + free base | New peaks + free base | Shifts suggest salt by $^{1}$H-NMR |
| | | | | | |
| -16 | BuOAc | Suspension | New peaks + free base (loss of crystallinity) | New peaks + free base (loss of crystallinity) | n/a |
| -17 | DCM | Suspension | New peaks + free base (loss of crystallinity) | New peaks + free base (loss of crystallinity) | n/a |
| -18 | tBuOH | Suspension | New peaks + free base | Free base | |
| -19 | NMP | Solution | n/a (evaporation yielded oil) | n/a | n/a |
| -20 | DMSO | Solution | n/a (evaporation yielded oil) | n/a | n/a |

Formula V free base (ca. 30 mg) was suspended in selected solvents (THF, nitromethane, acetonitrile and MEK; 50 volumes, 1.5 mL) at 50°C with stirring. The corresponding acid (1 equivalent) was added and the samples were stirred for 30 minutes, prior to ramping at 1 °C -min_1 to 25°C. The samples were placed in a shaker and subjected to heating/cooling cycles, between room temperature and 50°C, with four hours at each condition. Details about the acids used for these experiments, including input materials and amounts, are summarized in Table 3 below.

Table 3. Input materials used for the salt screen

| Entry | Acid | Stock solution | Amount |
|---|---|---|---|
| -01 | Hydrochloric acid | 1M in THF | 83 μl |
| -02 | Sulfuric acid | 1M in THF | 83 μl |
| -03 | 1-2-Ethane disulfonic acid | 1M in THF | 83 μl |
| -04 | p-Toluene sulfonic acid | 1M in EtOH | 83 μl |
| -05 | Methane sulfonic add | 1M in THF | 83 μl |
| -06 | Benzene sulfonic acid | 1M in THF | 83 μl |
| -07 | Oxalic acid | 1M in THF | 83 μl |
| -08 | 2-Hydroxy ethanesulfonic acid | Added as Na salt with 1 equivalent of HCl (1M solution in THF) | 12.2 mg in 83 μl solution in THF |
| | | | |
| -09 | L-Aspartic acid | Added as solid | 10.99 ing |
| -10 | Maleic acid | 1M in THF | 83 μl |
| -11 | Phosphoric acid | 1M in THF | 83 μl |
| -12 | Ethane sulfonic acid | 1M in THF | 83 μl |
| -13 | Hydrobromic acid | 1M in THF | 83 μl |
| -14 | Naphtalene sulfonic acid | Added as Na salt with 1 equivalent of HCl (1M solution in THF) | 20.9 mg in 83 μl solution in THF |
| -15 | Naphthalene di-sulfonic acid | Added as Na salt with 2 equivalents of HCl (1M solution in THF) | 28.9 mg in 166 μl solution in THF |

After 16 hours, an aliquot was taken, isolated by vacuum filtration, dried by suction and analyzed by XRPD. Further solvent (25 volumes) was added, and the suspensions were allowed to cycle for four more days. An aliquot was taken, isolated by vacuum filtration, dried by suction and analyzed by XRPD. Further acid (0.2 equivalents, 17 μl) and solvent (25 volumes) were added to those samples showing the presence of free base in the XRPD pattern, suggesting incomplete salt formation. The samples were stored in a shaker and subjected to heating/cooling cycles, between room temperature and 50°C, with four hours at each condition for one week. An aliquot was taken, isolated by vacuum filtration, dried by suction and analyzed by XRPD. Water (10% for MeCN, and 5% for THF, MEK and nitromethane, based on ca. 1.5 mL solvent) was added and the samples were cycled for a further 5 days. An aliquot was taken, isolated by vacuum filtration, dried by suction and analyzed by XRPD. For samples exhibiting new XRPD patterns, the remaining solid was isolated by vacuum filtration, dried by suction and further characterization was undertaken as follows.

Table 4. XRPD results from the salt screen in acetonitrile.

| Entry | On acid addition | Post 3 days maturation | Post addition of+25 vols solvent | +0.2 eqs acid + 25 vols solvent | +10% water |
|---|---|---|---|---|---|
| -01 | Suspension | HCl1 +FB | HCl1 +FB | HCl 1 +FB | Partially crystalline HCl 1 |
| -02 | Suspension | Partially crystalline FB | Partially crystalline FB | Amorphous | Amorphous |
| -03 | Suspension | FB | Partially crystalline FB | Partially crystalline FB | Amorphous |
| -04 | Suspension | New peaks + FB | New peaks, ISOLATED | n/a | n/a |
| -05 | Suspension | New peaks + FB | New peaks + FB | New peaks + FB | Insufficient solid |
| -06 | Suspension | FB | FB | FB | FB |

(continued)

| Entry | On acid addition | Post 3 days maturation | Post addition of+25 vols solvent | +0.2 eqs acid + 25 vols solvent | +10% water |
|---|---|---|---|---|---|
| -07 | Suspension | New peaks + FB | New peaks + FB | New peaks + FB | FB |
| -08 | Suspension | New peaks+ HCl1 + FB | New peaks + HCl1 + FB | New peaks + HCl1 + FB | n/a |
| -09 | Suspension | FB + acid | FB + acid | FB + acid | FB + acid |
| -10 | Suspension | FB | FB | FB | FB |
| -11 | Suspension | New peaks + FB | n/a | New peaks + FB | New peaks + FB |
| -12 | Suspension | FB | n/a | New peaks + FB | New peak + FB |
| -13 | Suspension | *Partially crystalline - new peaks ISOLATED* | n/a | n/a | n/a |
| -14 | Suspension | New peaks+ HCl1 + FB | n/a | New peaks + HCl1 + FB | n/a |
| -15 | Suspension | HCl1+FB | n/a | HCl1 +FB | n/a |

Table 5. XRPD results from the salt screen in MEK

| Entry | On acid addition | Post 3 days maturation | Post addition of + 25 vols solvent | +0.2 eqs acid + 25 vols solvent | + 5% water |
|---|---|---|---|---|---|
| -01 | Suspension | HCl 1 +FB | n/a | HCl 1 | Oil |
| -02 | Suspension | Partially crystalline FB | n/a | Amorphous | Oil |
| -03 | Suspension | FB | n/a | Partially crystalline FB + new peaks | Oil |
| -04 | Suspension | New peaks + FB | n/a | New peaks + FB | Oil |
| -05 | Suspension | New peaks + FB | n/a | FB | Sticky solid |
| -06 | Suspension | FB | n/a | Amorphous | Gum |
| -07 | Suspension | New peaks ISOLATED | n/a | n/a | n/a |
| -08 | Suspension | New peaks+ HCl1 + FB | n/a | Partially crystalline - new peaks + FB | Gum |
| | | | | | |
| -09 | Suspension | FB + acid | n/a | FB + acid | Sticky solid |
| -10 | Suspension | FB | n/a | FB | Sticky solid |
| -11 | Suspension | New peaks + FB | n/a | Partially crystalline - new peaks + FB | Oil |
| -12 | Suspension | New peaks + FB | n/a | New peaks + FB | Oil |
| -13 | Suspension | New peaks + FB | n/a | n/a | n/a |

(continued)

| Entry | On acid addition | Post 3 days maturation | Post addition of + 25 vols solvent | +0.2 eqs acid + 25 vols solvent | + 5% water |
|---|---|---|---|---|---|
| -14 | Suspension | New peaks+ HCl1 + FB | n/a | Amorphous | Oil |
| -15 | Suspension | New peaks HCl1 + FB | n/a | HCl 1 +FB | Sticky solid |

Table 6. XRPD results from the salt screen in THF

| Entry | On acid addition | Post 3 days maturation | Post addition of + 25 vols solvent | + 0.2 eqs acid + 25 vols solvent | + 5% water |
|---|---|---|---|---|---|
| -01 | Suspension | HCl 1 ISOLATED | n/a | n/a | n/a |
| -02 | Suspension | New peaks + FB | n/a | Partially crystalline - new peaks + FB | Oil |
| -03 | Suspension | FB | n/a | Amorphous | Oil |
| -04 | Suspension | Amorphous | n/a | New peaks + FB | Oil |
| -05 | Suspension | New peaks + FB | n/a | Amorphous | Gum |
| -06 | Suspension | FB | n/a | FB | Gum |
| -07 | Suspension | FB | n/a | FB | Sticky solid |
| -08 | Suspension | New peaks + FB | n/a | New peaks + FB | Gum |
| -09 | Suspension | FB + acid | n/a | FB + acid | Sticky solid |
| -10 | Suspension | FB | n/a | FB | Sticky solid |
| | | | | | |
| -11 | Suspension | New peaks + FB | n/a | Partially crystalline- new peaks + FB | Gum |
| -12 | Suspension | New peaks + FB | n/a | New peaks + FB | Oil |
| -13 | Suspension | New pattern ISOLATED | n/a | n/a | n/a |
| -14 | Suspension | New peaks + HCl 1 + FB | n/a | New peaks + FB | Oil |
| -15 | Suspension | New peaks + HCl 1 + FB | n/a | HCl1 +FB | Sticky solid |

Table 7. XRPD results from the salt screen in nitromethane

| Entry | One acid addition | Post 3 days maturation | Post addition of + 25 vols solvent | + 0.2 eqs acid + 25 vols solvent | + 5% water SDR-714-24D |
|---|---|---|---|---|---|
| -01 | Suspension | HCl 1 | HCl 1 ISOLATED | n/a | n/a |
| -02 | Suspension | Amorphous | Amorphous | Gum | Oil |
| -03 | Suspension | Partially crystalline FB | FB | New peaks + FB | Oil |

(continued)

| Entry | One acid addition | Post 3 days maturation | Post addition of + 25 vols solvent | + 0.2 eqs acid + 25 vols solvent | + 5% water SDR-714-24D |
|---|---|---|---|---|---|
| -04 | Suspension | FB | New peaks + FB | Amorphous | Oil |
| -05 | Suspension | New peaks + FB | Amorphous | Partially crystalline FB | Gum |
| -06 | Suspension | FB | FB | FB | Amorphous |
| -07 | Suspension | New peaks + FB | New peaks + FB | New peaks + FB | New peaks + FB |
| -08 | Suspension | New peaks + FB | New peaks + HCl 1 +FB | Gum | n/a |
| -09 | Suspension | FB + acid | FB + acid | FB + acid | FB + acid |
| -10 | Suspension | New peaks + FB | FB | Partially crystalline FB | n/a |
| -11 | Suspension | New peaks + FB | New peaks + FB | Gum | Oil |
| -12 | Suspension | FB | FB | Oil | Oil |
| -13 | Suspension | New pattern | New pattern ISOLATED | n/a | n/a |
| -14 | Suspension | New peaks + FB | New peaks + FB | Gum | Insufficient solid |
| -15 | Suspension | Amorphous | Amorphous | Gum | Sticky solid |

The highlighted samples were filtered under suction and dried under vacuum at room temperature for ca. 68 hours. The solids obtained from HCl and HBr were characterized by XRPD, 'H-NMR, DSC and Ion Chromatography and the results from these are discussed as follows. Formula V HCl salt was obtained by maturation with additional solvent in nitromethane.

Table 8. Analysis of HCl salt from nitromethane

| Analysin | Details |
|---|---|
| XRPD (vacuum dried) | HCl Pattern 1 |
| $^1$H-NMR | Consistent with salt formation, no residual solvent |
| DSC | Several endothermic events, consistant with results from solvent screen |
| Ion Chromatography | 0.8 eqs Cl |

Formula V HBr salts were obtained by maturation for 3 days in acetonitrile and THF, and by maturation with additional solvent in nitromethane.

Table 9. HBr salts obtained in the salt screen

| Analysis | Acetonitrile: | THF: | Nitromethane: |
|---|---|---|---|
| XRPD (vacuum dried) | HBr Pattern 1, slight evidence of FB | HBr Pattern 1 | HBr Pattern 1, slight evidence of FB |
| $^1$H-NMR | Consistent with salt 0.1 eqs residual MeCN | Consistent with salt No residual solvent | Consistent with salt 0.1 eqs $MeNO_2$ |
| DSC | Double overlapping endotherm, onset at ca. 278°C | Double overlapping endotherm, onset at ca. 273°C | Endotherm, onset at ca. 289°C followed by decomposition |

(continued)

| Analysis | Acetonitrile: | THF: | Nitromethane: |
|---|---|---|---|
| Ion Chromatography | 1.1 eqs C 1 | 0.8 eqs Cl | not determined |

A summary of the XRPD data of all solids obtained in the organic solvent screen are shown in Table 10.

Table 10. XRPD of all solids obtained from the main salt screen

| Entry | Acid | MeCN | MEK | THF | MeNO2 |
|---|---|---|---|---|---|
| -01 | Hydrochloric acid | New pattern + free base # | New pattern + free base # | New pattern * | New pattern * |
| -02 | Sulfuric acid | Free base | Free base | New pattern + free base # | Free base |
| -03 | 1 -2-Ethanedisulfonic acid | Free base | Free base | Free base | Free base |
| -04 | p-Toluene sulfonic acid | New pattern * | New pattern + free base # | Amorphous | Free base |
| -05 | Methane sulfonic acid | New pattern + free base # | New patient + free base # | New pattern + free base # | New pattern + free base # |
| -06 | Benzene sulfonic acid | Free base | Free base | Free base | Free base |
| -07 | Oxalic acid | New pattern + free base # | New pattern * | Free base | New pattern + free base # |
| -08 | 2-Hydroxy ethanesulfonic acid | New pattern + free base + HCl salt # | New pattern + free base + HCl salt # | New pattern + free base # | New pattern + free base # |
| -09 | L-Aspartic acid | New pattern + free base + acid # | Free base + acid | Free base + acid | Free base + acid |
| -10 | Maleic acid | Free base | Free base | Free base | New pattern + free base # |
| -11 | Phosphoric acid | New pattern + free base # | New pattern + free base # | New pattern + free base # | New pattern + free base # |
| -12 | Ethane sulfonic acid | Free base | New pattern + free base # | New pattern + free base # | Free base |
| -13 | Hydrobromic acid | New pattern * | New pattern + free base # | New pattern * | New pattern * |
| -14 | Naphthalene sulfonic acid | New pattern + free base + HCl salt # | New pattern + free base # | New pattern + free base # | New pattern + free base + HCl salt # |
| -15 | Naphthalene di-sulfonic acid | Free base + HCl salt | New pattern + free base + HCl salt # | New pattern + free base + HCl salt # | Amorphous |

| Key: |
|---|
| *Significantly Different XRPD pattern |
| # Mixture new pattern + free base/acid |
| No new peaks – either free base or mixtures free base/acid |

An aqueous screen was performed. Formula V free base (ca. 30 mg) was slowly added into acidic solutions containing the corresponding acid (1.2 equivalents) in water (50 volumes), at 50°C, yielding white suspensions, which were subjected to heating/cooling cycles between room temperature and 50°C, four hours under each condition for 24 hours. An aliquot was taken, isolated by vacuum filtration and dried by suction and analyzed by XRPD.

Table 11. Aqueous salt screen

| Entry | Acid | Appearance | XRPD |
|---|---|---|---|
| 01 | Hydrochloric acid | White suspension | Free base |

(continued)

| Entry | Acid | Appearance | XRPD |
|---|---|---|---|
| 02 | Sulfuric acid | White suspension | Free base |
| 03 | 1-2-Ethane disulfonic acid | White suspension | Free base |
| 04 | *p*-Toluene sulfonic acid | White suspension | Free base |
| 05 | Methane sulfonic acid | White suspension | Free base |
| 06 | Benzene sulfonic acid | White suspension | Free base |
| 07 | Oxalic acid | White suspension | Free base |
| 08 | 2-Hydroxy ethanesulfonic acid | White suspension | Free base |
| 09 | L-Aspartic acid | White suspension + large crystals | Free base + acid |
| 10 | Maleic acid | White suspension | Free base |
| 11 | Phosphoric acid | White suspension | Free base |
| 12 | Ethane sulfonic acid | White suspension | Free base |
| 13 | Hydrobromic acid | White suspension | HBr Pattern 3 |
| 14 | Naphthalene sulfonic acid | Yellow suspension | Free base |
| 15 | Naphthalene di-sulfonic acid | Yellow suspension | New peaks + Free base |

The HBr sample showed a new pattern denoted as HBr Pattern 3. The remaining solid was filtered by suction, dried under vacuum at room temperature for 16 hours and characterized by XRPD, 'H-NMR, DSC and Ion Chromatography.

Table 12. Characterization of HBr salt from aqueous screen

| Analysis | Details |
|---|---|
| XRPD (vacuum dried) | HBr Pattern 3 |
| $^1$H-NMR | Consistent with salt formation, no residual solvent |
| DSC | Two broad endotherms at 66°C and 189 °C |
| Ion Chromatography | 0.8 eqs Cl |

The preparation of salts was conducted by dissolution in concentrated aqueous inorganic acids followed by precipitation with THF. Two procedures were performed, on 10 mg or 50 mg scale, investigating the effect of the order of addition of THF on the product produced.

Procedure A: Formula V free base (ca. 10 mg) was dissolved in the corresponding concentrated acid (50 μl) with stirring at room temperature and was added dropwise over THF (500 μl). Samples using HBr and HCl yielded solids and were shaken for 10 minutes and the solid was vacuum filtered, and dried under suction. H3PO4 yielded a solution that was subjected to heating/cooling cycles between room temperature and 50°C, four hours under each condition for 7 days. Procedure B: Formula V free base (ca. 50 mg) was dissolved in the corresponding concentrated acids (250 μl) with stirring at room temperature. THF (2.5 mL) was added dropwise to the solutions, and precipitation started. The samples were shaken for 30 minutes before being placed at 2-8°C for ca. 16 hours. HBr and HCl produced solids, which were vacuum filtered, washed with THF, dried under suction and under vacuum at 40°C for 16 hours. Results are shown in Table 13.

Table 13. Results from the salt screen from concentrated aqueous acids

| Acid | Procedure | Observations after adda of THF | Maturation |
|---|---|---|---|
| HBr | A | While suspension | n/a |
| HCl | A | White suspension | n/a |
| $H_3PO_4$ | A | Solution | Oil |

(continued)

| Acid | Procedure | Observations after adda of THF | Maturation |
|---|---|---|---|
| HCl | B | White suspension | n/a |
| HBr | B | White suspension | n/a |
| $H_3PO_4$ | B | Oil | n/a |

The obtained solids were characterized by XRPD, DSC, 1H-NMR and Ion Chromatography. The results are shown in Table 14 and Table 15 below.

Table 14. Characterization of HCl salts from concentrated acids

| Analysis | Details | Details |
|---|---|---|
| XRPD (air dried) | HCl Pattern 2 | HCl Pattern 2 |
| XRPD (vacuum dried) | n/a | HCl Pattern 2<br>Slight changes, more peaks |
| $^1$H-NMR | n/a | Consistent with salt formation<br>Slight shifts with respect to formula I free base |
| DSC | Multiple overlapping events | Multiple overlapping events |
| Ion Chromatography | 1.6 eqs Cl | 1.7 eqs Cl |

Table 15. Characterization of HBr s alts from concentrated acids

| Analysis | Details | Details |
|---|---|---|
| XRPD (air dried) | HBr Pattern 2 | HBr Pattern 4 |
| XRPD (vacuum dried) | n/a | HBr Pattern 4<br>Slight changes, more peaks |
| $^1$H-NMR | Consistent with salt<br>0.1 eqs residual THF | Consistent with salt<br>Slight shifts with respect to previous batch |
| DSC | Multiple overlapping events | Multiple overlapping events |
| Ion Chromatography | 1.7 eqs Br | 1.8 eqs Br |

Hydrochloride and hydrobromide salts gave crystalline salts obtained from this screen. No difference was observed between the results from the two procedures. The ion chromatography results were consistent across the two methodologies. Formation of HCl Pattern 2 was observed using this methodology. Ion chromatography evidenced >1.5 equivalents of counter ion, equating to 2 equivalents with an estimated 15% water. Formation of two new HBr patterns was also observed, denoted HBr Pattern 2 and 4, with ion chromatography suggesting possible bis-salt formation. Analogously to HCl Pattern 2, DSC analysis of HBr Patterns 2 and 4 gave multiple overlapping endotherms, which may indicate hydrate formation (as no residual solvent was noted by NMR). Correction of the ion chromatography results taking into account the possible presence of water results in the relevant equivalents of bromide being closer to 2 equivalents.

Example 9: Scale-up of HCl salt forms: HCl Pattern 1 : Formula V free base (ca. 500 mg) was suspended in nitromethane (50 volumes, 25 mL) with stirring. HCl (1M in THF; 1.2 equivalents; 1.65 mL) was added. The suspension was subjected to heating/cooling cycles between RT and 50°C, 4 hours at each condition for one week An aliquot was taken and analyzed by XRPD, which indicated that crystalline free base was still present. Further HCl (0.3 equiv, 400 μl) was added, and maturation was continued for a further week, when XRPD analysis showed the desired HCl Pattern 1. The solid was isolated by vacuum filtration and dried under vacuum at RT for 16 hours. XRPD analysis indicated the material had partially reverted to the free base, although IC suggested mono-HCl salt formation. HCl Pattern 2: Formula V free base (ca. 500 mg) was dissolved in HCl (37% aqueous solution, 2.5 mL) with stirring for 1 hour. THF (25 mL) was added dropwise over 30 minutes. Precipitation started after ca. 5 mL THF had been added. The suspension was

left stirring for five days. The solid was filtered by suction and air dried, showing a new pattern (Pattern 3) by XRPD. The solid was dried under vacuum at room temperature for ca. 16 hours, whereupon XRPD analysis confirmed the formation ofHCl Pattern 2.The observed change in XRPD pattern implies HCl Pattern 3 to be an unstable salt form, possibly solvated, which converts to HCl Pattern 2 upon drying. Full characterization of HCl Pattern 2 was undertaken and details can be found in Tables 16-17 below.

Table 16. Characterization of scale-up HCl Pattern 2

| Experiment | Details |
|---|---|
| XRPD (air dried) | New pattern (HCl Pattern 3) |
| XRPD (vacuum dried) | Crystalline. HCl Pattern 2<br>Slight differences compared to HCl Pattern 2, possibly due to differences in crystallinity. |
| $^1$H-NMR | The spectrum shows shifts suggesting salt formation with no residual solvent. |
| TGA | Weight loss in three steps:<br>8% by 100°C, 2.3% to 140°C and 8.7% to 210°C, possibly due to solvent release and dissociation |
| DSC | Multiple overlapping endothermic events. |

Table 17.Further Characterization of scale-up HCl Pattern 2

| Experiment | Details |
|---|---|
| VT-XRPD Heating to 100°C, then cool | Conversion to a new pattern at 80°C (HCl 3), which returns to less crystalline HCl 2 upon cooling. |
| VT-XRPD Heating to 140°C | Conversion to HCl Pattern 3 at 80°C that remains unchanged to 120°C, before losing crystallinity at higher temperature |
| TGA heat / cool experiments | Sample heated to 100°C, cooled and re-analysed by XRPD and IC: Partially crystalline HCl Pattern 2, 1.6eqsCl<br>Sample heated to 140°C,cooled and re-analysed by XRPD and IC: Partially crystalline HCl Pattern 2, 1.7 eqs Cl<br>Sample heated to 210°C, decomposition observed |
| Chemical purity by HPLC | Parent purity 96.8 area%<br>Sum impurities≥ 0.1 % = 3.25<br>Sum impurities ≤ 0.1% = 0.00 |
| GVS | Reversible uptake of ca. 6 wt% between 40-90%RH.<br>Reversible dehydration observed between 30-40%RH.<br>Further reversible weight toss below 30%RH may indicate lower stoichiometry hydrate.<br>The sample contained 10.6 wt% water (3.0 eqs) at the end of the experiment, with no significant changes noted by XRPD |
| | |
| VH-XRPD | The material starts to change below 25%RH, with full conversion to HCl Pattern 3 at 0.5%RH.<br>At 13%RH the material starts to revert back to HCl Pattern 2 with complete conversion by 45%RH.<br>No change was noted between 45 and 80%RH |
| Stability at 40°C/75%RH | No significant changes observed by XRPD after 3 weeks storage |
| Thermodynamic solubility | Water: 1.6 mg.mL$^{-1}$(pH 1.6)<br>0.1N HCl: 3.5 mg.ml$^{-1}$ (pH 0.9)<br>SGF:2.8mg.mL$^{-1}$(pH1.0) |

(continued)

| Experiment | Details |
|---|---|
| IC | 1.7 eqs Cl (as determined)<br>2.0 eqs Cl (corrected with 15% w/w water content) |
| KF | 15.2 % water,<br>corresponding to 4.3 eqs water, assuming 2 eqs Cl |

HCl Pattern 2 was observed after drying under vacuum over night at room temperature, showing clear differences with respect to the wet solid. Salt formation was confirmed by 'H-NMR, and high chemical purity was determined by HPLC. Ion chromatography analysis, with correction for the presence of ca. 15% water, suggested formation of a bis-HCl salt. VT-XRPD showed conversion to a new pattern, given the identification of HCl Pattern 3, upon heating above 80°C, which reverted to a partially crystalline HCl Pattern 2 upon cooling to room temperature in air. Similarly, another sample that was heated to 100°C on the TGA (8 wt% loss equates to 2.3 eqs H20), showed less crystalline HCl Pattern 2 by XRPD upon cooling, whilst IC confirmed no loss of chloride. These suggest HCl Pattern 2 to be a hydrated form that dehydrates upon heating above 50°C and readily re-hydrates upon cooling. A heat / cool TGA experiment to 140°C (2.3 wt% loss equates to 0.7 eqs H20) also yielded less crystalline HCl Pattern 2 material with no loss of chloride. Further heating lead to decomposition likely due to dissociation. GVS analysis (Figures 6 and 14 of WO/2013/071272) showed formation of a hydrated species from 30 to 40%RH, with an uptake of 10.6 wt% at the end of the analysis equating to a tri-hydrate. HCl Pattern 2 appeared to be stable to 30%RH, however dehydration was observed below 30% with rehydration occurring by 40%. Further weight loss was observed to 0%RH which may be indicative of the loss of water from the unstable mono-hydrate observed in the VT-XRPD and TGA experiments giving an unstable possibly anhydrous form.These results showthe existence of two hydrated forms; tri-hydrate HCl Pattern 2 and a mono-hydrate that readily rehydrates to give HCl Pattern 2. Dehydration of the unstable mono-hydrate by heating yielded a further unstable, possibly anhydrous, material that either showed dissociation of the salt upon further heating, or rehydration to tri-hydrate HCl Pattern 2 upon cooling. Variable humidity XRPD analysis was undertaken in order to determine if two hydrated species were present. VH-XRPD showed HCl Pattern 2 to be stable to 14%RH, whereupon the material started to change to HCl Pattern 3 with complete conversion at 0.5%RH. On increasing the humidity, the material started to re-hydrate from 13%RH, and reverted completely back to HCl Pattern 2 by 45%RH. Interestingly, and unlike VT-XRPD, no loss of crystallinity was observed upon rehydration. Only one XRPD pattern was observed upon dehydration and it is not possible to conclude whether this is a totally anhydrous or partially hydrated form. Although the sample was at 0.5%RH for 6 hours the kinetics of the VH-XRPD differs significantly from those of the GVS instrument. In a further experiment, HCl Pattern 2 was used for a polymorphism assessment. Amorphous material was generating by starting with Pattern 2 of HCl salt ofFormula V(ca. 500 mg) and suspending it in 150 mL water, resulting in a cloudy solution, which was filtered by suction. The solution was freeze- dried over night, yielding a partially crystalline HCl Pattern 2 material. The least crystalline batch of HCl Pattern 2 material was used for the polymorph assessment. Freeze-dried material (ca. 8mg) was weighed into a vial and the selected solvent (20 vols, 200 μl) was added. The suspensions were subjected to heat/cool cycles between RT and 50°C, four hours at each condition, for three days. An aliquot was taken, isolated by vacuum filtration, dried by suction and analyzed by XRPD and the results are summarized in Table 18. Samples showed the known form HCl Pattern 2, including those in alcoholic solvents which had given amorphous material during the solvent screen, with improved crystallinity in all cases compared with the starting material.

Table 18. Results from the polymorph assessment

| Entry | Solvent | XRPD Result |
|---|---|---|
| -01 | MeOH | HCl Pattern 2 |
| -02 | EtOH | HCl Pattern 2 |
| -03 | IPA | HCl Pattern 2 |
| -04 | THF | HCl Pattern 2 |
| -05 | MeCN | HCl Pattern 2 |
| -06 | IPA (10% water) | HCl Pattern 2 |
| -07 | THF (5% water) | HCl Pattern 2 |
| -08 | EtOAc | HCl Pattern 2 |

(continued)

| Entry | Solvent | XRPD Result |
|---|---|---|
| -09 | MEK | HCl Pattern 2 |
| -10 | TBME | HCl Pattern 2 |
| -11 | DCM | HCl Pattern 2 |
| -12 | Nitromethane | HCl Pattern 2 |

The invention relates to an administration unit comprising crystalline form of HCl salt ({drug5a}) or HBr salt ({drug5b}) of compound of formula V ({drug5}). The administration unit according to the invention is useful for treating various diseases and disorders which include but are not limited to benign and malignant proliferative diseases, disorders such as allergic contact dermatitis, rheumatoid arthritis, osteoarthritis, inflammatory bowel diseases, chronic obstructive pulmonary disorder, psoriasis, multiple sclerosis, asthma.

SPECIFIC INORMATION CONCERNING ASPECT (VI) OF THE INVENTION

Aspect (vi) of the invention concerns forms of {drug6}, especially N-methyl-2-[3-((E)-2-pyridin-2-yl-vinyl)-1H-indazol-6-ylsulfanyl]-benzamide for the treatment of chronic myelogenous leukemia = N-methyl-2-[3-((E)-2-pyridin-2-yl-vinyl)-1H-indazol-6-ylsulfanyl]-benzamide for the treatment of chronic myelogenous leukemia, particularly solid form of free molecule or pharmaceutical acceptable salts of N-methyl-2-[3-((E)-2-pyridin-2-yl-vinyl)-1H-indazol-6-ylsulfanyl]-benzamide for the treatment of chronic myelogenous leukemia = solid form of free molecule or pharmaceutical acceptable salts of N-methyl-2-[3-((E)-2-pyridin-2-yl-vinyl)-1H-indazol-6-ylsulfanyl]-benzamide for the treatment of chronic myelogenous leukemia. The invention relates to a form of N-methyl-2-[3-((E)-2-pyridin-2-yl-vinyl)-1H-indazol-6-ylsulfanyl]-benzamide for the treatment of chronic myelogenous leukemia, namely to solid form of free molecule or pharmaceutical acceptable salts of N-methyl-2-[3-((E)-2-pyridin-2-yl-vinyl)-1H-indazol-6-ylsulfanyl]-benzamide for the treatment of chronic myelogenous leukemia, which is useful for treating chronic myelogenous leukemia. N-methyl-2-[3-((E)-2-pyridin-2-yl-vinyl)-1H-indazol-6-ylsulfanyl]-benzamide for the treatment of chronic myelogenous leukemia and / or solid form of free molecule or pharmaceutical acceptable salts of N-methyl-2-[3-((E)-2-pyridin-2-yl-vinyl)-1H-indazol-6-ylsulfanyl]-benzamide for the treatment of chronic myelogenous leukemia is described in WO/2013/068909, which is incorporated by reference. The compound N-methyl-2-[3-((E)-2-pyridin-2-yl-vinyl)-1H-indazol-6-ylsulfanyl]-benzamide or 6-[2-(methylcarbamoyl)phenylsulfanyl]-3-E-[2-(pyridin-2-yl)ethenyl]indazole, of the following structure:

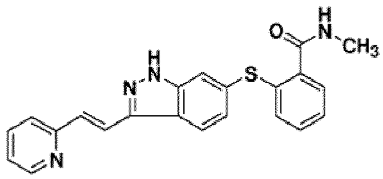

is known as axitinib or AG-013736. The invention relates to an administration unit comprising N-methyl-2-[3-((E)-2-pyridin-2-yl-vinyl)-1H-indazol-6-ylsulfanyl]-benzamide for the treatment of chronic myelogenous leukemia and / or solid form of free molecule or pharmaceutical acceptable salts of N-methyl-2-[3-((E)-2-pyridin-2-yl-vinyl)-1H-indazol-6-ylsulfanyl]-benzamide for the treatment of chronic myelogenous leukemia. The administration unit according to the invention is useful for treating various diseases and disorders which include but are not limited to chronic myelogenous leukemia. Preferred embodiments of the administration unit are as described in WO/2013/068909: The present invention relates to a method of treating chronic myelogenous leukemia in a subject comprising administering to the subject a compound, such as N-methyl-2-[3-((E)-2-pyridin-2-yl-vinyl)-1H-indazol-6-ylsulfanyl]-benzamide, that inhibits the T315I mutation in BCR-ABL tyrosine kinase, or a pharmaceutically acceptable salt thereof. The present invention also relates to a pharmaceutical composition comprising a compound such as N-methyl-2-[3-((E)-2-pyridin-2-yl-vinyl)-1H-indazol-6-ylsulfanyl]-benzamide, that inhibits the T315I mutation in BCR-ABL tyrosine kinase, or a pharmaceutically acceptable salt thereof, and a pharmaceutically acceptable carrier or diluent.

Chronic myelogenous leukemia (CML) is a hematological stem cell disorder caused by increased and unregulated growth of myeloid cells in the bone marrow, and the accumulation of excessive white blood cells. Abelson tyrosine kinase (ABL) is a non-receptor tyrosine kinase involved in cell growth and proliferation and is usually under tight control. However, 95 % of CML patients have the ABL gene from chromosome 9 fused with the breakpoint cluster (BCR) gene from chromosome 22, resulting in a short chromosome known as the Philadelphia chromosome. This Philadelphia

chromosome is responsible for the production of the BCR-ABL fusion protein, a constitutively active tyrosine kinase that causes uncontrolled cellular proliferation. An ABL inhibitor, imatinib, was approved by the FDA for the treatment of CML, and is currently used as first-line therapy. It has been reported that 80 % of CML patients respond to imatinib with under 3 % progressing to advanced disease within 5 years (Schwartz, P.A., et al., Bioorg Chem 39: 192 (201 1)). The durability of clinical response, however, is adversely affected by the development of resistance to drug therapy. In 2001, the first imatinib resistant mutant was reported as a T315I BCR-ABL "gatekeeper" mutation. Subsequent analysis revealed that reoccurrence arises with over fifty-five documented point mutations occurring throughout the catalytic and regulatory domains, with a large percentage located in the G-loop and the gatekeeper position (Id.). Clinical success has been achieved towards most mutations, but there is no approved agent directed towards the gatekeeper T315I mutation (O'Hare, T., et al., Clin Cancer Res 17: 212 (201 1)).

The compound, N-methyl-2-[3-((E)-2-pyridin-2-yl-vinyl)-1H-indazol-6-ylsulfanyl]-benzamide or 6-[2-(methylcarbamoyl)-phenylsulfanyl]-3-E-[2-(pyridin-2-yl)ethenyl]indazole, of the following structure:

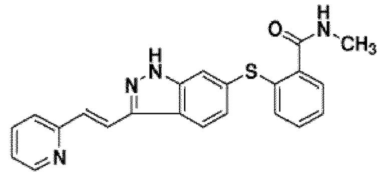

is known as axitinib or AG-013736.

Axitinib is a potent and selective inhibitor of vascular endothelial growth factor (VEGF) receptors 1, 2 and 3. These receptors are implicated in pathologic angiogenesis, tumor growth, and metastatic progression of cancer. Axitinib has been shown to potently inhibit VEGF-mediated endothelial cell proliferation and survival (Hu-Lowe, D.D., et al., Clin Cancer Res 14: 7272-7283 (2008); Solowiej, S., et al, Biochemistry 48: 7019-31 (2009)). Clinical trials are currently ongoing to study the use of axitinib for the treatment of various cancers, including liver cancer, melanoma, mesothelioma, non-small cell lung cancer, prostate cancer, renal cell carcinoma, soft tissue sarcomas and solid tumors. Inlyta® (axitinib) has been approved in the United States, Europe, Japan and other jurisdictions for the treatment of renal cell carcinoma. Axitinib, as well as pharmaceutically acceptable salts thereof, is described in U.S. Patent No. 6,534,524. Methods of making axitinib are described in U.S. Patent Nos. 6,884,890 and 7,232,910, in U.S. Publication Nos. 2006-0091067 and 2007-0203196 and in International Publication No. WO 2006/048745. Dosage forms of axitinib are described in U.S. Publication No. 2004-0224988. Polymorphic forms and pharmaceutical compositions of axitinib are also described in U.S. Publication Nos. 2006-0094763, 2008-0274192 and 2010-0179329. The patents and patent applications listed above are incorporated herein by reference.

Each of the embodiments described below can be combined with any other embodiment described herein not inconsistent with the embodiment with which it is combined. Some embodiments relate to a method of treating chronic myelogenous leukemia in a subject comprising administering to the subject a compound that inhibits the T315I mutation in BCR-ABL tyrosine kinase, or a pharmaceutically acceptable salt thereof. Additional embodiments relate to the method described above, wherein the subject has the T315I mutation in BCR-ABL tyrosine kinase. Further embodiments relate to the methods described above, wherein the compound is N-methyl-2-[3-((E)-2-pyridin-2-yl-vinyl)-1H-indazol-6-ylsulfanyl]-benzamide, or a pharmaceutically acceptable salt thereof. More embodiments relate to a method of treating chronic myelogenous leukemia in a subject comprising administering to the subject a compound that inhibits the T315I mutation in BCR-ABL tyrosine kinase, wherein the compound is N-methyl-2-[3-((E)-2-pyridin-2-yl-vinyl)-1H-indazol-6-ylsulfanyl]-benzamide, or a pharmaceutically acceptable salt thereof. Additional embodiments relate to a method of treating chronic myelogenous leukemia in a subject having the T315I mutation in BCR-ABL tyrosine kinase, comprising administering to the subject a compound that inhibits the T315I mutation in BCR-ABL tyrosine kinase, or a pharmaceutically acceptable salt thereof. Further embodiments relate to a method of treating chronic myelogenous leukemia in a subject having the T315I mutation in BCR-ABL tyrosine kinase, comprising administering to the subject a compound, which is N-methyl-2-[3-((E)-2-pyridin-2-yl-vinyl)-1H-indazol-6-ylsulfanyl]-benzamide, or a pharmaceutically acceptable salt thereof. Additional embodiments relate to a method of treating chronic myelogenous leukemia in a subject having the T315I mutation in BCR-ABL tyrosine kinase, comprising administering to the subject a compound that inhibits the T315I mutation in BCR-ABL tyrosine kinase, wherein the compound is N-methyl-2-[3-((E)-2-pyridin-2-yl-vinyl)-1H-indazol-6-ylsulfanyl]-benzamide, or a pharmaceutically acceptable salt thereof. Some embodiments relate to a pharmaceutical composition comprising a compound that inhibits the T315I mutation in BCR-ABL tyrosine kinase, or a pharmaceutically acceptable salt thereof, and a pharmaceutically acceptable carrier or diluent. Further embodiments relate to the pharmaceutical composition described above, wherein the compound is N-methyl-2-[3-((E)-2-pyridin-2-yl-vinyl)-1H-indazol-6-ylsulfanyl]-benzamide, or a pharmaceutically acceptable salt thereof. Additional embodiments relate to a pharmaceutical composition comprising a compound that inhibits the T315I mutation in BCR-ABL tyrosine kinase, wherein the compound

is N-methyl-2-[3-((E)-2-pyridin-2-yl-vinyl)-1H-indazol-6-ylsulfanyl]-benzamide, or a pharmaceutically acceptable salt thereof, and a pharmaceutically acceptable carrier or diluent. Further embodiments relate to a pharmaceutical composition for treating chronic myelogenous leukemia in a subject comprising a compound that inhibits the T315I mutation in BCR-ABL tyrosine kinase, wherein the compound is N-methyl-2-[3-((E)-2-pyridin-2-yl-vinyl)-1H-indazol-6-ylsulfanyl]-benzamide, or a pharmaceutically acceptable salt thereof, and a pharmaceutically acceptable carrier or diluent. Additional embodiments relate to a pharmaceutical composition for treating chronic myelogenous leukemia in a subject having the T315I mutation in BCR-ABL tyrosine kinase, comprising a compound that inhibits the T315I mutation in BCR-ABL tyrosine kinase, wherein the compound is N-methyl-2-[3-((E)-2-pyridin-2-yl-vinyl)-1H-indazol-6-ylsulfanyl]-benzamide, or a pharmaceutically acceptable salt thereof, and a pharmaceutically acceptable carrier or diluent.

The following abbreviations may be used herein: ABLtide (synthetic peptide sequence ([5-carboxyfluorescein]-EAIYAAPFAKKK-COHN2) based on the C-terminus of ABL); ADP (adenosine diphosphate); ATP (adenosine triphosphate); BRIJ-35 (polyoxyethylene lauryl ether); BSA (bovine serum albumin); DMSO (dimethylsulphoxide); dithiothreitol (DTT); EGTA (ethylene glycol tetraacetic acid); ELISA (enzyme linked immunosorbent assay); FBS (fetal bovine serum); HEPES (4-(2-hydroxyethyl)piperazine-1 -ethanesulfonic acid or /V-(2-hydroxyethyl)piperazine-/V-(2-ethanesulfonic acid)); NADH (reduced form of nicotinamide adenine dinucleotide or nicotinamide adenine dinucleotide plus hydrogen); RPMI (Roswell Park Memorial Institute); SDS (sodium dodecylsulfate); Tyr (tyrosine); Tyr 02 peptide (peptide sequence (EAIYAAPF)); and WT (wild-type).

The phrase "pharmaceutically acceptable salt(s)", as used herein, unless otherwise indicated, includes salts of acidic or basic groups which may be present in the compounds of described herein. The compounds described herein that are basic in nature are capable of forming a wide variety of salts with various inorganic and organic acids. The acids that may be used to prepare pharmaceutically acceptable acid addition salts of such basic compounds described herein are those that form non-toxic acid addition salts, e.g., salts containing pharmacologically acceptable anions, such as the hydrochloride, hydrobromide, hydroiodide, nitrate, sulfate, bisulfate, phosphate, acid phosphate, isonicotinate, acetate, lactate, salicylate, citrate, acid citrate, tartrate, pantothenate, bitartrate, ascorbate, succinate, maleate, gentisinate, fumarate, gluconate, glucuronate, saccharate, formate, benzoate, glutamate, methanesulfonate, ethanesulfonate, benzenesulfonate, p-toluenesulfonate and pamoate [i.e., 1,1 '-methylene-bis-(2-hydroxy-3-naphthoate)] salts. The compounds described herein that include a basic moiety, such as an amino group, may form pharmaceutically acceptable salts with various amino acids, in addition to the acids mentioned above. The term "treating", as used herein, unless otherwise indicated, means reversing, alleviating, inhibiting the progress of, or preventing the disorder or condition to which such term applies, or one or more symptoms of such disorder or condition. The term "treatment", as used herein, unless otherwise indicated, refers to the act of treating as "treating" is defined immediately above. The term "subject", as used herein, may be a human or non-human mammal (e.g., rabbit, rat, mouse, horse, monkey, other lower-order primate, etc.). In an embodiment, the term "subject" refers to a human.

Administration of the compounds described herein can be effected by any method or route that enables delivery of the compounds to the site of action. These methods include oral routes, intraduodenal routes, parenteral injection (including intravenous, subcutaneous, intramuscular, intravascular or infusion), topical, and rectal administration. The dosage administered will, of course, vary with the compound employed, the mode of administration, the treatment desired and the disorder indicated. The dosage may be as a single dose or according to a multiple dose regimen, alone or in combination with other compounds, agents or substances. One of ordinary skill in the art would be able to determine such amounts based on such factors as a subject's size, the severity of a subject's symptoms, and the particular composition or route of administration selected. The daily dosage of the compound, or pharmaceutically acceptable salt thereof, may be in the range from 0.1 mg to 1 gram, preferably 0.1 mg to 250 mg, more preferably 0.1 mg to 50 mg. In some embodiments, satisfactory results are obtained when axitinib, or a pharmaceutically acceptable salt thereof, is administered at a daily dosage of from of from about 0.01 mg/kg to about 0.4 mg/kg of body weight, optionally given in divided doses two to four times a day. The total daily dosage is projected to be from about 0.1 to about 25 mg, preferably from about 1 mg to about 10 mg two times a day, and more preferably from about 2 to about 10 mg two times a day. This dosage regimen maybe adjusted to provide the optimal therapeutic response. For example, several divided doses may be administered daily or the dose may be proportionally reduced or increased as indicated by the exigencies of the therapeutic situation. The pharmaceutical composition may, for example, be in a form suitable for oral administration as a tablet, capsule, pill, powder, sustained release formulation, solution, or suspension, for parenteral injection as a sterile solution, suspension or emulsion, for topical administration as an ointment or cream or for rectal administration as a suppository. The pharmaceutical composition may be in unit dosage forms suitable for single administration of precise dosages. The pharmaceutical composition includes a conventional pharmaceutically acceptable carrier or excipient and a compound described herein as an active ingredient. In addition, it may include other medicinal or pharmaceutical agents, carriers, or adjuvants. Exemplary parenteral administration forms include solutions or suspensions of active compounds in sterile aqueous solutions, for example, aqueous propylene glycol or dextrose solutions. Such dosage forms can be suitably buffered, if desired. Suitable pharmaceutical carriers include inert diluents or fillers, water and various organic solvents. The pharmaceutical compositions may, if desired, contain additional ingredients such as flavorings, binders, excipients and the like. Thus for oral administration, tablets containing various excipients, such as citric acid may be employed together with various disintegrants such as starch, alginic acid and certain complex silicates and with binding agents such as sucrose, gelatin and acacia. Additionally, lubricating agents such as magnesium stearate, sodium lauryl sulfate and talc are often useful for tableting purposes. Solid compositions of a similar type may also be employed in soft and hard filled gelatin capsules. Preferred materials, therefore, include lactose or milk sugar and high molecular weight polyethylene glycols. When aqueous suspensions or elixirs are desired for oral administration the active compound therein may be combined with various sweetening or flavoring agents, coloring matters or dyes and, if desired, emulsifying agents or suspending agents, together with diluents such as water, ethanol, propylene glycol, glycerin, or combinations thereof.

Example 1: ABL1 Kinase Spectrophotometric Coupled Enzymatic Assay: Axitinib was tested in a spectrophotometric coupled enzymatic assay used to measure ABL1 and ABL1 [T315I] enzymatic activity, which has been previously described (Solowiej, J., et ai, Biochemistry, 48: 7019-7031 (2009)). The kinase-catalyzed production of ADP that accompanies phosphoryl transfer of the γ-phosphate of ATP to the tyrosine residue in human minigastrin 1 peptide (LEEEEEAYGWMDF-NH2) was coupled to the oxidation of NADH through the activities of pyruvate kinase (PK) and lactate dehydrogenase (LDH). Concommitant with the production of ADP is the β-NADH (reduced form of nicotinamide adenine dinucleotide) conversion to p-NAD+ (oxidized form of nicotinamide adenine dinucleotide) which was monitored by the decrease in absorbance at 340 nm (ε = 6220 cm"1 M"1) using a Beckman DU800 spectrophotometer at 25 °C. Typical reaction solutions contained 2 mM phosphoenolpyruvate, 0.28 mM NADH, 50 mM MgCl2, 2 mM DTT, ATP, minigastrin, 15 units/mL PK, 15 units/mL LDH in 25 mM HEPES, pH 7.5. To determine kinetic parameters, ATP was varied from 5 to 1000 μM. Assays were initiated with the addition of 20 nM full length ABL1 or 100 nM ABL1 [T315I]. The same coupled enzymatic assay format was used for percent inhibition and K, determinations. Axitinib was prepared in 100 % DMSO. The final concentration of DMSO in the assay was 1 %. The concentration of ATP was 3X km (1 65 μM ABL1 and 40 μM ABL1 [T315I]. The concentration of minigastrin was 500 μM. K, determinations were made from a plot of the fractional velocity as a function of inhibitor concentration fit to the quadratic equation for competitive inhibition "Morrison equation" with the enzyme concentration as a variable (Morrison, J. F., Biochimica et biophysica acta, 185: 269-286 (1969)). As shown in Table 1, the % inhibition data generated for axitinib indicated a shift in potency toward the ABL1 [T315I] mutation.

Table 1. Axitinib Inhibitory Potencies Toward Enzymatic Activities of ABL1 Proteins Determined by the ABL1 Kinase Spectrophotometric Coupled Enzymatic Assay

| **Kinase** | **% Inh @ 20nM** |
|---|---|
| ABL WT | 25 |
| ABL(T315I) | 80 |

Example 2: ABL1 Kinase Mobility-Shift Assay: Axitinib was tested with the Caliper LabChip3000 assay (Caliper Life Science, Hopkinton, MA), which is a mobility-shift assay (MSA) that combines the basic principles of capillary electrophoresis in a micro-fluidic environment. Axitinib was prepared in 100 % DMSO, diluted to 25 % DMSO with 20 mM HEPES pH 7.5, and added to the reaction for a final DMSO concentration of 6 %. Inhibitor concentrations varied from 1.0 μM to 0.00003 μM. Typical reactions were 20 μl_, contained ABL1 or ABL1 [T315I], ATP (ABL1; 25 μM and ABL1 [T315I]; 5 μM), 1.0 μM ABLtide, 5 mM MgCl2, 2 mM DTT, 0.01 % Triton® X-100 (Sigma-Aldrich, St. Louis, MO), 6 % DMSO in 20 mM HEPES pH 7.5. The mixture was incubated in a 384-well polypropylene plate at room temperature for an hour and terminated by the addition of 60 μl of QuickScout Screening Assist MSA Buffer (Carna Biosciences, Kobe, Japan). The reaction mixture was applied to a LabChip3000 system, and the product /substrate peptide peaks were separated. The kinase reaction was quantitated by the product ratio calculated from peak heights of product (P) and substrate (S) peptides (P/(P+S)). As shown in Table 2, the data generated for axitinib demonstrated a 14 fold shift in potency for the gatekeeper mutation versus the wild type enzyme.

Table 2. Axitinib Inhibitory Potencies Toward Enzymatic Activities of ABL1 Proteins Determined by the ABL1 Kinase Mobility-Shift Assay

| **Kinase** | **$IC_{50}$(nM)** |
|---|---|
| ABL WT | 7.784 |
| ABL(T315I) | 0.5511 |

Example 3: ABL1 Kinase Z'-LYTE Screening Assay: Axitinib was tested using a Z'-LYTE Screening Protocol

(Invitrogen, Carlsbad, CA). Axitinib was prepared in 1 00 % DMSO, and added to the reaction for a final DMSO concentration of 1 %. Inhibitor concentrations varied from 1.0 μM to 0.00003 μM. Typical reactions were 10 μl_, contained ABL1 or ABL1 [T315I], ATP (ABL1; 10 μM and ABL1 [T315I]; 5 μM), 2.0 μM Tyr 02 peptide, 10 mM MgCl2, 1.0 mM EGTA, 0.01 % BRIJ-35, 1 % DMSO in 50 mM HEPES pH 7.5. The mixture was incubated in a 384-well polypropylene plate at room temperature for an hour and terminated by the addition of 5 μl_ of a 1 :64 dilution of the development reagent utilized with Z'-LYTE® Screening Protocol, followed by a 30 second shake. The development reaction was allowed to incubate at room temperature for one hour. The resulting fluorescence was read on a fluorescence plate reader and the data were analyzed.

As shown in Table 3, the data generated for axitinib demonstrated a 6.2 fold shift in potency for the gatekeeper mutation vs. the wild type enzyme.

Table 3. Axitinib Inhibitory Potencies Toward Enzymatic Activities of ABL1 Proteins Determined by the ABL1 Kinase Z'-LYTE® Screening Assay

| **Kinase** | **$IC_{50}$(nM)** |
|---|---|
| ABEL WOT | 2.6 |
| ABL(T315I) | 0.418 |

Example 4: BCR-ABL Engineered BaF3 Cell Autophosphorylation ELISA: BaF3 WT BCR-ABL and BaF3-T3151 mutant BCR-ABL cell lines were obtained from Oregon Health and Science University and grown in RPMI-1 640 medium supplemented with 10 % FBS and 1 % penicillin streptomycin (100X stock = 5000 units of penicillin and 5000 μg of streptomycin per mL). For the c-ABL phospho-Tyr ELISA, cells were pipeted into a 50 mL tube, centrifuged at 1000 RPM, and the cell pellet was re-suspended in assay medium (RPMI-1 640 with 0.1 % FBS, 0.05 % BSA wt/vol, and 1 % penicillin streptomycin). The cells were counted using an Innovatis Cedex Cell Counter, seeded into a 96 well flat bottom plate in assay medium at 40,000 cells per well and incubated 2 hours with assay medium at 37 °C, 5 % CO2, 95 % air. Axitinib was dissolved in 100 % DMSO to make 10 mM stocks. Axitinib was then diluted in 100 % DMSO in a polypropylene 96 well plate using 3 fold serial dilution. Control wells contained 100 % DMSO without test compound (uninhibited controls). The DMSO drug dilution plate was diluted 40 fold into assay medium (RPMI-1 640 with 0.1 % FBS, 0.05 % BSA wt/vol, and 1 % penicillin streptomycin) to yield a 5X drug source plate for the assay. Twenty five microliters was transferred from the 5X source plate to the cell assay plate and the assay plate was incubated with axitinib for an additional 2 hours at 37 °C, 5 % CO2, 95 % air. Following this incubation, the cells were centrifuged at 1500 RPM for 5 mins and 80 μl. of the supernatant removed. Cells were lysed by adding 100 μl. per well of freshly prepared Cell Signaling Technology lysis buffer (#9803) supplemented with 1 % SDS, protease inhibitors (Sigma P8340), and phosphatase inhibitors (Sigma P0044 and Sigma P5726). The cell assay plate with lysis buffer was shaken for 10 min at 4 °C and then 100 μl. of cell lysate from each well was transferred to a goat anti-rabbit 96 well ELISA plate (Pierce #15135) which was previously incubated with rabbit anti-c-ABL antibody (Cell Signaling Technology #2862) diluted 1 :200 in blocking buffer (Pierce StartingBlock). The cell lysate was incubated with the anti-c-ABL coated ELISA plate for 1 hour at room temperature and then washed 4 times with Cell Signaling Technology ELISA Wash Buffer (from kit #7903). The final wash was removed by inverting the plate and 100 μl_ of mouse monoclonal (IgG2b) anti-phospho-Tyr antibody (Santa Cruz Biotechnology SC508 HRP) diluted 1 :5000 was added to each well. The ELISA plate was then incubated for 45 min at room temperature with 100 μl_ per well. The plate was washed 4 times as described above, the final wash removed, and 100 μl_ of TMB substrate (Santa Cruz Biotechnology SC286967) was added to each well. The plate was read at 655 nm during color development or stopped by adding 50-100 μl-per well of 0.1 6 M sulfuric acid stop solution and read at 450 nm. As shown in Table 4, the data generated for axitinib demonstrated an 8.6 fold shift in potency for the gatekeeper mutation vs. the wild type enzyme.

Table 4. Axitinib Inhibitory Potencies Toward the Inhibition of T315I Mutation of BCR-ABL Autophosphorylation Determined by the BCR-ABL Engineered BaF3 Cell ELISA

| **Kinase** | **$IC_{50}$(nM)** |
|---|---|
| ABL WT | 177 ± 58(n=2) |
| ABL(T315I) | 20.7 ± 4.6 (n=3) |

Example 5: BCR-ABL Engineered BaF3 Cell Proliferation Assay (Method A): BaF3 WT BCR-ABL and BaF3-T3151 mutant BCR-ABL cell lines were obtained from Oregon Health and Science University and grown in RPMI-1 640 with 10 % FBS as described above. For the proliferation assay, cells were pipeted into a 50 mL tube, centrifuged at

1000 RPM, and the cell pellet was re-suspended in RPMI-1640 with 1 % FBS, and 1 % penicillin streptomycin. The cells were counted using an Innovatis Cedex Cell Counter and seeded into a 96 well flat bottom plate at 1,500 cells per well. Axitinib was serially diluted in 100 % DMSO as described above and then diluted 40 fold into RPMI-1 640 with 1 % FBS and 1 % penicillin streptomycin to yield a 5X source plate. Twenty five microliters was transferred from the 5X source plate to the cell assay plate and the cells incubated with axitinib for 4 days at 37 °C, 5% CO2, 95% air. On Day 4 post drug addition, the cell assay plate was centrifuged at 1000 RPM for 2 mins, 80 μl. of supernatant was removed from each well, and 100 μl. of fresh medium was added to each well. Fifteen μl of 1 mg/mL Resazurin (Sigma R7017) was then added to each well and the cell assay plate was incubated for 6 hour at 37 °C, 5% CO2, 95% air. The fluorescent signal was read using 530 nm excitation and 595 nm emission wavelengths. As shown in Table 5, the data generated for axitinib demonstrated a 10 fold shift in potency for the gatekeeper mutation vs. the wild type enzyme.

Table 5. Axitinib Inhibitory Potencies Toward the T315I Mutation of BCR-ABL Mediated Cell Proliferation Determined by the BCR-ABL Engineered BaF3 Cell Proliferation Assay

| **Kinase** | **$IC_{50}$(nM)** |
|---|---|
| ABL WT | 217 ± 46 (n=2) |
| ABL(T315I) | 21.0 ± 3.6 (n=3) |

Example 6: Wild-type BCR-ABL CML Cell Proliferation Assay: Using the method of Example 5, Table 6 shows the data generated for axitinib wild-type BCR-ABL CML cell lines.

Table 6. Axitinib Inhibitory Potencies Toward Wild-type BCR-ABL Mediated Cell Proliferation Determined by the BCR-ABL CML Cell Proliferation Assay

| **WT BCR-ABL CML cell line** | **$IC_{50}$(nM)** |
|---|---|
| MEG01 (1 % FBS) | 105 |
| MEG01 (10 % FBS) | 314 |
| K562 (10 % FBS) | 323 |

Example 7: BCR-ABL Engineered BaF3 Cell Proliferation Assay (Method B): Using the previously published method (le Coutre P, Mologni L, Cleris L, Marchesi E, Buchdunger E, Giardini R, Formelli F, Gambacorti-Passerini C, "In vivo eradication of human BCR/ABL-positive leukemia cells with an ABL kinase inhibitor." J Natl Cancer Inst. 1999 Jan 20;91 (2):1 63-8.), cells were seeded at a concentration of 104cells/well (10 % FBS) in 96-well round bottom cell culture plates with complete medium and in presence of increasing concentration of inhibitors (range 10 to 0 μM). Cell proliferation was measured at 72 hours using the tritiated thymidine incorporation assay as described previously. The only difference from the previously disclosed method was that the labeling time was 8 hours (64 hours after seeding, the cells were labelled with 3H thyimidine, incubate for 8 hours and harvest). Each test was performed in quadruplicate and repeated at least twice. Calculation of IC50 values was performed using Graphpad Prism software. As shown in Table 7, the data generated for axitinib demonstrated a 8.4 fold shift in potency for the gatekeeper mutation vs. the wild type enzyme.

Table 7. Axitinib Inhibitory Potencies Toward the T315I Mutation of BCR-ABL Determined by the BCR-ABL Engineered BaF3 Cell Proliferation Assay

| **Kinase** | **$IC_{50}$(nM)** |
|---|---|
| ABL WT | 823 |
| ABL(T315I) | 98 |

Very preferred are the claims of WO 2013/068909 which are defined as preferred embodiments hereinafter.

Embodiment 1. A method of treating chronic myelogenous leukemia in a subject comprising administering to the subject a compound that inhibits the T315I mutation in BCR-ABL tyrosine kinase, wherein the compound is N-methyl-2-[3-((E)-2-pyridin-2-yl-vinyl)-1H-indazol-6-ylsulfanyl]-benzamide, or a pharmaceutically acceptable salt thereof.

Embodiment 2. The method of Embodiment 1, wherein the subject is human.

Embodiment 3. A method of treating chronic myelogenous leukemia in a subject having the T315I mutation in BCR-ABL tyrosine kinase, comprising administering to the subject a compound, which is N-methyl-2-[3-((E)-2-pyridin-2-yl-vinyl)-1H-indazol-6-ylsulfanyl]-benzamide, or a pharmaceutically acceptable salt thereof.
Embodiment 4. The method of Embodiment 3, wherein the subject is human.
Embodiment 5. A method of treating chronic myelogenous leukemia in a subject having the T315I mutation in BCR-ABL tyrosine kinase, comprising administering to the subject a compound that inhibits the T315I mutation in BCR-ABL tyrosine kinase, wherein the compound is N-methyl-2-[3-((E)-2-pyridin-2-yl-vinyl)-1H-indazol-6-ylsulfanyl]-benzamide, or a pharmaceutically acceptable salt thereof.
Embodiment 6. The method of Embodiment 5, wherein the subject is human.
Embodiment 7. A pharmaceutical composition for treating chronic myelogenous leukemia in a subject comprising a compound that inhibits the T315I mutation in BCR-ABL tyrosine kinase, wherein the compound is N-methyl-2-[3-((E)-2-pyridin-2-yl-vinyl)-1H-indazol-6-ylsulfanyl]-benzamide, or a pharmaceutically acceptable salt thereof, and a pharmaceutically acceptable carrier or diluent.

SPECIFIC INORMATION CONCERNING ASPECT (VII) OF THE INVENTION

Aspect (vii) of the invention concerns forms of {drug7}, especially a solid form of 4-Methyl-3-[[4-(3-pyridinyl)-2-pyrimidinyl]amino]-N-[5-(4-methyl-1H-imidazol-1-yl)-3-(trifluoromethyl)phenyl] benzamide or pharmaceutical acceptable salt thereof ({drug7a})

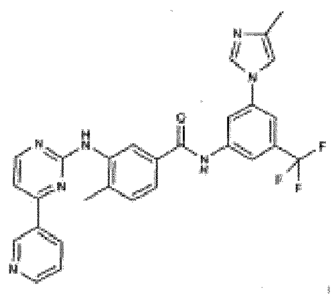

VII.

The invention relates to a solid form of 4-Methyl-3-[[4-(3-pyridinyl)-2-pyrimidinyl]amino]-N-[5-(4-methyl-1H-imidazol-1-yl)-3-(trifluoromethyl)phenyl] benzamide or pharmaceutical acceptable salt thereof (Compound VII, {drug7}), namely to a solid dosage form comprising: (a) a core comprising Compound VII ({drug7}) thereof and excipients; and (b) at least one polymer, said polymer coating said core, wherein disintegration of said solid dosage form is delayed by 4-15 minutes, which is useful for treating chronic myeloid leukemia and gastrointestinal stromal tumors. A solid dosage form comprising: (a) a core comprising Compound VII ({drug7}) thereof and excipients; and (b) at least one polymer, said polymer coating said core, wherein disintegration of said solid dosage form is delayed by 4-15 minutes is described in WO/2013/074432, which is incorporated by reference. 4-Methyl-3-[[4-(3-pyridinyl)-2-pyrimidinyl]amino]-N-[5-(4-methyl-1H-imidazol-1-yl)-3-(trifluoromethyl)phenyl] benzamide or pharmaceutical acceptable salt thereof (Compound VII ({drug7}); pharmaceutical acceptable salts are included in the meaning of formula / Compound VII ({drug7})) is described in WO/2013/074432 as follows:

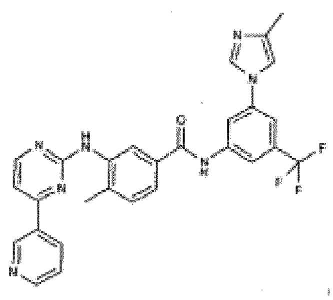

VII.

A solid dosage form comprising: (a) a core comprising Compound VII ({drug7}) thereof and excipients; and (b) at least one polymer, said polymer coating said core, wherein disintegration of said solid dosage form is delayed by 4-15 minutes is preferred as described in the claims of WO/2013/074432 and is defined as preferred embodiments hereinafter:

Embodiment 1. A solid dosage form comprising: (a) a core comprising 4-Methyl-3-[[4-(3-pyridinyl)-2- pyrimidinyl]amino]-N-[5-(4-methyl-1 H-imidazol-1-yl)-3-(trifluoromethyl)phenyl] benzamide or a pharmaceutically acceptable salt thereof and excipients; and (b) at least one polymer, said polymer coating said core, wherein disintegration of said solid dosage form is delayed by 4- 15 minutes.
Embodiment 2. The solid dosage form of Embodiment 1, wherein the polymer is hydroxypropylmethyl cellulose.
Embodiment 3. The solid dosage form of Embodiment 2, wherein 7-13 % of the solid dosage form is the polymer that coats the core.
Embodiment 4. The solid dosage form of Embodiment 1, wherein 0-8 % of the solid dosage form is dissolved after 5 minutes at pH 2.0.
Embodiment 5. The solid dosage form of Embodiment 1, wherein 45-60 % of the solid dosage form is dissolved after 30 minutes at pH 2.0.
Embodiment 6. A solid dosage form comprising: (a) a core comprising 4-Methyl-3-[[4-(3-pyridinyl)-2- pyrimidinyl]amino]-N-[5-(4-methyl-1 H-imidazol-1-yl)-3-(trifluoromethyl)phenyl] benzamide or a pharmaceutically acceptable salt thereof and excipients; and (b) at least one polymer, said polymer coating said core, having a fasted state bioavailabilty equivalent to a hard gelatin capsule, wherein its Gmax and AUC are in the bioequivalent range when compared with capsules comprising 4-Methyl-3-[[4-(3-pyridinyl)-2-pyrimidinyl]amino]-N-[5-(4-methyl-1 H- imidazol-1-yl)-3-(trifluoromethyl)phenyl] benzamide or a pharmaceutically acceptable salt thereof.
Embodiment 7. The solid dosage form of Embodiment 6, wherein the polymer is hydroxypropylmethyl cellulose.
Embodiment 8. The solid dosage form of Embodiment 6, wherein 7-13 % by weight of the solid dosage form is the polymer that coats the core.
Embodiment 9. The solid dosage form of Embodiment 6, wherein 0-8 % by weight of the solid dosage form is dissolved after 5 minutes at pH 2.0.
Embodiment 10. The solid dosage form of Embodiment 6, wherein 45-60 % by weight of the solid dosage form is dissolved after 30 minutes at pH 2.0.
Embodiment 11. A method for preparing a solid dosage form comprising amorphous 4-Methyl-3-[[4-(3- pyridinyl)-2-pyrimidinyl]amino]-N-[5-(4-methyl-1H-imidazol-1-yl)-3-(triftuoromethyl)phenyl] benzamide or a pharmaceutically acceptable salt thereof comprising the steps of: (i) roller compacting a core comprising 4-Methyl-3-[[4-(3-pyridinyl)-2-pyrimidinyl]amino]-N-[5-(4- methyl-1H-imidazol-1-yl)-3-(trifluoromethyl)phenyl] benzamide or a pharmaceutically acceptable salt thereof and excipients; and (ii) coating said core with at least one polymer.
Embodiment 12. The method of Embodiment 11 wherein the solid dosage form is a tablet.
Embodiment 13. The method of Embodiment 12 wherein the polymer is hydroxypropylmethyl cellulose.
Embodiment 14. The method of Embodiment 13 wherein disintegration of said solid dosage form is delayed by 4-15 minutes. 4-Methyl-3-[[4-(3-pyridinyl)-2-pyrimidinyl]amino]-N-[5-(4-methyl-1 H-imidazol-1-yl)-3-(trifluoromethyl)phenyl] benzamide or pharmaceutical acceptable salt thereof (Compound VII ({drug7}) = formula I) and a solid dosage form comprising: (a) a core comprising Compound VII ({drug7}) thereof and excipients; and (b) at least one polymer, said polymer coating said core, wherein disintegration of said solid dosage form is delayed by 4-15 minutes are described in in WO/2013/074432 as cited here: The present invention relates to a pharmaceutical composition comprising a therapeutic compound of nilotinib (Formula II). In particular, the present invention is directed to a pharmaceutical composition that comprises a nilotinib tablet core and that further comprises at least one polymeric coating over the nilotinib core, providing a rapidly disintegrating tablet with a lag time, as compared to an uncoated tablet formulation.

Nilotinib is 4-Methyl-3-[[4-(3-pyridinyl)-2-pyrimidinyl]amino]-N-[5-(4-methyl-1H-imidazol-1-yl)-3-(trifluoromethyl)-phenyl] benzamide. A particularly useful salt of nilotinib is nilotinib hydrochloride monohydrate. These therapeutic compounds have utility as inhibitors of the protein tyrosine kinase (TK) activity of Bcr-Abl. Examples of conditions that may be treated by such therapeutic compounds include, but are not limited to, chronic myeloid leukemia and gastrointestinal stromal tumors. There is a need to formulate nilotinib and the other therapeutic compounds hereinafter disclosed into pharmaceutical compositions, especially solid oral dosage forms, such that the therapeutic benefits of the compounds may be delivered to a patient in need thereof. One problem to providing such compositions including nilotinib is the physiochemical properties of nilotinib, since nilotinib and its salts are poorly water soluble compounds and are difficult to formulate and deliver (i.e., made bioavailable when ingested orally). It is also difficult to achieve matching pharmacokinetic profiles with different dosage forms, i.e. tablets versus capsules. Another problem is a food effect, as food increases the bioavailability of nilotinib. Compared to a fasted state, nilotinib systemic exposure, as reflected by AUC and Cmax, increases markedly when the unit dosage is given shortly after food is ingested, leading to potential adverse

effects in patients.

The present invention provides a solid dosage form comprising: (a) a core comprising 4-Methyl-3-[[4-(3-pyridinyl)-2-pyrimidinyl]amino]-N-[5-(4-methyl-1 H-imidazol-1-yl)-3-(trifluoromethyl)phenyl] benzamide or a pharmaceutically acceptable salt thereof and excipients; and (b) at least one polymer, said polymer coating said core, wherein disintegration of said solid dosage form is delayed by 4-15 minutes. The present invention also provides a solid dosage form comprising: (a) a core comprising 4-Methyl-3-[[4-(3-pyridinyl)-2-pyrimidinyl]amino]-N-[5-(4-methyl-1H-imidazol-1-yl)-3-(trifluoromethyl)-phenyl] benzamide or a pharmaceutically acceptable salt thereof and excipients; and (b) at least one polymer, said polymer coating said core, wherein disintegration of said solid dosage form is delayed by 4-15 minutes, said solid dosage form having a fasted state bioavailability equivalent to a hard-gelatin capsule comprising 4-Methyl-3-[[4-(3-pyridinyl)-2-pyrimidinyl]amino]-N-[5-(4-methyl-1 H-imidazol-1 -yl)-3-(trifluoromethyl)phenyl] benzamide. The present invention also provides a solid dosage form comprising: (a) a core comprising 4-Methyl-3-[[4-(3-pyridinyl)-2-pyrimidinyl]amino]-N-[5-(4-methyl-1 H-imidazol-1-yl)-3-(trifluoromethyl)phenyl] benzamide or a pharmaceutically acceptable salt thereof and excipients; and (b) at least one polymer, said polymer coating said core, wherein disintegration of said solid dosage form is delayed by 4-15 minutes, said solid dosage form having a reduced Cmax as compared to an uncoated solid dosage form comprising 4-Methyl-3-[[4-(3-pyridinyl)-2-pyrimidinyl]amino]-N-[5-(4-methyl-1 H-imidazol-1-yl)-3-(trifluoromethyl)-phenyl] benzamide.

Figure 1 of WO/2013/074432 summarizes dissolution rates for nilotinib tablets (wet granulated and roller compacted) as compared to a nilotinib capsule. Figure 2 of WO/2013/074432 summarizes dissolution rates for film coated nilotinib tablets (7-10 % film coating) at pH 2.0. Figure 3 of WO/2013/074432 summarizes dissolution rates for film coated nilotinib tablets (10-13 % film coating) at pH 2.0. Figure 4 of WO/2013/074432 summarizes dissolution rates for film coated nilotinib tablets prepared by roller compaction (10% film coating) as compared to uncoated nilotinib tablets prepared by wet granulation at pH 2.0. Figure 5 of WO/2013/074432 summarizes a comparison of mean nilotinib concentration versus time profiles for different nilotinib solid dosage forms.

The present invention provides crystalline pharmaceutical compositions of nilotinib or a pharmaceutically acceptable salt thereof formulated in a tablet form to have bioequivalent pharmacokinetic profiles with that of commercially available nilotinib capsule forms. As used herein, nilotinib refers to 4-Methyl-3-[[4-(3-pyridinyl)-2-pyrimidinyl]amino]-N-[5-(4-methyl-1H-imidazol-1-yl)-3-(triftuoromethyl)phenyl] benzamide of formula VII:

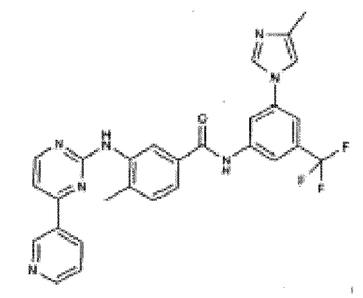

VII.

Nilotinib is a member of compounds of formula (VIII)

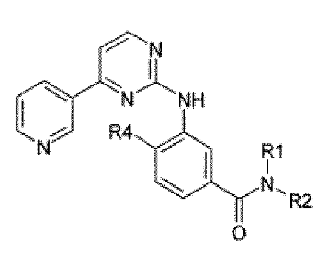

VIII,

wherein

R1 represents hydrogen, lower alkyl, lower alkoxy-lower alkyl, acyloxy-lower alkyl, carboxy-lower alkyl, lower alkoxycarbonyl-lower alkyl, or phenyl-lower alkyl; R2 represents hydrogen, lower alkyl, optionally substituted by one or more identical or different radicals R3, cycloalkyl, benzcycloalkyl, heterocyclyl, an aryl group, or a mono- or bicyclic

heteroaryl group comprising zero, one, two or three ring nitrogen atoms and zero or one oxygen atom and zero or one sulfur atom, which groups in each case are unsubstituted or mono- or polysubstituted; and R3 represents hydroxy, lower alkoxy, acyloxy, carboxy, lower alkoxycarbonyl, carbamoyl, N-mono- or N,N-disubstituted carbamoyl, amino, mono- or disubstituted amino, cycloalkyl, heterocyclyl, an aryl group, or a mono- or bicyclic heteroaryl group comprising zero, one, two or three ring nitrogen atoms and zero or one oxygen atom and zero or one sulfur atom, which groups in each case are unsubstituted or mono- or polysubstituted; or wherein R1 and R2 together represent alkylene with four, five or six carbon atoms optionally mono- or disubstituted by lower alkyl, cycloalkyl, heterocyclyl, phenyl, hydroxy, lower alkoxy, amino, mono- or disubstituted amino, oxo, pyridyl, pyrazinyl or pyrimidinyl; benzalkylene with four or five carbon atoms; oxaalkylene with one oxygen and three or four carbon atoms; or azaalkylene with one nitrogen and three or four carbon atoms wherein nitrogen is unsubstituted or substituted by lower alkyl, phenyl-lower alkyl, lower alkoxycarbonyl-lower alkyl, carboxy-lower alkyl, carbamoyl-lower alkyl, N-mono- or N,N-disubstituted carbamoyl-lower alkyl, cycloalkyl, lower alkoxycarbonyl, carboxy, phenyl, substituted phenyl, pyridinyl, pyrimidinyl, or pyrazinyl; R4 represents hydrogen, lower alkyl, or halogen; and a N-oxide and to the pharmaceutically acceptable salts of such a compound. Such therapeutic compounds are suitable for the preparation of a pharmaceutical composition for the treatment of kinase dependent diseases, especially Bcr-Abl and Tie-2 kinase dependent diseases, for example, as drugs to treat one or more proliferative diseases. Within the definition of "therapeutic compound," the prefix "lower" denotes a radical having up to and including a maximum of seven, especially up to and including a maximum of four carbon atoms, the radicals in question being either linear or branched with single or multiple branching. As used herein, where the plural form is used for compounds, salts, and the like, this is taken to mean also a single compound, salt, or the like. Any asymmetric carbon atoms may be present in the (R)-, (S)-or (Reconfiguration, for example in the (R)- or (S)-configuration. The compounds may thus be present as mixtures of isomers or as pure isomers, for example as enantiomer-pure diastereomers. Also contemplated within the present invention is the use of any possible tautomers of the compounds of formula VII and VIII, respectively. Lower alkyl is for example alkyl with from and including one up to and including seven, for example from and including one to and including four, and is linear or branched; for example, lower alkyl is butyl, such as n-butyl, sec-butyl, isobutyl, tert-butyl, propyl, such as n-propyl or isopropyl, ethyl or methyl. For example lower alkyl is methyl, propyl or tert-butyl. Lower acyl is for example formyl or lower alkylcarbonyl, in particular acetyl. An aryl group is an aromatic radical which is bound to the molecule via a bond located at an aromatic ring carbon atom of the radical. In an exemplary embodiment, aryl is an aromatic radical having six to fourteen carbon atoms, especially phenyl, naphthyl, tetrahydronaphthyl, fluorenyl or phenanthrenyl, and is unsubstituted or substituted by one or more, for example up to three, especially one or two substituents, especially selected from amino, mono- or disubstituted amino, halogen, lower alkyl, substituted lower alkyl, lower alkenyl, lower alkynyl, phenyl, hydroxy, etherified or esterified hydroxy, nitro, cyano, carboxy, esterified carboxy, alkanoyl, benzoyl, carbamoyl, N-mono- or N,N-disubstituted carbamoyl, amidino, guanidino, ureido, mercapto, sulfo, lower alkylthio, phenylthio, phenyl-lower alkylthio, lower alkylphenylthio, lower alkylsulfinyl, phenylsulfinyl, phenyl-lower alkylsulfinyl, lower alkylphenylsulfinyl, lower alkylsulfonyl, phenylsulfonyl, phenyl-lower alkylsulfonyl, lower alkylphenylsulfonyl, halogen-lower alkylmercapto, halogen-lower alkylsulfonyl, such as especially trifluoromethanesulfonyl, dihydroxybora (-B(OH)2), heterocyclyl, a mono-or bicyclic heteroaryl group and lower alkylene dioxy bound at adjacent C-atoms of the ring, such as methylene dioxy. Aryl is for example phenyl, naphthyl or tetrahydronaphthyl, which in each case is either unsubstituted or independently substituted by one or two substituents selected from the group comprising halogen, especially fluorine, chlorine, or bromine; hydroxy; hydroxy etherified by lower alkyl, e.g. by methyl, by halogen-lower alkyl, e.g. trifluoromethyl, or by phenyl; lower alkylene dioxy bound to two adjacent C-atoms, e.g. methylenedioxy, lower alkyl, e.g. methyl or propyl; halogen-lower alkyl, e.g. trifluoromethyl; hydroxy-lower alkyl, e.g. hydroxymethyl or 2-hydroxy-2-propyl; lower alkoxy-lower alkyl; e.g. methoxymethyl or 2-methoxyethyl; lower alkoxycarbonyl-lower alkyl, e.g. methoxycarbonylmethyl; lower alkynyl, such as 1-propynyl; esterified carboxy, especially lower alkoxycarbonyl, e.g. methoxycarbonyl, n-propoxy carbonyl or iso-propoxy carbonyl; N-mono-substituted carbamoyl, in particular carbamoyl monosubstituted by lower alkyl, e.g. methyl, n-propyl or iso-propyl; amino; lower alkylamino, e.g. methylamino; di-lower alkylamino, e.g. dimethylamino or diethylamino; lower alkylene-amino, e.g. pyrrolidino or piperidino; lower oxaalkylene-amino, e.g. morpholino, lower azaalkylene-amino, e.g. piperazino, acylamino, e.g. acetylamino or benzoylamino; lower alkylsulfonyl, e.g. methylsulfonyl; sulfamoyl; or phenylsulfonyl. A cycloalkyl group is for example cyclopropyl, cyclopentyl, cyclohexyl or cycloheptyl, and may be unsubstituted or substituted by one or more, especially one or two, substitutents selected from the group defined above as substituents for aryl, e.g., by lower alkyl, such as methyl, lower alkoxy, such as methoxy or ethoxy, or hydroxy, and further by oxo or fused to a benzo ring, such as in benzcyclopentyl or benzcyclohexyl. Substituted alkyl is alkyl as last defined, especially lower alkyl, for example methyl; where one or more, especially up to three, substituents may be present, primarily from the group selected from halogen, especially fluorine, amino, N-lower alkylamino, N,N-di-lower alkylamino, N-lower alkanoylamino, hydroxy, cyano, carboxy, lower alkoxycarbonyl, and phenyl-lower alkoxycarbonyl. Trifluoromethyl is especially useful. Mono- or disubstituted amino is especially amino substituted by one or two radicals selected independently of one another from lower alkyl,

such as methyl; hydroxy-lower alkyl, such as 2-hydroxyethyl; lower alkoxy lower alkyl, such as methoxy ethyl; phenyl-lower alkyl, such as benzyl or 2-phenylethyl; lower alkanoyl, such as acetyl; benzoyl; substituted benzoyl, wherein the phenyl radical is especially substituted by one or more, for example one or two, substituents selected from nitro, amino, halogen, N-lower alkylamino, N,N-di-lower alkylamino, hydroxy, cyano, carboxy, lower alkoxycarbonyl, lower alkanoyl, and carbamoyl; and phenyl-lower alkoxycarbonyl, wherein the phenyl radical is unsubstituted or especially substituted by one or more, for example one or two, substituents selected from nitro, amino, halogen, N-lower alkylamino, N,N-di-lower alkylamino, hydroxy, cyano, carboxy, lower alkoxycarbonyl, lower alkanoyl, and carbamoyl; and is for example N-lower alkylamino, such as N-methylamino, hydroxy-lower alkylamino, such as 2-hydroxyethylamino or 2-hydroxypropyl, lower alkoxy lower alkyl, such as methoxy ethyl, phenyl-lower alkylamino, such as benzylamino, N,N-di-lower alkylamino, N-phenyl-lower alkyl-N-lower alkylamino, N,N-di-lower alkylphenylamino, lower alkanoylamino, such as acetylamino, or a substituent selected from the group comprising benzoylamino and phenyl-lower alkoxycarbonylamino, wherein the phenyl radical in each case is unsubstituted or especially substituted by nitro or amino, or also by halogen, amino, N-lower alkylamino, N,N-di-lower alkylamino, hydroxy, cyano, carboxy, lower alkoxycarbonyl, lower alkanoyl, carbamoyl or aminocarbonylamino. Disubstituted amino is also lower alkylene-amino, e.g. pyrrolidino, 2-oxopyrrolidino or piperidino; lower oxaalkylene-amino, e.g. morpholino, or lower azaalkylene-amino, e.g. piperazino or N-substituted piperazino, such as N-methylpiperazino or N-methoxycarbonylpiperazino. Halogen is especially fluorine, chlorine, bromine, or iodine, especially fluorine, chlorine, or bromine. Etherified hydroxy is especially C8-C20alkyloxy, such as n-decyloxy, lower alkoxy, such as methoxy, ethoxy, isopropyloxy, or tert-butyloxy, phenyl-lower alkoxy, such as benzyloxy, phenyloxy, halogen-lower alkoxy, such as trifluoromethoxy, 2,2,2-trifluoroethoxy or 1,1,2,2-tetrafluoroethoxy, or lower alkoxy which is substituted by mono- or bicyclic heteroaryl comprising one or two nitrogen atoms, for example lower alkoxy which is substituted by imidazolyl, such as 1H-imidazol-1-yl, pyrrolyl, benzimidazolyl, such as 1-benzimidazolyl, pyridyl, especially 2-, 3- or 4-pyridyl, pyrimidinyl, especially 2-pyrimidinyl, pyrazinyl, isoquinolinyl, especially 3-isoquinolinyl, quinolinyl, indolyl or thiazolyl. Esterified hydroxy is especially lower alkanoyloxy, benzoyloxy, lower alkoxycarbonyloxy, such as tert-butoxycarbonyloxy, or phenyl-lower alkoxycarbonyloxy, such as benzyloxycarbonyloxy. Esterified carboxy is especially lower alkoxycarbonyl, such as tert-butoxycarbonyl, iso-propoxycarbonyl, methoxycarbonyl or ethoxycarbonyl, phenyl-lower alkoxycarbonyl, or phenyloxycarbonyl. Alkanoyl is primarily alkylcarbonyl, especially lower alkanoyl, e.g. acetyl. N-Mono- or N,N-disubstituted carbamoyl is especially substituted by one or two substituents independently selected from lower alkyl, phenyl-lower alkyl and hydroxy-lower alkyl, or lower alkylene, oxa-lower alkylene or aza-lower alkylene optionally substituted at the terminal nitrogen atom. A mono- or bicyclic heteroaryl group comprising zero, one, two or three ring nitrogen atoms and zero or one oxygen atom and zero or one sulfur atom, which groups in each case are unsubstituted or mono- or polysubstituted, refers to a heterocyclic moiety that is unsaturated in the ring binding the heteroaryl radical to the rest of the molecule in formula I and is for example a ring, where in the binding ring, but optionally also in any annealed ring, at least one carbon atom is replaced by a heteroatom selected from the group consisting of nitrogen, oxygen and sulfur; where the binding ring for example has five to twelve, e.g., five or six ring atoms; and which may be unsubstituted or substituted by one or more, especially one or two, substitutents selected from the group defined above as substitutents for aryl, most for example by lower alkyl, such as methyl, lower alkoxy, such as methoxy or ethoxy, or hydroxy. For example the mono-or bicyclic heteroaryl group is selected from 2H-pyrrolyl, pyrrolyl, imidazolyl, benzimidazolyl, pyrazolyl, indazolyl, purinyl, pyridyl, pyrazinyl, pyrimidinyl, pyridazinyl, 4H-quinolizinyl, isoquinolyl, quinolyl, phthalazinyl, naphthyridinyl, quinoxalyl, quinazolinyl, quinnolinyl, pteridinyl, indolizinyl, 3H-indolyl, indolyl, isoindolyl, oxazolyl, isoxazolyl, thiazolyl, isothiazolyl, triazolyl, tetrazolyl, furazanyl, benzo[d]pyrazolyl, thienyl and furanyl. For example the mono- or bicyclic heteroaryl group is selected from the group consisting of pyrrolyl, imidazolyl, such as 1 H-imidazol-1-yl, benzimidazolyl, such as 1 -benzimidazolyl, indazolyl, especially 5-indazolyl, pyridyl, especially 2-, 3- or 4-pyridyl, pyrimidinyl, especially 2-pyrimidinyl, pyrazinyl, isoquinolinyl, especially 3-isoquinolinyl, quinolinyl, especially 4-or 8-quinolinyl, indolyl, especially 3-indolyl, thiazolyl, benzo[d]pyrazolyl, thienyl, and furanyl. In one exemplary embodiment of the invention the pyridyl radical is substituted by hydroxy in ortho position to the nitrogen atom and hence exists at least partially in the form of the corresponding tautomer which is pyridin-(1H)2-one. In another exemplary embodiment, the pyrimidinyl radical is substituted by hydroxy both in position 2 and 4 and hence exists in several tautomeric forms, e.g. as pyrimidine-(1H, 3H)2,4-dione. Heterocyclyl is especially a five, six or seven-membered heterocyclic system with one or two heteroatoms selected from the group comprising nitrogen, oxygen, and sulfur, which may be unsaturated or wholly or partly saturated, and is unsubstituted or substituted especially by lower alkyl, such as methyl, phenyl-lower alkyl, such as benzyl, oxo, or heteroaryl, such as 2-piperazinyl; heterocyclyl is especially 2- or 3-pyrrolidinyl, 2-oxo-5-pyrrolidinyl, piperidinyl, N-benzyl-4-piperidinyl, N-lower alkyl-4-piperidinyl, N-lower alkyl-piperazinyl, morpholinyl, e.g. 2- or 3-morpholinyl, 2-oxo-1H-azepin-3-yl, 2-tetrahydrofuranyl, or 2-methyl-1,3-dioxolan-2-yl. Salts are especially the pharmaceutically acceptable salts of compounds of formula I. Such salts are formed, for example, as acid addition salts, for example with organic or inorganic acids, from compounds of formula I with a basic nitrogen atom, especially the pharmaceutically acceptable salts. Suitable inorganic acids include, but are not limited to, halogen acids, such as hydrochloric

acid, sulfuric acid, or phosphoric acid. Suitable organic acids are, for example, carboxylic, phosphonic, sulfonic or sulfamic acids, for example acetic acid, propionic acid, octanoic acid, decanoic acid, dodecanoic acid, glycolic acid, lactic acid, fumaric acid, succinic acid, adipic acid, pimelic acid, suberic acid, azelaic acid, malic acid, tartaric acid, citric acid, amino acids, such as glutamic acid or aspartic acid, maleic acid, hydroxymaleic acid, methylmaleic acid, cyclohexanecarboxylic acid, adamantanecarboxylic acid, benzoic acid, salicylic acid, 4-aminosalicylic acid, phthalic acid, phenylacetic acid, mandelic acid, cinnamic acid, methane- or ethane-sulfonic acid, 2-hydroxyethanesulfonic acid, ethane-1,2-disulfonic acid, benzenesulfonic acid, 2-naphthalenesulfonic acid, 1,5-naphthalene-disulfonic acid, 2-, 3- or 4-methylbenzenesulfonic acid, methylsulfuric acid, ethylsulfuric acid, dodecylsulfuric acid, N-cyclohexylsulfamic acid, N-methyl-, N-ethyl- or N-propyl-sulfamic acid, or other organic protonic acids, such as ascorbic acid. One useful salt of nilotinib is nilotinib hydrochloride monohydrate, or 4-Methyl-N-[3-(4-methyl-1H-imidazol-1-yl)-5-(trifiuromethyl)phenyl]-3-[(4-pyridine-3-ylpyrimidin-2-yl)amino]benzamide hydrochloride hydrate. Suitable salts of nilotinib and polymorphs thereof are disclosed in more general in WO2007/015870 and WO2007/015871. As used herein the term "pharmaceutical composition" means, for example, a mixture containing a specified amount of a therapeutic compound, e.g. a therapeutically effective amount, of a therapeutic compound in a pharmaceutically acceptable carrier to be administered to a mammal, e.g., a human in order to treat kinase dependent diseases. As used herein the term "pharmaceutically acceptable" refers to those compounds, materials, compositions and/or dosage forms, which are, within the scope of sound medical judgment, suitable for contact with the tissues of mammals, especially humans, without excessive toxicity, irritation, allergic response and other problem complications commensurate with a reasonable benefit/risk ratio. The concentration of therapeutic compound in the pharmaceutical composition is present in an amount, e.g. in a therapeutically effective amount, which will depend on absorption, inactivation and excretion rates of the drug as well as other factors known to one of ordinary skill in the art. Furthermore, it is to be noted that dosage values will also vary with the severity of the condition to be alleviated. It is to be further understood that for any particular recipient, specific dosage regimens should be adjusted over time according to the individual need and the professional judgment of the person administering or supervising the administration of the pharmaceutical compositions. The therapeutic compound may be administered once, or may be divided into a number of smaller doses to be administered at varying intervals of time. Thus, an appropriate amount, e.g. an appropriate therapeutically effective amount, is known to one of ordinary skill in the art. For example, the dose of the therapeutic compound will be in the range from about 0.1 to about 100 mg per kilogram body weight of the recipient per day. Alternatively lower doses may be given, for example doses of 0.5 to 100 mg; 0.5 to 50 mg; or 0.5 to 20 mg per kilogram body weight per day. The effective dosage range of the pharmaceutically acceptable salts may be calculated based on the weight of the active moiety to be delivered. If the salt exhibits activity itself, the effective dosage may be estimated as above using the weight of the salt, or by other means known to those skilled in the art. As used herein the term "immediate-release" refers to the rapid release of the majority of the therapeutic compound, e.g., greater than about 50%, about 55%, about 60%, about 65%, about 70%, about 75%, about 80%, or about 90% within a relatively short time, e.g., within 1 hour, 40 minutes, 30 minutes or 20 minutes after oral ingestion. Particularly useful conditions for immediate-release are release of at least or equal to about 80% of the therapeutic compound within thirty minutes after oral ingestion. The particular immediate-release conditions for a specific therapeutic compound will be recognized or known by one of ordinary skill in the art. As used herein the term "lag time" refers to period of time the majority of the therapeutic compound is delayed from being released after oral ingestion. As used herein the term "excipient" refers to a pharmaceutically acceptable ingredient that is commonly used in the pharmaceutical technology for preparing granule and/or solid oral dosage formulations. Examples of categories of excipients include, but are not limited to, binders, disintegrants, lubricants, glidants, stabilizers, fillers and diluents. One of ordinary skill in the art may select one or more of the aforementioned excipients with respect to the particular desired properties of the granule and/or solid oral dosage form by routine experimentation and without any undue burden. The amount of each excipient used may vary within ranges conventional in the art. The following references which are all hereby incorporated by reference disclose techniques and excipients used to formulate oral dosage forms. See The Handbook of Pharmaceutical Excipients, 4th edition, Rowe et al., Eds., American Pharmaceuticals Association (2003); and Remington: the Science and Practice of Pharmacy, 20th edition, Gennaro, Ed., Lippincott Williams & Wilkins (2000). In an exemplary embodiment of the present invention, the invented solid dosage forms of nilotinib are prepared by roller compacting nilotinib tablet cores and film-coating the nilotinib tablet cores with a functional polymer, wherein disintegration of said solid dosage form is delayed by 4-15 minutes. The present invention also provides a method of increasing bioavailability by administering the composition or the pharmaceutical composition of the invention, respectively, to an animal or to a patient, wherein the increased bioavailability is determined by comparing the Cmax value or the AUC value of the composition or the pharmaceutical composition of the invention with the composition disclosed in the present invention. Preferably the method increases bioavailability of a drug in administered animal or patient by least 1.3 fold, preferably at least two fold, even more preferably by at least three fold. In one preferred embodiment of the method, the composition or the pharmaceutical composition of the invention, respectively, comprises 4-methyl-3-[[4-(3-pyridinyl)-2-pyrimidinyl]amino]-N-[5-(4-methyl-1H-imidazol-1-yl)-3-(triftuoromethyl)phenyl] benzamide and has comparable bioavailability when compared with 4-methyl-3-[[4-(3-pyridinyl)-2-pyrimidinyl]amino]-Λ-[5-(4-methyl-1 H-imidazol-1-yl)-3-(trifluoromethyl)phenyl] benzamide in the marketed, commercially available Tasigna™ hard-gelatin capsule manufactured by Novartis. Comparable is defined as 90% CI of Cmax and AUC within the range of 0.8 and 1.25 when expressed as ratio between the tested (invention formulation) and reference (Tasigna™ capsule formulation) for Cmax and AUC. Bioavailability can be measured by skilled artisan by conventional methods. For example, tablets, capsules, liquids, powders, etc., are given orally to humans or animals and blood levels are measured. The composition or the pharmaceutical composition according to the invention may also comprise one or more binding agents, filling agents, lubricating agents, suspending agents, sweeteners, flavoring agents, preservatives, buffers, wetting agents, effervescent agents and other excipients. Such excipients are known in the art. Examples of filling agents are lactose monohydrate, lactose anhydrous, microcrystalline cellulose, such as Avicel® PH101 and Avicel® PH102, microcrystalline cellulose and silicified microcrystalline cellulose (ProSolv SMCC®), and various starches; examples of binding agents are various celluloses and cross-linked polyvinylpyrrolidone. Suitable lubricants, including agents that act on the flowability of the powder to be compressed, are colloidal silicon dioxide, such as Aerosil® 200, talc, stearic acid, magnesium stearate, calcium stearate and silica gel. Examples of sweeteners are any natural or artificial sweetener, such as sucrose, xylitol, sodium saccharin, cyclamate, aspartame, sucralose, maltitol and acsulfame. Examples of flavoring agents are Magnasweet® (trademark of MAFCO), bubble gum flavor, and fruit flavors, and the like. Suitable diluents include pharmaceutically acceptable inert fillers, such as microcrystalline cellulose, lactose, dibasic calcium phosphate, saccharides and/or mixtures of any of the foregoing. Examples of diluents include microcrystalline cellulose, such as Avicel® PH101 and Avicel® PH1 02; lactose, such as lactose monohydrate, lactose anhydrous, and Pharmatose® DCL21; dibasic calcium phosphate, such as Emcompress; mannitol; starch; sorbitol; sucrose; and glucose. Examples of effervescent agents are effervescent couples, such as an organic acid and a carbonate or bicarbonate. Niliotinib exhibits compressibility problem, coupled with high drug loading > 45%, the formulation is also prone to sticking and picking on punches thus requiring high Mg stearate level. The formulation also has friability issues if a suitable binder is not present. To overcome all these challenges, the formulation needs the selected excipients in their optimized amounts. In one embodiment, the invented core tablets comprise nilotinib in amounts from 30-70 % by weight based on the weight of the tablet, Avicel® PH102 (microcrystalline cellulose) as a filler in the range 20-60 % by weight, HPC EXF as a binder in the range of 2- 6 % by weight, crospovidone as a super disintegrant in the range of 2-14 % by weight, Aerosil as a glidant or flow enhancer with the range of 0.25 to 4 % by weight, Magnesium stearate as an intra-granular(I) component in the range of 0.25-2 % by weight and Magnesium stearate as an extra-granular (II) componernt in the range of 0.7-3.5 % by weight based on the weight of the tablet. In one embodiment, the composition is in an oral solid dosage form. The oral solid dosage form includes tablets, pills, capsules, powders. The oral liquid dosage form includes solutions and suspensions. In one embodiment, the solid dosage form is a polymeric film coated tablet. Different classes of polymers that may be used to delay the initial release from the tablet are selected from: hydroxypropyl cellulose, hydroxy propyl methyl cellulose, hydroxy propyl ethyl cellulose, ethyl cellulose, shellac, polyvinyl pyrrolidone (e.g 30, K90), polyvinyl acetate, Kollidon VA 64 {Copovidone or (Polyvinyl acetate 40% and polyvinyl pyrrolidone 60%}, Kollidon SR (Polyvinyl acetate 80% and polyvinyl pyrrolidone20%), methacrylic acid (polymers and graft co polymers), carbomer polymers (e.g Carbopol 971 P NF, Carbopol 974P NF), veegum, glyceryl behenate / di behenate (Compritol®, hydroxy propyl methyl cellulose acetate succinate (HPMC AS) and hydroxy propyl methyl cellulose phthallate (HPMC-P).

In one aspect, the present invention provides a process of making the composition comprising the steps of blending nilotinib and excipients and roller compacting them to form granules. The granules are compressed into tablets or pills. The nilotinib tablet cores are then film coated to various thicknesses with a polymer coating, providing a lag time before disintegration.

The following examples are given to illustrate the present invention. It should be understood, however, that the invention is not to be limited to the specific conditions or details described in the examples below. The following examples are illustrative, but do not serve to limit the scope of the invention described herein. The examples are meant only to suggest a method of practicing the present invention. Quantities of ingredients, represented by percentage by weight of the pharmaceutical composition, used in each example are set forth in the respective tables located after the respective descriptions. For a capsule, when calculating the weight of the pharmaceutical composition (i.e. the capsule fill weight), the weight of the capsule shell itself is excluded from the calculation.

Example 1 Nilotinib Tablet Core: One example of a nilotinib tablet core (Formulation A) is summarized in Table 1. The nilotinib tablet cores were prepared by roller compaction. Compared to a commercially available nilotinib capsule formulation developed using a wet granulation technique, the invented nilotinib tablet cores prepared by roller compaction consistently provides nilotinib tablet cores exhibiting excellent compression characteristics, including but not limited to a 6-10 kp compression window, tablet cores having low friability, fast disintegration times (1-2 minutes) and tablet cores that can be compressed at a high speeds.

Table 1. Nilotinib Tablet Core (Formulation A)

| Component | % | 200 mg | 300mg | 400mg |
|---|---|---|---|---|
| | | mg/ unit | mg/ unit | mg /unit |
| AMN107 (Nilotinib Hydrochloride) | 47.96 | 220.6 | 330.9 | 441.2 |
| Microcrystalline cellulose | 39.04 | 179.6 | 269.4 | 359.2 |
| Hydroxypropylcellulose (HPC EXF) | 3.04 | 14.0 | 21.0 | 28.0 |
| Crospovidone | 6.09 | 28.0 | 42.0 | 56.0 |
| Aerosil 200 PH | 1.00 | 4.6 | 6.9 | 9.2 |
| Magnesium stearate (I) | 0.87 | 4.0 | 6.0 | 8.0 |
| Magnesium stearate (II) | 2.00 | 9.2 | 13.8 | 18.4 |
| Core tablet weight | 100.00 | 460.0 | 690.0 | 920.0 |

Unit dosages of 50mg and 100mg were also manufactured from nilotinib tablet core formulation A. The unit dosages were prepared in proportion to the 200mg, 300mg and 400mg unit doses.

Example 2 Nilotinib Tablet Core: Another example of a nilotinib tablet core (Formulation B) is summarized in Table 2. The nilotinib tablet cores were prepared by roller compaction. Compared to a commercially available nilotinib capsule formulation developed using a wet granulation technique, the invented nilotinib tablet cores prepared by roller compaction consistently provides nilotinib tablet cores exhibiting excellent compression characteristics, including but not limited to a6-10 kp compression window, tablet cores having low friability, fast disintegration times (1-2 minutes) and tablet cores that can be compressed at a high speeds.

Table 2. Nilotinib Tablet Core (Formulation B)

| Component | % | 200 mg | 300mg | 400mg |
|---|---|---|---|---|
| | | mg/ unit | mg/ unit | mg/ unit |
| AMN107 (Nilotinib Hydrochloride) | 47.96 | 220.6 | 330.9 | 441.2 |
| Microcrystalline cellulose | 35.13 | 161.6 | 242.4 | 323.2 |
| Hydroxypropylcellulose | 3.04 | 14.0 | 21.0 | 28.0 |
| Crospovidone | 10.00 | 46.0 | 69.0 | 92.0 |
| Aerosil 200 PH | 1.00 | 4.6 | 6.9 | 9.2 |
| Magnesium stearate (I) | 0.87 | 4.0 | 6.0 | 8.0 |
| Magnesium stearate (II) | 2.00 | 9.2 | 13.8 | 18.4 |
| Core tablet weight | 100.00 | 460.0 | 690.0 | 920.0 |

Unit dosages of 50mg and 100mg were also manufactured from nilotinib tablet core formulation A. The unit dosages were prepared in proportion to the 200mg, 300mg and 400mg unit doses. Manufacturing process: Nilotinib was mixed with Aerosil 200 PH, HPC EXF, and Crospovidone. Microcrystalline cellulose was added and the mixture was blended. The blended mixture was then sieved through a #16 to #35 screen. Magnesium stearate (I) was added to the sieved mixture and was again blended to distribute Magnesium stearate. This mixture was roller compacted using a compaction force of 15-40 kN on a 50mm roller compactor. The ribbons were then milled through a sieve (range 10-18 US mesh size). Milled granules were blended with magnesium stearate (II) to distribute magnesium stearate.

Dissolution: Two step dissolution conditions were used for the following nilotinib table cores (formulation A and B), wet granulated nilotinib formulation capsule, and nilotinib capsule formulation: 37°C; Step 1, 0-60 minutes 500ml pH 2 buffer, Step 2, > 60 minutes 1000ml pH 6.8 buffer; Paddle at 75rpm. The invented nilotinib tablet cores prepared from roller compacted nilotinib formulations A and B exhibit fast disintegration times (< 2min), irrespective of the compression force and hardness of the tablet, as compared to the commercially available nilotinib capsule formulation (Figure 1 of WO/2013/074432). For the invented nilotinib tablet cores to be bio-equivalent with the commercial nilotinib capsule formulation, a dissolution lag time was required to delay the disintegration time of the nilotinib tablet cores. This lag time

(4-12 minutes) is achieved using a functional polymer based coating over core tablets, preventing the tablets from disintegrating before the lag time.

Film Coated Nilotinib Tablet Cores: The composition of film coated nilotinib tablets is summarized in Table 3. Film coated nilotinib tablet cores were prepared from nilotinib formulations A and B.

Table 3. Composition of film coated nilotinib tablets (Formulations A and B)

| Composition | RC Formulation A | | RC Formulation B | |
|---|---|---|---|---|
| | % | (mg) | % | (mg) |
| AMN107 HCl | 43.60 | 330.90 | 43.57 | 330.90 |
| Avicel PH102 | 35.61 | 273.30 | 31.95 | 242.70 |
| HPC EXF | 2.73 | 20.70 | 2.73 | 20.70 |
| Crospovidone | 5.45 | 41.40 | 9.08 | 69.00 |
| Aerosil 200 | 0.91 | 6.90 | 0.91 | 6.90 |
| Mg stearate | 2.61 | 19.80 | 2.61 | 19.80 |
| PEG 4000 | | | 0.48 | 3.68 |
| HPMCE50 | 1.52 | 11.50 | 3.23 | 2450 |
| Opadry White | 7.32 | 56.57 | 5.20 | 39.46 |
| Opadry Yellow | 0.24 | 1.84 | 0.17 | 1.31 |
| Opadry Red | 0.01 | 0.09 | 0.08 | 0.60 |
| **Total** | **100.00** | **759.00** | **100.00** | **759.55** |

Film coating thickness can be varied based upon weight gain of nilotinib tablet cores. An increased disintegration time is observed with corresponding increase in the weight gain of film coating. The opadry white, yellow and red impart a pale yellow color to the tablets and are only present for aesthetic value, whereas HPMC E50 is the functional polymer that delays the disintegration time. The functional coating provides a unique dissolution profile with the following characterstics:

1) For 7% functional coating weight gain the following dissolution profile in 900ml pH 2.0 is observed

- 0 - 8% dissolved at 5 minutes
- 20 - 30% dissolved at 10 minutes
- 35 - 45% dissolved at 15 minutes
- 45 - 60% dissolved in 30 minutes

2) For 10% functional coating weight gain the following dissolution profile in 900ml pH 2.0 is observed

- 0 - 5% dissolved at 5 minutes
- 10 - 25% dissolved at 10 minutes
- 25 - 45% dissolved at 15 minutes
- 45 - 55% dissolved in 30 minutes

3) For 13% functional coating weight gain the following dissolution profile in 900ml pH 2.0 is observed

- 0 % dissolved at 5 minutes
- 2 - 10% dissolved at 10 minutes
- 20 - 35% dissolved at 15 minutes
- 45 - 55% dissolved in 30 minutes

Table 4: Dissolution rofiles of different weight % functional coatin wei ht ain at pH 2.0

| Batches | Average % released at time (min) | | | | | | | | Paddle |
|---|---|---|---|---|---|---|---|---|---|
| | 0 | 5 | 10 | 16 | 20 | 30 | 45 | 60 | (rpm) |
| TRD-2704-084_10% RC1 | 0 | 0 | 13 | 29 | 38 | 49 | 60 | 66 | 50 |

(continued)

| Batches | Average % released at time (min) | | | | | | | | Paddle (rpm) |
|---|---|---|---|---|---|---|---|---|---|
| | 0 | 5 | 10 | 16 | 20 | 30 | 45 | 60 | |
| TRD-2704-084_13% RC1 | 0 | 0 | 4 | 27 | 38 | 50 | 60 | 66 | 50 |
| TRD-2704-082_10% RC2 | 0 | 0 | 21 | 36 | 45 | 56 | 65 | 70 | 50 |
| TRD-2704-082_13% RC2 | 0 | 0 | 8 | 30 | 41 | 54 | 64 | 70 | 50 |
| AEUS2010-0116 10% (n=12) RC1 | 0 | 2 | 21 | 32 | 41 | 51 | 61 | 67 | 50 |
| AEUS2010-0117 10% (n=12) RC2 | 0 | 2 | 20 | 35 | 45 | 55 | 64 | 70 | 50 |
| TRD-2704-040_7% RC1 | 0 | 2 | 24 | 36 | 43 | 53 | 62 | 66 | 50 |
| TRD-2104-042_7 RC2 | 0 | 2 | 27 | 40 | 46 | 56 | 64 | 70 | 50 |

Human PK results: In the first study the tablet formulation without any functional coating were tested in humans. The results are as given below:

## 2101 Pilot PK Data Summary

| | 200 mg | | | |
|---|---|---|---|---|
| | RC1 | RC2 | WG | Ref |
| AUC inf | 8560 | 9606 | 9669 | 8650 |
| Cmax | 387.6 | 418.7 | 375.9 | 340.3 |
| Cmax ratio | 1.139 | 1.231 | 1.106 | |
| 90% CI | (0.981-1.323) | (1.056-1.435) | (0.951-1.283) | |
| AUC ratio | 0.989 | 1.110 | 1.115 | |
| 90% CI | (0.877-1.116) | (0.982-1.254) | (0.989-1.258) | |

| | 300 mg | | | | 400 mg | | | |
|---|---|---|---|---|---|---|---|---|
| | RC1 | RC2 | WG | Ref | RC1 | RC2 | WG | Ref |
| AUC inf | 12874 | 12099 | 14377 | 11394 | 11974 | 12565 | 13294 | 11049 |
| Cmax | 514.5 | 472.3 | 493.2 | 396.3 | 482.0 | 543.7 | 467.7 | 373.1 |
| Cmax ratio | 1.298 | 1.192 | 1.244 | | 1.274 | 1.433 | 1.247 | |
| 90% CI | (1.127-1.495) | (1.030-1.378) | (1.081-1.433) | | (1.103-1.471) | (1.243-1.652) | (1.075-1.445) | |
| AUC ratio | 1.130 | 1.051 | 1.262 | | 1.116 | 1.161 | 1.216 | |
| 90% CI | (1.016-1.256) | (0.942-1.172) | (1.135-1.1403) | | (0.981-1.270) | (1.020-1.322) | (1.063-1.391) | |

May pass in BE with relaxed criteria [0.7-1.43] & 176 patients

Var A = WG
Var B = RC-1
Var C = RC-2

It can be seen above that none of the formulations was bioequivalent to the reference marketed capsule formulation, whereas the all dosage forms exhibited a higher Cmax as compared with the reference marketed capsule formulation, whereas the ratio of Cmax is disproportionally higher than the ratio of AUC. In another study, 300mg RC variants with 10% film coating were tested for BE and the results are as shown below. PK results of the variants BB (RC1 with 10% film coating) and Variant CC (RC2 with 10% film coating). Results of AMN107C2104: summary of PK parameters - geometric mean ratio for all formulations within BE range (80-125%):

Results of AMN107C2104: summary of PK parameters – geometric mean ratio for all formulations within BE range (80-125%)

| Parameter | A (N =19) | B (N=19) | C (N = 18) | D (N = 18) | Geometric Mean Ratio (90% CIs) B vs. A | C vs. A | D vs. A |
|---|---|---|---|---|---|---|---|
| $C_{max}$ (ng/mL) | 449.27 (29.8) | 479.97 (25.4) | 453.18 (33.6) | 478.66 (37.4) | [illegible] | 1.01 (0.87, 1.17) | 1.07 (0.92, 1.23) |
| $AUC_{0-tlast}$ (ng•h/mL) | 10948.36 (32.2) | 10193.73 (27.2) | 10026.16 (33.5) | 9898.25 (37.3) | [illegible] | 0.92 (0.81, 1.04) | 0.90 (0.80, 1.02) |
| $AUC_{0-inf}$ (ng•h/mL) | 12151.57 (38.6) | 10632.62 (29.1) | 11303.04 (36.8) | 10784.07 (42.7) | [illegible] | 0.93 (0.81, 1.06) | 0.89 (0.78, 1.02) |
| $t_{max}$ (h) | 4.00(2.05, 10.00) | 4.00 (2.00, 10.02) | 4.00 (2.00, 10.00) | 4.00 (2.00; 10.00) | [illegible] | 0.00 (-3.00, 1.98) | 0.00 (-2.00, 1.97) |

**A**: FMI capsule; **B**: WG tablet; **C**: RC-1 tablet; **D**: RC-2 tablet

A: FMI capsule; B: WG tablet; C: RC-1 tablet; D: RC-2 tablet

Bioequivalence was demonstrated for RC1 and RC2 variants with functional film coating for 300mg strength.

The invention relates to an administration unit comprising a solid dosage form comprising: (a) a core comprising Compound VII ({drug7}) thereof and excipients; and (b) at least one polymer, said polymer coating said core, wherein disintegration of said solid dosage form is delayed by 4-15 minutes. The administration unit according to the invention is useful for treating various diseases and disorders which include but are not limited to chronic myeloid leukemia and gastrointestinal stromal tumors.

PREFERRED EMBODIMENTS CONCERNING ASPECTS (I), (II), (III), (IV), (V), (VI), (VII), (VIII), (IX), AND (X) OF THE INVENTION

The invention relates to an administration unit comprising (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (via) {drug6a}; or (vii) {drug7a}.

Preferably, the administration unit according to the invention comprises a therapeutically effective amount of(i) {drug1}, (ii) {drug2}, (iii) {drug3}, (iv) {drug4}, (v) {drug5}, (vi) {drug6}, or (vii) {drug7}, wherein at least 10 % by weight of said therapeutically effective amount of (i) {drug1}, (ii) {drug2}, (iii) {drug3}, (iv) {drug4}, (v) {drug5}, (vi) {drug6}, or (vii) {drug7} are present as (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}. Preferably, at least 20 % by weight, or at least 30 % by weight, or at least 40 % by weight, or at least 50 % by weight, or at least 60 % by weight, or at least 70 % by weight, or at least 80 % by weight, or at least 90 % by weight of sail therapeutically effective amount of(i) {drug1}, (ii) {drug2}, (iii) {drug3}, (iv) {drug4}, (v) {drug5}, (vi) {drug6}, or (vii) {drug7} are present as (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vi) {drug7a}. Preferably, at least 95 % by weight, or at least 96 % by weight, or at least 97 % by weight, or at least 98 % by weight, or at least 99 % by weight, or at least 99.5 % by weight, or at least 99.8 % by weight, or at least 99.9 % by weight of said therapeutically effective amount of (i) {drug1}, (ii) {drug2}, (iii) {drug3}, (iv) {drug4}, (v) {drug5}, (vi) {drug6}, or (vii) {drug7} are present as (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}.

The total content of (i) {drug1}, (ii) {drug2}, (iii) {drug3}, (iv) {drug4}, (v) {drug5}, (vi) {drug6}, or (vii) {drug7} as well as the relative content of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} can be determined by standard analysis known to the skilled artisan. Suitable methods include but are not

limited to thermal analysis, HPLC and XRPD.

In a preferred embodiment of the administration unit according to the invention, not more than 90 % by weight of said therapeutically effective amount of (i) {drug1}, (ii) {drug2}, (iii) {drug3}, (iv) {drug4}, (v) {drug5}, (vi) {drug6}, or (vii) {drug7} are amorphous. Preferably, not more than 80 % by weight, or not more than 70 % by weight, or not more than 60 % by weight, or not more than 50 % by weight, or not more than 40 % by weight, or not more than 30 % by weight, or not more than 20 % by weight, or not more than 10 % by weight of said therapeutically effective amount of (i) {drug1}, (ii) {drug2}, (iii) {drug3}, (iv) {drug4}, (v) {drug5}, (vi) {drug6}, or (vii) {drug7} are amorphous. Preferably, not more than 5 % by weight, or not more than 4 % by weight, or not more than 3 % by weight, or not more than 2 % by weight, or not more than 1 % by weight, or not more than 0.5 % by weight, or not more than 0.2 % by weight, or not more than 0.1 % by weight of said therapeutically effective amount of (i) {drug1}, (ii) {drug2}, (iii) {drug3}, (iv) {drug4}, (v) {drug5}, (vi) {drug6}, or (vii) {drug7} are amorphous.

The total content of (i) {drug1}, (ii) {drug2}, (iii) {drug3}, (iv) {drug4}, (v) {drug5}, (vi) {drug6}, or (vii) {drug7} as well as the relative content of amorphous (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} can be determined by standard analysis known to the skilled artisan. Suitable methods include but are not limited to thermal analysis, HPLC and XRPD (see e.g. K.D. Harris, Powder diffraction crystallography of molecular solids, Top Curr Chem., 2012, 315:133-77).

In a preferred embodiment of the administration unit according to the invention, not more than 90 % by weight of said therapeutically effective amount of (i) {drug1}, (ii) {drug2}, (iii) {drug3}, (iv) {drug4}, (v) {drug5}, (vi) {drug6}, or (vii) {drug7} are polymorphs of (i) {drug1}, (ii) {drug2}, (iii) {drug3}, (iv) {drug4}, (v) {drug5}, (vi) {drug6}, or (vii) {drug7} other than (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}. Preferably, not more than 80 % by weight, or not more than 70 % by weight, or not more than 60 % by weight, or not more than 50 % by weight, or not more than 40 % by weight, or not more than 30 % by weight, or not more than 20 % by weight, or not more than 10 % by weight of said therapeutically effective amount of (i) {drug1}, (ii) {drug2}, (iii) {drug3}, (iv) {drug4}, (v) {drug5}, (vi) {drug6}, or (vii) {drug7} are polymorphs of (i) {drug1}, (ii) {drug2}, (iii) {drug3}, (iv) {drug4}, (v) {drug5}, (vi) {drug6}, or (vii) {drug7} other than (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}. Preferably, not more than 5 % by weight, or not more than 4 % by weight, or not more than 3 % by weight, or not more than 2 % by weight, or not more than 1 % by weight, or not more than 0.5 % by weight, or not more than 0.2 % by weight, or not more than 0.1 % by weight of said therapeutically effective amount of (i) {drug1}, (ii) {drug2}, (iii) {drug3}, (iv) {drug4}, (v) {drug5}, (vi) {drug6}, or (vii) {drug7} arepolymorphs of (i) {drug1}, (ii) {drug2}, (iii) {drug3}, (iv) {drug4}, (v) {drug5}, (vi) {drug6}, or (vii) {drug7} other than (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}.

The total content of (i) {drug1}, (ii) {drug2}, (iii) {drug3}, (iv) {drug4}, (v) {drug5}, (vi) {drug6}, or (vii) {drug7} as well as the relative content of polymorphs other than (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} can be determined by standard analysis known to the skilled artisan. Suitable methods include but are not limited to thermal analysis, HPLC and XRPD (see e.g. N. Chieng, T. Rades, J.Aaltonen, An overview of recent studies on the analysis of pharmaceutical polymorphs, J Pharm Biomed Anal. 2011, 25:55(4):618-44; R. Hilfiker, Polymorphism, Wiley-VCH, 1st ed. 2006; H.G. Brittain, Polymorphism in Pharmaceutical Solids (Drugs and the Pharmaceutical Sciences), Informa Healthcare, 2nd ed. 2009).

In a preferred embodiment of the administration unit according to the invention, the therapeutically effective amount of (i) {drug1}, (ii) {drug2}, (iii) {drug3}, (iv) {drug4}, (v) {drug5}, (vi) {drug6}, or (vii) {drug7} is 0.1 μg to 2 g of (i) {drug1}, (ii) {drug2}, (iii) {drug3}, (iv) {drug4}, (v) {drug5}, (vi) {drug6}, or (vii) {drug7}. Preferably, the therapeutically effective amount of (i) {drug1}, (ii) {drug2}, (iii) {drug3}, (iv) {drug4}, (v) {drug5}, (vi) {drug6}, or (vii) {drug7} is 1 μg to 2.5 μg of (i) {drug1}, (ii) {drug2}, (iii) {drug3}, (iv) {drug4}, (v) {drug5}, (vi) {drug6}, or (vii) {drug7}, or 2.5 μg to 5 μg of (i) {drug1}, (ii) {drug2}, (iii) {drug3}, (iv) {drug4}, (v) {drug5}, (vi) {drug6}, or (vii) {drug7}, or 5 μg to 10 μg of (i) {drug1}, (ii) {drug2}, (iii) {drug3}, (iv) {drug4}, (v) {drug5}, (vi) {drug6}, or (vii) {drug7}, or 10 μg to 25 μg of (i) {drug1}, (ii) {drug2}, (iii) {drug3}, (iv) {drug4}, (v) {drug5}, (vi) {drug6}, or (vii) {drug7}, or 25 μg to 50 μg of (i) {drug1}, (ii) {drug2}, (iii) {drug3}, (iv) {drug4}, (v) {drug5}, (vi) {drug6}, or (vii) {drug7}, or 50 μg to 100 μg of (i) {drug1}, (ii) {drug2}, (iii) {drug3}, (iv) {drug4}, (v) {drug5}, (vi) {drug6}, or (vii) {drug7}, or 100 μg to 250 μg of (i) {drug1}, (ii) {drug2}, (iii) {drug3}, (iv) {drug4}, (v) {drug5}, (vi) {drug6}, or (vii) {drug7}, or 250 μg to 500 μg of (i) {drug1}, (ii) {drug2}, (iii) {drug3}, (iv) {drug4}, (v) {drug5}, (vi) {drug6}, or (vii) {drug7}, or 500 μg to 1 mg of (i) {drug1}, (ii) {drug2}, (iii) {drug3}, (iv) {drug4}, (v) {drug5}, (vi) {drug6}, or (vii) {drug7}, or 1 mg to 2.5 mg of (i) {drug1}, (ii) {drug2}, (iii) {drug3}, (iv) {drug4}, (v) {drug5}, (vi) {drug6}, or (vii) {drug7}, or 2.5 mg to 5 mg of (i) {drug1}, (ii) {drug2}, (iii) {drug3}, (iv) {drug4}, (v) {drug5}, (vi) {drug6}, or (vii) {drug7}, or 5 mg to 10 mg of (i) {drug1}, (ii) {drug2}, (iii) {drug3}, (iv) {drug4}, (v) {drug5}, (vi) {drug6}, or (vii) {drug7}, or 10 mg to 25 mg of (i)

{drug1}, (ii) {drug2}, (iii) {drug3}, (iv) {drug4}, (v) {drug5}, (vi) {drug6}, or (vii) {drug7}, or 25 mg to 50 mg of (i) {drug1}, (ii) {drug2}, (iii) {drug3}, (iv) {drug4}, (v) {drug5}, (vi) {drug6}, or (vii) {drug7}, or 50 mg to 100 mg of (i) {drug1}, (ii) {drug2}, (iii) {drug3}, (iv) {drug4}, (v) {drug5}, (vi) {drug6}, or (vii) {drug7}, or 100 mg to 250 mg of (i) {drug1}, (ii) {drug2}, (iii) {drug3}, (iv) {drug4}, (v) {drug5}, (vi) {drug6}, or (vii) {drug7}, or 250 mg to 500 mg of (i) {drug1}, (ii) {drug2}, (iii) {drug3}, (iv) {drug4}, (v) {drug5}, (vi) {drug6}, or (vii) {drug7}, or 500 mg to 1 g of (i) {drug1}, (ii) {drug2}, (iii) {drug3}, (iv) {drug4}, (v) {drug5}, (vi) {drug6}, or (vii) {drug7}, or 1 g to 1.25 g of (i) {drug1}, (ii) {drug2}, (iii) {drug3}, (iv) {drug4}, (v) {drug5}, (vi) {drug6}, or (vii) {drug7}, or 1.25 g to 1.5 g of (i) {drug1}, (ii) {drug2}, (iii) {drug3}, (iv) {drug4}, (v) {drug5}, (vi) {drug6}, or (vii) {drug7}, or 1.5 g to 1.75 g of (i) {drug1}, (ii) {drug2}, (iii) {drug3}, (iv) {drug4}, (v) {drug5}, (vi) {drug6}, or (vii) {drug7}, or 1.75 g to 2 g of (i) {drug1}, (ii) {drug2}, (iii) {drug3}, (iv) {drug4}, (v) {drug5}, (vi) {drug6}, or (vii) {drug7}.

Preferably, the administration unit according to the invention comprises 0.1 $\mu$g to 2 g of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}. Preferably, it comprises 1 $\mu$g to 2.5 $\mu$g of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, or 2.5 $\mu$g to 5 $\mu$g of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, or 5 $\mu$g to 10 $\mu$g of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, or 10 $\mu$g to 25 $\mu$g of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, or 25 $\mu$g to 50 $\mu$g of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, or 50 $\mu$g to 100 $\mu$g of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, or 100 $\mu$g to 250 $\mu$g of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, or 250 $\mu$g to 500 $\mu$g of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, or 500 $\mu$g to 1 mg of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, or 1 mg to 2.5 mg of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, or 2.5 mg to 5 mg of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, or 5 mg to 10 mg of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, or 10 mg to 25 mg of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, or 25 mg to 50 mg of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, or 50 mg to 100 mg of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, or 100 mg to 250 mg of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, or 250 mg to 500 mg of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, or 500 mg to 1 g of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, or 1 g to 1.25 g of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, or 1.25 gto 1.5 g of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, or 1.5 gto 1.75 g of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, or 1.75 g to 2 g of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}.

Preferably, the administration unit according to the invention contains (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v)

{drug5a}, or {drug5b}; (vi) {drug6a}; or (vii) {drug7a} as substantially the only form of (i) {drug1}, (ii) {drug2}, (iii) {drug3}, (iv) {drug4}, (v) {drug5}, (vi) {drug6}, or (vii) {drug7}, i.e. preferably contains neither non-crystalline forms of (i) {drug1}, (ii) {drug2}, (iii) {drug3}, (iv) {drug4}, (v) {drug5}, (vi) {drug6}, or (vii) {drug7} nor crystalline forms other than (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}.

The administration unit according to the invention and its constituents, i.e. (i) {drug1}, (ii) {drug2}, (iii) {drug3}, (iv) {drug4}, (v) {drug5}, (vi) {drug6}, or (vii) {drug7} and (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, respectively, as well as pharmaceutical excipients, can be characterized by analytic methods that are known to the skilled artisan and described in Ph. Eur. and USP (see e.g. H.G. Brittain, S.J. Bodanowich, D.E. Bugay, J. DeVoncentis, G. Lewen, A.W. Newman, Physical characterization of pharmaceutical solids, Pharm Res. 1991, 8(8):963-73; D.E. Bugay, Characterization of the solid-state: spoectroscopic techniques, Adv Drug Deliv Rev., 2001, 48(1):43-65; P.A. Tishmack, D.E. Bugay, S.R. Bym, Solid-state nuclear magnetic resonance spectroscopy - pharmaceutical application, J Pharm Sci, 2003, 92(3):441-74; C.J. Lee, C.J. Strachan, P.J. Manson, T. Rades, Characterization of the bulk properties of pharmaceutical solids using nonlinear optics - a review, J Pharm Pharmacol., 2007, 59(2):241-50); R.A. Storey, Solid State Characterization of Pharmaceuticals, Wiley-Blackwell, 2011).

In a preferred embodiment, the administration unit according to the invention contains (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} in form of particles, wherein the particle size distribution of X90 is about 500 μm or less, i.e. a particle size distribution (preferably by volume) such that at least 90 % of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} has a particle size smaller than 500 μm. Preferably, for (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} the particle size distribution of X90 is about 480 μm or less, or about 460 μm or less, or about 440 μm or less, or about 420 μm or less, or about 400 μm or less, or about 380 μm or less, or about 360 μm or less, or about 340 μm or less, or about 320 μm or less, or about 300 μm or less, or about 280 μm or less, or about 260 μm or less, or about 240 μm or less, or about 220 μm or less, or about 200 μm or less, or about 180 μm or less, or about 160 μm or less, or about 140 μm or less, or about 120 μm or less, or about 100 μm or less, or about 80 μm or less, or about 60 μm or less, or about 40 μm or less, or about 20 μm or less.

In a preferred embodiment, the administration unit according to the invention contains (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} in form of particles, wherein the particle size distribution of X50 is about 400 μm or less, i.e. a particle size distribution (preferably by volume) such that at least 50 % of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} has a particle size smaller than 400 μm. Preferably, for (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} the particle size distribution of X50 is about 380 μm or less, or about 360 μm or less, or about 340 μm or less, or about 320 μm or less, or about 300 μm or less, or about 280 μm or less, or about 260 μm or less, or about 240 μm or less, or about 220 μm or less, or about 200 μm or less, or about 180 μm or less, or about 160 μm or less, or about 140 μm or less, or about 120 μm or less, or about 100 μm or less, or about 80 μm or less, or about 70 μm or less, or about 60 μm or less, or about 50 μm or less. Preferably, for (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} the particle size distribution of X50 is about 45 μm or less, or about 40 μm or less, or about 35 μm or less, or about 30 μm or less, or about 25 μm or less, or about 20 μm or less, or about 15 μm or less, or about 10 μm or less. According to USP, the following parameters may be defined based on the cumulative distribution. QR(X) = cumulative distribution of particles with a dimension less than or equal to X [μm] where the subscript R reflects the distribution type: 0 Number, 1 Length, 2 Area, 3 Volume. Therefore, by definition: QR(X) = 0.90 when X = X90; QR(X) = 0.50 when X = X50; and QR(X) = 0.10 when X = X10.

In preferred embodiments, the administration unit according to the invention contains (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} in form of particles having a cumulative distribution by volume basis Q3(X) (X in μm) of Q3(355) <0.50; or Q3(180) <0.50 and Q3(355) ≥0.50; or Q3(125) <0.50 and Q3(180) ≥0.50; or Q3(125) ≥0.50.

In preferred embodiments, the administration unit according to the invention contains (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} in form of particles having a cumulative distribution

by volume basis Q3(X) (X in μm) of Q3(268) <0.50 and Q3(355) ≥0.50; or Q3(180) <0.50 and Q3(268) ≥0.50; or Q3(153) <0.50 and Q3(180) ≥0.50; or Q3(125) <0.50 and Q3(153) ≥0.50; or Q3(111) <0.50 and Q3(125) ≥0.50; or Q3(98) <0.50 and Q3(111) ≥0.50; or Q3(98) ≥0.50.

In preferred embodiments, the administration unit according to the invention contains (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} in form of particles having a cumulative distribution by volume basis Q3(X) (X in μm) of Q3(312) <0.50 and Q3(355) ≥0.50; or Q3(268) <0.50 and Q3(312) ≥0.50; or Q3(224) <0.50 and Q3(268) ≥0.50; or Q3(180) <0.50 and Q3(224) ≥0.50; or Q3(167) <0.50 and Q3(180) ≥0.50; or Q3(153) <0.50 and Q3(167) ≥0.50; or Q3(139) <0.50 and Q3(153) ≥0.50; or Q3(125) <0.50 and Q3(139) ≥0.50; or Q3(111) <0.50 and Q3(125) ≥0.50; or Q3(98) <0.50 and Q3(111) ≥0.50; or Q3(84) <0.50 and Q3(98) ≥0.50; or Q3(84) ≥0.50.

The indicated particle size properties are determined by laser-diffraction method, in particular low angle laser light scattering, i.e. Fraunhofer diffraction. Alternatively, the particle size properties can be also determined by microscopy (e.g. electron microscopy or scanning electron microscopy). The powder fineness is preferably determined in accordance with USP35 <811>. The results of the particle size distribution determined by different techniques can be correlated with one another. Preferably, the administration unit according to the invention is solid, semisolid or liquid.

Preferably, the administration unit according to the invention is for administration once daily, or twice daily, or thrice daily, or four times daily, or five times daily, or six times daily.

Preferably, the administration unit according to the invention is monolithic or multiparticulate.

In a preferred embodiment of the administration unit according to the invention, (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} is homogeneously distributed over the administration unit.

Excipient composition and homogeneity can be determined by standard analysis known to the skilled artisan. Suitable methods include but are not limited to near-infrared chemical imaging.

In another preferred embodiment of the administration unit according to the invention, (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} is not homogeneously distributed over the administration unit.

In a preferred embodiment, the administration unit according to the invention provides controlled release of (i) {drug1}, (ii) {drug2}, (iii) {drug3}, (iv) {drug4}, (v) {drug5}, (vi) {drug6}, or (vii) {drug7}. Preferably, the administration unit according to the invention comprises a controlled release matrix or a controlled release coating.

In a preferred embodiment, under in vitro conditions (paddle apparatus, 75 rpm, 900 mL artificial gastric juice, pH 1.2) the administration unit according to the invention has released after 1 hour not more than 90 % of (i) {drug1}, (ii) {drug2}, (iii) {drug3}, (iv) {drug4}, (v) {drug5}, (vi) {drug6}, or (vii) {drug7}. Preferably, the administration unit according to the invention has released after 1 hour not more than 80 % of (i) {drug1}, (ii) {drug2}, (iii) {drug3}, (iv) {drug4}, (v) {drug5}, (vi) {drug6}, or (vii) {drug7}, or not more than 70 % of (i) {drug1}, (ii) {drug2}, (iii) {drug3}, (iv) {drug4}, (v) {drug5}, (vi) {drug6}, or (vii) {drug7}, or not more than 60 % of (i) {drug1}, (ii) {drug2}, (iii) {drug3}, (iv) {drug4}, (v) {drug5}, (vi) {drug6}, or (vii) {drug7}, or not more than 50 % of (i) {drug1}, (ii) {drug2}, (iii) {drug3}, (iv) {drug4}, (v) {drug5}, (vi) {drug6}, or (vii) {drug7}, or not more than 40 % of (i) {drug1}, (ii) {drug2}, (iii) {drug3}, (iv) {drug4}, (v) {drug5}, (vi) {drug6}, or (vii) {drug7}, or not more than 30 % of (i) {drug1}, (ii) {drug2}, (iii) {drug3}, (iv) {drug4}, (v) {drug5}, (vi) {drug6}, or (vii) {drug7}, or not more than 20 % of (i) {drug1}, (ii) {drug2}, (iii) {drug3}, (iv) {drug4}, (v) {drug5}, (vi) {drug6}, or (vii) {drug7}, or not more than 10 % of (i) {drug1}, (ii) {drug2}, (iii) {drug3}, (iv) {drug4}, (v) {drug5}, (vi) {drug6}, or (vii) {drug7}.

In a preferred embodiment, under in vitro conditions (paddle apparatus, 75 rpm, 900 mL artificial gastric juice, pH 1.2) the administration unit according to the invention has released after 2 hours not more than 90 % of (i) {drug1}, (ii) {drug2}, (iii) {drug3}, (iv) {drug4}, (v) {drug5}, (vi) {drug6}, or (vii) {drug7}. Preferably, the administration unit according to the invention has released after 2 hours not more than 80 % of (i) {drug1}, (ii) {drug2}, (iii) {drug3}, (iv) {drug4}, (v) {drug5}, (vi) {drug6}, or (vii) {drug7}, or not more than 70 % of (i) {drug1}, (ii) {drug2}, (iii) {drug3}, (iv) {drug4}, (v) {drug5}, (vi) {drug6}, or (vii) {drug7}, or not more than 60 % of (i) {drug1}, (ii) {drug2}, (iii) {drug3}, (iv) {drug4}, (v) {drug5}, (vi) {drug6}, or (vii) {drug7}, or not more than 50 % of (i) {drug1}, (ii) {drug2}, (iii) {drug3}, (iv) {drug4}, (v) {drug5}, (vi) {drug6}, or (vii) {drug7}, or not more than 40 % of (i) {drug1}, (ii) {drug2}, (iii) {drug3}, (iv) {drug4}, (v) {drug5}, (vi) {drug6}, or (vii) {drug7}, or not more than 30 % of (i) {drug1}, (ii) {drug2}, (iii) {drug3}, (iv) {drug4}, (v) {drug5}, (vi) {drug6}, or (vii) {drug7}, or not more than 20 % of (i) {drug1}, (ii) {drug2}, (iii) {drug3}, (iv) {drug4}, (v) {drug5}, (vi) {drug6}, or (vii) {drug7}, or not more than 10 % of (i) {drug1}, (ii) {drug2}, (iii) {drug3}, (iv) {drug4}, (v) {drug5}, (vi) {drug6}, or (vii) {drug7}.

In a preferred embodiment, under in vitro conditions (paddle apparatus, 75 rpm, 900 mL artificial gastric juice, pH 1.2) the administration unit according to the invention has released after 4 hours not more than 90 % of (i) {drug1}, (ii) {drug2}, (iii) {drug3}, (iv) {drug4}, (v) {drug5}, (vi) {drug6}, or (vii) {drug7}. Preferably, the administration unit according to the invention has released after 4 hours not more than 80 % of (i) {drug1}, (ii) {drug2}, (iii) {drug3}, (iv) {drug4}, (v) {drug5}, (vi) {drug6}, or (vii) {drug7}, or not more than 70 % of (i) {drug1}, (ii) {drug2}, (iii) {drug3}, (iv) {drug4}, (v) {drug5}, (vi) {drug6}, or (vii) {drug7}, or not more than 60 % of (i) {drug1}, (ii) {drug2}, (iii) {drug3}, (iv) {drug4}, (v) {drug5}, (vi)

{drug6}, or (vii) {drug7}, or not more than 50 % of (i) {drug1}, (ii) {drug2}, (iii) {drug3}, (iv) {drug4}, (v) {drug5}, (vi) {drug6}, or (vii) {drug7}, or not more than 40 % of (i) {drug1}, (ii) {drug2}, (iii) {drug3}, (iv) {drug4}, (v) {drug5}, (vi) {drug6}, or (vii) {drug7}, or not more than 30 % of (i) {drug1}, (ii) {drug2}, (iii) {drug3}, (iv) {drug4}, (v) {drug5}, (vi) {drug6}, or (vii) {drug7}, or not more than 20 % of (i) {drug1}, (ii) {drug2}, (iii) {drug3}, (iv) {drug4}, (v) {drug5}, (vi) {drug6}, or (vii) {drug7}, or not more than 10 % of (i) {drug1}, (ii) {drug2}, (iii) {drug3}, (iv) {drug4}, (v) {drug5}, (vi) {drug6}, or (vii) {drug7}.

In a preferred embodiment, under in vitro conditions (paddle apparatus, 75 rpm, 900 mL artificial gastric juice, pH 1.2) the administration unit according to the invention has released after 8 hours not more than 90 % of (i) {drug1}, (ii) {drug2}, (iii) {drug3}, (iv) {drug4}, (v) {drug5}, (vi) {drug6}, or (vii) {drug7}. Preferably, the administration unit according to the invention has released after 8 hours not more than 80 % of (i) {drug1}, (ii) {drug2}, (iii) {drug3}, (iv) {drug4}, (v) {drug5}, (vi) {drug6}, or (vii) {drug7}, or not more than 70 % of (i) {drug1}, (ii) {drug2}, (iii) {drug3}, (iv) {drug4}, (v) {drug5}, (vi) {drug6}, or (vii) {drug7}, or not more than 60 % of (i) {drug1}, (ii) {drug2}, (iii) {drug3}, (iv) {drug4}, (v) {drug5}, (vi) {drug6}, or (vii) {drug7}, or not more than 50 % of (i) {drug1}, (ii) {drug2}, (iii) {drug3}, (iv) {drug4}, (v) {drug5}, (vi) {drug6}, or (vii) {drug7}, or not more than 40 % of (i) {drug1}, (ii) {drug2}, (iii) {drug3}, (iv) {drug4}, (v) {drug5}, (vi) {drug6}, or (vii) {drug7}, or not more than 30 % of (i) {drug1}, (ii) {drug2}, (iii) {drug3}, (iv) {drug4}, (v) {drug5}, (vi) {drug6}, or (vii) {drug7}, or not more than 20 % of (i) {drug1}, (ii) {drug2}, (iii) {drug3}, (iv) {drug4}, (v) {drug5}, (vi) {drug6}, or (vii) {drug7}, or not more than 10 % of (i) {drug1}, (ii) {drug2}, (iii) {drug3}, (iv) {drug4}, (v) {drug5}, (vi) {drug6}, or (vii) {drug7}.

In another preferred embodiment, the administration unit according to the invention provides immediate release of (i) {drug1}, (ii) {drug2}, (iii) {drug3}, (iv) {drug4}, (v) {drug5}, (vi) {drug6}, or (vii) {drug7}.

In a preferred embodiment, under in vitro conditions (paddle apparatus, 75 rpm, 900 mL artificial gastric juice, pH 1.2) the administration unit according to the invention has released after 0.5 hours at least 10 % of (i) {drug1}, (ii) {drug2}, (iii) {drug3}, (iv) {drug4}, (v) {drug5}, (vi) {drug6}, or (vii) {drug7}. Preferably, the administration unit according to the invention has released after 0.5 hours at least 20 % of (i) {drug1}, (ii) {drug2}, (iii) {drug3}, (iv) {drug4}, (v) {drug5}, (vi) {drug6}, or (vii) {drug7}, or at least 30 % of (i) {drug1}, (ii) {drug2}, (iii) {drug3}, (iv) {drug4}, (v) {drug5}, (vi) {drug6}, or (vii) {drug7}, or at least 40 % of (i) {drug1}, (ii) {drug2}, (iii) {drug3}, (iv) {drug4}, (v) {drug5}, (vi) {drug6}, or (vii) {drug7}, or at least 50 % of (i) {drug1}, (ii) {drug2}, (iii) {drug3}, (iv) {drug4}, (v) {drug5}, (vi) {drug6}, or (vii) {drug7}, or at least 60 % of (i) {drug1}, (ii) {drug2}, (iii) {drug3}, (iv) {drug4}, (v) {drug5}, (vi) {drug6}, or (vii) {drug7}, or at least 70 % of (i) {drug1}, (ii) {drug2}, (iii) {drug3}, (iv) {drug4}, (v) {drug5}, (vi) {drug6}, or (vii) {drug7}, or at least 80 % of (i) {drug1}, (ii) {drug2}, (iii) {drug3}, (iv) {drug4}, (v) {drug5}, (vi) {drug6}, or (vii) {drug7}, or at least 90 % of (i) {drug1}, (ii) {drug2}, (iii) {drug3}, (iv) {drug4}, (v) {drug5}, (vi) {drug6}, or (vii) {drug7}.

Preferably, the administration unit according to the invention has a total weight of 10 mg to 3 g. Preferably, the administration unit according to the invention has a total weight of 10 to 25 mg, or 25 mg to 50 mg, or 50 mg to 100 mg, or 100 mg to 250 mg, or 250 mg to 500 mg, or 500 mg to 1000 mg, or 1000 mg to 2500 mg.

In a preferred embodiment, the administration unit according to the invention is for systemic or topical administration.

In another preferred embodiment, the administration unit according to the invention is for parenteral administration. Preferably, the administration unit according to the invention is for oral, buccal, opthalmic, nasal, rectal, transmucosal, or intestinal administration. Preferably, it is for oral administration.

Preferably, the administration unit according to the invention is selected from the group consisting of tablets, capsules, effervescent tablets, orodispersible tablets, dragees, sachets, drops, suspensions, and powders. Preferably, it is a tablet. Preferably, the administration unit according to the invention is round or oblong; and/or is planar or biconvex.

In a preferred embodiment, the administration unit according to the invention has a round cross-section with a diameter of 1 mm to 20 mm. Preferably, the diameter of the round cross-section is 1 mm to 3 mm, or 3 mm to 5 mm, or 5 mm to 8 mm, or 8 mm to 10 mm, or 10 mm to 13 mm, or 13 mm to 15 mm, or 15 mm to 18 mm, or 18 mm to 20 mm.

In a preferred embodiment, the administration unit according to the invention is oblong and has an aspect ratio of at least 1.1:1. Preferably, the aspect ratio is at least 4:3, or at least 21/2:1, or at least 3:2, or at least 16:10, or at least □:1, or at least 5:3, or at least 16:9.

In a preferred embodiment, the administration unit according to the invention has been manufactured by direct compression. In another preferred embodiment, the administration unit according to the invention has been manufactured by granulation and subsequent compression of granules. Preferably, granulation is wet granulation or dry granulation.

Preferably, the administration unit according to the invention has been compacted at a pressure of 10 MPa to 10,000 MPa. Preferably, the administration unit according to the invention has been compacted at a pressure of 10 MPa to 25 MPa, or 25 MPa to 50 MPa, or 50 MPa to 100 MPa, or 100 MPa to 250 MPa, or 250 MPa to 500 MPa, or 500 MPa to 1000 MPa, or 1000 MPa to 2500 MPa, or 2500 MPa to 5000 MPa, or 5000 MPa to 10,000 MPa.

In a preferred embodiment, the administration unit according to the invention comprises a film coating. Preferably, the film coating is enteric. Suitable enteric coating materials are known to the skilled artisan and include but are not limited to methyl acrylate-methacrylic acid copolymers, cellulose acetate succinate, hydroxy propyl methyl cellulose phthalate, hydroxy propyl methyl cellulose acetate succinate (hypromellose acetate succinate), polyvinyl acetate phthalate (PVAP), methyl methacrylate-methacrylic acid copolymers, sodium alginate and stearic acid.

In a preferred embodiment of the administration unit according to the invention, the film coating comprises a cellulose ether (e.g. hydroxypropylmethyl cellulose) or a synthetic polymer (e.g. polyvinyl alcohol).

In a preferred embodiment of the administration unit according to the invention, the film coating has an average thickness of 0.01 μm to 0.03 μm, or 0.03 to 0.05 μm, or 0.05 to 0.1 μm, or 0.1 to 0.25 μm, or 0.25 to 0.5 μm, or 0.5 to 1.0 μm, or 1.0 μm to 2.5 μm, or 2.5 μm to 5.0 μm, or 5.0 μm to 10 μm, or 10 μm to 25 μm, or 25 μm to 50 μm, or 50 μm to 100 μm, or 100 μm to 250 μm, or 250 μm to 500 μm, or 500 μm to 1000 μm.

In a preferred embodiment, the administration unit according to the invention has a tablet porosity of not more than 90 %. Preferably, it has a tablet porosity of not more than 80 %, or not more than 70 %, or not more than 60 %, or not more than 50 %, or not more than 40 %, or not more than 30 %, or not more than 20 %, or not more than 10 %. Preferably, it has a tablet porosity of not more than 5 %, or not more than 4 %, or not more than 3 %, or not more than 2 %, or not more than 1 %, or not more than 0.5 %, or not more than 0.2 %, or not more than 0.1 %.

The most common strength test in pharmaceutical applications is the diametral compression test, which is used to calculate the radial tensile strength of a tablet (Fell, J.T., Newton, J.M., 1970. Determination of tablet strength by diametral compression test. J. Pharm. Sci. 59, 688-691). In order to calculate the radial tensile strength, the stress conditions have to be such that the tablet fails in tension. During radial tensile strength measurements, the fracture occurs through a predetermined diametral cross section of the tablet. Therefore, the radial tensile strength is likely to reflect the average strength of tablet rather than the strength of the weakest plane in the tablet.

In a preferred embodiment, the administration unit according to the invention has a radial tensile strength of 0.1 to 100 MPa. Preferably, it has a radial tensile strength of 0.1 MPa to 0.3 MPa, or 0.3 MPa to 0.5 MPa, or 0.5 MPa to 1.0 MPa, or 1.0 MPa to 2.5 MPa, or 2.5 MPa to 5.0 MPa, or 5.0 MPa to 10 MPa, or 10 MPa to 25 MPa, or 25 MPa to 50 MPa, or 50 MPa to 100 MPa.

Another method for determining mechanical strength is to measure the axial tensile strength. The force necessary to break the tablet is obtained by pulling the tablet parallel to the applied force during the formation of the tablet and this force is then used to calculate the axial tensile strength (Nyström, C., Malmqvist, K., Mazur, J., Alex, W., Hölzer, A.W., 1978. Measurement of axial and radial tensile strength of tablets and their relation to capping. Acta Pharm. Suec. 15, 226-232). During axial tensile strength measurements, the fracture will occur through the weakest plane in the tablet. Consequently, this method allows detection of capping tendencies in a tablet.

In a preferred embodiment, the administration unit according to the invention has an axial tensile strength of 0.1 to 100 MPa. Preferably, it has an axial tensile strength of 0.1 MPa to 0.3 MPa, or 0.3 MPa to 0.5 MPa, or 0.5 MPa to 1.0 MPa, or 1.0 MPa to 2.5 MPa, or 2.5 MPa to 5.0 MPa, or 5.0 MPa to 10 MPa, or 10 MPa to 25 MPa, or 25 MPa to 50 MPa, or 50 MPa to 100 MPa.

In a preferred embodiment, the administration unit according to the invention has an average pore size of 0.001 μm to 1000 μm. Preferably, it has an average pore size of 0.001 μm to 0.003 μm, or 0,003 μm to 0.005 μm, or 0.005 μm to 0.01 μm, or 0.01 μm to 0.025 μm, or 0.025 μm to 0.05 μm, or 0.05 μm to 0.1 μm, or 0.1 μm to 0.25 μm, or 0.25 μm to 0.5 μm, or 0.5 μm to 1 μm, or 1 μm to 2.5 μm, or 2.5 μm to 5 μm, or 5 μm to 10 μm, or 10 μm to 25 μm, or 25 μm to 50 μm, or 50 μm to 100 μm, or 100 μm to 250 μm, or 250 μm to 500 μm, or 500 μm to 1000 μm.

The pore size distribution of a tablet may be assessed by methods that are known to the skilled artisan and include but are not limited to gas adsorption (e.g. Stanley-Wood, N.G., Johansson, M.E., 1980. Variation of intra- and inter-particle porosity with degree of compaction. Analyst 105, 1104-1112; Westermarck, S., Juppo, A.M., Kervinen, L., Yliruusi, J., 1998. Pore structure and surface area of mannitol powder, granules and tablets determined with mercury porosimetry and nitrogen adsorption. Eur. J. Pharm. Biopharm. 46, 61-86) or mercury porosimetry (e.g. Stanley-Wood, N.G., Johansson, M.E., 1980. Variation of intra- and inter-particle porosity with degree of compaction. Analyst 105, 1104-1112; Juppo, A.M., 1996. Relationship between breaking force and pore structure of lactose, glucose and mannitol tablets. Int. J. Pharm.127, 95-102; Westermarck, S., Juppo, A.M., Kervinen, L., Yliruusi, J., 1998. Pore structure and surface area of mannitol powder, granules and tablets determined with mercury porosimetry and nitrogen adsorption. Eur. J. Pharm. Biopharm. 46, 61-86). These techniques are complementary, in that mercury porosimetry can be used to measure larger pores (the lower size limit is about 0.003 μm in diameter) while gas adsorption allows measurement of smaller pores. For further details it is also referred to S. Lowell et al., Characterization of Porous Solids and Powders: Surface Area, Pore Size and Density (Particle Technology Series), Springer, 2010.

In a preferred embodiment, the administration unit according to the invention has a pore size distribution such that at least 10 % of the pores have a pore size of not more than 0.1 μm. Preferably, the pore size distribution is such that at least 20 %, or at least 30 %, or at least 40 %, or at least 50 %, or at least 60 %, or at least 70 %, or at least 80 %, or at least 90 % of the pores have a pore size of not more than 0.1 μm.

In another preferred embodiment, the administration unit according to the invention has a pore size distribution such that at least 10 % of the pores have a pore size of not less than 0.1 μm. Preferably, the pore size distribution is such that at least 20 %, or at least 30 %, or at least 40 %, or at least 50 %, or at least 60 %, or at least 70 %, or at least 80 %, or at least 90 % of the pores have a pore size of not less than 0.1 μm.

In preferred embodiment, the administration unit according to the invention has a pore size distribution such

that at least 10 % of the pores have a pore size of not more than μm. Preferably, the pore size distribution is such that at least 20 %, or at least 30 %, or at least 40 %, or at least 50 %, or at least 60 %, or at least 70 %, or at least 80 %, or at least 90 % of the pores have a pore size of not more than μm.

In another preferred embodiment, the administration unit according to the invention has a pore size distribution such that at least 10 % of the pores have a pore size of not less than μm. Preferably, the pore size distribution is such that at least 20 %, or at least 30 %, or at least 40 %, or at least 50 %, or at least 60 %, or at least 70 %, or at least 80 %, or at least 90 % of the pores have a pore size of not less than 1 μm.

In a preferred embodiment, the administration unit according to the invention has a pore size distribution such that at least 10 % of the pores have a pore size of not more than 10 μm. Preferably, the pore size distribution is such that at least 20 %, or at least 30 %, or at least 40 %, or at least 50 %, or at least 60 %, or at least 70 %, or at least 80 %, or at least 90 % of the pores have a pore size of not more than 10 μm.

In another preferred embodiment, the administration unit according to the invention has a pore size distribution such that at least 10 % of the pores have a pore size of not less than 10 μm. Preferably, the pore size distribution is such that at least 20 %, or at least 30 %, or at least 40 %, or at least 50 %, or at least 60 %, or at least 70 %, or at least 80 %, or at least 90 % of the pores have a pore size of not less than 10 μm.

In a preferred embodiment, the administration unit according to the invention has a water content of not more than 5 % by weight, relative to the total weight of the tablet. Preferably, it has a water content of not more than 4 % by weight, or not more than 3 % by weight, or not more than 2 % by weight, or not more than 1 % by weight, or not more than 0.5 % by weight, or not more than 0.1 % by weight, relative to the total weight of the tablet.

In a preferred embodiment, the administration unit according to the invention has a true density of 0.60 to 1.40 g cm-3. Preferably, it has a true density of 0.60 to 0.65 g cm-3, or 0.65 to 0.70 g cm-3, or 0.70 to 0.75 g cm-3, or 0.75 to 0.80 g cm-3, or 0.80 to 0.85 g cm-3, or 0.85 to 0.90 g cm-3, or 0.90 to 0.95 g cm-3, or 0.95 to 1.00 g cm-3, or 1.00 to 1.05 g cm-3, or 1.05 to 1.10 g cm-3, or 1.10 to 1.15 g cm-3, or 1.15 to 1.20 g cm-3, or 1.20 to 1.25 g cm-3, or 1.25 to 1.30 g cm-3, or 1.30 to 1.35 g cm-3, or 1.35 to 1.40 g cm-3.

In a preferred embodiment, the administration unit according to the invention has an apparent density of 0.60 to 1.40 g cm-3. Preferably, it has an apparent density of 0.60 to 0.65 g cm-3, or 0.65 to 0.70 g cm-3, or 0.70 to 0.75 g cm-3, or 0.75 to 0.80 g cm-3, or 0.80 to 0.85 g cm-3, or 0.85 to 0.90 g cm-3, or 0.90 to 0.95 g cm-3, or 0.95 to 1.00 g cm-3, or 1.00 to 1.05 g cm-3, or 1.05 to 1.10 g cm-3, or 1.10 to 1.15 g cm-3, or 1.15 to 1.20 g cm-3, or 1.20 to 1.25 g cm-3, or 1.25 to 1.30 g cm-3, or 1.30 to 1.35 g cm-3, or 1.35 to 1.40 g cm-3.

In a preferred embodiment, the administration unit according to the invention disintegrates within 6000 seconds. Preferably, it disintegrates within 5500 seconds, or within 5000 seconds, or within 4500 seconds, or within 4000 seconds, or within 3500 seconds, or within 3000 seconds, or within 2500 seconds, or within 2000 seconds, or within 1500 seconds, or within 1000 seconds, or within 750 seconds, or within 500 seconds, or within 250 seconds.

In a preferred embodiment, the administration unit according to the invention is a capsule. Preferably, it comprises a multitude of particulates comprising (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}. Preferably, it comprises at least 2 particulates, or at least 3 particulates, or at least 4 particulates, or at least 5 particulates, or at least 6 particulates, or at least 7 particulates, or at least 8 particulates, or at least 9 particulates, or at least 10 particulates.

In a preferred embodiment of the administration unit according to the invention, the capsule material comprises hard gelatine. In a preferred embodiment, the administration unit according to the invention comprises at least 2 different types of particulates that differ from each other in at least one property selected from the group consisting of nature of excipients, content of excipients, content of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, content of (i) {drug1}, (ii) {drug2}, (iii) {drug3}, (iv) {drug4}, (v) {drug5}, (vi) {drug6}, or (vii) {drug7}, water content, total weight, size, and shape.

In a preferred embodiment, the administration unit according to the invention passes the storage stability test according to Ph. Eur. under accelerated storage conditions at 40°C and 75 % rel. humidity. Preferably, it has a storage stability for at least 3 months, or at least 4 months, or at least 5 months, or at least 6 months, or at least 7 months, or at least 8 months, or at least 9 months, or at least 10 months, or at least 11 months, or at least 12 months according to Ph. Eur. under accelerated storage conditions at 40°C and 75 % rel. humidity.

In a preferred embodiment, the administration unit according to the invention provides the peak plasma level within 0.1 hour to 36 hours after administration. Preferably, it provides the peak plasma level within 0.1 hour to 1 hour, or 1 hour to 2 hours, or 2 hours to 3 hours, or 3 hours to 4 hours, or 4 hours to 5 hours, or 5 hours to 6 hours, or 6 hours to 7 hours, or 7 hours to 8 hours, or 8 hours to 9 hours, or 9 hours to 10 hours, or 10 hours to 11 hours, or 11 hours to 12 hours, or 12 hours to 13 hours, or 13 hours to 14 hours, or 14 hours to 15 hours, or 15 hours to 16 hours, or 16 hours to 17 hours, or 17 hours to 18 hours, or 18 hours to 19 hours, or 19 hours to 20 hours, or 20 hours to 21 hours, or 21 hours to 22 hours, or 22 hours to 23 hours, or 23 hours to 24 hours, after administration.

Preferably, the administration unit according to the invention comprises one or more excipients independently selected from the group consisting of antiadherents, binders, disintegrants, fillers, diluents, flavors, colors, lubricants, glidants, sorbents, surfactants, preservatives and sweeteners.

In a preferred embodiment of the administration unit according to the invention, the antiadherent is selected from the group consisting of silicon dioxide, talc, talc waxes, stearates (e.g. magnesium, calcium and sodium), stearic acid, stearowet, boric acid, sodium chloride, DL-leucine, sodium oleate, sodium benzoate, sodium acetate, sodium lauryl sulfate, and magnesium lauryl sulfate. Magnesium stearate is particularly preferred.

In a preferred embodiment of the administration unit according to the invention, the binder is selected from the group consisting of saccharides and their derivatives; proteins; gums; silicates; and synthetic polymers. Preferably, the binder is a saccharide or a derivative thereof selected from the group consisting of disaccharides (e.g. sucrose, glucose or lactose); polysaccharides and their derivatives (e.g. starch, pregelatinized starch, cellulose, microcrystalline cellulose, polysaccharide acids, cellulose ethers [e.g. methyl cellulose, ethyl cellulose, hydroxyethyl cellulose, hydroxypropyl cellulose, hydroxypropylmethyl cellulose or carboxymethylcellulose sodium]); alginic acid and salts thereof (e.g. sodium alginate); and sugar alcohols (e.g. xylitol, sorbitol or maltitol); or wherein the binder is a protein selected from gelatin; or wherein the binder is a gum (e.g. acacia gum, guar gum, or tragacanth gum); or wherein the binder is a silicate (e.g. magnesium aluminum silicate or bentonite); or wherein the binder is a synthetic polymer selected from the group consisting of polyvinylpyrrolidone, polyvinylpyrrolidone-vinylacetate copolymer, polymethacrylate polymer, polyvinylalcohol, and polyethylene glycol.

In a preferred embodiment of the administration unit according to the invention, the disintegrant is selected from the group consisting of cross-linked polymers (e.g. cross-linked polyvinylpyrrolidone [crospovidone] or cross-linked sodium carboxymethyl cellulose [croscarmellose sodium]), starches (e.g. corn or potato), pregelatinized starch, modified starch (e.g. sodium starch glycolate), algenic acid, alginates (e.g. sodium alginate), carboxymethylcellulose, microcrystalline cellulose, clays (e.g. magnesium aluminum silicate), and gums (e.g. agar, guar, locust bean, karaya, pectin, or tragacanth gum).

In a preferred embodiment of the administration unit according to the invention, the filler (or diluent) is selected from the group consisting of plant cellulose, microcrystalline cellulose, inorganic phosphates (e.g. dibasic calcium phosphate or tricalcium phosphate), inorganic carbonates such as calcium carbonate, inorganic sulfates such as calcium sulfate dihydrate, sugars and sugar alcohols (e.g. lactose, glucose, inositol, mannitol, sorbitol, sucrose, dextrose, maltodextrins, and fructose), magnesium stearate, calcium lactate trihydrate, starch (e.g. corn, wheat, maize, potato or rice starch), and modified starch (e.g. carboxymethyl starch).

In a preferred embodiment of the administration unit according to the invention, the lubricant is hydrophilic or hydrophobic. Preferably, the lubricant is selected from the group consisting of talc, silica, stearin, fatty acids (e.g. stearic acid) or fatty acid derivatives, metal stearates (e.g. magnesium stearate, calcium stearate, or zinc stearate), glyceryl monostearate, glyceryl palmitostearate, glyceryl behenate, sodium lauryl sulfate, magnesium lauryl sulfate, sodium stearyl fumarate, hydrogenated vegetable oil, polyalkylene glycols (e.g. polyethylene glycol), starch, light mineral oil, sodium benzoate, and sodium chloride.

In a preferred embodiment of the administration unit according to the invention, the glidant is selected from the group consisting of fumed silica, cornstarch, talc, and magnesium carbonate.

In a preferred embodiment of the administration unit according to the invention, the surfactant is selected from the group consisting of sodium lauryl sulfate, polyethylene-polypropylene glycol co-polymers, poly(oxyethylene)-poly(oxypropylene)-block-copolymers (e.g. poloxamers).

In a preferred embodiment of the administration unit according to the invention, the preservative is selected from the group consisting of antioxidants (e.g. vitamin A, vitamin C, vitamin E, retinyl palmitate, selenium, ascorbyl palmitate, sodium ascorbate), amino acids (e.g. cysteine or methionine), citric acid and its salts (e.g. sodium citrate), synthetic preservatives (e.g. parabens such as methyl paraben or propyl paraben; butylated hydroxyanisole, butylated hydroxytoluene, monothioglycerol, propyl gallate), hypophosphorous acid, sodium bisulfite, sodium formaldehyde sulfoxylate, and sodium metabisulfite.

In a preferred embodiment, the administration unit according to the invention passes the friability test according to Ph. Eur. Preferably, the weight loss in the friability test according to Ph. Eur. is not more than 2.0 % by weight, or not more than 1.5 % by weight, or not more than 1.0 % by weight, or not more than 0.5 % by weight, or not more than 0.4 % by weight, or not more than 0.3 % by weight, or not more than 0.2 % by weight, or not more than 0.1 % by weight.

In a preferred embodiment, the administration unit according to the invention passes the test uniformity of content of single-dose preparations according to Ph. Eur.

In a preferred embodiment, the administration unit according to the invention does not comprise any active pharmaceutical ingredient other than (i) {drug1}, (ii) {drug2}, (iii) {drug3}, (iv) {drug4}, (v) {drug5}, (vi) {drug6}, or (vii) {drug7}.

In a preferred embodiment, the administration unit according to the invention is for storage not above 50 °C. Preferably, it is for storage not above 25 °C.

In a preferred embodiment, the administration unit according to the invention is not identical with any of the administration units that are individualized in the experimental section of WO/2013/055584, WO/2013/059265, WO/2013/057722, WO/2013/057124, WO/2013/071272, WO/2013/068909, and WO/2013/074432, respectively, which is incorporated by reference.

The invention also relates to the administration unit according to the invention for use in the treatment of a disorder or a disease in a mammal. Preferably, the mammal is a human. Preferably, the mammal is an adult.

Preferably, the mammal does not suffer from liver damage and/or kidney damage.

Preferably, the mammal is not pregnant.

Preferably, the mammal is not allergic against (i) {drug1}, (ii) {drug2}, (iii) {drug3}, (iv) {drug4}, (v) {drug5}, (vi) {drug6}, or (vii) {drug7}.

In a preferred embodiment, the administration unit according to the invention is administered orally with a liquid. Preferably, the liquid is water, milk, juice or lemonade.

In a preferred embodiment of the administration unit according to the invention, the administered daily dose of (i) {drug1}, (ii) {drug2}, (iii) {drug3}, (iv) {drug4}, (v) {drug5}, (vi) {drug6}, or (vii) {drug7} is 0.0001 mg/kg body weight to 100 mg/kg body weight. Preferably, the administered daily dose of (i) {drug1}, (ii) {drug2}, (iii) {drug3}, (iv) {drug4}, (v) {drug5}, (vi) {drug6}, or (vii) {drug7} is 0.0001 mg/kg body weight to 0.0003 mg/kg body weight, or 0.0003 mg/kg body weight to 0.0005 mg/kg body weight, or 0.0005 mg/kg body weight to 0.001 mg/kg body weight, or 0.001 mg/kg body weight to 0.0025 mg/kg body weight, or 0.0025 mg/kg body weight to 0.005 mg/kg body weight, or 0.005 mg/kg body weight to 0.01 mg/kg body weight, or 0.01 mg/kg body weight to 0.025 mg/kg body weight, or 0.025 mg/kg body weight to 0.05 mg/kg body weight, or 0.05 mg/kg body weight to 0.1 mg/kg body weight, or 0.1 mg/kg body weight to 0.25 mg/kg body weight, or 0.25 mg/kg body weight to 0.5 mg/kg body weight, or 0.5 mg/kg body weight to 1 mg/kg body weight, or 1 mg/kg body weight to 2.5 mg/kg body weight, or 2.5 mg/kg body weight to 5 mg/kg body weight, or 5 mg/kg body weight to 10 mg/kg body weight, or 10 mg/kg body weight to 25 mg/kg body weight, or 25 mg/kg body weight to 50 mg/kg body weight, or 50 mg/kg body weight to 100 mg/kg body weight.

In a preferred embodiment of the administration unit according to the invention, the administered daily dose of (i) {drug1}, (ii) {drug2}, (iii) {drug3}, (iv) {drug4}, (v) {drug5}, (vi) {drug6}, or (vii) {drug7} is 0.001 mg to 5000 mg. Preferably, the administered daily dose of (i) {drug1}, (ii) {drug2}, (iii) {drug3}, (iv) {drug4}, (v) {drug5}, (vi) {drug6}, or (vii) {drug7} is 0.001 mg to 0.003 mg, or 0.003 mg to 0.005 mg, or 0.005 mg to 0.01 mg, or 0.01 mg to 0.025 mg, or 0.025 mg to 0.05 mg, or 0.05 mg to 0.1 mg, or 0.1 mg to 0.25 mg, or 0.25 mg to 0.5 mg, or 0.5 mg to 1 mg, or 1 mg to 2.5 mg, or 2.5 mg to 5 mg, or 5 mg to 10 mg, or 10 mg to 25 mg, or 25 mg to 50 mg, or 50 mg to 100 mg, or 100 mg to 250 mg, or 250 mg to 500 mg, or 500 mg to 1000 mg, or 1000 mg to 1250 mg, or 1250 mg to 1500 mg, or 1500 mg to 1750 mg, or 1750 mg to 2000 mg.

The invention also relates to a packaging comprising one or more administration units according to the invention.

In a preferred embodiment, the packaging according to the invention comprises a material selected from the group consisting of paper, cardboard, paperboard, metal foil and plastic foil.

In a preferred embodiment, the packaging according to the invention is a blister packaging.

A preferred blister packaging includes but is not limited to PVC-blister, PVDC-blister, PVC/PVDC-blister, moisture-proof packaging material such as aluminium foil blister pack, alu/alu blister, transparent or opaque polymer blister with pouch.

An alternative packaging according to the invention is a polypropylene tube, glass bottle and HDPE bottle optionally containing a child-resistant feature. The primary packaging material may comprise a desiccant such as molecular sieve or silica gel to improve chemical stability of (i) {drug1}, (ii) {drug2}, (iii) {drug3}, (iv) {drug4}, (v) {drug5}, (vi) {drug6}, or (vii) {drug7}. Opaque packaging such as colored blister materials, tubes, brown glass bottles or the like can be used to prolong shelflife of (i) {drug1}, (ii) {drug2}, (iii) {drug3}, (iv) {drug4}, (v) {drug5}, (vi) {drug6}, or (vii) {drug7} by reduction of photodegradation.

In a preferred embodiment, the packaging according to the invention comprises a multitude of at least 2, or at least 3, or at least 4, or at least 5, or at least 6, or at least 7, or at least 8, or at least 9, or at least 10 administration units according to the invention.

In a preferred embodiment of the packaging according to the invention, the administration units do not substantially differ in at least one property selected from the group consisting of nature of excipients, content of excipients, content of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, content of (i) {drug1}, (ii) {drug2}, (iii) {drug3}, (iv) {drug4}, (v) {drug5}, (vi) {drug6}, or (vii) {drug7}, water content, total weight, release profile, disintegration time, size, shape, porosity, apparent density, true density, average pore size, pore size distribution, axial tensile strength, radial tensile strength, friability, and storage stability. Preferably, the administration units differ in said at least one property by not more than 5 %, or not more than 4 %, or not more than 3 %, or not more than 2 %, or not more than 1 %, or not more than 0.5 %, or not more than 0.1 %. Preferably, all administration units are substantially identical.

The invention also relates to the packaging according to the invention for use in the treatment of a disorder or a disease, wherein one or more administration units of said multitude are administered on a first day, and one or more administration units of said multitude are administered on a second day following said first day.

In a preferred embodiment of the packaging according to the invention, one or more administration units of said multitude are administered on one or more subsequent days following said second day. Preferably, the time interval between the administration of an administration unit and the administration of a subsequent administration unit is not more than 48 hours. Preferably, the time interval is not more than 36 hours, or not more than 24 hours, or not more than 12 hours, or not more than 8 hours, or not more than 6 hours, or not more than 4 hours.

In a preferred embodiment of the packaging according to the invention, the administration units are administered on not more than 30 subsequent days. Preferably, the administration units are administered on not more than 25 subsequent days, or on not more than 20 subsequent days, or on not more than 14 subsequent days, or on not more than 10 subsequent days, or on not more than 7 subsequent days, or on not more than 3 subsequent days.

In a preferred embodiment of the packaging according to the invention, the administered daily dose of (i) {drug1}, (ii) {drug2}, (iii) {drug3}, (iv) {drug4}, (v) {drug5}, (vi) {drug6}, or (vii) {drug7} is 0.0001 mg/kg body weight to 100 mg/kg body weight. Preferably, the administered daily dose of (i) {drug1}, (ii) {drug2}, (iii) {drug3}, (iv) {drug4}, (v) {drug5}, (vi) {drug6}, or (vii) {drug7} is 0.0001 mg/kg body weight to 0.0003 mg/kg body weight, or 0.0003 mg/kg body weight to 0.0005 mg/kg body weight, or 0.0005 mg/kg body weight to 0.001 mg/kg body weight, or 0.001 mg/kg body weight to 0.0025 mg/kg body weight, or 0.0025 mg/kg body weight to 0.005 mg/kg body weight, or 0.005 mg/kg body weight to 0.01 mg/kg body weight, or 0.01 mg/kg body weight to 0.025 mg/kg body weight, or 0.025 mg/kg body weight to 0.05 mg/kg body weight, or 0.05 mg/kg body weight to 0.1 mg/kg body weight, or 0.1 mg/kg body weight to 0.25 mg/kg body weight, or 0.25 mg/kg body weight to 0.5 mg/kg body weight, or 0.5 mg/kg body weight to 1 mg/kg body weight, or 1 mg/kg body weight to 2.5 mg/kg body weight, or 2.5 mg/kg body weight to 5 mg/kg body weight, or 5 mg/kg body weight to 10 mg/kg body weight, or 10 mg/kg body weight to 25 mg/kg body weight, or 25 mg/kg body weight to 50 mg/kg body weight, or 50 mg/kg body weight to 100 mg/kg body weight.

In a preferred embodiment of the packaging according to the invention, the administered daily dose of (i) {drug1}, (ii) {drug2}, (iii) {drug3}, (iv) {drug4}, (v) {drug5}, (vi) {drug6}, or (vii) {drug7} is 0.001 mg to 5000 mg. Preferably, the administered daily dose of (i) {drug1}, (ii) {drug2}, (iii) {drug3}, (iv) {drug4}, (v) {drug5}, (vi) {drug6}, or (vii) {drug7} is 0.001 mg to 0.003 mg, or 0.003 mg to 0.005 mg, or 0.005 mg to 0.01 mg, or 0.01 mg to 0.025 mg, or 0.025 mg to 0.05 mg, or 0.05 mg to 0.1 mg, or 0.1 mg to 0.25 mg, or 0.25 mg to 0.5 mg, or 0.5 mg to 1 mg, or 1 mg to 2.5 mg, or 2.5 mg to 5 mg, or 5 mg to 10 mg, or 10 mg to 25 mg, or 25 mg to 50 mg, or 50 mg to 100 mg, or 100 mg to 250 mg, or 250 mg to 500 mg, or 500 mg to 1000 mg, or 1000 mg to 1250 mg, or 1250 mg to 1500 mg, or 1500 mg to 1750 mg, or 1750 mg to 2000 mg.

In a preferred embodiment, the packaging according to the invention does not comprise any administration unit comprising an active pharmaceutical ingredient other than (i) {drug1}, (ii) {drug2}, (iii) {drug3}, (iv) {drug4}, (v) {drug5}, (vi) {drug6}, or (vii) {drug7}.

The invention also relates to a method for manufacturing an administration unit according to the invention or a packaging according to the invention, comprising the steps:

(a) providing a quantity of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} that exceeds the dose of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} to be contained in a single administration unit by a factor of at least 10;
(b) mixing the quantity of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} with one or more pharmaceutical excipients;
(c) dividing the mixture obtained in step (b) in fractions each containing the dose of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} to be contained in a single administration unit;
(d) optionally, forming the fractions obtained in step (c) to administration units; and
(e) optionally, packing the fractions obtained in step (c) and the administration units obtained in step (d), respectively.
In a preferred embodiment of the method according to the invention, in step (a) the quantity of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} exceeds the dose of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} to be contained in a single administration unit by a factor of at least 25, or at least 50, or at least 100, or at least 250, or at least 500, or at least 1000, or at least 2500, or at least 5000, or at least 10,000.

The invention also relates to particles of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} having a particle size distribution X90 of about 500 μm or less, i.e. a particle size distribution (preferably by volume) such that at least 90 % of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} has a particle size smaller than 500 μm. Preferably, for (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} the particle size distribution of X90 is about 480 μm or less, or about 460 μm or less, or about 440 μm or less, or about 420 μm or less, or about 400 μm or less, or about 380 μm or less, or about 360 μm or less, or about 340 μm or less, or about 320 μm or less, or about 300 μm or less, or about 280 μm or less, or about 260 μm or less, or about 240 μm or less, or about 220 μm or less, or about 200 μm or less, or about 180 μm or less, or about 160 μm or less, or about 140 μm or less, or about 120 μm or less, or about 100 μm or less, or about 80 μm or less, or about 60 μm or less, or about 40 μm or less, or about 20 μm or less.

In a preferred embodiment, the particles of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} according to the invention have a particle size distribution X50 of about 400 μm or less, i.e. a particle size distribution (preferably by volume) such that at least 50 % of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} has a particle size smaller than 400 μm. Preferably, for (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} the particle size distribution of X50 is about 380 μm or less, or about 360 μm or less, or about 340 μm or less, or about 320 μm or less, or about 300 μm or less, or about 280 μm or less, or about 260 μm or less, or about 240 μm or less, or about 220 urn or less, or about 200 μm or less, or about 180 μm or less, or about 160 μm or less, or about 140 μm or less, or about 120 μm or less, or about 100 μm or less, or about 80 μm or less, or about 70 μm or less, or about 60 μm or less, or about 50 μm or less. Preferably, for (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} the particle size distribution of X50 is about 45 μm or less, or about 40 μm or less, or about 35 μm or less, or about 30 μm or less, or about 25 μm or less, or about 20 μm or less, or about 15 μm or less, or about 10 μm or less.

In preferred embodiments, the particles of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} have a cumulative distribution by volume basis Q3(X) (X in μm) of Q3(355) <0.50; or Q3(180) <0.50 and Q3(355) ≥0.50; or Q3(125) <0.50 and Q3(180) ≥0.50; or Q3(125) ≥0.50.

In preferred embodiments, the particles of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} have a cumulative distribution by volume basis Q3(X) (X in μm) of Q3(268) <0.50 and Q3(355) ≥0.50; or Q3(180) <0.50 and Q3(268) ≥0.50; or Q3(153) <0.50 and Q3(180) ≥0.50; or Q3(125) <0.50 and Q3(153) ≥0.50; or Q3(111) <0.50 and Q3(125) ≥0.50; or Q3(98) <0.50 and Q3(111) ≥0.50; or Q3(98) ≥0.50.

In preferred embodiments, the particles of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} have a cumulative distribution by volume basis Q3(X) (X in μm) of Q3(312) <0.50 and Q3(355) ≥0.50; or Q3(268) <0.50 and Q3(312) ≥0.50; or Q3(224) <0.50 and Q3(268) ≥0.50; or Q3(180) <0.50 and Q3(224) ≥0.50; or Q3(167) <0.50 and Q3(180) ≥0.50; or Q3(153) <0.50 and Q3(167) ≥0.50; or Q3(139) <0.50 and Q3(153) ≥0.50; or Q3(125) <0.50 and Q3(139) ≥0.50; or Q3(111) <0.50 and Q3(125) ≥0.50; or Q3(98) <0.50 and Q3(111) ≥0.50; or Q3(84) <0.50 and Q3(98) ≥0.50; or Q3(84) ≥0.50.

The indicated particle size properties are determined by laser-diffraction method, in particular low angle laser light scattering, i.e. Fraunhofer diffraction. Alternatively, the particle size properties can be also determined by microscopy (e.g. electron microscopy or scanning electron microscopy). The powder fineness is preferably determined in accordance with USP35 <811>. The results of the particle size distribution determined by different techniques can be correlated with one another.

Suitable milling and grinding techniques that are suitable for obtaining a specific particle size distribution are known to the skilled artisan (see e.g. N. Rasenack, B.W. Müller, Micron-size drug particles: common and novel micronization techniques, Pharm Dev Technol., 2004, 9(1):1-13; A. Martin, M.J. Cocero, Micronization processes with supercritical fluids: fundamentals and mechanisms, Adv Drug Deliv Rev. 2008, 60(3):339-50; S.C. Gad, Pharmaceutical Manufacturing Handbook: Production and Processes (Pharmaceutical Development Series), Wiley Interscience, 1st ed. 2008; A.J. Hickey, Pharmaceutical Process Engineering (Drugs and the Pharmaceutical Sciences), Informa Healthcare, 2nd ed. 2009; B. Nickerson, Sample Preparation of Pharmaceutical Dosage Forms: Challenges and Strategies for Sample Preparation and Extraction, Springer, 1st ed. 2011; D. Dragoman, Optical Characteriaztion of Solids, Springer,

1st ed. 2010; and D. Schulze; Powders and Bulk Solids: Behavior, Characterization, Storage and Flow, Springer, 2008).

PREFERRED EMBODIMENTS OF THE INVENTION ACCORDING TO ITEMS 1 TO 137

1. An administration unit comprising (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}.
2. The administration unit according to item 1, comprising a therapeutically effective amount of (i) {drug1}, (ii) {drug2}, (iii) {drug3}, (iv) {drug4}, (v) {drug5}, (vi) {drug6}, or (vii) {drug7}, wherein at least 10 % by weight of said therapeutically effective amount of (i) {drug1}, (ii) {drug2}, (iii) {drug3}, (iv) {drug4}, (v) {drug5}, (vi) {drug6}, or (vii) {drug7} are present as (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}.
3. The administration unit according to item 2, wherein at least 20 % by weight, or at least 30 % by weight, or at least 40 % by weight, or at least 50 % by weight, or at least 60 % by weight, or at least 70 % by weight, or at least 80 % by weight, or at least 90 % by weight of said therapeutically effective amount of (i) {drug1}, (ii) {drug2}, (iii) {drug3}, (iv) {drug4}, (v) {drug5}, (vi) {drug6}, or (vii) {drug7} are present as (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}.
4. The administration unit according to any of items 2 to 3, wherein at least 95 % by weight, or at least 96 % by weight, or at least 97 % by weight, or at least 98 % by weight, or at least 99 % by weight, or at least 99.5 % by weight, or at least 99.8 % by weight, or at least 99.9 % by weight of said therapeutically effective amount of (i) {drug1}, (ii) {drug2}, (iii) {drug3}, (iv) {drug4}, (v) {drug5}, (vi) {drug6}, or (vii) {drug7} are present as (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}.
5. The administration unit according to any of items 2 to 4, wherein not more than 90 % by weight of said therapeutically effective amount of (i) {drug1}, (ii) {drug2}, (iii) {drug3}, (iv) {drug4}, (v) {drug5}, (vi) {drug6}, or (vii) {drug7} are amorphous.
6. The administration unit according to item 5, wherein not more than 80 % by weight, or not more than 70 % by weight, or not more than 60 % by weight, or not more than 50 % by weight, or not more than 40 % by weight, or not more than 30 % by weight, or not more than 20 % by weight, or not more than 10 % by weight of said therapeutically effective amount of (i) {drug1}, (ii) {drug2}, (iii) {drug3}, (iv) {drug4}, (v) {drug5}, (vi) {drug6}, or (vii) {drug7} are amorphous.
7. The administration unit according to item 5 or 6, wherein not more than 5 % by weight, or not more than 4 % by weight, or not more than 3 % by weight, or not more than 2 % by weight, or not more than 1 % by weight, or not more than 0.5 % by weight, or not more than 0.2 % by weight, or not more than 0.1 % by weight of said therapeutically effective amount of (i) {drug1}, (ii) {drug2}, (iii) {drug3}, (iv) {drug4}, (v) {drug5}, (vi) {drug6}, or (vii) {drug7} are amorphous.
8. The administration unit according to any of items 2 to 7, wherein not more than 90 % by weight of said therapeutically effective amount of (i) {drug1}, (ii) {drug2}, (iii) {drug3}, (iv) {drug4}, (v) {drug5}, (vi) {drug6}, or (vii) {drug7} are polymorphs of (i) {drug1}, (ii) {drug2}, (iii) {drug3}, (iv) {drug4}, (v) {drug5}, (vi) {drug6}, or (vii) {drug7} other than (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}.
9. The administration unit according to item 8, wherein not more than 80 % by weight, or not more than 70 % by weight, or not more than 60 % by weight, or not more than 50 % by weight, or not more than 40 % by weight, or not more than 30 % by weight, or not more than 20 % by weight, or not more than 10 % by weight of said therapeutically effective amount of (i) {drug1}, (ii) {drug2}, (iii) {drug3}, (iv) {drug4}, (v) {drug5}, (vi) {drug6}, or (vii) {drug7} are polymorphs of (i) {drug1}, (ii) {drug2}, (iii) {drug3}, (iv) {drug4}, (v) {drug5}, (vi) {drug6}, or (vii) {drug7} other than (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}.
10. The administration unit according to item 8 or 9, wherein not more than 5 % by weight, or not more than 4 % by weight, or not more than 3 % by weight, or not more than 2 % by weight, or not more than 1 % by weight, or not more than 0.5 % by weight, or not more than 0.2 % by weight, or not more than 0.1 % by weight of said therapeutically effective amount of (i) {drug1}, (ii) {drug2}, (iii) {drug3}, (iv) {drug4}, (v) {drug5}, (vi) {drug6}, or (vii) {drug7} are polymorphs of (i) {drug1}, (ii) {drug2}, (iii) {drug3}, (iv) {drug4}, (v) {drug5}, (vi) {drug6}, or (vii) {drug7} other than (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}.

11. The administration unit according to any of items 2 to 10, wherein the therapeutically effective amount of (i) {drug1}, (ii) {drug2}, (iii) {drug3}, (iv) {drug4}, (v) {drug5}, (vi) {drug6}, or (vii) {drug7} is 0.1 μg to 2 g of (i) {drug1}, (ii) {drug2}, (iii) {drug3}, (iv) {drug4}, (v) {drug5}, (vi) {drug6}, or (vii) {drug7}.

12. The administration unit according to item 11, wherein the therapeutically effective amount of (i) {drug1}, (ii) {drug2}, (iii) {drug3}, (iv) {drug4}, (v) {drug5}, (vi) {drug6}, or (vii) {drug7} is 1 μg to 2.5 μg of (i) {drug1}, (ii) {drug2}, (iii) {drug3}, (iv) {drug4}, (v) {drug5}, (vi) {drug6}, or (vii) {drug7}, or 2.5 μg to 5 μg of (i) {drug1}, (ii) {drug2}, (iii) {drug3}, (iv) {drug4}, (v) {drug5}, (vi) {drug6}, or (vii) {drug7}, or 5 μg to 10 μg of (i) {drug1}, (ii) {drug2}, (iii) {drug3}, (iv) {drug4}, (v) {drug5}, (vi) {drug6}, or (vii) {drug7}, or 10 μg to 25 μg of (i) {drug1}, (ii) {drug2}, (iii) {drug3}, (iv) {drug4}, (v) {drug5}, (vi) {drug6}, or (vii) {drug7}, or 25 μg to 50 μg of (i) {drug1}, (ii) {drug2}, (iii) {drug3}, (iv) {drug4}, (v) {drug5}, (vi) {drug6}, or (vii) {drug7}, or 50 μg to 100 μg of (i) {drug1}, (ii) {drug2}, (iii) {drug3}, (iv) {drug4}, (v) {drug5}, (vi) {drug6}, or (vii) {drug7}, or 100 μg to 250 μg of (i) {drug1}, (ii) {drug2}, (iii) {drug3}, (iv) {drug4}, (v) {drug5}, (vi) {drug6}, or (vii) {drug7}, or 250 μg to 500 μg of (i) {drug1}, (ii) {drug2}, (iii) {drug3}, (iv) {drug4}, (v) {drug5}, (vi) {drug6}, or (vii) {drug7}, or 500 μg to 1 mg of (i) {drug1}, (ii) {drug2}, (iii) {drug3}, (iv) {drug4}, (v) {drug5}, (vi) {drug6}, or (vii) {drug7}, or 1 mg to 2.5 mg of (i) {drug1}, (ii) {drug2}, (iii) {drug3}, (iv) {drug4}, (v) {drug5}, (vi) {drug6}, or (vii) {drug7}, or 2.5 mg to 5 mg of (i) {drug1}, (ii) {drug2}, (iii) {drug3}, (iv) {drug4}, (v) {drug5}, (vi) {drug6}, or (vii) {drug7}, or 5 mg to 10 mg of (i) {drug1}, (ii) {drug2}, (iii) {drug3}, (iv) {drug4}, (v) {drug5}, (vi) {drug6}, or (vii) {drug7}, or 10 mg to 25 mg of (i) {drug1}, (ii) {drug2}, (iii) {drug3}, (iv) {drug4}, (v) {drug5}, (vi) {drug6}, or (vii) {drug7}, or 25 mg to 50 mg of (i) {drug1}, (ii) {drug2}, (iii) {drug3}, (iv) {drug4}, (v) {drug5}, (vi) {drug6}, or (vii) {drug7}, or 50 mg to 100 mg of (i) {drug1}, (ii) {drug2}, (iii) {drug3}, (iv) {drug4}, (v) {drug5}, (vi) {drug6}, or (vii) {drug7}, or 100 mg to 250 mg of (i) {drug1}, (ii) {drug2}, (iii) {drug3}, (iv) {drug4}, (v) {drug5}, (vi) {drug6}, or (vii) {drug7}, or 250 mg to 500 mg of (i) {drug1}, (ii) {drug2}, (iii) {drug3}, (iv) {drug4}, (v) {drug5}, (vi) {drug6}, or (vii) {drug7}, or 500 mg to 1 g of (i) {drug1}, (ii) {drug2}, (iii) {drug3}, (iv) {drug4}, (v) {drug5}, (vi) {drug6}, or (vii) {drug7}, or 1 g to 1.25 g of (i) {drug1}, (ii) {drug2}, (iii) {drug3}, (iv) {drug4}, (v) {drug5}, (vi) {drug6}, or (vii) {drug7}, or 1.25 g to 1.5 g of (i) {drug1}, (ii) {drug2}, (iii) {drug3}, (iv) {drug4}, (v) {drug5}, (vi) {drug6}, or (vii) {drug7}, or 1.5 g to 1.75 g of (i) {drug1}, (ii) {drug2}, (iii) {drug3}, (iv) {drug4}, (v) {drug5}, (vi) {drug6}, or (vii) {drug7}, or 1.75 g to 2 g of (i) {drug1}, (ii) {drug2}, (iii) {drug3}, (iv) {drug4}, (v) {drug5}, (vi) {drug6}, or (vii) {drug7}.

13. The administration unit according to any of items 1 to 12, comprising 0.1 μg to 2 g of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}.

14. The administration unit according to item 13, comprising 1 μg to 2.5 μg of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, or 2.5 μg to 5 μg of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, or 5 μg to 10 μg of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, or 10 μg to 25 μg of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, or 25 μg to 50 μg of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, or 50 μg to 100 μg of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, or 100 μg to 250 μg of(i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, or 250 μg to 500 μg of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, or 500 μg to 1 mg of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, or 1 mg to 2.5 mg of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, or 2.5 mg to 5 mg of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, or 5 mg to 10 mg of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, or 10 mg to 25 mg of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, or 25 mg to 50 mg of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a},

or 50 mg to 100 mg of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, or 100 mg to 250 mg of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, or 250 mg to 500 mg of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, or 500 mg to 1 g of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, or 1 g to 1.25 g of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, or 1.25 gto 1.5 g of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, or 1.5 gto 1.75 g of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, or 1.75 g to 2 g of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}.

15. The administration unit according to any of items 1 to 14, which is solid, semisolid or liquid.

16. The administration unit according to any of items 1 to 15, which is for administration once daily, or twice daily, or thrice daily, or four times daily, or five times daily, or six times daily.

17. The administration unit according to any of items 1 to 16, which is monolithic or multiparticulate.

18. The administration unit according to any of items 1 to 17, wherein (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} is homogeneously distributed over the administration unit.

19. The administration unit according to any of items 1 to 17, wherein (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} is not homogeneously distributed over the administration unit.

20. The administration unit according to any of items 1 to 19, providing controlled release of (i) {drug1}, (ii) {drug2}, (iii) {drug3}, (iv) {drug4}, (v) {drug5}, (vi) {drug6}, or (vii) {drug7}.

21. The administration unit according to item 20, comprising a controlled release matrix or a controlled release coating.

22. The administration unit according to any of items 1 to 19, providing immediate release of (i) {drug1}, (ii) {drug2}, (iii) {drug3}, (iv) {drug4}, (v) {drug5}, (vi) {drug6}, or (vii) {drug7}.

23. The administration unit according to any of items 1 to 22, having a total weight of 10 mg to 3 g.

24. The administration unit according to item 23, having a total weight of 10 to 25 mg, or 25 mg to 50 mg, or 50 mg to 100 mg, or 100 mg to 250 mg, or 250 mg to 500 mg, or 500 mg to 1000 mg, or 1000 mg to 2500 mg.

25. The administration unit according to any of items 1 to 24, which is for systemic or topical administration.

26. The administration unit according to any of items 1 to 25, which is for parenteral administration.

27. The administration unit according to any of items 1 to 26, which is for oral, buccal, opthalmic, nasal, rectal, transmucosal, or intestinal administration.

28. The administration unit according to any of items 1 to 27, which is for oral administration.

29. The administration unit according to item 28, which is selected from the group consisting of tablets, capsules, effervescent tablets, orodispersible tablets, dragees, sachets, drops, suspensions, and powders.

30. The administration unit according to any of items 1 to 29, which is a tablet.

31. The administration unit according to item 30, which is round or oblong; and/or which is planar or biconvex.

32. The administration unit according to any of items 30 to 31, having been manufactured by direct compression.

33. The administration unit according to any of items 30 to 31, having been manufactured by granulation and subsequent compression of granules.

34. The administration unit according to item 33, wherein granulation is wet granulation or dry granulation.

35. The administration unit according to any of items 30 to 34, having been compacted at a pressure of 10 MPa to 10,000 MPa.

36. The administration unit according to item 35, having been compacted at a pressure of 10 MPa to 25 MPa, or 25 MPa to 50 MPa, or 50 MPa to 100 MPa, or 100 MPa to 250 MPa, or 250 MPa to 500 MPa, or 500 MPa to 1000 MPa, or 1000 MPa to 2500 MPa, or 2500 MPa to 5000 MPa, or 5000 MPa to 10,000 MPa.

37. The administration unit according to any of items 30 to 36, comprising a film coating.

38. The administration unit according to item 37, wherein the film coating is enteric.

39. The administration unit according to any of items 37 to 38, wherein the film coating comprises a cellulose ether (e.g. hydroxypropylmethyl cellulose) or a synthetic polymer (e.g. polyvinyl alcohol).

40. The administration unit according to any of items 37 to 39, wherein the film coating has an average thickness of 0.01 to 1000 μm.

41. The administration unit according to item 40, wherein the film coating has an average thickness of 0.01 μm to 0.03 μm, or 0.03 to 0.05 μm, or 0.05 to 0.1 μm, or 0.1 to 0.25 μm, or 0.25 to 0.5 μm, or 0.5 to 1.0 μm, or 1.0 μm to 2.5 μm, or 2.5 μm to 5.0 μm, or 5.0 μm to 10 μm, or 10 μm to 25 μm, or 25 μm to 50 μm, or 50 μm to 100 μm, or 100 μm to 250 μm, or 250 μm to 500 μm, or 500 μm to 1000 μm.
42. The administration unit according to any of items 30 to 41, having a tablet porosity of not more than 90 %.
43. The administration unit according to item 42, having a tablet porosity of not more than 80 %, or not more than 70 %, or not more than 60 %, or not more than 50 %, or not more than 40 %, or not more than 30 %, or not more than 20 %, or not more than 10 %.
44. The administration unit according to item 42 to 43, having a tablet porosity of not more than 5 %, or not more than 4 %, or not more than 3 %, or not more than 2 %, or not more than 1 %, or not more than 0.5 %, or not more than 0.2 %, or not more than 0.1 %.
45. The administration unit according to any of items 30 to 44, having a radial tensile strength of 0.1 to 100 MPa.
46. The administration unit according to item 45, having a radial tensile strength of 0.1 MPa to 0.3 MPa, or 0.3 MPa to 0.5 MPa, or 0.5 MPa to 1.0 MPa, or 1.0 MPa to 2.5 MPa, or 2.5 MPa to 5.0 MPa, or 5.0 MPa to 10 MPa, or 10 MPa to 25 MPa, or 25 MPa to 50 MPa, or 50 MPa to 100 MPa.
47. The administration unit according to any of items 30 to 46, having an axial tensile strength of 0.1 to 100 MPa.
48. The administration unit according to item 47, having an axial tensile strength of 0.1 MPa to 0.3 MPa, or 0.3 MPa to 0.5 MPa, or 0.5 MPa to 1.0 MPa, or 1.0 MPa to 2.5 MPa, or 2.5 MPa to 5.0 MPa, or 5.0 MPa to 10 MPa, or 10 MPa to 25 MPa, or 25 MPa to 50 MPa, or 50 MPa to 100 MPa.
49. The administration unit according to any of items 30 to 48, having an average pore size of 0.001 μm to 1000 μm.
50. The administration unit according to item 49, having an average pore size of 0.001 μm to 0.003 μm, or 0,003 μm to 0.005 μm, or 0.005 μm to 0.01 μm, or 0.01 μm to 0.025 μm, or 0.025 μm to 0.05 μm, or 0.05 μm to 0.1 μm, or 0.1 μm to 0.25 μm, or 0.25 μm to 0.5 μm, or 0.5 μm to 1 μm, or 1 μm to 2.5 μm, or 2.5 μm to 5 μm, or 5 μm to 10 μm, or 10 μm to 25 μm, or 25 μm to 50 μm, or 50 μm to 100 μm, or 100 μm to 250 μm, or 250 μm to 500 μm, or 500 μm to 1000 μm.
51. The administration unit according to any of items 30 to 50, having a pore size distribution such that at least 10 % of the pores have a pore size of not more than 0.1 μm.
52. The administration unit according to item 51, having a pore size distribution such that at least 20 %, or at least 30 %, or at least 40 %, or at least 50 %, or at least 60 %, or at least 70 %, or at least 80 %, or at least 90 % of the pores have a pore size of not more than 0.1 μm.
53. The administration unit according to any of items 30 to 52, having a pore size distribution such that at least 10 % of the pores have a pore size of not less than 0.1 μm.
54. The administration unit according to item 53, having a pore size distribution such that at least 20 %, or at least 30 %, or at least 40 %, or at least 50 %, or at least 60 %, or at least 70 %, or at least 80 %, or at least 90 % of the pores have a pore size of not less than 0.1 μm.
55. The administration unit according to any of items 30 to 54, having a pore size distribution such that at least 10 % of the pores have a pore size of not more than 1 μm.
56. The administration unit according to item 55, having a pore size distribution such that at least 20 %, or at least 30 %, or at least 40 %, or at least 50 %, or at least 60 %, or at least 70 %, or at least 80 %, or at least 90 % of the pores have a pore size of not more than 1 μm.
57. The administration unit according to any of items 30 to 56, having a pore size distribution such that at least 10 % of the pores have a pore size of not less than 1 μm.
58. The administration unit according to item 57, having a pore size distribution such that at least 20 %, or at least 30 %, or at least 40 %, or at least 50 %, or at least 60 %, or at least 70 %, or at least 80 %, or at least 90 % of the pores have a pore size of not less than 1 μm.
59. The administration unit according to any of items 30 to 58, having a pore size distribution such that at least 10 % of the pores have a pore size of not more than 10 μm.
60. The administration unit according to item 59, having a pore size distribution such that at least 20 %, or at least 30 %, or at least 40 %, or at least 50 %, or at least 60 %, or at least 70 %, or at least 80 %, or at least 90 % of the pores have a pore size of not more than 10 μm.
61. The administration unit according to any of items 30 to 60, having a pore size distribution such that at least 10 % of the pores have a pore size of not less than 10 μm.
62. The administration unit according to item 61, having a pore size distribution such that at least 20 %, or at least 30 %, or at least 40 %, or at least 50 %, or at least 60 %, or at least 70 %, or at least 80 %, or at least 90 % of the pores have a pore size of not less than 10 μm.
63. The administration unit according to any of items 30 to 62, having a water content of not more than 5 % by weight, relative to the total weight of the tablet.
64. The administration unit according to item 63, having a water content of not more than 4 % by weight, or not more

than 3 % by weight, or not more than 2 % by weight, or not more than 1 % by weight, or not more than 0.5 % by weight, or not more than 0.1 % by weight, relative to the total weight of the tablet.

65. The administration unit according to any of items 30 to 64, having a true density of 0.60 to 1.40 g cm-3.

66. The administration unit according to item 65, having a true density of 0.60 to 0.65 g cm-3, or 0.65 to 0.70 g cm-3, or 0.70 to 0.75 g cm-3, or 0.75 to 0.80 g cm-3, or 0.80 to 0.85 g cm-3, or 0.85 to 0.90 g cm-3, or 0.90 to 0.95 g cm-3, or 0.95 to 1.00 g cm-3, or 1.00 to 1.05 g cm-3, or 1.05 to 1.10 g cm-3, or 1.10 to 1.15 g cm-3, or 1.15 to 1.20 g cm-3, or 1.20 to 1.25 g cm-3, or 1.25 to 1.30 g cm-3, or 1.30 to 1.35 g cm-3, or 1.35 to 1.40 g cm-3.

67. The administration unit according to any of items 30 to 66, having an apparent density of 0.60 to 1.40 g cm-3.

68. The administration unit according to item 67, having an apparent density of 0.60 to 0.65 g cm-3, or 0.65 to 0.70 g cm-3, or 0.70 to 0.75 g cm-3, or 0.75 to 0.80 g cm-3, or 0.80 to 0.85 g cm-3, or 0.85 to 0.90 g cm-3, or 0.90 to 0.95 g cm-3, or 0.95 to 1.00 g cm-3, or 1.00 to 1.05 g cm-3, or 1.05 to 1.10 g cm-3, or 1.10 to 1.15 g cm-3, or 1.15 to 1.20 g cm-3, or 1.20 to 1.25 g cm-3, or 1.25 to 1.30 g cm-3, or 1.30 to 1.35 g cm-3, or 1.35 to 1.40 g cm-3.

69. The administration unit according to any of items 30 to 68, disintegrating within 6000 seconds.

70. The administration unit according to item 69, disintegrating within 5500 seconds, or within 5000 seconds, or within 4500 seconds, or within 4000 seconds, or within 3500 seconds, or within 3000 seconds, or within 2500 seconds, or within 2000 seconds, or within 1500 seconds, or within 1000 seconds, or within 750 seconds, or within 500 seconds, or within 250 seconds.

71. The administration unit according to any of items 1 to 29, which is a capsule.

72. The administration unit according to item 71, comprising a multitude of particulates comprising (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}.

73. The administration unit according to any of items 71 to 72, comprising at least 2 particulates, or at least 3 particulates, or at least 4 particulates, or at least 5 particulates, or at least 6 particulates, or at least 7 particulates, or at least 8 particulates, or at least 9 particulates, or at least 10 particulates.

74. The administration unit according to any of items 71 to 73, wherein the capsule material comprises hard gelatine.

75. The administration unit according to any of items 71 to 74, comprising at least 2 different types of particulates that differ from each other in at least one property selected from the group consisting of nature of excipients, content of excipients, content of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a}, content of (i) {drug1}, (ii) {drug2}, (iii) {drug3}, (iv) {drug4}, (v) {drug5}, (vi) {drug6}, or (vii) {drug7}, water content, total weight, size, and shape.

76. The administration unit according to any of items 1 to 75, passing the storage stability test according to Ph. Eur. under accelerated storage conditions at 40°C and 75 % rel. humidity.

77. The administration unit according to item 76, having a storage stability for at least 3 months, or at least 4 months, or at least 5 months, or at least 6 months, or at least 7 months, or at least 8 months, or at least 9 months, or at least 10 months, or at least 11 months, or at least 12 months according to Ph. Eur. under accelerated storage conditions at 40°C and 75 % rel. humidity.

78. The administration unit according to any of items 1 to 77, providing the peak plasma level within 0.1 hour to 36 hours after administration.

79. The administration unit according to item 78, providing the peak plasma level within 0.1 hour to 1 hour, or 1 hour to 2 hours, or 2 hours to 3 hours, or 3 hours to 4 hours, or 4 hours to 5 hours, or 5 hours to 6 hours, or 6 hours to 7 hours, or 7 hours to 8 hours, or 8 hours to 9 hours, or 9 hours to 10 hours, or 10 hours to 11 hours, or 11 hours to 12 hours, or 12 hours to 13 hours, or 13 hours to 14 hours, or 14 hours to 15 hours, or 15 hours to 16 hours, or 16 hours to 17 hours, or 17 hours to 18 hours, or 18 hours to 19 hours, or 19 hours to 20 hours, or 20 hours to 21 hours, or 21 hours to 22 hours, or 22 hours to 23 hours, or 23 hours to 24 hours, after administration.

80. The administration unit according to any of items 1 to 79, comprising one or more excipients independently selected from the group consisting of antiadherents, binders, disintegrants, fillers, flavors, colors, lubricants, glidants, sorbents, surfactants, preservatives and sweeteners.

81. The administration unit according to item 80, wherein the antiadherent is selected from the group consisting of silicon dioxide, talc, talc waxes, stearates (e.g. magnesium, calcium or sodium), stearic acid, stearowet, boric acid, sodium chloride, DL-leucine, sodium oleate, sodium benzoate, sodium acetate, sodium lauryl sulfate, and magnesium lauryl sulfate.

82. The administration unit according to any of items 80 to 81, wherein the binder is selected from the group consisting of saccharides and their derivatives; proteins; gums; silicates; and synthetic polymers.

83. The administration unit according to item 82, wherein the binder is a saccharide or a derivative thereof selected from the group consisting of disaccharides (e.g. sucrose, glucose or lactose); polysaccharides and their derivatives (e.g. starch, pregelatinized starch, cellulose, microcrystalline cellulose, polysaccharide acids, cellulose ethers [e.g. methyl cellulose, ethyl cellulose, hydroxyethyl cellulose, hydroxypropyl cellulose, hydroxypropylmethyl cellulose or

carboxymethylcellulose sodium]); alginic acid and salts thereof (e.g. sodium alginate); and sugar alcohols (e.g. xylitol, sorbitol or maltitol); or wherein the binder is a protein selected from gelatin; or wherein the binder is a gum (e.g. acacia gum, guar gum, or tragacanth gum); or wherein the binder is a silicate (e.g. magnesium aluminum silicate or bentonite); or wherein the binder is a synthetic polymer selected from the group consisting of polyvinylpyrrolidone, polyvinylpyrrolidone-vinylacetate copolymer, polymethacrylate polymer, polyvinylalcohol, and polyethylene glycol.

84. The administration unit according to any of items 80 to 83, wherein the disintegrant is selected from the group consisting of cross-linked polymers (e.g. cross-linked polyvinylpyrrolidone [crospovidone] or cross-linked sodium carboxymethyl cellulose [croscarmellose sodium]), starches (e.g. corn or potato), pregelatinized starch, modified starch (e.g. sodium starch glycolate), algenic acid, alginates (e.g. sodium alginate), carboxymethylcellulose, microcrystalline cellulose, clays (e.g. magnesium aluminum silicate), and gums (e.g. agar, guar, locust bean, karaya, pectin, or tragacanth gum).

85. The administration unit according to any of items 80 to 84, wherein the filler is selected from the group consisting of plant cellulose, microcrystalline cellulose, inorganic phosphates (e.g. dibasic calcium phosphate or tricalcium phosphate), inorganic carbonates such as calcium carbonate, inorganic sulfates such as calcium sulfate dihydrate, sugars and sugar alcohols (e.g. lactose, glucose, inositol, mannitol, sorbitol, sucrose, dextrose, maltodextrins, and fructose), magnesium stearate, calcium lactate trihydrate, starch (e.g. corn, wheat, maize, potato or rice starch), and modified starch (e.g. carboxymethyl starch).

86. The administration unit according to any of items 80 to 85, wherein the lubricant is hydrophilic or hydrophobic.

87. The administration unit according to item 86, wherein the lubricant is selected from the group consisting of talc, silica, stearin, fatty acids (e.g. stearic acid) or fatty acid derivatives, metal stearates (e.g. magnesium stearate, calcium stearate, or zinc stearate), glyceryl monostearate, glyceryl palmitostearate, glyceryl behenate, sodium lauryl sulfate, magnesium lauryl sulfate, sodium stearyl fumarate, hydrogenated vegetable oil, polyalkylene glycols (e.g. polyethylene glycol), starch, light mineral oil, sodium benzoate, and sodium chloride.

88. The administration unit according to any of items 80 to 87, wherein the glidant is selected from the group consisting of fumed silica, cornstarch, talc, and magnesium carbonate.

89. The administration unit according to any of items 80 to 88, wherein the preservative is selected from the group consisting of antioxidants (e.g. vitamin A, vitamin C, vitamin E, retinyl palmitate, selenium, ascorbyl palmitate, sodium ascorbate), amino acids (e.g. cysteine or methionine), citric acid and its salts (e.g. sodium citrate), synthetic preservatives (e.g. parabens such as methyl paraben or propyl paraben; butylated hydroxyanisole, butylated hydroxytoluene, monothioglycerol, propyl gallate), hypophosphorous acid, sodium bisulfite, sodium formaldehyde sulfoxylate, and sodium metabisulfite.

90. The administration unit according to any of items 1 to 89, passing the friability test according to Ph. Eur.

91. The administration unit according to item 90, wherein the weight loss in the friability test according to Ph. Eur. is not more than 2.0 % by weight, or not more than 1.5 % by weight, or not more than 1.0 % by weight, or not more than 0.5 % by weight, or not more than 0.4 % by weight, or not more than 0.3 % by weight, or not more than 0.2 % by weight, or not more than 0.1 % by weight.

92. The administration unit according to any of items 1 to 91, passing the test uniformity of content of single-dose preparations according to Ph. Eur.

93. The administration unit according to any of items 1 to 92, not comprising any active pharmaceutical ingredient other than (i) {drug1}, (ii) {drug2}, (iii) {drug3}, (iv) {drug4}, (v) {drug5}, (vi) {drug6}, or (vii) {drug7}.

94. The administration unit according to any of items 1 to 93, which is for storage not above 50 °C.

95. The administration unit according to item 94, which is for storage not above 25 °C.

96. The administration unit according to any of items 1 to 95, with the proviso that the administration unit is not identical with any of the administration units that are individualized in the experimental section of WO/2013/055584, WO/2013/059265, WO/2013/057722, WO/2013/057124, WO/2013/071272, WO/2013/068909, and WO/2013/074432, respectively.

97. The administration unit according to any of items 1 to 96 for use in the treatment of a disorder or a disease in a mammal.

98. The administration unit according to item 97, wherein the mammal is a human.

99. The administration unit according to any of items 97 to 98, wherein the mammal is an adult.

100. The administration unit according to any of items 97 to 99 wherein the mammal does not suffer from liver damage.

101. The administration unit according to any of items 97 to 100, wherein the mammal does not suffer from kidney damage.

102. The administration unit according to any of items 97 to 101, wherein the mammal is not pregnant.

103. The administration unit according to any of items 97 to 102, wherein the mammal is not allergic against (i) {drug1}, (ii) {drug2}, (iii) {drug3}, (iv) {drug4}, (v) {drug5}, (vi) {drug6}, or (vii) {drug7}.

104. The administration unit according to any of items 97 to 103, which is administered orally with a liquid.

105. The administration unit according to item 104, wherein the liquid is water, milk, juice or lemonade.
106. The administration unit according to any of items 97 to 105, wherein the administered daily dose of (i) {drug1}, (ii) {drug2}, (iii) {drug3}, (iv) {drug4}, (v) {drug5}, (vi) {drug6}, or (vii) {drug7} is 0.0001 mg/kg body weight to 100 mg/kg body weight.
107. The administration unit according to item 106, wherein the administered daily dose of (i) {drug1}, (ii) {drug2}, (iii) {drug3}, (iv) {drug4}, (v) {drug5}, (vi) {drug6}, or (vii) {drug7} is 0.0001 mg/kg body weight to 0.0003 mg/kg body weight, or 0.0003 mg/kg body weight to 0.0005 mg/kg body weight, or 0.0005 mg/kg body weight to 0.001 mg/kg body weight, or 0.001 mg/kg body weight to 0.0025 mg/kg body weight, or 0.0025 mg/kg body weight to 0.005 mg/kg body weight, or 0.005 mg/kg body weight to 0.01 mg/kg body weight, or 0.01 mg/kg body weight to 0.025 mg/kg body weight, or 0.025 mg/kg body weight to 0.05 mg/kg body weight, or 0.05 mg/kg body weight to 0.1 mg/kg body weight, or 0.1 mg/kg body weight to 0.25 mg/kg body weight, or 0.25 mg/kg body weight to 0.5 mg/kg body weight, or 0.5 mg/kg body weight to 1 mg/kg body weight, or 1 mg/kg body weight to 2.5 mg/kg body weight, or 2.5 mg/kg body weight to 5 mg/kg body weight, or 5 mg/kg body weight to 10 mg/kg body weight, or 10 mg/kg body weight to 25 mg/kg body weight, or 25 mg/kg body weight to 50 mg/kg body weight, or 50 mg/kg body weight to 100 mg/kg body weight.
108. The administration unit according to any of items 97 to 107, wherein the administered daily dose of (i) {drug1}, (ii) {drug2}, (iii) {drug3}, (iv) {drug4}, (v) {drug5}, (vi) {drug6}, or (vii) {drug7} is 0.001 mg to 5000 mg.
109. The administration unit according to item 108, wherein the administered daily dose of (i) {drug1}, (ii) {drug2}, (iii) {drug3}, (iv) {drug4}, (v) {drug5}, (vi) {drug6}, or (vii) {drug7} is 0.001 mg to 0.003 mg, or 0.003 mg to 0.005 mg, or 0.005 mg to 0.01 mg, or 0.01 mg to 0.025 mg, or 0.025 mg to 0.05 mg, or 0.05 mg to 0.1 mg, or 0.1 mg to 0.25 mg, or 0.25 mg to 0.5 mg, or 0.5 mg to 1 mg, or 1 mg to 2.5 mg, or 2.5 mg to 5 mg, or 5 mg to 10 mg, or 10 mg to 25 mg, or 25 mg to 50 mg, or 50 mg to 100 mg, or 100 mg to 250 mg, or 250 mg to 500 mg, or 500 mg to 1000 mg, or 1000 mg to 1250 mg, or 1250 mg to 1500 mg, or 1500 mg to 1750 mg, or 1750 mg to 1999 mg.
110. A packaging comprising one or more administration units according to any of items 1 to 109.
111. The packaging according to item 110, comprising a material selected from the group consisting of paper, cardboard, paperboard, metal foil and plastic foil.
112. The packaging according to any of items 110 to 111, which is a blister packaging.
113. The packaging according to any of items 110 to 112, comprising a multitude of at least 2, or at least 3, or at least 4, or at least 5, or at least 6, or at least 7, or at least 8, or at least 9, or at least 10 administration units according to any of items 1 to 109.
114. The packaging according to item 113, wherein the administration units do not substantially differ in at least one property selected from the group consisting of nature of excipients, content of excipients, content of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or(vii) {drug7a}, content of (i) {drug1}, (ii) {drug2}, (iii) {drug3}, (iv) {drug4}, (v) {drug5}, (vi) {drug6}, or (vii) {drug7}, water content, total weight, release profile, disintegration time, size, shape, porosity, apparent density, true density, average pore size, pore size distribution, axial tensile strength, radial tensile strength, friability, and storage stability.
115. The packaging according to item 114, wherein the administration units differ in said at least one property by not more than 5 %, or not more than 4 %, or not more than 3 %, or not more than 2 %, or not more than 1 %, or not more than 0.5 %, or not more than 0.1 %.
116. The packaging according to any of items 113 to 115, wherein all administration units are substantially identical.
117. The packaging according to any of items 113 to 116 for use in the treatment of a disorder or a disease, wherein one or more administration units of said multitude are administered on a first day, and one or more administration units of said multitude are administered on a second day following said first day.
118. The packaging according to item 117, wherein one or more administration units of said multitude are administered on one or more subsequent days following said second day.
119. The packaging according to any of items 117 to 118, wherein the time interval between the administration of an administration unit and the administration of a subsequent administration unit is not more than 48 hours.
120. The packaging according to item 119, wherein the time interval is not more than 36 hours, or not more than 24 hours, or not more than 12 hours, or not more than 8 hours, or not more than 6 hours, or not more than 4 hours.
121. The packaging according to any of items 117 to 120, wherein the administration units are administered on not more than 30 subsequent days.
122. The packaging according to item 121, wherein the administration units are administered on not more than 25 subsequent days, or on not more than 20 subsequent days, or on not more than 14 subsequent days, or on not more than 10 subsequent days, or on not more than 7 subsequent days, or on not more than 3 subsequent days.
123. The packaging according to any of items 117 to 122, wherein the administered daily dose of (i) {drug1}, (ii) {drug2}, (iii) {drug3}, (iv) {drug4}, (v) {drug5}, (vi) {drug6}, or (vii) {drug7} is 0.0001 mg/kg body weight to 100 mg/kg body weight.

124. The packaging according to item 123, wherein the administered daily dose of (i) {drug1}, (ii) {drug2}, (iii) {drug3}, (iv) {drug4}, (v) {drug5}, (vi) {drug6}, or (vii) {drug7} is 0.0001 mg/kg body weight to 0.0003 mg/kg body weight, or 0.0003 mg/kg body weight to 0.0005 mg/kg body weight, or 0.0005 mg/kg body weight to 0.001 mg/kg body weight, or 0.001 mg/kg body weight to 0.0025 mg/kg body weight, or 0.0025 mg/kg body weight to 0.005 mg/kg body weight, or 0.005 mg/kg body weight to 0.01 mg/kg body weight, or 0.01 mg/kg body weight to 0.025 mg/kg body weight, or 0.025 mg/kg body weight to 0.05 mg/kg body weight, or 0.05 mg/kg body weight to 0.1 mg/kg body weight, or 0.1 mg/kg body weight to 0.25 mg/kg body weight, or 0.25 mg/kg body weight to 0.5 mg/kg body weight, or 0.5 mg/kg body weight to 1 mg/kg body weight, or 1 mg/kg body weight to 2.5 mg/kg body weight, or 2.5 mg/kg body weight to 5 mg/kg body weight, or 5 mg/kg body weight to 10 mg/kg body weight, or 10 mg/kg body weight to 25 mg/kg body weight, or 25 mg/kg body weight to 50 mg/kg body weight, or 50 mg/kg body weight to 100 mg/kg body weight.

125. The packaging according to any of items 117 to 124, wherein the administered daily dose of (i) {drug1}, (ii) {drug2}, (iii) {drug3}, (iv) {drug4}, (v) {drug5}, (vi) {drug6}, or (vii) {drug7} is 0.001 mg to 5000 mg.

126. The packaging according to item 125, wherein the administered daily dose of (i) {drug1}, (ii) {drug2}, (iii) {drug3}, (iv) {drug4}, (v) {drug5}, (vi) {drug6}, or (vii) {drug7} is 0.001 mg to 0.003 mg, or 0.003 mg to 0.005 mg, or 0.005 mg to 0.01 mg, or 0.01 mg to 0.025 mg, or 0.025 mg to 0.05 mg, or 0.05 mg to 0.1 mg, or 0.1 mg to 0.25 mg, or 0.25 mg to 0.5 mg, or 0.5 mg to 1 mg, or 1 mg to 2.5 mg, or 2.5 mg to 5 mg, or 5 mg to 10 mg, or 10 mg to 25 mg, or 25 mg to 50 mg, or 50 mg to 100 mg, or 100 mg to 250 mg, or 250 mg to 500 mg, or 500 mg to 1000 mg, or 1000 mg to 1250 mg, or 1250 mg to 1500 mg, or 1500 mg to 1750 mg, or 1750 mg to 2000 mg.

127. The packaging according to any of items 117 to 126, not comprising any administration unit comprising an active pharmaceutical ingredient other than (i) {drug1}, (ii) {drug2}, (iii) {drug3}, (iv) {drug4}, (v) {drug5}, (vi) {drug6}, or (vii) {drug7}.

128. A method for manufacturing an administration unit according to any of items 1 to 109 or a packaging according to any of items 110 to 127, comprising the steps: (a) providing a quantity of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} that exceeds the dose of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} to be contained in a single administration unit by a factor of at least 10; (b) mixing the quantity of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} with one or more pharmaceutical excipients; (c) dividing the mixture obtained in step (b) in fractions each containing the dose of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} to be contained in a single administration unit; (d) optionally, forming the fractions obtained in step (c) to administration units; and (e) optionally, packing the fractions obtained in step (c) and the administration units obtained in step (d), respectively.

129. The method according to item 133, wherein in step (a) the quantity of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} exceeds the dose of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} to be contained in a single administration unit by a factor of at least 25, or at least 50, or at least 100, or at least 250, or at least 500, or at least 1000, or at least 2500, or at least 5000, or at least 10,000.

130. Particles of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} having a particle size distribution X90 of 500 μm or less.

131. The particles of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} according to item 130, having a particle size distribution X90 of 480 μm or less, or 460 μm or less, or 440 μm or less, or 420 μm or less, or 400 μm or less, of 380 μm or less, or 360 μm or less, or 340 μm or less, or 320 μm or less, or 300 μm or less, of 280 μm or less, or 260 μm or less, or 240 μm or less, or 220 μm or less, or 200 μm or less, of 180 μm or less, or 160 μm or less, or 140 μm or less, or 120 μm or less, or 100 μm or less, or 80 μm or less, or 60 μm or less, or 40 μm or less, or 20 μm or less.

132. The particles of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} according to any of items 130 to 131, having a particle size distribution X50 of 400 μm or less.

133. The particles of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} according to item 132, having a particle size distribution X50 of 380 μm or less, or 360 μm or less, or 340 μm or

less, or 320 μm or less, or 300 μm or less, of 280 μm or less, or 260 μm or less, or 240 μm or less, or 220 μm or less, or 200 μm or less, 180 μm or less, or 160 μm or less, or 140 μm or less, or 120 μm or less, or 100 μm or less, or 80 μm or less, or 70 μm or less, or 60 μm or less, or 50 μm or less.

134. The particles of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} according to item 133, having a particle size distribution X50 of 45 μm or less, or 40 μm or less, or 35 μm or less, or 30 μm or less, or 25 μm or less, or 20 μm or less, or 15 μm or less, or 10 μm or less.

135. The particles of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} according to any of items 130 to 134, having a cumulative distribution by volume basis Q3(X) (X in μm) of Q3(355) <0.50; or Q3(180) <0.50 and Q3(355) ≥0.50; or Q3(125) <0.50 and Q3(180) ≥0.50; or Q3(125) ≥0.50.

136. The particles of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} according to any of items 130 to 135, having a cumulative distribution by volume basis Q3(X) (X in μm) of Q3(268) <0.50 and Q3(355) ≥0.50; or Q3(180) <0.50 and Q3(268) ≥0.50; or Q3(153) <0.50 and Q3(180) ≥0.50; or Q3(125) <0.50 and Q3(153) ≥0.50; or Q3(111) <0.50 and Q3(125) ≥0.50; or Q3(98) <0.50 and Q3(111) ≥0.50; or Q3(98) ≥0.50.

137. The particles of (i) {drug1a}, {drug1b}, {drug1c}, {drug1d}, or {drug1e}; (ii) {drug2a}; (iii) {drug3a}; (iv) {drug4a}, {drug4b}, {drug4c}, {drug4d}, {drug4e}, {drug4f}, or {drug4g}; (v) {drug5a}, or {drug5b}; (vi) {drug6a}; or (vii) {drug7a} according to any of items 130 to 136, having a cumulative distribution by volume basis Q3(X) (X in μm) of Q3(312) <0.50 and Q3(355) ≥0.50; or Q3(268) <0.50 and Q3(312) ≥0.50; or Q3(224) <0.50 and Q3(268) ≥0.50; or Q3(180) <0.50 and Q3(224) ≥0.50; or Q3(167) <0.50 and Q3(180) ≥0.50; or Q3(153) <0.50 and Q3(167) ≥0.50; or Q3(139) <0.50 and Q3(153) ≥0.50; or Q3(125) <0.50 and Q3(139) ≥0.50; or Q3(111) <0.50 and Q3(125) ≥0.50; or Q3(98) <0.50 and Q3(111) ≥0.50; or Q3(84) <0.50 and Q3(98) ≥0.50; or Q3(84) ≥0.50.

## Claims

1. A packaging comprising one or more administration units comprising a compound of formula

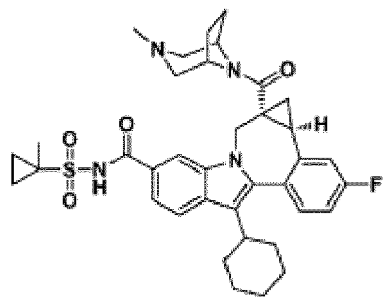

and/or a pharmaceutically acceptable salt thereof.

2. The packaging according to claim 1, comprising a material selected from the group consisting of paper, cardboard, paperboard, metal foil and plastic foil.

3. The packaging according to any of claims 1 to 2, wherein the administration unit is solid, semisolid or liquid.

4. The packaging according to any of claims 1 to 3, wherein the administration unit is for administration once daily, or twice daily, or thrice daily, or four times daily, or five times daily, or six times daily.

5. The packaging according to any of claims 1 to 4, wherein the administration unit provides the peak plasma level within 0.1 hour to 36 hours after administration.

6. The packaging according to any of claims 1 to 5, comprising a multitude of at least 2, or at least 3, or at least 4, or at least 5, or at least 6, or at least 7, or at least 8, or at least 9, or at least 10 administration units according to any of claims 1 to 5.

7. The packaging according to claim 6, wherein the administration units do not substantially differ in at least one

property selected from the group consisting of nature of excipients, content of excipients, content of compound and/or pharmaceutically acceptable salt thereof, water content, total weight, release profile, disintegration time, size, shape, porosity, apparent density, true density, average pore size, pore size distribution, axial tensile strength, radial tensile strength, friability, and storage stability.

**8.** The packaging according to any of claims 1 to 7, wherein all administration units are substantially identical.

**9.** The packaging according to any of claims 1 to 8, wherein the administration unit is for oral, buccal, opthalmic, nasal, rectal, transmucosal, or intestinal administration.

**10.** The packaging according to any of claims 6 to 9 for use in the treatment of a disorder or a disease, wherein one or more administration units of said multitude are administered on a first day, and one or more administration units of said multitude are administered on a second day following said first day.

**11.** The packaging according to claim 10, wherein one or more administration units of said multitude are administered on one or more subsequent days following said second day.

**12.** The packaging according to any of claims 10 to 11, wherein the time interval between the administration of an administration unit and the administration of a subsequent administration unit is not more than 48 hours.

**13.** The packaging according to any of claims 1 to 12, wherein the administration unit is for use in the treatment of a disorder or a disease in a mammal.

**14.** The packaging according to claim 13, wherein the mammal is a human.

**15.** The packaging according to claim 13 or 14, wherein the disorder or disease is hepatitis C.

Europäisches Patentamt
European Patent Office
Office européen des brevets

# EUROPEAN SEARCH REPORT

Application Number
EP 14 16 5031

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D,P | WO 2013/059265 A1 (SQUIBB BRISTOL MYERS CO [US]) 25 April 2013 (2013-04-25)<br>* the whole document *<br>----- | 1-15 | INV.<br>A61K31/55<br>C07D487/04 |
| Y | US 7 399 758 B2 (MEANWELL NICHOLAS A [US] ET AL) 15 July 2008 (2008-07-15)<br>* the whole document *<br>----- | 1-15 | |
| Y | US 8 129 367 B2 (HEWAWASAM PIYASENA [US] ET AL) 6 March 2012 (2012-03-06)<br>* the whole document *<br>----- | 1-15 | |

TECHNICAL FIELDS SEARCHED (IPC)
A61K
C07D

The present search report has been drawn up for all claims

1

| Place of search | Date of completion of the search | Examiner |
|---|---|---|
| Munich | 12 September 2014 | Engl, Brigitte |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 14 16 5031

This annex lists the patent family members relating to the patent documents cited in the above-mentioned European search report. The members are as contained in the European Patent Office EDP file on
The European Patent Office is in no way liable for these particulars which are merely given for the purpose of information.

12-09-2014

| Patent document cited in search report | Publication date | Patent family member(s) | Publication date |
|---|---|---|---|
| WO 2013059265 A1 | 25-04-2013 | AR 088470 A1 | 11-06-2014 |
| | | EP 2768831 A1 | 27-08-2014 |
| | | TW 201323426 A | 16-06-2013 |
| | | US 2013102589 A1 | 25-04-2013 |
| | | UY 34400 A | 31-05-2013 |
| | | WO 2013059265 A1 | 25-04-2013 |
| US 7399758 B2 | 15-07-2008 | AR 057810 A1 | 19-12-2007 |
| | | PE 06762007 A1 | 10-08-2007 |
| | | US 2007060565 A1 | 15-03-2007 |
| | | US 2007184024 A1 | 09-08-2007 |
| | | WO 2007033032 A1 | 22-03-2007 |
| US 8129367 B2 | 06-03-2012 | CN 101918406 A | 15-12-2010 |
| | | DK 2209784 T3 | 29-10-2012 |
| | | EP 2209784 A1 | 28-07-2010 |
| | | EP 2518073 A1 | 31-10-2012 |
| | | ES 2390732 T3 | 16-11-2012 |
| | | HR P20120707 T1 | 30-09-2012 |
| | | JP 5306366 B2 | 02-10-2013 |
| | | JP 2011504179 A | 03-02-2011 |
| | | PT 2209784 E | 09-10-2012 |
| | | SI 2209784 T1 | 28-02-2013 |
| | | US 2009130057 A1 | 21-05-2009 |
| | | US 2012115847 A1 | 10-05-2012 |
| | | WO 2009067481 A1 | 28-05-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 13164373 A
- EP 13165452 A
- EP 13165455 A
- EP 13165458 A
- EP 13168149 A
- EP 13168148 A
- EP 13169045 A
- WO 2013055584 A
- WO 2013059265 A
- WO 2013057722 A
- WO 2013057124 A
- WO 2013071272 A
- WO 2013068909 A
- WO 2013074432 A
- WO 2010129500 A
- WO 03024222 A
- US 3060084 A
- WO 9113546 A
- US 4172714 A
- US 4144050 A
- US 3920442 A
- DE 3246493
- US 5180587 A
- US 5232701 A
- US 5208030 A
- GB 2095558 A
- US 3299566 A
- US 3235361 A
- US 3309 A
- US 192 A
- US 2891855 A
- US 6406690 B
- US 7399758 B
- US 7485633 B
- US 2009130057 A
- WO 2005047288 A
- WO 2010079443 A
- US 6106864 A
- WO 0035298 A
- WO 9111172 A
- WO 9402518 A
- WO 9855148 A
- WO 2009071476 A
- US 6534524 B
- US 6884890 B
- US 7232910 B
- US 20060091067 A
- US 20070203196 A
- WO 2006048745 A
- US 20040224988 A
- US 20060094763 A
- US 20080274192 A
- US 20100179329 A
- WO 2007015870 A
- WO 2007015871 A


### Non-patent literature cited in the description


- **GOODMAN C et al.** *Health Policy Plan.,* November 2007, vol. 22 (6), 393-403
- **SOW PS et al.** *Int J Infect Dis.,* June 2002, vol. 6 (2), 108-12
- **MARSDEN.** Solvents Guide. Interscience, 1950
- **MCCUTCHEON 'S.** Functional Materials, annual International and North American. McCutcheon's Division, The Manufacturing Confectioner Publishing Co, vol. 2
- Agglomeration. **BROWNING.** Chemical Engineering. 04 December 1967
- Perry 's Chemical Engineer 's Handbook. McGraw-Hill, 1963, 8-57
- The Formulator's Toolbox - Product Forms for Modern Agriculture. **T. S. WOODS.** Pesticide Chemistry and Bioscience. The Royal Society of Chemistry, 1999, 120-133
- **KLINGMAN.** Weed Control as a Science. John Wiley and Sons, Inc, 1961, 81-96
- **HANCE et al.** Weed Control Handbook. Blackwell Scientific Publications, 1989
- Developments in formulation technology. PJB Publications, 2000

- The Pesticide Manual. British Crop Protection Council, 2003
- The BioPesticide Manual. British Crop Protection Council, 2001
- **ERIC CHAK.** *Liver International,* 2011, 1090-1 101
- **LAUER, G. M. ; WALKER, B. D. N.** *Engl. J. Med.,* 2001, vol. 345, 41-52
- **TOMEI L ; ALTAMURA S ; PAONESSA G et al.** *Antivir Chem Chemother,* 2005, vol. 16, 225-245
- **BRESSANELLI; S. et al.** *Journal of Virology,* 2002, 3482-3492
- **DEFRANCESCO ; RICE.** *Clinics in Liver Disease,* 2003, vol. 7, 21 1-242
- **LESBURG C.A. ; CABLE, M.B. ; FERRARI et al.** *Nat Struc Biol,* 1999, vol. 6, 937-943
- **EL HAGE N ; LUO G.** *J Gen Virol,* 2003, vol. 84, 2761-2769
- **GAO L ; AIZAKI H ; HE JW et al.** *J Virol,* 2004, vol. 75, 3480-3488
- **JENNINGS et al.** *Biochemistry,* 2008, vol. ^7, 1126-1135
- **MCCORMICK CJ ; BROWN D ; GRIFFIN S et al.** *J Gen Virol,* 2006, vol. 87, 93-102
- **GELMAN, MA ; JS. GLENN.** Mixing the right hepatitis C inhibitor cocktail. *Trends in Molecular Medicine,* 2011, vol. 17 (1), 34-46
- **SORIANO V. ; E. VISPO ; E. POVEDA ; P. LABARGA ; L. MARTIN- CARBONERO ; JV. FEMANDEZ-MONTERO ; P. BARREIRO.** Directly acting antivirals against hepatitis C virus. *J Antimicrob Chemother,* 2011, vol. 66, 1673-1686
- **MA FL ; V. LEVEQUE ; A. DE WITTE ; W. LI ; T. HENDRICKS ; SM. CLAUSEN ; N. CAMMACK ; K. KLUMPP.** Inhibition of native hepatitis C virus replicase by nucleotide and non-nucleoside inhibitors. *Virology,* 2005, vol. 332, 8-15
- **BEAULIEU, P.** Recent advances in the development of NS5B polymerase inhibitors for the treatment of hepatitis C virus infection. *Expert Opinion on Therapeutic Patents,* 2009, vol. 19, 145-64
- **POYNARD, T. et al.** *Lancet,* vol. 352, 1426-1432
- **ZEUZEM, S. et al.** *N. Engl. J. Med.,* 2000, vol. 343, 1666-1672
- **GHANY, MG. ; DR. NELSON ; DB. STRADER ; DL. THOMAS ; LB. SEEFF.** An Update on Treatment of Genotype 1 Chronic Hepatitis C Virus Infection: 201 1 Practice Guideline by the American Association for the Study of Liver Diseases. *Hepatology,* 2011, vol. 54 (4), 1433-1444
- **GELMAN, MA. ; JS. GLENN.** Mixing the right hepatitis C inhibitor cocktail. *Trends in Molecular Medicine,* 2011, vol. 1, 34-46
- **SORIANO V. ; E. VISPO ; E. POVEDA ; P. LABARGA ; L. MARTIN-CARBONERO ; JV. FERNANDEZ-MONTERO ; P. BARREIRO.** Directly acting antivirals against hepatitis C virus. *J Antimicrob Chemother,* 2011, vol. 66, 1673-1686
- **REDFERN, W. S. ; CARLSSON, L. ; DAVIS, A. S. ; LYNCH, W. G. ; MACKENZIE, I. ; PALETHORPE, S. ; SIEGL, P. K. S. ; STRANG, I. ; SULLIVAN, A. T. ; WALLIS, R.** Relationships between preclinical cardiac electrophysiology, clinical QT interval prolongation and torsade de pointes for a broad range of drugs: evidence for a provisional safety margin in drug development. *Cardiovascular Research,* 2003, vol. 58 (1), 32-45
- **DE BRUIN, M. L. ; PETTERSSON, M. ; MEYBOOM, R. H. B. ; HOES, A. W. ; LEUFKENS, H. G. M.** Anti-HERG activity and the risk of drug-induced arrhythmias and sudden death. *European Heart Journal,* 2005, vol. 26 (6), 590-597
- Remington 's Pharmaceutical Sciences. Mack Publishing Company, 1985
- *Synlett,* 2006, vol. 5, 725-278
- **WANGY-K ; RIGAT K ; ROBERTS S ; GAO M.** *Anal Biochem,* 2006, vol. 359, 106-1 11
- Remington's Pharmaceutical Sciences. Mack Publishing Company, 1995
- **LIANG ; CHEN.** *Expert Opinion in Therapeutic Patents,* 2001, vol. V (6), 981-986
- **H. LIEBERMAN ; L. LACHMAN.** Pharmaceutical Dosage Forms: Tablets. 1980, vol. 1
- **VERMA et al.** *Pharmaceutical Technology On-line,* 2001, vol. 25 (2), 1-14
- **FINNIN ; MORGAN.** *J Pharm Sci,* October 1999, vol. 88 (10), 955-958
- **W. MCCRONE.** *Phys. Chem. Org. Solid State,* 1965, vol. 2, 725767
- **D. GIRON.** *J. Therm. Anal. Cal.,* 2004, vol. 77, 709
- **S.L. MORISSETTE et al.** *Adv. Drug Del. Rev.,* 2004, vol. 56, 275-300
- **V.J. STELLA.** *Adv. Drug Del. Rev.,* 2007, vol. 59, 677-694
- **T. UYAR et al.** *Cryst. Growth Des.,* 2006, vol. 6, 1113-1119
- **T. TOROPAINEN et al.** *Pharm. Res.,* 2007, vol. 24, 1058-1066
- **I.C. MUNRO et al.** *Regulatory Toxicology and Pharmacology,* 2004, vol. 39, S3-S13
- **T. UYAR.** *Cryst. Growth Des.,* 2006, vol. 6, 1113-1119
- **G.L. AMIDON ; H. LENNERNAS ; V.P. SHAH ; J.R. CRISON.** *Pharm. Res.,* 1995, vol. 12, 413-420
- IUPAC - Compendium of Chemical Terminology. Blackwell Scientific Publications, 1997
- **W.H. POWELL.** *Pure & Appl. Chem.,* 1984, vol. 56 (6), 769-778
- **T. TOROPAINEN.** *Pharm. Res.,* 2007, vol. 24, 1058-1066

- **ANSEL H.C.** Ansel's Pharmaceutical Dosage Forms and Drug Delivery Systems. Lippincott, Williams & Wilkins, 2004
- **GENNARO A.R.** Remington: The Science and Practice of Pharmacy. Lippincott, Williams & Wilkins, 2000
- **ROWE R.C.** Handbook of Pharmaceutical Excipients. Pharmaceutical Press, 2005
- **T. HIGUCHI et al.** *Adv. Anal. Chem. Instrum.,* 1965, vol. 4, 117-212
- **M. E. BREWSTER et al.** *Adv. Drug Delivery Rev.,* 2007, vol. 59, 645-666
- **GALIA E. et al.** *Pharm. Res.,* 1996, vol. 13, S-262
- **SCHWARTZ, P.A. et al.** *Bioorg Chem,* 2011, vol. 39, 192
- **O'HARE, T. et al.** *Clin Cancer Res,* 2011, vol. 17, 212
- **HU-LOWE, D.D. et al.** *Clin Cancer Res,* 2008, vol. 14, 7272-7283
- **SOLOWIEJ, S. et al.** *Biochemistry,* 2009, vol. 48, 7019-31
- **SOLOWIEJ, J.** *Biochemistry,* 2009, vol. 48, 7019-7031
- **MORRISON, J. F.** *Biochimica et biophysica acta,* 1969, vol. 185, 269-286
- The Handbook of Pharmaceutical Excipients. American Pharmaceuticals Association, 2003
- Remington: the Science and Practice of Pharmacy. Lippincott Williams & Wilkins, 2000
- **K.D. HARRIS.** Powder diffraction crystallography of molecular solids. *Top Curr Chem.,* 2012, vol. 315, 133-77
- *J Pharm Biomed Anal.,* 2011, vol. 55 (4), 618-44
- **R. HILFIKER.** Polymorphism. Wiley-VCH, 2006
- Polymorphism in Pharmaceutical Solids. **H.G. BRITTAIN.** Drugs and the Pharmaceutical Sciences. Informa Healthcare, 2009
- **H.G. BRITTAIN ; S.J. BODANOWICH ; D.E. BUGAY ; J. DEVONCENTIS ; G. LEWEN ; A.W. NEWMAN.** Physical characterization of pharmaceutical solids. *Pharm Res.,* 1991, vol. 8 (8), 963-73
- **D.E. BUGAY.** Characterization of the solid-state: spoectroscopic techniques. *Adv Drug Deliv Rev.,* 2001, vol. 48 (1), 43-65
- **P.A. TISHMACK ; D.E. BUGAY ; S.R. BYM.** Solid-state nuclear magnetic resonance spectroscopy - pharmaceutical application. *J Pharm Sci,* 2003, vol. 92 (3), 441-74
- **C.J. LEE ; C.J. STRACHAN ; P.J. MANSON ; T. RADES.** Characterization of the bulk properties of pharmaceutical solids using nonlinear optics - a review. *J Pharm Pharmacol.,* 2007, vol. 59 (2), 241-50
- **R.A. STOREY.** Solid State Characterization of Pharmaceuticals. Wiley-Blackwell, 2011
- **FELL, J.T. ; NEWTON, J.M.** Determination of tablet strength by diametral compression test. *J. Pharm. Sci.,* 1970, vol. 59, 688-691
- **NYSTRÖM, C. ; MALMQVIST, K. ; MAZUR, J. ; ALEX, W. ; HÖLZER, A.W.** Measurement of axial and radial tensile strength of tablets and their relation to capping. *Acta Pharm.,* 1978, vol. 15, 226-232
- **STANLEY-WOOD, N.G. ; JOHANSSON, M.E.** Variation of intra- and inter-particle porosity with degree of compaction. *Analyst,* 1980, vol. 105, 1104-1112
- **WESTERMARCK, S. ; JUPPO, A.M. ; KERVINEN, L. ; YLIRUUSI, J.** Pore structure and surface area of mannitol powder, granules and tablets determined with mercury porosimetry and nitrogen adsorption. *Eur. J. Pharm. Biopharm.,* 1998, vol. 46, 61-86
- **JUPPO, A.M.** Relationship between breaking force and pore structure of lactose, glucose and mannitol tablets. *Int. J. Pharm.,* 1996, vol. 127, 95-102
- Characterization of Porous Solids and Powders: Surface Area, Pore Size and Density. **S. LOWELL et al.** Particle Technology Series. Springer, 2010
- **N. RASENACK ; B.W. MÜLLER.** Micron-size drug particles: common and novel micronization techniques. *Pharm Dev Technol.,* 2004, vol. 9 (1), 1-13
- **A. MARTIN ; M.J. COCERO.** Micronization processes with supercritical fluids: fundamentals and mechanisms. *Adv Drug Deliv Rev.,* 2008, vol. 60 (3), 339-50
- Pharmaceutical Manufacturing Handbook: Production and Processes. **S.C. GAD.** Pharmaceutical Development Series. Wiley Interscience, 2008
- **A.J. HICKEY.** Pharmaceutical Process Engineering. *Drugs and the Pharmaceutical Sciences,* 2009
- **B. NICKERSON.** Sample Preparation of Pharmaceutical Dosage Forms: Challenges and Strategies for Sample Preparation and Extraction. Springer, 2011
- **D. DRAGOMAN.** Optical Characteriaztion of Solids. Springer, 2010
- Powders and Bulk Solids: Behavior, Characterization. **D. SCHULZE.** Storage and Flow. Springer, 2008